(19) Europäisches Patentamt
European Patent Office
Office européen des brevets

(11) EP 1 238 970 B1

(12) **EUROPEAN PATENT SPECIFICATION**

(45) Date of publication and mention of the grant of the patent:
**22.11.2006 Bulletin 2006/47**

(21) Application number: **00979945.3**

(22) Date of filing: **06.12.2000**

(51) Int Cl.:
*C07D 207/09* (2006.01) *C07D 211/26* (2006.01)
*C07D 405/12* (2006.01) *C07D 409/12* (2006.01)
*C07D 401/12* (2006.01) *C07D 401/04* (2006.01)
*C07D 409/14* (2006.01) *C07D 405/14* (2006.01)
*C07D 401/14* (2006.01) *C07D 401/06* (2006.01)
*A61K 31/40* (2006.01) *A61K 31/4025* (2006.01)
*A61K 31/445* (2006.01)

(86) International application number:
**PCT/JP2000/008627**

(87) International publication number:
**WO 2001/042208 (14.06.2001 Gazette 2001/24)**

(54) **CYCLOAMINE CCR5 RECEPTOR ANTAGONISTS**

ZYKLISCHE AMINVERBINDUNGEN ALS CCR5-REZEPTOR ANTAGONISTEN

ANTAGONISTES DU RECEPTEUR CCR5 DE LA CYCLOAMINE

(84) Designated Contracting States:
**AT BE CH CY DE DK ES FI FR GB GR IE IT LI LU MC NL PT SE TR**

(30) Priority: **08.12.1999 JP 34877899**

(43) Date of publication of application:
**11.09.2002 Bulletin 2002/37**

(73) Proprietor: **TEIJIN LIMITED**
**Osaka-shi**
**Osaka 541-0054 (JP)**

(72) Inventors:
- **SHIOTA, Tatsuki**
  **c/o Teijin Limited**
  **Hino-shi, Tokyo 191-0065 (JP)**
- **YOKOYAMA, Tomonori**
  **c/o Teijin Limited**
  **Hino-shi, Tokyo 191-0065 (JP)**
- **KAMIMURA, Takashi**
  **c/o Teijin Limited**
  **Hino-shi, Tokyo 191-0065 (JP)**

(74) Representative: **Hallybone, Huw George et al**
**Carpmaels and Ransford,**
**43 Bloomsbury Square**
**London WC1A 2RA (GB)**

(56) References cited:
**EP-A- 1 201 239** **WO-A-98/30218**
**WO-A-98/50534** **WO-A-99/01127**
**WO-A-99/25686**

- **RAPORT C.J.: 'Molecular cloning and functional characterization of a novel human CC chemokine receptor (CCR5) for RANTES, MIP-1 beta and MIP-1 alpha' J. BIOL. CHEM. vol. 271, no. 29, 1996, pages 17161 - 17166, XP002936301**
- **MURAI M.: 'Active participation of CCR5+CD8+ T lymphocytes in the pathogenesis of liver injury in graft-versus-host disease' J. CLIN. INVEST. vol. 104, no. 1, July 1999, pages 49 - 57, XP002936302**
- **BALASHOV K.E.: 'CCR5+ and CXCR3+ T cells are increased in multiple sclerosis and their ligands MIP-1 alpha and IP-10 are expressed in demyelinating brain lesions' PROC. NATL. ACAD. SCI. USA vol. 96, no. 12, June 1999, pages 6873 - 6878, XP002936303**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 238 970 B1

## Description

Technical Field

The present invention relates to the use of CCR5 antagonists in the manufacture of a medicament for the treatment of diseases associated with CCR5 selected from the group consisting of rejection after organ transplantation, graft-versus host diseases and diabetes.

Background Art

The CCR5 is a receptor for MIP-1α (an abbreviation for macrophage inflammatory protein-1α), MIP-1β (an abbreviation for macrophage inflammatory protein-1β) or RANTES (an abbreviation for regulated upon activation normal T-cell expressed and secreted) and is known to be expressed in lymphoid tissues such as thymus and spleen, monocytes/macrophages, T-cells or the like (see, for example, Samson, M. et al., Boichemistry, 1996, 35, 3362; Raport, C.J. et al., J. Biol. Chem., 1996, 271, 17161, and Combadiere, C. et al., J. Leukoc. Biol., 1996, 60, 147).

As to information about the relationship between the CCR5 and diseases, it has been reported that the CCR5 was expressed in leukocytes such as T-cells in arthrosynovial tissues and synovial fluid of patients suffering from rheumatoid arthritis (see Loetscher, P. et al., Nature, 1998, 391, 344; Mack, M. et al., Arthritis Rheum., 1999, 42, 981 and the like), CCR5 deficient homozygotes were not found in patients suffering from rheumatoid arthritis (see Gomez-Reino, J.J. et al., Arthritis Rheum., 1999, 42, 989), CCR5 was expressed in T-cells in renal biopsy samples of patients suffering from glomerulonephritis, interstitial nephritis and rejection after transplantation (see Segerer, S. et al., Kidney Int., 1999, 56, 52), many T-cells expressing CCR5 were found in blood of patients suffering from multiple sclerosis (see Balashov, K.E., Proc. Natl. Acad. Sci. USA, 1999, 96, 6873), CCR5 was expressed in T-cells infiltrated into liver injury sites of a mouse graft-versus-host disease (GVHD) model and the infiltration of the T-cells was suppressed by administration of an anti-CCR5 antibody (see Murai, M. et al., J. Clin. Invest., 1999, 104, 49), the progression of morbid states in a mouse diabetes model was associated with MIP-1α and CCR5 (see Cameron, M.J. et al., J. Immunol., 2000, 165, 1102) and the like.

Accordingly, CCR5 is thought to be associated with initiation, progression and maintenance of diseases in which the accumulation and activation of monocytes/macrophages and/or T-cells in disease sites can be assumed to be deeply associated with progression of lesions, for example rheumatoid arthritis, nephritis (nephropathy), multiple sclerosis, rejection after organ transplantation, graft-versus-host diseases (GVHD) and diabetes.

Furthermore, based on a report that the CCR5 is specifically expressed in Th1 cells among the T-cells, CCR5 is thought to be associated with initiation, progression and maintenance of many autoimmune diseases and inflammatory diseases such as chronic obstructive pulmonary diseases (COPD), asthma, atopic dermatitis, sarcoidosis, fibrosis, atherosclerosis, psoriasis and inflammatory bowl diseases in which Th1 cells can be assumed to be associated with morbid states including the above diseases (see Bonecchi, R. et al., J. Exp. Med., 1998, 187, 129; Loetscher, P. et al., Nature, 1998, 391, 344 and the like).

On the other hand, although CD4 is known as a receptor when a host cell is infected with HIV (human immunodeficiency virus), it has been suggested that a second receptor (a coreceptor receptor) is necessary because the infection of HIV is not established only with the CD4. Usually, HIV-1 is roughly classified into a macrophage-tropic (M-tropic) strain and a T-cell-tropic (T-trophic) strain depending on the species of cells that the virus can infect, and it has been elucidated that a coreceptor essential to the infection of the macrophage-tropic strain is CCR5 (see, for example, Deng, H. et al., Nature, 1996, 381, 661; Dragic, T. et al., Nature, 1996, 381, 667; Alkhatib, G. et al., Science, 1996, 272, 1955; Choe, H. et al., Cell, 1996, 85,1135; and Doranz, B.J. et al., Cell, 1996, 85,1149).

Therefore, drugs capable of inhibiting the binding of HIV-1 to CCR5 are thought to be effective as new remedies and/or prophylactics for AIDS (acquired immunodeficiency syndrome) (see Michael, N.L. et al., Nature Med., 1999, 5, 740; Proudfoot, A.E.I. et al., Biochem. Pharmacol., 1999, 57, 451; Murakami et al., Protein, Nucleic Acid and Enzyme, 1998, 43, 677 and the like). As information supporting the above inference, it has been reported that RANTES, MIP-1α and MIP-1β which are ligands of CCR5 were suppressive factors for HIV-1 infection (see Cocchi, F. et al., Science, 1995, 270, 1811), humans without expressing normal CCR5 at all by deficiency of 32 base pairs of CCR5 gene had resistance to HIV-1 infection and any other abnormality in health is not caused by the deficiency (see Liu, R. et al., Cell, 1996, 86, 367; Samson, M. et al., Nature, 1996, 382, 722; Dean, M. et al., Science, 1996, 273, 1856 and the like), anti-CCR5 monoclonal antibodies inhibited the infection of peripheral blood monocytes by macrophage-tropic HIV-1 (see Wu, L. et al., J. Exp. Med., 1997, 185, 1681), RANTES in which the amino terminals were missing or modified was an antagonist of the RANTES to inhibit the infection with macrophage-tropic HIV-1 (see Arenzana-Seisdedos, F. et al., Nature, 1996, 383, 400; Proost, P. et al., J. Biol. Chem., 1998, 273, 7222; Simmons, G. et al., Science, 1997, 276, 276 and the like) and the like.

As mentioned above, a compound which inhibits the binding of MIP-1α, MIP-1β or RANTES that is an in vivo

ligand of the CCR5 to the CCR5 or the binding of HIV-1 which is a pathogenic virus of AIDS to the CCR5, i.e. a CCR5 antagonist is thought to be useful as a remedy and/or prophylactic for diseases such as AIDS, rheumatoid arthritis, nephritis (nephropathy), multiple sclecrosis, rejection after organ transplantation, graft-versus-host diseases (GVHD), diabetes, chronic obstructive pulmonary diseases (COPD), asthma, atopic dermatitis, sarcoidosis, fibrosis, atherosclerosis, psoriasis or inflammatory bowel diseases.

It has recently been reported that substituted bis-acridine derivatives (see W09830218), substituted anilide derivatives (see WO9901127; WO0006085; WO0006146; WO0006153; WO0040239;and W00042852), substituted alkenanilide derivatives (see WO9932100; WO0010965; WO0037455; and Baba, et al., Proc. Natl. Acad. Sci. USA, 1999, 96, 5698), 3-(4-piperidinyl)indole derivatives (see WO9917773 and W0042045), azacycloalkane derivatives (see EP1013276; WO0038680; and WO0039125), benzodipyran derivatives (see W00053175) and pyrrolidine derivatives (see WO0059497; WO0059498; WO0059502; and WO0059503) have an antagonistic activity against CCR5. These compounds, however, are different from the compounds used in the present invention.

On the other hand, although the compounds used in the present invention are the same as those described in WO9925686, the compounds are not known to have the antagonistic activity against the CCR5.

Disclosure of the Invention

It is an object of the present invention to provide the use of a CCR5 antagonist in the manufacture of a medicament for the treatment of diseases associated with CCR5 selected from the group consisting of rejection after organ transplantation, graft-versus host diseases and diabetes.

As a result of intensive studies, the inventors have found that cyclic amine derivatives having an arylalkyl group, pharmaceutically acceptable $C_1$-$C_6$ alkyl addition salts thereof or pharmaceutically acceptable acid addition salts thereof have the CCR5 antagonistic activity. Furthermore, studies have been promoted according to findings that those compounds can be useful as remedies or prophylactics for diseases considered to be in association with CCR5. Thereby, the present invention has been accomplished.

Namely, according to the present invention, there are provided the use of a compound having the CCR5 antagonistic activity represented by the following formula (I), a pharmaceutically acceptable acid addition salt thereof or a pharmaceutically acceptable $C_{1-}C_6$ alkyl addition salt thereof, as an active ingredient:

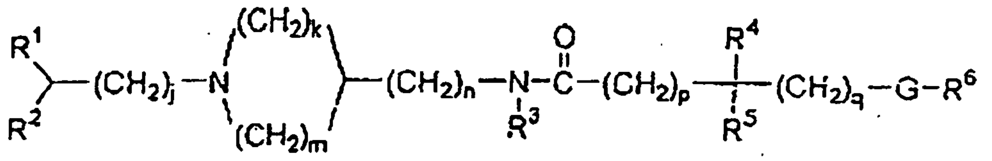
(I)

wherein, $R^1$ is a phenyl group, a $C_3$-$C_8$ cycloalkyl group or an aromatic heterocyclic group having one to three oxygen atoms, sulfur atoms and/or nitrogen atoms as heteroatoms; the phenyl group or the aromatic heterocyclic group in the above $R^1$ may be condensed with a benzene ring, or an aromatic heterocyclic group having one to three oxygen atoms, sulfur atoms and/or nitrogen atoms as heteroatoms to form a condensed ring; the phenyl group, the $C_3$-$C_8$ cycloalkyl group, the aromatic heterocyclic group or the condensed ring in the above $R^1$ may be substituted with an optional number of halogen atoms, hydroxy groups, cyano groups, nitro groups, carboxy groups, carbamoyl groups, $C_1$-$C_6$ alkyl groups, $C_3$-$C_8$ cycloalkyl groups, $C_2$-$C_6$ alkenyl groups, $C_1$-$C_6$ alkoxy groups, $C_1$-$C_6$ alkylthio groups, $C_3$-$C_5$ alkylene groups, $C_2$-$C_4$ alkylenoxy groups, $C_1$-$C_3$ alkylenedioxy groups, phenyl groups, phenoxy groups, phenylthio groups, benzyl groups, benzyloxy groups, benzoylamino groups, $C_2$-$C_7$ alkanoyl groups, $C_2$-$C_7$ alkoxycarbonyl groups, $C_2$-$C_7$ alkanoyloxy groups, $C_2$-$C_7$ alkanoylamino groups, $C_2$-$C_7$ N-alkylcarbamoyl groups, $C_4$-$C_9$ N-cycloalkylcarbamoyl groups, $C_1$-$C_6$ alkylsulfonyl groups, $C_3$-$C_8$ (alkoxycarbonyl)methyl groups, N-phenylcarbamoyl groups, piperidinocarbonyl groups, morpholinocarbonyl groups; 1-pyrrolidinylcarbonyl groups, bivalent groups represented by the formula: -NH(C=O)O-, bivalent groups represented by the formula:
-NH(C=S)O-, amino groups, mono($C_1$-$C_6$ alkyl)amino groups or di($C_1$-$C_6$ alkyl)amino groups; the substituents of the phenyl group, the $C_3$-$C_8$ cycloalkyl group, the aromatic heterocyclic group or the condensed ring may further be substituted with an optional number of halogen atoms, hydroxy groups, amino groups, trifluoromethyl groups, $C_1$-$C_6$ alkyl groups or $C_1$-$C_6$ alkoxy groups;
$R^2$ is a hydrogen atom, a $C_1$-$C_6$ alkyl group, a $C_2$-$C_7$ alkoxycarbonyl group, a hydroxy group or a phenyl group; the $C_1$-$C_6$ alkyl group or the phenyl group in the $R^2$ may be substituted with an optional number of halogen atoms, hydroxy groups, $C_1$-$C_6$ alkyl groups or $C_1$-$C_6$ alkoxy groups, with the proviso that $R^2$ is not a hydroxy group when j is 0;
j is an integer of 0 to 2;

k is an integer of 0 to 2;
m is an integer of 2 to 4;
n is 0 or 1;
$R^3$ is a hydrogen atom or a $C_1$-$C_6$ alkyl group which may be substituted with (one or two phenyl groups which may respectively be substituted with the same or different optional number of halogen atoms, hydroxy groups, $C_1$-$C_6$ alkyl groups or $C_1$-$C_6$ alkoxy groups);
$R^4$ and $R^5$ are the same or different and are each a hydrogen atom, a hydroxy group, a phenyl group or a $C_1$-$C_6$ alkyl group; the $C_1$-$C_6$ alkyl group in the $R^4$ and $R^5$ may be substituted with an optional number of halogen atoms, hydroxy groups, cyano groups, nitro groups, carboxy groups, carbamoyl groups, mercapto groups, guanidino groups, $C_3$-$C_8$ cycloalkyl groups, $C_1$-$C_6$ alkoxy groups, $C_1$-$C_6$ alkylthio groups, phenyl groups (which may be substituted with an optional number of halogen atoms, hydroxy groups, $C_1$-$C_6$ alkyl groups, $C_1$-$C_6$ alkoxy groups or benzyloxy groups), phenoxy groups, benzyloxy groups, benzyloxycarbonyl groups, $C_2$-$C_7$ alkanoyl groups, $C_2$-$C_7$ alkoxycarbonyl groups, $C_2$-$C_7$ alkanoyloxy groups, $C_2$-$C_7$ alkanoylamino groups, $C_2$-$C_7$ N-alkylcarbamoyl groups, $C_1$-$C_6$ alkylsulfonyl groups, amino groups, mono($C_1$-$C_6$ alkyl)amino groups, di($C_1$-$C_6$ alkyl)amino groups or (aromatic heterocyclic groups having one to three oxygen atoms, sulfur atoms and/or nitrogen atoms as heteroatoms or condensed rings formed by condensation of the aromatic heterocyclic groups having the one to three oxygen atoms, sulfur atoms and/or nitrogen atoms as the heteroatoms with a benzene ring), or both $R^4$ and $R^6$ together may form a three- to a six-membered cyclic hydrocarbon;
p is 0 or 1;
q is 0 or 1;
G is a group represented by -CO-, -$SO_2$-, -CO-O-, -$NR^7$-CO-, -CO-$NR^7$-, -NH-CO-NH-, -NH-CS-NH-, -$NR^7$-$SO_2$-, -$SO_2$-$NR^7$-, -NH-CO-O- or -O-CO-NH-, wherein, $R^7$ is a hydrogen atom or a $C_1$-$C_6$ alkyl group or $R^7$, together with $R^6$, may form a $C_2$-$C_6$ alkylene group;
$R^6$ is a phenyl group, a $C_3$-$C_8$ cycloalkyl group, a $C_3$-$C_6$ cycloalkenyl group, a benzyl group or an aromatic heterocyclic group having one to three oxygen atoms, sulfur atoms and/or nitrogen atoms as heteroatoms; the phenyl group, the benzyl group or the aromatic heterocyclic group in the $R^6$ may be condensed with a benzene ring or an aromatic heterocyclic group having one to three oxygen atoms, sulfur atoms and/or nitrogen atoms as heteroatoms to form a condensed ring; the phenyl group, the $C_3$-$C_8$ cycloalkyl group, the $C_3$-$C_6$ cycloalkenyl group, the benzyl group, the aromatic heterocyclic group or the condensed ring in the above $R^6$ may further be substituted with an optional number of halogen atoms, hydroxy groups; mercapto groups, cyano groups, nitro groups, thiocyanato groups, carboxy groups, carbamoyl groups, trifluoromethyl groups, $C_1$-$C_6$ alkyl groups, $C_3$-$C_8$ cycloalkyl groups, $C_2$-$C_6$ alkenyl groups, $C_1$-$C_6$ alkoxy groups, $C_3$-$C_8$ cycloalkyloxy groups, $C_1$-$C_6$ alkylthio groups, $C_1$-$C_3$ alkylenedioxy groups, phenyl groups, phenoxy groups, phenylamino groups, benzyl groups, benzoyl groups, phenylsulfinyl groups, phenylsulfonyl groups, 3-phenylureido groups, $C_2$-$C_7$ alkanoyl groups, $C_2$-$C_7$ alkoxycarbonyl groups, $C_2$-$C_7$ alkanoyloxy groups, $C_2$-$C_7$ alkanoylamino groups, $C_2$-$C_7$ N-alkylcarbamoyl groups, $C_1$-$C_6$ alkylsulfonyl groups, phenylcarbamoyl groups, N,N-di($C_1$-$C_6$ alkyl)sulfamoyl groups, amino groups, mono($C_1$-$C_6$ alkyl)amino groups, di($C_1$-$C_6$ alkyl)amino groups, benzylamino groups, $C_2$-$C_7$ (alkoxycarbonyl)amino groups, $C_1$-$C_6$ (alkylsulfonyl)amino groups or bis($C_1$-$C_6$ alkylsulfonyl)amino groups; the substituents of the phenyl group, the $C_3$-$C_8$ cycloalkyl group, the $C_3$-$C_8$ cycloalkenyl group, the benzyl group, the aromatic heterocyclic group or the condensed ring may further be substituted with an optional number of halogen atoms, cyano groups, hydroxy groups, amino groups, trifluoromethyl groups, $C_1$-$C_6$ alkyl groups, $C_1$-$C_6$ alkoxy groups, $C_1$-$C_6$ alkylthio groups, mono($C_1$-$C_6$ alkyl)amino groups or di($C_1$-$C_6$ alkyl)amino groups, in the manufacture of a medicament for the treatment of diseases associated with CCR5 selected from the group consisting of rejection after organ transplantation, graft-versus-host diseases and diabetes.

The compound represented by the above formula (I) has the CCR5 antagonistic activity and the inhibitory activity against physiological actions of in vivo ligands of CCR5 on target cells, i.e. the compound represented by the above formula (I) are a CCR5 antagonist.

Best Mode for Carrying Out the Invention

In the above formula (I), $R^1$ is a phenyl group, a $C_3$-$C_8$ cycloalkyl group or an aromatic heterocyclic group having one to three oxygen atoms, sulfur atoms and/or nitrogen atoms as heteroatoms; the phenyl group or the aromatic heterocyclic group in the above $R^1$ may be condensed with a benzene ring or an aromatic heterocyclic group having one to three oxygen atoms, sulfur atoms and/or nitrogen atoms as heteroatoms to form a condensed ring; the phenyl group, the $C_3$-$C_8$ cycloalkyl group, the aromatic heterocyclic group or the condensed ring in the above $R^1$ may further be substituted with an optional number of halogen atoms, hydroxy groups, cyano groups, nitro groups, carboxy groups, carbamoyl groups, $C_1$-$C_6$ alkyl groups, $C_3$-$C_8$ cycloalkyl groups, $C_2$-$C_6$aLkenyl groups, $C_1$-$C_6$ alkoxy groups, $C_1$-$C_6$ alkylthio groups, $C_3$-$C_5$ alkylene groups, $C_2$-$C_4$ alkylenoxy groups, $C_1$-$C_3$ alkylenedioxy groups, phenyl groups, phenoxy groups, phenylthio groups, benzyl groups, benzyloxy groups, benzoylamino groups, $C_2$-$C_7$ alkanoyl groups, $C_2$-$C_7$ alkoxycarbonyl groups, $C_2$-$C_7$ alkanoyloxy groups, $C_2$-$C_7$ alkanoylamino groups, $C_2$-$C_7$ N-alkylcarbamoyl groups, $C_4$-$C_9$

N-cycloalkylcarbamoyl groups, $C_1$-$C_6$ alkylsulfonyl groups, $C_3$-$C_8$ (alkoxycarbonyl)methyl groups, N-phenylcarbamoyl groups, piperidinocarbonyl groups, morpholinocarbonyl groups, 1-pyrrolidinylcarbonyl groups, bivalent groups represented by the formula: -NH(C=O)O-, bivalent groups represented by the formula: -NH(C=S)O-, amino groups, mono ($C_1$-$C_6$ alkyl)amino groups or di($C_1$-$C_6$ alkyl)amino groups.

The "$C_3$-$C_8$ cycloalkyl group" in $R^1$ means a cyclic alkyl group, and includes for example cyclopropyl group, cyclobutyl group, cyclopentyl group, cyclohexyl group, cycloheptyl group, cyclooctyl group and the like. The "$C_3$-$C_8$ cycloalkyl group" is preferably cyclopropyl group, cyclopentyl group, cyclohexyl group and the like.

The "aromatic heterocyclic group having one to three oxygen atoms, sulfur atoms and/or nitrogen atoms as heteroatoms" in $R^1$ means an aromatic heterocyclic group, and includes for example thienyl group, furyl group, pyrrolyl group, imidazolyl group, pyrazolyl group, oxazolyl group, isoxazolyl group, thiazolyl group, isothiazolyl group, pyridyl group, pyrimidinyl group, triazinyl group, triazolyl group, oxadiazolyl (furazanyl) group, thiadiazolyl group and the like. The "aromatic heterocyclic group having one to three oxygen atoms, sulfur atoms and/or nitrogen atoms as heteroatoms" is preferably thienyl group, furyl group, pyrrolyl group, isoxazolyl group, pyridyl group and the like.

The "condensed ring" in $R^1$ means a bicyclic aromatic heterocyclic group formed by condensing the phenyl group or the aromatic heterocyclic group with a benzene ring or the aromatic heterocyclic group having one to three oxygen atoms, sulfur atoms and/or nitrogen atoms as heteroatoms in an optional position, and includes for example naphthyl group, indolyl group, benzofuranyl group, benzothienyl group, quinolyl group, benzimidazolyl group, benzoxazolyl group, benzotriazolyl group, benzoxadiazolyl (benzofurazanyl), group, benzothiadiazolyl group and the like.

Among them, it is especially preferable for $R^1$ to be a phenyl group, a thienyl group, a pyrrolyl group, a pyrazolyl group, an isoxazolyl group or an indolyl group.

The "halogen atoms" as the substituents of the phenyl group, the $C_3$-$C_8$ cycloalkyl group, the aromatic heterocyclic group or the condensed ring in $R^1$ mean a fluorine atom, a chlorine atom, a bromine atom, an iodine atom and the like, and fluorine atom, chlorine atom, bromine atom and iodine atom are specifically preferable.

The "$C_1$-$C_6$ alkyl groups" as the substituents of $R^1$ mean $C_1$-$C_6$ straight or branched alkyl groups, and include for example, methyl group, ethyl group, n-propyl group; n-butyl group, n-pentyl group, n-hexyl group, n-heptyl group, n-octyl group, isopropyl group, isobutyl group, sec-butyl group, tert-butyl group, isopentyl group, neopentyl group, tert-pentyl group, isohexyl group, 2-methylpentyl group, 1-ethylbutyl group and the like. The "$C_1$-$C_6$ alkyl groups" are, as specifically preferable concrete examples, methyl group, ethyl group, propyl group, isopropyl group, tert-butyl group and the like.

The "$C_3$-$C_8$ cycloalkyl groups" as the substituents of $R^1$ are the same as defined in the "$C_3$-$C_8$ cycloalkyl group" in the above $R^1$, and specifically preferably include for example the same groups.

The "$C_2$-$C_6$ alkenyl groups" as the substituents of $R^1$ mean $C_2$-$C_6$ straight or branched alkenyl groups, and include for example vinyl group, allyl group, 1-propenyl group, 2-butenyl group, 3-butenyl group, 2-methyl-1-propenyl group, 4-pentenyl group, 5-hexenyl group, 4-methyl-3-pentenyl group and the like. The "$C_2$-$C_6$ alkenyl groups" are specifically preferably vinyl group and 2-methyl-1-propenyl group or the like.

The "$C_1$-$C_6$ alkoxy groups" as the substituents of $R^1$ mean groups composed of the above $C_1$-$C_6$ alkyl groups and oxy group, and methoxy group, ethoxy group or the like is specifically preferable.

The "$C_1$-$C_6$ alkylthio groups" as the substituents of $R^1$ mean groups composed of the above $C_1$-$C_6$ alkyl groups and thio group, and methylthio group, ethylthio group or the like is specifically preferable.

The "$C_3$-$C_5$ alkylene groups" as the substituents of $R^1$ mean $C_3$-$C_5$ bivalent alkylene groups, and include for example, trimethylene group, tetramethylene group, pentamethylene group, 1-methyltrimethylene group and the like. The "$C_3$-$C_5$ alkylene groups" are specifically preferably trimethylene group, tetramethylene group or the like.

The "$C_2$-$C_4$ alkylenoxy groups" as the substituents of $R^1$ mean groups composed of $C_2$-$C_4$ bivalent alkylene groups and oxy group and include, for example, ethylenoxy group ($-CH_2CH_2O-$), trimethylenoxy group ($-CH_2CH_2CH_2O-$), tetramethylenoxy group ($-CH_2CH_2CH_2CH_2O-$), 1,1-dimethylethylenoxy group ($-CH_2C(CH_3)_2O-$) and the like. The "$C_2$-$C_4$ alkylenoxy groups" are specifically preferably ethylenoxy group, trimethylenoxy group or the like.

The "$C_1$-$C_3$ alkylenedioxy groups" as the substituents of $R^1$ mean groups composed of $C_1$-$C_3$ bivalent alkylene groups and two oxy groups and include, for example, methylenedioxy group ($-OCH_2O-$), ethylenedioxy group ($-OCH_2CH_2O-$), trimethylenedioxy ($-OCH_2CH_2CH_2O-$) group and propylenedioxy ($-OCH_2CH(CH_3)O-$) group and the like. The "$C_1$-$C_3$ alkylenedioxy groups" are specifically preferably methylenedioxy group, ethylenedioxy group or the like.

The "$C_2$-$C_7$ alkanoyl groups" as the substituents of $R^1$ mean $C_2$-$C_7$ straight or branched alkanoyl groups, and include for example, acetyl group, propanoyl group, butanoyl group, pentanoyl group, hexanoyl group, heptanoyl group, isobutyryl group, 3-methylbutanoyl group, 2-methylbutanoyl group, pivaloyl group, 4-methylpentanoyl group, 3,3-dimethylbutanoyl group, 5-methylhexanoyl group and the like, and acetyl group or the like is specifically preferable.

The "$C_2$-$C_7$ alkoxycarbonyl groups" as the substituents of $R^1$ mean groups composed of the above $C_1$-$C_6$ alkoxy groups and carbonyl group, and methoxycarbonyl group, ethoxycarbonyl group or the like is specifically preferable.

The "$C_2$-$C_7$ alkanoyloxy groups" as the substituents of $R^1$ mean groups composed of the above $C_2$-$C_7$ alkanoyl groups and oxy group, and acetyloxy group or the like is specifically preferable.

The "$C_2$-$C_7$ alkanoylamino groups" as the substituents of $R^1$ mean groups composed of the above $C_2$-$C_7$ alkanoyl groups and amino group, and acetylamino group or the like is specifically preferable.

The "$C_2$-$C_7$ alkylcarbamoyl groups" as the substituents of $R^1$ mean groups composed of the above $C_1$-$C_6$ alkyl groups and carbamoyl group, and N-methylcarbamoyl group, N-ethylcarbamoyl group or the like is specifically preferable.

The "$C_4$-$C_9$ N-cycloalkylcarbamoyl groups" as the substituents of $R^1$ mean the above $C_3$-$C_8$ cycloalkyl groups and carbamoyl group, and N-cyclopentylcarbamoyl group, N-cyclohexylcarbamoyl group or the like is preferable.

The "$C_1$-$C_6$ alkylsulfonyl groups" as the substituents of $R^1$ mean groups composed of the above $C_1$-$C_6$ alkyl groups and sulfonyl group, and methylsulfonyl group or the like is specifically preferable.

The "$C_3$-$C_8$ (alkoxycarbonyl)methyl groups" as the substituents of $R^1$ mean groups composed of the above $C_2$-$C_7$ alkoxycarbonyl groups and methyl group, and (methoxycarbonyl)methyl group, (ethoxycarbonyl)methyl group or the like is specifically preferable.

The "mono($C_1$-$C_6$ alkyl)amino groups" as the substituents of $R^1$ mean amino groups substituted with the above $C_1$-$C_6$ alkyl groups, and methylamino group, ethylamino group or the like is specifically preferable.

The "di($C_1$-$C_6$ alkyl)amino groups" as the substituents of $R^1$ mean amino groups substituted with the same or different two $C_1$-$C_6$ alkyl groups described above, and dimethylamino group, diethylamino group, N-ethyl-N-methylamino group or the like is specifically preferable.

Among those described above, examples of the substituents of the phenyl group, the $C_3$-$C_8$ cycloalkyl group, the aromatic heterocyclic group or the condensed ring in $R^1$ are specifically preferably halogen atoms, hydroxy groups, cyano groups, $C_1$-$C_6$ alkyl groups, $C_2$-$C_6$ alkenyl groups, $C_1$-$C_6$ alkoxy groups, $C_1$-$C_6$ alkylthio groups, $C_3$-$C_5$ alkylene groups, $C_2$-$C_4$ alkylenoxy groups, alkylenedioxy groups, acetyl groups, phenyl groups, amino groups and di($C_1$-$C_6$ alkyl) amino groups, and halogen atoms, hydroxy groups, cyano groups, $C_1$-$C_6$ alkyl groups, $C_1$-$C_6$ alkoxy groups, Cs-$C_5$ alkylene groups, methylenedioxy groups and amino groups are especially preferable.

Moreover, the substituents of the phenyl group, the $C_3$-$C_8$ cycloalkyl group, the aromatic heterocyclic group or the condensed ring in $R^1$, may further be substituted with an optional number of halogen atoms, hydroxy groups, amino groups, trifluoromethyl groups, $C_1$-$C_6$ alkyl groups or $C_1$-$C_6$ alkoxy groups. The halogen atoms, $C_1$-$C_6$ alkyl groups and $C_1$-$C_6$ alkoxy groups are the same as defined for the substituents of the phenyl group, the $C_3$-$C_8$ cycloalkyl group, the aromatic heterocyclic group or the condensed ring in $R^1$, and the same groups are specifically preferable.

In the above formula (I), $R^2$ is a hydrogen atom, a $C_1$-$C_6$ alkyl group, a $C_2$-$C_7$ alkoxycarbonyl group, a hydroxy group or a phenyl group; and the $C_1$-$C_6$ alkyl group or phenyl group in $R^2$ may be substituted with an optional number of halogen atoms, hydroxy groups, $C_1$-$C_6$ alkyl groups or $C_1$-$C_6$ alkoxy groups, with the proviso that $R^2$ is not a hydroxy group when j is 0.

The $C_1$-$C_6$ alkyl group and $C_2$-$C_7$ alkoxycarbonyl group in $R^2$ are each the same as defined for the substituents of the phenyl group, the $C_{3-}$Cs cycloalkyl group, the aromatic heterocyclic group or the condensed ring in $R^1$, and the same examples are specifically preferable.

The halogen atoms, $C_1$-$C_6$ alkyl groups and $C_1$-$C_6$ alkoxy groups as the substituents of the $C_1$-$C_6$ alkyl group or the phenyl, group in $R^2$ are the same as defined for the substituents of the phenyl group, the $C_3$-$C_8$ cycloalkyl group, the aromatic heterocyclic group or the condensed ring in the above $R^1$, and the same examples are specifically preferable.

Among them, it is especially preferable for $R^2$ to be a hydrogen atom.

In the above formula (I), j is an integer of 0 to 2, and it is especially preferable for j to be 0.

In the above formula (I), k is an integer of 0 to 2; m is an integer of 2 to 4. Among them, it is especially preferable for the compounds to be 2-substituted pyrrolidines wherein k is 0 and m is 3; 3-substituted pyrrolidines wherein k is 1 and m is 2; 3-substituted piperidines wherein k is 1 and m is 3; 4-substituted piperidines wherein k is 2 and m is 2; or 3-substituted hexahydroazepines wherein k is 1 and m is 4, and 3-substituted pyrrolidines wherein k is 1 and m is 2 and 4-substituted piperidines wherein k is 2 and m is 2 are especially preferable.

In the above formula (I), n is 0 or 1.

In particular, 3-amidopyrrolidines wherein k is 1; m is 2 and n is 0 and 4-(amidomethyl)piperidines wherein k is 2; m is 2 and n is 1 are especially preferable.

In the above formula (I), $R^3$ is a hydrogen atom or a $C_1$-$C_6$ alkyl group which may be substituted with (one or two phenyl groups which may respectively be substituted with an optional number of the same or different halogen atoms, hydroxy groups, $C_1$-$C_6$ alkyl groups or $C_1$-$C_6$ alkoxy groups).

The $C_1$-$C_6$ alkyl group in $R^3$ is the same as defined for the substituent of the phenyl group, the $C_3$-$C_8$ cycloalkyl group, the aromatic heterocyclic group or the condensed ring in the above $R^1$, and methyl group, ethyl group and propyl group are specifically preferable.

The halogen atoms, $C_1$-$C_6$ alkyl groups and $C_1$-$C_6$ alkoxy groups as the substituents of the phenyl groups as the substituents of the $C_1$-$C_6$ alkyl group in $R^3$ are each the same as defined for substituents of the phenyl group, the $C_3$-$C_8$ cycloalkyl group, the aromatic heterocyclic group or the condensed ring in the above $R^1$, and the same examples are specifically preferable.

Among them, it is especially preferable for $R^3$ to be a hydrogen atom and an unsubstituted $C_1$-$C_6$ alkyl group.

In the above formula (I), $R^4$ and $R^5$ are each the same or different and are each a hydrogen atom, a hydroxy group, a phenyl group or a $C_1$-$C_6$ alkyl group; and the $C_1$-$C_6$ alkyl group in $R^4$ and $R^5$ may be substituted with an optional number of halogen atoms, hydroxy groups, cyano groups, nitro groups, carboxy groups, carbamoyl groups, mercapto groups, guanidino groups, $C_3$-$C_8$ cycloalkyl groups, $C_1$-$C_6$ alkoxy groups, $C_1$-$C_6$ alkylthio groups, (phenyl groups which may be substituted with an optional number of halogen atoms, hydroxy groups, $C_1$-$C_6$ alkyl groups, $C_{1-}C_6$ alkoxy groups or benzyloxy groups), phenoxy groups, benzyloxy groups, benzyloxycarbonyl groups, $C_2$-$C_7$ alkanoyl groups, $C_2$-$C_7$ alkoxycarbonyl groups, $C_2$-$C_7$ alkanoyloxy groups, $C_2$-$C_7$ alkanoylamino groups, $C_2$-$C_7$ N-alkylcarbamoyl groups, $C_1$-$C_6$ alkylsulfonyl groups, amino groups, mono($C_1$-$C_6$ alkyl)amino groups, di($C_1$-$C_6$ alkyl)amino groups or (aromatic heterocyclic groups having one to three oxygen atoms, sulfur atoms and/or nitrogen atoms as heteroatoms or condensed rings formed by condensation thereof with benzene rings) or both $R^4$ and $R^5$ together may form a three- to a six-membered cyclic hydrocarbon.

The $C_1$-$C_6$ alkyl group in $R^4$ and $R^5$ is the same as defined for the substituents of the phenyl group, the $C_3$-$C_8$ cycloalkyl group, the aromatic heterocyclic group or the condensed ring in the above $R^1$, and the same examples are specifically preferable.

The halogen atoms, $C_1$-$C_6$ alkoxy groups, $C_1$-$C_6$ alkylthio groups, $C_2$-$C_7$ alkanoyl groups, $C_2$-$C_7$ alkoxycarbonyl groups, $C_2$-$C_7$ alkanoyloxy groups, $C_2$-$C_7$ alkanoylamino groups, $C_2$-$C_7$ N-alkylcarbamoyl groups, $C_1$-$C_6$ alkylsulfonyl groups, mono($C_1$-$C_6$ alkyl)amino groups and di($C_1$-$C_6$ alkyl)amino groups as the substituents of the $C_1$-$C_6$ alkyl group in $R^4$ and $R^5$ are the same as defined for the substituents of the phenyl group, the $C_{3-}Cs$ cycloalkyl group, the aromatic heterocyclic group or the condensed ring in the above $R^1$, and the same examples are specifically preferable.

The $C_3$-$C_8$ cycloalkyl groups and the aromatic heterocyclic groups having one to three oxygen atoms, sulfur atoms and/or nitrogen atoms as heteroatoms as the substituents of the $C_1$-$C_6$ alkyl group in $R^4$ and $R^5$ are the same as defined for the above $R^1$, and the same examples are preferable.

The halogen atoms, $C_1$-$C_6$ alkyl groups and $C_1$-$C_6$ alkoxy groups as the substituents of the phenyl groups as the substituents of the $C_1$-$C_6$ alkyl group in $R^4$ and $R^5$ are the same as defined for the substituents of the phenyl group, the $C_3$-$C_8$ cycloalkyl group, the aromatic heterocyclic group or the condensed ring in the above $R^1$, and the same examples are specifically preferable.

The "three- to a six-membered cyclic hydrocarbon" composed of $R^4$, $R^5$ and the adjacent carbon atoms are specifically preferably cyclopropane, cyclobutane, cyclopentane, cyclohexane and the like.

Among them, the hydrogen atom and $C_1$-$C_6$ alkyl group are especially preferable for $R^4$ and $R^5$.

In the above formula (I), p is 0 or 1; and q is 0 or 1. Both p and q are especially preferably 0.

In the above formula (I), G is a group represented by -CO-, $-SO_2-$, -CO-O-, $-NR^7$-CO-, -CO-$NR^7$-, -NH-CO-NH-, -NH-CS-NH-, $-NR^7-SO_2-$, $-SO_2-NR^7-$, -NH-CO-O- or -O-CO-NH-,
wherein, $R^7$ is a hydrogen atom or a $C_1$-$C_6$ alkyl group or $R^7$, together with $R^5$, may form a $C_2$-$C_5$ alkylene group,
wherein, -CO- is a carbonyl group, $-SO_2-$ is a sulfonyl group and -CS- is a thiocarbonyl group. G is especially preferably the group represented by $-NR^7$-CO- or -NH-CO-NH-.

The $C_1$-$C_6$ alkyl group in $R^7$ is the same as defined for the substituents of the phenyl group, the $C_3$-$C_8$ cycloalkyl group, the aromatic heterocyclic group or the condensed ring in the above $R^1$, and the same examples are specifically preferable.

The "$C_2$-$C_5$ alkylene group" composed of $R^5$ and $R^7$ means a $C_2$-$C_5$ straight or branched alkylene group, for example, methylene group, ethylene group, propylene group, trimethylene group, tetramethylene group, 1-methyltrimethylene group, pentamethylene group and the like, and ethylene group, trimethylene group, tetramethylene group or the like is specifically preferable.

Among them, it is especially preferable for $R^7$ to be a hydrogen atom.

In the above formula (I), $R^6$ is a phenyl group, a $C_3$-$C_8$ cycloalkyl group, a $C_3$-$C_6$ cycloalkenyl group, a benzyl group or an aromatic heterocyclic group having one to three oxygen atoms, sulfur atoms and/or nitrogen atoms as heteroatoms; and the phenyl group, the benzyl group or the aromatic heterocyclic group in the above $R^6$ may be condensed with a benzene ring or the aromatic heterocyclic group having one to three oxygen atoms, sulfur atoms and/or nitrogen atoms as heteroatoms to form a condensed ring; and the phenyl group, the $C_3$-$C_8$ cycloalkyl group, the $C_3$-$C_6$ cycloalkenyl group, the benzyl group, the aromatic heterocyclic group or the condensed ring in the above $R^6$ may be substituted with an optional number of halogen atoms, hydroxy groups, mercapto groups, cyano groups, nitro groups, thiocyanato groups, carboxy groups, carbamoyl groups, trifluoromethyl groups, $C_1$-$C_6$ alkyl groups, $C_3$-$C_8$ cycloalkyl groups, $C_2$-$C_6$ alkenyl groups, $C_1$-$C_6$ alkoxyl groups, $C_3$-$C_8$ cycloalkyloxy groups, $C_1$-$C_6$ alkylthio groups, $C_1$-$C_3$ alkylenedioxy groups, phenyl groups, phenoxy groups, phenylamino groups, benzyl groups, benzoyl groups, phenylsulfinyl groups, phenylsulfonyl groups, 3-phenylureido groups, $C_2$-$C_7$ alkanoyl groups, $C_2$-$C_7$ alkoxycarbonyl groups, $C_2$-$C_7$ alkanoyloxy groups, $C_2$-$C_7$ alkanoylamino groups, $C_2$-$C_7$ N-alkylcarbamoyl groups, $C_1$-$C_6$ alkylsulfonyl groups, phenylcarbamoyl groups, N,N-di($C_1$-$C_6$ alkyl)sulfamoyl groups, amino groups, mono($C_1$-$C_6$ alkyl)amino groups, di($C_1$-$C_6$ alkyl) amino groups, benzylamino groups, $C_2$-$C_7$ (alkoxycarbonyl)amino groups, $C_1$-$C_6$ (alkylsulfonyl)amino groups or bis ($C_1$-$C_6$ alkylsulfonyl)amino groups.

The $C_3$-$C_8$ cycloalkyl groups, aromatic heterocyclic groups having oxygen atoms, sulfur atoms and/or nitrogen atoms as heteroatoms, or condensed rings in $R^6$ are the same as defined for the above $R^1$, and the same examples are specifically preferable.

The "$C_3$-$C_8$ cycloalkenyl groups" in $R^6$ mean cycloalkenyl groups, for example, cyclobutenyl group, cyclopentenyl group, cyclohexenyl group, cycloheptenyl group and cyclooctenyl group, and 1-cyclopentenyl group, 1-cyclohexenyl group or the like is specifically preferable.

Among them, it is especially preferable for $R^6$ to be a phenyl group, a furyl group, a thienyl group, a pyrazolyl group, a benzothienyl group and an indolyl group.

The halogen atoms, $C_1$-$C_6$ alkyl groups, $C_1$-$C_6$ alkenyl groups, $C_1$-$C_6$ alkoxy groups, $C_1$-$C_6$ alkylthio groups, $C_1$-$C_3$ alkylenedioxy groups, $C_2$-$C_7$ alkanoyl groups, $C_2$-$C_7$ alkoxycarbonyl groups, $C_2$-$C_7$ alkanoyloxy groups, $C_2$-$C_7$ alkanoylamino groups, $C_2$-$C_7$ N-alkylcarbamoyl groups, $C_1$-$C_6$ alkylsulfonyl groups, mono($C_1$-$C_6$ alkyl)amino groups and di($C_1$-$C_6$ alkyl)amino groups as the substituents of the phenyl group, the $C_3$-$C_8$ cycloalkyl group, the $C_3$-$C_8$ cycloalkenyl group, the benzyl group, the aromatic heterocyclic group or the condensed ring in $R^6$ are the same as defined for the substituents of the phenyl group, the $C_3$-$C_8$ cycloalkyl group, the aromatic heterocyclic group or the condensed ring in the above $R^1$, and the same examples are specifically preferable.

The $C_3$-$C_8$ cycloalkyl groups as the substituents of $R^6$ are the same as defined for the $C_3$-$C_8$ cycloalkyl groups in the above $R^1$, and the same examples are specifically preferable.

The "$C_3$-$C_8$ cycloalkyloxy groups" as the substituents of $R^6$ mean groups composed of the above $C_3$-$C_8$ cycloalkyl groups and oxy groups, and cyclopropyloxy group, cyclopentyloxy group, cyclohexyloxy group or the like is specifically preferable.

The "N,N-di($C_1$-$C_6$ alkyl)sulfamoyl groups" as the substituents of $R^6$ mean sulfamoyl groups substituted with the same or different two $C_1$-$C_6$ alkyl groups described above, and N,N-dimethylsulfamoyl group, N,N-diethylsulfamoyl group, N-ethyl-N-methylsulfamoyl group or the like is specifically preferable.

The "$C_2$-$C_7$ (alkoxycarbonyl)amino groups" as the substituents of $R^6$ mean groups composed of the above $C_2$-$C_7$ alkoxycarbonyl groups and amino groups, and (methoxycarbonyl)amino group, (ethoxycarbonyl)amino group or the like is specifically preferable.

The "$C_1$-$C_6$ (alkylsulfonyl)amino groups" as the substituents of $R^6$ mean groups composed of the above $C_1$-$C_6$ alkylsulfonyl groups and amino groups, and (methylsulfonyl)amino group or the like is specifically preferable.

The "bis($C_1$-$C_6$ alkylsulfonyl)amino groups" as the substituents of $R^6$ mean amino groups substituted with the same or different two $C_1$-$C_6$ alkylsulfonyl groups described above, and bis(methylsulfonyl)amino group or the like is specifically preferable.

Among them, halogen atoms, nitro groups, trifluoromethyl groups, $C_1$-$C_6$ alkyl groups, $C_1$-$C_6$ alkoxy groups, phenyl groups, phenylsulfonyl groups, amino groups, benzylamino groups and the like are preferable for the substituents of the phenyl group, the $C_3$-$C_8$ cycloalkyl group, the $C_3$-$C_8$ cycloalkenyl group, the benzyl group, the aromatic heterocyclic group or the condensed group in $R^6$, and halogen atoms, nitro groups, trifluoromethyl groups, $C_1$-$C_6$ alkyl groups, $C_1$-$C_6$ alkoxy groups, phenylsulfonyl groups and amino group are especially preferable.

Furthermore, the substituents of the phenyl group, the $C_3$-$C_8$ cycloalkyl group, the $C_3$-$C_8$ cycloalkenyl group, the benzyl group, the aromatic heterocyclic group or the condensed ring in such $R^6$ may further be substituted with an optional number of halogen atoms, cyano groups, hydroxy groups, amino groups, trifluoromethyl groups, $C_1$-$C_6$ alkyl groups, $C_1$-$C_6$ alkoxy groups, $C_1$-$C_6$ alkylthio groups, mono($C_1$-$C_6$ alkyl)amino groups or di($C_1$-$C_6$ alkyl)amino groups.

The halogen atoms, $C_1$-$C_6$ alkyl groups, $C_1$-$C_6$ alkoxy groups, $C_1$-$C_6$ alkylthio groups, mono($C_1$-$C_6$ alkyl)amino groups and di($C_1$-$C_6$ alkyl)amino groups as the substituents of the substituents of the phenyl group, the $C_3$-$C_8$ cycloalkyl group, the $C_3$-$C_8$ cycloalkenyl group, the benzyl group, the aromatic heterocyclic group or the condensed ring in $R^6$ are the same as defined for the substituents of the phenyl group, the $C_3$-$C_8$ cycloalkyl group, the aromatic heterocyclic aromatic group or the condensed ring in the above $R^1$, and the same examples are specifically preferable.

A pharmaceutical composition, which is prepared with the remedially effective amount of the compound represented by the above formula (I), the pharmaceutically acceptable acid addition salt thereof or the pharmaceutically acceptable $C_1$-$C_6$ alkyl-addition salt thereof together with a pharmaceutically acceptable carrier and/or diluent, can be the medicine capable of inhibiting the binding of an in vivo ligand of CCR5 and/or HIV to the CCR5 on target cells, the medicine having inhibitory actions on physiological actions of the ligand of CCR5 on the target cells, or further the remedy or prophylactic for diseases considered to be in association with the CCR5.

Namely, the cyclic amine derivative represented by the above formula (I), the pharmaceutically acceptable acid addition salt thereof or the pharmaceutically acceptable $C_1$-$C_6$ alkyl addition salt thereof can be administered orally or parenterally such as intravenously, subcutaneously, intramuscularly, percutaneously or intrarectally.

For example, a tablet, a pill, a granule, a powder, a liquid, a suspension or a capsule can be cited as the dosage form of the oral administration.

The tablet can be prepared by using a vehicle, for example, lactose, starch or crystalline cellulose; a binder, for example, carboxymethylcellulose, methylcellulose or polyvinylpyrrolidone; or a disintegrator, for example, sodium

alginate, sodium bicarbonate or sodium lauryl sulfate or the like according to a conventional method.

The pill, powder and granule can similarly be prepared with using the above vehicle or the like according to a conventional method. The liquid and suspension are prepared with using glycerin esters, for example, tricaprylin or triacetin or alcohols, for example, ethanol according to a conventional method. The capsule is prepared with filling a granule, powder or liquid in a capsule made from gelatin on the like.

A parenteral injection such as the form of an aqueous or a nonaqueous solution formulation is cited as the dosage form of subcutaneous, intramuscular or intravenous administration. For example, an isotonic sodium chloride solution is used as the aqueous solution. Propylene glycol, poly(ethylene glycol), olive oil or ethyl oleate is, for example, used for the nonaqueous solution. An antiseptic, a stabilizer or the like, if necessary, is added thereto. The parenteral injection is sterilized by suitably carrying out treatment such as filtration through a bacterial filter or combination of a disinfectant.

For example, an ointment or a cream is cited as the dosage form of percutaneous administration. The ointment is prepared by using fats and fatty oils such as castor oil or olive oil or vaseline, and the cream is prepared by using a fatty oil or an emulsifying agent such as di(ethylene glycol) or sorbitan mono-fatty acid ester according to a conventional method.

A usual suppository such as a gelatin soft capsule is used for intrarectal administration.

The dose of the cyclic amine derivative, pharmaceutically acceptable acid addition salt thereof or pharmaceutically acceptable $C_1$-$C_6$ alkyl addition salt thereof, in the present invention, varies with the types of diseases, routes of administration, age and sex of patients and severity of diseases and the like, but is usually 1 to 500 mg/day for an adult.

Concrete examples of the cyclic amine derivative represented by the above formula (I) preferably includes compounds having respective substituents shown in the following Tables 1.1 to 1.221.

In Tables 1.1 to 1.221, and "Compd. No." means "compound number". "Chirality" means the "absolute configuration", and the "chirality (absolute configuration)" means the absolute configuration of asymmetric carbon on the ring of the cyclic amine. "R" means that the asymmetric carbon atom on the ring of the cyclic amine has the absolute configuration of R, and "S" means that the asymmetric carbon atom has the absolute configuration of S. "-" means that the compound is a racemate or the compound has no asymmetric carbon atom on the cyclic amines.

**Table 1.1**

| Compd. No. | 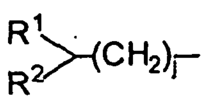 | k | m | n | chirality | $R^3$ | 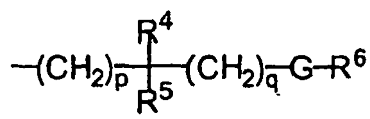 |
|---|---|---|---|---|---|---|---|
| 1 | 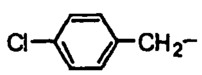 | 1 | 2 | 0 | - | H | 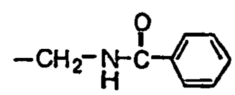 |
| 2 | 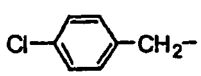 | 1 | 2 | 0 | - | H | 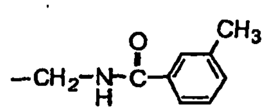 |
| 3 | 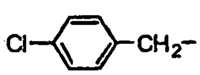 | 1 | 2 | 0 | - | H | 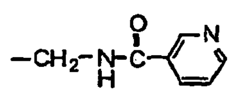 |
| 4 | 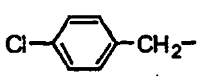 | 1 | 2 | 0 | - | H | 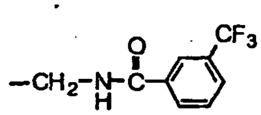 |
| 5 | 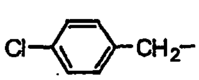 | 1 | 2 | 0 | S | H | 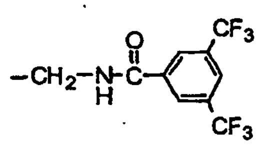 |

(continued)

| Compd. No. | 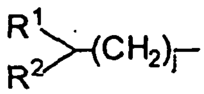 | k | m | n | chirality | $R^3$ | 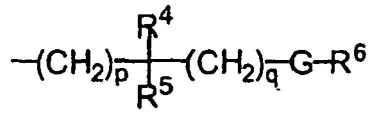 |
|---|---|---|---|---|---|---|---|
| 6 | 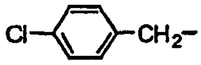 | 1 | 2 | 0 | S | H | 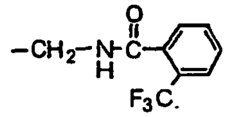 |
| 7 | 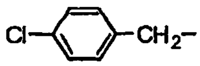 | 1 | 2 | 0 | S | H | 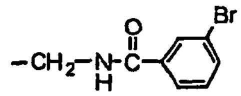 |
| 8 | 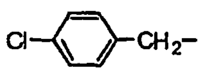 | 1 | 2 | 0 | S | H | 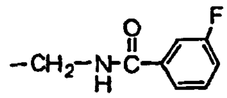 |
| 9 | 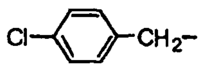 | 1 | 2 | 0 | S | H | 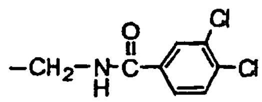 |
| 10 | 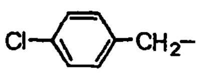 | 1 | 2 | 0 | S | H | 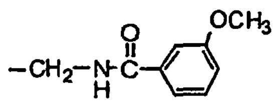 |
| 11 | 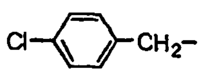 | 1 | 2 | 0 | S | H | 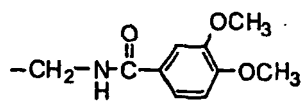 |

**Table 1.2**

| Compd. No. | 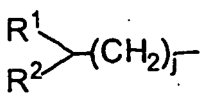 | k | m | n | chirality | $R^3$ | 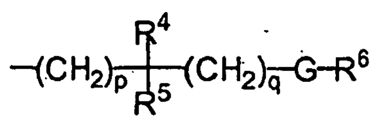 |
|---|---|---|---|---|---|---|---|
| 12 | 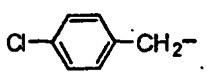 | 1 | 2 | 0 | S | H | 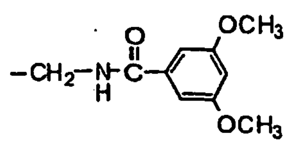 |
| 13 | 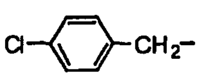 | 1 | 2 | 0 | S | H | 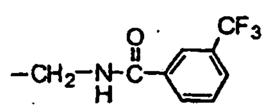 |
| 14 | 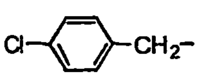 | 1 | 2 | 0 | S | H | 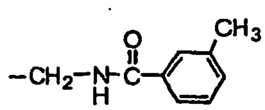 |

(continued)

| Compd. No. | 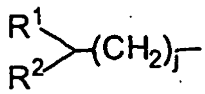 | k | m | n | chirality | $R^3$ | 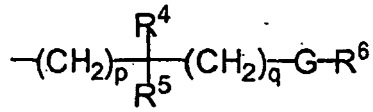 |
|---|---|---|---|---|---|---|---|
| 15 | 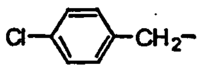 | 1 | 2 | 0 | S | H | 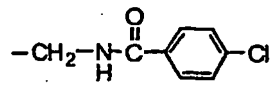 |
| 16 | 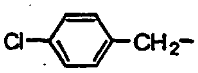 | 1 | 2 | 0 | S | H | 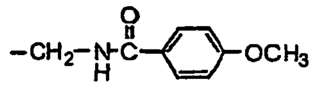 |
| 17 | 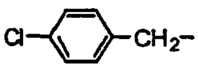 | 1 | 2 | 0 | S | H | 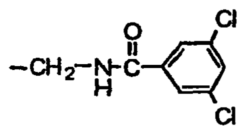 |
| 18 | 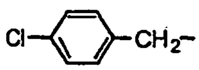 | 1 | 2 | 0 | S | H | 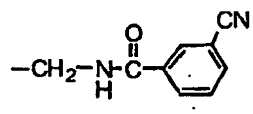 |
| 19 | 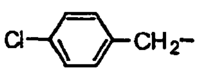 | 1 | 2 | 0 | S | H | 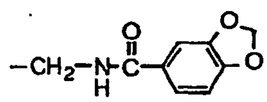 |
| 20 | 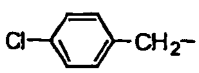 | 1 | 2 | 0 | S | H | 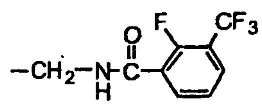 |
| 21 | 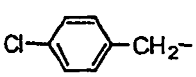 | 1 | 2 | 0 | S | H | 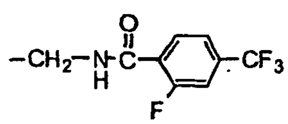 |
| 22 | 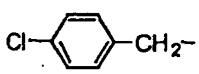 | 1 | 2 | 0 | S | H | 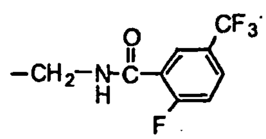 |

**Table 1.3**

| Compd. No. | 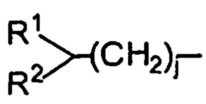 | k | m | n | chirality | $R^3$ | 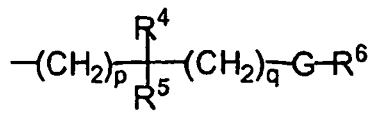 |
|---|---|---|---|---|---|---|---|
| 23 | 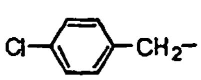 | 1 | 2 | 0 | S | H | 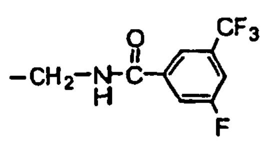 |

(continued)

| Compd. No. | 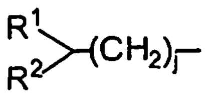 | k | m | n | chirality | $R^3$ | 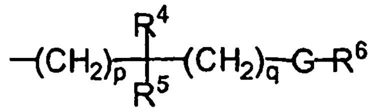 |
|---|---|---|---|---|---|---|---|
| 24 | 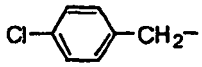 | 1 | 2 | 0 | S | H | 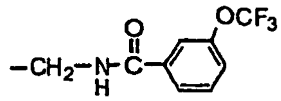 |
| 25 | 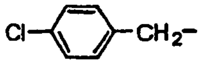 | 1 | 2 | 0 | S | H | 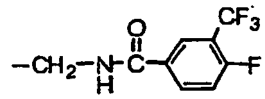 |
| 26 | 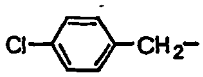 | 1 | 2 | 0 | S | H | 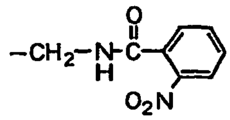 |
| 27 | 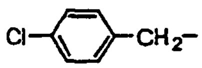 | 1 | 2 | 0 | S | H | 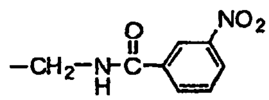 |
| 28 | 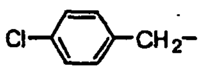 | 1 | 2 | 0 | S | H | 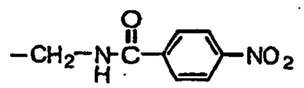 |
| 29 | 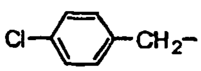 | 1 | 2 | 0 | R | H | 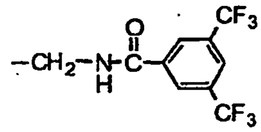 |
| 30 | 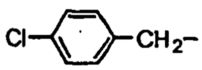 | 1 | 2 | 0 | R | H | 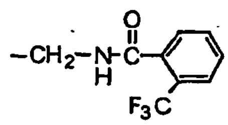 |
| 31 | 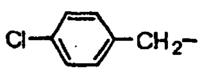 | 1 | 2 | 0 | R | H | 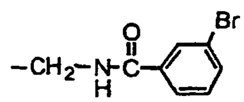 |
| 32 | 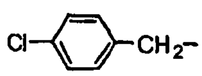 | 1 | 2 | 0 | R | H | 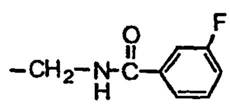 |
| 33 | 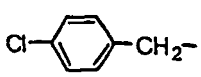 | 1 | 2 | 0 | R | H | 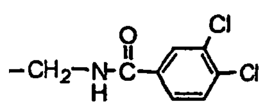 |

**Table 1.4**

| Compd. No. | 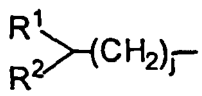 | k | m | n | chirality | $R^3$ | 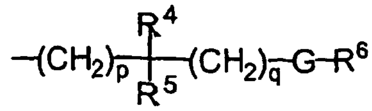 |
|---|---|---|---|---|---|---|---|
| 34 | 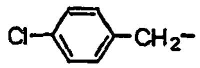 | 1 | 2 | 0 | R | H | 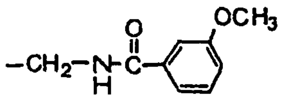 |
| 35 | 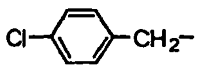 | 1 | 2 | 0 | R | H | 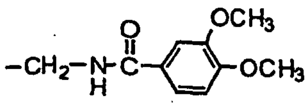 |
| 36 | 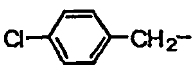 | 1 | 2 | 0 | R | H | 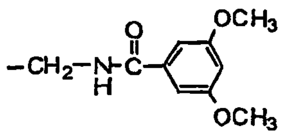 |
| 37 | 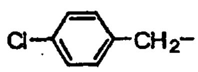 | 1 | 2 | 0 | R | H | 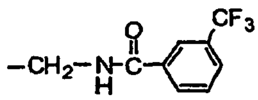 |
| 38 | 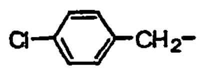 | 1 | 2 | 0 | R | H | 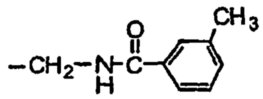 |
| 39 | 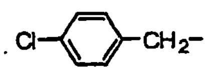 | 1 | 2 | 0 | R | H | 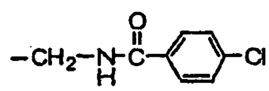 |
| 40 | 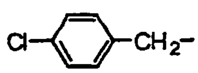 | 1 | 2 | 0 | R | H | 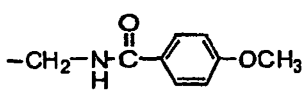 |
| 41 | 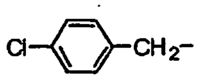 | 1 | 2 | 0 | R | H | 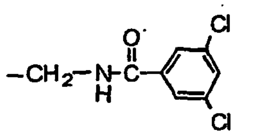 |
| 42 | 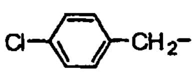 | 1 | 2 | 0 | R | H | 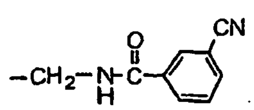 |
| 43 | 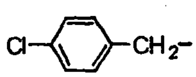 | 1 | 2 | 0 | R | H | 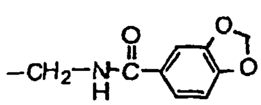 |
| 44 | 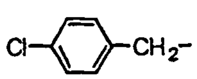 | 1 | 2 | 0 | R | H | 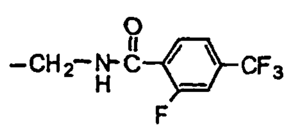 |

**Table 1.5**

| Compd. No. | 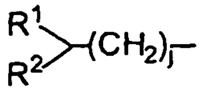 | k | m | n | chirality | $R^3$ | 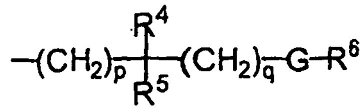 |
|---|---|---|---|---|---|---|---|
| 45 | 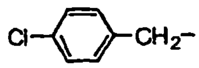 | 1 | 2 | 0 | R | H | 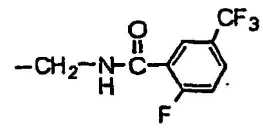 |
| 46 | 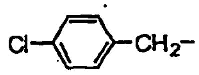 | 1 | 2 | 0 | R | H | 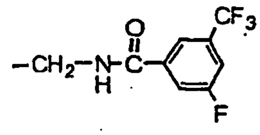 |
| 47 | 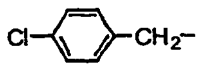 | 1 | 2 | 0 | R | H | 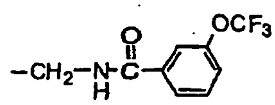 |
| 48 | 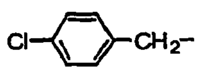 | 1 | 2 | 0 | R | H | 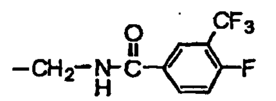 |
| 49 | 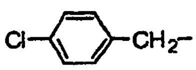 | 1 | 2 | 0 | R | H | 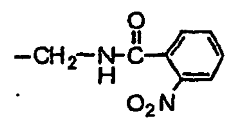 |
| 50 | 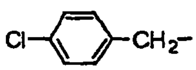 | 1 | 2 | 0 | R | H | 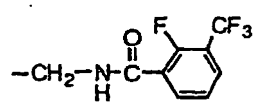 |
| 51 | 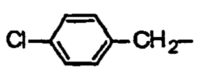 | 1 | 2 | 0 | R | H | 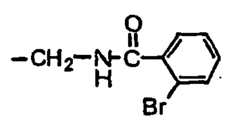 |
| 52 | 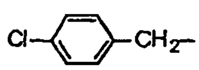 | 1 | 2 | 0 | R | H | 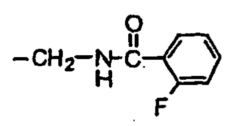 |
| 53 | 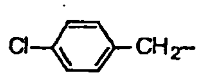 | 1 | 2 | 0 | R | H | 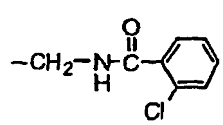 |
| 54 | 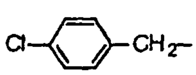 | 1 | 2 | 0 | R | H | 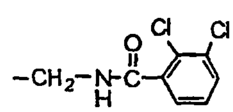 |

(continued)

| Compd. No. | 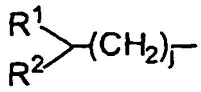 | k | m | n | chirality | $R^3$ | 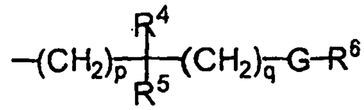 |
|---|---|---|---|---|---|---|---|
| 55 | 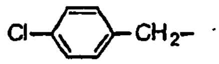 | 1 | 2 | 0 | R | H | 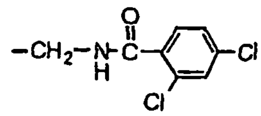 |

**Table 1.6**

| Compd. No. | 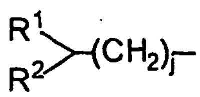 | k | m | n | chirality | $R^3$ | 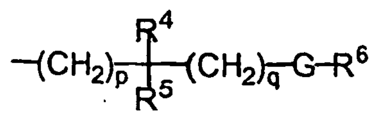 |
|---|---|---|---|---|---|---|---|
| 56 | 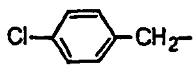 | 1 | 2 | 0 | R | H | 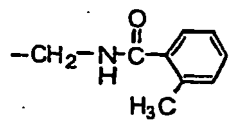 |
| 57 | 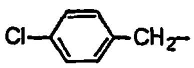 | 1 | 2 | 0 | R | H | 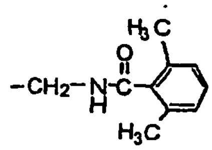 |
| 58 | 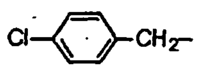 | 1 | 2 | 0 | R | H | 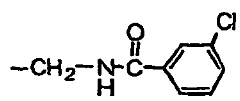 |
| 59 | 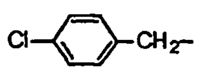 | 1 | 2 | 0 | R | H | 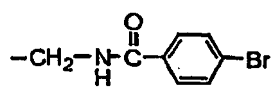 |
| 60 | 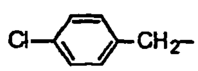 | 1 | 2 | 0 | R | H | 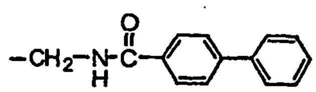 |
| 61 | 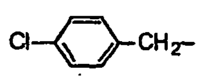 | 1 | 2 | 0 | R | H | 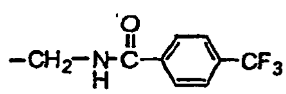 |
| 62 | 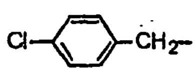 | 1 | 2 | 0 | R | H | 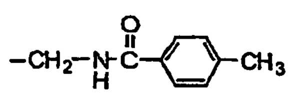 |
| 63 | 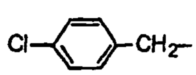 | 1 | 2 | 0 | R | H | 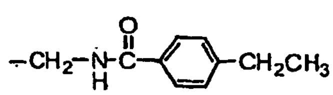 |

(continued)

| Compd. No. | 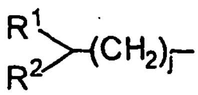 | k | m | n | chirality | $R^3$ | 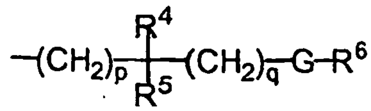 |
|---|---|---|---|---|---|---|---|
| 64 | 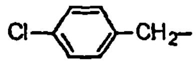 | 1 | 2 | 0 | R | H | 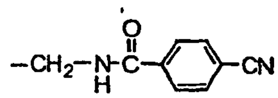 |
| 65 | 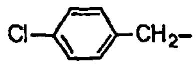 | 1 | 2 | 0 | R | H | 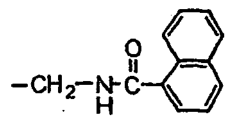 |
| 66 | 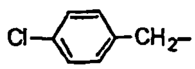 | 1 | 2 | 0 | R | H | 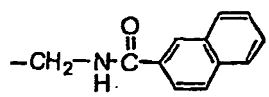 |

**Table 1.7**

| Compd. No. | 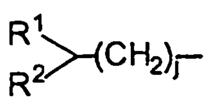 | k | m | n | chirality | $R^3$ | 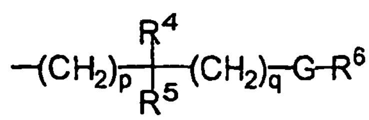 |
|---|---|---|---|---|---|---|---|
| 67 | 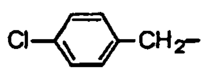 | 1 | 2 | 0 | R | H | 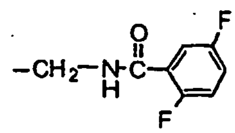 |
| 68 | 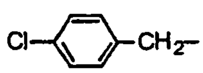 | 1 | 2 | 0 | R | H | 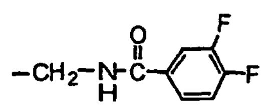 |
| 69 | 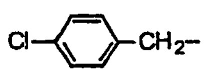 | 1 | 2 | 0 | R | H | 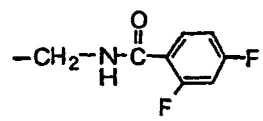 |
| 70 | 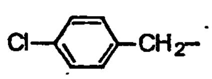 | 1 | 2 | 0 | R | H | 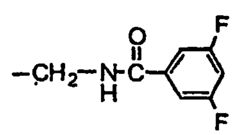 |
| 71 | 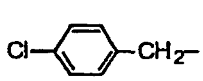 | 1 | 2 | 0 | R | H | 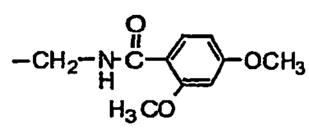 |
| 72 | 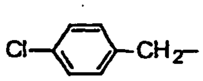 | 1 | 2 | 0 | R | H | 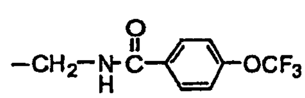 |

(continued)

| Compd. No. | 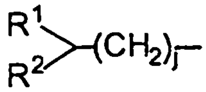 | k | m | n | chirality | $R^3$ | 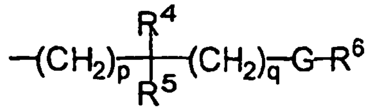 |
|---|---|---|---|---|---|---|---|
| 73 | 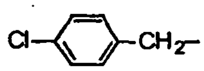 | 1 | 2 | 0 | R | H | 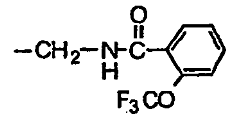 |
| 74 | 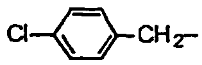 | 1 | 2 | 0 | R | H | 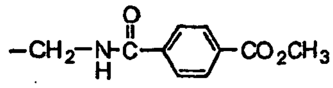 |
| 75 | 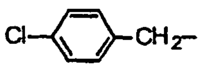 | 1 | 2 | 0 | R | H | 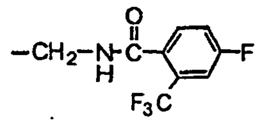 |
| 76 | 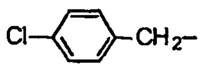 | 1 | 2 | 0 | R | H | 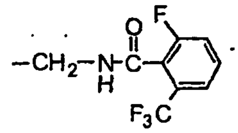 |
| 77 | 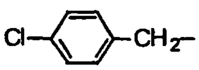 | 1 | 2 | 0 | R | H | 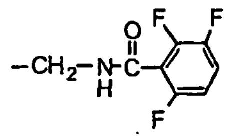 |

**Table 1.8**

| Compd. No. | 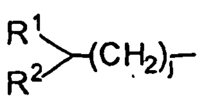 | k | m | n | chirality | $R^3$ | 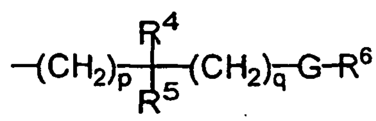 |
|---|---|---|---|---|---|---|---|
| 78 | 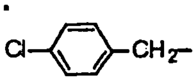 | 1 | 2 | 0 | R | H | 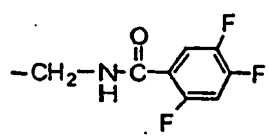 |
| 79 | 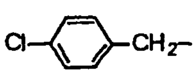 | 1 | 2 | 0 | R | H | 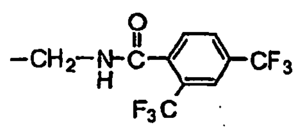 |
| 80 | 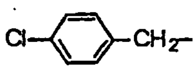 | 1 | 2 | 0 | R | H | 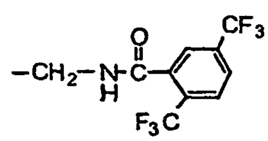 |

(continued)

| Compd. No. | 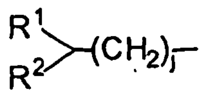 | k | m | n | chirality | $R^3$ | 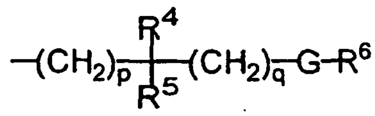 |
|---|---|---|---|---|---|---|---|
| 81 | 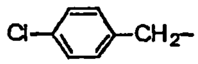 | 1 | 2 | 0 | R | H | 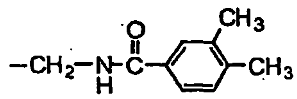 |
| 82 | 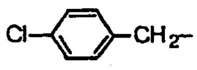 | 1 | 2 | 0 | - | $-CH_3$ | 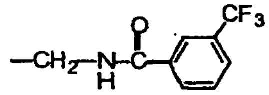 |
| 83 | 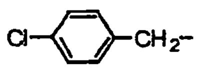 | 1 | 2 | 0 | R | H | 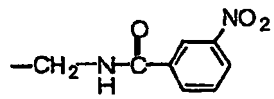 |
| 84 | 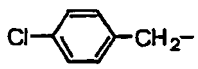 | 1 | 2 | 0 | R | H | 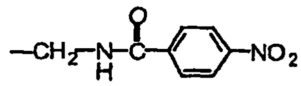 |
| 85 | 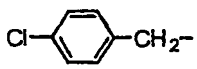 | 1 | 2 | 0 | - | H | 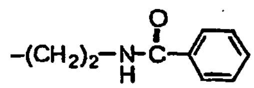 |
| 86 | 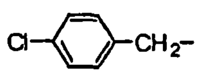 | 1 | 2 | 0 | - | H | 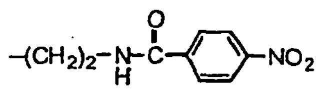 |
| 87 | 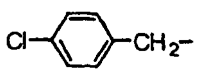 | 1 | 2 | 0 | S | H | 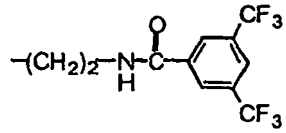 |
| 88 | 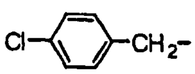 | 1 | 2 | 0 | S | H | 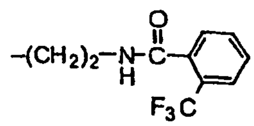 |

**Table 1.9**

| Compd. No. | 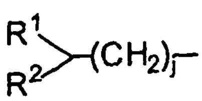 | k | m | n | chirality | $R^3$ | 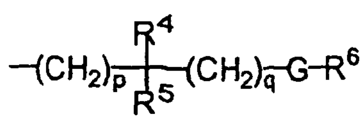 |
|---|---|---|---|---|---|---|---|
| 89 | 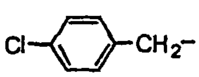 | 1 | 2 | 0 | S | H | 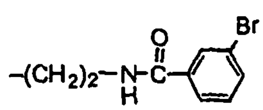 |

(continued)

| Compd. No. | 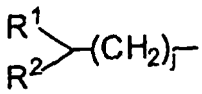 | k | m | n | chirality | $R^3$ | 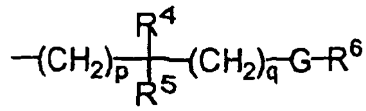 |
|---|---|---|---|---|---|---|---|
| 90 | 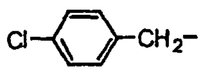 | 1 | 2 | 0 | S | H | 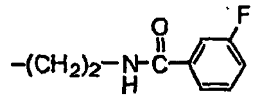 |
| 91 | 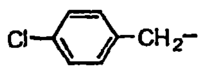 | 1 | 2 | 0 | S | H | 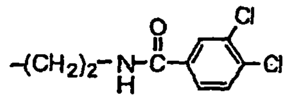 |
| 92 | 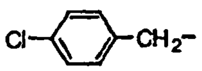 | 1 | 2 | 0 | S | H | 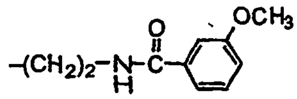 |
| 93 | 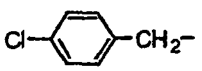 | 1 | 2 | 0 | S | H | 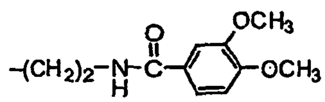 |
| 94 | 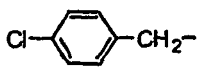 | 1 | 2 | 0 | S | H | 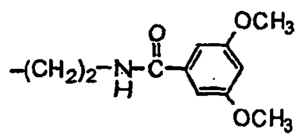 |
| 95 | 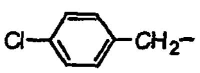 | 1 | 2 | 0 | S | H | 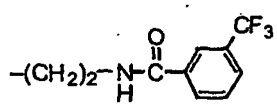 |
| 96 | 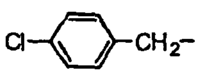 | 1 | 2 | 0 | S | H | 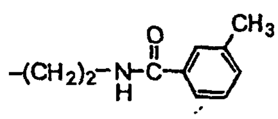 |
| 97 | 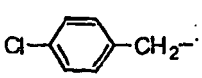 | 1 | 2 | 0 | S | H | 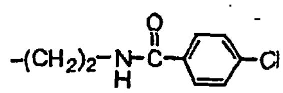 |
| 98 | 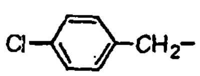 | 1 | 2 | 0 | S | H | 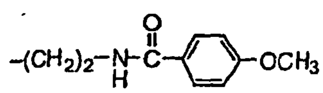 |
| 99 | 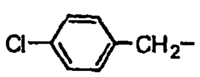 | 1 | 2 | 0 | S | H | 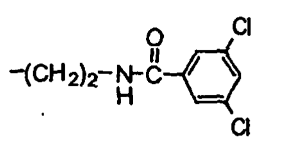 |

**Table 1.10**

| Compd. No. | 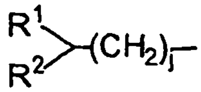 | k | m | n | chirality | $R^3$ | 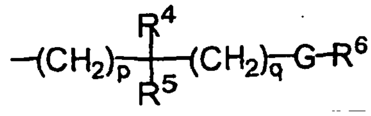 |
|---|---|---|---|---|---|---|---|
| 100 | 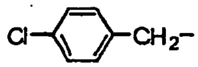 | 1 | 2 | 0 | S | H | 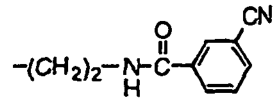 |
| 101 | 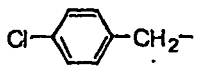 | 1 | 2 | 0 | S | H | 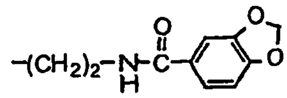 |
| 102 | 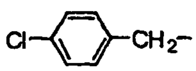 | 1 | 2 | 0 | S | H | 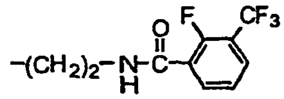 |
| 103 | 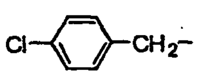 | 1 | 2 | 0 | S | H | 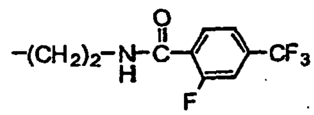 |
| 104 | 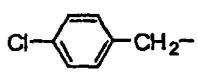 | 1 | 2 | 0 | S | H | 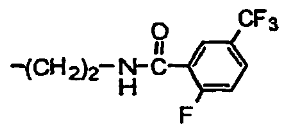 |
| 105 | 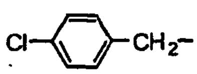 | 1 | 2 | 0 | S | H | 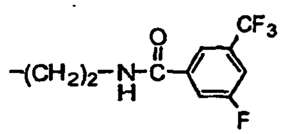 |
| 106 | 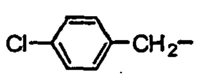 | 1 | 2 | 0 | S | H | 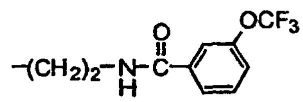 |
| 107 | 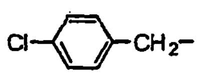 | 1 | 2 | 0 | S | H | 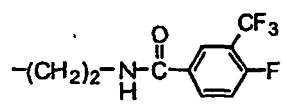 |
| 108 | 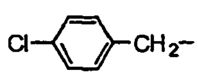 | 1 | 2 | 0 | S | H | 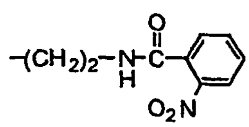 |
| 109 | 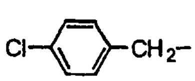 | 1 | 2 | 0 | S | H | 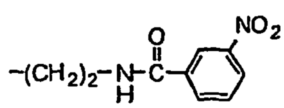 |
| 110 | 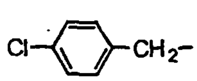 | 1 | 2 | 0 | S | H | 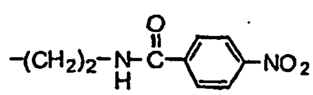 |

**Table 1.11**

| Compd. No. | 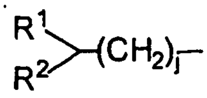 | k | m | n | chirality | $R^3$ | 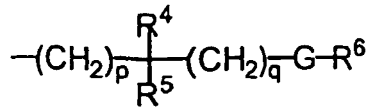 |
|---|---|---|---|---|---|---|---|
| 111 | 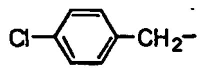 | 1 | 2 | 0 | R | H | 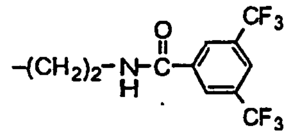 |
| 112 | 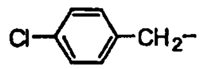 | 1 | 2 | 0 | R | H | 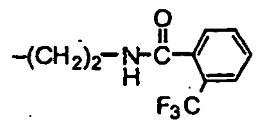 |
| 113 | 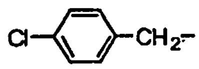 | 1 | 2 | 0 | R | H | 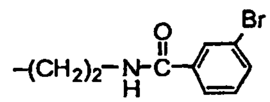 |
| 114 | 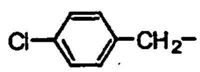 | 1 | 2 | 0 | R | H | 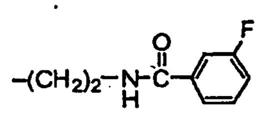 |
| 115 | 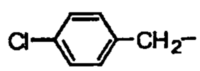 | 1 | 2 | 0 | R | H | 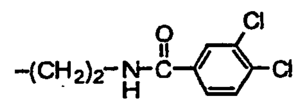 |
| 116 | 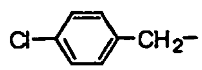 | 1 | 2 | 0 | R | H | 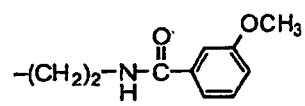 |
| 117 | 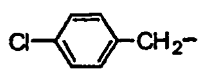 | 1 | 2 | 0 | R | H | 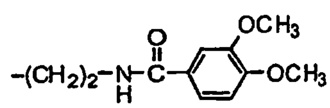 |
| 118 | 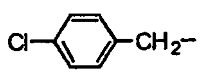 | 1 | 2 | 0 | R | H | 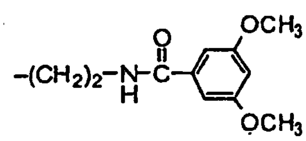 |
| 119 | 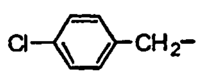 | 1 | 2 | 0 | R | H | 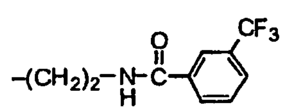 |
| 120 | 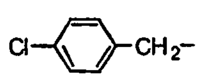 | 1 | 2 | 0 | R | H | 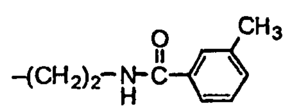 |

(continued)

| Compd. No. | 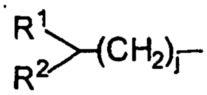 | k | m | n | chirality | $R^3$ | 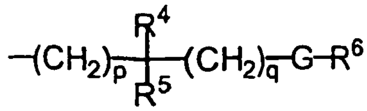 |
|---|---|---|---|---|---|---|---|
| 121 | 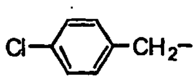 | 1 | 2 | 0 | R | H | 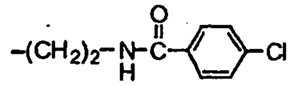 |

**Table 1.12**

| Compd. No. | 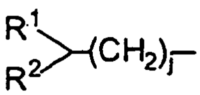 | k | m | n | chirality | $R^3$ | 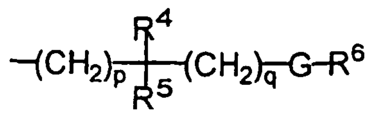 |
|---|---|---|---|---|---|---|---|
| 122 | 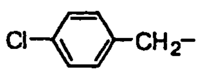 | 1 | 2 | 0 | R | H | 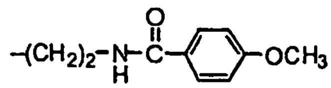 |
| 123 | 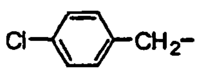 | 1 | 2 | 0 | R | H | 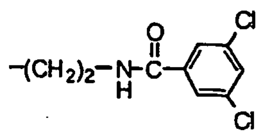 |
| 124 | 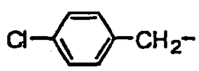 | 1 | 2 | 0 | R | H | 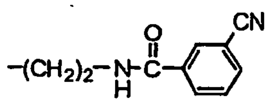 |
| 125 | 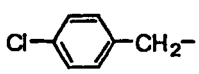 | 1 | 2 | 0 | R | H | 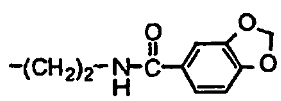 |
| 126 | 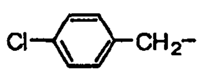 | 1 | 2 | 0 | R | H | 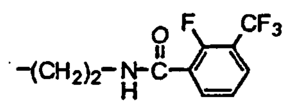 |
| 127 | 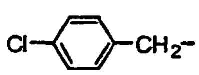 | 1 | 2 | 0 | R | H | 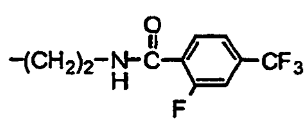 |
| 128 | 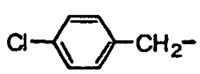 | 1 | 2 | 0 | R | H | 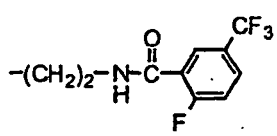 |
| 129 | 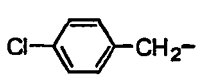 | 1 | 2 | 0 | R | H | 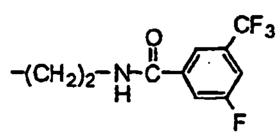 |
| 130 | 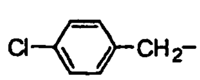 | 1 | 2 | 0 | R | H | 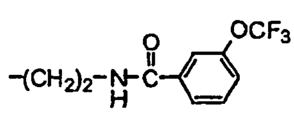 |

(continued)

| Compd. No. | 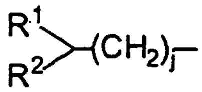 | k | m | n | chirality | $R^3$ | 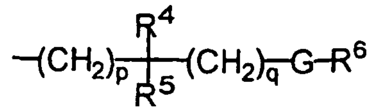 |
|---|---|---|---|---|---|---|---|
| 131 | 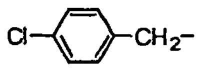 | 1 | 2 | 0 | R | H | 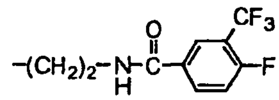 |
| 132 | 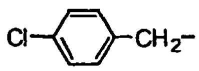 | 1 | 2 | 0 | R | H | 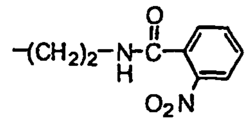 |

**Table 1.13**

| Compd. No. | 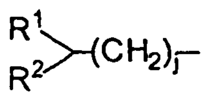 | k | m | n | chirality | $R^3$ | 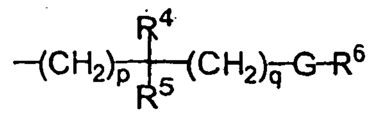 |
|---|---|---|---|---|---|---|---|
| 133 | 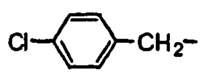 | 1 | 2 | 0 | R | H | 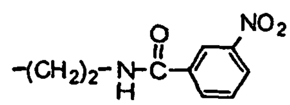 |
| 134 | 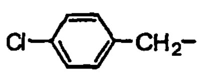 | 1 | 2 | 0 | R | H | 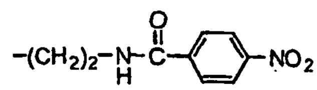 |
| 135 | 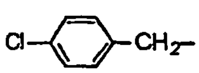 | 1 | 2 | 0 | R | H | 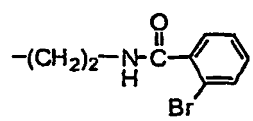 |
| 136 | 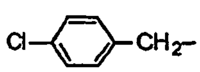 | 1 | 2 | 0 | R | H | 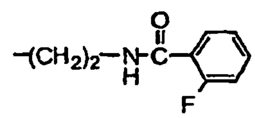 |
| 137 | 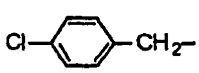 | 1 | 2 | 0 | R | H | 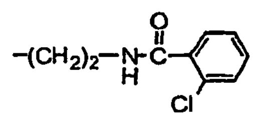 |
| 138 | 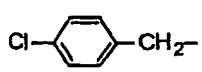 | 1 | 2 | 0 | R | H | 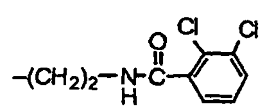 |
| 139 | 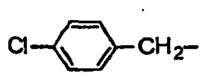 | 1 | 2 | 0 | R | H | 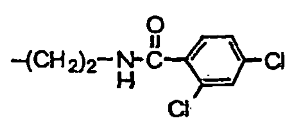 |

(continued)

| Compd. No. | 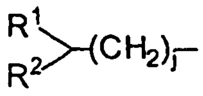 | k | m | n | chirality | $R^3$ | 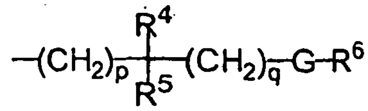 |
|---|---|---|---|---|---|---|---|
| 140 | 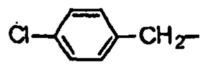 | 1 | 2 | 0 | R | H | 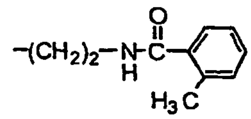 |
| 141 | 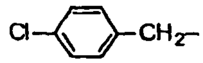 | 1 | 2 | 0 | R | H | 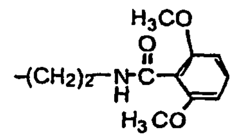 |
| 142 | 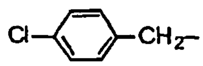 | 1 | 2 | 0 | R | H | 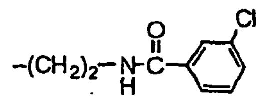 |
| 143 | 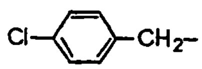 | 1 | 2 | 0 | R | H | 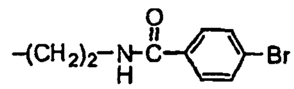 |

**Table 1.14**

| Compd. No. | 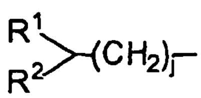 | k | m | n | chirality | $R^3$ | 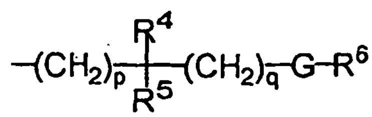 |
|---|---|---|---|---|---|---|---|
| 144 | 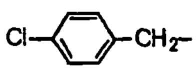 | 1 | 2 | 0 | R | H | 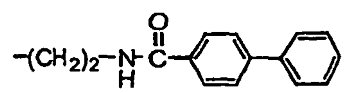 |
| 145 | 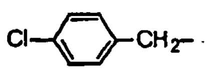 | 1 | 2 | 0 | R | H | 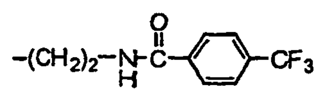 |
| 146 | 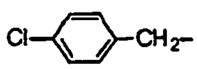 | 1 | 2 | 0 | R | H | 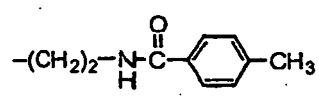 |
| 147 | 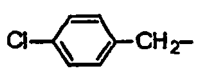 | 1 | 2 | 0 | R | H | 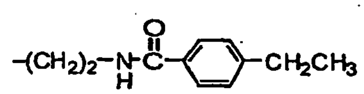 |
| 148 | 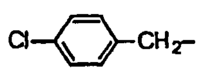 | 1 | 2 | 0 | R | H | 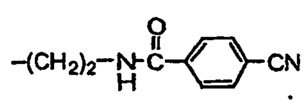 |

(continued)

| Compd. No. | 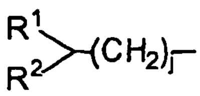 | k | m | n | chirality | $R^3$ | 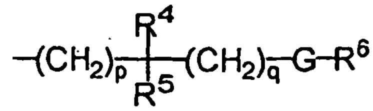 |
|---|---|---|---|---|---|---|---|
| 149 | 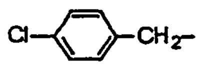 | 1 | 2 | 0 | R | H | 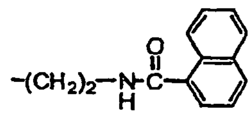 |
| 150 | 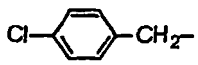 | 1 | 2 | 0 | R | H | 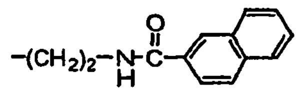 |
| 151 | 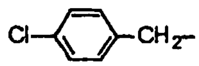 | 1 | 2 | 0 | R | H | 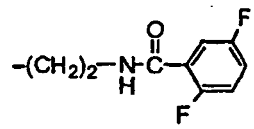 |
| 152 | 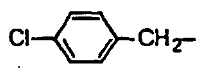 | 1 | 2 | 0 | R | H | 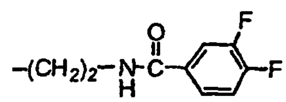 |
| 153 | 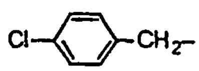 | 1 | 2 | 0 | R | H | 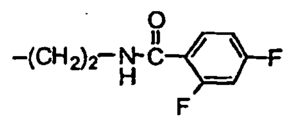 |
| 154 | 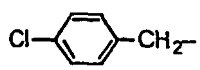 | 1 | 2 | 0 | R | H | 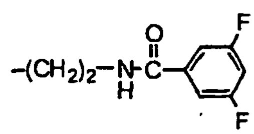 |

**Table 1.15**

| Compd. No. | 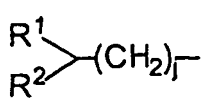 | k | m | n | chirality | $R^3$ | 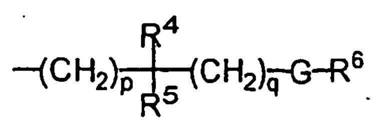 |
|---|---|---|---|---|---|---|---|
| 155 | 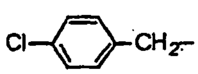 | 1 | 2 | 0 | R | H | 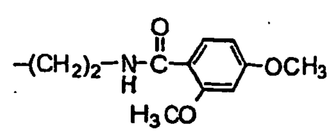 |
| 156 | 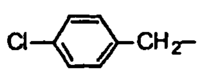 | 1 | 2 | 0 | R | H | 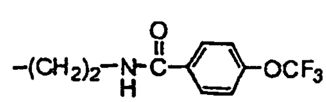 |
| 157 | 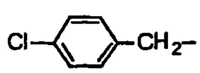 | 1 | 2 | 0 | R | H | 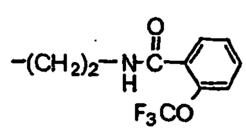 |

(continued)

| Compd. No. | 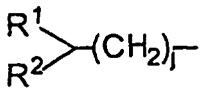 | k | m | n | chirality | $R^3$ | 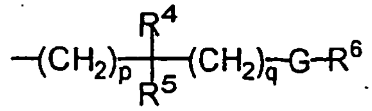 |
|---|---|---|---|---|---|---|---|
| 158 | 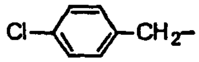 | 1 | 2 | 0 | R | H | 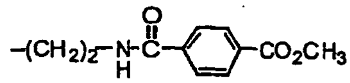 |
| 159 | 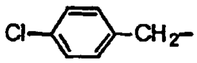 | 1 | 2 | 0 | R | H | 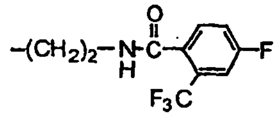 |
| 160 | 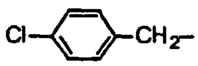 | 1 | 2 | 0 | R | H | 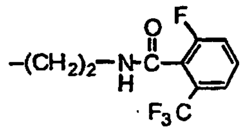 |
| 161 | 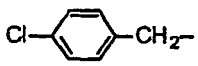 | 1 | 2 | 0 | R | H | 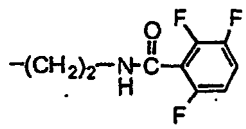 |
| 162 | 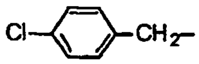 | 1 | 2 | 0 | R | H | 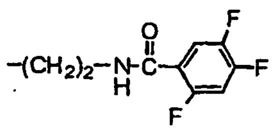 |
| 163 | 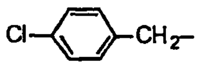 | 1 | 2 | 0 | R | H | 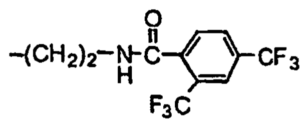 |
| 164 | 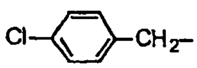 | 1 | 2 | 0 | R | H | 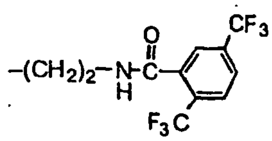 |
| 165 | 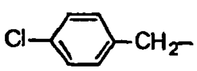 | 1 | 2 | 0 | R | H | 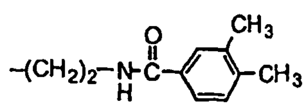 |

**Table 1.16**

| Compd. No. | 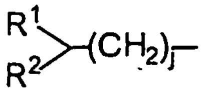 | k | m | n | chirality | $R^3$ | 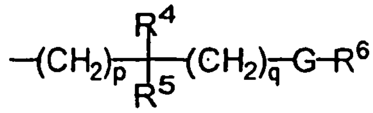 |
|---|---|---|---|---|---|---|---|
| 166 | 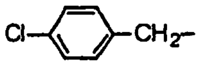 | 1 | 2 | 0 | R | H | 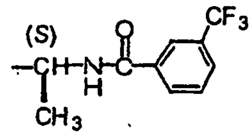 |
| 167 | 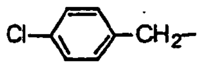 | 1 | 2 | 0 | R | H | 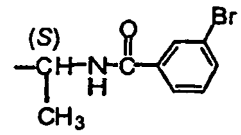 |
| 168 | 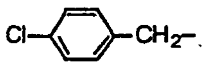 | 1 | 2 | 0 | R | H | 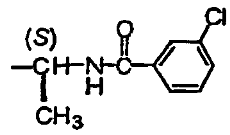 |
| 169 | 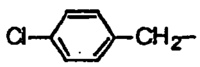 | 1 | 2 | 0 | R | H | 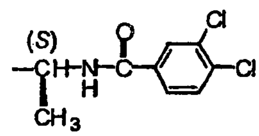 |
| 170 | 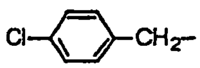 | 1 | 2 | 0 | R | H | 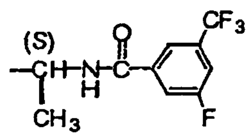 |
| 171 | 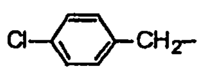 | 1 | 2 | 0 | R | H | 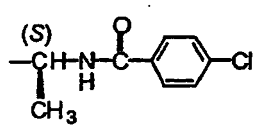 |
| 172 | 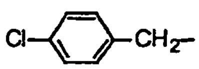 | 1 | 2 | 0 | R | H | 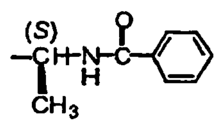 |
| 173 | 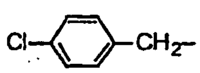 | 1 | 2 | 0 | R | H | 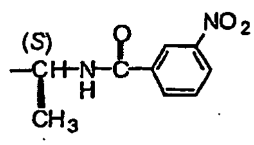 |
| 174 | 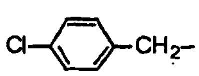 | 1 | 2 | 0 | R | H | 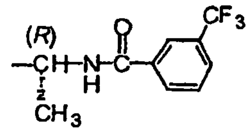 |
| 175 | 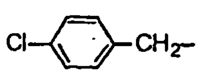 | 1 | 2 | 0 | R | H | 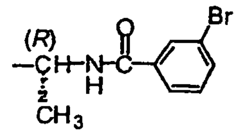 |

(continued)

| Compd. No. | 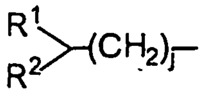 | k | m | n | chirality | $R^3$ | 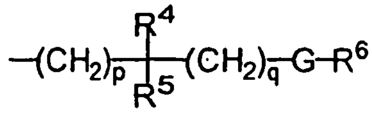 |
|---|---|---|---|---|---|---|---|
| 176 | 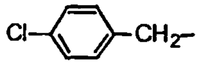 | 10 | 2 | 0 | R | H | 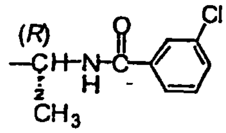 |

**Table 1.17**

| Compd. No. | 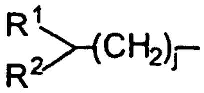 | k | m | n | chirality | $R^3$ | 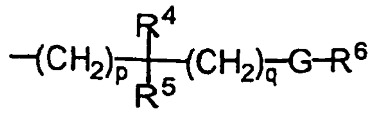 |
|---|---|---|---|---|---|---|---|
| 177 | 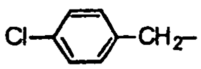 | 1 | 2 | 0 | R | H | 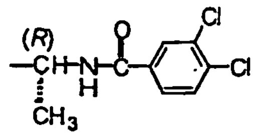 |
| 178 | 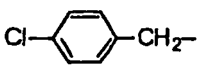 | 1 | 2 | 0 | R | H | 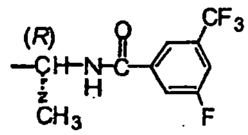 |
| 179 | 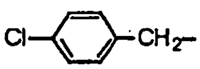 | 1 | 2 | 0 | R | H | 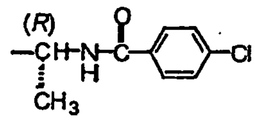 |
| 180 | 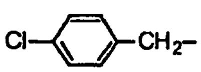 | 1 | 2 | 0 | R | H | 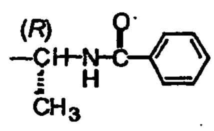 |
| 181 | 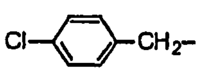 | 1 | 2 | 0 | R | H | 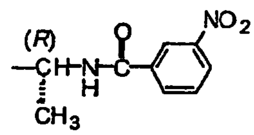 |
| 182 | 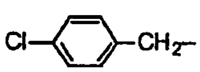 | 1 | 2 | 0 | R | H | 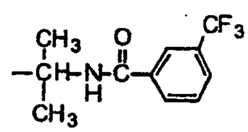 |
| 183 | 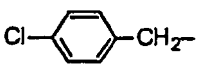 | 1 | 2 | 0 | R | H | 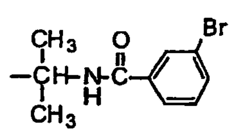 |

(continued)

| Compd. No. | 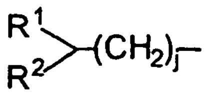 | k | m | n | chirality | $R^3$ | 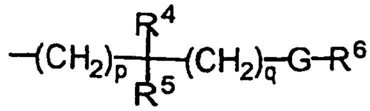 |
|---|---|---|---|---|---|---|---|
| 184 | 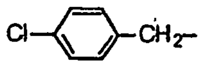 | 1 | 2 | 0 | R | H | 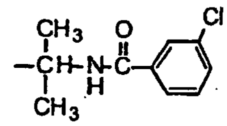 |
| 185 | 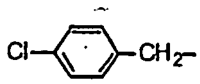 | 1 | 2 | 0 | R | H | 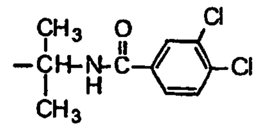 |
| 186 | 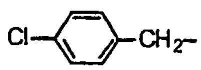 | 1 | 2 | 0 | R | H | 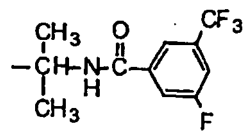 |
| 187 | 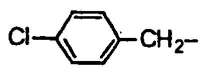 | 1 | 2 | 0 | R | H | 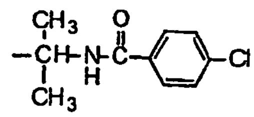 |

**Table 1.18**

| Compd. No. | 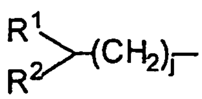 | k | m | n | chirality | $R^3$ | 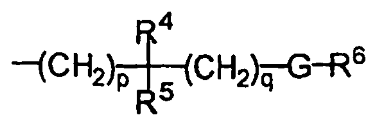 |
|---|---|---|---|---|---|---|---|
| 188 | 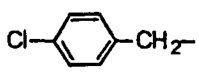 | 1 | 2 | 0 | R | H | 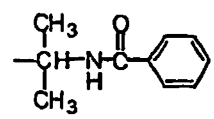 |
| 189 | 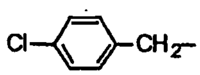 | 1 | 2 | 0 | R | H | 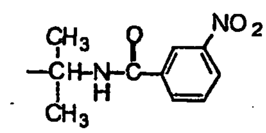 |
| 190 | 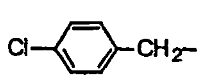 | 1 | 2 | 0 | R | H | 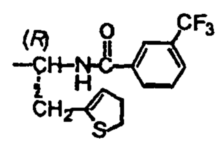 |
| 191 | 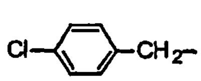 | 1 | 2 | 0 | R | H | 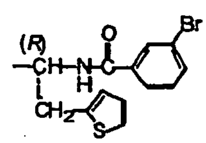 |

(continued)

| Compd. No. | 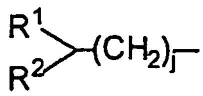 | k | m | n | chirality | $R^3$ | 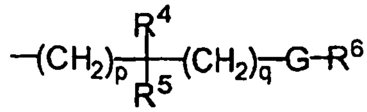 |
|---|---|---|---|---|---|---|---|
| 192 | 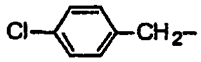 | 1 | 2 | 0 | R | H | 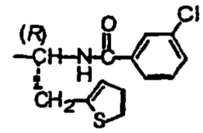 |
| 193 | 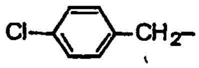 | 1 | 2 | 0 | R | H | 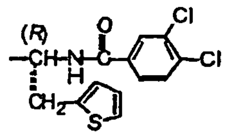 |
| 194 | 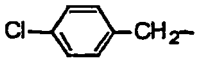 | 1 | 2 | 0 | R | H | 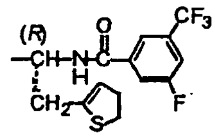 |
| 195 | 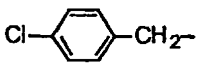 | 1 | 2 | 0 | R | H | 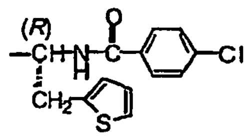 |
| 196 | 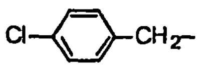 | 1 | 2 | 0 | R | H | 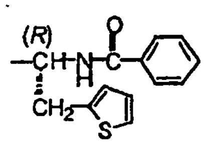 |
| 197 | 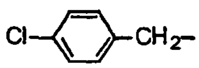 | 1 | 2 | 0 | R | H | 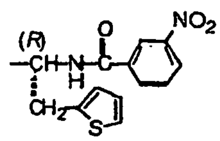 |
| 198 | 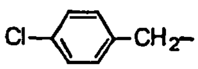 | 1 | 2 | 0 | R | H | 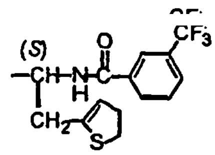 |

**Table 1.19**

| Compd. No. | 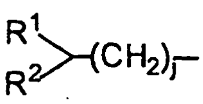 | k | m | n | chirality | $R^3$ | 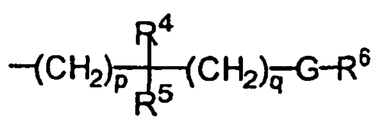 |
|---|---|---|---|---|---|---|---|
| 199 | 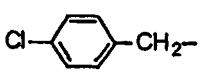 | 1 | 2 | 0 | R | H | 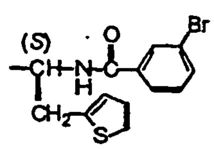 |

(continued)

| Compd. No. | 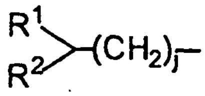 | k | m | n | chirality | $R^3$ | 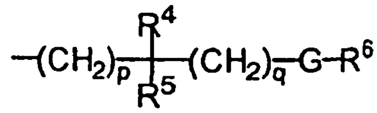 |
|---|---|---|---|---|---|---|---|
| 200 | 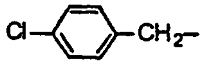 | 1 | 2 | 0 | R | H | 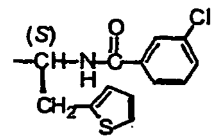 |
| 201 | 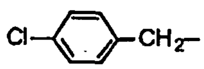 | 1 | 2 | 0 | R | H | 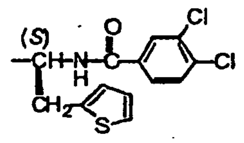 |
| 202 | 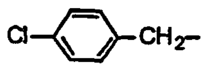 | 1 | 2 | 0 | R | H | 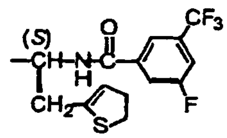 |
| 203 | 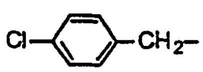 | 1 | 2 | 0 | R | H | 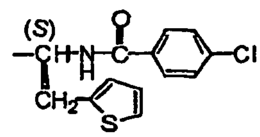 |
| 204 | 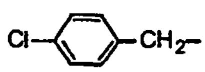 | 1 | 2 | 0 | R | H | 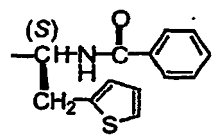 |
| 205 | 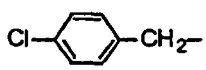 | 1 | 2 | 0 | R | H | 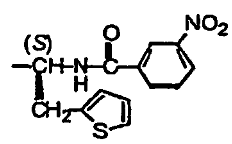 |
| 206 | 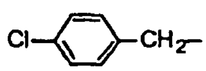 | 1 | 2 | 0 | R | H | 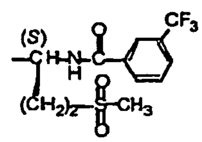 |
| 207 | 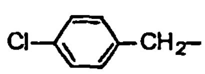 | 1 | 2 | 0 | R | H | 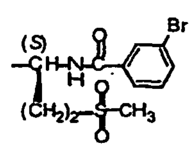 |
| 208 | 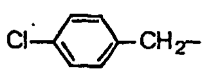 | 1 | 2 | 0 | R | H | 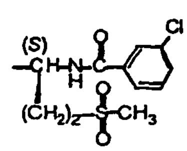 |

(continued)

| Compd. No. | 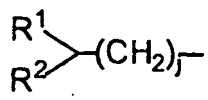 | k | m | n | chirality | $R^3$ | 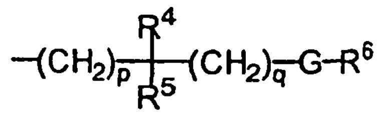 |
|---|---|---|---|---|---|---|---|
| 209 | 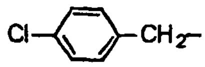 | 1 | 2 | 0 | R | H | 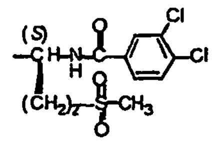 |

**Table 1.20**

| Compd. No. | 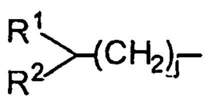 | k | m | n | chirality | $R^3$ | 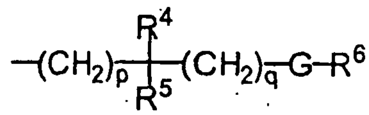 |
|---|---|---|---|---|---|---|---|
| 210 | 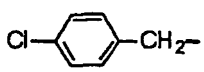 | 1 | 2 | 0 | R | H | 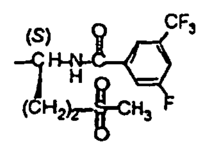 |
| 211 | 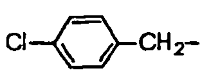 | 1 | 2 | 0 | R | H | 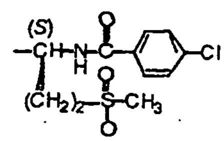 |
| 212 | 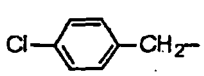 | 1 | 2 | 0 | R | H | 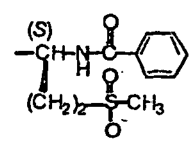 |
| 213 | 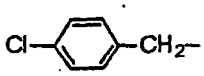 | 1 | 2 | 0 | R | H | 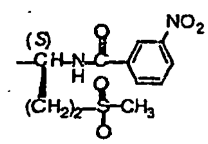 |
| 214 | 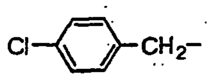 | 1 | 2 | 0 | - | H | 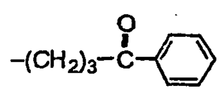 |
| 215 | 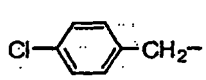 | 1 | 2 | 0 | - | H | 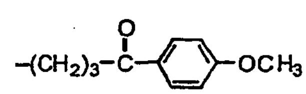 |
| 216 | 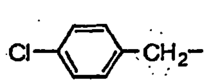 | 1 | 2 | 0 | - | H | 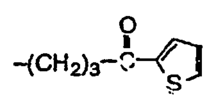 |
| 217 | 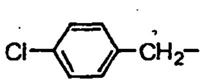 | 1 | 2 | 0 | - | H | 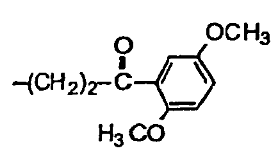 |

(continued)

| Compd. No. | 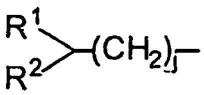 | k | m | n | chirality | $R^3$ | 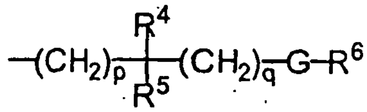 |
|---|---|---|---|---|---|---|---|
| 218 | 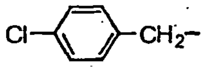 | 1 | 2 | 0 | - | H | 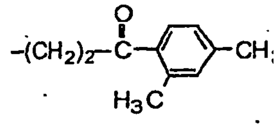 |
| 219 | 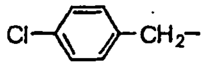 | 1 | 2 | 0 | - | H | 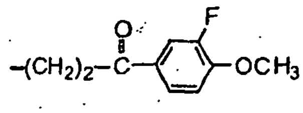 |
| 220 | 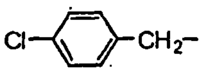 | 1 | 2 | 0 | - | H | 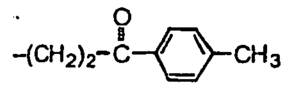 |

**Table 1.21**

| Compd. No. | 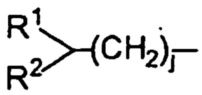 | k | m | n | chirality | $R^3$ | 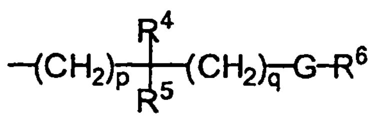 |
|---|---|---|---|---|---|---|---|
| 221 | 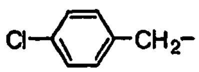 | 1 | 2 | 0 | - | H | 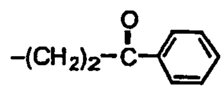 |
| 222 | 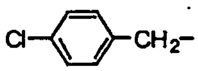 | 1 | 2 | 0 | - | H | 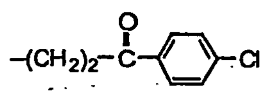 |
| 223 | 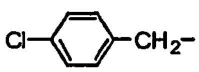 | 1 | 2 | 0 | - | H | 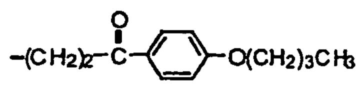 |
| 224 | 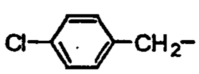 | 1 | 2 | 0 | - | H | 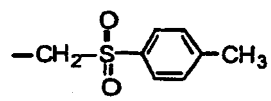 |
| 225 | 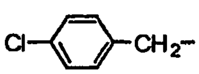 | 1 | 2 | 0 | - | H | 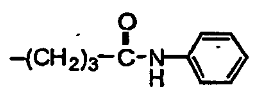 |
| 226 | 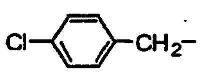 | 1 | 2 | 0 | - | H | 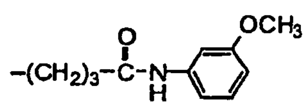 |
| 227 | 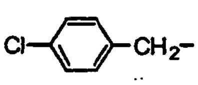 | 1 | 2 | 0 | - | H | 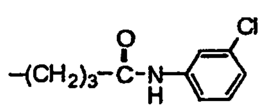 |

(continued)

| Compd. No. | 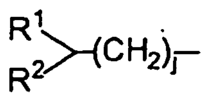 | k | m | n | chirality | $R^3$ | 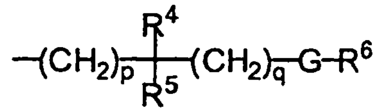 |
|---|---|---|---|---|---|---|---|
| 228 | 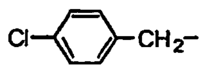 | 1 | 2 | 0 | - | H | 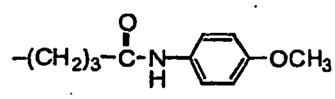 |
| 229 | 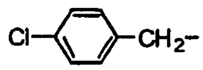 | 1 | 2 | 0 | - | H | 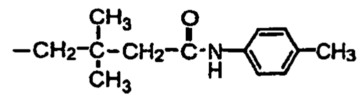 |
| 230 | 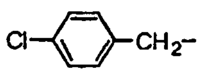 | 1 | 2 | 0 | - | H | 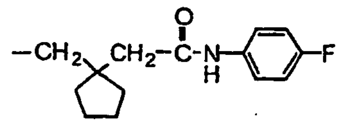 |
| 231 | 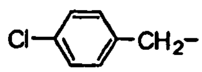 | 1 | 2 | 0 | - | H | 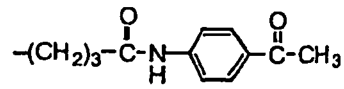 |

**Table 1.22**

| Compd. No. | 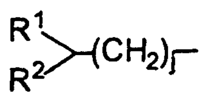 | k | m | n | chirality | $R^3$ | 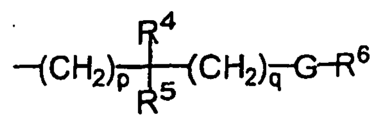 |
|---|---|---|---|---|---|---|---|
| 232 | 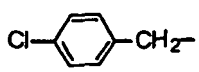 | 1 | 2 | 0 | - | H | 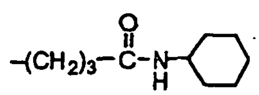 |
| 233 | 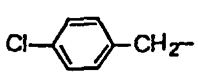 | 1 | 2 | 0 | - | H | 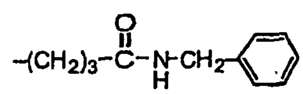 |
| 234 | 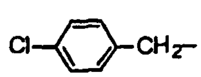 | 1 | 2 | 0 | - | H | 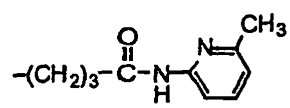 |
| 235 | 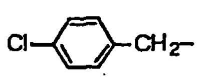 | 1 | 2 | 0 | - | H | 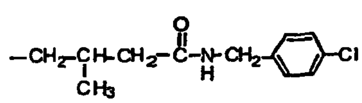 |
| 236 | 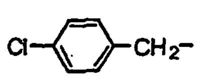 | 1 | 2 | 0 | - | H | 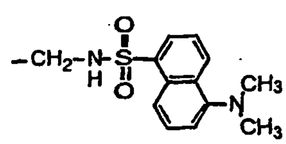 |
| 237 | 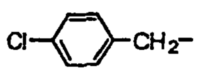 | 1 | 2 | 0 | - | H | 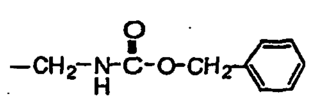 |

(continued)

| Compd. No. | 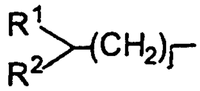 | k | m | n | chirality | $R^3$ | 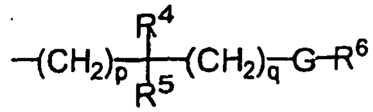 |
|---|---|---|---|---|---|---|---|
| 238 | 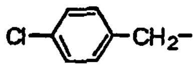 | 1 | 2 | 0 | - | H | 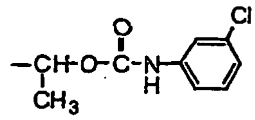 |
| 239 | 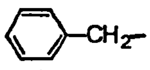 | 1 | 2 | 0 | S | H | 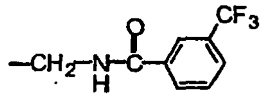 |
| 240 | 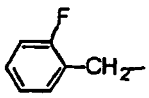 | 1 | 2 | 0 | S | H | 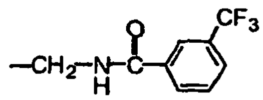 |
| 241 | 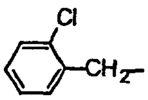 | 1 | 2 | 0 | S | H | 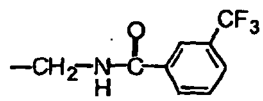 |
| 242 | 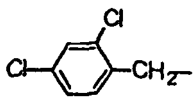 | 1 | 2 | 0 | S | H | 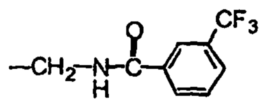 |

**Table 1.23**

| Compd. No. | 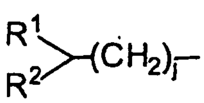 | k | m | n | chirality | $R^3$ | 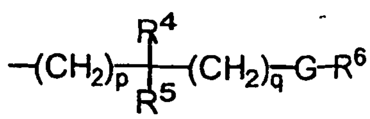 |
|---|---|---|---|---|---|---|---|
| 243 | 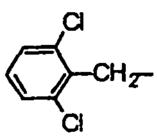 | 1 | 2 | 0 | S | H | 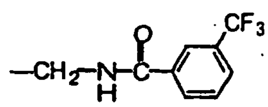 |
| 244 | 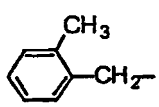 | 1 | 2 | 0 | S | H | 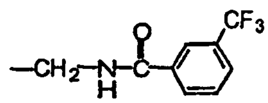 |
| 245 | 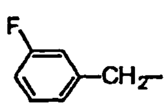 | 1 | 2 | 0 | S | H | 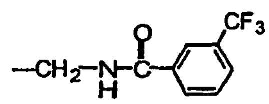 |
| 246 | 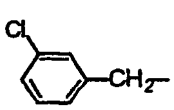 | 1 | 2 | 0 | S | H | 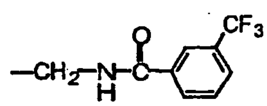 |

(continued)

| Compd. No. | 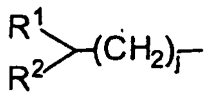 | k | m | n | chirality | $R^3$ | 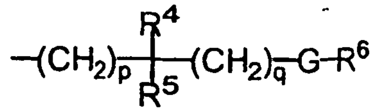 |
|---|---|---|---|---|---|---|---|
| 247 | 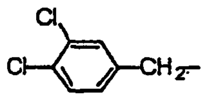 | 1 | 2 | 0 | S | H | 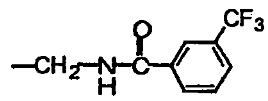 |
| 248 | 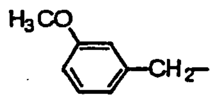 | 1 | 2 | 0 | S | H | 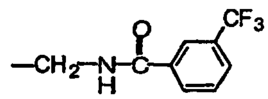 |
| 249 | 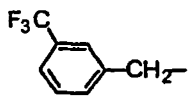 | 1 | 2 | 0 | S | H | 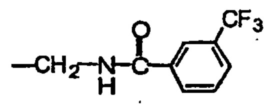 |
| 250 | 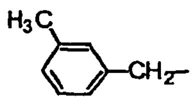 | 1 | 2 | 0 | S | H | 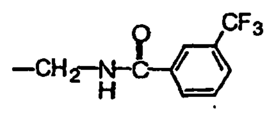 |
| 251 | 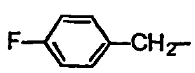 | 1 | 2 | 0 | S | H | 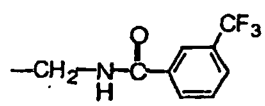 |
| 252 | 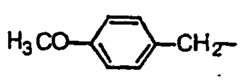 | 1 | 2 | 0 | S | H | 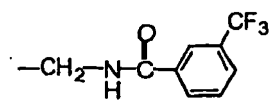 |
| 253 | 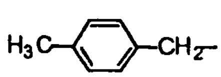 | 1 | 2 | 0 | S | H | 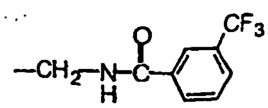 |

**Table 1.24**

| Compd. No. | 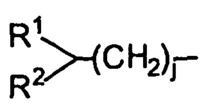 | k | m | n | chirality | $R^3$ | 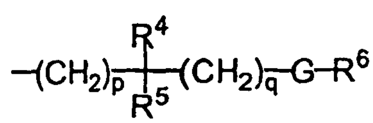 |
|---|---|---|---|---|---|---|---|
| 254 | 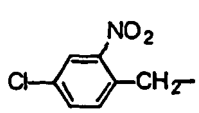 | 1 | 2 | 0 | S | H | 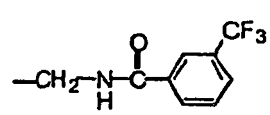 |
| 255 | 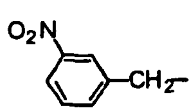 | 1 | 2 | 0 | S | H | 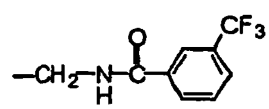 |

(continued)

| Compd. No. | 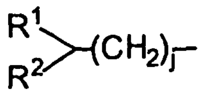 | k | m | n | chirality | $R^3$ | 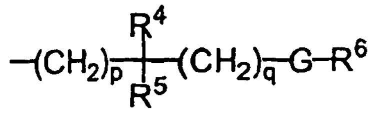 |
|---|---|---|---|---|---|---|---|
| 256 | 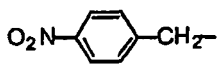 | 1 | 2 | 0 | S | H | 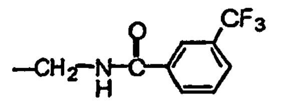 |
| 257 | 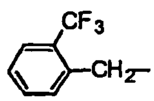 | 1 | 2 | 0 | S | H | 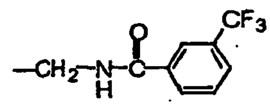 |
| 258 | 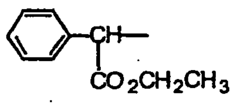 | 1 | 2 | 0 | S | H | 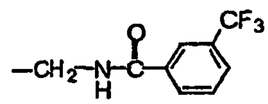 |
| 259 | 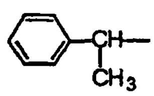 | 1 | 2 | 0 | S | H | 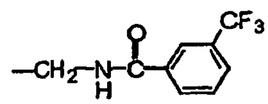 |
| 260 | 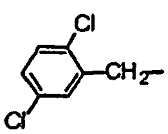 | 1 | 2 | 0 | S | H | 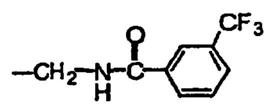 |
| 261 | 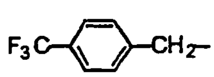 | 1 | 2 | 0 | S | H | 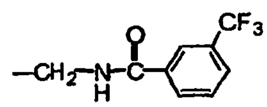 |
| 262 | 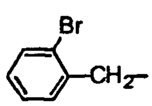 | 1 | 2 | 0 | S | H | 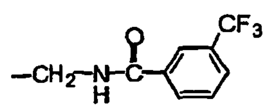 |
| 263 | 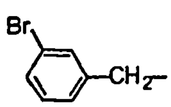 | 1 | 2 | 0 | S | H | 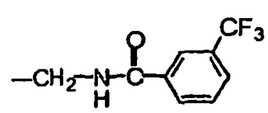 |
| 264 | 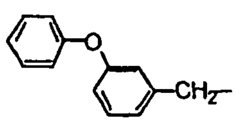 | 1 | 2 | 0 | S | H | 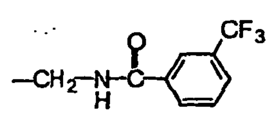 |

**Table 1.25**

| Compd. No. | 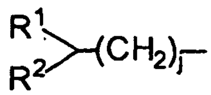 | k | m | n | chirality | $R^3$ | 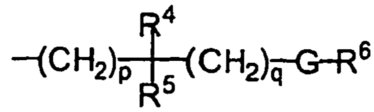 |
|---|---|---|---|---|---|---|---|
| 265 | 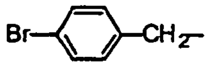 | 1 | 2 | 0 | S | H | 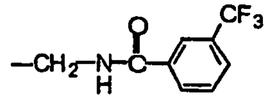 |
| 266 | 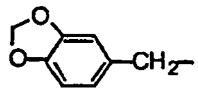 | 1 | 2 | 0 | S | H | 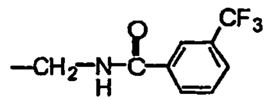 |
| 267 | 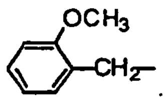 | 1 | 2 | 0 | S | H | 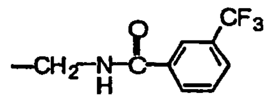 |
| 268 | 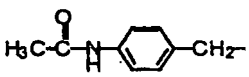 | 1 | 2 | 0 | S | H | 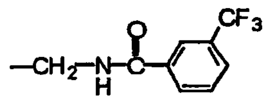 |
| 269 | 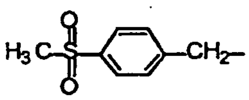 | 1 | 2 | 0 | S | H | 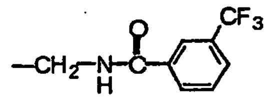 |
| 270 | 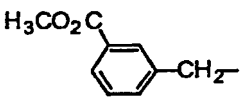 | 1 | 2 | 0 | S | H | 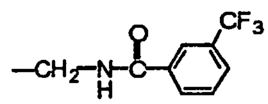 |
| 271 | 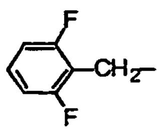 | 1 | 2 | 0 | S | H | 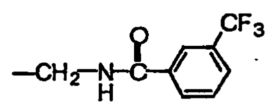 |
| 272 | 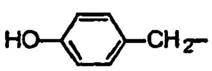 | 1 | 2 | 0 | S | H | 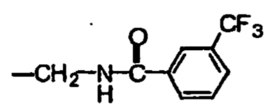 |
| 273 | 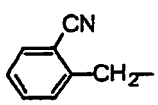 | 1 | 2 | 0 | S | H | 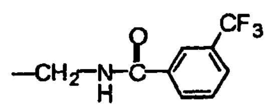 |
| 274 | 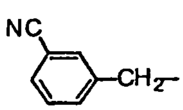 | 1 | 2 | 0 | S | H | 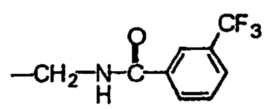 |
| 275 | 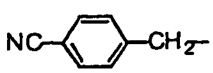 | 1 | 2 | 0 | S | H | 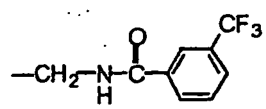 |

**Table 1.26**

| Compd. No. | 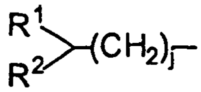 | k | m | n | chirality | $R^3$ | 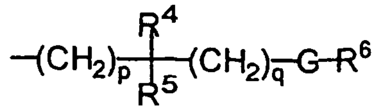 |
|---|---|---|---|---|---|---|---|
| 276 | 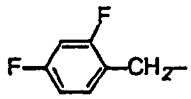 | 1 | 2 | 0 | S | H | 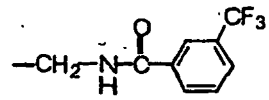 |
| 277 | 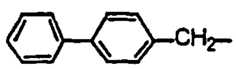 | 1 | 2 | 0 | S | H | 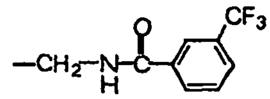 |
| 278 | 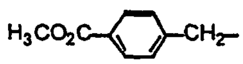 | 1 | 2 | 0 | S | H | 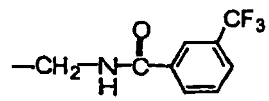 |
| 279 | 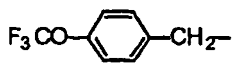 | 1 | 2 | 0 | S | H | 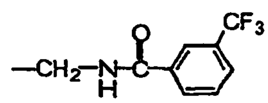 |
| 280 | 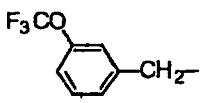 | 1 | 2 | 0 | S | H | 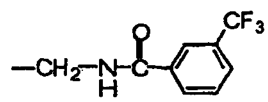 |
| 281 | 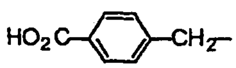 | 1 | 2 | 0 | S | H | 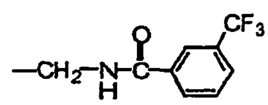 |
| 282 | 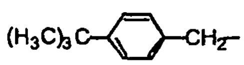 | 1 | 2 | 0 | S | H | 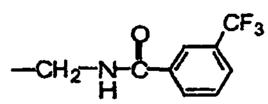 |
| 283 | 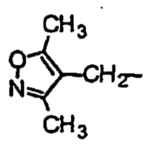 | 1 | 2 | 0 | S | H | 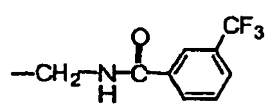 |
| 284 | 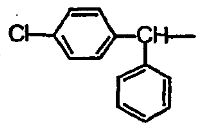 | 1 | 2 | 0 | S | H | 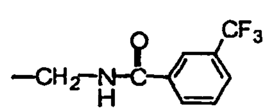 |
| 285 | 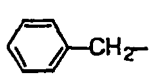 | 1 | 2 | 0 | R | H | 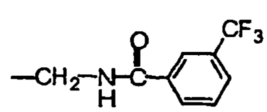 |

(continued)

| Compd. No. | 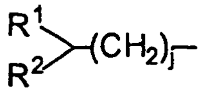 | k | m | n | chirality | $R^3$ | 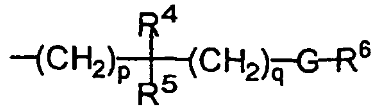 |
|---|---|---|---|---|---|---|---|
| 286 | 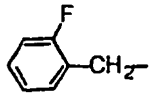 | 1 | 2 | 0 | R | H | 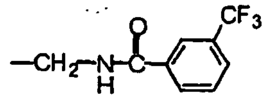 |

**Table 1.27**

| Compd. No. | 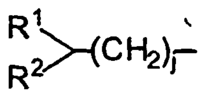 | k | m | n | chirality | $R^3$ | 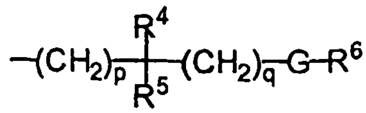 |
|---|---|---|---|---|---|---|---|
| 287 | 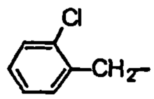 | 1 | 2 | 0 | R | H | 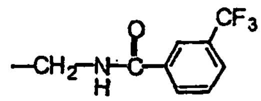 |
| 288 | 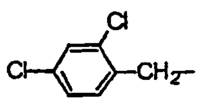 | 1 | 2 | 0 | R | H | 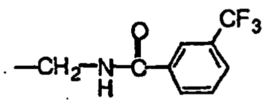 |
| 289 | 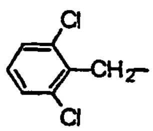 | 1 | 2 | 0 | R | H | 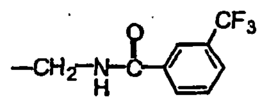 |
| 290 | 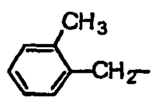 | 1 | 2 | 0 | R | H | 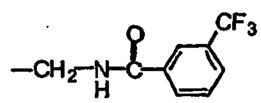 |
| 291 | 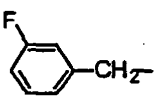 | 1 | 2 | 0 | R | H | 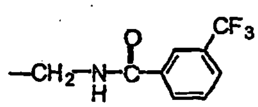 |
| 292 | 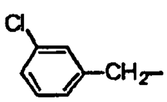 | 1 | 2 | 0 | R | H | 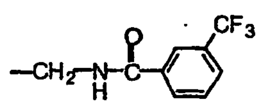 |
| 293 | 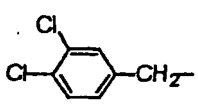 | 1 | 2 | 0 | R | H | 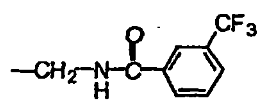 |

(continued)

| Compd. No. | 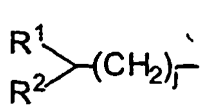 | k | m | n | chirality | $R^3$ | 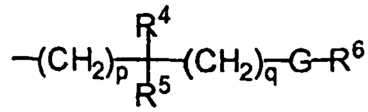 |
|---|---|---|---|---|---|---|---|
| 294 | 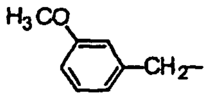 | 1 | 2 | 0 | R | H | 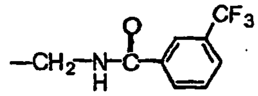 |
| 295 | 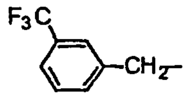 | 1 | 2 | 0 | R | H | 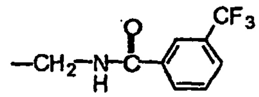 |
| 296 | 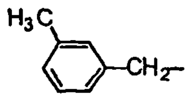 | 1 | 2 | 0 | R | H | 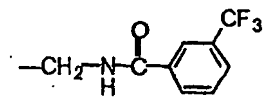 |
| 297 | 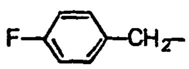 | 1 | 2 | 0 | R | H | 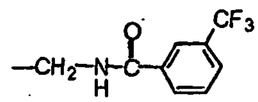 |

**Table 1.28**

| Compd. No. | 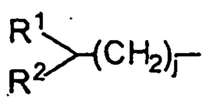 | k | m | n | chirality | $R^3$ | 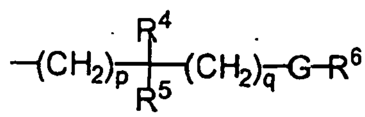 |
|---|---|---|---|---|---|---|---|
| 298 | 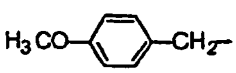 | 1 | 2 | 0 | R | H | 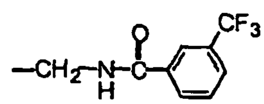 |
| 299 | 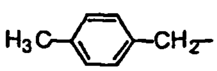 | 1 | 2 | 0 | R | H | 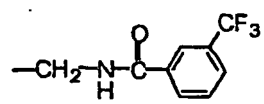 |
| 300 | 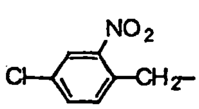 | 1 | 2 | 0 | R | H | 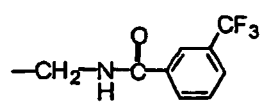 |
| 301 | 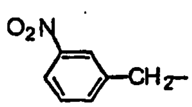 | 1 | 2 | 0 | R | H | 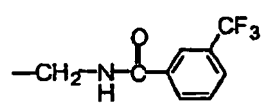 |
| 302 | 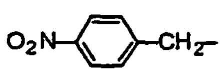 | 1 | 2 | 0 | R | H | 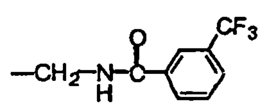 |

(continued)

| Compd. No. | 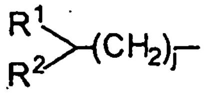 | k | m | n | chirality | $R^3$ | 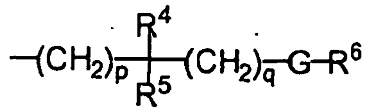 |
|---|---|---|---|---|---|---|---|
| 303 | 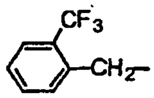 | 1 | 2 | 0 | R | H | 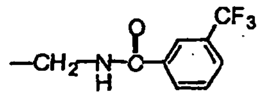 |
| 304 | 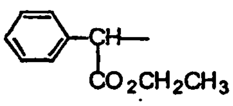 | 1 | 2 | 0 | R | H | 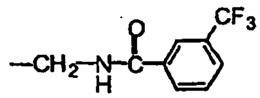 |
| 305 | 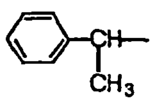 | 1 | 2 | 0 | R | H | 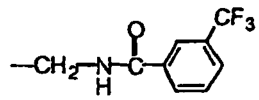 |
| 306 | 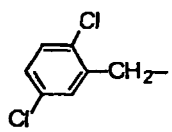 | 1 | 2 | 0 | R | H | 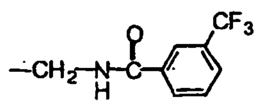 |
| 307 | 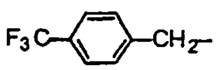 | 1 | 2 | 0 | R | H | 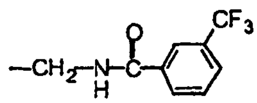 |
| 308 | 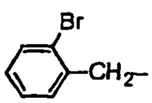 | 1 | 2 | 0 | R | H | 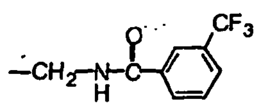 |

**Table 1.29**

| Compd. No. | 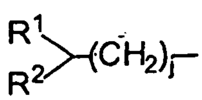 | k | m | n | chirality | $R^3$ | 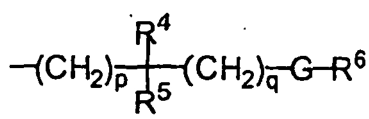 |
|---|---|---|---|---|---|---|---|
| 309 | 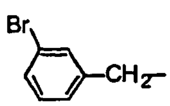 | 1 | 2 | 0 | R | H | 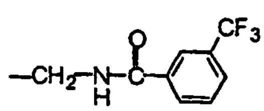 |
| 310 | 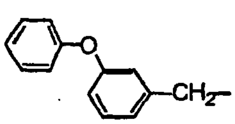 | 1 | 2 | 0 | R | H | 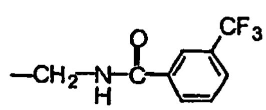 |
| 311 | 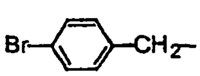 | 1 | 2 | 0 | R | H | 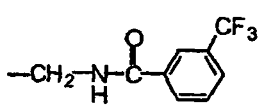 |

(continued)

| Compd. No. | 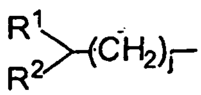 | k | m | n | chirality | $R^3$ | 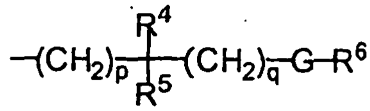 |
|---|---|---|---|---|---|---|---|
| 312 | 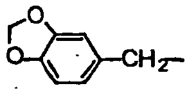 | 1 | 2 | 0 | R | H | 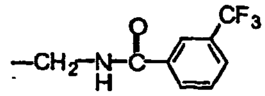 |
| 313 | 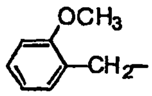 | 1 | 2 | 0 | R | H | 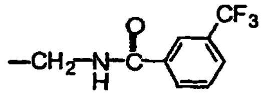 |
| 314 | 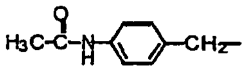 | 1 | 2 | 0 | R | H | 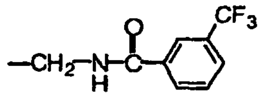 |
| 315 | 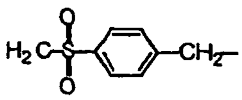 | 1 | 2 | 0 | R | H | 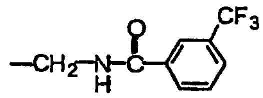 |
| 316 | 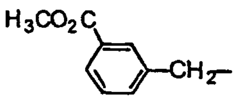 | 1 | 2 | 0 | R | H | 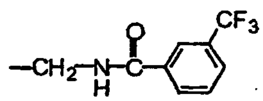 |
| 317 | 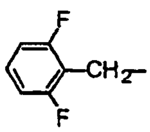 | 1 | 2 | 0 | R | H | 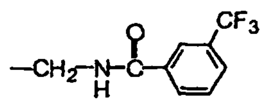 |
| 318 | 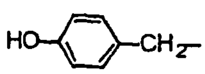 | 1 | 2 | 0 | R | H | 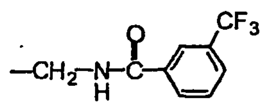 |
| 319 | 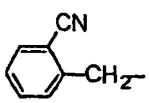 | 1 | 2 | 0 | R | H | 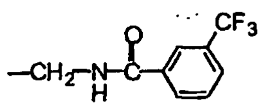 |

**Table 1.30**

| Compd. No. | 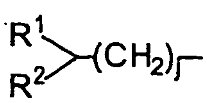 | k | m | n | chirality | $R^3$ | 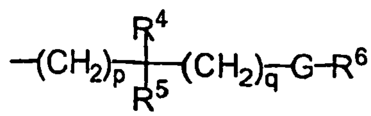 |
|---|---|---|---|---|---|---|---|
| 320 | 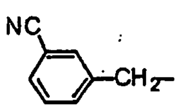 | 1 | 2 | 0 | R | H | 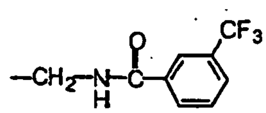 |

(continued)

| Compd. No. | 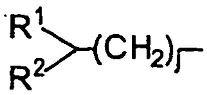 | k | m | n | chirality | $R^3$ | 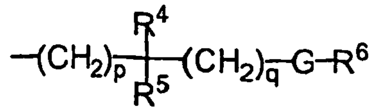 |
|---|---|---|---|---|---|---|---|
| 321 | 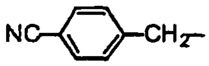 | 1 | 2 | 0 | R | H | 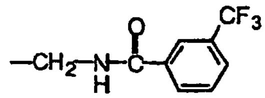 |
| 322 | 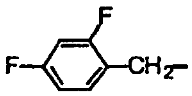 | 1 | 2 | 0 | R | H | 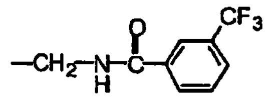 |
| 323 | 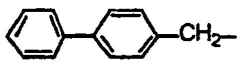 | 1 | 2 | 0 | R | H | 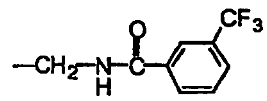 |
| 324 | 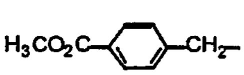 | 1 | 2 | 0 | R | H | 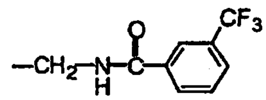 |
| 325 | 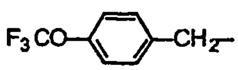 | 1 | 2 | 0 | R | H | 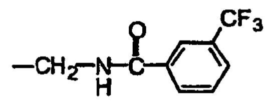 |
| 326 | 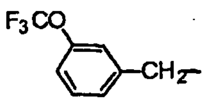 | 1 | 2 | 0 | R | H | 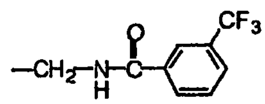 |
| 327 | 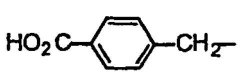 | 1 | 2 | 0 | R | H | 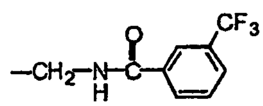 |
| 328 | 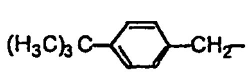 | 1 | 2 | 0 | R | H | 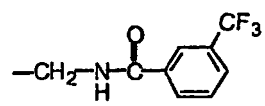 |
| 329 | 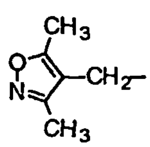 | 1 | 2 | 0 | R | H | 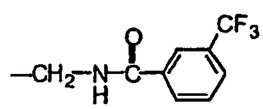 |
| 330 | 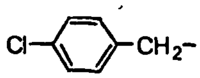 | 0 | 3 | 1 | - | H | 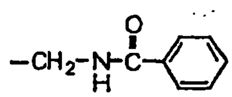 |

**Table 1.31**

| Compd. No. | 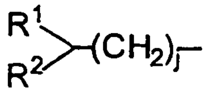 | k | m | n | chirality | $R^3$ | 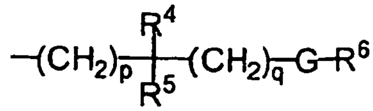 |
|---|---|---|---|---|---|---|---|
| 331 | 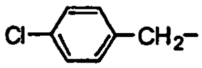 | 0 | 3 | 1 | - | H | 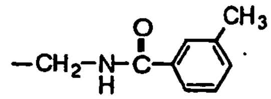 |
| 332 | 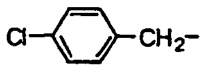 | 0 | 3 | 1 | - | H | 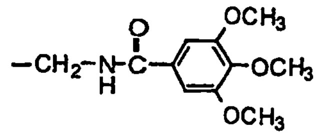 |
| 333 | 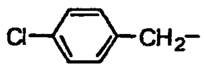 | 0 | 3 | 1 | - | H | 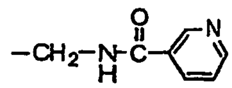 |
| 334 | 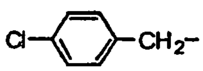 | 0 | 3 | 1 | - | H | 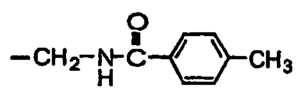 |
| 335 | 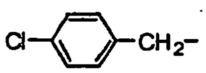 | 0 | 3 | 1 | - | H | 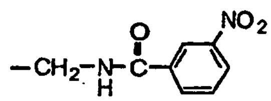 |
| 336 | 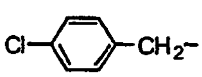 | 0 | 3 | 1 | - | H | 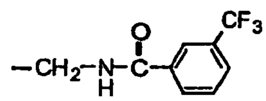 |
| 337 | 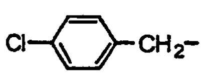 | 0 | 3 | 1 | - | H | 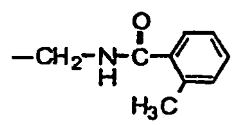 |
| 338 | 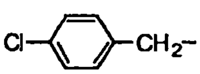 | 0 | 3 | 1 | - | H | 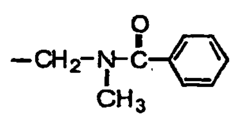 |
| 339 | 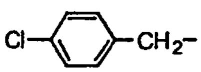 | 0 | 3 | 1 | R | H | 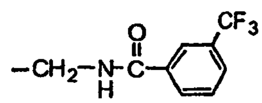 |
| 340 | 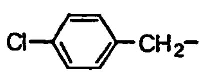 | 0 | 3 | 1 | S | H | 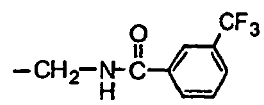 |
| 341 | 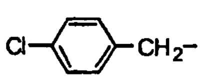 | 0 | 3 | 1 | - | H | 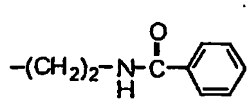 |

**Table 1.32**

| Compd. No. | 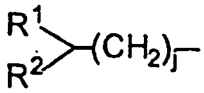 | k | m | n | chirality | $R^3$ | 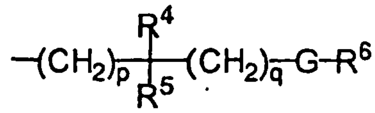 |
|---|---|---|---|---|---|---|---|
| 342 | 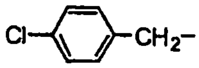 | 0 | 3 | 1 | - | H | 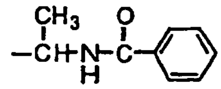 |
| 343 | 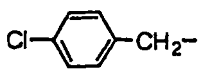 | 0 | 3 | 1 | - | H | 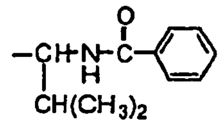 |
| 344 | 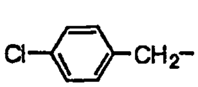 | 0 | 3 | 1 | - | H | 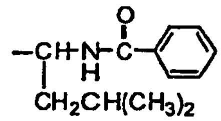 |
| 345 | 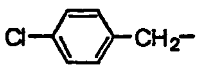 | 0 | 3 | 1 | - | H | 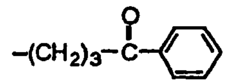 |
| 346 | 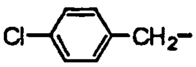 | 0 | 3 | 1 | - | H | 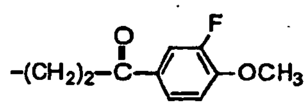 |
| 347 | 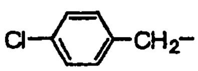 | 0 | 3 | 1 | - | H | 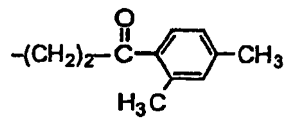 |
| 348 | 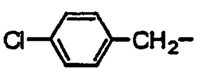 | 0 | 3 | 1 | - | H | 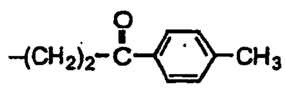 |
| 349 | 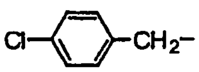 | 0 | 3 | 1 | - | H | 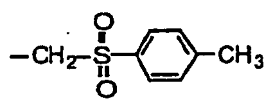 |
| 350 | 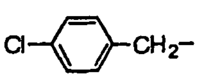 | 0 | 3 | 1 | - | H | 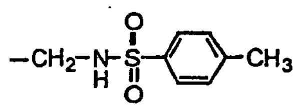 |
| 351 | 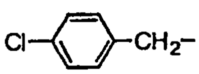 | 0 | 3 | 1 | - | H | 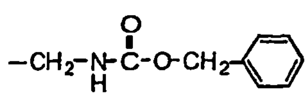 |

(continued)

| Compd. No. | 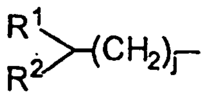 | k | m | n | chirality | $R^3$ | 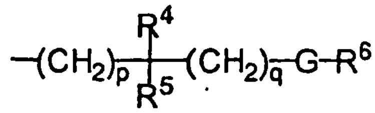 |
|---|---|---|---|---|---|---|---|
| 352 | 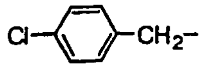 | 0 | 3 | 1 | - | H | 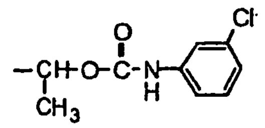 |

**Table 1.33**

| Compd. No. | 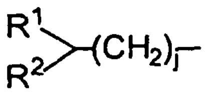 | k | m | n | chirality | $R^3$ | 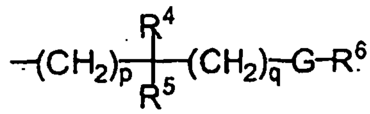 |
|---|---|---|---|---|---|---|---|
| 353 | 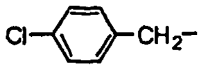 | 1 | 2 | 1 | - | H | 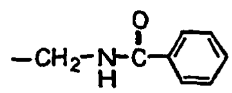 |
| 354 | 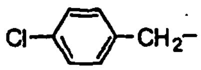 | 1 | 3 | 0 | - | H | 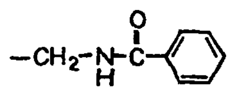 |
| 355 | 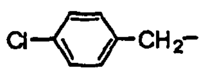 | 1 | 3 | 0 | - | H | 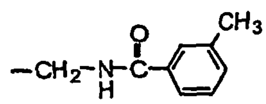 |
| 356 | 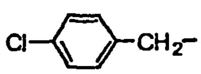 | 1 | 3 | 0 | - | H | 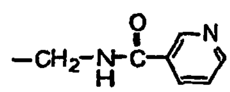 |
| 357 | 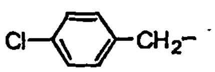 | 1 | 3 | 0 | - | H | 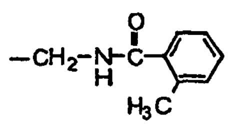 |
| 358 | 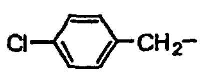 | 1 | 3 | 0 | - | H | 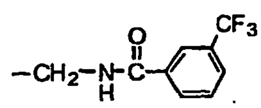 |
| 359 | 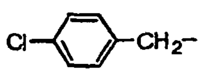 | 1 | 3 | 0 | - | H | 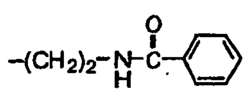 |

(continued)

| Compd. No. | 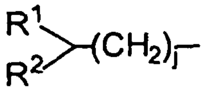 | k | m | n | chirality | $R^3$ | 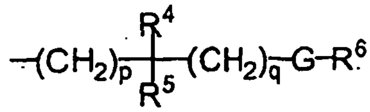 |
|---|---|---|---|---|---|---|---|
| 360 | 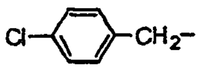 | 1 | 3 | 0 | - | H | 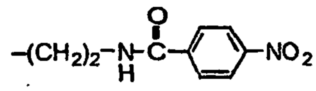 |
| 361 | 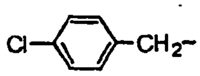 | 1 | 3 | 0 | - | H | 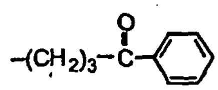 |
| 362 | 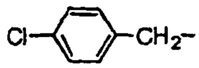 | 1 | 3 | 0 | - | H | 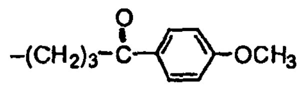 |
| 363 | 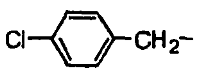 | 1 | 3 | 0 | - | H | 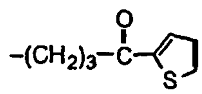 |

**Table 1.34**

| Compd. No. | 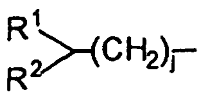 | k | m | n | chirality | $R^3$ | 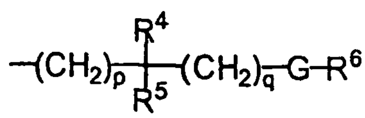 |
|---|---|---|---|---|---|---|---|
| 364 | 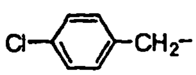 | 1 | 3 | 0 | - | H | 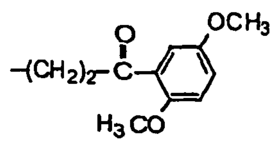 |
| 365 | 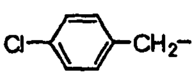 | 1 | 3 | 0 | - | H | 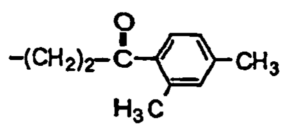 |
| 366 | 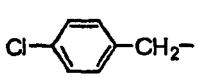 | 1 | 3 | 0 | - | H | 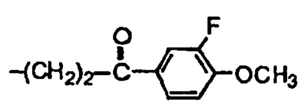 |
| 367 | 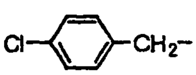 | 1 | 3 | 0 | - | H | 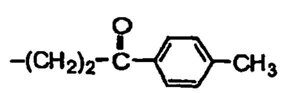 |
| 368 | 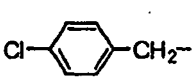 | 1 | 3 | 0 | - | H | 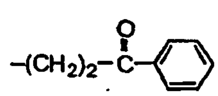 |

(continued)

| Compd. No. | 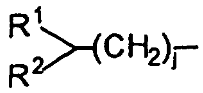 | k | m | n | chirality | $R^3$ | 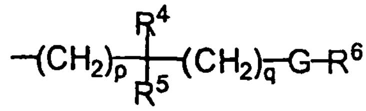 |
|---|---|---|---|---|---|---|---|
| 369 | 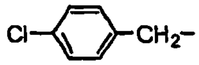 | 1 | 3 | 0 | - | H | 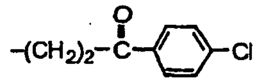 |
| 370 | 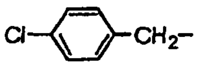 | 1 | 3 | 0 | - | H | 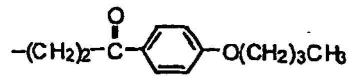 |
| 371 | 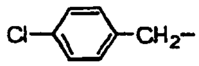 | 1 | 3 | 0 | - | H | 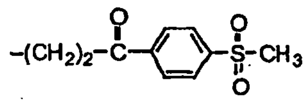 |
| 372 | 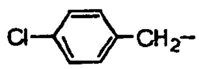 | 1 | 3 | 0 | - | H | 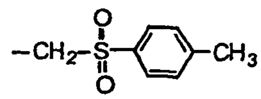 |
| 373 | 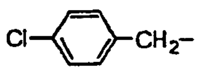 | 1 | 3 | 0 | - | H | 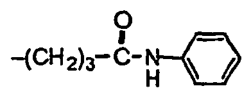 |
| 374 | 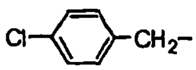 | 1 | 3 | 0 | - | H | 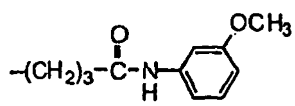 |

**Table 1.35**

| Compd. No. | 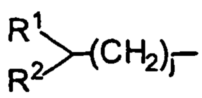 | k | m | n | chirality | $R^3$ | 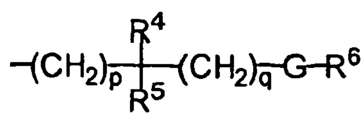 |
|---|---|---|---|---|---|---|---|
| 375 | 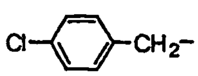 | 1 | 3 | 0 | - | H | 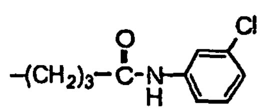 |
| 376 | 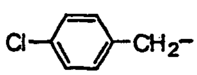 | 1 | 3 | 0 | - | H | 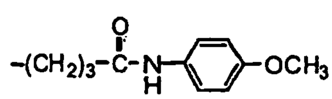 |
| 377 | 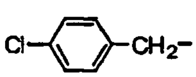 | 1 | 3 | 0 | - | H | 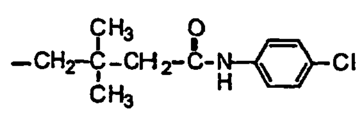 |

(continued)

| Compd. No. | 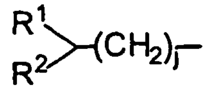 | k | m | n | chirality | $R^3$ | 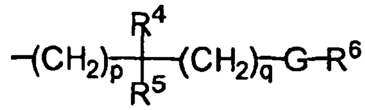 |
|---|---|---|---|---|---|---|---|
| 378 | 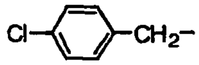 | 1 | 3 | 0 | - | H | 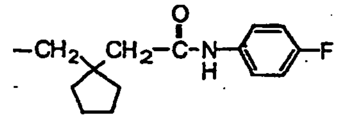 |
| 379 | 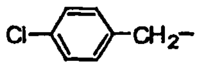 | 1 | 3 | 0 | - | H | 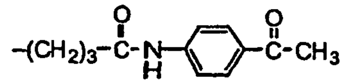 |
| 380 | 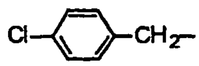 | 1 | 3 | 0 | - | H | 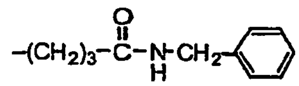 |
| 381 | 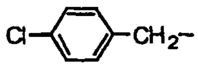 | 1 | 3 | 0 | - | H | 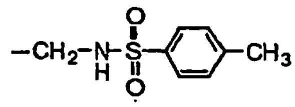 |
| 382 | 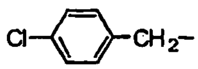 | 1 | 3 | 0 | - | H | 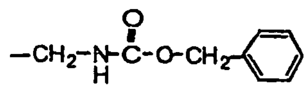 |
| 383 | 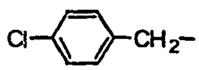 | 1 | 3 | 0 | - | H | 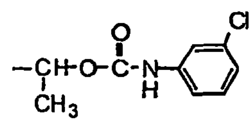 |
| 384 | 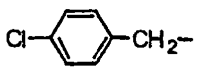 | 2 | 2 | 0 | - | H | 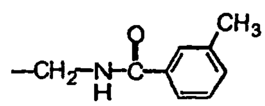 |
| 385 | 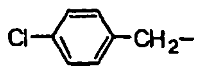 | 2 | 2 | 0 | - | H | 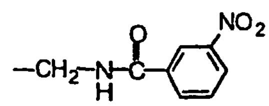 |

**Table 1.36**

| Compd. No. | 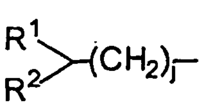 | k | m | n | chirality | $R^3$ | 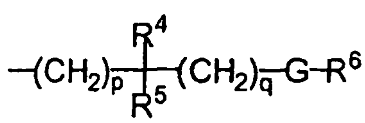 |
|---|---|---|---|---|---|---|---|
| 386 | 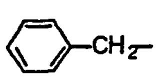 | 2 | 2 | 0 | - | H | 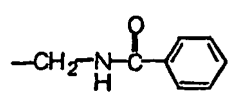 |
| 387 | 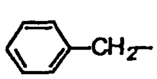 | 2 | 2 | 0 | - | H | 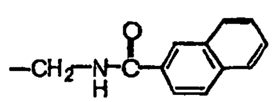 |

(continued)

| Compd. No. | 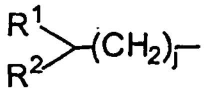 | k | m | n | chirality | $R^3$ | 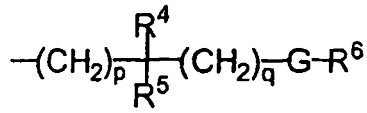 |
|---|---|---|---|---|---|---|---|
| 388 | 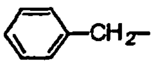 | 2 | 2 | 0 | - | H | 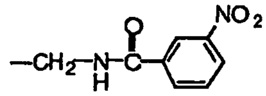 |
| 389 | 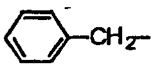 | 2 | 2 | 0 | - | H | 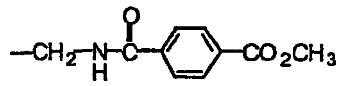 |
| 390 | 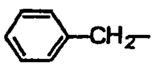 | 2 | 2 | 0 | - | H | 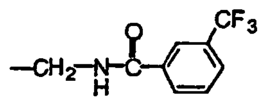 |
| 391 | 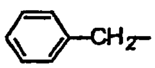 | 2 | 2 | 0 | - | H | 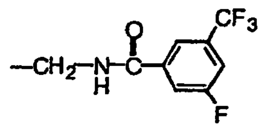 |
| 392 | 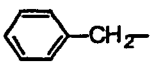 | 2 | 2 | 0 | - | H | 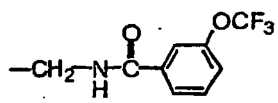 |
| 393 | 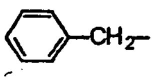 | 2 | 2 | 0 | - | H | 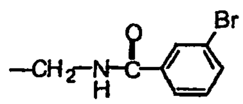 |
| 394 | 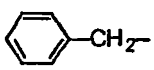 | 2 | 2 | 0 | - | H | 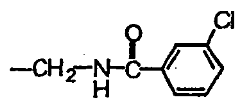 |
| 395 | 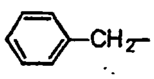 | 2 | 2 | 0 | - | H | 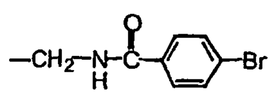 |
| 396 | 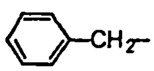 | 2 | 2 | 0 | - | H | 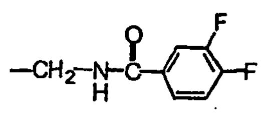 |

Table 1.37

| Compd. No. | 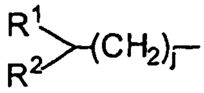 | k | m | n | chirality | $R^3$ | 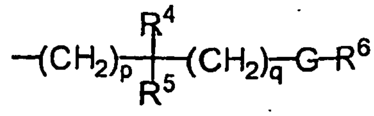 |
|---|---|---|---|---|---|---|---|
| 397 | 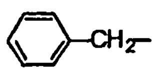 | 2 | 2 | 0 | - | H | 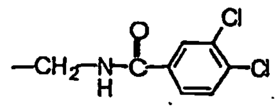 |
| 398 | 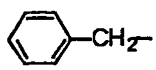 | 2 | 2 | 0 | - | H | 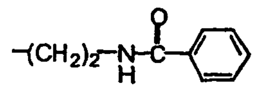 |
| 399 | 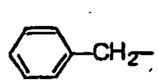 | 2 | 2 | 0 | - | H | 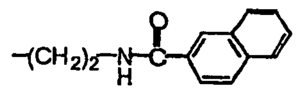 |
| 400 | 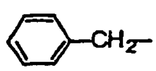 | 2 | 2 | 0 | - | H | 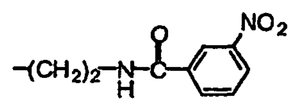 |
| 401 | 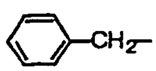 | 2 | 2 | 0 | - | H | 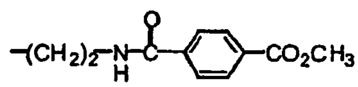 |
| 402 | 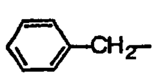 | 2 | 2 | 0 | - | H | 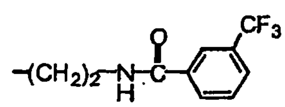 |
| 403 | 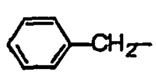 | 2 | 2 | 0 | - | H | 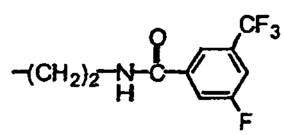 |
| 404 | 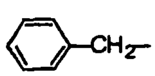 | 2 | 2 | 0 | - | H | 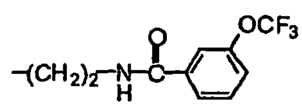 |
| 405 | 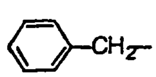 | 2 | 2 | 0 | - | H | 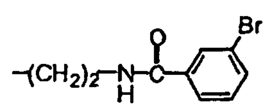 |
| 406 | 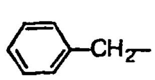 | 2 | 2 | 0 | - | H | 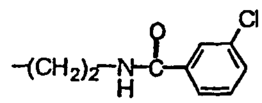 |
| 407 | 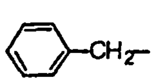 | 2 | 2 | 0 | - | H | 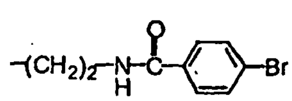 |

**Table 1.38**

| Compd. No. | 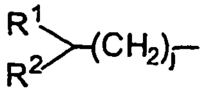 | k | m | n | chirality | $R^3$ | 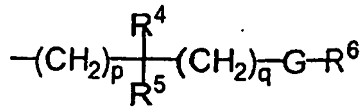 |
|---|---|---|---|---|---|---|---|
| 408 | 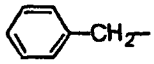 | 2 | 2 | 0 | - | H | 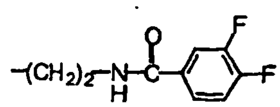 |
| 409 | 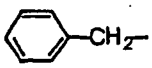 | 2 | 2 | 0 | - | H | 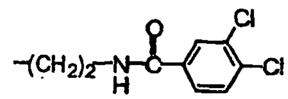 |
| 410 | 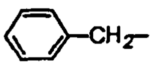 | 2 | 2 | 0 | - | H | 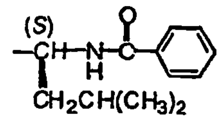 |
| 411 | 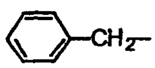 | 2 | 2 | 0 | - | H | 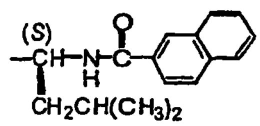 |
| 412 | 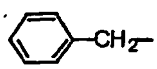 | 2 | 2 | 0 | - | H | 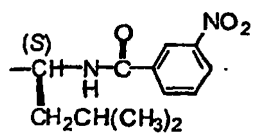 |
| 413 | 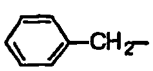 | 2 | 2 | 0 | - | H | 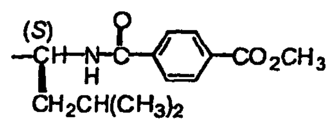 |
| 414 | 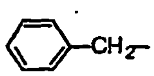 | 2 | 2 | 0 | - | H | 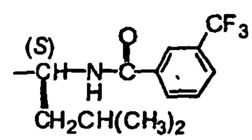 |
| 415 | 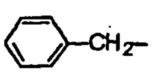 | 2 | 2 | 0 | - | H | 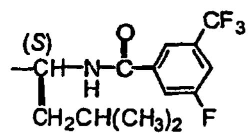 |
| 416 | 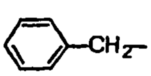 | 2 | 2 | 0 | - | H | 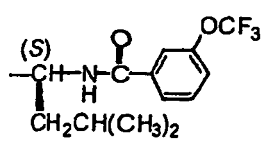 |
| 417 | 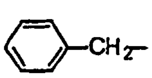 | 2 | 2 | 0 | - | H | 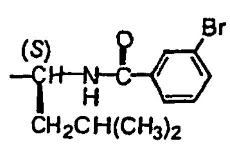 |

(continued)

| Compd. No. | 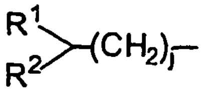 | k | m | n | chirality | $R^3$ | 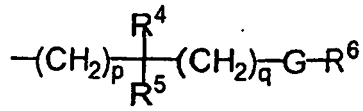 |
|---|---|---|---|---|---|---|---|
| 418 | 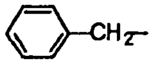 | 2 | 2 | 0 | - | H | 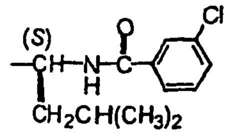 |

**Table 1.39**

| Compd. No. | 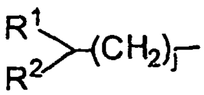 | k | m | n | chirality | $R^3$ | 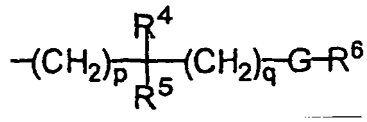 |
|---|---|---|---|---|---|---|---|
| 419 | 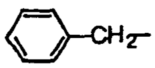 | 2 | 2 | 0 | - | H | 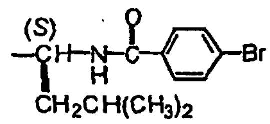 |
| 420 | 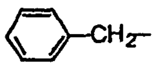 | 2 | 2 | 0 | - | H | 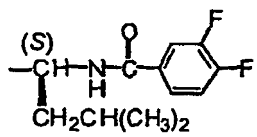 |
| 421 | 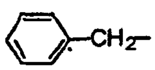 | 2 | 2 | 0 | - | H | 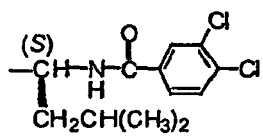 |
| 422 | 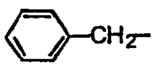 | 2 | 2 | 0 | - | H | 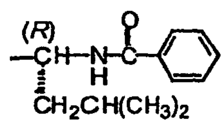 |
| 423 | 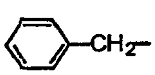 | 2 | 2 | 0 | - | H | 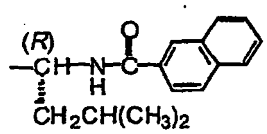 |
| 424 | 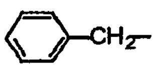 | 2 | 2 | 0 | - | H | 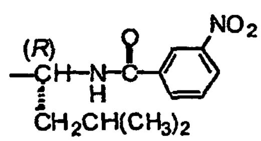 |
| 425 | 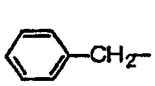 | 2 | 2 | 0 | - | H | 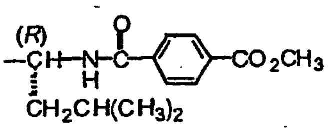 |

(continued)

| Compd. No. | 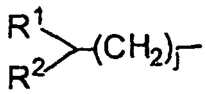 | k | m | n | chirality | $R^3$ | 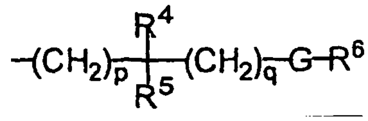 |
|---|---|---|---|---|---|---|---|
| 426 | 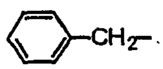 | 2 | 2 | 0 | - | H | 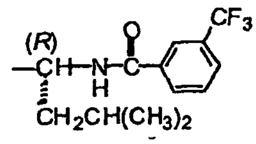 |
| 427 | 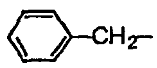 | 2 | 2 | 0 | - | H | 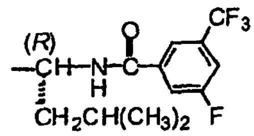 |
| 428 | 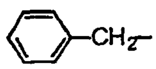 | 2 | 2 | 0 | - | H | 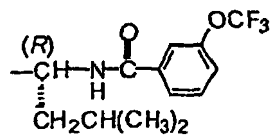 |
| 429 | 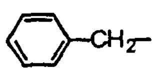 | 2 | 2 | 0 | - | H | 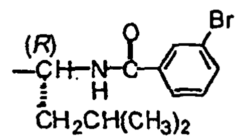 |

**Table 1.40**

| Compd. No. | 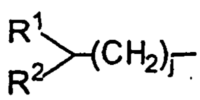 | k | m | n | chirality | $R^3$ | 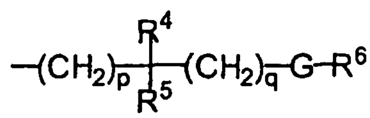 |
|---|---|---|---|---|---|---|---|
| 430 | 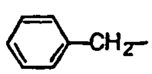 | 2 | 2 | 0 | - | H | 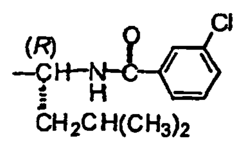 |
| 431 | 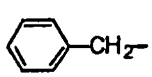 | 2 | 2 | 0 | - | H | 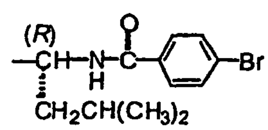 |
| 432 | 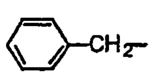 | 2 | 2 | 0 | - | H | 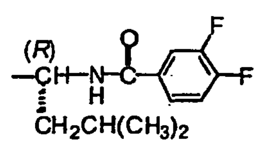 |
| 433 | 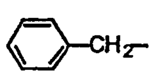 | 2 | 2 | 0 | - | H | 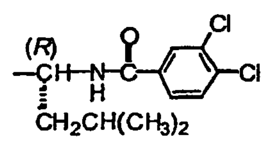 |

(continued)

| Compd. No. | 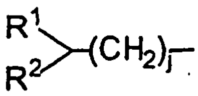 | k | m | n | chirality | $R^3$ | 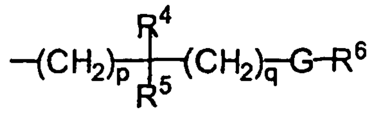 |
|---|---|---|---|---|---|---|---|
| 434 | 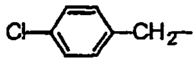 | 1 | 3 | 1 | - | H | 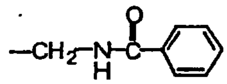 |
| 435 | 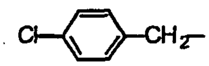 | 1 | 3 | 1 | - | H | 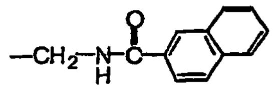 |
| 436 | 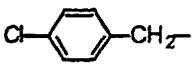 | 1 | 3 | 1 | - | H | 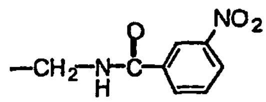 |
| 437 | 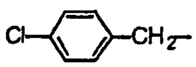 | 1 | 3 | 1 | - | H | 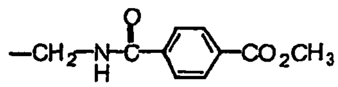 |
| 438 | 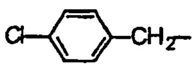 | 1 | 3 | 1 | - | H | 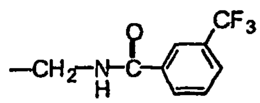 |
| 439 | 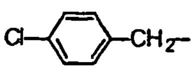 | 1 | 3 | 1 | - | H | 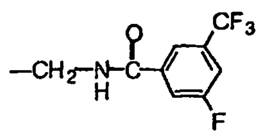 |
| 440 | 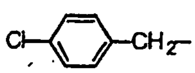 | 1 | 3 | 1 | - | H | 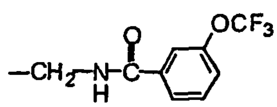 |

**Table 1.41**

| Compd. No. | 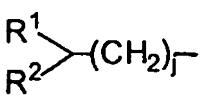 | k | m | n | chirality | $R^3$ | 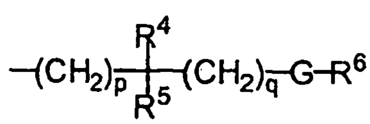 |
|---|---|---|---|---|---|---|---|
| 441 | 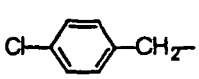 | 1 | 3 | 1 | - | H | 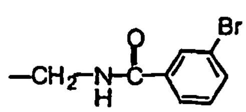 |
| 442 | 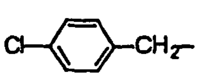 | 1 | 3 | 1 | - | H | 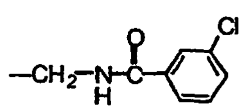 |

(continued)

| Compd. No. | 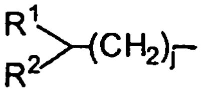 | k | m | n | chirality | $R^3$ | 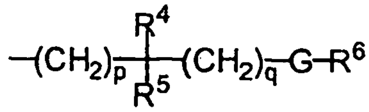 |
|---|---|---|---|---|---|---|---|
| 443 | 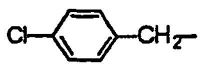 | 1 | 3 | 1 | - | H | 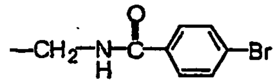 |
| 444 | 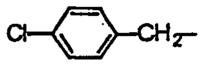 | 1 | 3 | 1 | - | H | 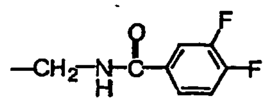 |
| 445 | 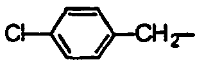 | 1 | 3 | 1 | - | H | 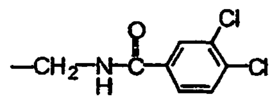 |
| 446 | 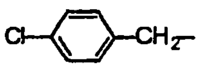 | 1 | 3 | 1 | - | H | 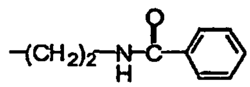 |
| 447 | 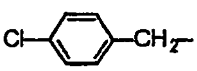 | 1 | 3 | 1 | - | H | 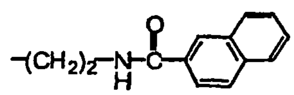 |
| 448 | 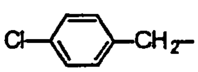 | 1 | 3 | 1 | - | H | 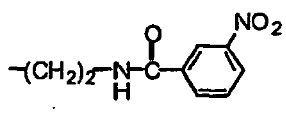 |
| 449 | 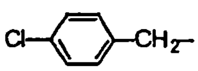 | 1 | 3 | 1 | - | H | 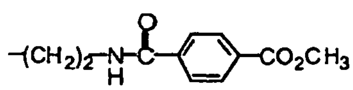 |
| 450 | 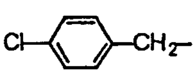 | 1 | 3 | 1 | - | H | 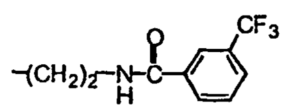 |
| 451 | 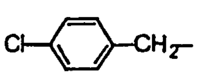 | 1 | 3 | 1 | - | H | 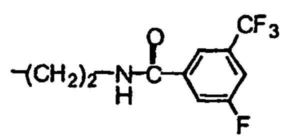 |

**Table 1.42**

| Compd. No. | 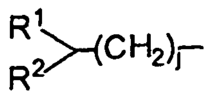 | k | m | n | chirality | $R^3$ | 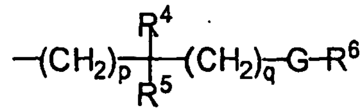 |
|---|---|---|---|---|---|---|---|
| 452 | 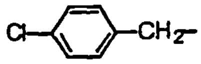 | 1 | 3 | 1 | - | H | 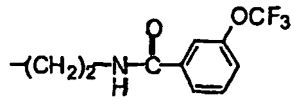 |
| 453 | 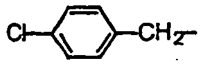 | 1 | 3 | 1 | - | H | 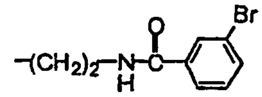 |
| 454 | 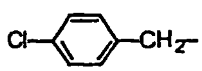 | 1 | 3 | 1 | - | H | 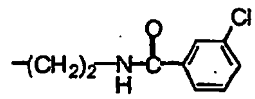 |
| 455 | 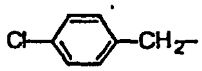 | 1 | 3 | 1 | - | H | 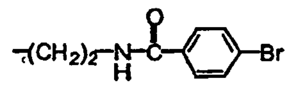 |
| 456 | 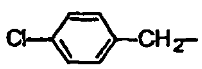 | 1 | 3 | 1 | - | H | 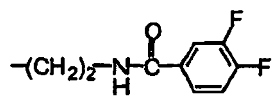 |
| 457 | 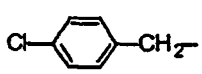 | 1 | 3 | 1 | - | H | 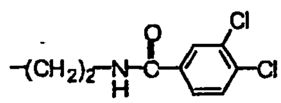 |
| 458 | 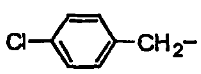 | 2 | 2 | 1 | - | H | 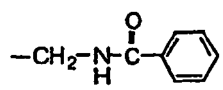 |
| 459 | 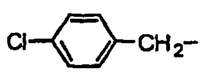 | 2 | 2 | 1 | - | H | 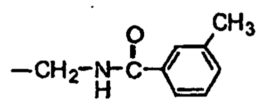 |
| 460 | 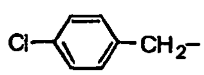 | 2 | 2 | 1 | - | H | 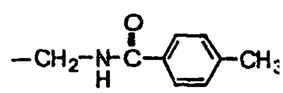 |
| 461 | 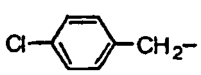 | 2 | 2 | 1 | - | H | 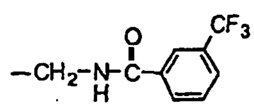 |
| 462 | 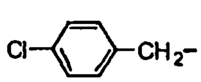 | 2 | 2 | 1 | - | H | 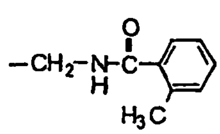 |

Table 1.43

| Compd. No. | 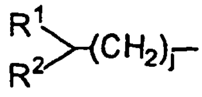 | k | m | n | chirality | $R^3$ | 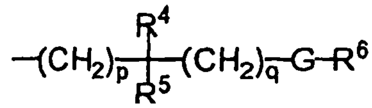 |
|---|---|---|---|---|---|---|---|
| 463 | 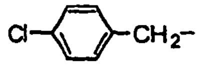 | 2 | 2 | 1 | - | H | 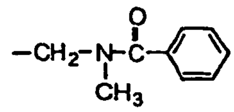 |
| 464 | 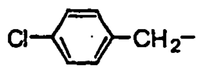 | 2 | 2 | 1 | - | H | 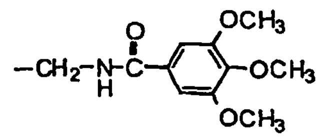 |
| 465 | 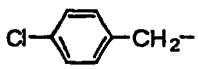 | 2 | 2 | 1 | - | H | 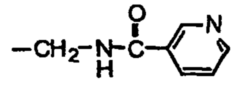 |
| 466 | 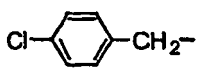 | 2 | 2 | 1 | - | H | 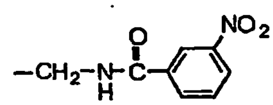 |
| 467 | 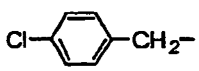 | 2 | 2 | 1 | - | H | 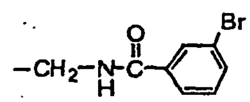 |
| 468 | 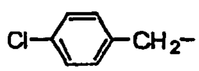 | 2 | 2 | 1 | - | H | 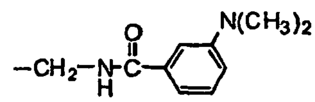 |
| 469 | 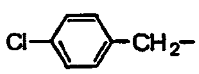 | 2 | 2 | 1 | - | H | 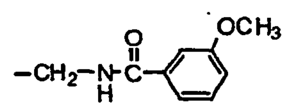 |
| 470 | 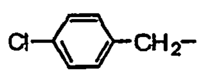 | 2 | 2 | 1 | - | H | 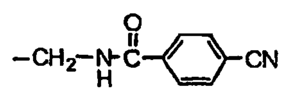 |
| 471 | 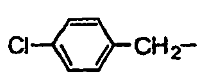 | 2 | 2 | 1 | - | H | 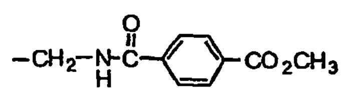 |
| 472 | 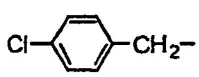 | 2 | 2 | 1 | - | H | 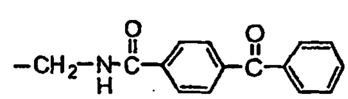 |
| 473 | 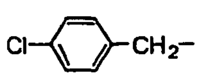 | 2 | 2 | 1 | - | H | 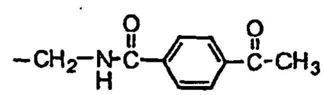 |

**Table 1.44**

| Compd. No. | 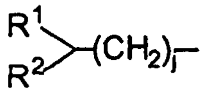 | k | m | n | chirality | $R^3$ | 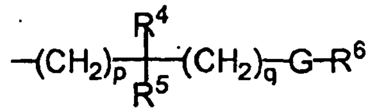 |
|---|---|---|---|---|---|---|---|
| 474 | 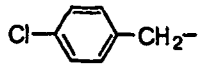 | 2 | 2 | 1 | - | H | 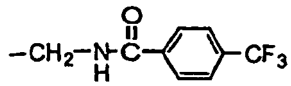 |
| 475 | 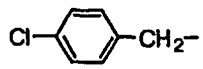 | 2 | 2 | 1 | - | H | 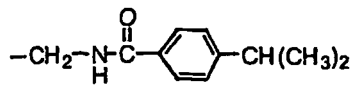 |
| 476 | 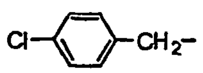 | 2 | 2 | 1 | - | H | 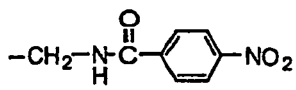 |
| 477 | 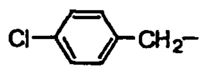 | 2 | 2 | 1 | - | H | 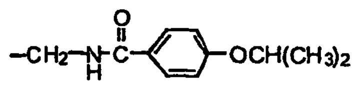 |
| 478 | 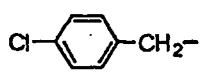 | 2 | 2 | 1 | - | H | 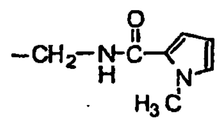 |
| 479 | 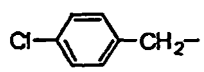 | 2 | 2 | 1 | - | H | 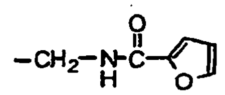 |
| 480 | 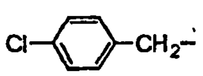 | 2 | 2 | 1 | - | H | 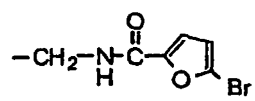 |
| 481 | 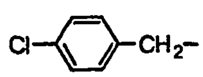 | 2 | 2 | 1 | - | H | 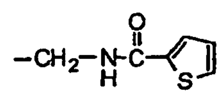 |
| 482 | 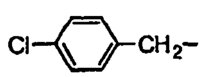 | 2 | 2 | 1 | - | H | 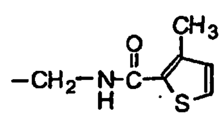 |
| 483 | 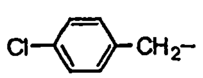 | 2 | 2 | 1 | - | H | 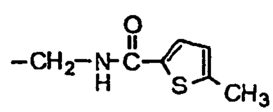 |
| 484 | 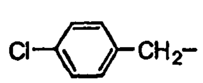 | 2 | 2 | 1 | - | H | 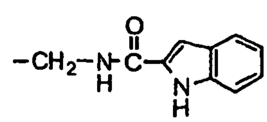 |

**Table 1.45**

| Compd No. | 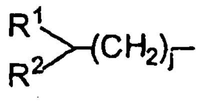 | k | m | n | chirality | $R^3$ | 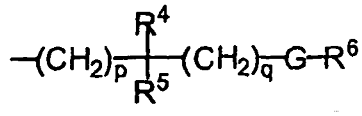 |
|---|---|---|---|---|---|---|---|
| 485 | 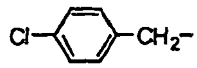 | 2 | 2 | 1 | - | H | 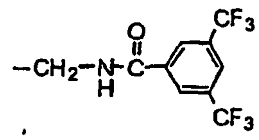 |
| 486 | 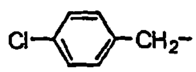 | 2 | 2 | 1 | - | H | 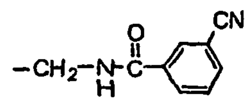 |
| 487 | 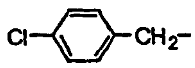 | 2 | 2 | 1 | - | H | 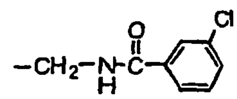 |
| 488 | 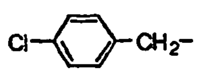 | 2 | 2 | 1 | - | H | 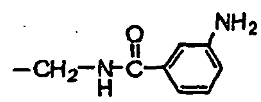 |
| 489 | 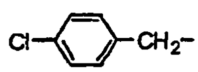 | 2 | 2 | 1 | - | H | 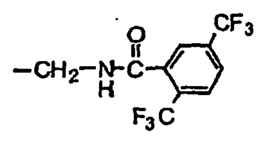 |
| 490 | 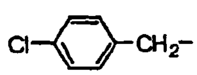 | 2 | 2 | 1 | - | H | 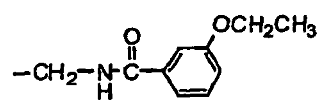 |
| 491 | 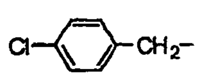 | 2 | 2 | 1 | - | H | 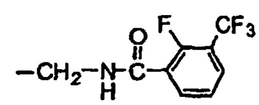 |
| 492 | 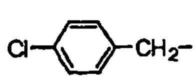 | 2 | 2 | 1 | - | H | 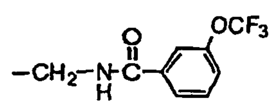 |
| 493 | 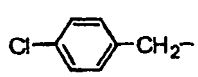 | 2 | 2 | 1 | - | H | 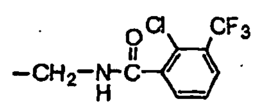 |
| 494 | 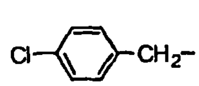 | 2 | 2 | 1 | - | H | 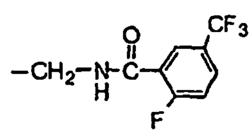 |
| 495 | 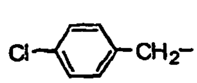 | 2 | 2 | 1 | - | H | 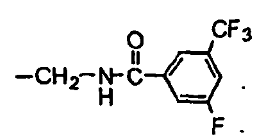 |

**Table 1.46**

| Compd. No. | 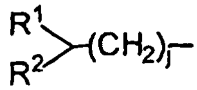 | k | m | n | chirality | $R^3$ | 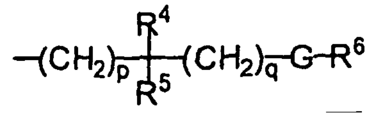 |
|---|---|---|---|---|---|---|---|
| 496 | 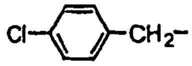 | 2 | 2 | 1 | - | H | 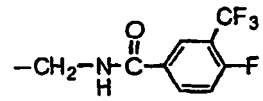 |
| 497 | 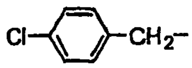 | 2 | 2 | 1 | - | H | 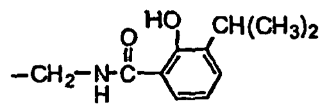 |
| 498 | 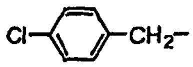 | 2 | 2 | 1 | - | H | 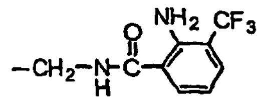 |
| 499 | 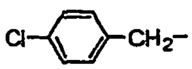 | 2 | 2 | 1 | - | H | 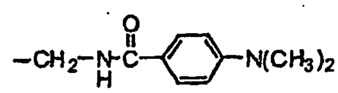 |
| 500 | 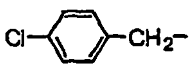 | 2 | 2 | 1 | - | H | 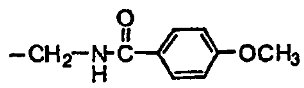 |
| 501 | 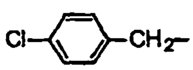 | 2 | 2 | 1 | - | H | 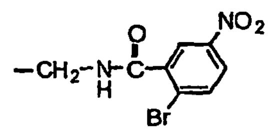 |
| 502 | 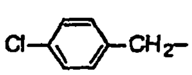 | 2 | 2 | 1 | - | H | 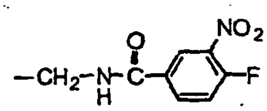 |
| 503 | 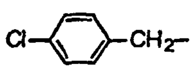 | 2 | 2 | 1 | - | H | 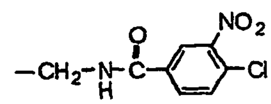 |
| 504 | 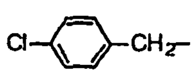 | 2 | 2 | 1 | - | H | 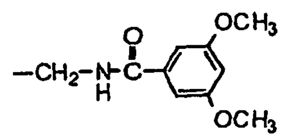 |
| 505 | 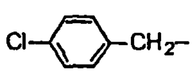 | 2 | 2 | 1 | - | H | 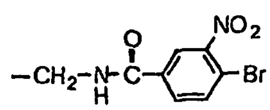 |
| 506 | 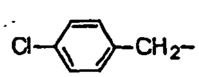 | 2 | 2 | 1 | - | H | 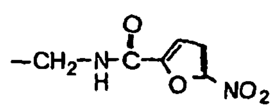 |

**Table 1.47**

| Compd. No. | 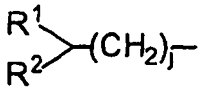 | k | m | n | chirality | $R^3$ | 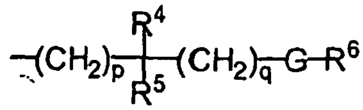 |
|---|---|---|---|---|---|---|---|
| 507 | 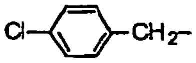 | 2 | 2 | 1 | - | H | 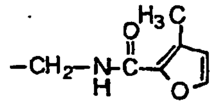 |
| 508 | 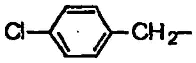 | 2 | 2 | 1 | - | H | 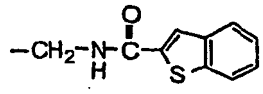 |
| 509 | 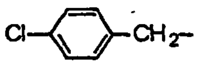 | 2 | 2 | 1 | - | H | 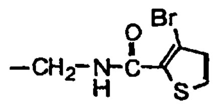 |
| 510 | 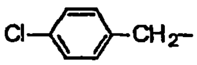 | 2 | 2 | 1 | - | H | 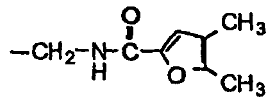 |
| 511 | 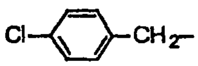 | 2 | 2 | 1 | - | H | 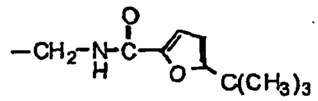 |
| 512 | 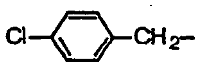 | 2 | 2 | 1 | - | H | 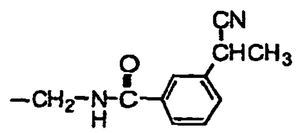 |
| 513 | 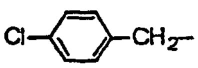 | 2 | 2 | 1 | - | H | 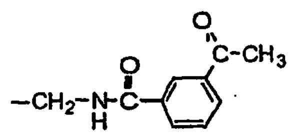 |
| 514 | 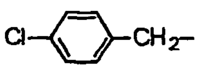 | 2 | 2 | 1 | - | H | 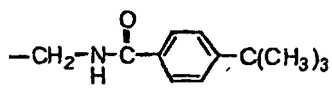 |
| 515 | 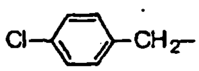 | 2 | 2 | 1 | - | H | 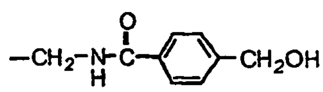 |
| 516 | 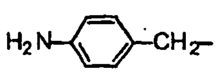 | 2 | 2 | 1 | - | H | 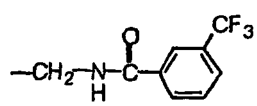 |
| 517 | 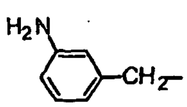 | 2 | 2 | 1 | - | H | 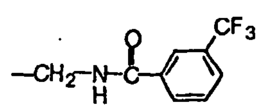 |

**Table 1.48**

| Compd. No. | 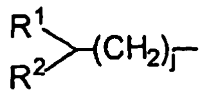 | k | m | n | chirality | $R^3$ | 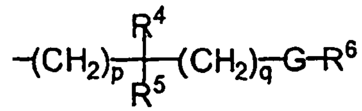 |
|---|---|---|---|---|---|---|---|
| 518 | 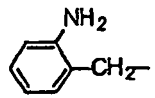 | 2 | 2 | 1 | - | H | 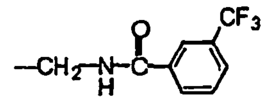 |
| 519 | 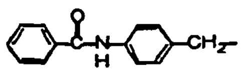 | 2 | 2 | 1 | - | H | 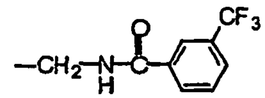 |
| 520 | 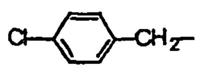 | 2 | 2 | 1 | - | $-CH_3$ | 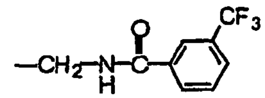 |
| 521 | 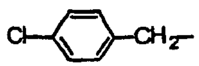 | 2 | 2 | 1 | - | 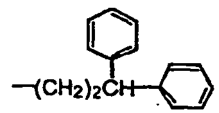 | 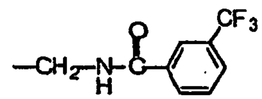 |
| 522 | 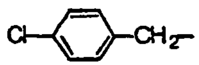 | 2 | 2 | 1 | - | 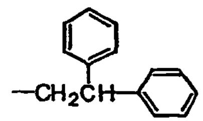 | 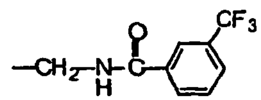 |
| 523 | 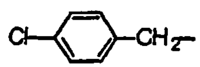 | 2 | 2 | 1 | - | 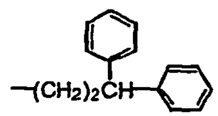 | 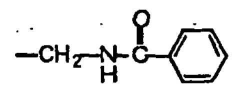 |
| 524 | 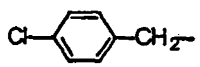 | 2 | 2 | 1 | - | 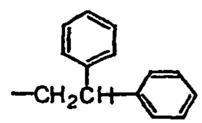 | 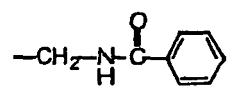 |

(continued)

| Compd. No. | 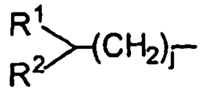 | k | m | n | chirality | $R^3$ | 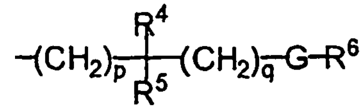 |
|---|---|---|---|---|---|---|---|
| 525 | 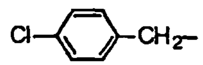 | 2 | 2 | 1 | - | H | 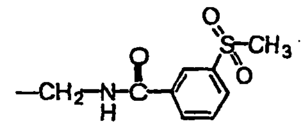 |
| 526 | 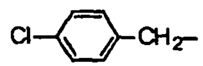 | 2 | 2 | 1 | - | H | 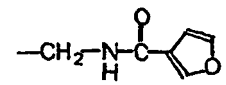 |
| 527 | 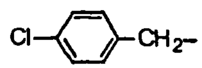 | 2 | 2 | 1 | - | H | 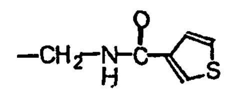 |
| 528 | 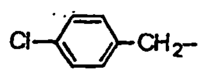 | 2 | 2 | 1 | - | H | 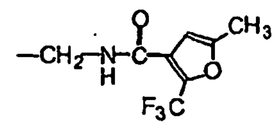 |

**Table 1.49**

| Compd. No. | 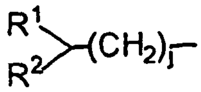 | k | m | n | chirality | $R^3$ | 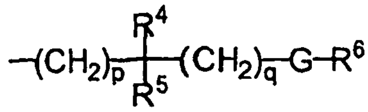 |
|---|---|---|---|---|---|---|---|
| 529 | 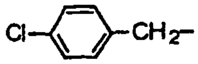 | 2 | 2 | 1 | - | H | 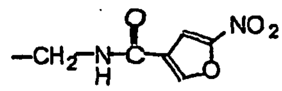 |
| 530 | 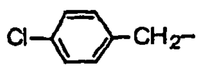 | 2 | 2 | 1 | - | H | 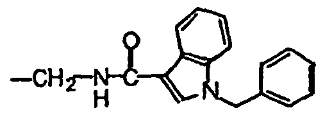 |
| 531 | 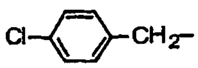 | 2 | 2 | 1 | - | H | 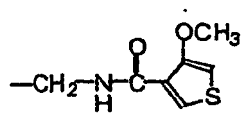 |
| 532 | 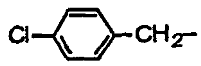 | 2 | 2 | 1 | - | H | 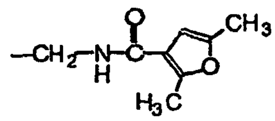 |
| 533 | 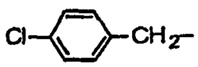 | 2 | 2 | 1 | - | H | 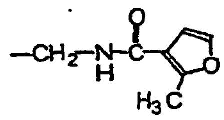 |
| 534 | 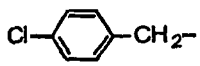 | 2 | 2 | 1 | - | H | 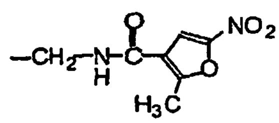 |
| 535 | 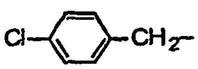 | 2 | 2 | 1 | - | H | 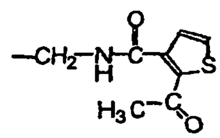 |
| 536 | 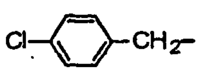 | 2 | 2 | 1 | - | H | 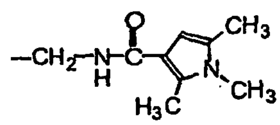 |
| 537 | 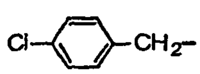 | 2 | 2 | 1 | - | H | 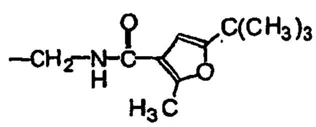 |
| 538 | 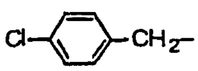 | 2 | 2 | 1 | - | H | 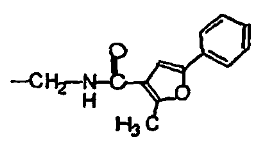 |

(continued)

| Compd. No. | 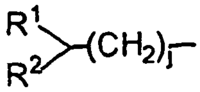 | k | m | n | chirality | $R^3$ | 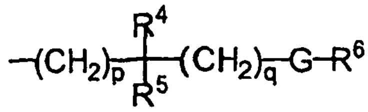 |
|---|---|---|---|---|---|---|---|
| 539 | 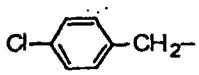 | 2 | 2 | 1 | - | H | 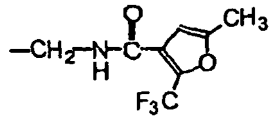 |

**Table 1.50**

| Compd. No. | 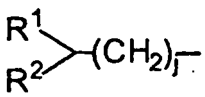 | k | m | n | chirality | $R^3$ | 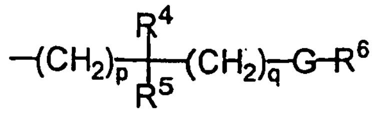 |
|---|---|---|---|---|---|---|---|
| 540 | 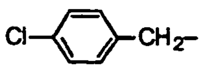 | 2 | 2 | 1 | - | H | 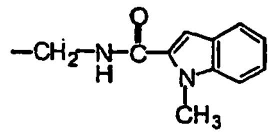 |
| 541 | 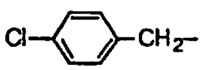 | 2 | 2 | 1 | - | H | 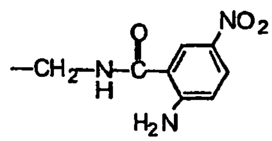 |
| 542 | 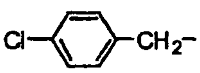 | 2 | 2 | 1 | - | H | 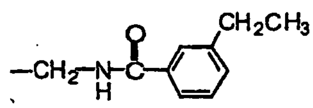 |
| 543 | 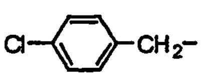 | 2 | 2 | 1 | - | H | 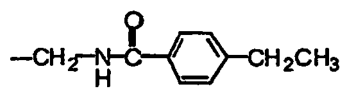 |
| 544 | 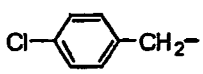 | 2 | 2 | 1 | - | H | 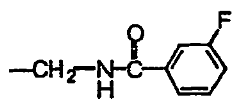 |
| 545 | 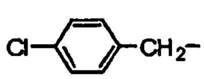 | 2 | 2 | 1 | - | H | 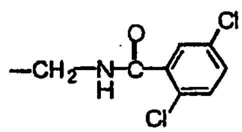 |
| 546 | 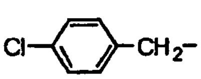 | 2 | 2 | 1 | - | H | 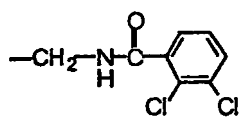 |
| 547 | 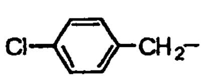 | 2 | 2 | 1 | - | H | 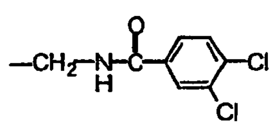 |

(continued)

| Compd. No. | 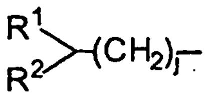 | k | m | n | chirality | $R^3$ | 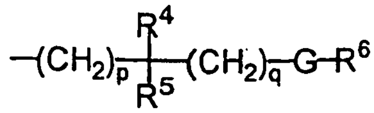 |
|---|---|---|---|---|---|---|---|
| 548 | 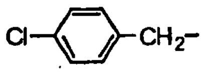 | 2 | 2 | 1 | - | H | 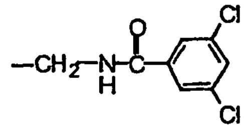 |
| 549 | 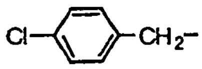 | 2 | 2 | 1 | - | H | 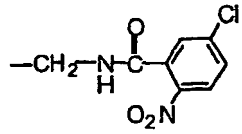 |
| 550 | 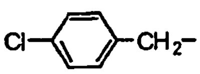 | 2 | 2 | 1 | - | H | 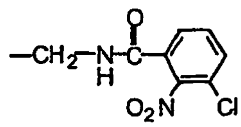 |

**Table 1.51**

| Compd. No. | 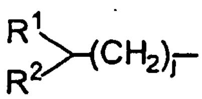 | k | m | n | chirality | $R^3$ | 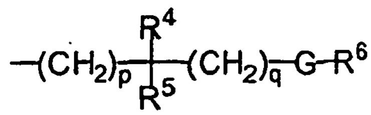 |
|---|---|---|---|---|---|---|---|
| 551 | 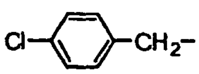 | 2 | 2 | 1 | - | H | 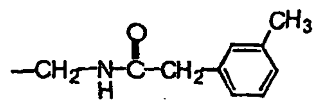 |
| 552 | 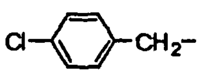 | 2 | 2 | 1 | - | H | 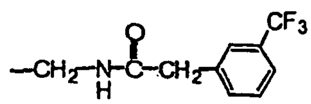 |
| 553 | 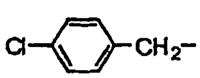 | 2 | 2 | 1 | - | H | 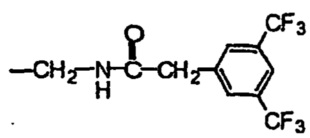 |
| 554 | 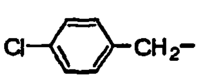 | 2 | 2 | 1 | - | H | 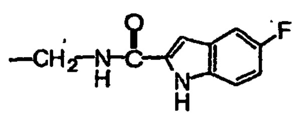 |
| 555 | 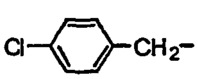 | 2 | 2 | 1 | - | H | 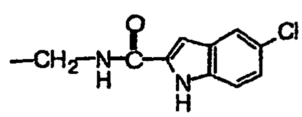 |
| 556 | 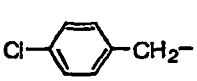 | 2 | 2 | 1 | - | H | 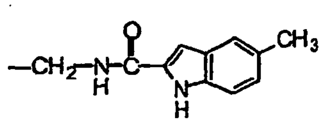 |

(continued)

| Compd. No. | 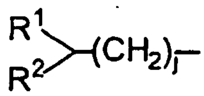 | k | m | n | chirality | $R^3$ | 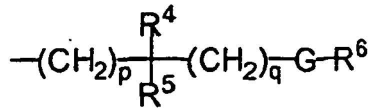 |
|---|---|---|---|---|---|---|---|
| 557 | 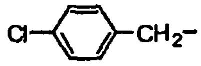 | 2 | 2 | 1 | - | H | 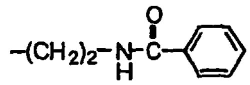 |
| 558 | 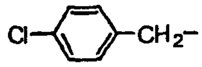 | 2 | 2 | 1 | - | H | 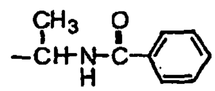 |
| 559 | 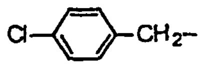 | 2 | 2 | 1 | - | H | 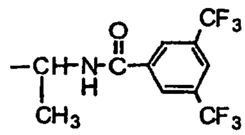 |
| 560 | 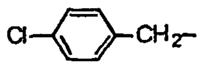 | 2 | 2 | 1 | - | H | 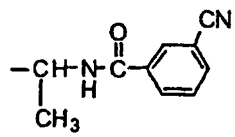 |
| 561 | 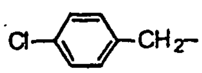 | 2 | 2 | 1 | - | H | 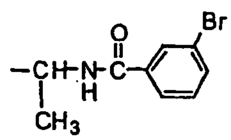 |

**Table 1.52**

| Compd. No. | 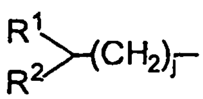 | k | m | n | chirality | $R^3$ | 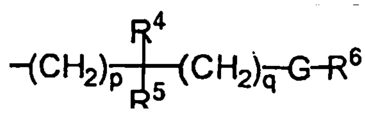 |
|---|---|---|---|---|---|---|---|
| 562 | 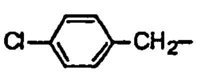 | 2 | 2 | 1 | - | H | 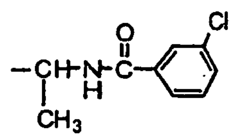 |
| 563 | 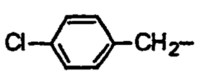 | 2 | 2 | 1 | - | H | 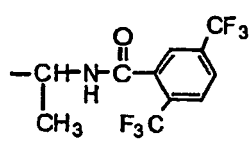 |
| 564 | 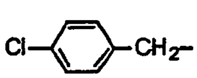 | 2 | 2 | 1 | - | H | 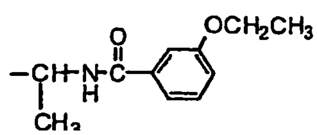 |
| 565 | 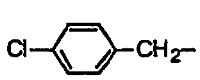 | 2 | 2 | 1 | - | H | 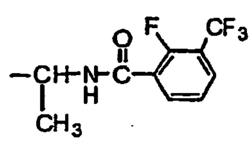 |

(continued)

| Compd. No. | 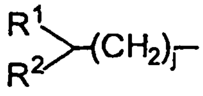 | k | m | n | chirality | $R^3$ | 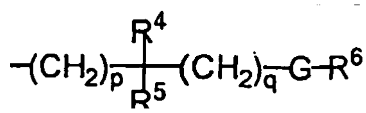 |
|---|---|---|---|---|---|---|---|
| 566 | 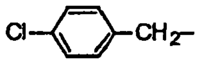 | 2 | 2 | 1 | - | H | 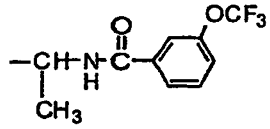 |
| 567 | 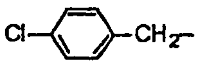 | 2 | 2 | 1 | - | H | 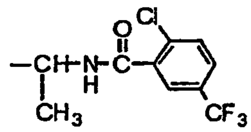 |
| 568 | 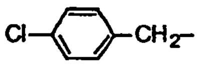 | 2 | 2 | 1 | - | H | 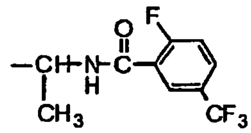 |
| 569 | 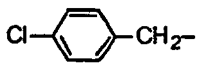 | 2 | 2 | 1 | - | H | 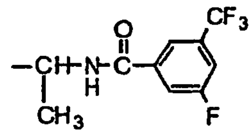 |
| 570 | 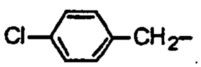 | 2 | 2 | 1 | - | H | 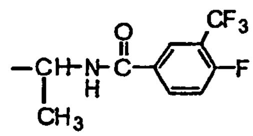 |
| 571 | 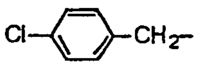 | 2 | 2 | 1 | - | H | 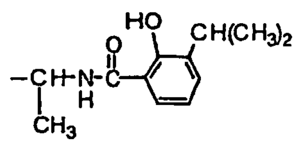 |
| 572 | 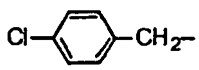 | 2 | 2 | 1 | - | H | 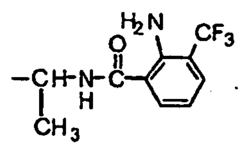 |

**Table 1.53**

| Compd. No. | 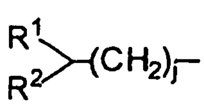 | k | m | n | chirality | $R^3$ | 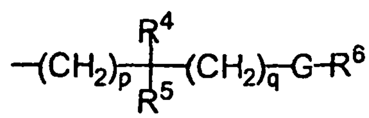 |
|---|---|---|---|---|---|---|---|
| 573 | 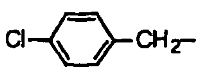 | 2 | 2 | 1 | - | H | 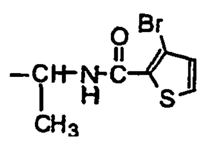 |
| 574 | 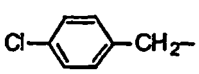 | 2 | 2 | 1 | - | H | 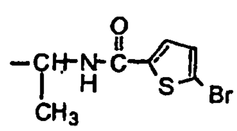 |

(continued)

| Compd. No. | 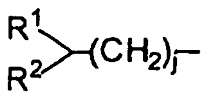 | k | m | n | chirality | $R^3$ | 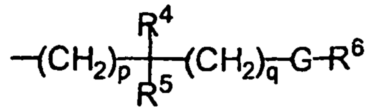 |
|---|---|---|---|---|---|---|---|
| 575 | 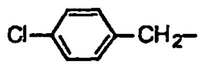 | 2 | 2 | 1 | - | H | 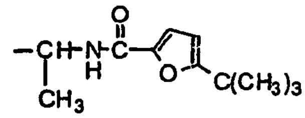 |
| 576 | 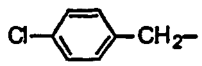 | 2 | 2 | 1 | - | H | 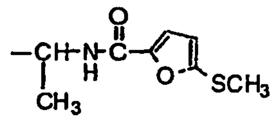 |
| 577 | 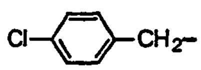 | 2 | 2 | 1 | - | H | 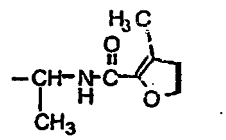 |
| 578 | 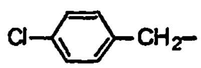 | 2 | 2 | 1 | - | H | 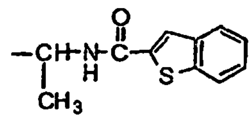 |
| 579 | 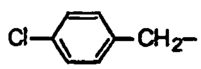 | 2 | 2 | 1 | - | H | 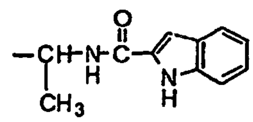 |
| 580 | 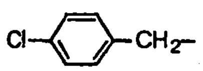 | 2 | 2 | 1 | - | H | 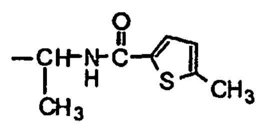 |
| 581 | 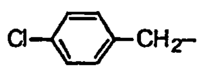 | 2 | 2 | 1 | - | H | 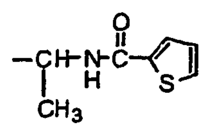 |
| 582 | 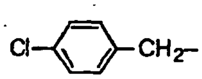 | 2 | 2 | 1 | - | H | 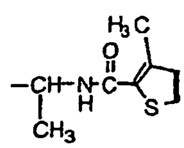 |
| 583 | 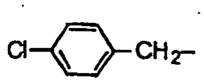 | 2 | 2 | 1 | - | H | 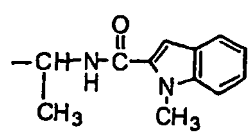 |

Table 1.54

| Compd. No. | 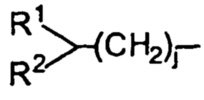 | k | m | n | chirality | $R^3$ | 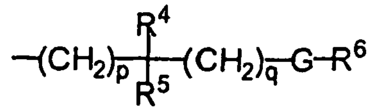 |
|---|---|---|---|---|---|---|---|
| 584 | 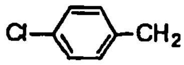 | 2 | 2 | 1 | - | H | 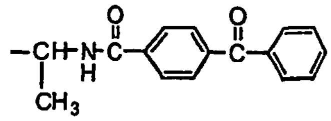 |
| 585 | 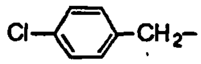 | 2 | 2 | 1 | - | H | 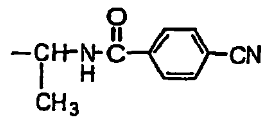 |
| 586 | 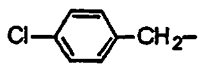 | 2 | 2 | 1 | - | H | 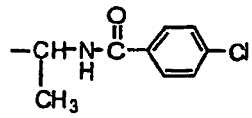 |
| 587 | 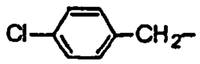 | 2 | 2 | 1 | - | H | 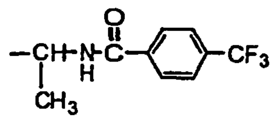 |
| 588 | 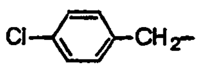 | 2 | 2 | 1 | - | H | 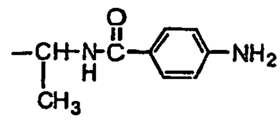 |
| 589 | 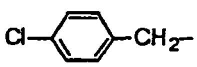 | 2 | 2 | 1 | - | H | 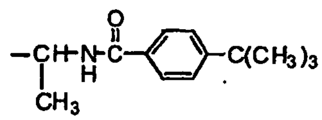 |
| 590 | 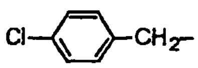 | 2 | 2 | 1 | - | H | 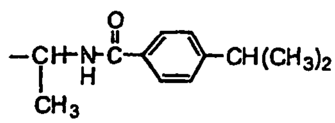 |
| 591 | 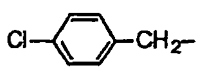 | 2 | 2 | 1 | - | H | 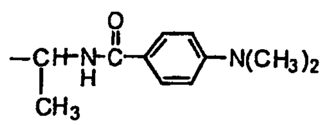 |
| 592 | 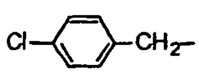 | 2 | 2 | 1 | - | H | 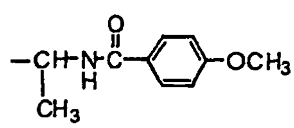 |
| 593 | 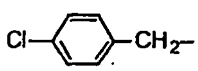 | 2 | 2 | 1 | - | H | 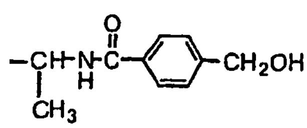 |
| 594 | 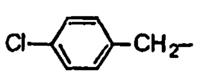 | 2 | 2 | 1 | - | H | 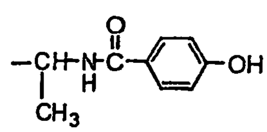 |

**Table 1.55**

| Compd. No. | 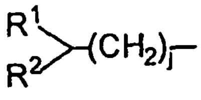 | k | m | n | chirality | $R^3$ | 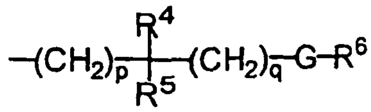 |
|---|---|---|---|---|---|---|---|
| 595 | 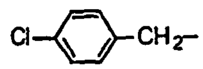 | 2 | 2 | 1 | - | H | 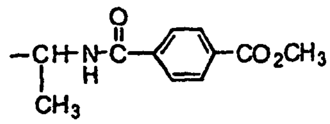 |
| 596 | 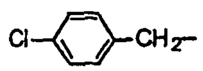 | 2 | 2 | 1 | - | H | 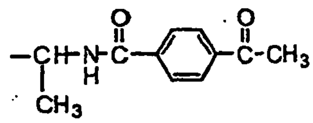 |
| 597 | 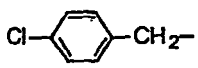 | 2 | 2 | 1 | - | H | 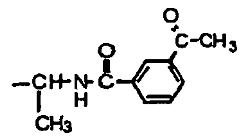 |
| 598 | 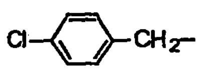 | 2 | 2 | 1 | - | H | 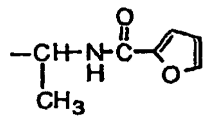 |
| 599 | 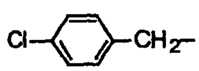 | 2 | 2 | 1 | - | H | 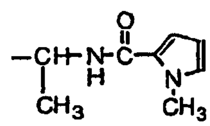 |
| 600 | 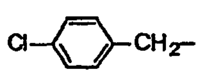 | 2 | 2 | 1 | - | H | 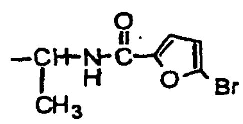 |
| 601 | 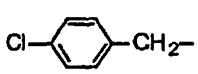 | 2 | 2 | 1 | - | H | 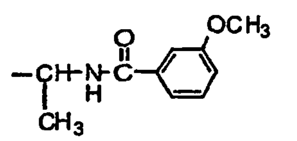 |
| 602 | 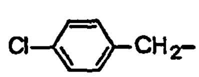 | 2 | 2 | 1 | - | H | 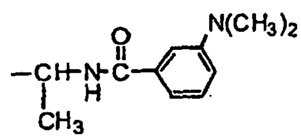 |
| 603 | 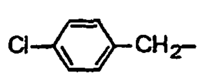 | 2 | 2 | 1 | - | H | 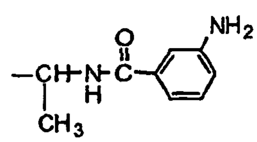 |
| 604 | 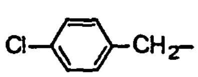 | 2 | 2 | 1 | - | H | 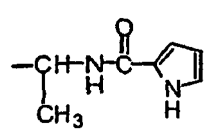 |

(continued)

| Compd. No. | 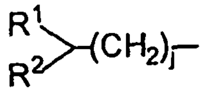 | k | m | n | chirality | $R^3$ | 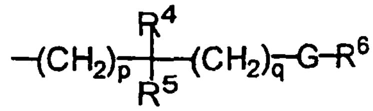 |
|---|---|---|---|---|---|---|---|
| 605 | 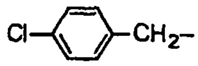 | 2 | 2 | 1 | - | H | 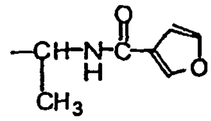 |

**Table 1.56**

| Compd. No. | 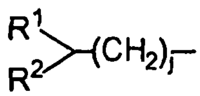 | k | m | n | chirality | $R^3$ | 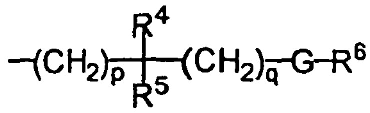 |
|---|---|---|---|---|---|---|---|
| 606 | 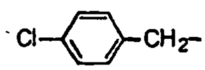 | 2 | 2 | 1 | - | H | 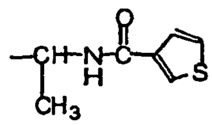 |
| 607 | 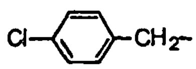 | 2 | 2 | 1 | - | H | 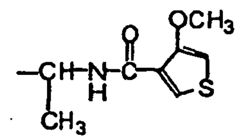 |
| 608 | 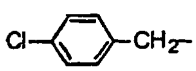 | 2 | 2 | 1 | - | H | 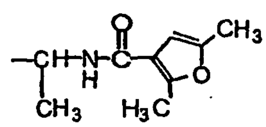 |
| 609 | 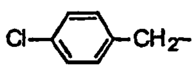 | 2 | 2 | 1 | - | H | 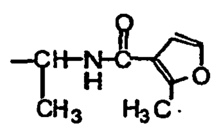 |
| 610 | 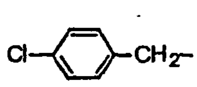 | 2 | 2 | 1 | - | H | 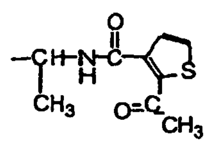 |
| 611 | 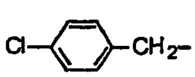 | 2 | 2 | 1 | - | H | 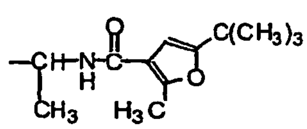 |
| 612 | 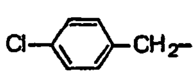 | 2 | 2 | 1 | - | H | 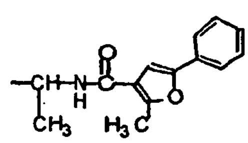 |

(continued)

| Compd. No. | 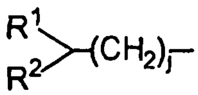 | k | m | n | chirality | $R^3$ | 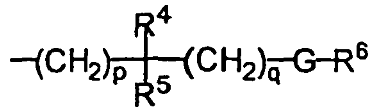 |
|---|---|---|---|---|---|---|---|
| 613 | 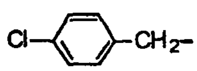 | 2 | 2 | 1 | - | H | 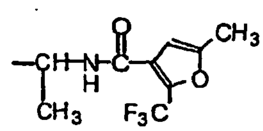 |
| 614 | 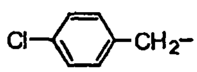 | 2 | 2 | 1 | - | H | 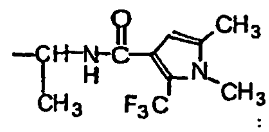 |
| 615 | 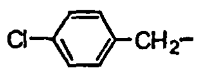 | 2 | 2 | 1 | - | H | 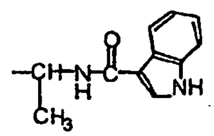 |
| 616 | 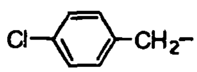 | 2 | 2 | 1 | - | H | 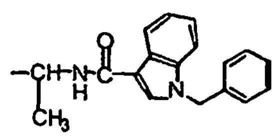 |

**Table 1.57**

| Compd. No. | 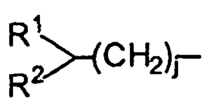 | k | m | n | chirality | $R^3$ | 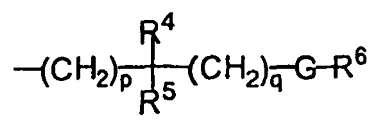 |
|---|---|---|---|---|---|---|---|
| 617 | 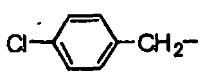 | 2 | 2 | 1 | - | H | 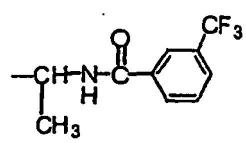 |
| 618 | 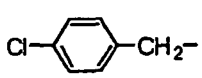 | 2 | 2 | 1 | - | H | 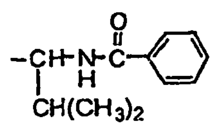 |
| 619 | 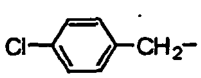 | 2 | 2 | 1 | - | H | 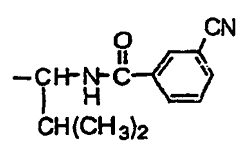 |
| 620 | 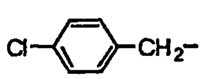 | 2 | 2 | 1 | - | H | 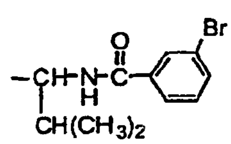 |

(continued)

| Compd. No. | 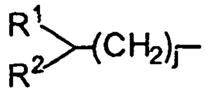 | k | m | n | chirality | $R^3$ | 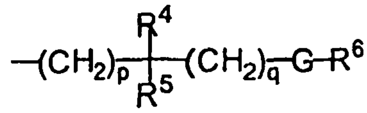 |
|---|---|---|---|---|---|---|---|
| 621 | 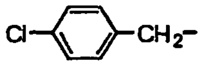 | 2 | 2 | 1 | - | H | 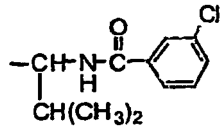 |
| 622 | 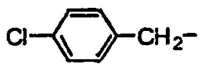 | 2 | 2 | 1 | - | H | 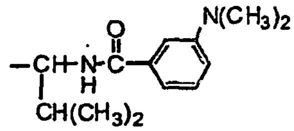 |
| 623 | 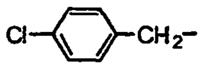 | 2 | 2 | 1 | - | H | 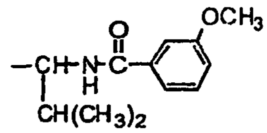 |
| 624 | 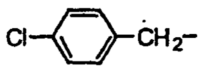 | 2 | 2 | 1 | - | H | 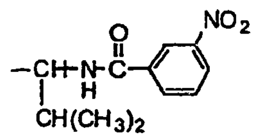 |
| 625 | 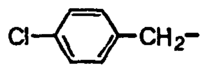 | 2 | 2 | 1 | - | H | 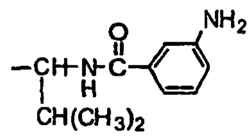 |
| 626 | 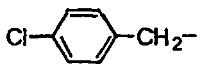 | 2 | 2 | 1 | - | H | 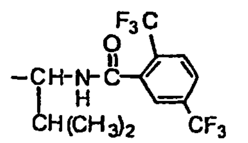 |
| 627 | 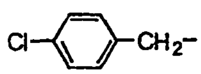 | 2 | 2 | 1 | - | H | 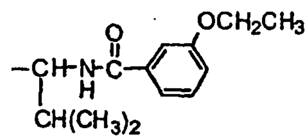 |

**Table 1.58**

| Compd. No. | 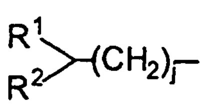 | k | m | n | chirality | $R^3$ | 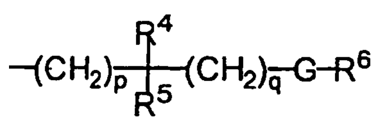 |
|---|---|---|---|---|---|---|---|
| 628 | 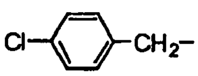 | 2 | 2 | 1 | - | H | 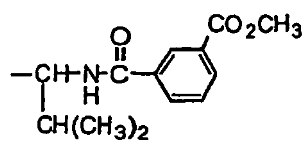 |

(continued)

| Compd. No. | 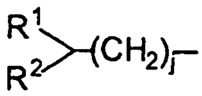 | k | m | n | chirality | $R^3$ | 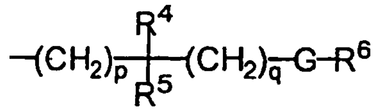 |
|---|---|---|---|---|---|---|---|
| 629 | 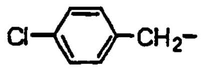 | 2 | 2 | 1 | - | H | 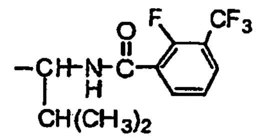 |
| 630 | 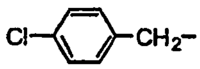 | 2 | 2 | 1 | - | H | 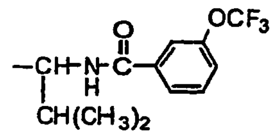 |
| 631 | 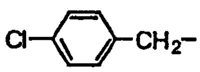 | 2 | 2 | 1 | - | H | 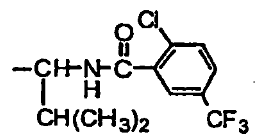 |
| 632 | 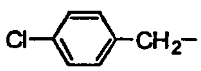 | 2 | 2 | 1 | - | H | 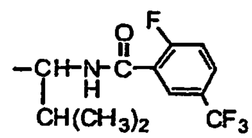 |
| 633 | 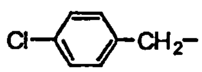 | 2 | 2 | 1 | - | H | 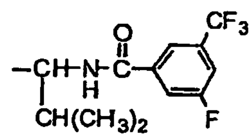 |
| 634 | 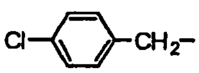 | 2 | 2 | 1 | - | H | 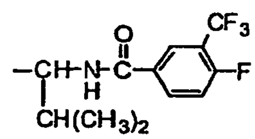 |
| 635 | 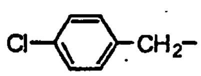 | 2 | 2 | 1 | - | H | 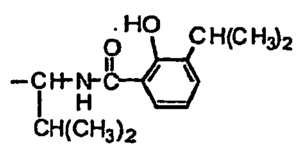 |
| 636 | 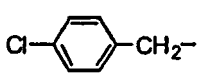 | 2 | 2 | 1 | - | H | 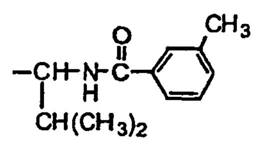 |
| 637 | 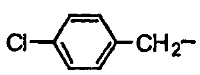 | 2 | 2 | 1 | - | H | 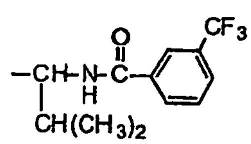 |
| 638 | 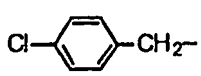 | 2 | 2 | 1 | - | H | 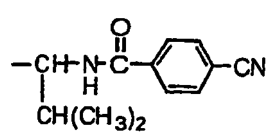 |

**Table 1.59**

| Compd. No. | 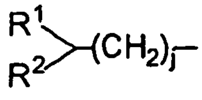 | k | m | n | chirality | $R^3$ | 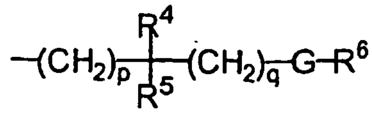 |
|---|---|---|---|---|---|---|---|
| 639 | 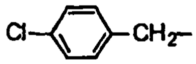 | 2 | 2 | 1 | - | H | 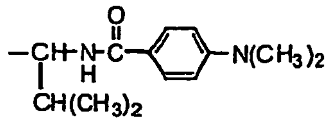 |
| 640 | 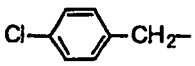 | 2 | 2 | 1 | - | H | 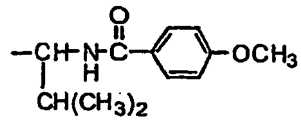 |
| 641 | 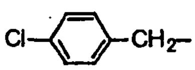 | 2 | 2 | 1 | - | H | 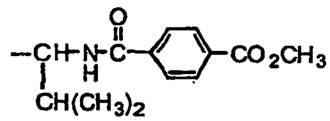 |
| 642 | 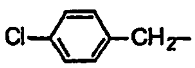 | 2 | 2 | 1 | - | H | 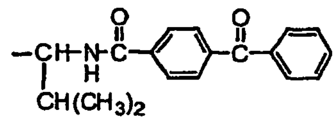 |
| 643 | 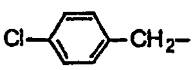 | 2 | 2 | 1 | - | H | 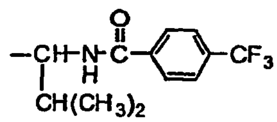 |
| 644 | 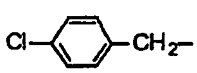 | 2 | 2 | 1 | - | H | 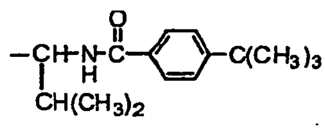 |
| 645 | 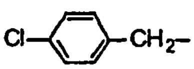 | 2 | 2 | 1 | - | H | 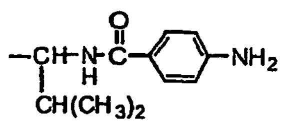 |
| 646 | 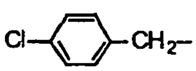 | 2 | 2 | 1 | - | H | 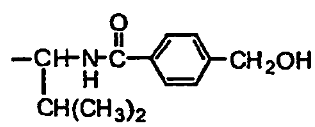 |
| 647 | 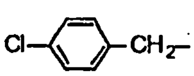 | 2 | 2 | 1 | - | H | 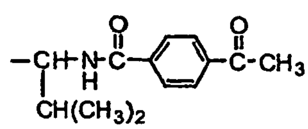 |
| 648 | 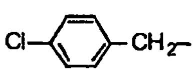 | 2 | 2 | 1 | - | H | 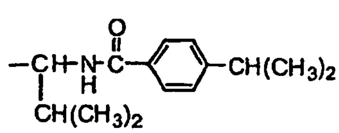 |

(continued)

| Compd. No. | 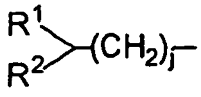 | k | m | n | chirality | $R^3$ | 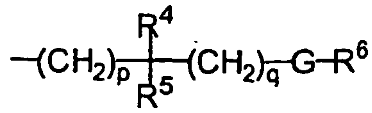 |
|---|---|---|---|---|---|---|---|
| 649 | 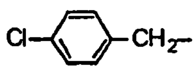 | 2 | 2 | 1 | - | H | 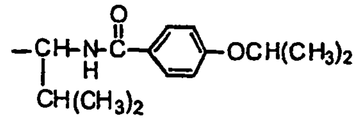 |

**Table 1.60**

| Compd. No. | 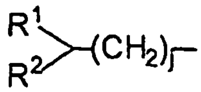 | k | m | n | chirality | $R^3$ | 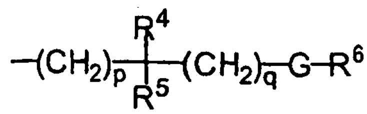 |
|---|---|---|---|---|---|---|---|
| 650 | 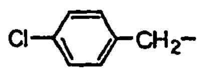 | 2 | 2 | 1 | - | H | 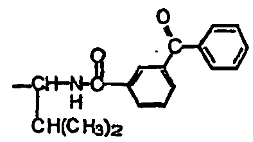 |
| 651 | 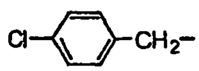 | 2 | 2 | 1 | - | H | 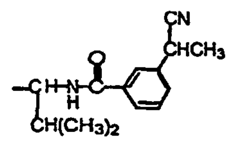 |
| 652 | 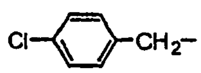 | 2 | 2 | 1 | - | H | 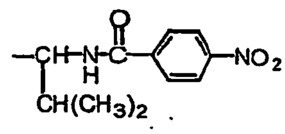 |
| 653 | 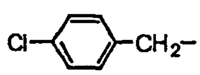 | 2 | 2 | 1 | - | H | 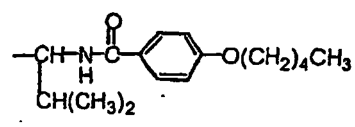 |
| 654 | 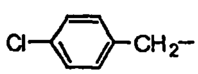 | 2 | 2 | 1 | - | H | 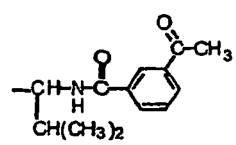 |
| 655 | 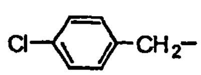 | 2 | 2 | 1 | - | H | 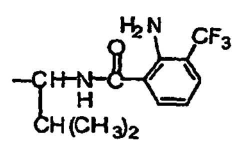 |
| 656 | 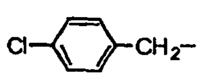 | 2 | 2 | 1 | - | H | 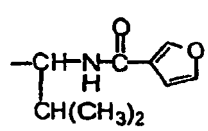 |
| 657 | 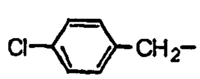 | 2 | 2 | 1 | - | H | 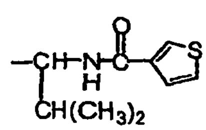 |

(continued)

| Compd. No. | 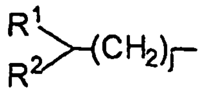 | k | m | n | chirality | $R^3$ | 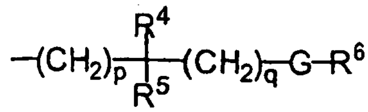 |
|---|---|---|---|---|---|---|---|
| 658 | 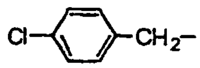 | 2 | 2 | 1 | - | H | 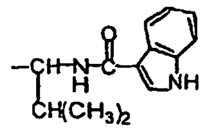 |
| 659 | 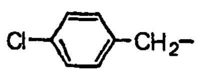 | 2 | 2 | 1 | - | H | 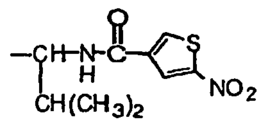 |
| 660 | 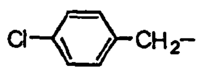 | 2 | 2 | 1 | - | H | 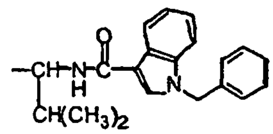 |

**Table 1.61**

| Compd. No. | 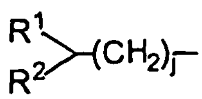 | k | m | n | chirality | $R^3$ | 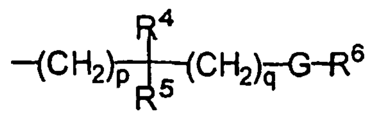 |
|---|---|---|---|---|---|---|---|
| 661 | 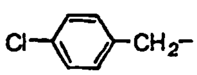 | 2 | 2 | 1 | - | H | 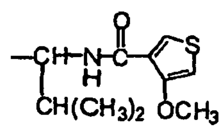 |
| 662 | 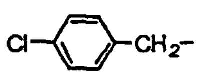 | 2 | 2 | 1 | - | H | 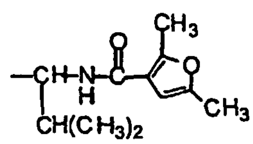 |
| 663 | 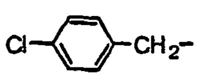 | 2 | 2 | 1 | - | H | 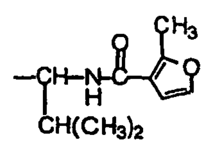 |
| 664 | 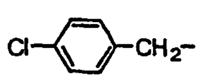 | 2 | 2 | 1 | - | H | 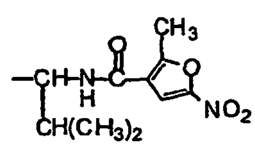 |
| 665 | 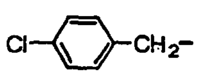 | 2 | 2 | 1 | - | H | 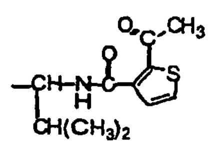 |

(continued)

| Compd. No. | 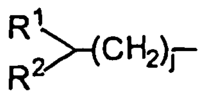 | k | m | n | chirality | $R^3$ | 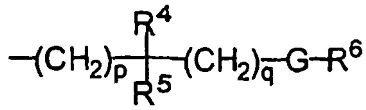 |
|---|---|---|---|---|---|---|---|
| 666 | 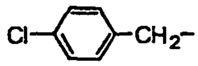 | 2 | 2 | 1 | - | H | 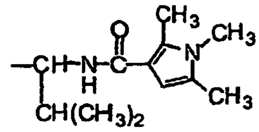 |
| 667 | 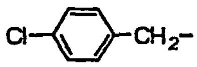 | 2 | 2 | 1 | - | H | 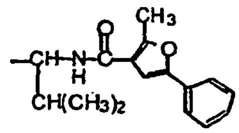 |
| 668 | 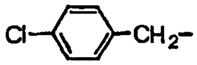 | 2 | 2 | 1 | - | H | 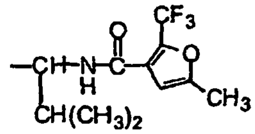 |
| 669 | 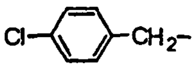 | 2 | 2 | 1 | - | H | 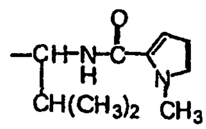 |
| 670 | 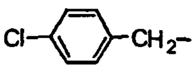 | 2 | 2 | 1 | - | H | 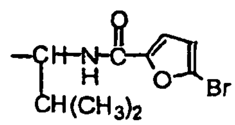 |
| 671 | 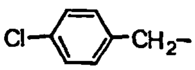 | 2 | 2 | 1 | - | H | 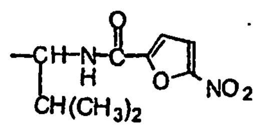 |

**Table 1.62**

| Compd. No. | 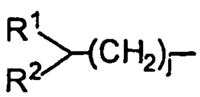 | k | m | n | chirality | $R^3$ | 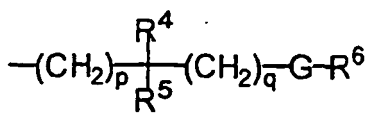 |
|---|---|---|---|---|---|---|---|
| 672 | 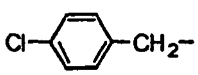 | 2 | 2 | 1 | - | H | 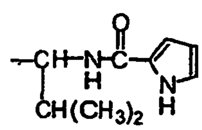 |
| 673 | 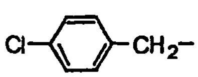 | 2 | 2 | 1 | - | H | 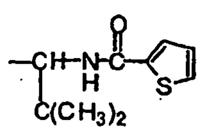 |

(continued)

| Compd. No. | 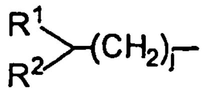 | k | m | n | chirality | $R^3$ | 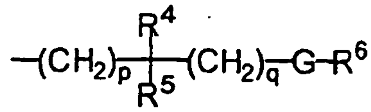 |
|---|---|---|---|---|---|---|---|
| 674 | 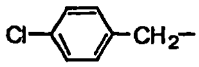 | 2 | 2 | 1 | - | H | 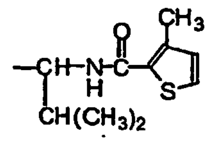 |
| 675 | 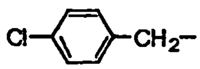 | 2 | 2 | 1 | - | H | 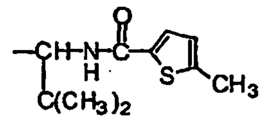 |
| 676 | 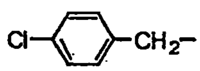 | 2 | 2 | 1 | - | H | 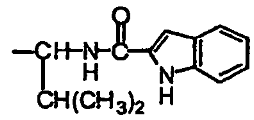 |
| 677 | 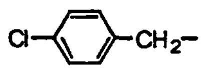 | 2 | 2 | 1 | - | H | 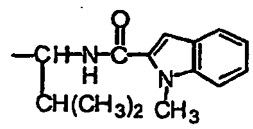 |
| 678 | 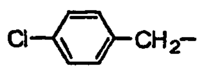 | 2 | 2 | 1 | - | H | 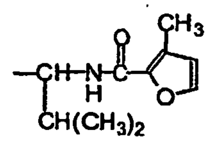 |
| 679 | 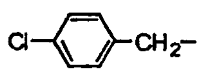 | 2 | 2 | 1 | - | H | 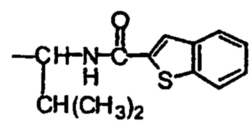 |
| 680 | 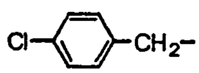 | 2 | 2 | 1 | - | H | 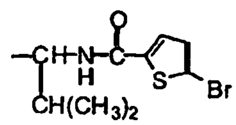 |
| 681 | 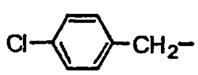 | 2 | 2 | 1 | - | H | 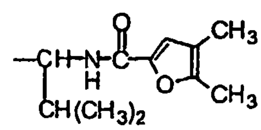 |
| 682 | 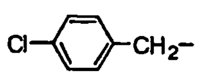 | 2 | 2 | 1 | - | H | 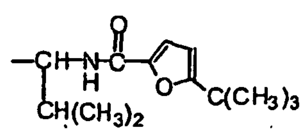 |

**Table 1.63**

| Compd. No. | 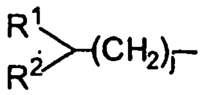 | k | m | n | chirality | $R^3$ | 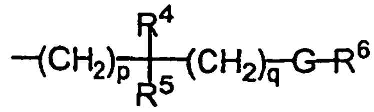 |
|---|---|---|---|---|---|---|---|
| 683 | 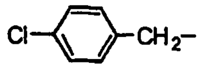 | 2 | 2 | 1 | - | H | 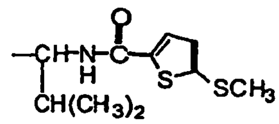 |
| 684 | 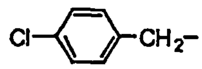 | 2 | 2 | 1 | - | H | 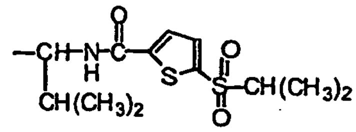 |
| 685 | 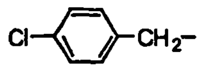 | 2 | 2 | 1 | - | H | 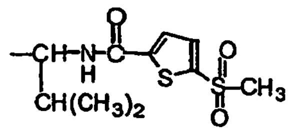 |
| 686 | 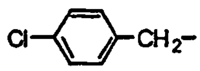 | 2 | 2 | 1 | - | H | 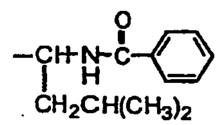 |
| 687 | 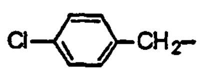 | 2 | 2 | 1 | - | H | 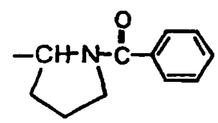 |
| 688 | 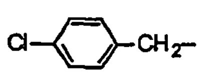 | 2 | 2 | 1 | - | H | 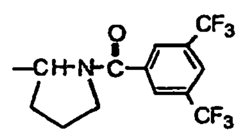 |
| 689 | 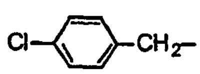 | 2 | 2 | 1 | - | H | 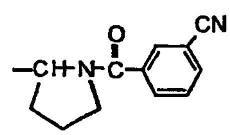 |
| 690 | 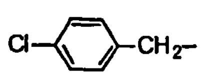 | 2 | 2 | 1 | - | H | 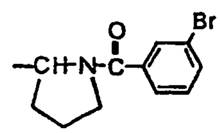 |
| 691 | 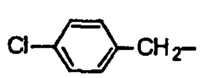 | 2 | 2 | 1 | - | H | 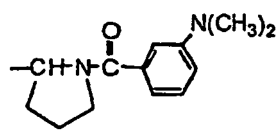 |
| 692 | 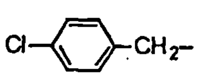 | 2 | 2 | 1 | - | H | 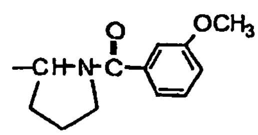 |

(continued)

| Compd. No. | 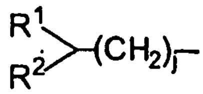 | k | m | n | chirality | $R^3$ | 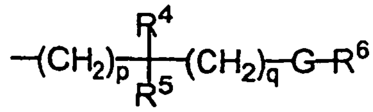 |
|---|---|---|---|---|---|---|---|
| 693 | 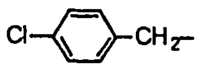 | 2 | 2 | 1 | - | H | 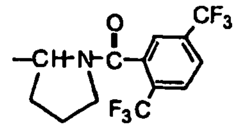 |

**Table 1.64**

| Compd. No. | 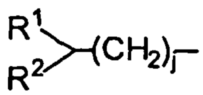 | k | m | n | chirality | $R^3$ | 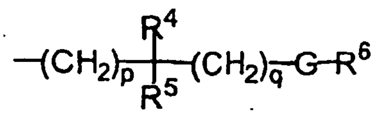 |
|---|---|---|---|---|---|---|---|
| 694 | 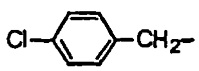 | 2 | 2 | 1 | - | H | 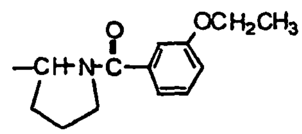 |
| 695 | 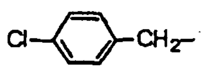 | 2 | 2 | 1 | - | H | 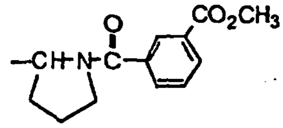 |
| 696 | 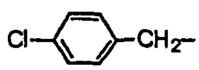 | 2 | 2 | 1 | - | H | 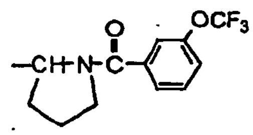 |
| 697 | 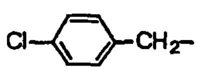 | 2 | 2 | 1 | - | H | 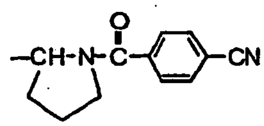 |
| 698 | 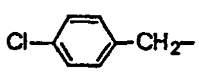 | 2 | 2 | 1 | - | H | 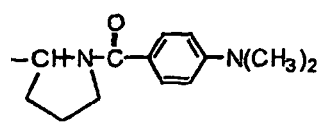 |
| 699 | 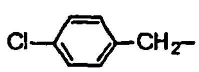 | 2 | 2 | 1 | - | H | 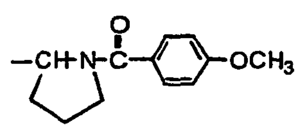 |
| 700 | 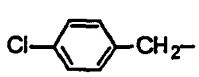 | 2 | 2 | 1 | - | H | 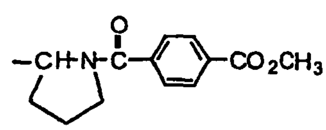 |

(continued)

| Compd. No. | 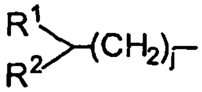 | k | m | n | chirality | $R^3$ | 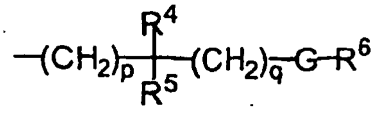 |
|---|---|---|---|---|---|---|---|
| 701 | 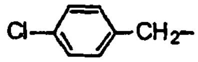 | 2 | 2 | 1 | - | H | 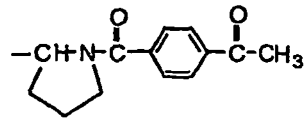 |
| 702 | 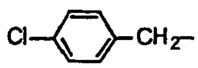 | 2 | 2 | 1 | - | H | 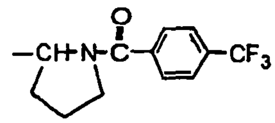 |
| 703 | 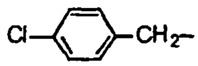 | 2 | 2 | 1 | - | H | 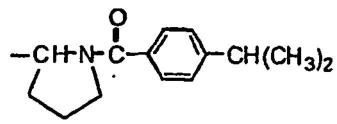 |
| 704 | 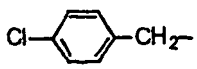 | 2 | 2 | 1 | - | H | 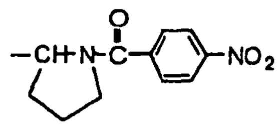 |

**Table 1.65**

| Compd. No. | 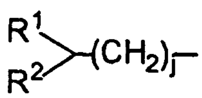 | k | m | n | chirality | $R^3$ | 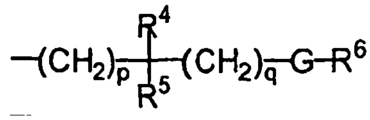 |
|---|---|---|---|---|---|---|---|
| 705 | 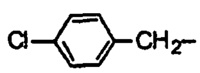 | 2 | 2 | 1 | - | H | 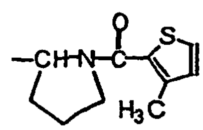 |
| 706 | 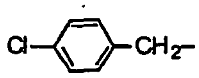 | 2 | 2 | 1 | - | H | 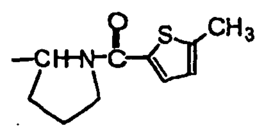 |
| 707 | 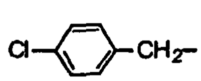 | 2 | 2 | 1 | - | H | 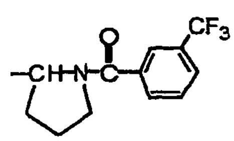 |
| 708 | 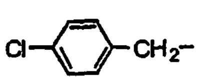 | 2 | 2 | 1 | - | H | 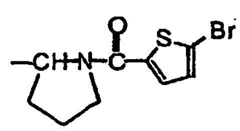 |

(continued)

| Compd. No. | 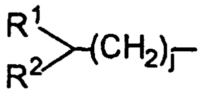 | k | m | n | chirality | $R^3$ | 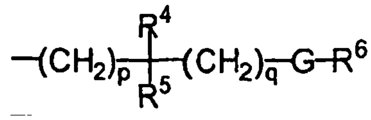 |
|---|---|---|---|---|---|---|---|
| 709 | 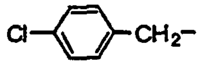 | 2 | 2 | 1 | - | H | 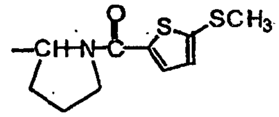 |
| 710 | 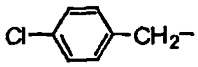 | 2 | 2 | 1 | - | H | 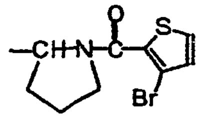 |
| 711 | 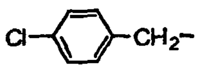 | 2 | 2 | 1 | - | H | 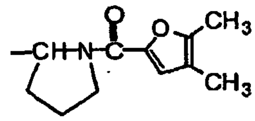 |
| 712 | 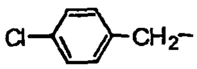 | 2 | 2 | 1 | - | H | 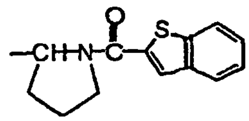 |
| 713 | 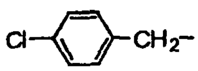 | 2 | 2 | 1 | - | H | 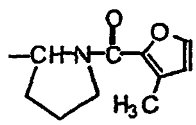 |
| 714 | 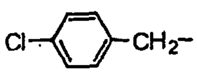 | 2 | 2 | 1 | - | H | 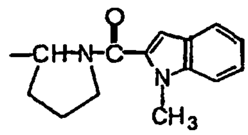 |
| 715 | 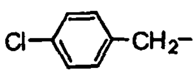 | 2 | 2 | 1 | - | H | 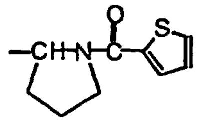 |

**Table 1.66**

| Compd. No. | 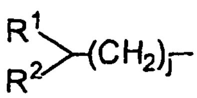 | k | m | n | chirality | $R^3$ | 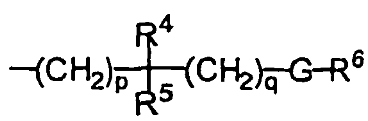 |
|---|---|---|---|---|---|---|---|
| 716 | 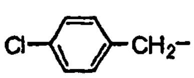 | 2 | 2 | 1 | - | H | 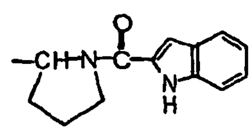 |

(continued)

| Compd. No. | 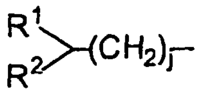 | k | m | n | chirality | $R^3$ | 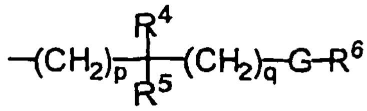 |
|---|---|---|---|---|---|---|---|
| 717 | 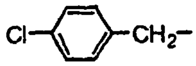 | 2 | 2 | 1 | - | H | 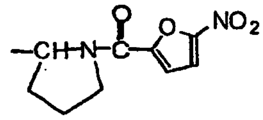 |
| 718 | 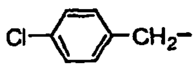 | 2 | 2 | 1 | - | H | 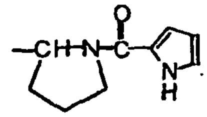 |
| 719 | 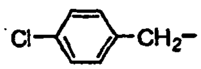 | 2 | 2 | 1 | - | H | 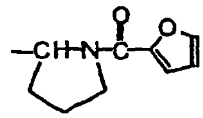 |
| 720 | 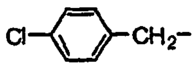 | 2 | 2 | 1 | - | H | 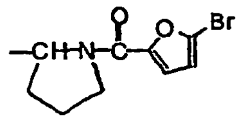 |
| 721 | 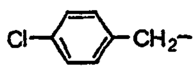 | 2 | 2 | 1 | - | H | 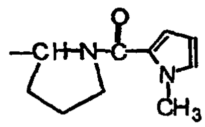 |
| 722 | 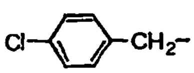 | 2 | 2 | 1 | - | H | 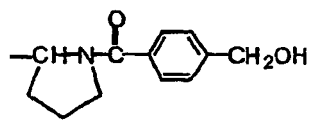 |
| 723 | 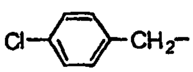 | 2 | 2 | 1 | - | H | 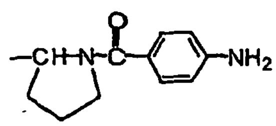 |
| 724 | 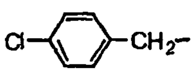 | 2 | 2 | 1 | - | H | 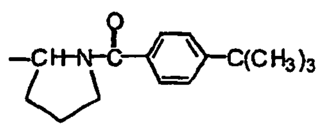 |
| 725 | 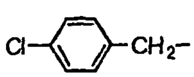 | 2 | 2 | 1 | - | H | 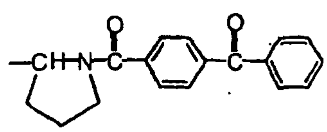 |
| 726 | 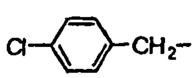 | 2 | 2 | 1 | - | H | 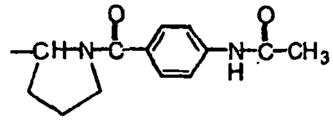 |

**Table 1.67**

| Compd. No. | 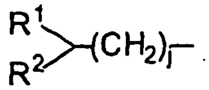 | k | m | n | chirality | $R^3$ | 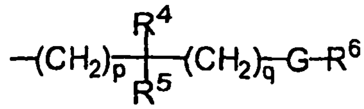 |
|---|---|---|---|---|---|---|---|
| 727 | 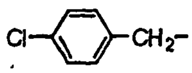 | 2 | 2 | 1 | - | H | 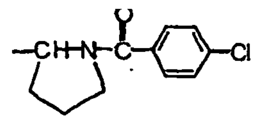 |
| 728 | 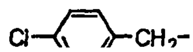 | 2 | 2 | 1 | - | H | 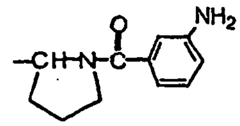 |
| 729 | 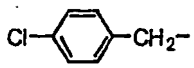 | 2 | 2 | 1 | - | H | 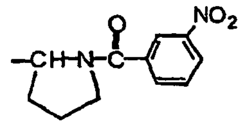 |
| 730 | 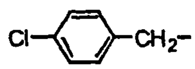 | 2 | 2 | 1 | - | H | 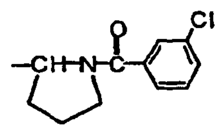 |
| 731 | 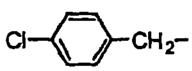 | 2 | 2 | 1 | - | H | 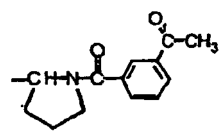 |
| 732 | 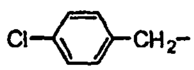 | 2 | 2 | 1 | - | H | 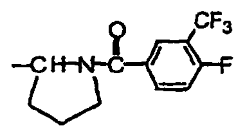 |
| 733 | 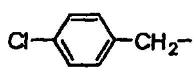 | 2 | 2 | 1 | - | H | 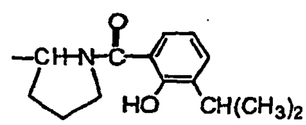 |
| 734 | 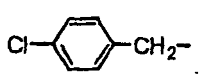 | 2 | 2 | 1 | - | H | 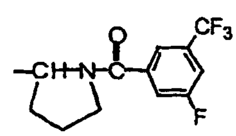 |
| 735 | 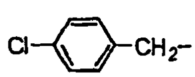 | 2 | 2 | 1 | - | H | 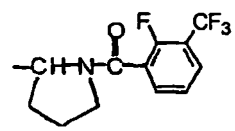 |
| 736 | 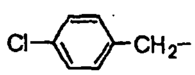 | 2 | 2 | 1 | - | H | 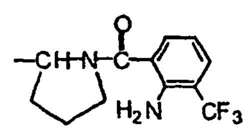 |

(continued)

| Compd. No. | 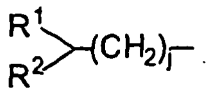 | k | m | n | chirality | $R^3$ | 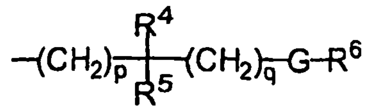 |
|---|---|---|---|---|---|---|---|
| 737 | 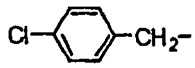 | 2 | 2 | 1 | - | H | 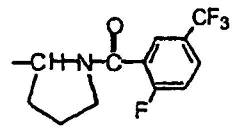 |

**Table 1.68**

| Compd. No. | 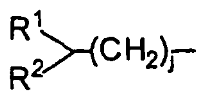 | k | m | n | chirality | $R^3$ | 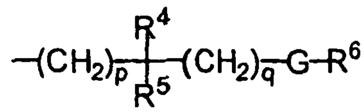 |
|---|---|---|---|---|---|---|---|
| 738 | 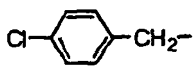 | 2 | 2 | 1 | - | H | 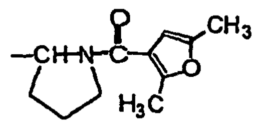 |
| 739 | 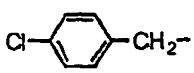 | 2 | 2 | 1 | - | H | 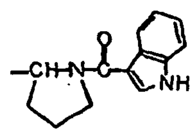 |
| 740 | 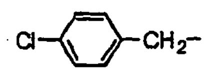 | 2 | 2 | 1 | - | H | 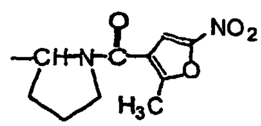 |
| 741 | 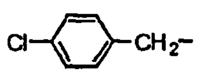 | 2 | 2 | 1 | - | H | 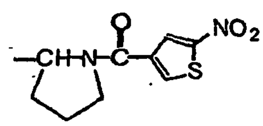 |
| 742 | 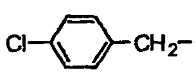 | 2 | 2 | 1 | - | H | 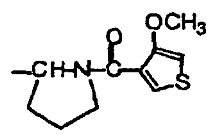 |
| 743 | 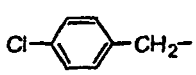 | 2 | 2 | 1 | - | H | 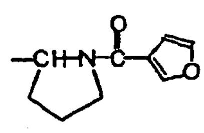 |
| 744 | 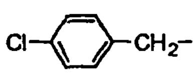 | 2 | 2 | 1 | - | H | 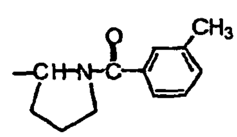 |

(continued)

| Compd. No. | 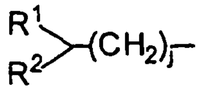 | k | m | n | chirality | $R^3$ | 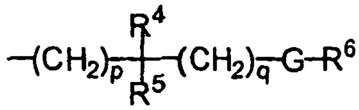 |
|---|---|---|---|---|---|---|---|
| 745 | 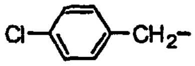 | 2 | 2 | 1 | - | H | 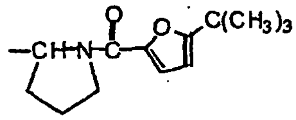 |
| 746 | 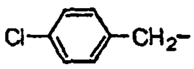 | 2 | 2 | 1 | - | H | 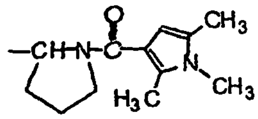 |
| 747 | 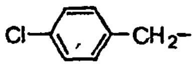 | 2 | 2 | 1 | - | H | 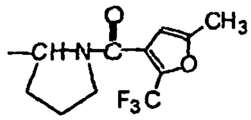 |
| 748 | 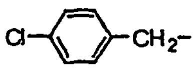 | 2 | 2 | 1 | - | H | 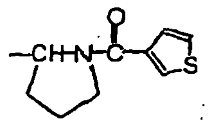 |

**Table 1.69**

| Compd. No. | 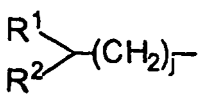 | k | m | n | chirality | $R^3$ | 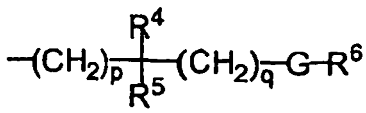 |
|---|---|---|---|---|---|---|---|
| 749 | 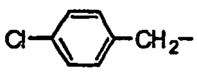 | 2 | 2 | 1 | - | H | 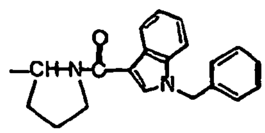 |
| 750 | 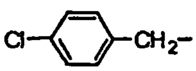 | 2 | 2 | 1 | - | H | 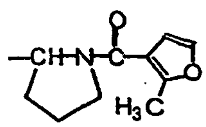 |
| 751 | 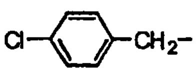 | 2 | 2 | 1 | - | H | 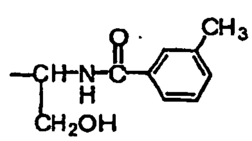 |
| 752 | 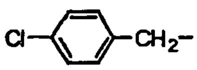 | 2 | 2 | 1 | - | H | 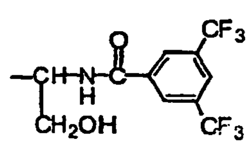 |

(continued)

| Compd. No. | 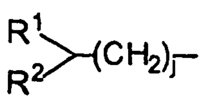 | k | m | n | chirality | $R^3$ | 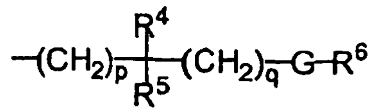 |
|---|---|---|---|---|---|---|---|
| 753 | 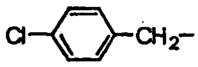 | 2 | 2 | 1 | - | H | 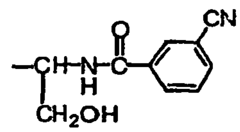 |
| 754 | 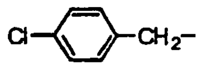 | 2 | 2 | 1 | - | H | 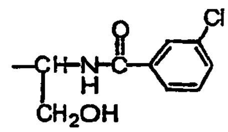 |
| 755 | 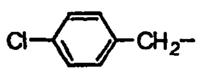 | 2 | 2 | 1 | - | H | 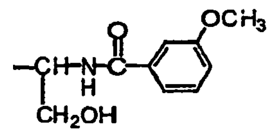 |
| 756 | 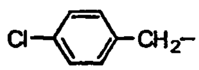 | 2 | 2 | 1 | - | H | 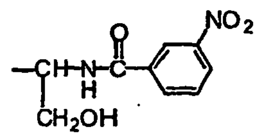 |
| 757 | 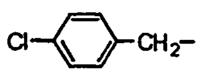 | 2 | 2 | 1 | - | H | 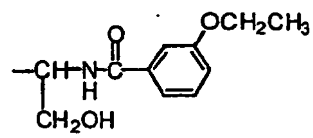 |
| 758 | 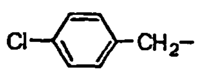 | 2 | 2 | 1 | - | H | 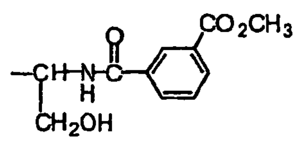 |
| 759 | 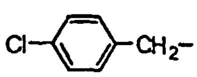 | 2 | 2 | 1 | - | H | 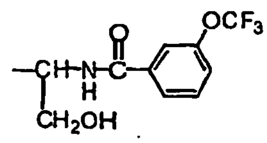 |

**Table 1.70**

| Compd. No. | 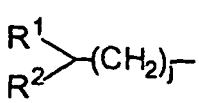 | k | m | n | chirality | $R^3$ | 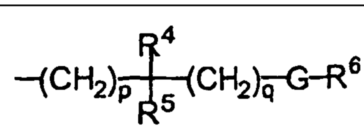 |
|---|---|---|---|---|---|---|---|
| 760 | 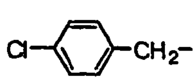 | 2 | 2 | 1 | - | H | 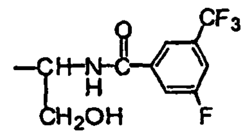 |

(continued)

| Compd. No. | 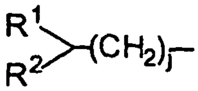 | k | m | n | chirality | $R^3$ | 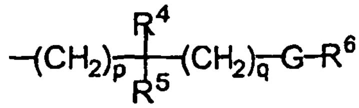 |
|---|---|---|---|---|---|---|---|
| 761 | 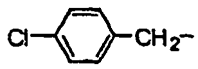 | 2 | 2 | 1 | - | H | 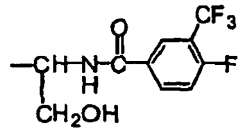 |
| 762 | 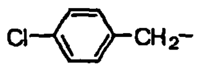 | 2 | 2 | 1 | - | H | 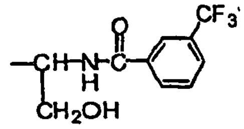 |
| 763 | 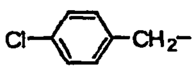 | 2 | 2 | 1 | - | H | 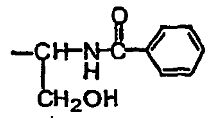 |
| 764 | 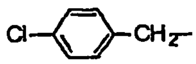 | 2 | 2 | 1 | - | H | 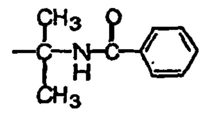 |
| 765 | 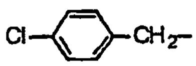 | 2 | 2 | 1 | - | H | 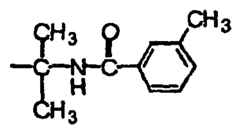 |
| 766 | 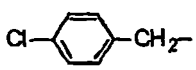 | 2 | 2 | 1 | - | H | 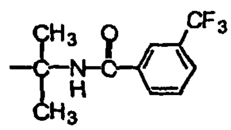 |
| 767 | 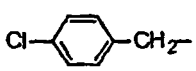 | 2 | 2 | 1 | - | H | 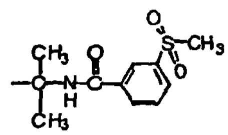 |
| 768 | 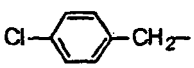 | 2 | 2 | 1 | - | H | 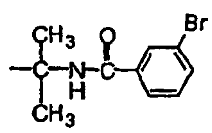 |
| 769 | 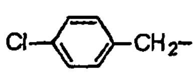 | 2 | 2 | 1 | - | H | 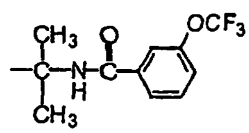 |
| 770 | 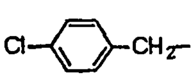 | 2 | 2 | 1 | - | H | 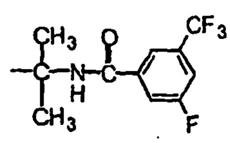 |

**Table 1.71**

| Compd. No. | 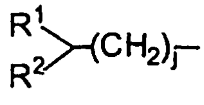 | k | m | n | chirality | $R^3$ | 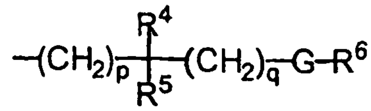 |
|---|---|---|---|---|---|---|---|
| 771 | 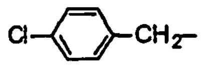 | 2 | 2 | 1 | - | H | 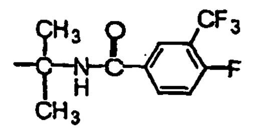 |
| 772 | 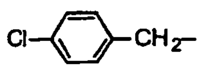 | 2 | 2 | 1 | - | H | 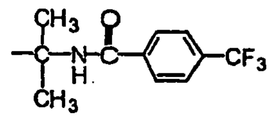 |
| 773 | 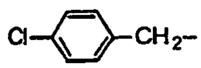 | 2 | 2 | 1 | - | H | 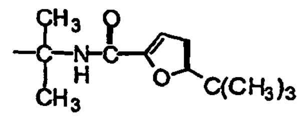 |
| 774 | 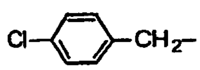 | 2 | 2 | 1 | - | H | 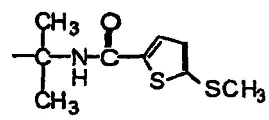 |
| 775 | 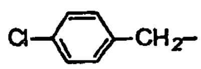 | 2 | 2 | 1 | - | H | 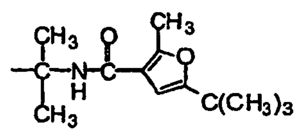 |
| 776 | 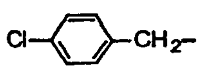 | 2 | 2 | 1 | - | H | 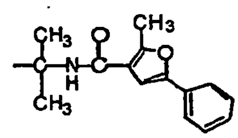 |
| 777 | 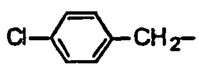 | 2 | 2 | 1 | - | H | 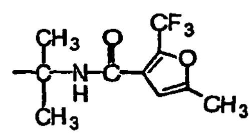 |
| 778 | 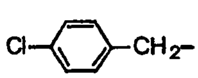 | 2 | 2 | 1 | - | H | 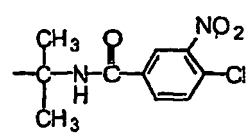 |
| 779 | 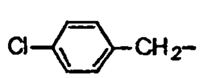 | 2 | 2 | 1 | - | H | 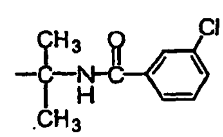 |
| 780 | 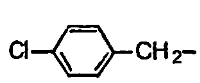 | 2 | 2 | 1 | - | H | 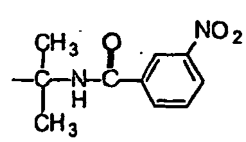 |

(continued)

| Compd. No. | 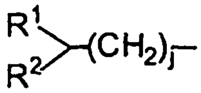 | k | m | n | chirality | $R^3$ | 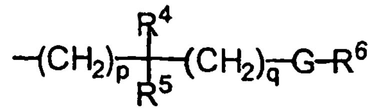 |
|---|---|---|---|---|---|---|---|
| 781 | 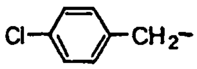 | 2 | 2 | 1 | - | H | 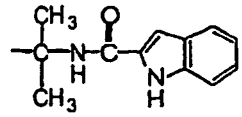 |

**Table 1.72**

| Compd. No. | 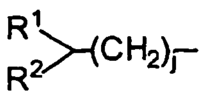 | k | m | n | chirality | $R^3$ | 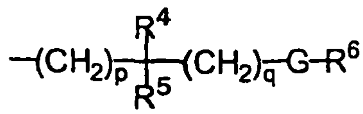 |
|---|---|---|---|---|---|---|---|
| 782 | 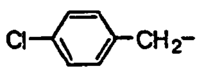 | 2 | 2 | 1 | - | H | 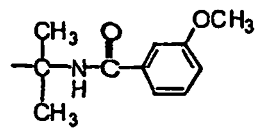 |
| 783 | 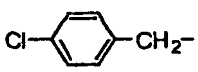 | 2 | 2 | 1 | - | H | 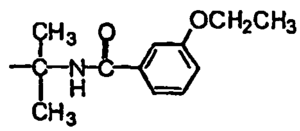 |
| 784 | 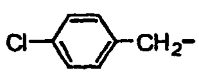 | 2 | 2 | 1 | - | H | 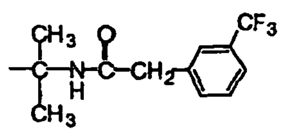 |
| 785 | 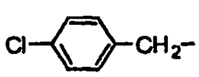 | 2 | 2 | 1 | - | H | 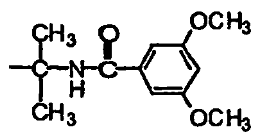 |
| 786 | 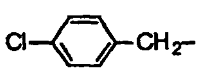 | 2 | 2 | 1 | - | H | 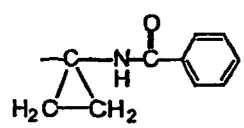 |
| 787 | 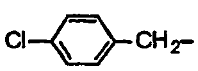 | 2 | 2 | 1 | - | H | 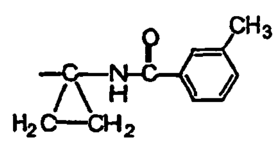 |
| 788 | 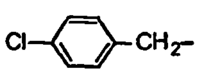 | 2 | 2 | 1 | - | H | 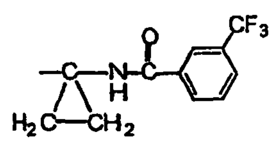 |

(continued)

| Compd. No. | 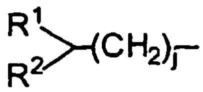 | k | m | n | chirality | $R^3$ | 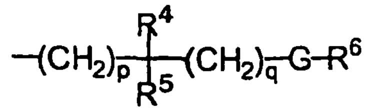 |
|---|---|---|---|---|---|---|---|
| 789 | 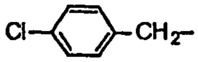 | 2 | 2 | 1 | - | H | 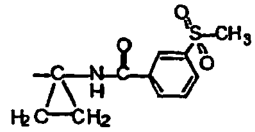 |
| 790 | 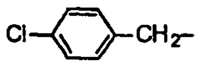 | 2 | 2 | 1 | - | H | 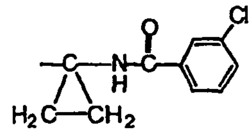 |
| 791 | 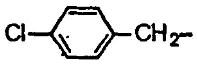 | 2 | 2 | 1 | - | H | 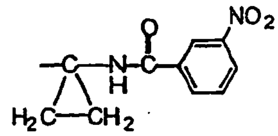 |
| 792 | 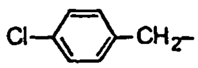 | 2 | 2 | 1 | - | H | 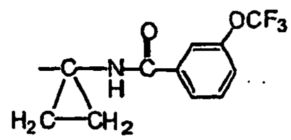 |

**Table 1.73**

| Compd. No. | 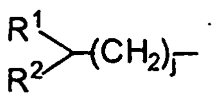 | k | m | n | chirality | $R^3$ | 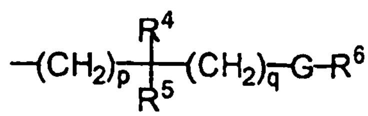 |
|---|---|---|---|---|---|---|---|
| 793 | 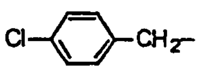 | 2 | 2 | 1 | - | H | 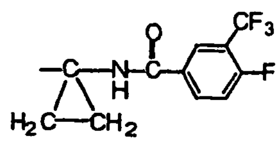 |
| 794 | 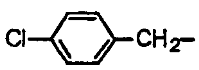 | 2 | 2 | 1 | - | H | 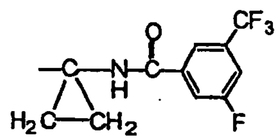 |
| 795 | 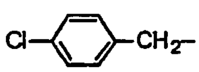 | 2 | 2 | 1 | - | H | 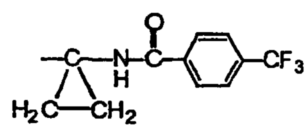 |
| 796 | 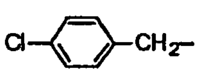 | 2 | 2 | 1 | - | H | 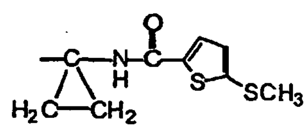 |

(continued)

| Compd. No. | 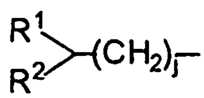 | k | m | n | chirality | $R^3$ | 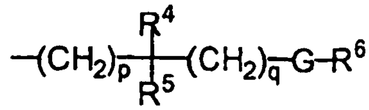 |
|---|---|---|---|---|---|---|---|
| 797 | 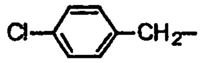 | 2 | 2 | 1 | - | H | 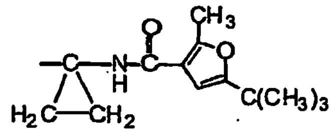 |
| 798 | 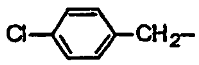 | 2 | 2 | 1 | - | H | 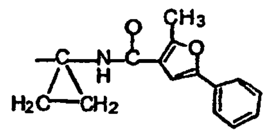 |
| 799 | 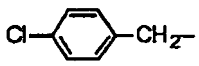 | 2 | 2 | 1 | - | H | 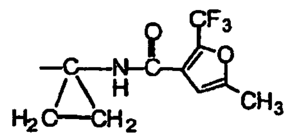 |
| 800 | 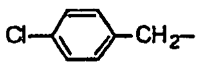 | 2 | 2 | 1 | - | H | 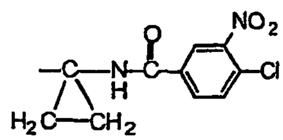 |
| 801 | 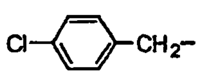 | 2 | 2 | 1 | - | H | 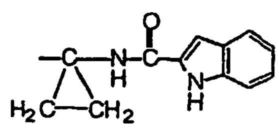 |
| 802 | 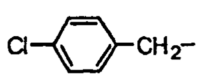 | 2 | 2 | 1 | - | H | 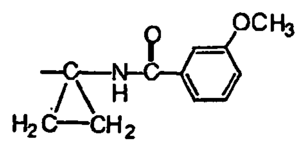 |
| 803 | 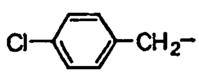 | 2 | 2 | 1 | - | H | 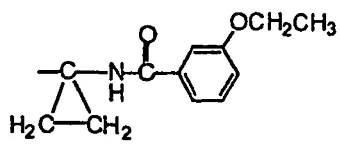 |

**Table 1.74**

| Compd. No. | 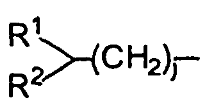 | k | m | n | chirality | $R^3$ | 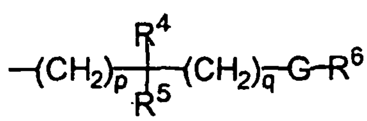 |
|---|---|---|---|---|---|---|---|
| 804 | 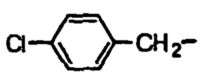 | 2 | 2 | 1 | - | H | 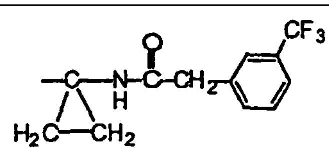 |

(continued)

| Compd. No. | 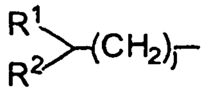 | k | m | n | chirality | $R^3$ | 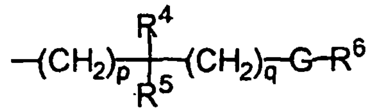 |
|---|---|---|---|---|---|---|---|
| 805 | 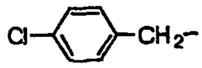 | 2 | 2 | 1 | - | H | 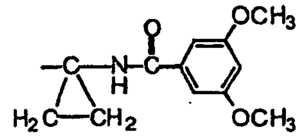 |
| 806 | 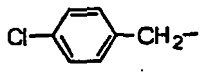 | 2 | 2 | 1 | - | H | 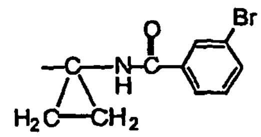 |
| 807 | 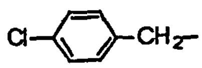 | 2 | 2 | 1 | - | H | 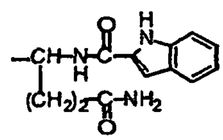 |
| 808 | 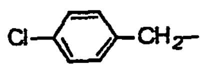 | 2 | 2 | 1 | - | H | 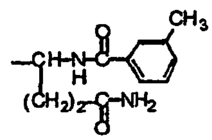 |
| 809 | 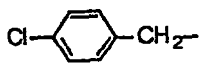 | 2 | 2 | 1 | - | H | 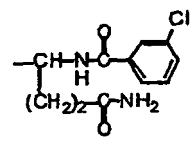 |
| 810 | 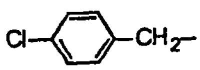 | 2 | 2 | 1 | - | H | 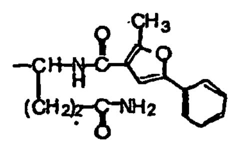 |
| 811 | 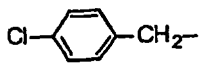 | 2 | 2 | 1 | - | H | 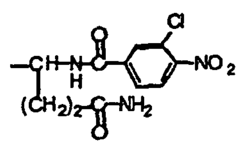 |
| 812 | 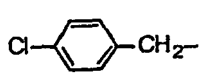 | 2 | 2 | 1 | - | H | 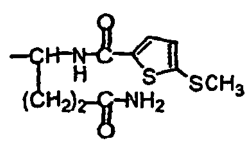 |
| 813 | 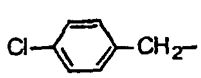 | 2 | 2 | 1 | - | H | 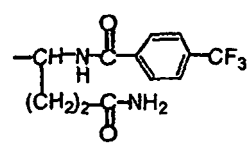 |
| 814 | 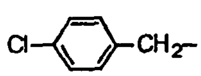 | 2 | 2 | 1 | - | H | 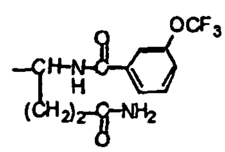 |

**Table 1.75**

| Compd No. | 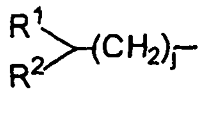 | k | m | n | chirality | $R^3$ | 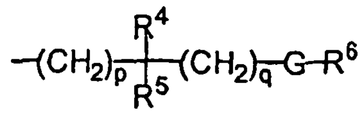 |
|---|---|---|---|---|---|---|---|
| 815 | 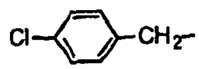 | 2 | 2 | 1 | - | H | 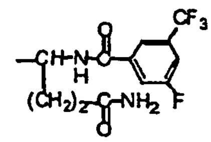 |
| 816 | 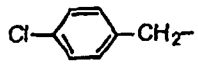 | 2 | 2 | 1 | - | H | 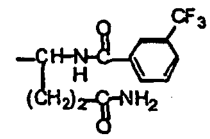 |
| 817 | 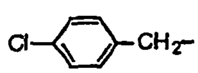 | 2 | 2 | 1 | - | H | 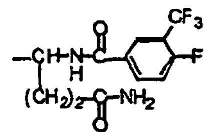 |
| 818 | 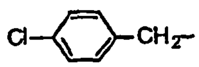 | 2 | 2 | 1 | - | H | 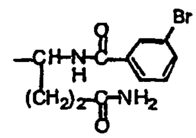 |
| 819 | 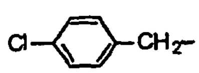 | 2 | 2 | 1 | - | H | 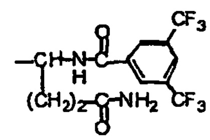 |
| 820 | 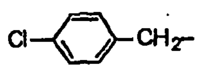 | 2 | 2 | 1 | - | H | 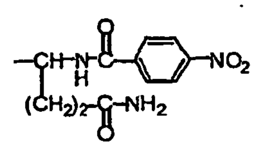 |
| 821 | 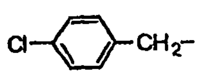 | 2 | 2 | 1 | - | H | 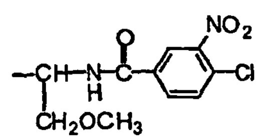 |
| 822 | 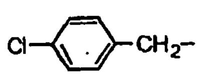 | 2 | 2 | 1 | - | H | 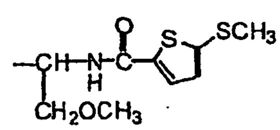 |
| 823 | 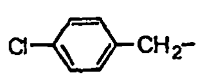 | 2 | 2 | 1 | - | H | 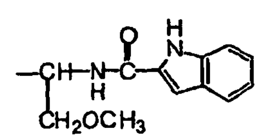 |
| 824 | 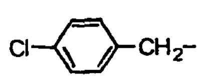 | 2 | 2 | 1 | - | H | 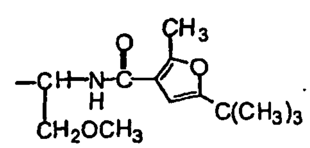 |

(continued)

| Compd No. | 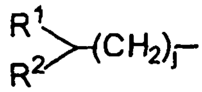 | k | m | n | chirality | $R^3$ | 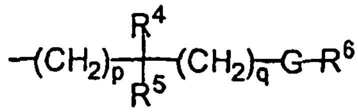 |
|---|---|---|---|---|---|---|---|
| 825 | 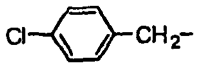 | 2 | 2 | 1 | - | H | 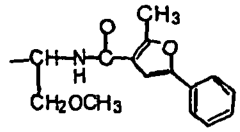 |

**Table 1.76**

| Compd. No. | 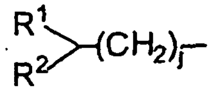 | k | m | n | chirality | $R^3$ | 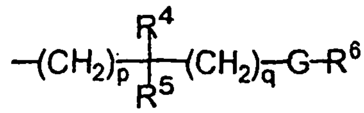 |
|---|---|---|---|---|---|---|---|
| 826 | 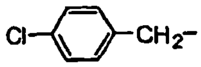 | 2 | 2 | 1 | - | H | 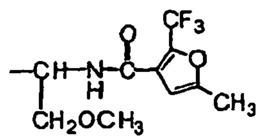 |
| 827 | 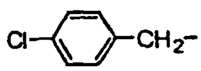 | 2 | 2 | 1 | - | H | 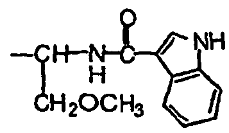 |
| 828 | 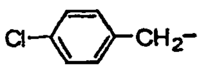 | 2 | 2 | 1 | - | H | 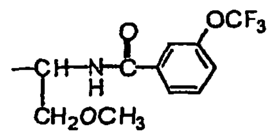 |
| 829 | 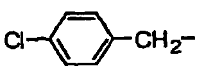 | 2 | 2 | 1 | - | H | 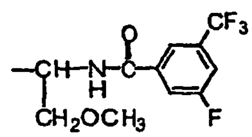 |
| 830 | 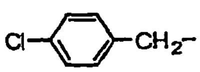 | 2 | 2 | 1 | - | H | 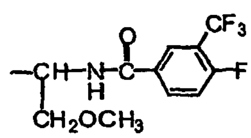 |
| 831 | 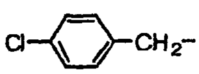 | 2 | 2 | 1 | - | H | 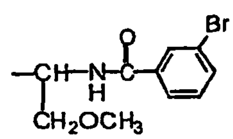 |
| 832 | 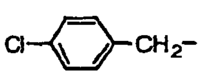 | 2 | 2 | 1 | - | H | 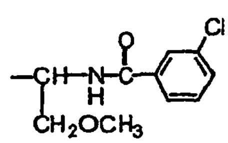 |

(continued)

| Compd. No. | 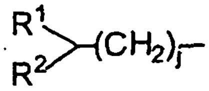 | k | m | n | chirality | $R^3$ | 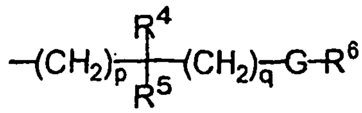 |
|---|---|---|---|---|---|---|---|
| 833 | 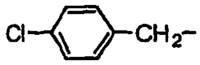 | 2 | 2 | 1 | - | H | 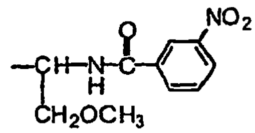 |
| 834 | 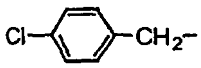 | 2 | 2 | 1 | - | H | 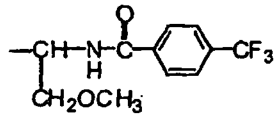 |
| 835 | 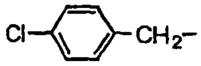 | 2 | 2 | 1 | - | H | 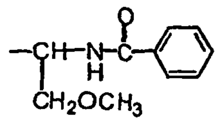 |
| 836 | 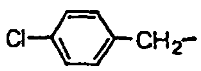 | 2 | 2 | 1 | - | H | 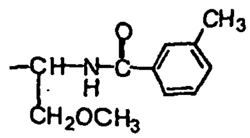 |

**Table 1.77**

| Compd. No. | 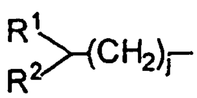 | k | m | n | chirality | $R^3$ | 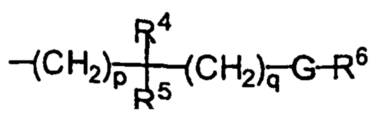 |
|---|---|---|---|---|---|---|---|
| 837 | 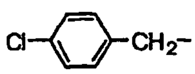 | 2 | 2 | 1 | - | H | 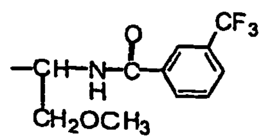 |
| 838 | 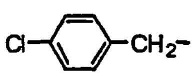 | 2 | 2 | 1 | - | H | 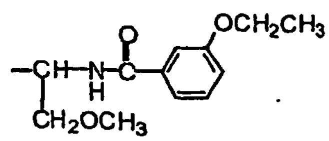 |
| 839 | 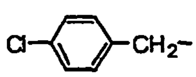 | 2 | 2 | 1 | - | H | 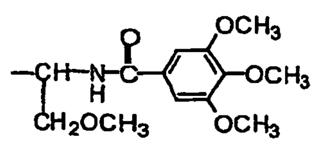 |
| 840 | 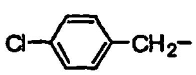 | 2 | 2 | 1 | - | H | 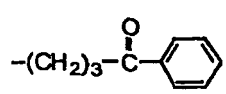 |

(continued)

| Compd. No. | 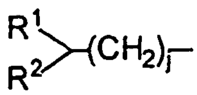 | k | m | n | chirality | $R^3$ | 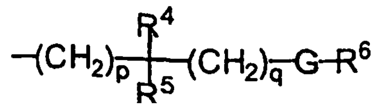 |
|---|---|---|---|---|---|---|---|
| 841 | 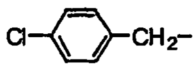 | 2 | 2 | 1 | - | H | 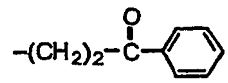 |
| 842 | 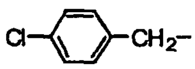 | 2 | 2 | 1 | - | H | 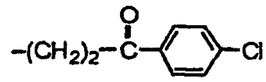 |
| 843 | 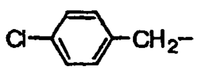 | 2 | 2 | 1 | - | H | 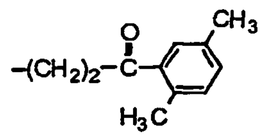 |
| 844 | 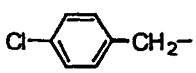 | 2 | 2 | 1 | - | H | 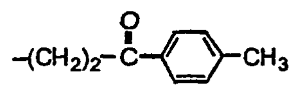 |
| 845 | 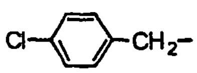 | 2 | 2 | 1 | - | H | 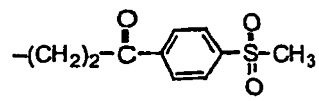 |
| 846 | 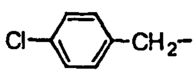 | 2 | 2 | 1 | - | H | 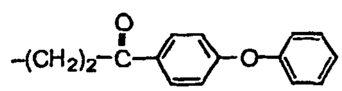 |
| 847 | 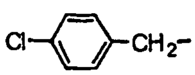 | 2 | 2 | 1 | - | H | 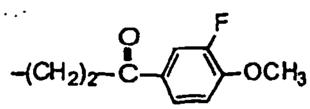 |

**Table 1.78**

| Compd. No. | 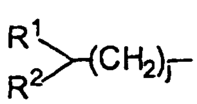 | k | m | n | chirality | $R^3$ | 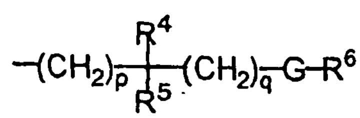 |
|---|---|---|---|---|---|---|---|
| 848 | 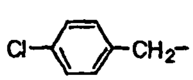 | 2 | 2 | 1 | - | H | 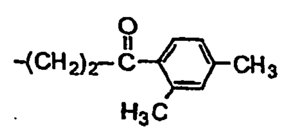 |

(continued)

| Compd. No. | 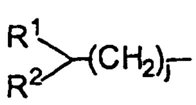 | k | m | n | chirality | $R^3$ | 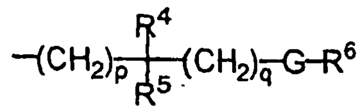 |
|---|---|---|---|---|---|---|---|
| 849 | 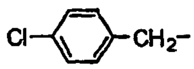 | 2 | 2 | 1 | - | H | 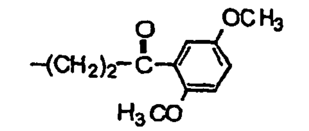 |
| 850 | 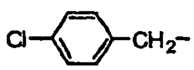 | 2 | 2 | 1 | - | H | 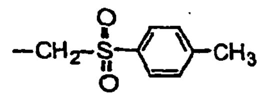 |
| 851 | 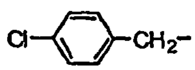 | 2 | 2 | 1 | - | H | 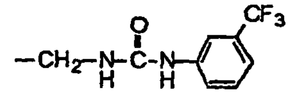 |
| 852 | 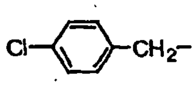 | 2 | 2 | 1 | - | H | 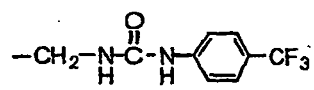 |
| 853 | 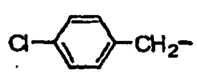 | 2 | 2 | 1 | - | H | 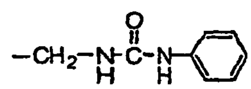 |
| 854 | 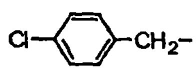 | 2 | 2 | 1 | - | H | 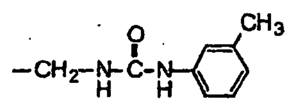 |
| 855 | 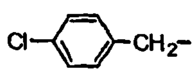 | 2 | 2 | 1 | - | H | 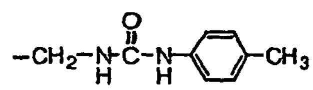 |
| 856 | 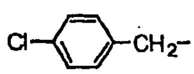 | 2 | 2 | 1 | - | H | 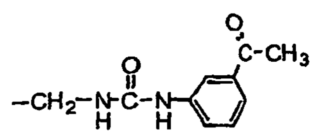 |
| 857 | 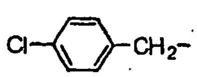 | 2 | 2 | 1 | - | H | 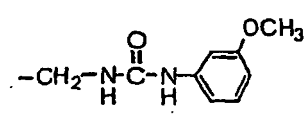 |
| 858 | 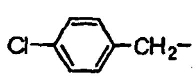 | 2 | 2 | 1 | - | H | 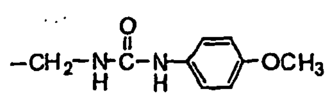 |

**Table 1.79**

| Compd. No. | 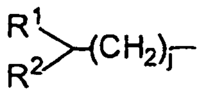 | k | m | n | chirality | $R^3$ | 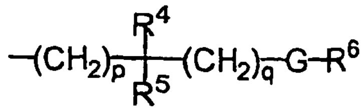 |
|---|---|---|---|---|---|---|---|
| 859 | 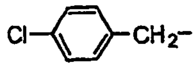 | 2 | 2 | 1 | - | H | 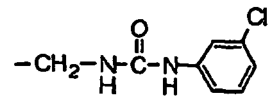 |
| 860 | 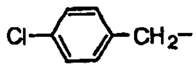 | 2 | 2 | 1 | - | H | 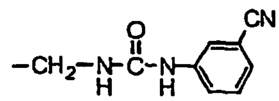 |
| 861 | 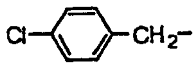 | 2 | 2 | 1 | - | H | 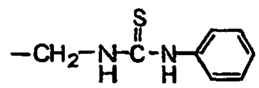 |
| 862 | 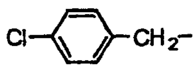 | 2 | 2 | 1 | - | H | 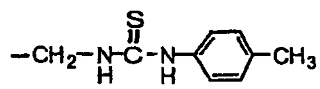 |
| 863 | 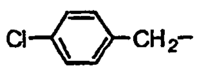 | 2 | 2 | 1 | - | H | 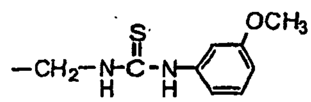 |
| 864 | 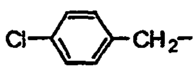 | 2 | 2 | 1 | - | H | 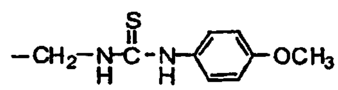 |
| 865 | 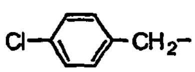 | 2 | 2 | 1 | - | H | 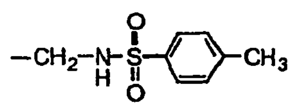 |
| 866 | 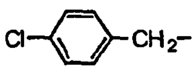 | 2 | 2 | 1 | - | H | 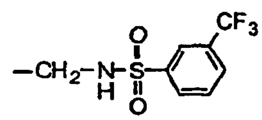 |
| 867 | 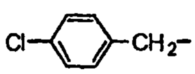 | 2 | 2 | 1 | - | H | 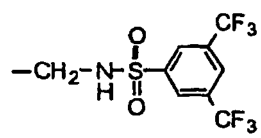 |
| 868 | 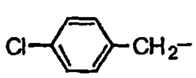 | 2 | 2 | 1 | - | H | 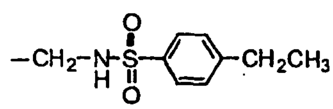 |
| 869 | 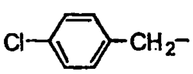 | 2 | 2 | 1 | - | H | 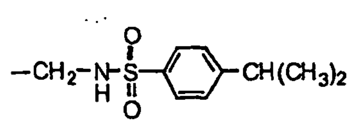 |

**Table 1.80**

| Compd. No. | 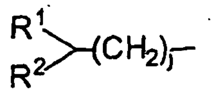 | k | m | n | chirality | $R^3$ | 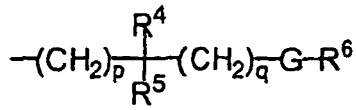 |
|---|---|---|---|---|---|---|---|
| 870 | 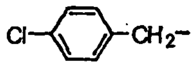 | 2 | 2 | 1 | - | H | 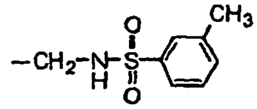 |
| 871 | 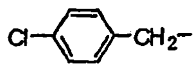 | 2 | 2 | 1 | - | H | 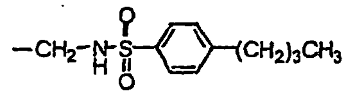 |
| 872 | 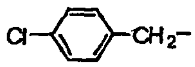 | 2 | 2 | 1 | - | H | 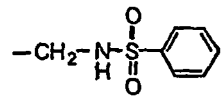 |
| 873 | 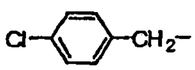 | 2 | 2 | 1 | - | H | 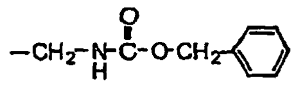 |
| 874 | 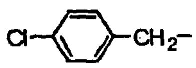 | 2 | 2 | 1 | - | H | 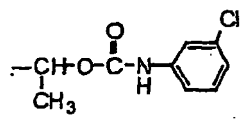 |
| 875 | 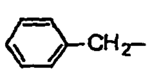 | 2 | 2 | 1 | - | H | 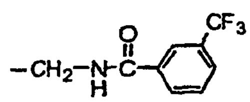 |
| 876 | 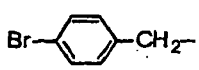 | 2 | 2 | 1 | - | H | 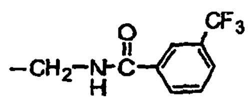 |
| 877 | 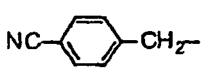 | 2 | 2 | 1 | - | H | 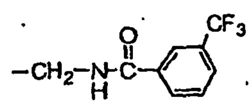 |
| 878 | 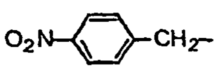 | 2 | 2 | 1 | - | H | 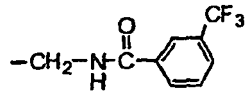 |
| 879 | 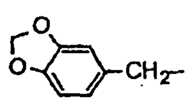 | 2 | 2 | 1 | - | H | 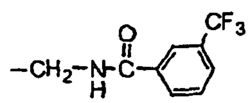 |
| 880 | 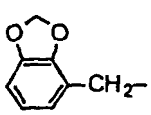 | 2 | 2 | 1 | - | H | 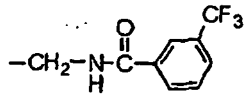 |

**Table 1.81**

| Compd. No. | 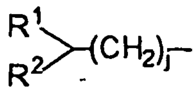 | k | m | n | chirality | $R^3$ | 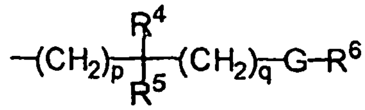 |
|---|---|---|---|---|---|---|---|
| 881 | 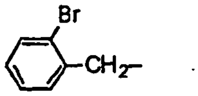 | 2 | 2 | 1 | - | H | 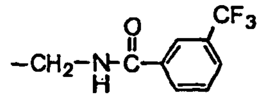 |
| 882 | 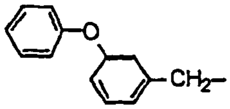 | 2 | 2 | 1 | - | H | 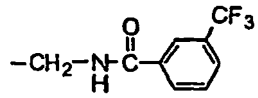 |
| 883 | 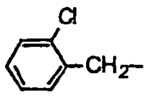 | 2 | 2 | 1 | - | H | 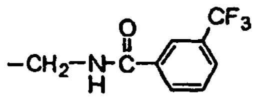 |
| 884 | 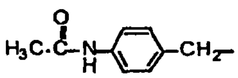 | 2 | 2 | 1 | - | H | 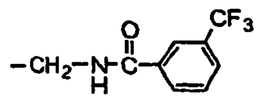 |
| 885 | 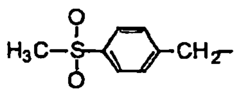 | 2 | 2 | 1 | - | H | 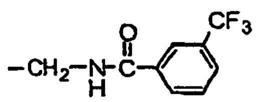 |
| 886 | 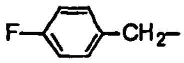 | 2 | 2 | 1 | - | H | 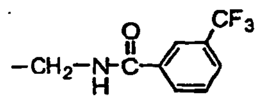 |
| 887 | 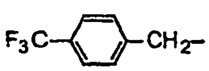 | 2 | 2 | 1 | - | H | 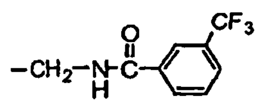 |
| 888 | 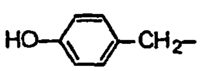 | 2 | 2 | 1 | - | H | 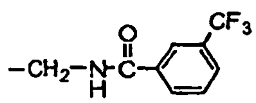 |
| 889 | 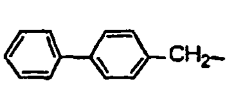 | 2 | 2 | 1 | - | H | 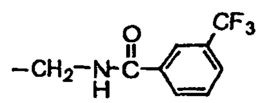 |
| 890 | 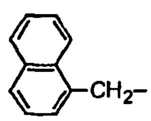 | 2 | 2 | 1 | - | H | 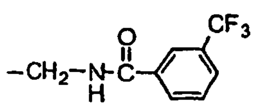 |
| 891 | 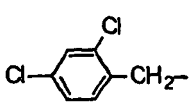 | 2 | 2 | 1 | - | H | 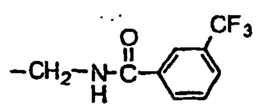 |

**Table 1.82**

| Compd. No. | 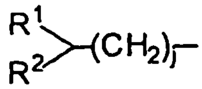 | k | m | n | chirality | $R^3$ | 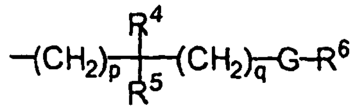 |
|---|---|---|---|---|---|---|---|
| 892 | 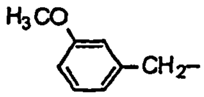 | 2 | 2 | 1 | - | H | 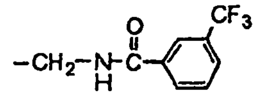 |
| 893 | 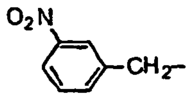 | 2 | 2 | 1 | - | H | 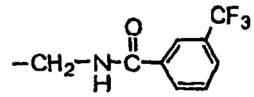 |
| 894 | 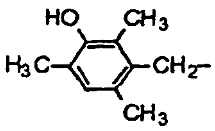 | 2 | 2 | 1 | - | H | 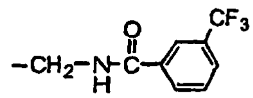 |
| 895 | 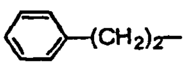 | 2 | 2 | 1 | - | H | 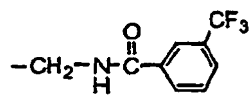 |
| 896 | 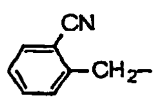 | 2 | 2 | 1 | - | H | 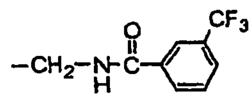 |
| 897 | 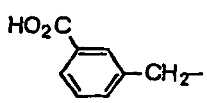 | 2 | 2 | 1 | - | H | 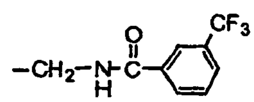 |
| 898 | 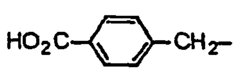 | 2 | 2 | 1 | - | H | 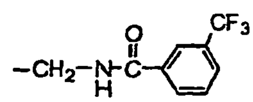 |
| 899 | 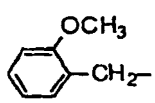 | 2 | 2 | 1 | - | H | 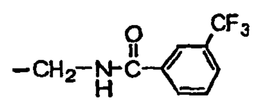 |
| 900 | 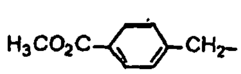 | 2 | 2 | 1 | - | H | 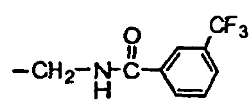 |
| 901 | 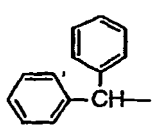 | 2 | 2 | 1 | - | H | 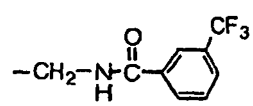 |
| 902 | 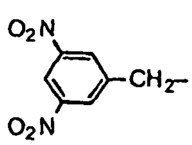 | 2 | 2 | 1 | - | H | 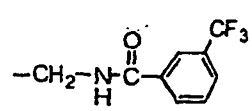 |

**Table 1.83**

| Compd. No. | 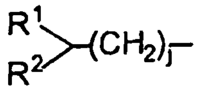 | k | m | n | chirality | $R^3$ | 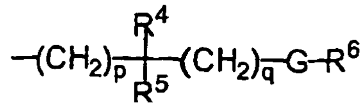 |
|---|---|---|---|---|---|---|---|
| 903 | 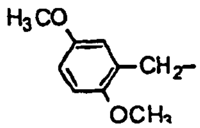 | 2 | 2 | 1 | - | H | 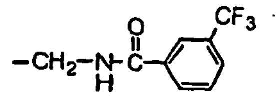 |
| 904 | 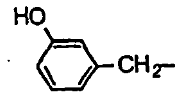 | 2 | 2 | 1 | - | H | 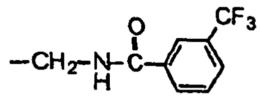 |
| 905 | 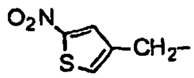 | 2 | 2 | 1 | - | H | 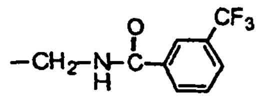 |
| 906 | 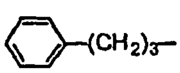 | 2 | 2 | 1 | - | H | 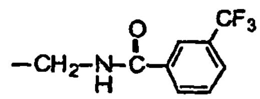 |
| 907 | 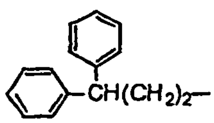 | 2 | 2 | 1 | - | H | 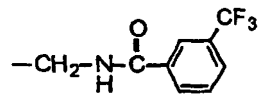 |
| 908 | 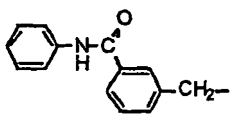 | 2 | 2 | 1 | - | H | 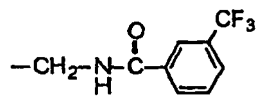 |
| 909 | 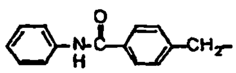 | 2 | 2 | 1 | - | H | 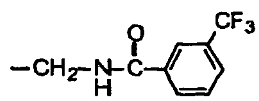 |
| 910 | 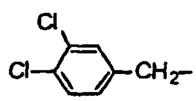 | 2 | 2 | 1 | - | H | 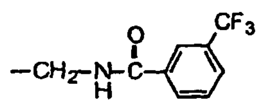 |
| 911 | 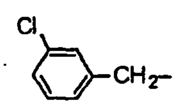 | 2 | 2 | 1 | - | H | 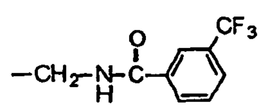 |
| 912 | 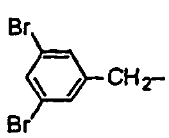 | 2 | 2 | 1 | - | H | 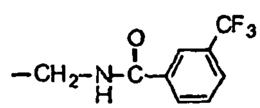 |
| 913 | 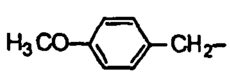 | 2 | 2 | 1 | - | H | 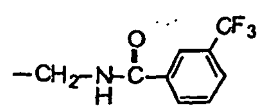 |

**Table 1.84**

| Compd. No. | 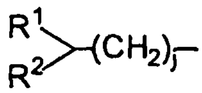 | k | m | n | chirality | $R^3$ | 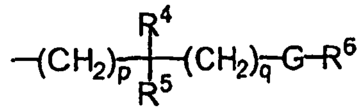 |
|---|---|---|---|---|---|---|---|
| 914 | 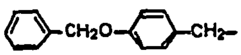 | 2 | 2 | 1 | - | H | 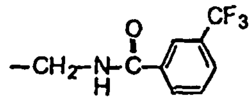 |
| 915 | 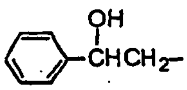 | 2 | 2 | 1 | - | H | 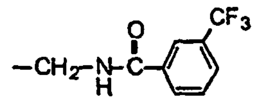 |
| 916 | 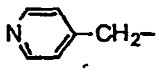 | 2 | 2 | 1 | - | H | 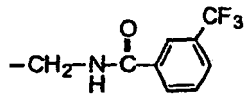 |
| 917 | 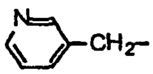 | 2 | 2 | 1 | - | H | 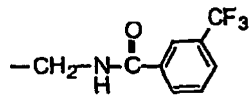 |
| 918 | 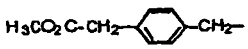 | 2 | 2 | 1 | - | H | 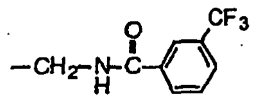 |
| 919 | 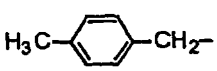 | 2 | 2 | 1 | - | H | 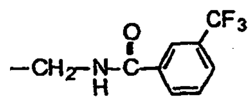 |
| 920 | 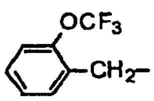 | 2 | 2 | 1 | - | H | 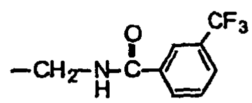 |
| 921 | 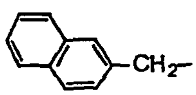 | 2 | 2 | 1 | - | H | 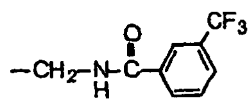 |
| 922 | 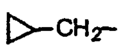 | 2 | 2 | 1 | - | H | 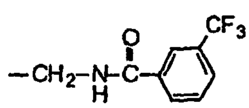 |
| 923 | 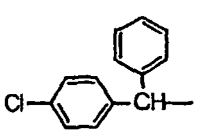 | 2 | 2 | 1 | - | H | 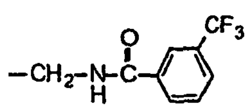 |

(continued)

| Compd. No. | 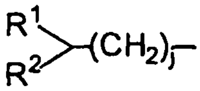 | k | m | n | chirality | $R^3$ | 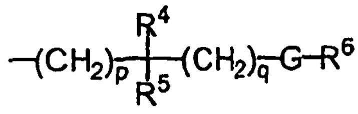 |
|---|---|---|---|---|---|---|---|
| 924 | 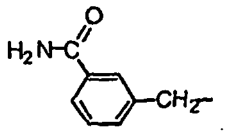 | 2 | 2 | 1 | - | H | 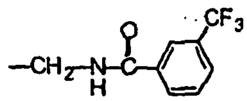 |

**Table 1.85**

| Compd. No. | 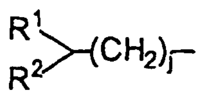 | k | m | n | chirality | $R^3$ | 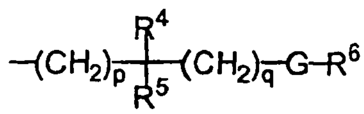 |
|---|---|---|---|---|---|---|---|
| 925 | 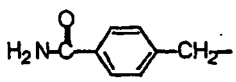 | 2 | 2 | 1 | - | H | 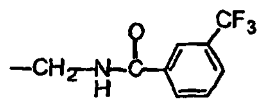 |
| 926 | 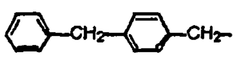 | 2 | 2 | 1 | - | H | 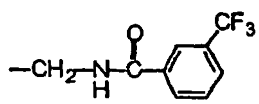 |
| 927 | 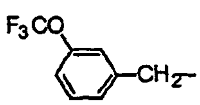 | 2 | 2 | 1 | - | H | 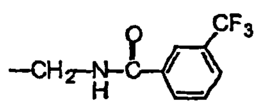 |
| 928 | 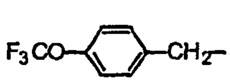 | 2 | 2 | 1 | - | H | 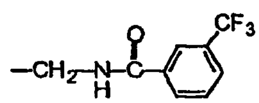 |
| 929 | 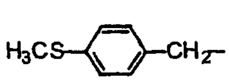 | 2 | 2 | 1 | - | H | 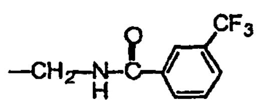 |
| 930 | 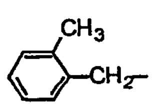 | 2 | 2 | 1 | - | H | 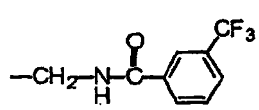 |
| 931 | 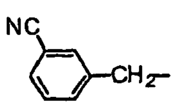 | 2 | 2 | 1 | - | H | 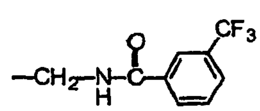 |
| 932 | 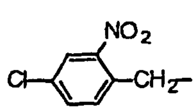 | 2 | 2 | 1 | - | H | 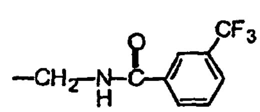 |

(continued)

| Compd. No. | 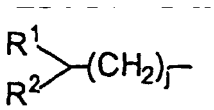 | k | m | n | chirality | $R^3$ | 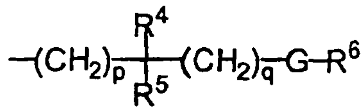 |
|---|---|---|---|---|---|---|---|
| 933 | 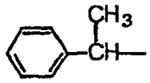 | 2 | 2 | 1 | - | H | 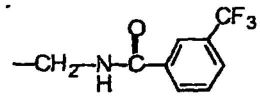 |
| 934 | 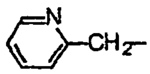 | 2 | 2 | 1 | - | H | 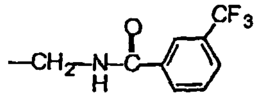 |
| 935 | 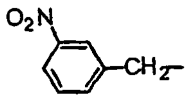 | 2 | 2 | 1 | - | H | 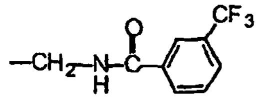 |

**Table 1.86**

| Compd. No. | 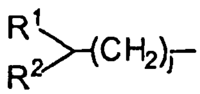 | k | m | n | chirality | $R^3$ | 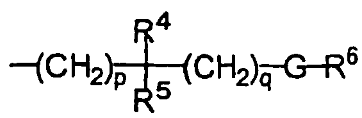 |
|---|---|---|---|---|---|---|---|
| 936 | 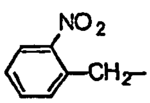 | 2 | 2 | 1 | - | H | 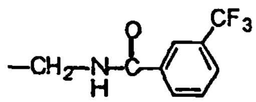 |
| 937 | 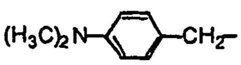 | 2 | 2 | 1 | - | H | 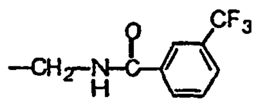 |
| 938 | 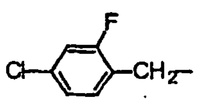 | 2 | 2 | 1 | - | H | 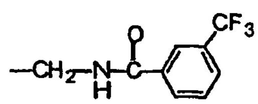 |
| 939 | 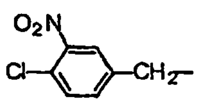 | 2 | 2 | 1 | - | H | 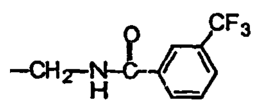 |
| 940 | 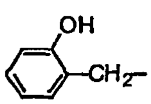 | 2 | 2 | 1 | - | H | 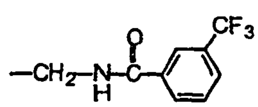 |
| 941 | 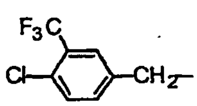 | 2 | 2 | 1 | - | H | 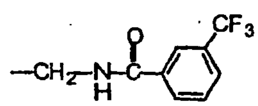 |

(continued)

| Compd. No. | 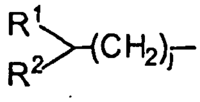 | k | m | n | chirality | $R^3$ | 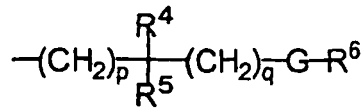 |
|---|---|---|---|---|---|---|---|
| 942 | 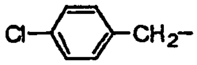 | 2 | 2 | 1 | - | H | 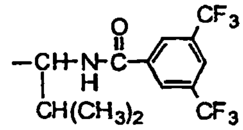 |
| 943 | 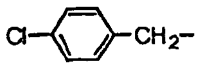 | 1 | 4 | 0 | - | H | 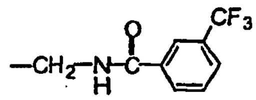 |
| 944 | 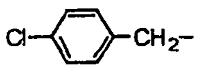 | 1 | 4 | 0 | - | H | 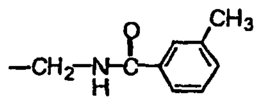 |
| 945 | 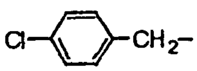 | 1 | 4 | 0 | - | H | 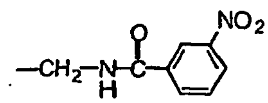 |
| 946 | 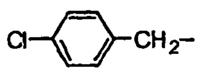 | 1 | 4 | 0 | - | H | 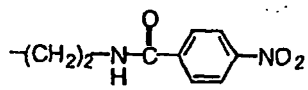 |

**Table 1.87**

| Compd. No. | 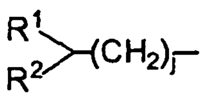 | k | m | n | chirality | $R^3$ | 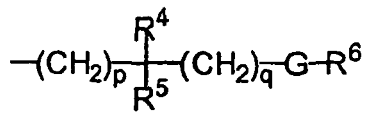 |
|---|---|---|---|---|---|---|---|
| 947 | 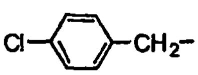 | 1 | 4 | 0 | - | H | 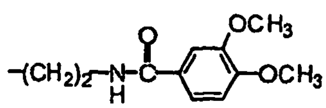 |
| 948 | 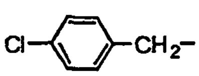 | 1 | 4 | 0 | - | H | 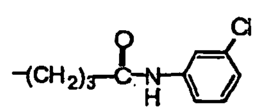 |
| 949 | 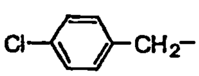 | 1 | 4 | 0 | - | H | 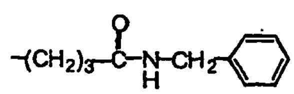 |
| 950 | 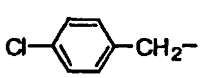 | 0 | 4 | 1 | - | H | 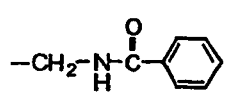 |
| 951 | 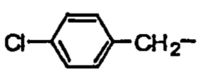 | 1 | 2 | 0 | R | H | 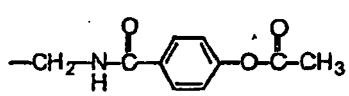 |

(continued)

| Compd. No. | 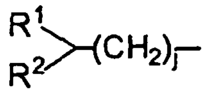 | k | m | n | chirality | $R^3$ | 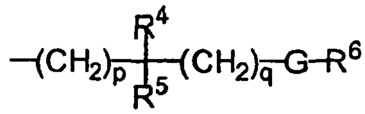 |
|---|---|---|---|---|---|---|---|
| 952 | 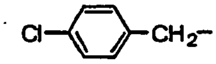 | 1 | 2 | 0 | R | H | 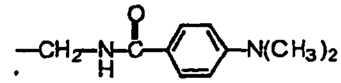 |
| 953 | 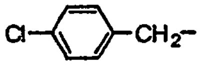 | 1 | 2 | 0 | R | H | 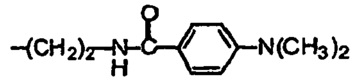 |
| 954 | 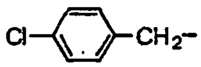 | 1 | 2 | 0 | R | H | 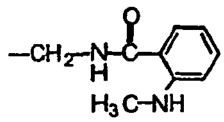 |
| 955 | 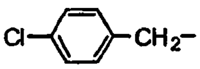 | 1 | 2 | 0 | R | H | 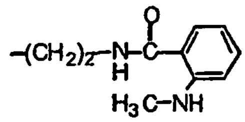 |
| 956 | 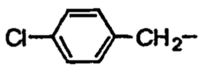 | 1 | 2 | 0 | R | H | 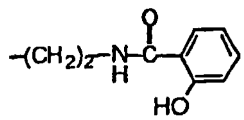 |
| 957 | 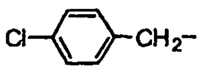 | 1 | 2 | 0 | R | H | 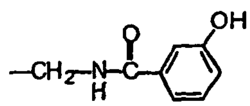 |

**Table 1.88**

| Compd. No. | 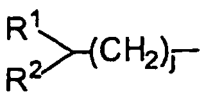 | k | m | n | chirality | $R^3$ | 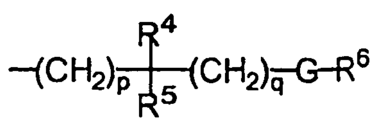 |
|---|---|---|---|---|---|---|---|
| 958 | 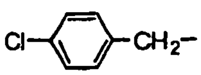 | 1 | 2 | 0 | R | H | 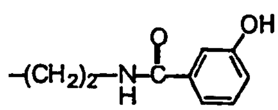 |
| 959 | 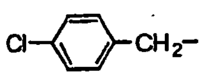 | 1 | 2 | 0 | R | H | 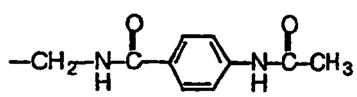 |
| 960 | 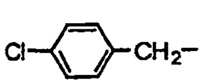 | 1 | 2 | 0 | R | H | 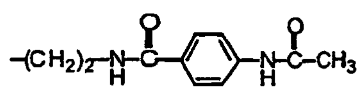 |

(continued)

| Compd. No. | 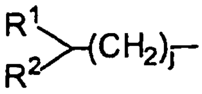 | k | m | n | chirality | $R^3$ | 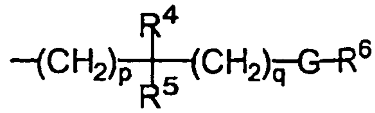 |
|---|---|---|---|---|---|---|---|
| 961 | 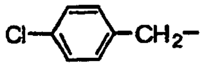 | 1 | 2 | 0 | R | H | 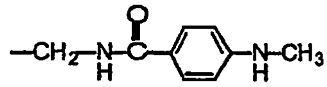 |
| 962 | 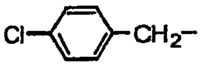 | 1 | 2 | 0 | R | H | 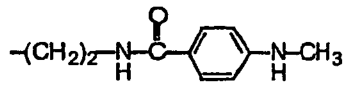 |
| 963 | 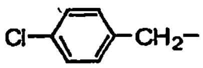 | 1 | 2 | 0 | R | H | 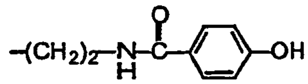 |
| 964 | 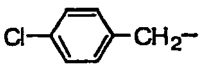 | 1 | 2 | 0 | R | H | 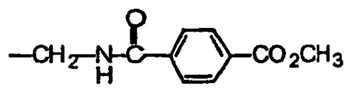 |
| 965 | 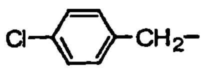 | 1 | 2 | 0 | R | H | 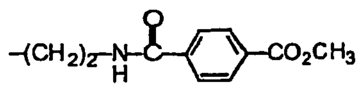 |
| 966 | 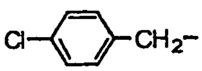 | 1 | 2 | 0 | R | H | 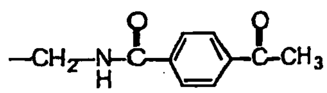 |
| 967 | 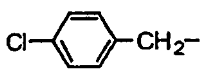 | 1 | 2 | 0 | R | H | 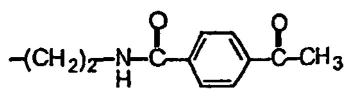 |
| 968 | 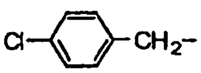 | 1 | 2 | 0 | R | H | 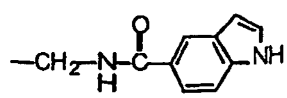 |

**Table 1.89**

| Compd. No. | 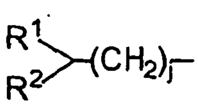 | k | m | n | chirality | $R^3$ | 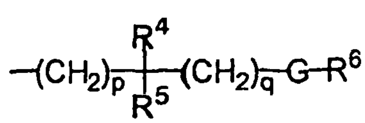 |
|---|---|---|---|---|---|---|---|
| 969 | 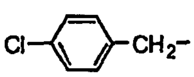 | 1 | 2 | 0 | R | H | 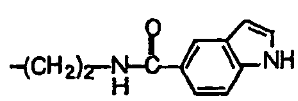 |
| 970 | 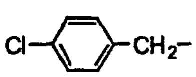 | 1 | 2 | 0 | R | H | 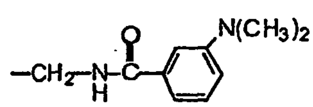 |

(continued)

| Compd. No. | 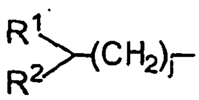 | k | m | n | chirality | $R^3$ | 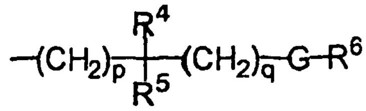 |
|---|---|---|---|---|---|---|---|
| 971 | 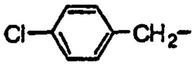 | 1 | 2 | 0 | R | H | 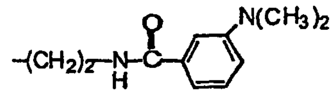 |
| 972 | 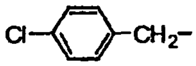 | 1 | 2 | 0 | R | H | 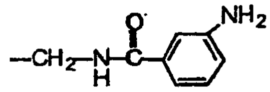 |
| 973 | 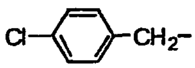 | 1 | 2 | 0 | R | H | 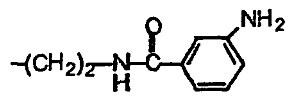 |
| 974 | 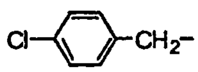 | 1 | 2 | 0 | R | H | 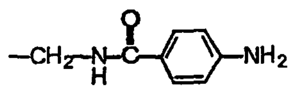 |
| 975 | 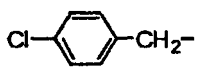 | 1 | 2 | 0 | R | H | 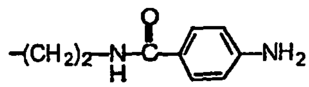 |
| 976 | 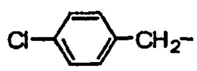 | 1 | 2 | 0 | R | H | 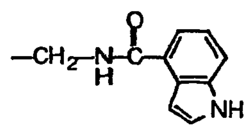 |
| 977 | 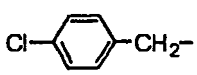 | 1 | 2 | 0 | R | H | 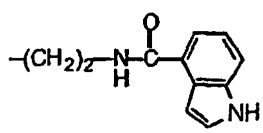 |
| 978 | 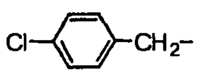 | 1 | 2 | 0 | R | H | 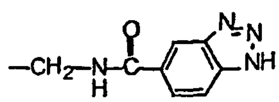 |
| 979 | 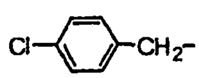 | 1 | 2 | 0 | R | H | 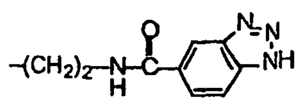 |

**Table 1.90**

| Compd: No. | 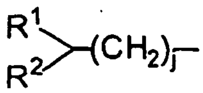 | k | m | n | chirality | $R^3$ | 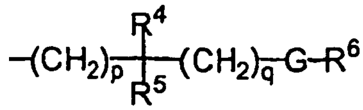 |
|---|---|---|---|---|---|---|---|
| 980 | 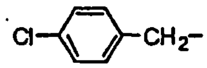 | 1 | 2 | 0 | R | H | 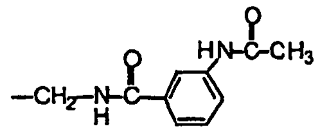 |
| 981 | 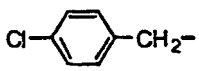 | 1 | 2 | 0 | R | H | 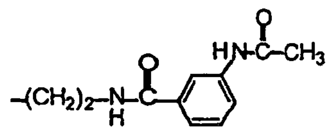 |
| 982 | 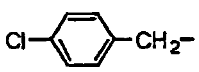 | 1 | 2 | 0 | R | H | 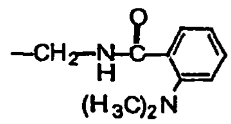 |
| 983 | 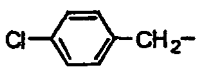 | 1 | 2 | 0 | R | H | 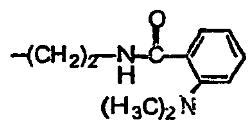 |
| 984 | 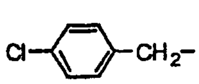 | 1 | 2 | 0 | R | H | 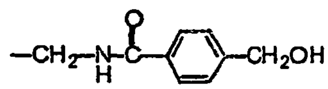 |
| 985 | 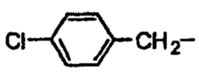 | 1 | 2 | 0 | R | H | 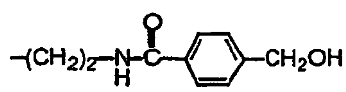 |
| 986 | 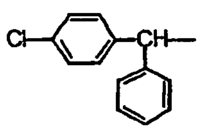 | 1 | 2 | 0 | R | H | 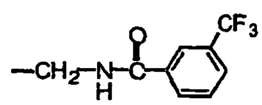 |
| 987 | 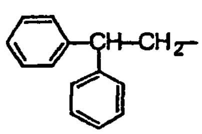 | 2 | 2 | 1 | - | H | 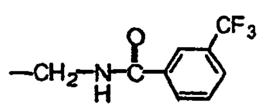 |
| 988 | 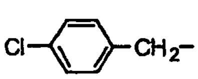 | 1 | 4 | 0 | - | H | 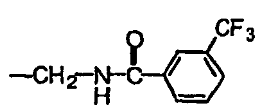 |
| 989 | 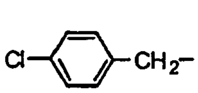 | 1 | 4 | 0 | - | H | 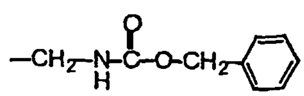 |

(continued)

| Compd: No. | 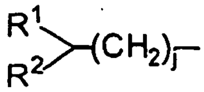 | k | m | n | chirality | $R^3$ | 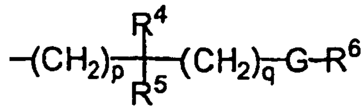 |
|---|---|---|---|---|---|---|---|
| 990 | 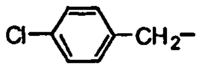 | 1 | 4 | 0 | - | H | 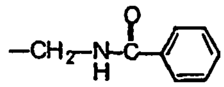 |

**Table 1.91**

| Compd. No. | 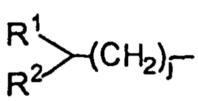 | k | m | n | chirality | $R^3$ | 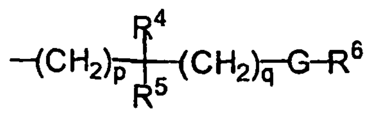 |
|---|---|---|---|---|---|---|---|
| 991 | 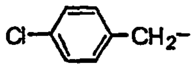 | 1 | 4 | 0 | - | H | 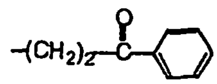 |
| 992 | 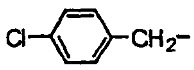 | 1 | 4 | 0 | - | H | 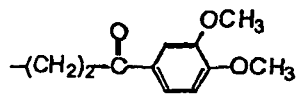 |
| 993 | 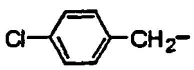 | 1 | 4 | 0 | - | H | 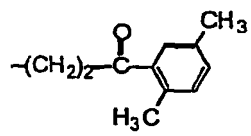 |
| 994 | 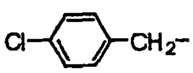 | 1 | 4 | 0 | - | H | 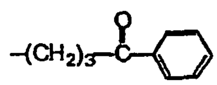 |
| 995 | 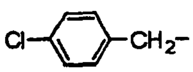 | 1 | 4 | 0 | - | H | 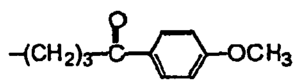 |
| 996 | 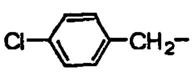 | 1 | 4 | 0 | - | H | 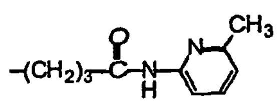 |
| 997 | 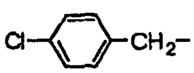 | 2 | 2 | 1 | - | H | 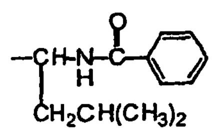 |
| 998 | 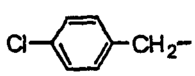 | 2 | 2 | 1 | - | H | 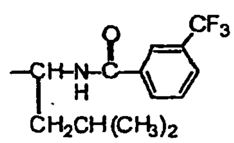 |

(continued)

| Compd. No. | 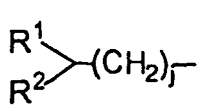 | k | m | n | chirality | $R^3$ | 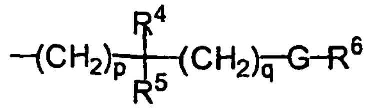 |
|---|---|---|---|---|---|---|---|
| 999 | 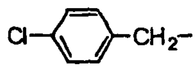 | 2 | 2 | 1 | - | H | 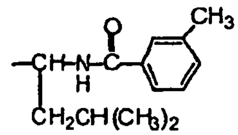 |
| 1000 | 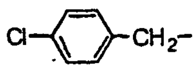 | 2 | 2 | 1 | - | H | 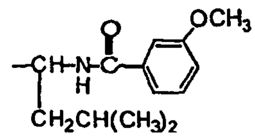 |
| 1001 | 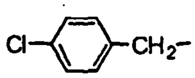 | 2 | 2 | 1 | - | H | 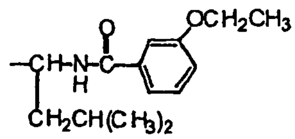 |

**Table 1.92**

| Compd. No. | 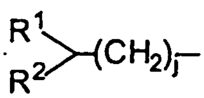 | k | m | n | chirality | $R^3$ | 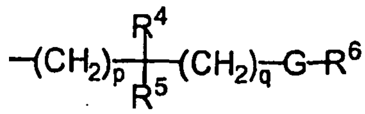 |
|---|---|---|---|---|---|---|---|
| 1002 | 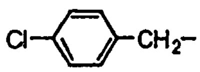 | 2 | 2 | 1 | - | H | 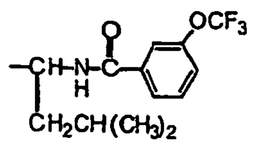 |
| 1003 | 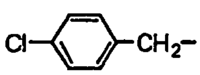 | 2 | 2 | 1 | - | H | 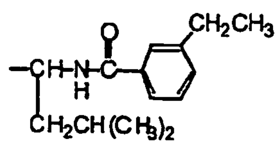 |
| 1004 | 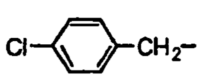 | 2 | 2 | 1 | - | H | 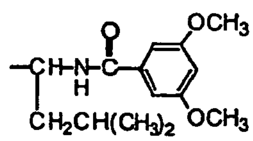 |
| 1005 | 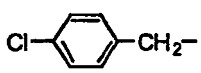 | 2 | 2 | 1 | - | H | 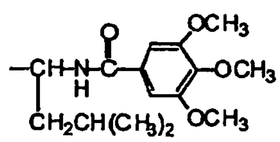 |
| 1006 | 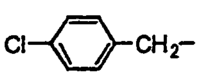 | 2 | 2 | 1 | - | H | 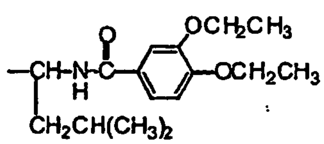 |

(continued)

| Compd. No. | 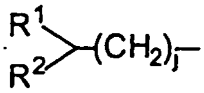 | k | m | n | chirality | $R^3$ | 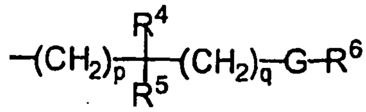 |
|---|---|---|---|---|---|---|---|
| 1007 | 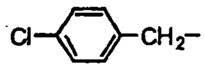 | 2 | 2 | 1 | - | H | 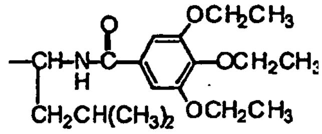 |
| 1008 | 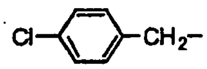 | 2 | 2 | 1 | - | H | 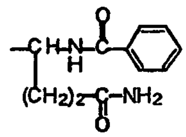 |
| 1009 | 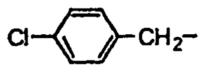 | 2 | 2 | 1 | - | H | 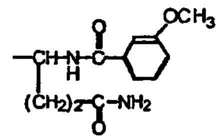 |
| 1010 | 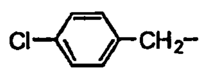 | 2 | 2 | 1 | - | H | 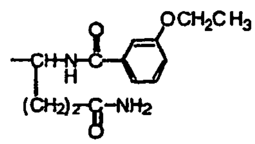 |
| 1011 | 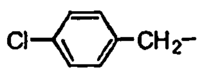 | 2 | 2 | 1 | - | H | 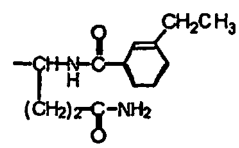 |
| 1012 | 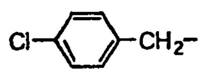 | 2 | 2 | 1 | - | H | 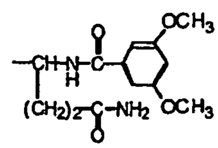 |

**Table 1.93**

| Compd. No. | 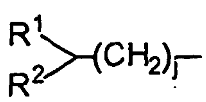 | k | m | n | chirality | $R^3$ | 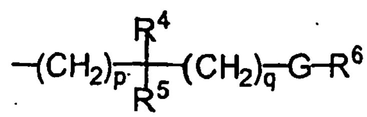 |
|---|---|---|---|---|---|---|---|
| 1013 | 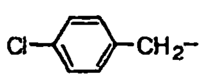 | 2 | 2 | 1 | - | H | 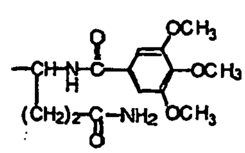 |
| 1014 | 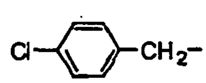 | 2 | 2 | 1 | - | H | 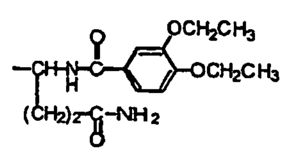 |

(continued)

| Compd. No. | 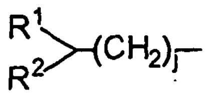 | k | m | n | chirality | $R^3$ | 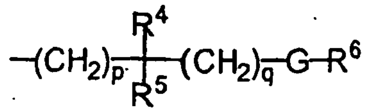 |
|---|---|---|---|---|---|---|---|
| 1015 | 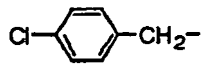 | 2 | 2 | 1 | - | H | 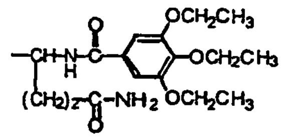 |
| 1016 | 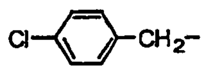 | 2 | 2 | 0 | - | H | 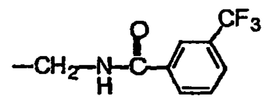 |
| 1017 | 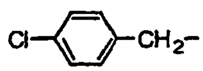 | 2 | 2 | 0 | - | H | 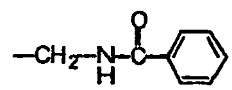 |
| 1018 | 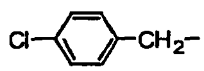 | 2 | 2 | 1 | - | H | 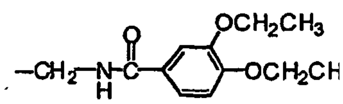 |
| 1019 | 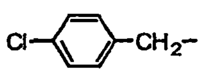 | 2 | 2 | 1 | - | H | 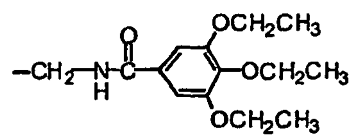 |
| 1020 | 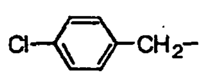 | 2 | 2 | 1 | - | H | 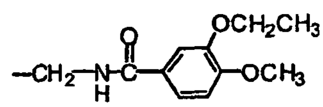 |
| 1021 | 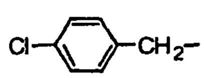 | 2 | 2 | 1 | - | H | 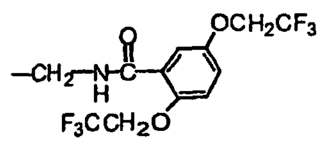 |
| 1022 | 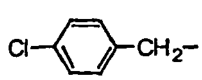 | 2 | 2 | 1 | - | H | 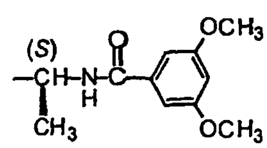 |
| 1023 | 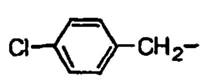 | 2 | 2 | 1 | - | H | 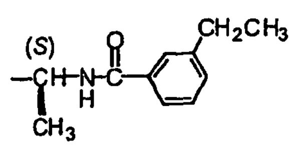 |

**Table 1.94**

| Compd. No. | 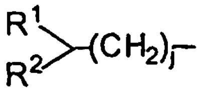 | k | m | n | chirality | $R^3$ | 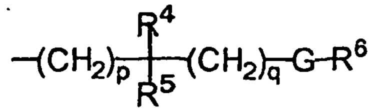 |
|---|---|---|---|---|---|---|---|
| 1024 | 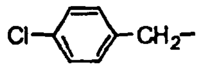 | 2 | 2 | 1 | - | H | 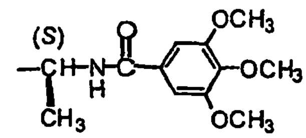 |
| 1025 | 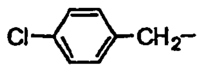 | 2 | 2 | 1 | - | H | 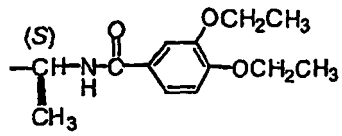 |
| 1026 | 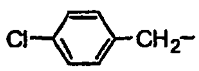 | 2 | 2 | 1 | - | H | 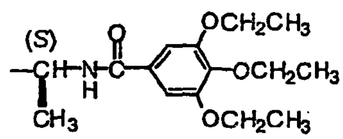 |
| 1027 | 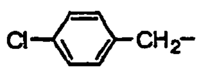 | 2 | 2 | 1 | - | H | 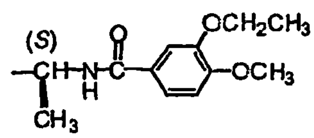 |
| 1028 | 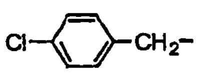 | 2 | 2 | 1 | - | H | 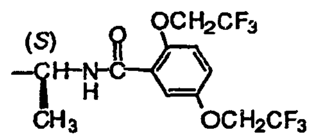 |
| 1029 | 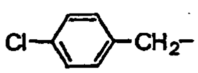 | 2 | 2 | 1 | - | H | 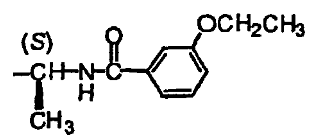 |
| 1030 | 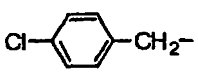 | 2 | 2 | 1 | - | H | 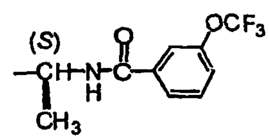 |
| 1031 | 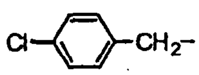 | 2 | 2 | 1 | - | H | 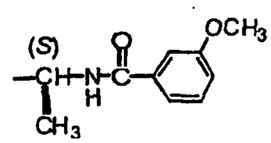 |
| 1032 | 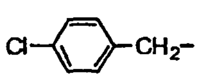 | 2 | 2 | 1 | - | H | 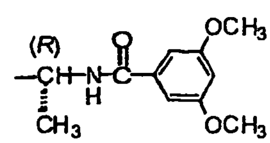 |
| 1033 | 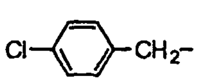 | 2 | 2 | 1 | - | H | 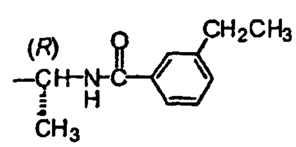 |

(continued)

| Compd. No. | 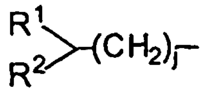 | k | m | n | chirality | R3 | 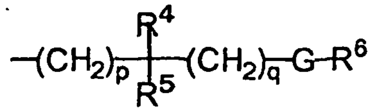 |
|---|---|---|---|---|---|---|---|
| 1034 | 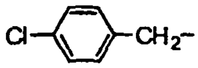 | 2 | 2 | 1 | - | H | 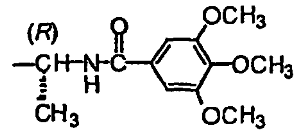 |

**Table 1.95**

| Compd. No. | 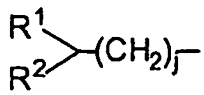 | k | m | n | chirality | R3 | 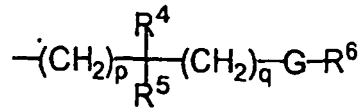 |
|---|---|---|---|---|---|---|---|
| 1035 | 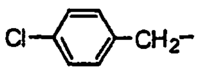 | 2 | 2 | 1 | - | H | 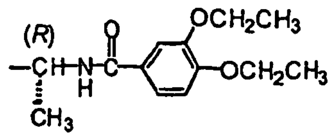 |
| 1036 | 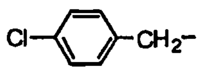 | 2 | 2 | 1 | - | H | 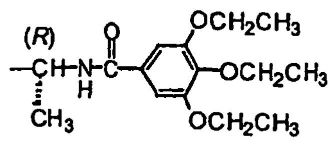 |
| 1037 | 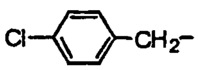 | 2 | 2 | 1 | - | H | 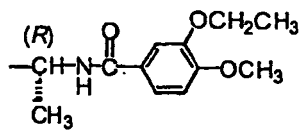 |
| 1038 | 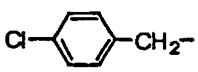 | 2 | 2 | 1 | - | H | 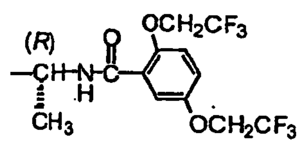 |
| 1039 | 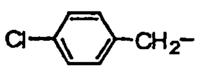 | 2 | 2 | 1 | - | H | 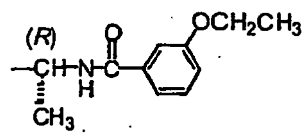 |
| 1040 | 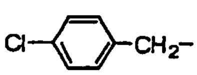 | 2 | 2 | 1 | - | H | 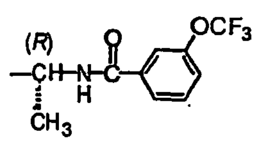 |
| 1041 | 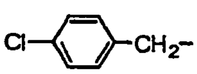 | 2 | 2 | 1 | - | H | 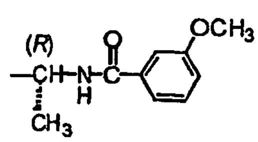 |

(continued)

| Compd. No. | 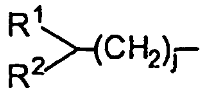 | k | m | n | chirality | $R^3$ | 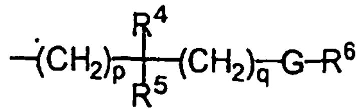 |
|---|---|---|---|---|---|---|---|
| 1042 | 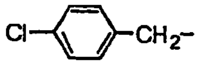 | 2 | 2 | 1 | - | H | 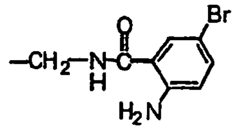 |
| 1043 | 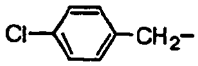 | 2 | 2 | 1 | - | H | 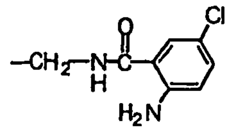 |
| 1044 | 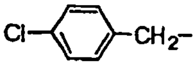 | 2 | 2 | 1 | - | H | 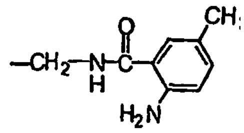 |
| 1045 | 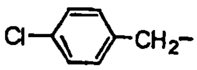 | 2 | 2 | 1 | - | H | 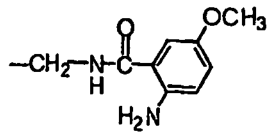 |

**Table 1.96**

| Compd. No. | 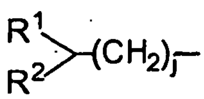 | k | m | n | chirality | $R^3$ | 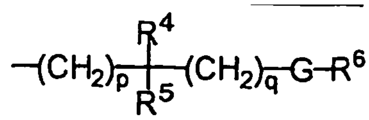 |
|---|---|---|---|---|---|---|---|
| 1046 | 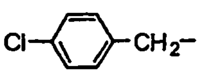 | 2 | 2 | 1 | - | H | 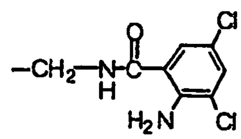 |
| 1047 | 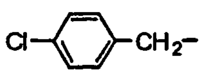 | 2 | 2 | 1 | - | H | 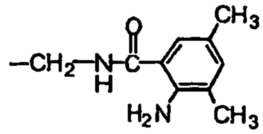 |
| 1048 | 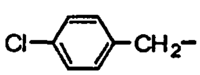 | 2 | 2 | 1 | - | H | 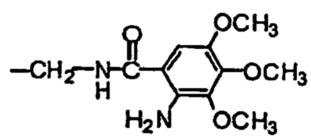 |
| 1049 | 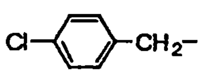 | 2 | 2 | 1 | - | H | 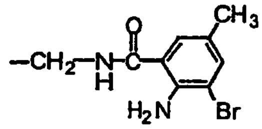 |

(continued)

| Compd. No. | 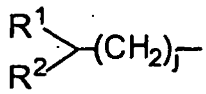 | k | m | n | chirality | $R^3$ | 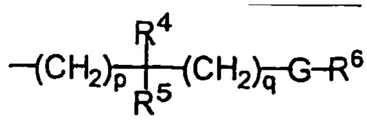 |
|---|---|---|---|---|---|---|---|
| 1050 | 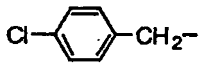 | 2 | 2 | 1 | - | H | 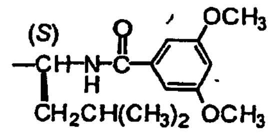 |
| 1051 | 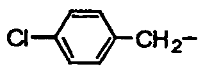 | 2 | 2 | 1 | - | H | 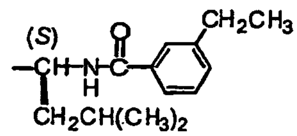 |
| 1052 | 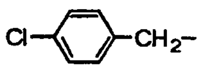 | 2 | 2 | 1 | - | H | 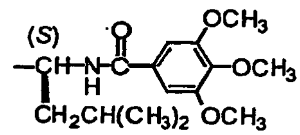 |
| 1053 | 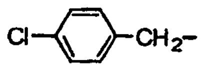 | 2 | 2 | 1 | - | H | 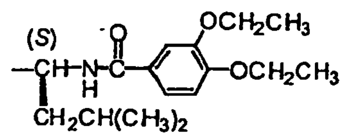 |
| 1054 | 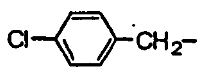 | 2 | 2 | 1 | - | H | 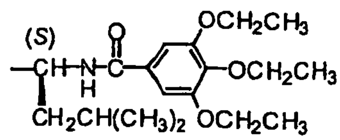 |
| 1055 | 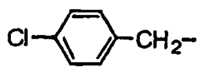 | 2 | 2 | 1 | - | H | 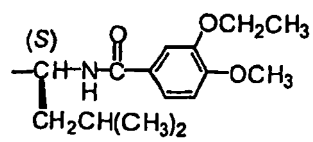 |
| 1056 | 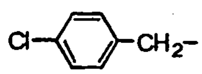 | 2 | 2 | 1 | - | H | 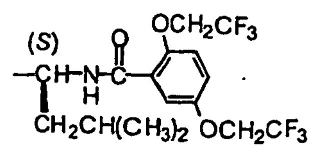 |

**Table 1.97**

| Compd. No. | 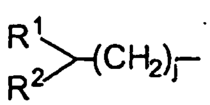 | k | m | n | chirality | $R^3$ | 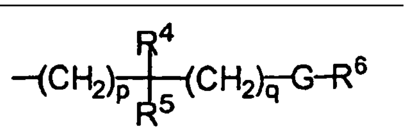 |
|---|---|---|---|---|---|---|---|
| 1057 | 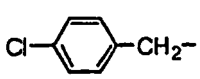 | 2 | 2 | 1 | - | H | 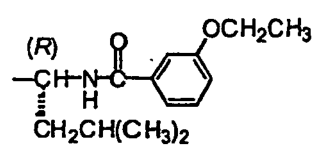 |

(continued)

| Compd. No. | 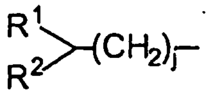 | k | m | n | chirality | $R^3$ | 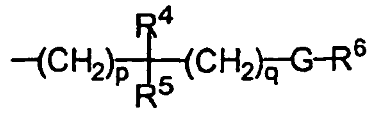 |
|---|---|---|---|---|---|---|---|
| 1058 | 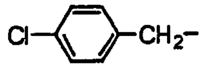 | 2 | 2 | 1 | - | H | 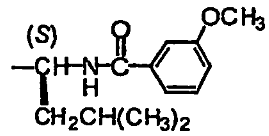 |
| 1059 | 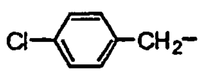 | 2 | 2 | 1 | - | H | 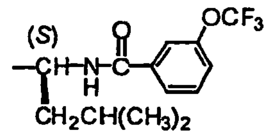 |
| 1060 | 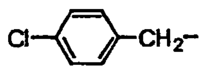 | 2 | 2 | 1 | - | H | 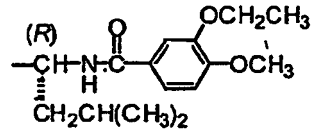 |
| 1061 | 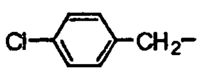 | 2 | 2 | 1 | - | H | 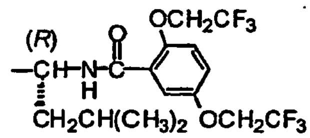 |
| 1062 | 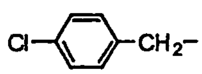 | 2 | 2 | 1 | - | H | 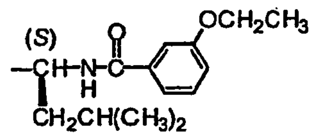 |
| 1063 | 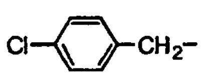 | 2 | 2 | 1 | - | H | 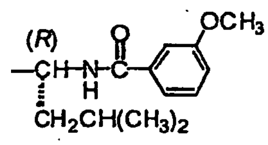 |
| 1064 | 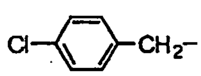 | 2 | 2 | 1 | - | H | 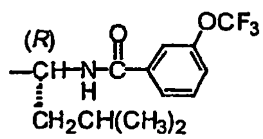 |
| 1065 | 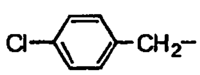 | 2 | 2 | 1 | - | H | 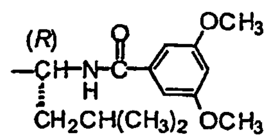 |
| 1066 | 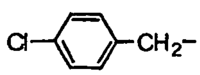 | 2 | 2 | 1 | - | H | 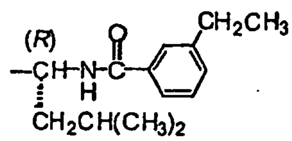 |
| 1067 | 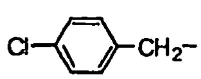 | 2 | 2 | 1 | - | H | 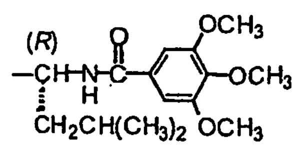 |

**Table 1.98**

| Compd. No. | 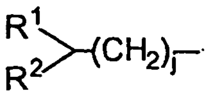 | k | m | n | chirality | $R^3$ | 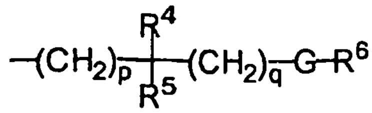 |
|---|---|---|---|---|---|---|---|
| 1068 | 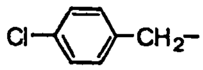 | 2 | 2 | 1 | - | H | 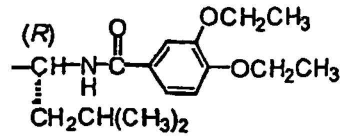 |
| 1069 | 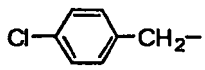 | 2 | 2 | 1 | - | H | 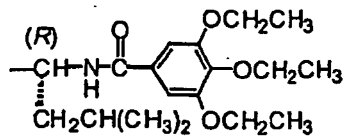 |
| 1070 | 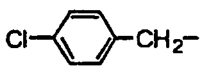 | 2 | 2 | 1 | - | H | 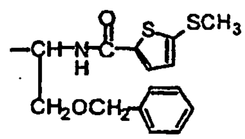 |
| 1071 | 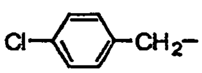 | 2 | 2 | 1 | - | H | 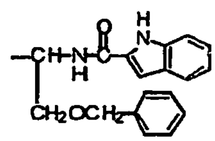 |
| 1072 | 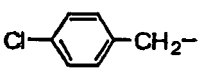 | 2 | 2 | 1 | - | H | 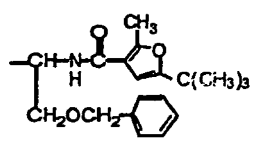 |
| 1073 | 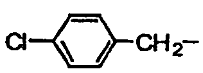 | 2 | 2 | 1 | - | H | 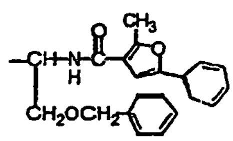 |
| 1074 | 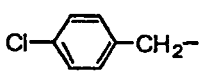 | 2 | 2 | 1 | - | H | 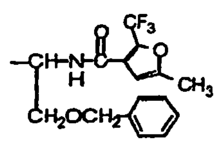 |
| 1075 | 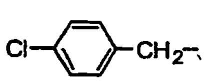 | 2 | 2 | 1 | - | H | 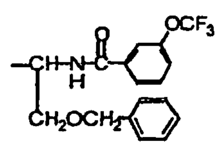 |
| 1076 | 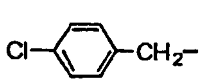 | 2 | 2 | 1 | - | H | 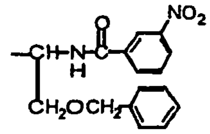 |
| 1077 | 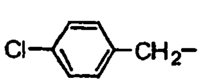 | 2 | 2 | 1 | - | H | 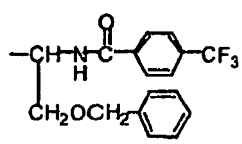 |

(continued)

| Compd. No. | 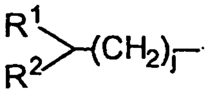 | k | m | n | chirality | $R^3$ | 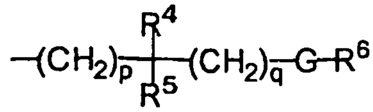 |
|---|---|---|---|---|---|---|---|
| 1078 | 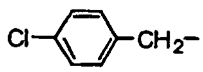 | 2 | 2 | 1 | - | H | 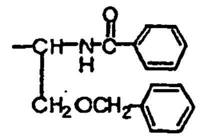 |

**Table 1.99**

| Compd. No. | 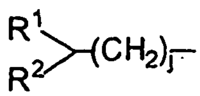 | k | m | n | chirality | $R^3$ | 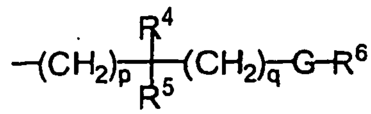 |
|---|---|---|---|---|---|---|---|
| 1079 | 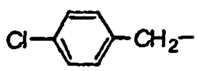 | 2 | 2 | 1 | - | H | 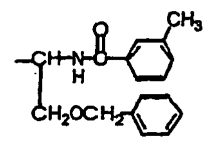 |
| 1080 | 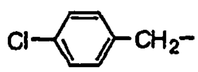 | 2 | 2 | 1 | - | H | 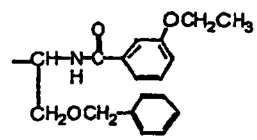 |
| 1081 | 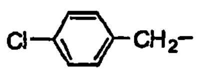 | 2 | 2 | 1 | - | H | 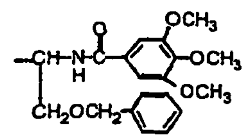 |
| 1082 | 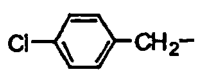 | 2 | 2 | 1 | - | H | 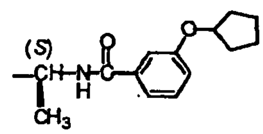 |
| 1083 | 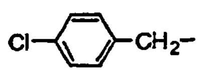 | 2 | 2 | 1 | - | H | 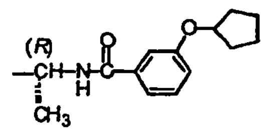 |
| 1084 | 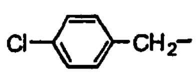 | 1 | 2 | 0 | R | H | 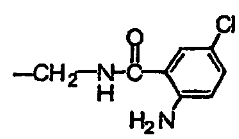 |
| 1085 | 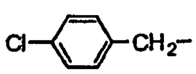 | 1 | 2 | 0 | R | H | 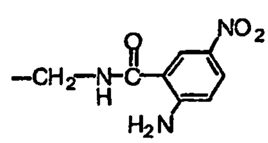 |
| 1086 | 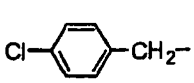 | 1 | 2 | 0 | R | H | 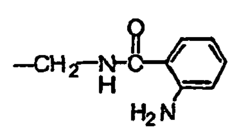 |

(continued)

| Compd. No. | 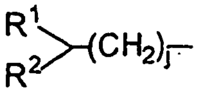 | k | m | n | chirality | $R^3$ | 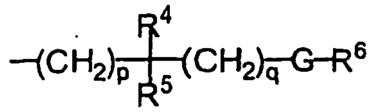 |
|---|---|---|---|---|---|---|---|
| 1087 | 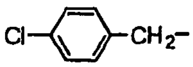 | 1 | 2 | 0 | R | H | 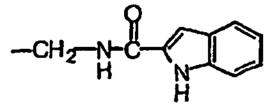 |
| 1088 | 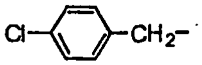 | 1 | 2 | 0 | R | H | 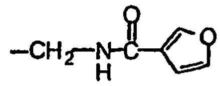 |
| 1089 | 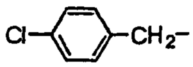 | 1 | 2 | 0 | R | H | 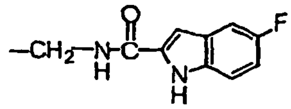 |

**Table 1.100**

| Compd. No. | 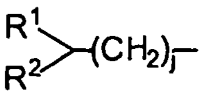 | k | m | n | chirality | $R^3$ | 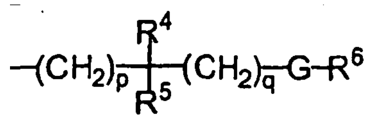 |
|---|---|---|---|---|---|---|---|
| 1090 | 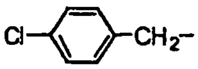 | 1 | 2 | 0 | R | H | 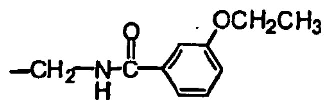 |
| 1091 | 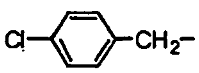 | 1 | 2 | 0 | R | H | 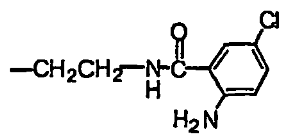 |
| 1092 | 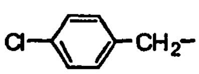 | 1 | 2 | 0 | R | H | 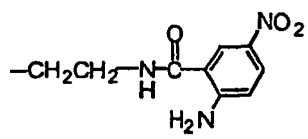 |
| 1093 | 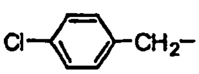 | 1 | 2 | 0 | R | H | 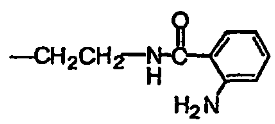 |
| 1094 | 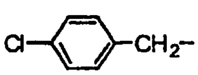 | 1 | 2 | 0 | R | H | 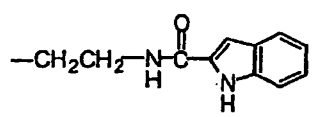 |
| 1095 | 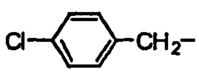 | 1 | 2 | 0 | R | H | 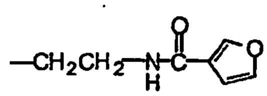 |

(continued)

| Compd. No. | 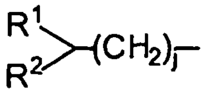 | k | m | n | chirality | $R^3$ | 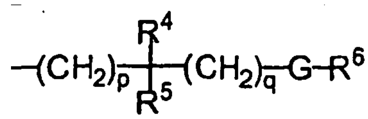 |
|---|---|---|---|---|---|---|---|
| 1096 | 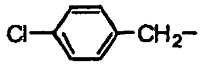 | 1 | 2 | 0 | R | H | 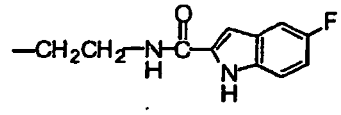 |
| 1097 | 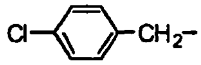 | 1 | 2 | 0 | R | H | 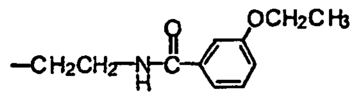 |
| 1098 | 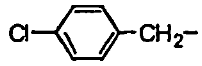 | 1 | 2 | 0 | R | H | 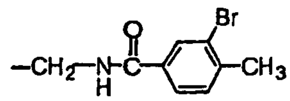 |
| 1099 | 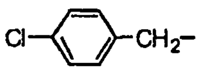 | 1 | 2 | 0 | R | H | 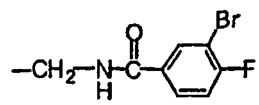 |
| 1100 | 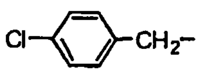 | 1 | 2 | 0 | R | H | 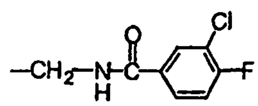 |

**Table 1.101**

| Compd. No. | 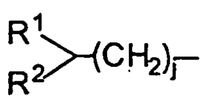 | k | m | n | chirality | $R^3$ | 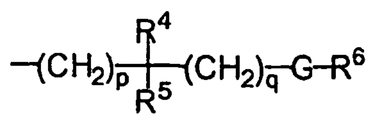 |
|---|---|---|---|---|---|---|---|
| 1101 | 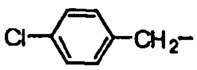 | 1 | 2 | 0 | R | H | 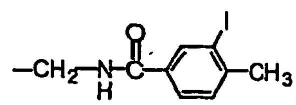 |
| 1102 | 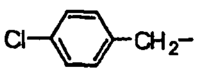 | 1 | 2 | 0 | R | H | 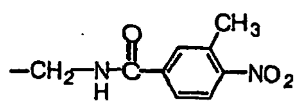 |
| 1103 | 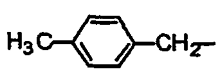 | 1 | 2 | 0 | R | H | 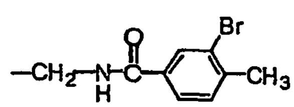 |
| 1104 | 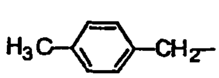 | 1 | 2 | 0 | R | H | 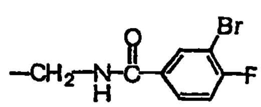 |

(continued)

| Compd. No. | 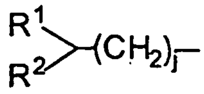 | k | m | n | chirality | $R^3$ | 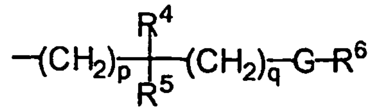 |
|---|---|---|---|---|---|---|---|
| 1105 | 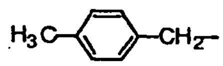 | 1 | 2 | 0 | R | H | 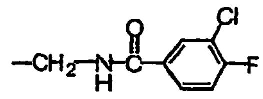 |
| 1106 | 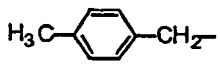 | 1 | 2 | 0 | R | H | 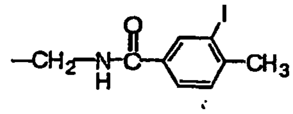 |
| 1107 | 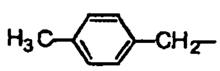 | 1 | 2 | 0 | R | H | 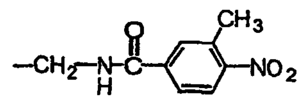 |
| 1108 | 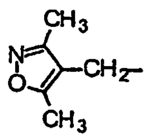 | 1 | 2 | 0 | R | H | 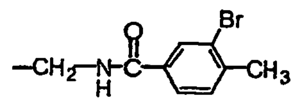 |
| 1109 | 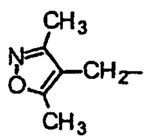 | 1 | 2 | 0 | R | H | 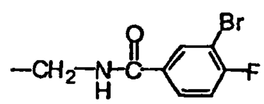 |
| 1110 | 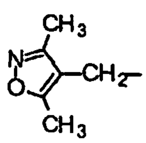 | 1 | 2 | 0 | R | H | 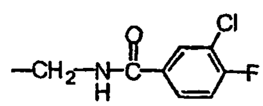 |
| 1111 | 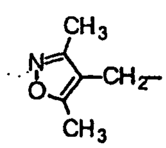 | 1 | 2 | 0 | R | H | 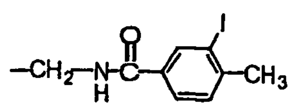 |

**Table 1.102**

| Compd. No. | 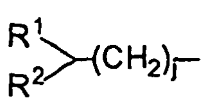 | k | m | n | chirality | $R^3$ | 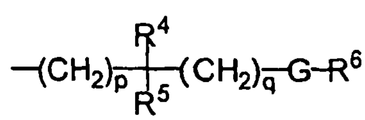 |
|---|---|---|---|---|---|---|---|
| 1112 | 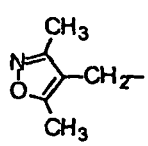 | 1 | 2 | 0 | R | H | 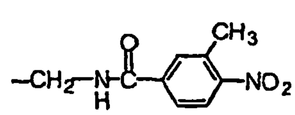 |

(continued)

| Compd. No. | 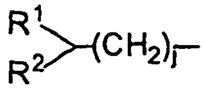 | k | m | n | chirality | $R^3$ | 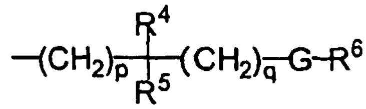 |
|---|---|---|---|---|---|---|---|
| 1113 | 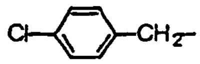 | 2 | 2 | 1 | - | H | 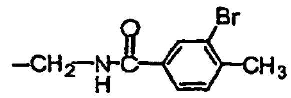 |
| 1114 | 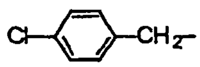 | 2 | 2 | 1 | - | H | 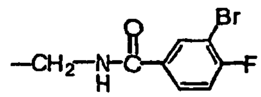 |
| 1115 | 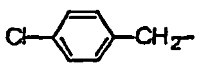 | 2 | 2 | 1 | - | H | 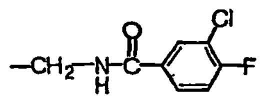 |
| 1116 | 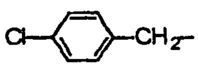 | 2 | 2 | 1 | - | H | 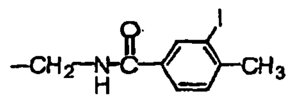 |
| 1117 | 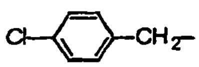 | 2 | 2 | 1 | - | H | 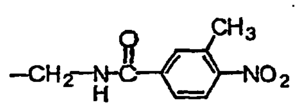 |
| 1118 | 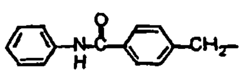 | 1 | 2 | 0 | R | H | 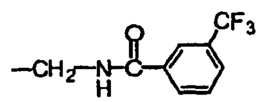 |
| 1119 | 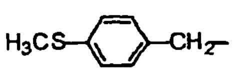 | 1 | 2 | 0 | R | H | 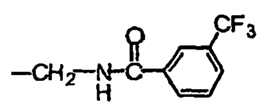 |
| 1120 | 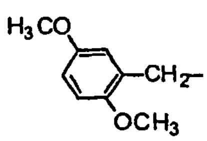 | 1 | 2 | 0 | R | H | 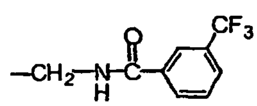 |
| 1121 | 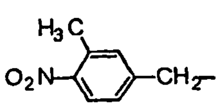 | 1 | 2 | 0 | R | H | 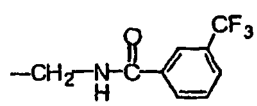 |
| 1122 | 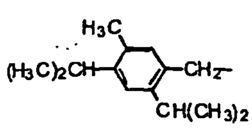 | 1 | 2 | 0 | R | H | 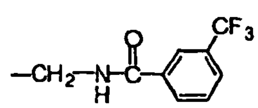 |

**Table 1.103**

| Compd. No. | 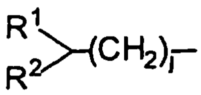 | k | m | n | chirality | $R^3$ | 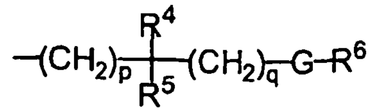 |
|---|---|---|---|---|---|---|---|
| 1123 | 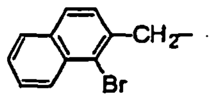 | 1 | 2 | 0 | R | H | 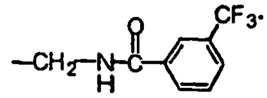 |
| 1124 | 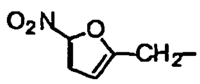 | 1 | 2 | 0 | R | H | 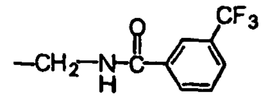 |
| 1125 | 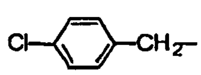 | 2 | 2 | 1 | - | H | 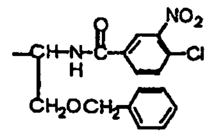 |
| 1126 | 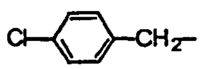 | 2 | 2 | 1 | - | H | 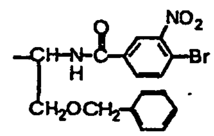 |
| 1127 | 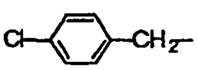 | 2 | 2 | 1 | - | H | 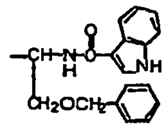 |
| 1128 | 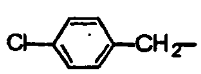 | 2 | 2 | 1 | - | H | 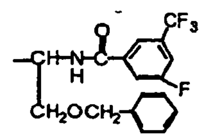 |
| 1129 | 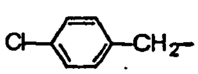 | 2 | 2 | 1 | - | H | 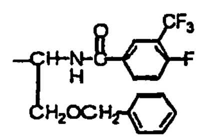 |
| 1130 | 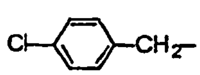 | 2 | 2 | 1 | - | H | 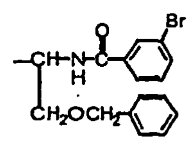 |
| 1131 | 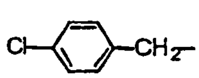 | 2 | 2 | 1 | - | H | 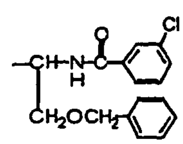 |
| 1132 | 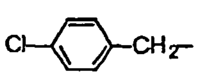 | 2 | 2 | 1 | - | H | 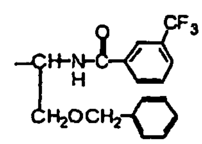 |

(continued)

| Compd. No. | 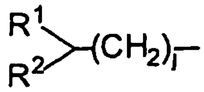 | k | m | n | chirality | $R^3$ | 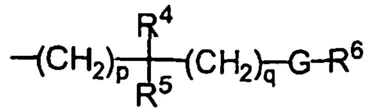 |
|---|---|---|---|---|---|---|---|
| 1133 | 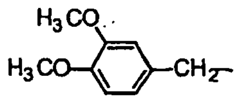 | 1 | 2 | 0 | R | H | 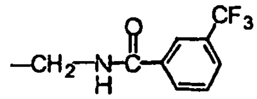 |

**Table 1.104**

| Compd. No. | 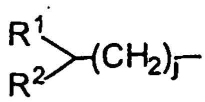 | k | m | n | chirality | $R^3$ | 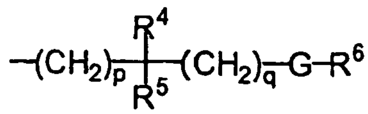 |
|---|---|---|---|---|---|---|---|
| 1134 | 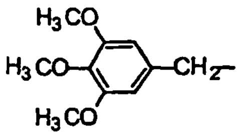 | 1 | 2 | 0 | R | H | 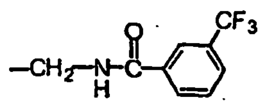 |
| 1135 | 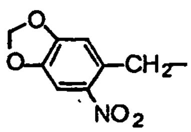 | 1 | 2 | 0 | R | H | 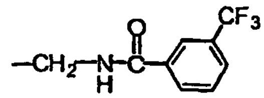 |
| 1136 | 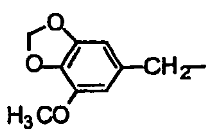 | 1 | 2 | 0 | R | H | 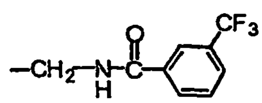 |
| 1137 | 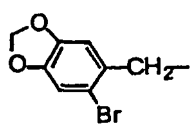 | 1 | 2 | 0 | R | H | 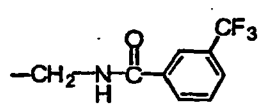 |
| 1138 | 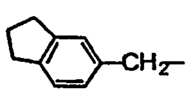 | 1 | 2 | 0 | R | H | 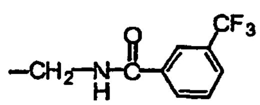 |
| 1139 | 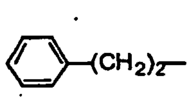 | 1 | 2 | 0 | R | H | 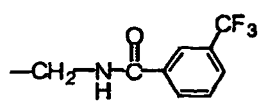 |
| 1140 | 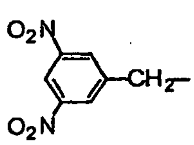 | 1 | 2 | 0 | R | H | 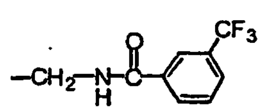 |
| 1141 | 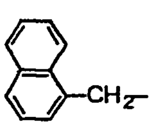 | 1 | 2 | 0 | R | H | 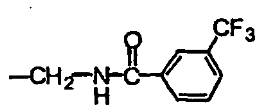 |

(continued)

| Compd. No. | 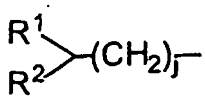 | k | m | n | chirality | $R^3$ | 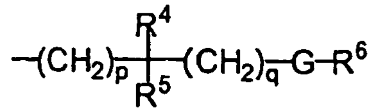 |
|---|---|---|---|---|---|---|---|
| 1142 | 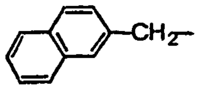 | 1 | 2 | 0 | R | H | 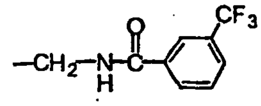 |
| 1143 | 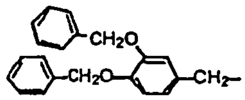 | 1 | 2 | 0 | R | H | 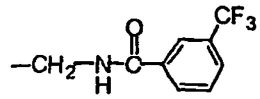 |
| 1144 | 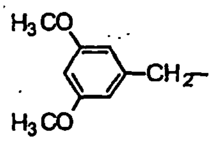 | 1 | 2 | 0 | R | H | 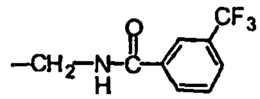 |

**Table 1.105**

| Compd. No. | 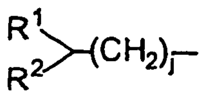 | k | m | n | chirality | $R^3$ | 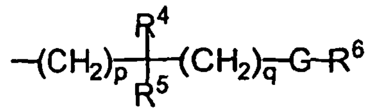 |
|---|---|---|---|---|---|---|---|
| 1145 | 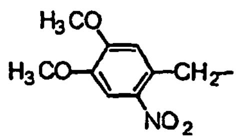 | 1 | 2 | 0 | R | H | 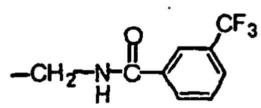 |
| 1146 | 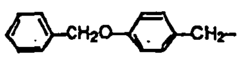 | 1 | 2 | 0 | R | H | 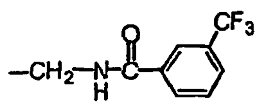 |
| 1147 | 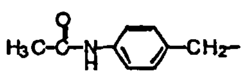 | 1 | 2 | 0 | R | H | 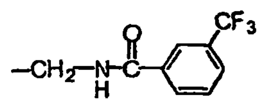 |
| 1148 | 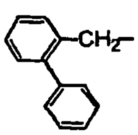 | 1 | 2 | 0 | R | H | 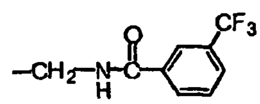 |
| 1149 | 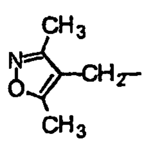 | 1 | 2 | 0 | R | H | 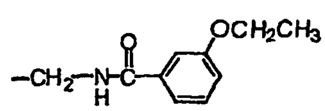 |
| 1150 | 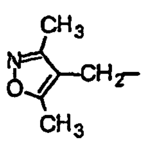 | 1 | 2 | 0 | R | H | 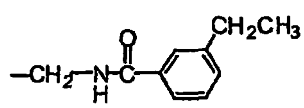 |

(continued)

| Compd. No. | 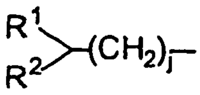 | k | m | n | chirality | $R^3$ | 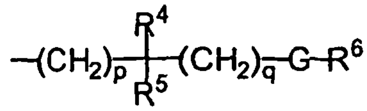 |
|---|---|---|---|---|---|---|---|
| 1151 | 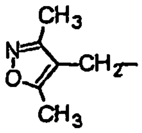 | 1 | 2 | 0 | R | H | 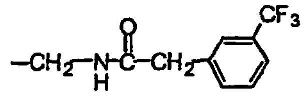 |
| 1152 | 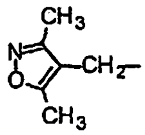 | 1 | 2 | 0 | R | H | 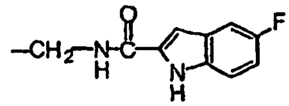 |
| 1153 | 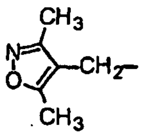 | 1 | 2 | 0 | R | H | 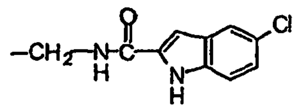 |
| 1154 | 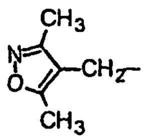 | 1 | 2 | 0 | R | H | 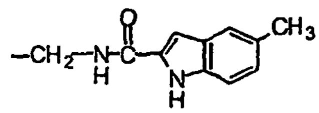 |
| 1155 | 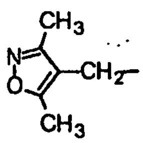 | 1 | 2 | 0 | R | H | 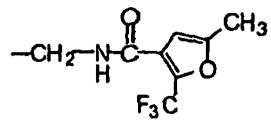 |

**Table 1.106**

| Compd. No. | 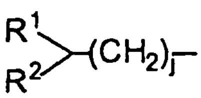 | k | m | n | chirality | $R^3$ | 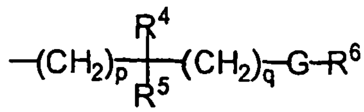 |
|---|---|---|---|---|---|---|---|
| 1156 | 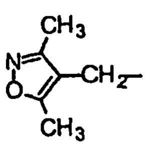 | 1 | 2 | 0 | R | H | 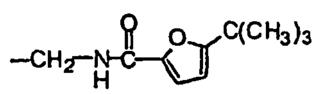 |
| 1157 | 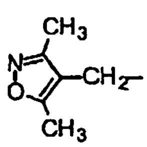 | 1 | 2 | 0 | R | H | 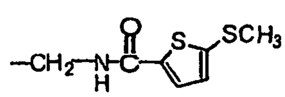 |
| 1158 | 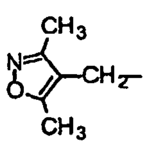 | 1 | 2 | 0 | R | H | 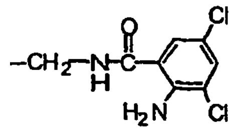 |

(continued)

| Compd. No. | 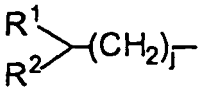 | k | m | n | chirality | $R^3$ | 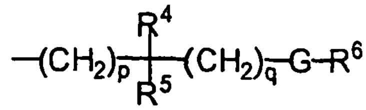 |
|---|---|---|---|---|---|---|---|
| 1159 | 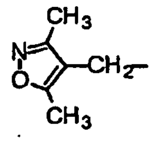 | 1 | 2 | 0 | R | H | 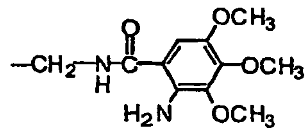 |
| 1160 | 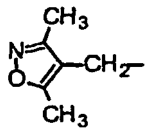 | 1 | 2 | 0 | R | H | 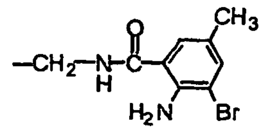 |
| 1161 | 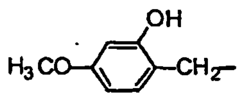 | 1 | 2 | 0 | R | H | 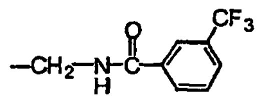 |
| 1162 | 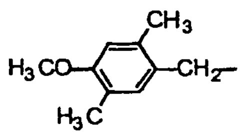 | 1 | 2 | 0 | R | H | 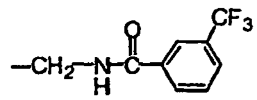 |
| 1163 | 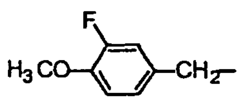 | 1 | 2 | 0 | R | H | 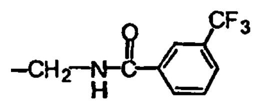 |
| 1164 | 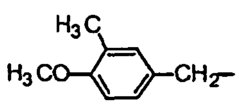 | 1 | 2 | 0 | R | H | 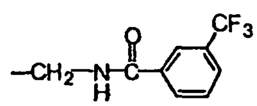 |
| 1165 | 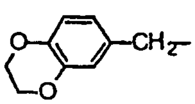 | 1 | 2 | 0 | R | H | 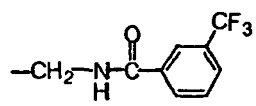 |
| 1166 | 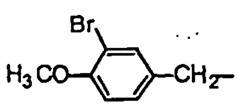 | 1 | 2 | 0 | R | H | 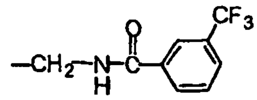 |

**Table 1.107**

| Compd. No. | 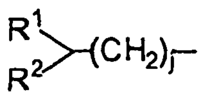 | k | m | n | chirality | $R^3$ | 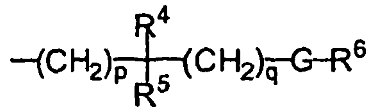 |
|---|---|---|---|---|---|---|---|
| 1167 | 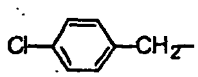 | 2 | 2 | 1 | - | H | 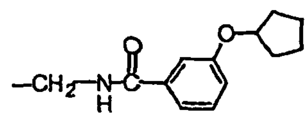 |
| 1168 | 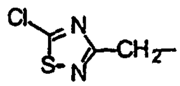 | 1 | 2 | 0 | R | H | 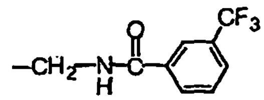 |
| 1169 | 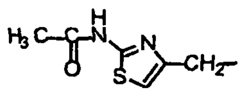 | 1 | 2 | 0 | R | H | 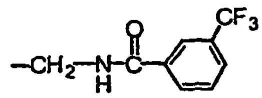 |
| 1170 | 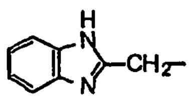 | 1 | 2 | | R | H | 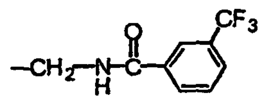 |
| 1171 | 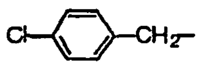 | 1 | 2 | 0 | R | H | 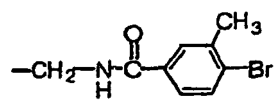 |
| 1172 | 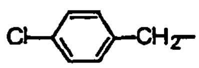 | 1 | 2 | 0 | R | H | 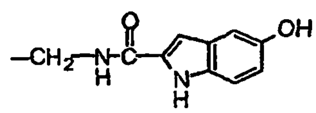 |
| 1173 | 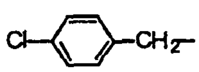 | 1 | 2 | 0 | R | H | 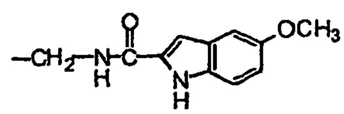 |
| 1174 | 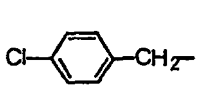 | 1 | 2 | 0 | R | H | 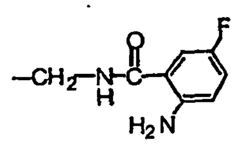 |
| 1175 | 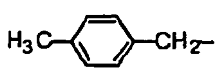 | 1 | 2 | 0 | R | H | 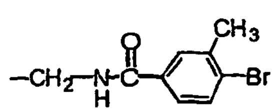 |
| 1176 | 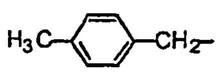 | 1 | 2 | 0 | R | H | 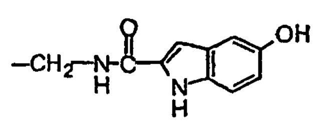 |

(continued)

| Compd. No. | 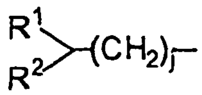 | k | m | n | chirality | $R^3$ | 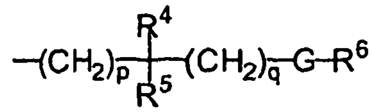 |
|---|---|---|---|---|---|---|---|
| 1177 | 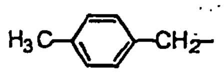 | 1 | 2 | 0 | R | H | 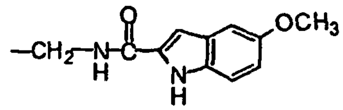 |

**Table 1.108**

| Compd. No. | 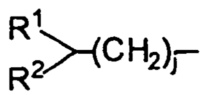 | k | m | n | chirality | $R^3$ | 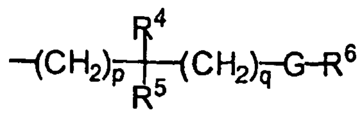 |
|---|---|---|---|---|---|---|---|
| 1178 | 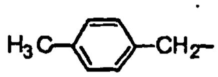 | 1 | 2 | 0 | R | H | 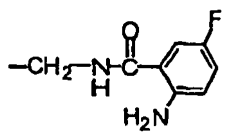 |
| 1179 | 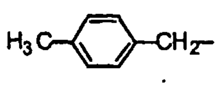 | 1 | 2 | 0 | R | H | 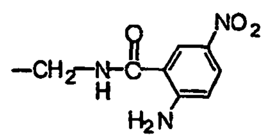 |
| 1180 | 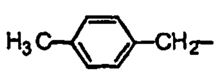 | 1 | 2 | 0 | R | H | 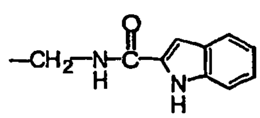 |
| 1181 | 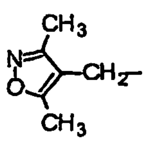 | 1 | 2 | 0 | R | H | 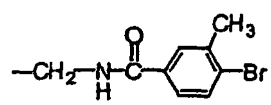 |
| 1182 | 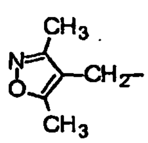 | 1 | 2 | 0 | R | H | 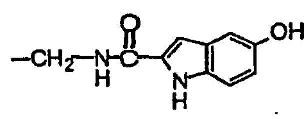 |
| 1183 | 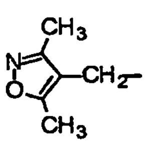 | 1 | 2 | 0 | R | H | 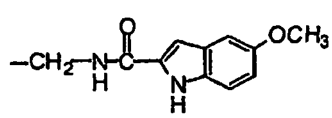 |
| 1184 | 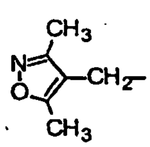 | 1 | 2 | 0 | R | H | 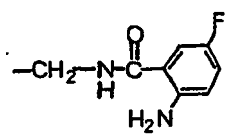 |

(continued)

| Compd. No. | 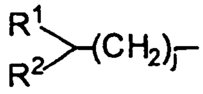 | k | m | n | chirality | $R^3$ | 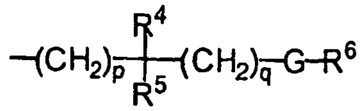 |
|---|---|---|---|---|---|---|---|
| 1185 | 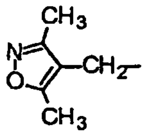 | 1 | 2 | 0 | R | H | 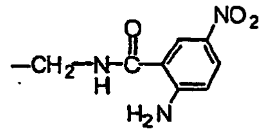 |
| 1186 | 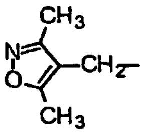 | 1 | 2 | 0 | R | H | 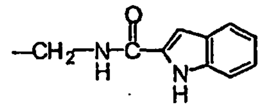 |
| 1187 | 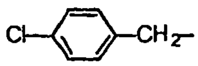 | 2 | 2 | 1 | - | H | 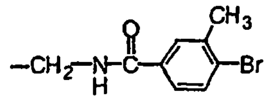 |
| 1188 | 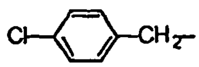 | 2 | 2 | 1 | - | H | 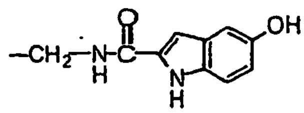 |

**Table 1.109**

| Compd. No. | 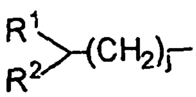 | k | m | n | chirality | $R^3$ | 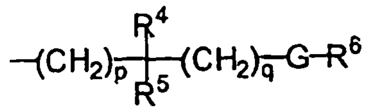 |
|---|---|---|---|---|---|---|---|
| 1189 | 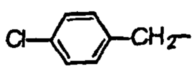 | 2 | 2 | 1 | - | H | 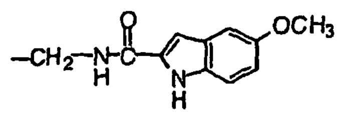 |
| 1190 | 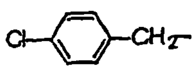 | 2 | 2 | 1 | - | H | 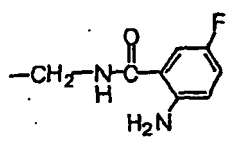 |
| 1191 | 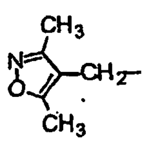 | 1 | 2 | 0 | R | H | 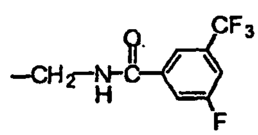 |
| 1192 | 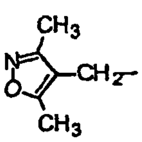 | 1 | 2 | 0 | R | H | 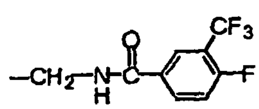 |

(continued)

| Compd. No. | 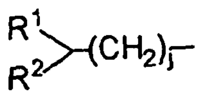 | k | m | n | chirality | $R^3$ | 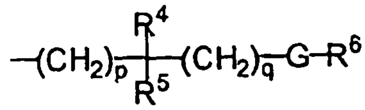 |
|---|---|---|---|---|---|---|---|
| 1193 | 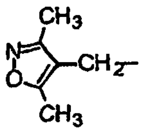 | 1 | 2 | 0 | R | H | 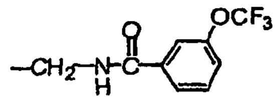 |
| 1194 | 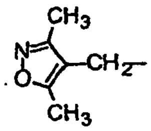 | 1 | 2 | 0 | R | H | 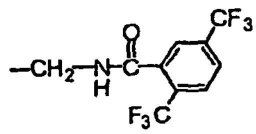 |
| 1195 | 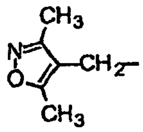 | 1 | 2 | 0 | R | H | 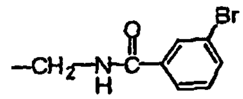 |
| 1196 | 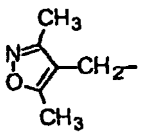 | 1 | 2 | 0 | R | H | 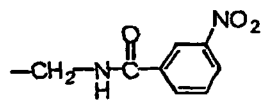 |
| 1197 | 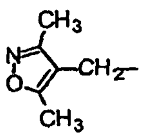 | 1 | 2 | 0 | R | H | 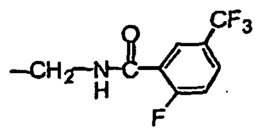 |
| 1198 | 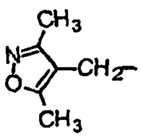 | 1 | 2 | 0 | R | H | 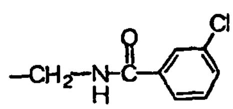 |
| 1199 | 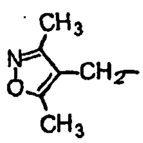 | 1 | 2 | 0 | R | H | 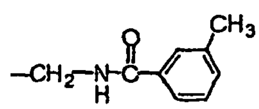 |

**Table 1.110**

| Compd. No. | 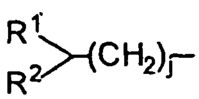 | k | m | n | chirality | $R^3$ | 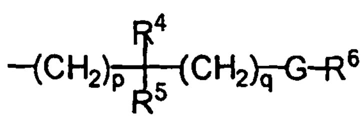 |
|---|---|---|---|---|---|---|---|
| 1200 | 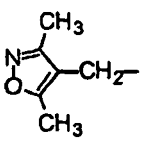 | 1 | 2 | 0 | R | H | 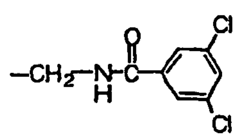 |

(continued)

| Compd. No. | 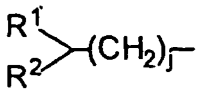 | k | m | n | chirality | $R^3$ | 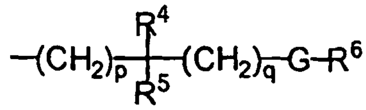 |
|---|---|---|---|---|---|---|---|
| 1201 | 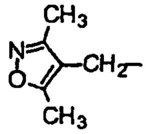 | 1 | 2 | 0 | R | H | 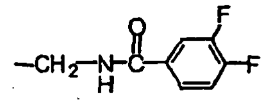 |
| 1202 | 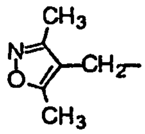 | 1 | 2 | 0 | R | H | 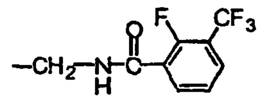 |
| 1203 | 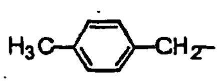 | 1 | 2 | 0 | R | H | 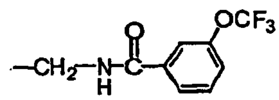 |
| 1204 | 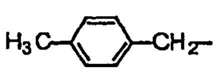 | 1 | 2 | 0 | R | H | 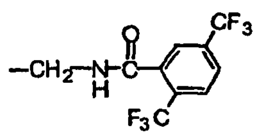 |
| 1205 | 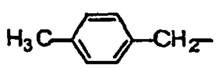 | 1 | 2 | 0 | R | H | 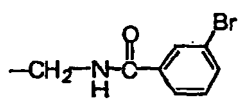 |
| 1206 | 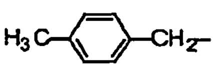 | 1 | 2 | 0 | R | H | 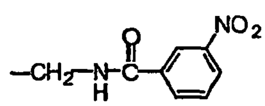 |
| 1207 | 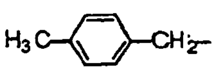 | 1 | 2 | 0 | R | H | 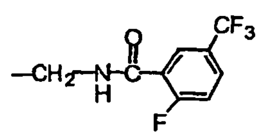 |
| 1208 | 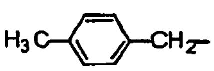 | 1 | 2 | 0 | R | H | 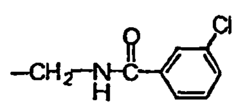 |
| 1209 | 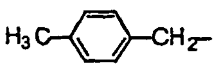 | 1 | 2 | 0 | R | H | 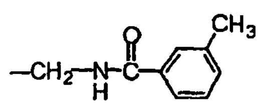 |
| 1210 | 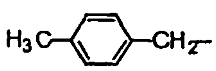 | 1 | 2 | 0 | R | H | 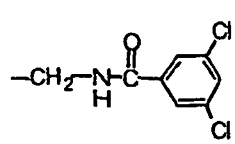 |

**Table 1.111**

| Compd. No. | 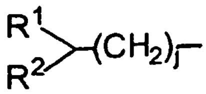 | k | m | n | chirality | $R^3$ | 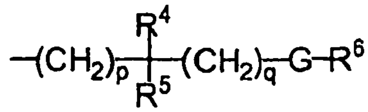 |
|---|---|---|---|---|---|---|---|
| 1211 | 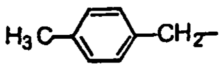 | 1 | 2 | 0 | R | H | 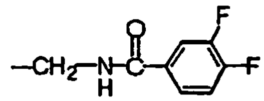 |
| 1212 | 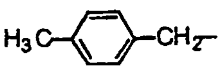 | 1 | 2 | 0 | R | H | 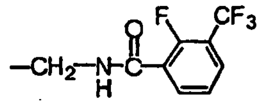 |
| 1213 | 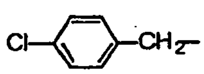 | 2 | 2 | 1 | - | H | 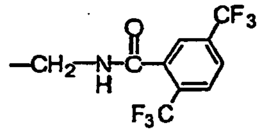 |
| 1214 | 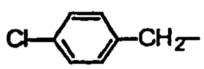 | 2 | 2 | 1 | - | H | 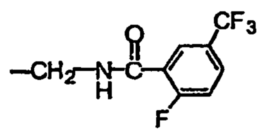 |
| 1215 | 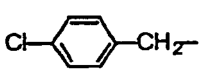 | 2 | 2 | 1 | - | H | 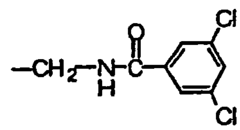 |
| 1216 | 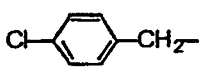 | 2 | 2 | 1 | - | H | 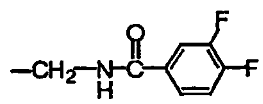 |
| 1217 | 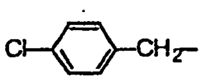 | 1 | 2 | 0 | R | H | 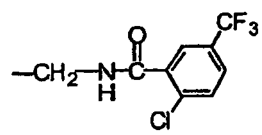 |
| 1218 | 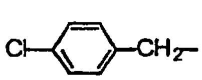 | 1 | 2 | 0 | R | H | 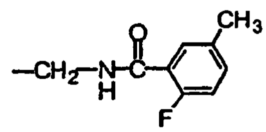 |
| 1219 | 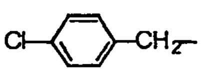 | 1 | 2 | 0 | R | H | 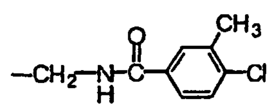 |
| 1220 | 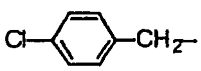 | 1 | 2 | 0 | R | H | 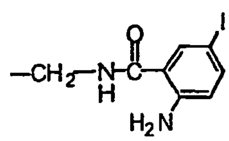 |

(continued)

| Compd. No. | 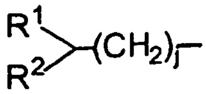 | k | m | n | chirality | $R^3$ | 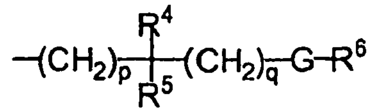 |
|---|---|---|---|---|---|---|---|
| 1221 | 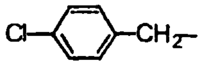 | 1 | 2 | 0 | R | H | 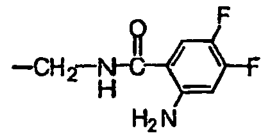 |

**Table 1.112**

| Compd. No. | 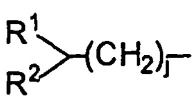 | k | m | n | chirality | $R^3$ | 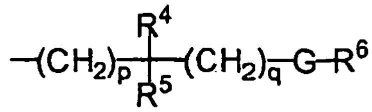 |
|---|---|---|---|---|---|---|---|
| 1222 | 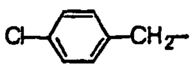 | 1 | 2 | 0 | R | H | 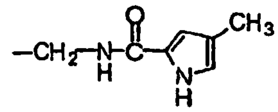 |
| 1223 | 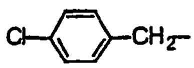 | 1 | 2 | 0 | R | H | 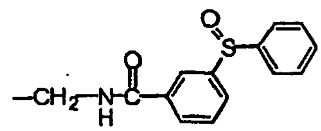 |
| 1224 | 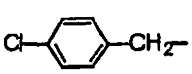 | 1 | 2 | 0 | R | H | 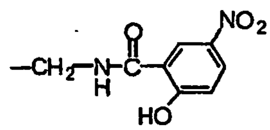 |
| 1225 | 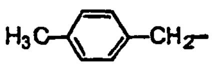 | 1 | 2 | 0 | R | H | 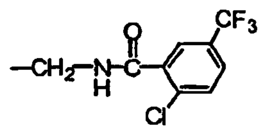 |
| 1226 | 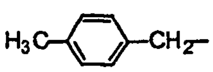 | 1 | 2 | 0 | R | H | 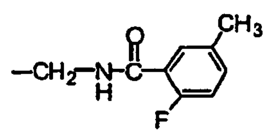 |
| 1227 | 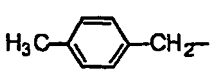 | 1 | 2 | 0 | R | H | 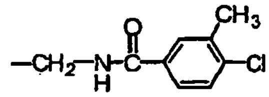 |
| 1228 | 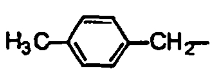 | 1 | 2 | 0 | R | H | 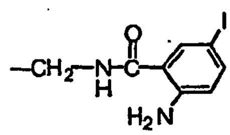 |

(continued)

| Compd. No. | 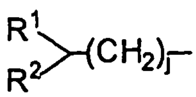 | k | m | n | chirality | $R^3$ | 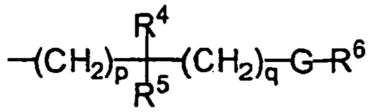 |
|---|---|---|---|---|---|---|---|
| 1229 | 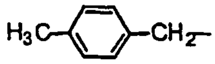 | 1 | 2 | 0 | R | H | 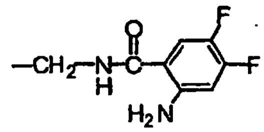 |
| 1230 | 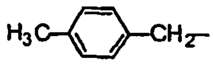 | 1 | 2 | 0 | R | H | 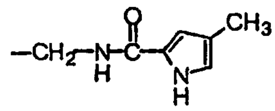 |
| 1231 | 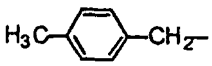 | 1 | 2 | 0 | R | H | 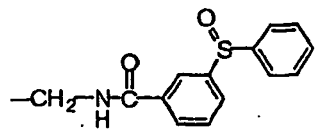 |
| 1232 | 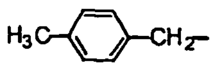 | 1 | 2 | 0 | R | H | 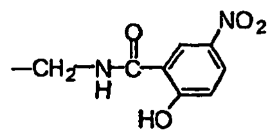 |

**Table 1.113**

| Compd. No. | 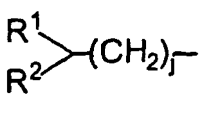 | k | m | n | chirality | $R^3$ | 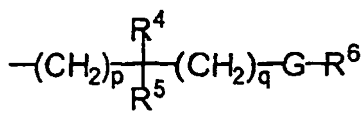 |
|---|---|---|---|---|---|---|---|
| 1233 | 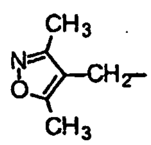 | 1 | 2 | 0 | R | H | 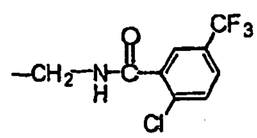 |
| 1234 | 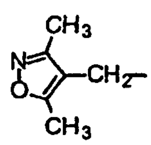 | 1 | 2 | 0 | R | H | 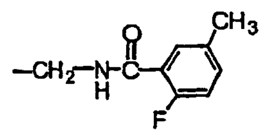 |
| 1235 | 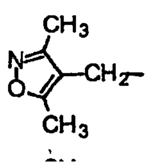 | 1 | 2 | 0 | R | H | 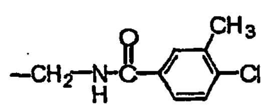 |
| 1236 | 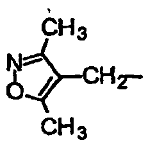 | 1 | 2 | 0 | R | H | 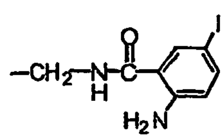 |

(continued)

| Compd. No. | 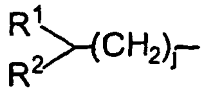 | k | m | n | chirality | $R^3$ | 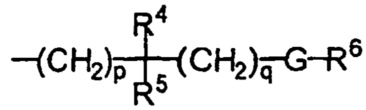 |
|---|---|---|---|---|---|---|---|
| 1237 | 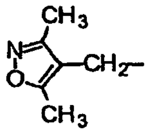 | 1 | 2 | 0 | R | H | 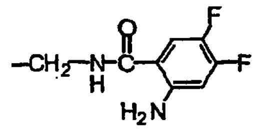 |
| 1238 | 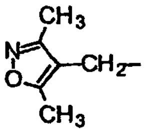 | 1 | 2 | 0 | R | H | 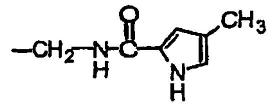 |
| 1239 | 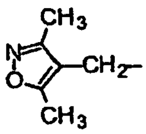 | 1 | 2 | 0 | R | H | 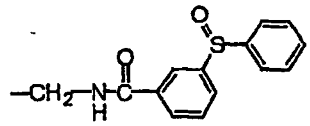 |
| 1240 | 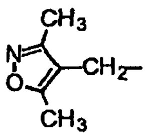 | 1 | 2 | 0 | R | H | 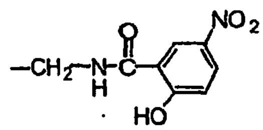 |
| 1241 | 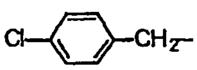 | 2 | 2 | 1 | - | H | 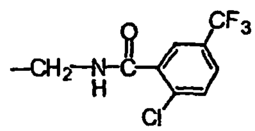 |
| 1242 | 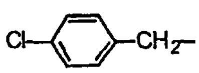 | 2 | 2 | 1 | - | H | 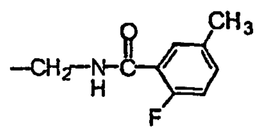 |
| 1243 | 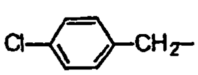 | 2 | 2 | 1 | - | H | 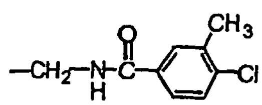 |

**Table 1.114**

| Compd. No. | 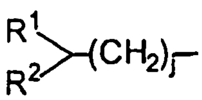 | k | m | n | chirality | $R^3$ | 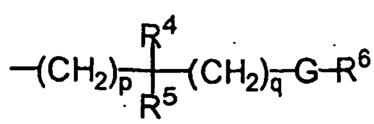 |
|---|---|---|---|---|---|---|---|
| 1244 | 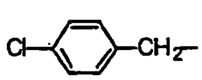 | 2 | 2 | 1 | - | H | 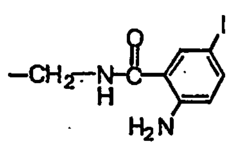 |

(continued)

| Compd. No. | 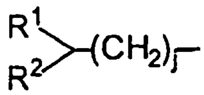 | k | m | n | chirality | $R^3$ | 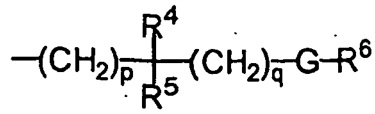 |
|---|---|---|---|---|---|---|---|
| 1245 | 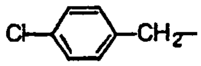 | 2 | 2 | 1 | - | H | 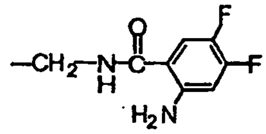 |
| 1246 | 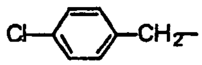 | 2 | 2 | 1 | - | H | 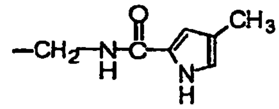 |
| 1247 | 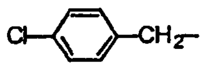 | 2 | 2 | 1 | - | H | 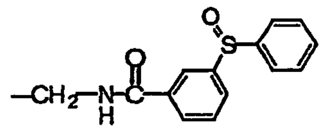 |
| 1248 | 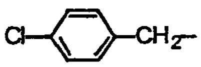 | 2 | 2 | 1 | - | H | 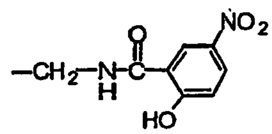 |
| 1249 | 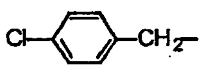 | 1 | 2 | 0 | R | H | 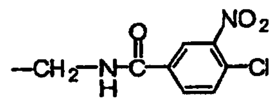 |
| 1250 | 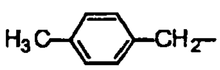 | 1 | 2 | 0 | R | H | 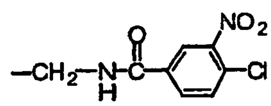 |
| 1251 | 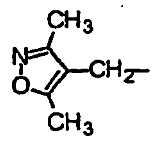 | 1 | 2 | 0 | R | H | 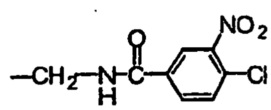 |
| 1252 | 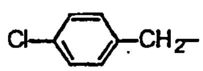 | 1 | 2 | 0 | R | H | 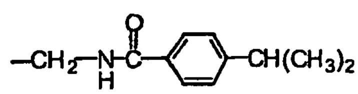 |
| 1253 | 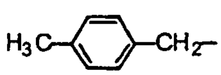 | 1 | 2 | 0 | R | H | 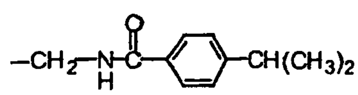 |
| 1254 | 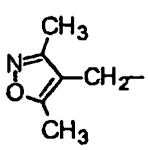 | 1 | 2 | 0 | R | H | 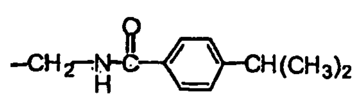 |

**Table 1.115**

| Compd. No. | 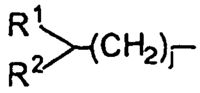 | k | m | n | chirality | $R^3$ | 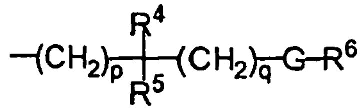 |
|---|---|---|---|---|---|---|---|
| 1255 | 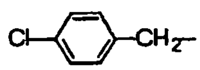 | 1 | 2 | 0 | R | H | 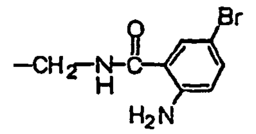 |
| 1256 | 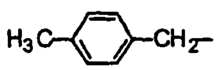 | 1 | 2 | 0 | R | H | 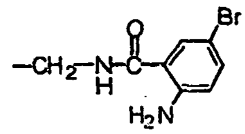 |
| 1257 | 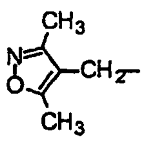 | 1 | 2 | 0 | R | H | 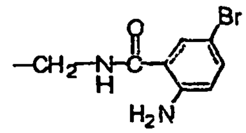 |
| 1258 | 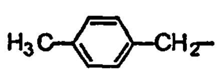 | 1 | 2 | 0 | R | H | 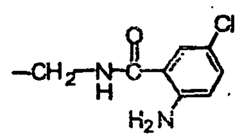 |
| 1259 | 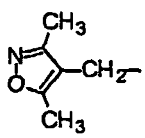 | 1 | 2 | 0 | R | H | 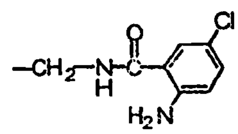 |
| 1260 | 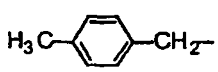 | 1 | 2 | 0 | R | H | 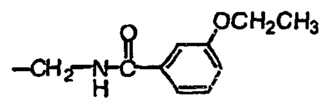 |
| 1261 | 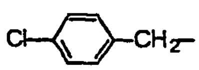 | 1 | 2 | 0 | R | H | 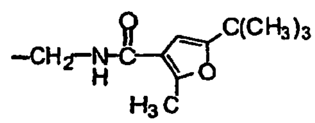 |
| 1262 | 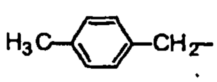 | 1 | 2 | 0 | R | H | 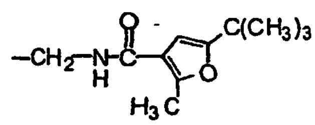 |
| 1263 | 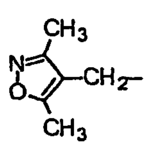 | 1 | 2 | 0 | R | H | 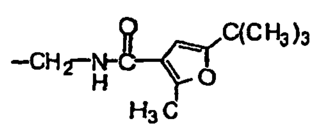 |
| 1264 | 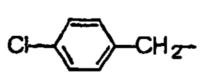 | 1 | 2 | 0 | R | H | 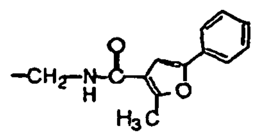 |

(continued)

| Compd. No. | 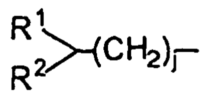 | k | m | n | chirality | $R^3$ | 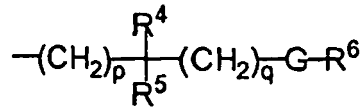 |
|---|---|---|---|---|---|---|---|
| 1265 | 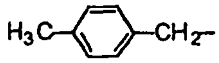 | 1 | 2 | 0 | R | H | 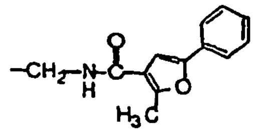 |

**Table 1.116**

| Compd. No. | 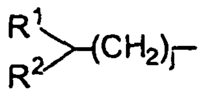 | k | m | n | chirality | $R^3$ | 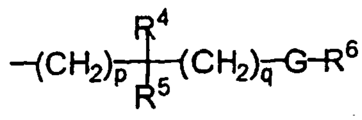 |
|---|---|---|---|---|---|---|---|
| 1266 | 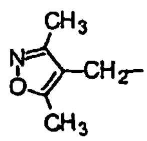 | 1 | 2 | 0 | R | H | 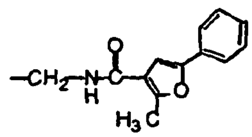 |
| 1267 | 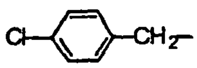 | 1 | 2 | 0 | R | H | 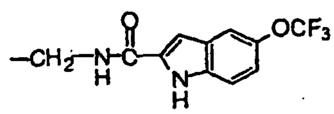 |
| 1268 | 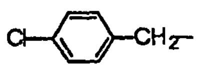 | 1 | 2 | 0 | R | H | 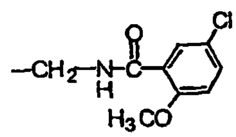 |
| 1269 | 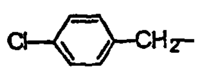 | 1 | 2 | 0 | R | H | 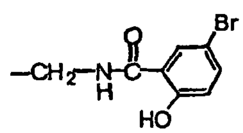 |
| 1270 | 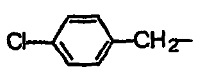 | 1 | 2 | 0 | R | H | 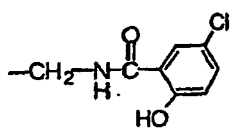 |
| 1271 | 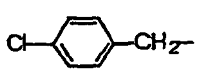 | 1 | 2 | 0 | R | H | 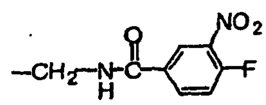 |
| 1272 | 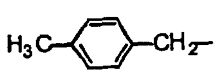 | 1 | 2 | 0 | R | H | 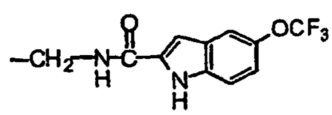 |
| 1273 | 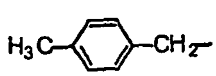 | 1 | 2 | 0 | R | H | 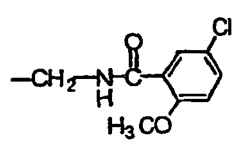 |

(continued)

| Compd. No. | 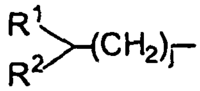 | k | m | n | chirality | $R^3$ | 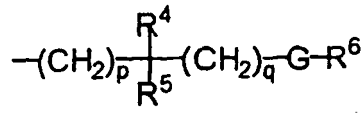 |
|---|---|---|---|---|---|---|---|
| 1274 | 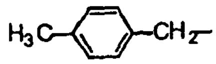 | 1 | 2 | 0 | R | H | 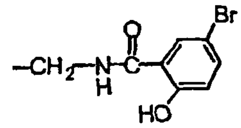 |
| 1275 | 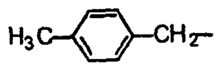 | 1 | 2 | 0 | R | H | 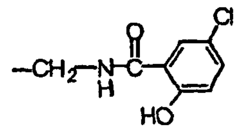 |
| 1276 | 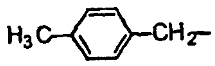 | 1 | 2 | 0 | R | H | 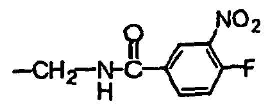 |

**Table 1.117**

| Compd. No. | 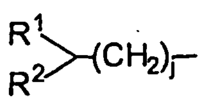 | k | m | n | chirality | $R^3$ | 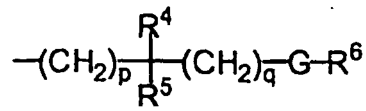 |
|---|---|---|---|---|---|---|---|
| 1277 | 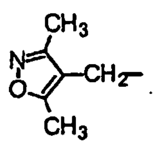 | 1 | 2 | 0 | R | H | 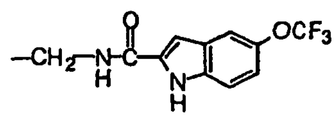 |
| 1278 | 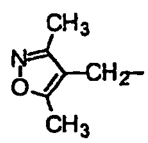 | 1 | 2 | 0 | R | H | 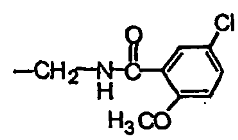 |
| 1279 | 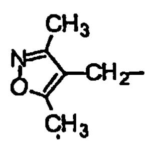 | 1 | 2 | 0 | R | H | 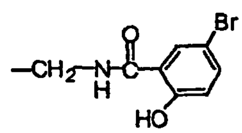 |
| 1280 | 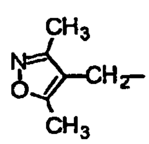 | 1 | 2 | 0 | R | H | 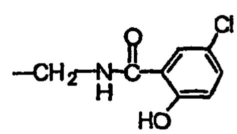 |
| 1281 | 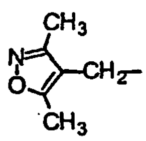 | 1 | 2 | 0 | R | H | 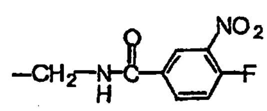 |
| 1282 | 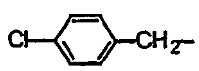 | 2 | 2 | 1 | - | H | 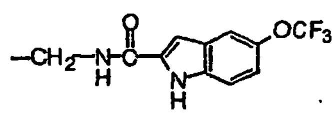 |

(continued)

| Compd. No. | 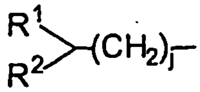 | k | m | n | chirality | $R^3$ | 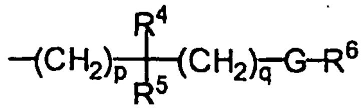 |
|---|---|---|---|---|---|---|---|
| 1283 | 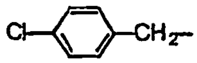 | 2 | 2 | 1 | - | H | 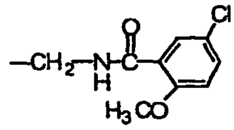 |
| 1284 | 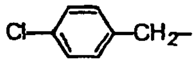 | 2 | 2 | 1 | - | H | 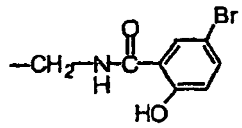 |
| 1285 | 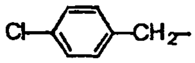 | 2 | 2 | 1 | - | H | 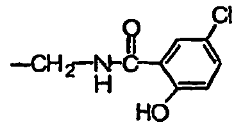 |
| 1286 | 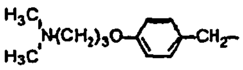 | 1 | 2 | 0 | R | H | 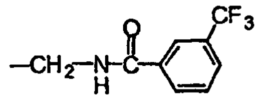 |
| 1287 | 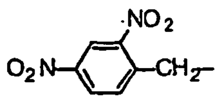 | 1 | 2 | 0 | R | H | 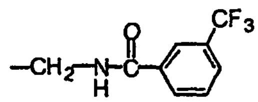 |

**Table 1.118**

| Compd. No. | 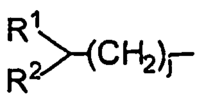 | k | m | n | chirality | $R^3$ | 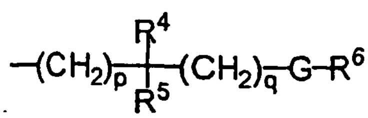 |
|---|---|---|---|---|---|---|---|
| 1288 | 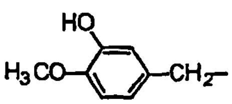 | 1 | 2 | 0 | R | H | 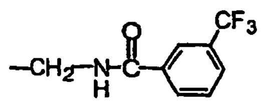 |
| 1289 | 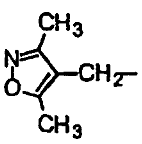 | 1 | 2 | 0 | R | H | 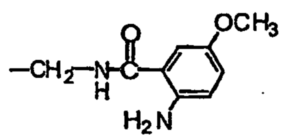 |
| 1290 | 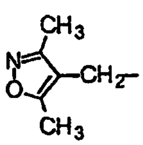 | 1 | 2 | 0 | R | H | 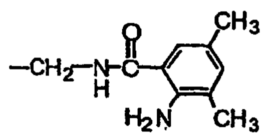 |
| 1291 | 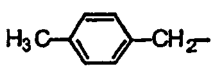 | 1 | 2 | 0 | R | H | 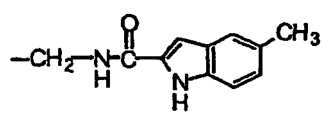 |

(continued)

| Compd. No. | 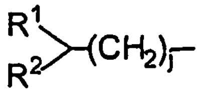 | k | m | n | chirality | $R^3$ | 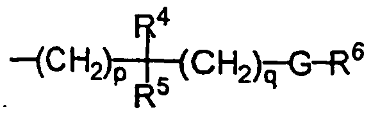 |
|---|---|---|---|---|---|---|---|
| 1292 | 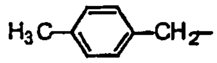 | 1 | 2 | 0 | R | H | 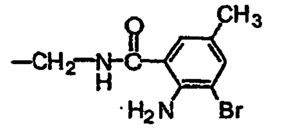 |
| 1293 | 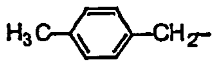 | 1 | 2 | 0 | R | H | 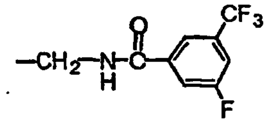 |
| 1294 | 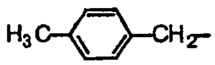 | 1 | 2 | 0 | R | H | 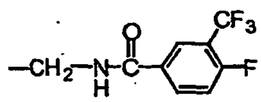 |
| 1295 | 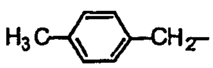 | 1 | 2 | 0 | R | H | 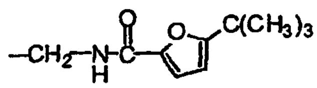 |
| 1296 | 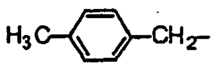 | 1 | 2 | 0 | R | H | 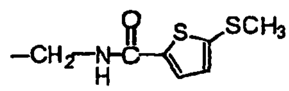 |
| 1297 | 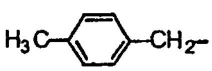 | 1 . | 2 | 0 | R | H | 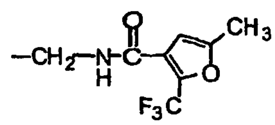 |
| 1298 | 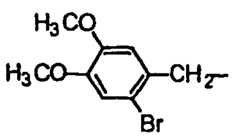 | 1 | 2 | 0 | R | H | 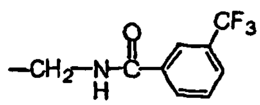 |

**Table 1.119**

| Compd. No. | 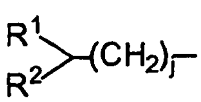 | k | m | n | chirality | $R^3$ | 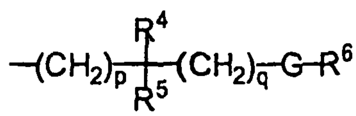 |
|---|---|---|---|---|---|---|---|
| 1299 | 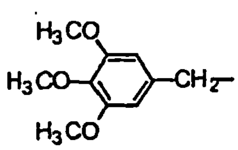 | 1 | 2 | 0 | R | H | 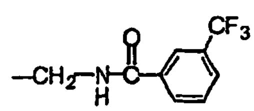 |
| 1300 | 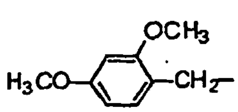 | 1 | 2 | 0 | R | H | 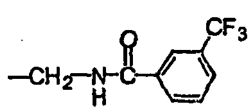 |

(continued)

| Compd. No. | 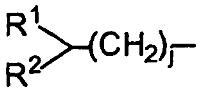 | k | m | n | chirality | $R^3$ | 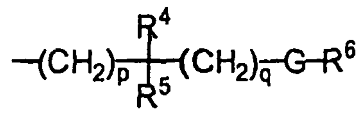 |
|---|---|---|---|---|---|---|---|
| 1301 | 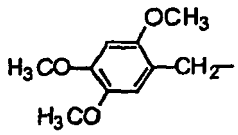 | 1 | 2 | 0 | R | H | 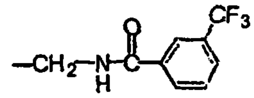 |
| 1302 | 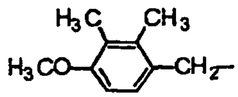 | 1 | 2 | 0 | R | H | 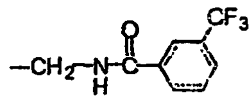 |
| 1303 | 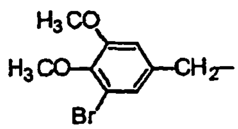 | 1 | 2 | 0 | R | H | 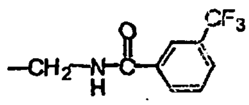 |
| 1304 | 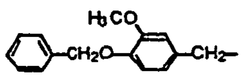 | 1 | 2 | 0 | R | H | 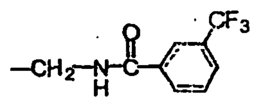 |
| 1305 | 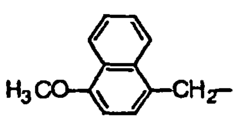 | 1 | 2 | 0 | R | H | 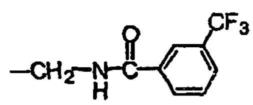 |
| 1306 | 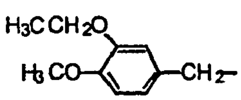 | 1 | 2 | 0 | R | H | 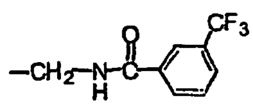 |
| 1307 | 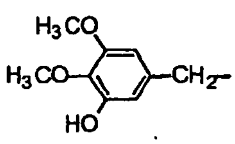 | 1 | 2 | 0 | R | H | 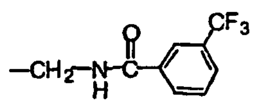 |
| 1308 | 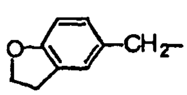 | 1 | 2 | 0 | R | H | 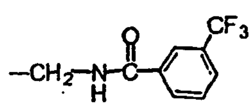 |
| 1309 | 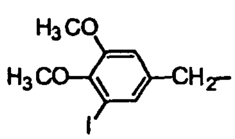 | 1 | 2 | 0 | R | H | 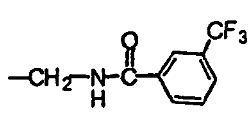 |

**Table 1.120**

| Compd. No. | 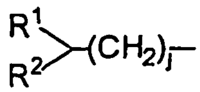 | k | m | n | chirality | $R^3$ | 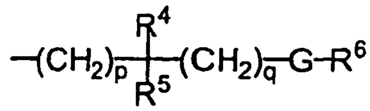 |
|---|---|---|---|---|---|---|---|
| 1310 | 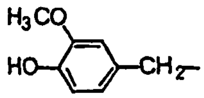 | 1 | 2 | 0 | R | H | 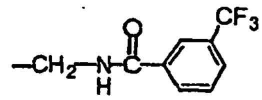 |
| 1311 | 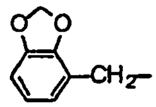 | 1 | 2 | 0 | R | H | 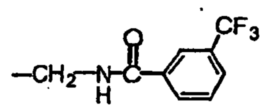 |
| 1312 | 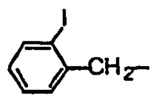 | 1 | 2 | 0 | R | H | 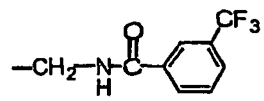 |
| 1313 | 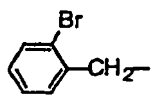 | 1 | 2 | 0 | R | H | 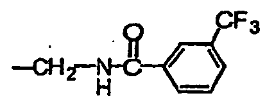 |
| 1314 | 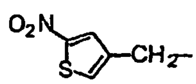 | 1 | 2 | 0 | R | H | 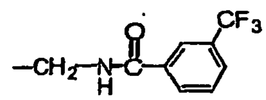 |
| 1315 | 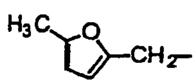 | 1 | 2 | 0 | R | H | 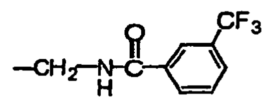 |
| 1316 | 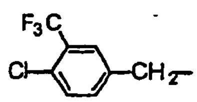 | 1 | 2 | 0 | R | H | 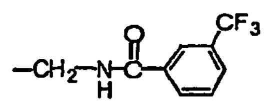 |
| 1317 | 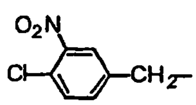 | 1 | 2 | 0 | R | H | 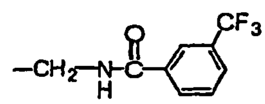 |
| 1318 | 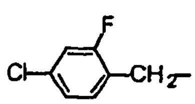 | 1 | 2 | 0 | R | H | 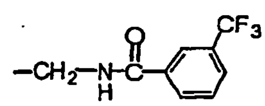 |
| 1319 | 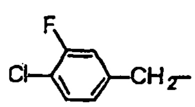 | 1 | 2 | 0 | R | H | 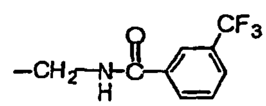 |
| 1320 | 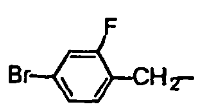 | 1 | 2 | 0 | R | H | 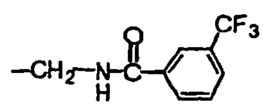 |

**Table 1.121**

| Compd. No. | 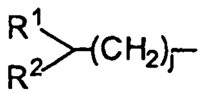 | k | m | n | chirality | $R^3$ | 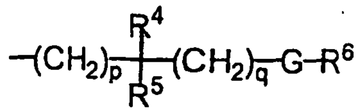 |
|---|---|---|---|---|---|---|---|
| 1321 | 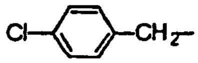 | 1 | 2 | 0 | R | H | 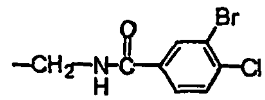 |
| 1322 | 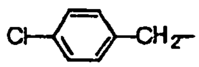 | 1 | 2 | 0 | R | H | 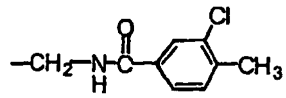 |
| 1323 | 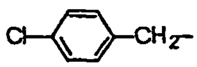 | 1 | 2 | 0 | R | H | 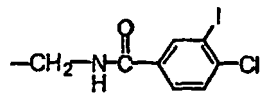 |
| 1324 | 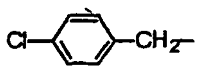 | 1 | 2 | 0 | R | H | 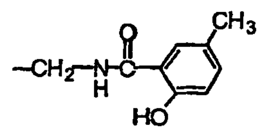 |
| 1325 | 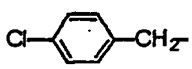 | 1 | 2 | 0 | R | H | 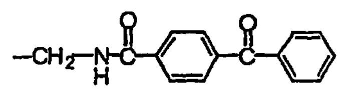 |
| 1326 | 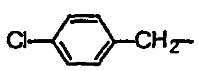 | 1 | 2 | 0 | R | H | 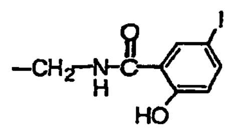 |
| 1327 | 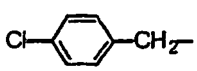 | 1 | 2 | 0 | R | H | 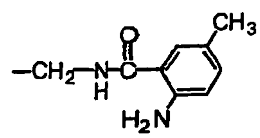 |
| 1328 | 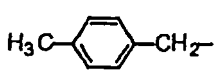 | 1 | 2 | 0 | R | H | 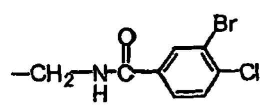 |
| 1329 | 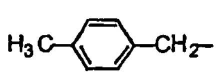 | 1 | 2 | 0 | R | H | 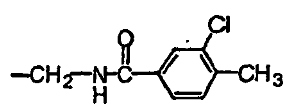 |
| 1330 | 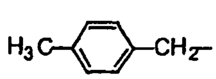 | 1 | 2 | 0 | R | H | 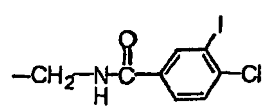 |
| 1331 | 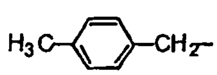 | 1 | 2 | 0 | R | H | 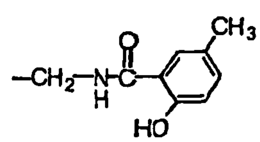 |

**Table 1.122**

| Compd. No. | 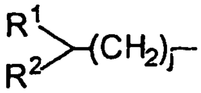 | k | m | n | chirality | $R^3$ | 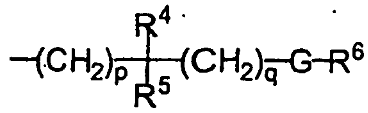 |
|---|---|---|---|---|---|---|---|
| 1332 | 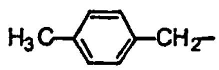 | 1 | 2 | 0 | R | H | 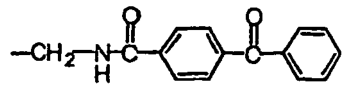 |
| 1333 | 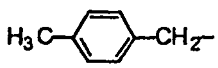 | 1 | 2 | 0 | R | H | 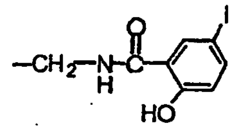 |
| 1334 | 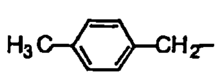 | 1 | 2 | 0 | R | H | 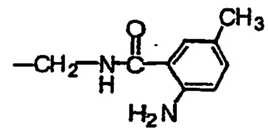 |
| 1335 | 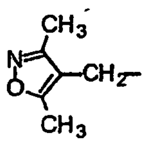 | 1 | 2 | 0 | R | H | 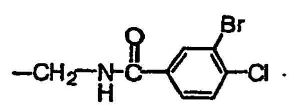 |
| 1336 | 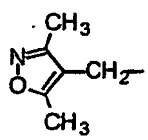 | 1 | 2 | 0 | R | H | 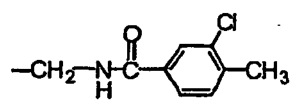 |
| 1337 | 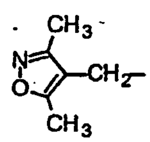 | 1 | 2 | 0 | R | H | 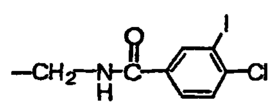 |
| 1338 | 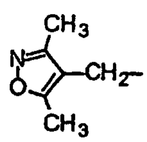 | 1 | 2 | 0 | R | H | 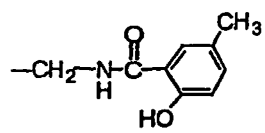 |
| 1339 | 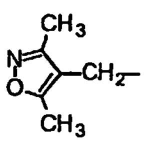 | 1 | 2 | 0 | R | H | 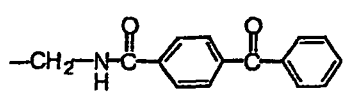 |
| 1340 | 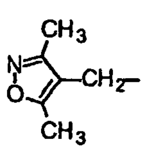 | 1 | 2 | 0 | R | H | 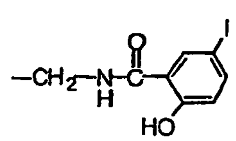 |

(continued)

| Compd. No. | 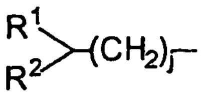 | k | m | n | chirality | $R^3$ | 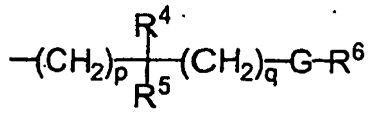 |
|---|---|---|---|---|---|---|---|
| 1341 | 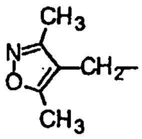 | 1 | 2 | 0 | R | H | 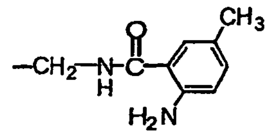 |
| 1342 | 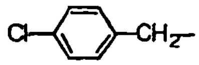 | 2 | 2 | 1 | | H | 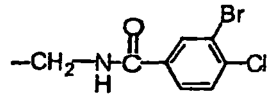 |

**Table 1.123**

| Compd. No. | 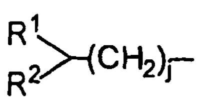 | k | m | n | chirality | $R^3$ | 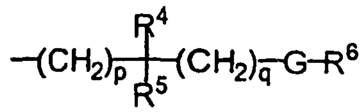 |
|---|---|---|---|---|---|---|---|
| 1343 | 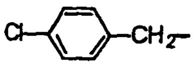 | 2 | 2 | 1 | - | H | 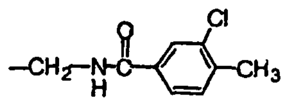 |
| 1344 | 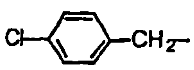 | 2 | 2 | 1 | - | H | 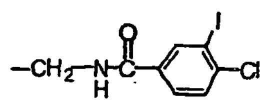 |
| 1345 | 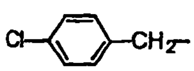 | 2 | 2 | 1 | - | H | 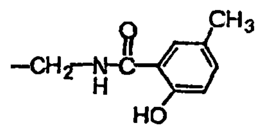 |
| 1346 | 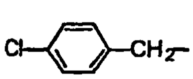 | 2 | 2 | 1 | - | H | 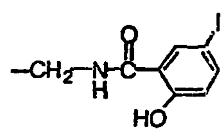 |
| 1347 | 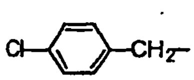 | 1 | 2 | 0 | R | H | 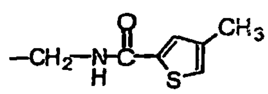 |
| 1348 | 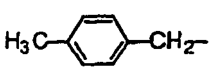 | 1 | 2 | 0 | R | H | 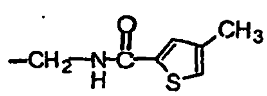 |
| 1349 | 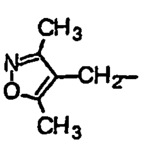 | 1 | 2 | 0 | R | H | 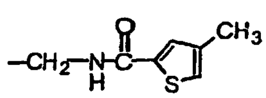 |

(continued)

| Compd. No. | 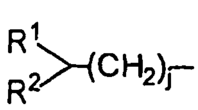 | k | m | n | chirality | $R^3$ | 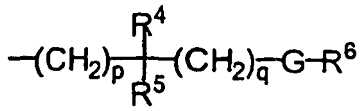 |
|---|---|---|---|---|---|---|---|
| 1350 | 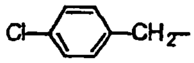 | 2 | 2 | 1 | - | H | 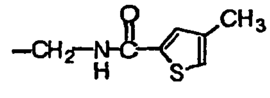 |
| 1351 | 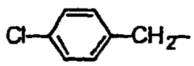 | 1 | 2 | 0 | R | H | 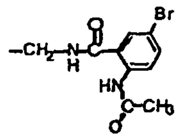 |
| 1352 | 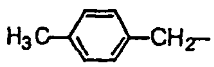 | 1 | 2 | 0 | R | H | 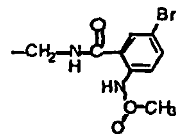 |
| 1353 | 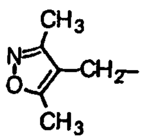 | 1 | 2 | 0 | R | H | 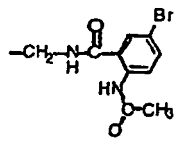 |

**Table 1.124**

| Compd. No. | 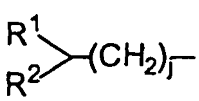 | k | m | n | chirality | $R^3$ | 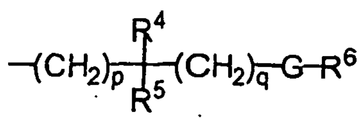 |
|---|---|---|---|---|---|---|---|
| 1354 | 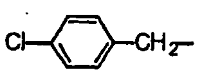 | 2 | 2 | 1 | - | H | 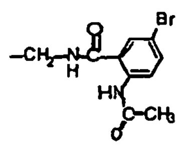 |
| 1355 | 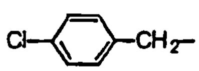 | 1 | 2 | 0 | R | H | 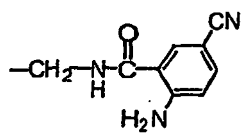 |
| 1356 | 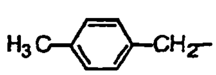 | 1 | 2 | 0 | R | H | 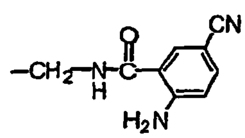 |
| 1357 | 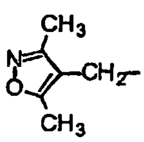 | 1 | 2 | 0 | R | H | 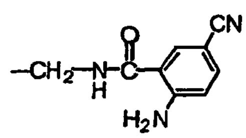 |

(continued)

| Compd. No. | 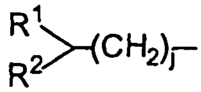 | k | m | n | chirality | $R^3$ | 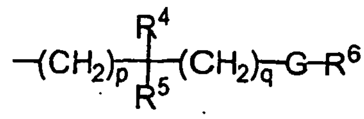 |
|---|---|---|---|---|---|---|---|
| 1358 | 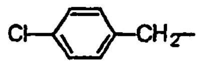 | 2 | 2 | 1 | - | H | 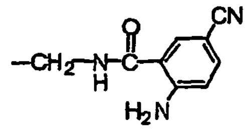 |
| 1359 | 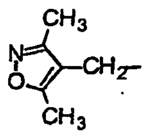 | 1 | 2 | 0 | R | H | 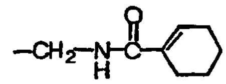 |
| 1360 | 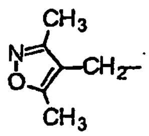 | 1 | 2 | 0 | R | H | 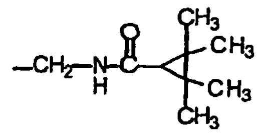 |
| 1361 | 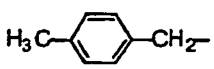 | 1 | 2 | 0 | R | H | 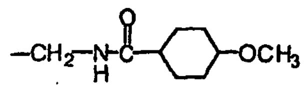 |
| 1362 | 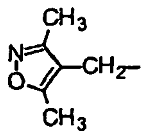 | 1 | 2 | 0 | R | H | 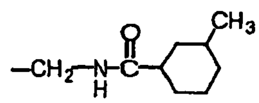 |
| 1363 | 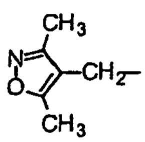 | 1 | 2 | 0 | R | H | 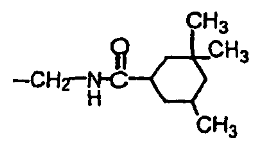 |
| 1364 | 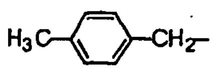 | 1 | 2 | 0 | R | H | 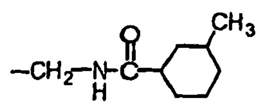 |

**Table 1.125**

| Compd. No. | 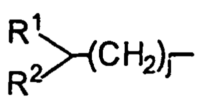 | K | m | n | chirality | $R^3$ | 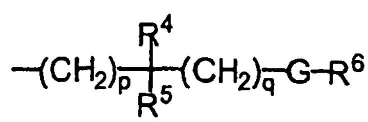 |
|---|---|---|---|---|---|---|---|
| 1365 | 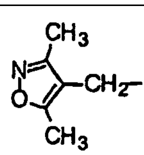 | 1 | 2 | 0 | R | H | 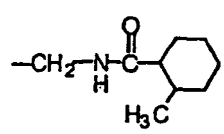 |

(continued)

| Compd. No. | 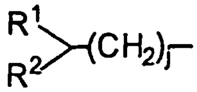 | K | m | n | chirality | $R^3$ | 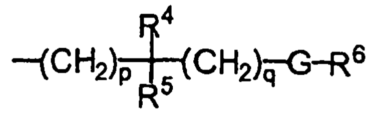 |
|---|---|---|---|---|---|---|---|
| 1366 | 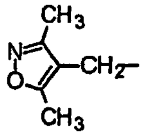 | 1 | 2 | 0 | R | H | 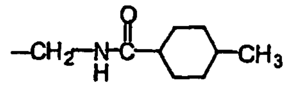 |
| 1367 | 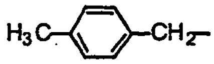 | 1 | 2 | 0 | R | H | 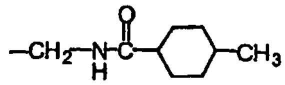 |
| 1368 | 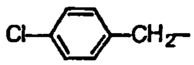 | 1 | 2 | 0 | R | H | 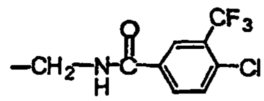 |
| 1369 | 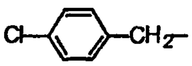 | 1 | 2 | 0 | R | H | 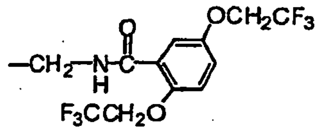 |
| 1370 | 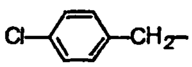 | 1 | 2 | 0 | R | H | 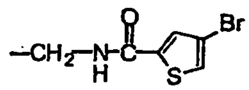 |
| 1371 | 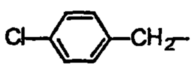 | 1 | 2 | 0 | R | H | 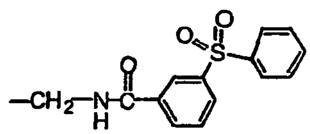 |
| 1372 | 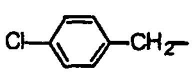 | 1 | 2 | 0 | R | H | 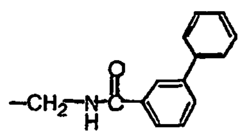 |
| 1373 | 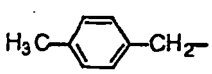 | 1 | 2 | 0 | R | H | 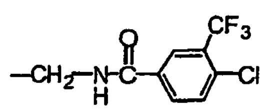 |
| 1374 | 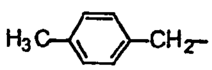 | 1 | 2 | 0 | R | H | 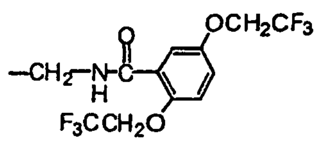 |
| 1375 | 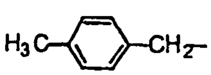 | 1 | 2 | 0 | R | H | 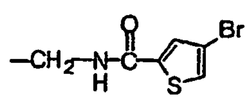 |

**Table 1.126**

| Compd. No. | 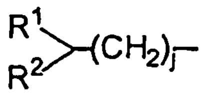 | k | m | n | chirality | $R^3$ | 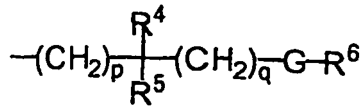 |
|---|---|---|---|---|---|---|---|
| 1376 | 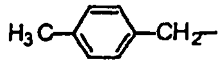 | 1 | 2 | 0 | R | H | 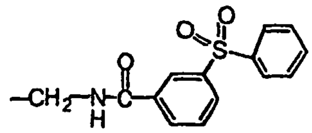 |
| 1377 | 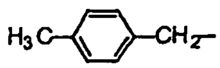 | 1 | 2 | 0 | R | H | 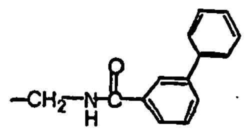 |
| 1378 | 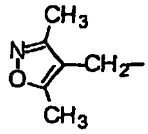 | 1 | 2 | 0 | R | H | 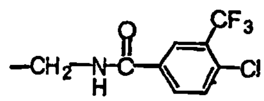 |
| 1379 | 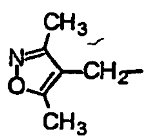 | 1 | 2 | 0 | R | H | 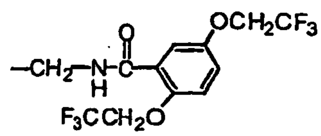 |
| 1380 | 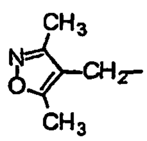 | 1 | 2 | 0 | R | H | 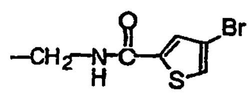 |
| 1381 | 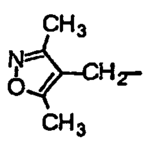 | 1 | 2 | 0 | R | H | 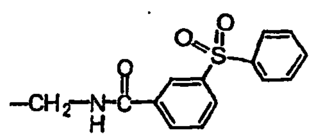 |
| 1382 | 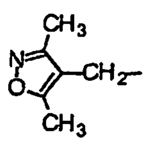 | 1 | 2 | 0 | R | H | 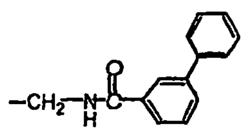 |
| 1383 | 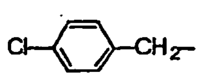 | 2 | 2 | 1 | - | H | 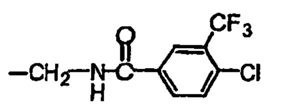 |
| 1384 | 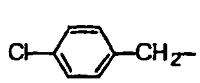 | 2 | 2 | 1 | - | H | 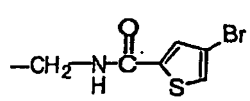 |
| 1385 | 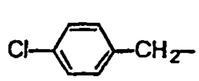 | 2 | 2 | 1 | - | H | 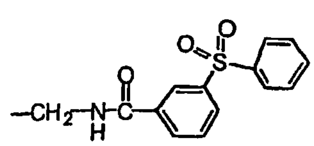 |

(continued)

| Compd. No. | 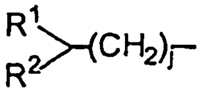 | k | m | n | chirality | $R^3$ | 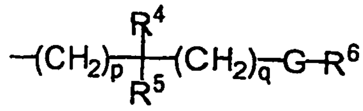 |
|---|---|---|---|---|---|---|---|
| 1386 | 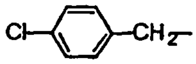 | 2 | 2 | 1 | - | H | 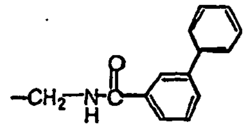 |

**Table 1.127**

| Compd. No. | 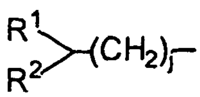 | k | m | n | chirality | $R^3$ | 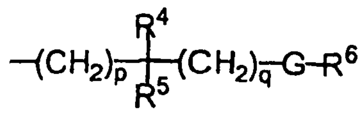 |
|---|---|---|---|---|---|---|---|
| 1387 | 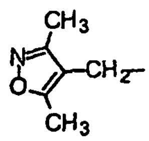 | 1 | 2 | 0 | R | H | 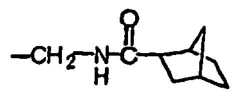 |
| 1388 | 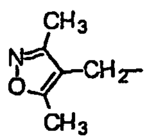 | 1 | 2 | 0 | R | H | 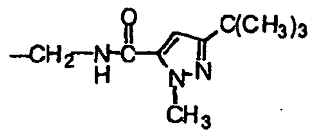 |
| 1389 | 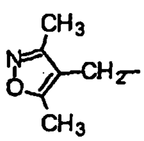 | 1 | 2 | 0 | R | H | 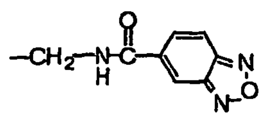 |
| 1390 | 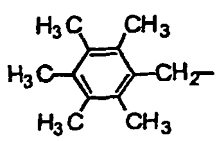 | 1 | 2 | 0 | R | H | 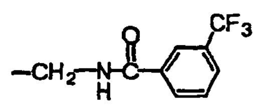 |
| 1391 | 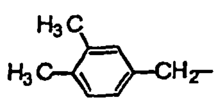 | 1 | 2 | 0 | R | H | 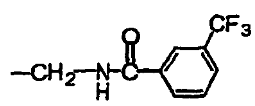 |
| 1392 | 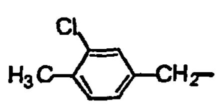 | 1 | 2 | 0 | R | H | 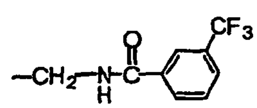 |
| 1393 | 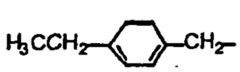 | 1 | 2 | 0 | R | H | 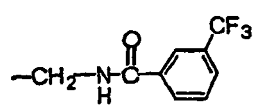 |

(continued)

| Compd. No. | 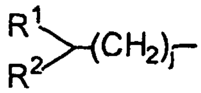 | k | m | n | chirality | $R^3$ | 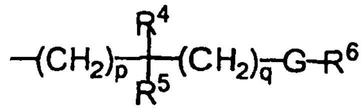 |
|---|---|---|---|---|---|---|---|
| 1394 | 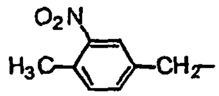 | 1 | 2 | 0 | R | H | 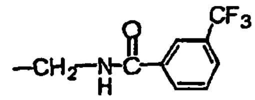 |
| 1395 | 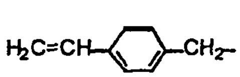 | 1 | 2 | 0 | R | H | 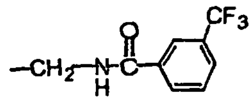 |
| 1396 | 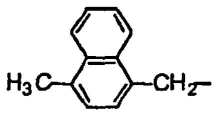 | 1 | 2 | 0 | R | H | 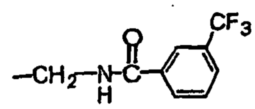 |
| 1397 | 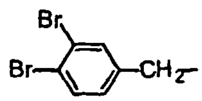 | 1 | 2 | 0 | R | H | 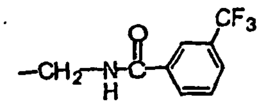 |

**Table 1.128**

| Compd. No. | 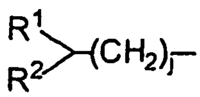 | k | m | n | chirality | $R^3$ | 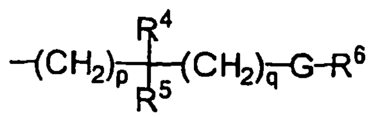 |
|---|---|---|---|---|---|---|---|
| 1398 | 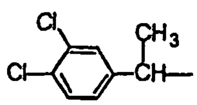 | 1 | 2 | 0 | R | H | 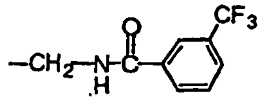 |
| 1399 | 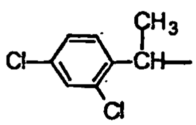 | 1 | 2 | 0 | R | H | 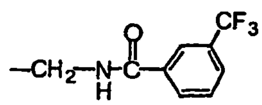 |
| 1400 | 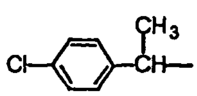 | 1 | 2 | 0 | R | H | 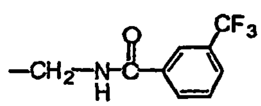 |
| 1401 | 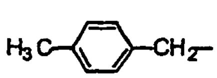 | 1 | 2 | 0 | R | H | 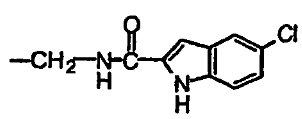 |
| 1402 | 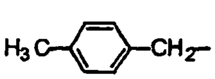 | 1 | 2 | 0 | R | H | 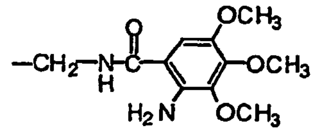 |

(continued)

| Compd. No. | 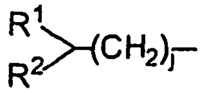 | k | m | n | chirality | $R^3$ | 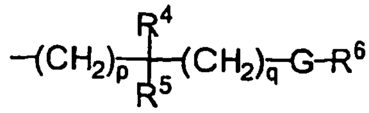 |
|---|---|---|---|---|---|---|---|
| 1403 | 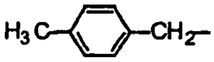 | 1 | 2 | 0 | R | H | 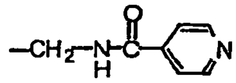 |
| 1404 | 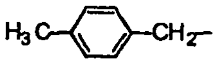 | 1 | 2 | 0 | R | H | 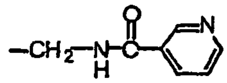 |
| 1405 | 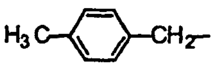 | 1 | 2 | 0 | R | H | 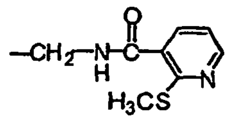 |
| 1406 | 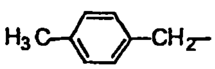 | 1 | 2 | 0 | R | H | 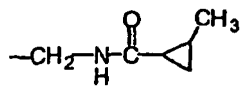 |
| 1407 | 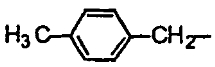 | I | 2 | 0 | R | H | 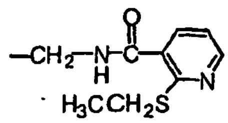 |
| 1408 | 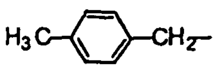 | | 2 | 0 | R | H | 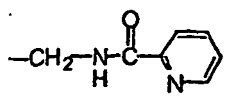 |

**Table 1.129**

| Compd. No. | 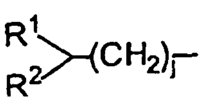 | k | m | n | chirality | $R^3$ | 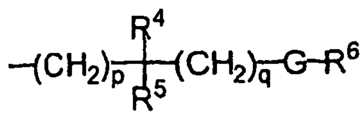 |
|---|---|---|---|---|---|---|---|
| 1409 | 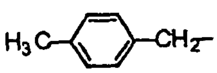 | 1 | 2 | 0 | R | H | 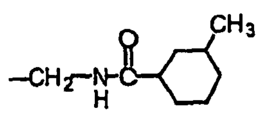 |
| 1410 | 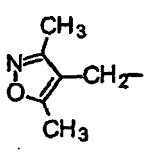 | 1 | 2 | 0 | R | H | 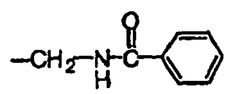 |
| 1411 | 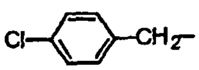 | 1 | 2 | 0 | R | H | 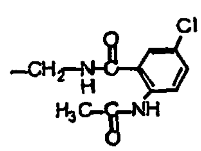 |

(continued)

| Compd. No. | 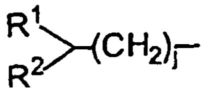 | k | m | n | chirality | $R^3$ | 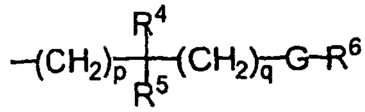 |
|---|---|---|---|---|---|---|---|
| 1412 | 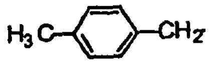 | 1 | 2 | 0 | R | H | 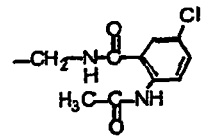 |
| 1413 | 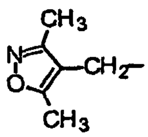 | 1 | 2 | 0 | R | H | 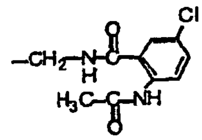 |
| 1414 | 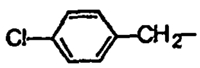 | 2 | 2 | 1 | - | H | 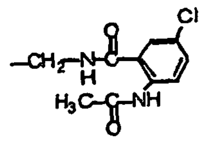 |
| 1415 | 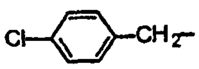 | 1 | 2 | 0 | R | H | 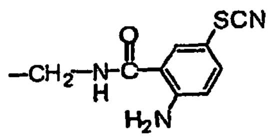 |
| 1416 | 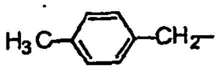 | 1 | 2 | 0 | R | H | 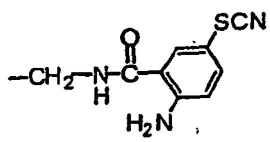 |
| 1417 | 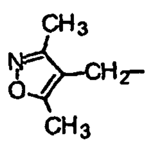 | 1 | 2 | 0 | R | H | 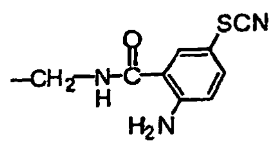 |
| 1418 | 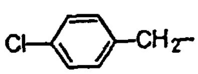 | 2 | 2 | 1 | - | H | 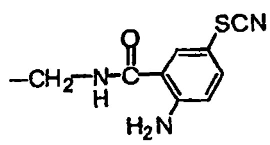 |
| 1419 | 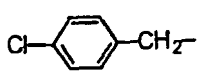 | 1 | 2 | 0 | R | H | 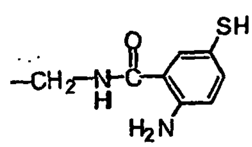 |

**Table 1.130**

| Compd. No. | 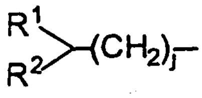 | k | m | n | chirality | $R^3$ | 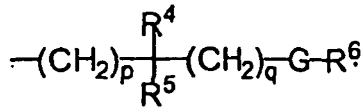 |
|---|---|---|---|---|---|---|---|
| 1420 | 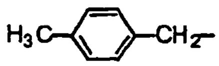 | 1 | 2 | 0 | R | H | 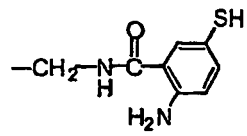 |
| 1421 | 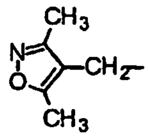 | 1 | 2 | 0 | R | H | 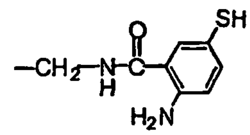 |
| 1422 | 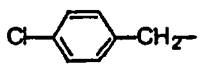 | 2 | 2 | 1 | - | H | 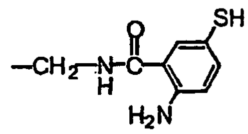 |
| 1423 | 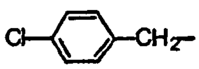 | 1 | 2 | 0 | R | H | 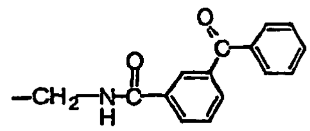 |
| 1424 | 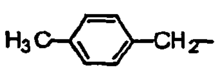 | 1 | 2 | 0 | R | H | 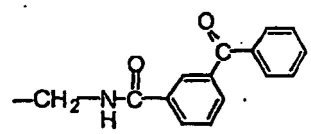 |
| 1425 | 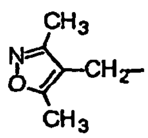 | 1 | 2 | 0 | R | H | 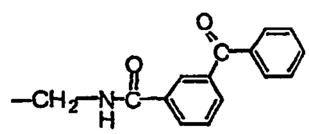 |
| 1426 | 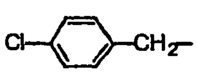 | 2 | 2 | 1 | - | H | 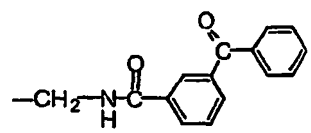 |
| 1427 | 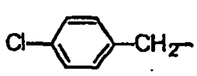 | 2 | 2 | 1 | - | H | 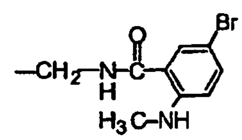 |
| 1428 | 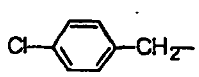 | 2 | 2 | 1 | - | H | 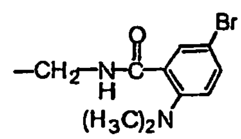 |
| 1429 | 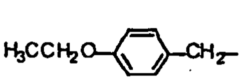 | 2 | 2 | 1 | - | H | 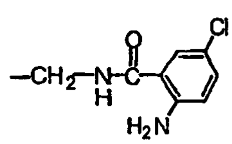 |

(continued)

| Compd. No. | 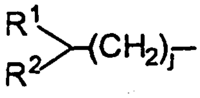 | k | m | n | chirality | $R^3$ | 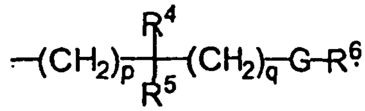 |
|---|---|---|---|---|---|---|---|
| 1430 | 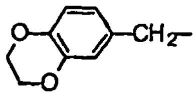 | 2 | 2 | 1 | - | H | 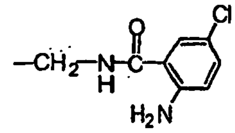 |

**Table 1.131**

| Compd. No. | 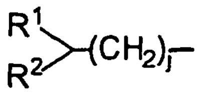 | k | m | n | chirality | $R^3$ | 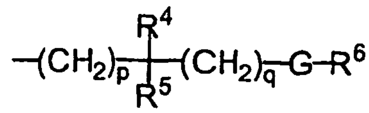 |
|---|---|---|---|---|---|---|---|
| 1431 | 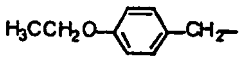 | 2 | 2 | 1 | - | H | 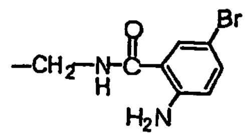 |
| 1432 | 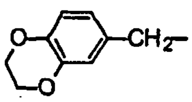 | 2 | 2 | 1 | - | H | 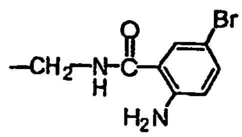 |
| 1433 | 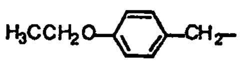 | 2 | 2 | 1 | - | H | 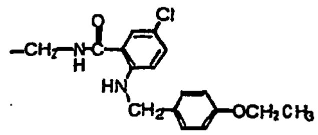 |
| 1434 | 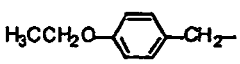 | 2 | 2 | 1 | - | H | 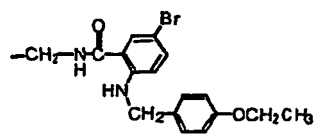 |
| 1435 | 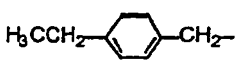 | 2 | 2 | 1 | - | H | 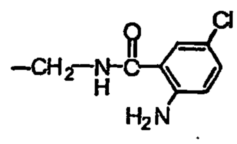 |
| 1436 | 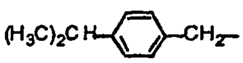 | 2 | 2 | 1 | - | H | 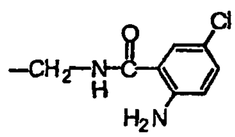 |
| 1437 | 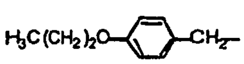 | 2 | 2 | 1 | - | H | 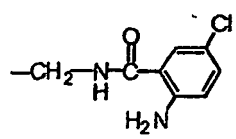 |

(continued)

| Compd. No. | 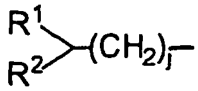 | k | m | n | chirality | $R^3$ | 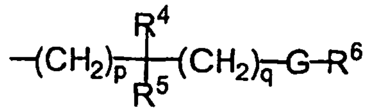 |
|---|---|---|---|---|---|---|---|
| 1438 | 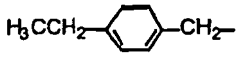 | 2 | 2 | 1 | - | H | 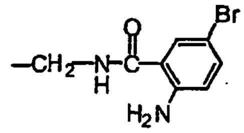 |
| 1439 | 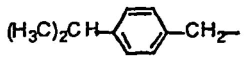 | 2 | 2 | 1 | - | H | 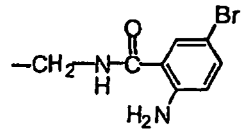 |
| 1440 | 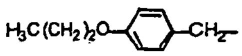 | 2 | 2 | 1 | - | H | 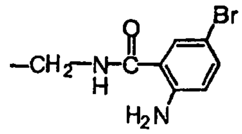 |
| 1441 | 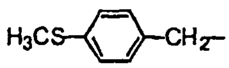 | 2 | 2 | 1 | - | H | 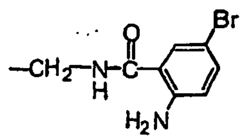 |

**Table 1.132**

| Compd. No. | 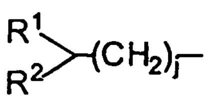 | k | m | n | chirality | $R^3$ | 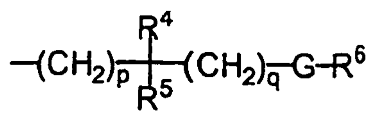 |
|---|---|---|---|---|---|---|---|
| 1442 | 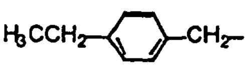 | 2 | 2 | 1 | - | H | 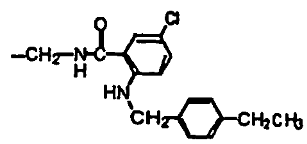 |
| 1443 | 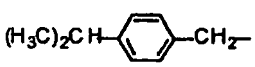 | 2 | 2 | 1 | - | H | 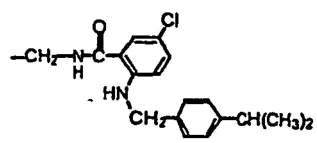 |
| 1444 | 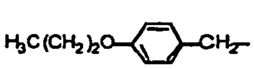 | 2 | 2 | 1 | - | H | 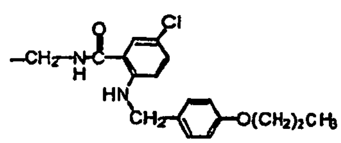 |
| 1445 | 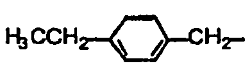 | 2 | 2 | 1 | - | H | 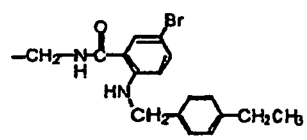 |

(continued)

| Compd. No. | 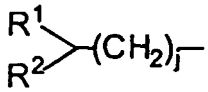 | k | m | n | chirality | $R^3$ | 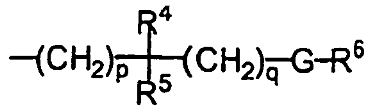 |
|---|---|---|---|---|---|---|---|
| 1446 | 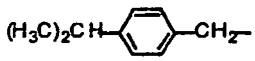 | 2 | 2 | 1 | - | H | 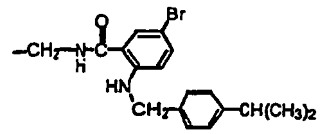 |
| 1447 | 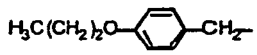 | 2 | 2 | 1 | - | H | 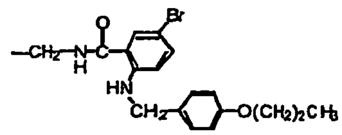 |
| 1448 | 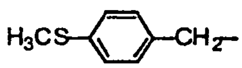 | 2 | 2 | 1 | - | H | 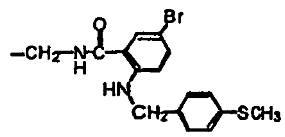 |
| 1449 | 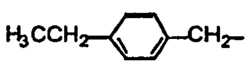 | 2 | 2 | 1 | - | H | 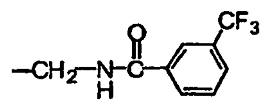 |
| 1450 | 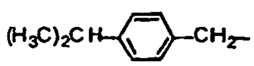 | 2 | 2 | 1 | - | H | 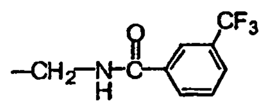 |
| 1451 | 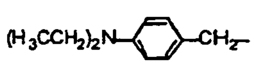 | 2 | 2 | 1 | - | H | 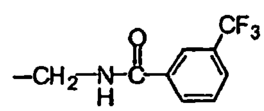 |
| 1452 | 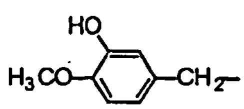 | 2 | 2 | 1 | - | H | 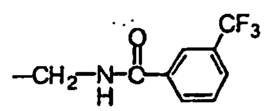 |

**Table 1.133**

| Compd. No. | 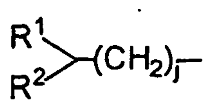 | k | m | n | chirality | $R^3$ | 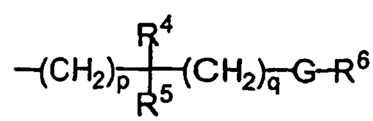 |
|---|---|---|---|---|---|---|---|
| 1453 | 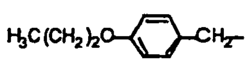 | 2 | 2 | 1 | - | H | 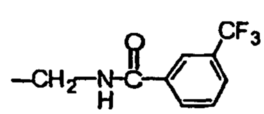 |
| 1454 | 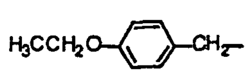 | 2 | 2 | 1 | - | H | 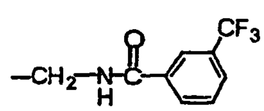 |

(continued)

| Compd. No. | 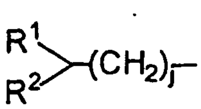 | k | m | n | chirality | $R^3$ | 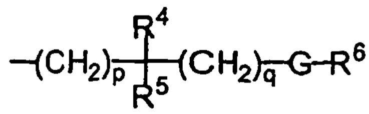 |
|---|---|---|---|---|---|---|---|
| 1455 | 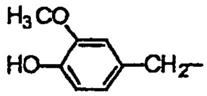 | 2 | 2 | 1 | - | H | 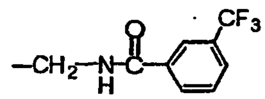 |
| 1456 | 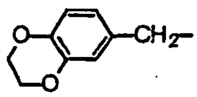 | 2 | 2 | 1 | - | H | 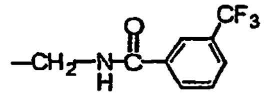 |
| 1457 | 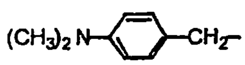 | 2 | 2 | 1 | - | H | 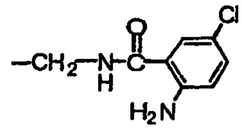 |
| 1458 | 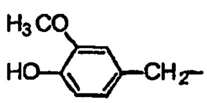 | 2 | 2 | 1 | - | H | 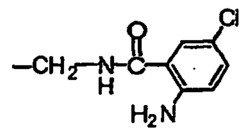 |
| 1459 | 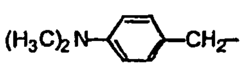 | 2 | 2 | 1 | - | H | 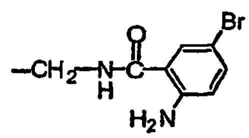 |
| 1460 | 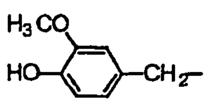 | 2 | 2 | 1 | - | H | 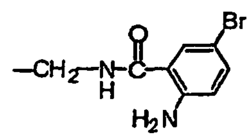 |
| 1461 | 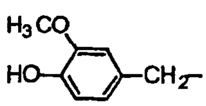 | 2 | 2 | 1 | - | H | 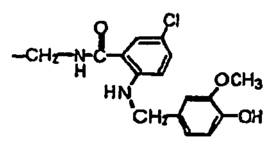 |
| 1462 | 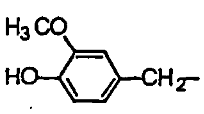 | 2 | 2 | 1 | - | H | 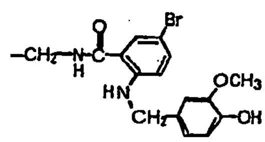 |
| 1463 | 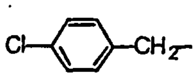 | 2 | 1 | 1 | - | H | 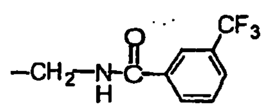 |

**Table 1.134**

| Compd. No. | 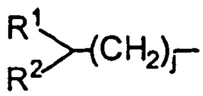 | k | m | n | chirality | $R^3$ | 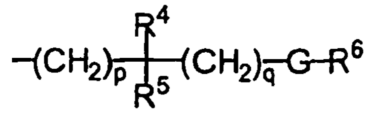 |
|---|---|---|---|---|---|---|---|
| 1464 | 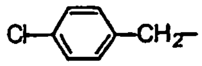 | 2 | 1 | 1 | - | H | 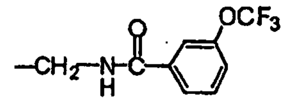 |
| 1465 | 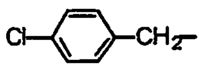 | 2 | 1 | 1 | - | H | 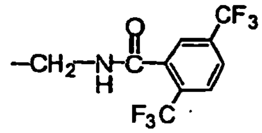 |
| 1466 | 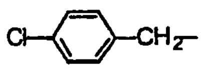 | 2 | 1 | 1 | - | H | 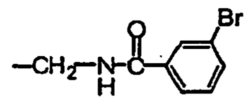 |
| 1467 | 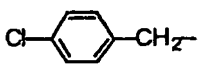 | 2 | 1 | 1 | - | H | 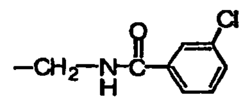 |
| 1468 | 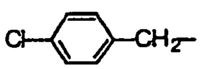 | 2 | 1 | 1 | - | H | 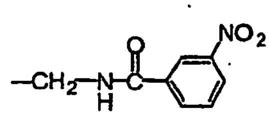 |
| 1469 | 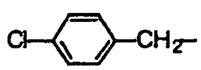 | 2 | 1 | 1 | - | H | 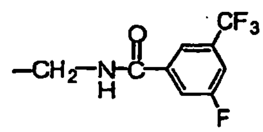 |
| 1470 | 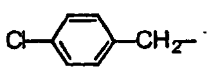 | 2 | 1 | 1 | - | H | 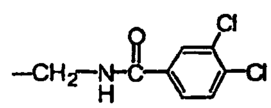 |
| 1471 | 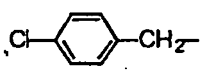 | 2 | 1 | 1 | - | H | 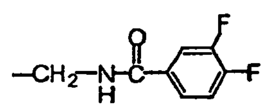 |
| 1472 | 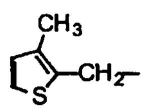 | 1 | 2 | 0 | R | H | 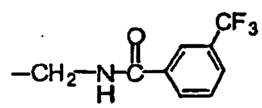 |
| 1473 | 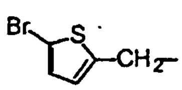 | 1 | 2 | 0 | R | H | 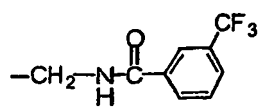 |

(continued)

| Compd. No. | 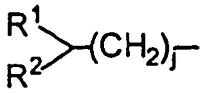 | k | m | n | chirality | $R^3$ | 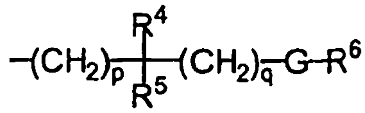 |
|---|---|---|---|---|---|---|---|
| 1474 | 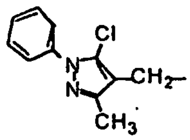 | 1 | 2 | 0 | R | H | 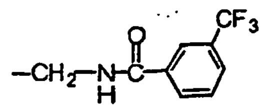 |

**Table 1.135**

| Compd. No. | 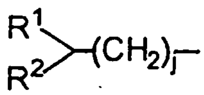 | k | m | n | chirality | $R^3$ | 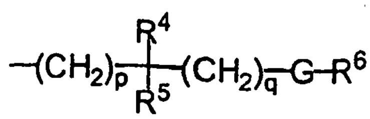 |
|---|---|---|---|---|---|---|---|
| 1475 | 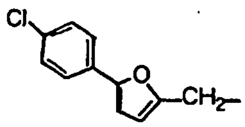 | 1 | 2 | 0 | R | H | 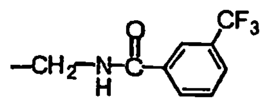 |
| 1476 | 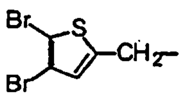 | 1 | 2 | 0 | R | H | 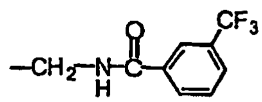 |
| 1477 | 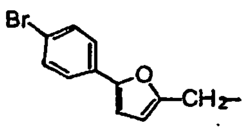 | 1 | 2 | 0 | R | H | 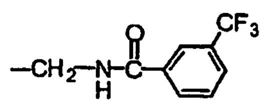 |
| 1478 | 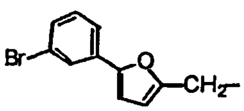 | 1 | 2 | 0 | R | H | 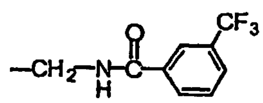 |
| 1479 | 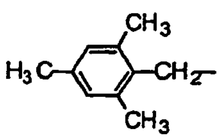 | 1 | 2 | 0 | R | H | 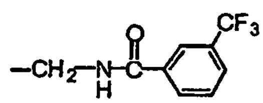 |
| 1480 | 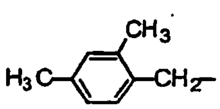 | 1 | 2 | 0 | R | H | 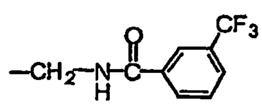 |
| 1481 | 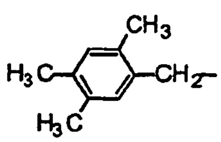 | 1 | 2 | 0 | R | H | 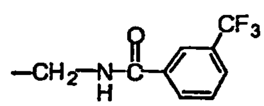 |

(continued)

| Compd. No. | 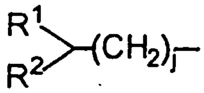 | k | m | n | chirality | $R^3$ | 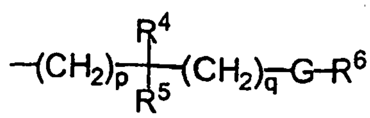 |
|---|---|---|---|---|---|---|---|
| 1482 | 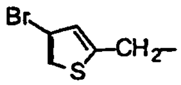 | 1 | 2 | 0 | R | H | 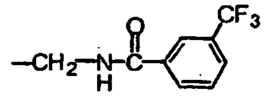 |
| 1483 | 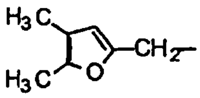 | 1 | 2 | 0 | R | H | 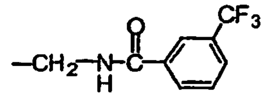 |
| 1484 | 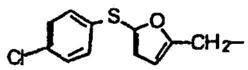 | 1 | 2 | 0 | R | H | 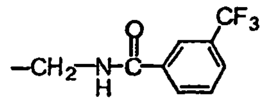 |
| 1485 | 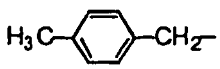 | 1 | 2 | 0 | R | H | 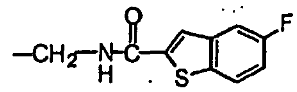 |

**Table 1.136**

| Compd. No. | 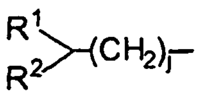 | k | m | n | chirality | $R^3$ | 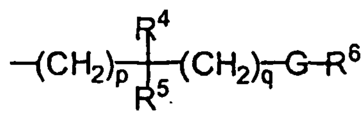 |
|---|---|---|---|---|---|---|---|
| 1486 | 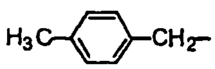 | 1 | 2 | 0 | R | H | 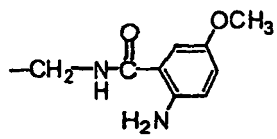 |
| 1487 | 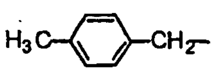 | 1 | 2 | 0 | R | H | 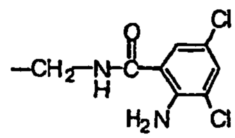 |
| 1488 | 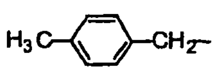 | 1 | 2 | 0 | R | H | 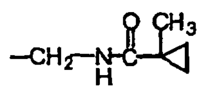 |
| 1489 | 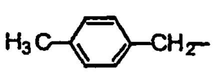 | 1 | 2 | 0 | R | H | 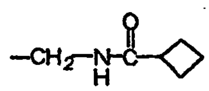 |
| 1490 | 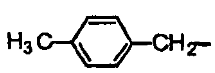 | 1 | 2 | 0 | R | H | 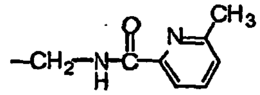 |

(continued)

| Compd. No. | 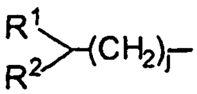 | k | m | n | chirality | $R^3$ | 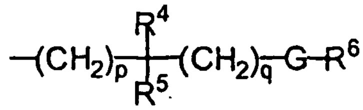 |
|---|---|---|---|---|---|---|---|
| 1491 | 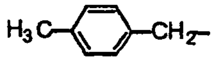 | 1 | 2 | 0 | R | H | 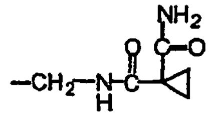 |
| 1492 | 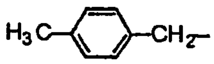 | 1 | 2 | 0 | R | H | 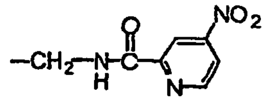 |
| 1493 | 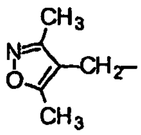 | 1 | 2 | 0 | R | H | 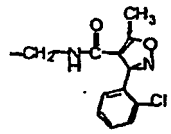 |
| 1494 | 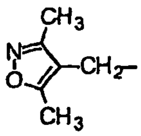 | 1 | 2 | 0 | R | H | 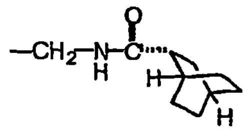 |
| 1495 | 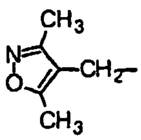 | 1 | 2 | 0 | R | H | 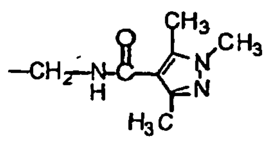 |
| 1496 | 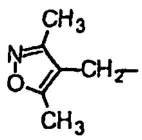 | 1 | 2 | 0 | R | H | 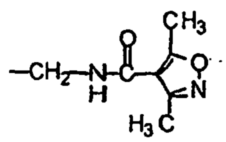 |

**Table 1.137**

| Compd. No. | 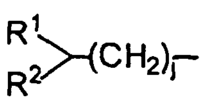 | k | m | n | chirality | $R^3$ | 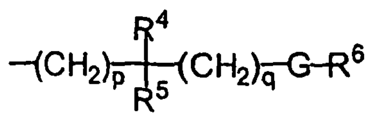 |
|---|---|---|---|---|---|---|---|
| 1497 | 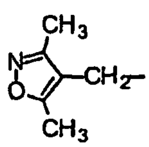 | 1 | 2 | 0 | R | H | 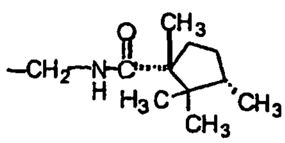 |
| 1498 | 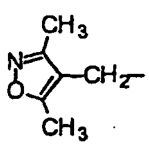 | 1 | 2 | 0 | R | H | 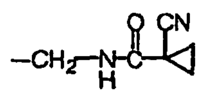 |

(continued)

| Compd. No. | 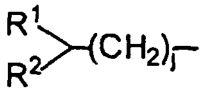 | k | m | n | chirality | $R^3$ | 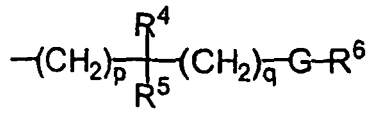 |
|---|---|---|---|---|---|---|---|
| 1499 | 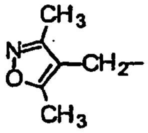 | 1 | 2 | 0 | R | H | 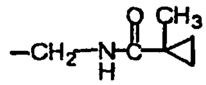 |
| 1500 | 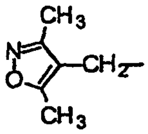 | 1 | 2 | 0 | R | H | 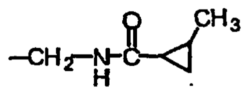 |
| 1501 | 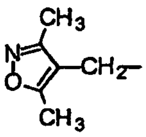 | 1 | 2 | 0 | R | H | 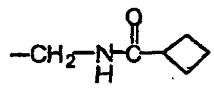 |
| 1502 | 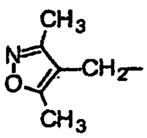 | 1 | 2 | 0 | R | H | 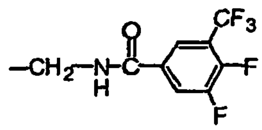 |
| 1503 | 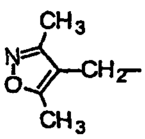 | 1 | 2 | 0 | R | H | 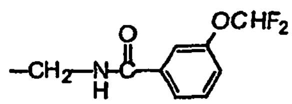 |
| 1504 | 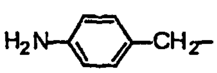 | 1 | 2 | 0 | R | H | 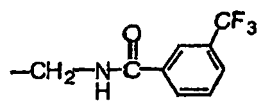 |
| 1505 | 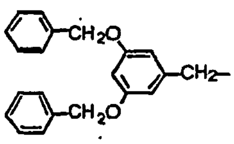 | 1 | 2 | 0 | R | H | 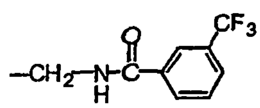 |
| 1506 | 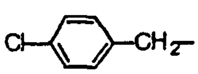 | 2 | 1 | 1 | - | H | 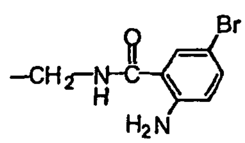 |
| 1507 | 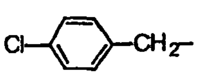 | 2 | 1 | 1 | - | H | 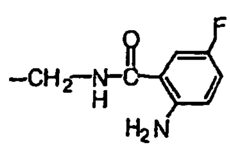 |

Table 1.138

| Compd. No. | 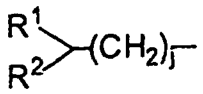 | k | m | n | chirality | $R^3$ | 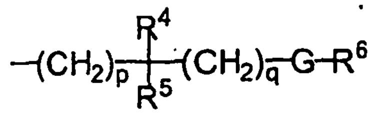 |
|---|---|---|---|---|---|---|---|
| 1508 | 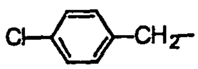 | 2 | 1 | 1 | - | H | 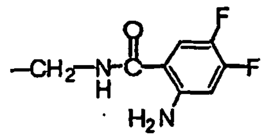 |
| 1509 | 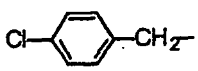 | 2 | 1 | 1 | - | H | 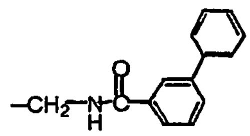 |
| 1510 | 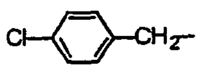 | 2 | 1 | 1 | - | H | 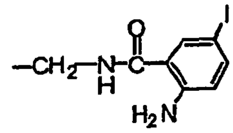 |
| 1511 | 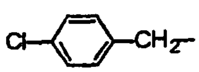 | 2 | 1 | 1 | - | H | 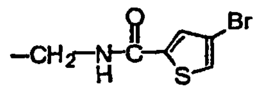 |
| 1512 | 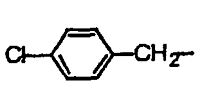 | 2 | 1 | 1 | - | H | 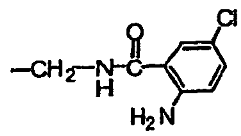 |
| 1513 | 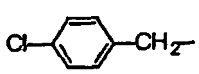 | 2 | 1 | 1 | - | H | 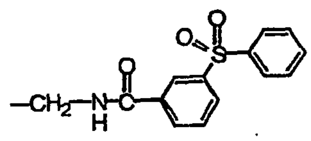 |
| 1514 | 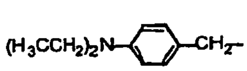 | 2 | 2 | 1 | - | H | 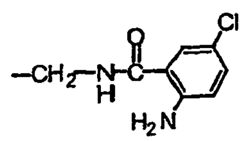 |
| 1515 | 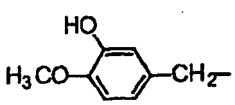 | 2 | 2 | 1 | - | H | 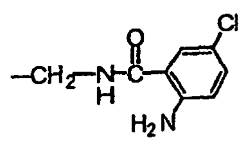 |
| 1516 | 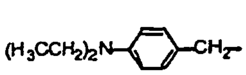 | 2 | 2 | 1 | - | H | 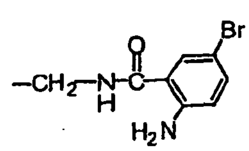 |
| 1517 | 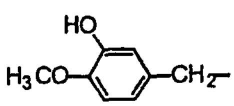 | 2 | 2 | 1 | - | H | 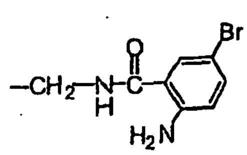 |

(continued)

| Compd. No. | 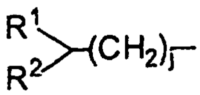 | k | m | n | chirality | $R^3$ | 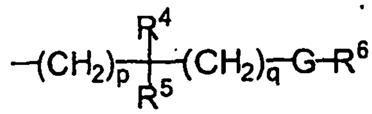 |
|---|---|---|---|---|---|---|---|
| 1518 | 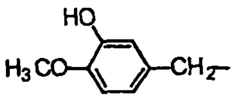 | 2 | 2 | 1 | - | H | 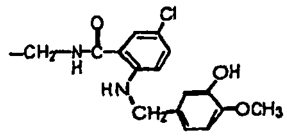 |

**Table 1.139**

| Compd. No. | 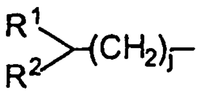 | k | m | n | chirality | $R^3$ | 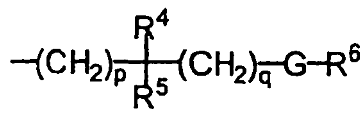 |
|---|---|---|---|---|---|---|---|
| 1519 | 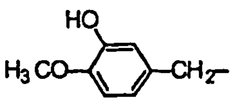 | 2 | 2 | 1 | - | H | 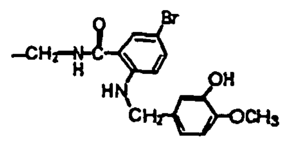 |
| 1520 | 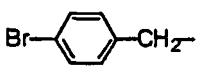 | 1 | 2 | 0 | R | H | 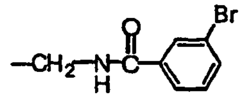 |
| 1521 | 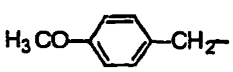 | 1 | 2 | 0 | R | H | 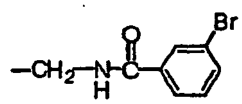 |
| 1522 | 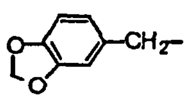 | 1 | 2 | 0 | R | H | 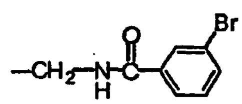 |
| 1523 | 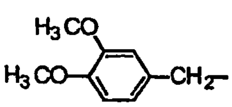 | 1 | 2 | 0 | R | H | 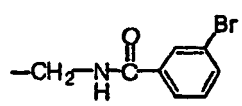 |
| 1524 | 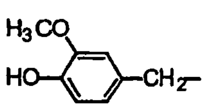 | 1 | 2 | 0 | R | H | 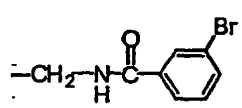 |
| 1525 | 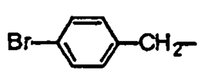 | 1 | 2 | 0 | R | H | 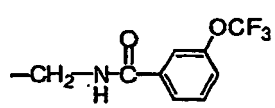 |

(continued)

| Compd. No. | 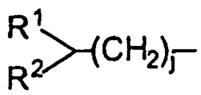 | k | m | n | chirality | $R^3$ | 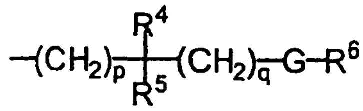 |
|---|---|---|---|---|---|---|---|
| 1526 | 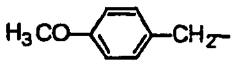 | 1 | 2 | 0 | R | H | 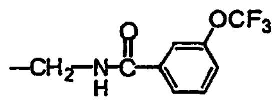 |
| 1527 | 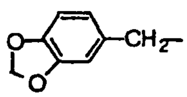 | 1 | 2 | 0 | R | H | 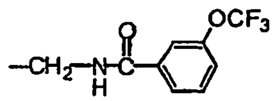 |
| 1528 | 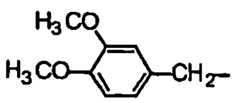 | 1 | 2 | 0 | R | H | 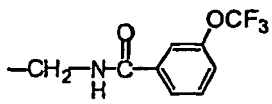 |
| 1529 | 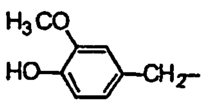 | 1 | 2 | 0 | R | H | 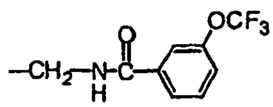 |

**Table 1.140**

| Compd. No. | 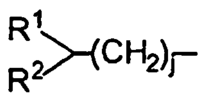 | k | m | n | chirality | $R^3$ | 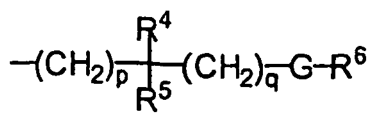 |
|---|---|---|---|---|---|---|---|
| 1530 | 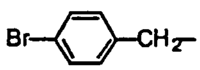 | 1 | 2 | 0 | R | H | 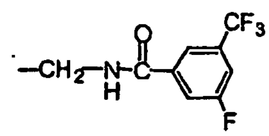 |
| 1531 | 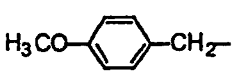 | 1 | 2 | 0 | R | H | 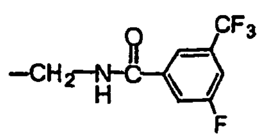 |
| 1532 | 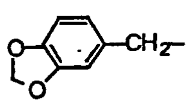 | 1 | 2 | 0 | R | H | 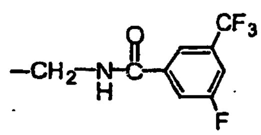 |
| 1533 | 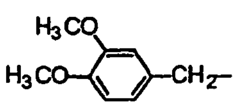 | 1 | 2 | 0 | R | H | 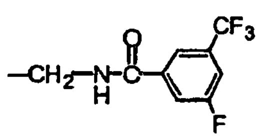 |
| 1534 | 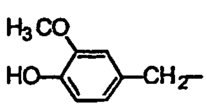 | 1 | 2 | 0 | R | H | 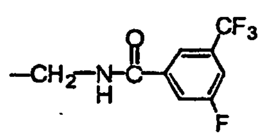 |

(continued)

| Compd. No. | 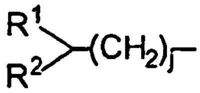 | k | m | n | chirality | $R^3$ | 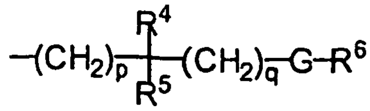 |
|---|---|---|---|---|---|---|---|
| 1535 | 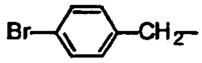 | 1 | 2 | 0 | R | H | 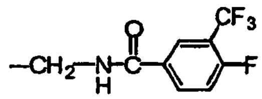 |
| 1536 | 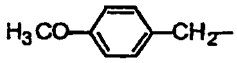 | 1 | 2 | 0 | R | H | 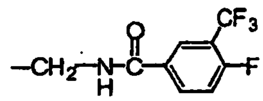 |
| 1537 | 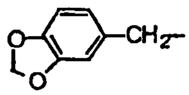 | 1 | 2 | 0 | R | H | 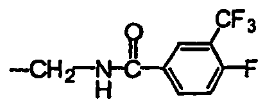 |
| 1538 | 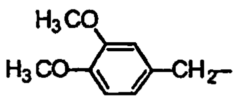 | 1 | 2 | 0 | R | H | 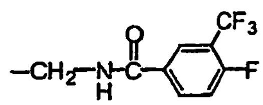 |
| 1539 | 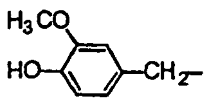 | 1 | 2 | 0 | R | H | 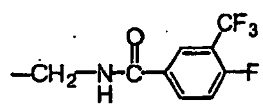 |
| 1540 | 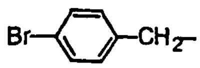 | 1 | 2 | 0 | R | H | 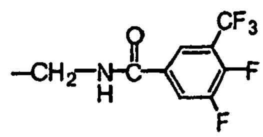 |

**Table 1.141**

| Compd. No. | 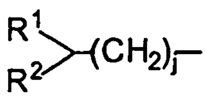 | k | m | n | chirality | $R^3$ | 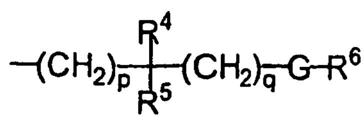 |
|---|---|---|---|---|---|---|---|
| 1541 | 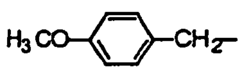 | 1 | 2 | 0 | R | H | 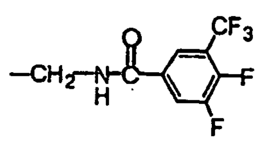 |
| 1542 | 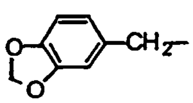 | 1 | 2 | 0 | R | H | 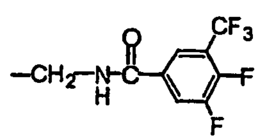 |

(continued)

| Compd. No. | 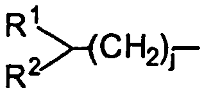 | k | m | n | chirality | $R^3$ | 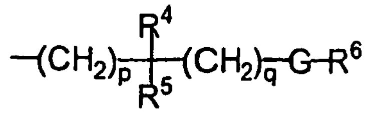 |
|---|---|---|---|---|---|---|---|
| 1543 | 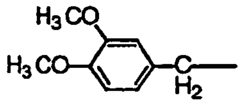 | 1 | 2 | 0 | R | H | 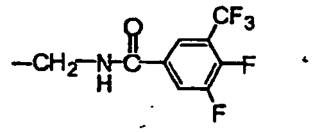 |
| 1544 | 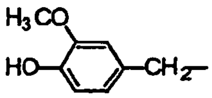 | 1 | 2 | 0 | R | H | 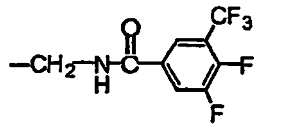 |
| 1545 | 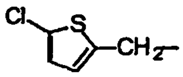 | 1 | 2 | 0 | R | H | 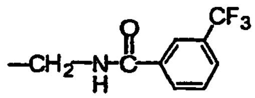 |
| 1546 | 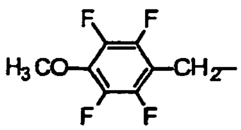 | 1 | 2 | 0 | R | H | 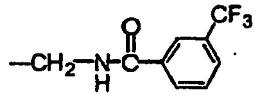 |
| 1547 | 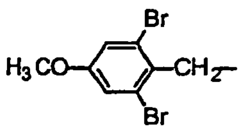 | 1 | 2 | 0 | R | H | 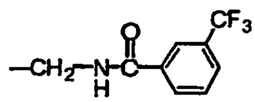 |
| 1548 | 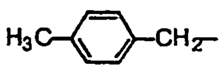 | 1 | 2 | 0 | R | H | 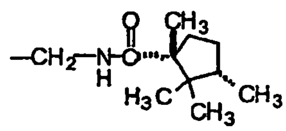 |
| 1549 | 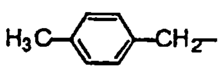 | 1 | 2 | 0 | R | H | 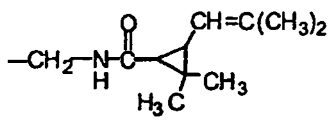 |
| 1550 | 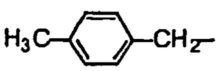 | 1 | 2 | 0 | R | H | 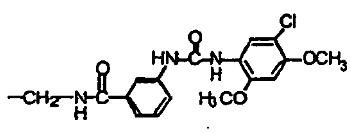 |
| 1551 | 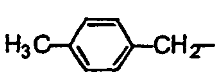 | 1 | 2 | 0 | R | H | 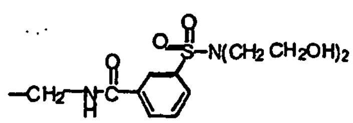 |

**Table 1.142**

| Compd. No. | 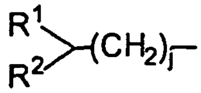 | k | m | n | chirality | $R^3$ | 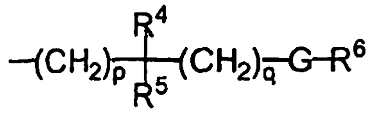 |
|---|---|---|---|---|---|---|---|
| 1552 | 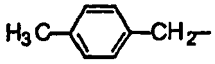 | 1 | 2 | 0 | R | H | 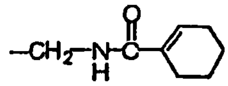 |
| 1553 | 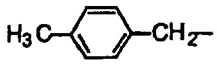 | 1 | 2 | 0 | R | H | 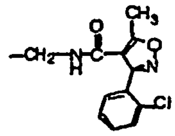 |
| 1554 | 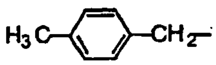 | 1 | 2 | 0 | R | H | 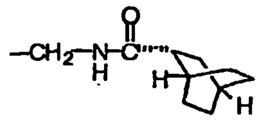 |
| 1555 | 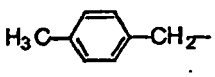 | 1 | 2 | 0 | R | H | 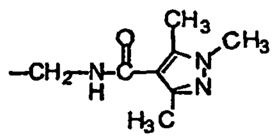 |
| 1556 | 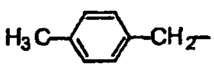 | 1 | 2 | 0 | R | H | 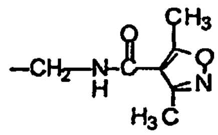 |
| 1557 | 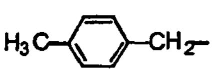 | 1 | 2 | 0 | R | H | 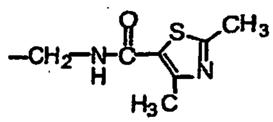 |
| 1558 | 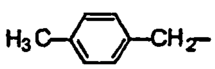 | 1 | 2 | 0 | R | H | 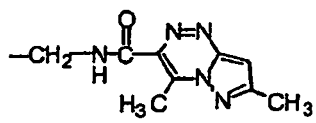 |
| 1559 | 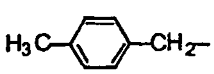 | 1 | 2 | 0 | R | H | 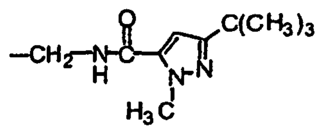 |
| 1560 | 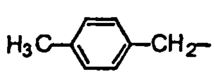 | 1 | 2 | 0 | R | H | 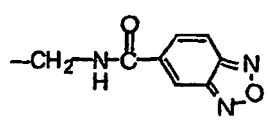 |
| 1561 | 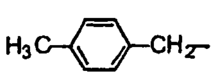 | 1 | 2 | 0 | R | H | 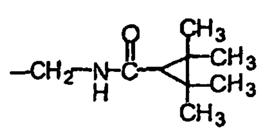 |

(continued)

| Compd. No. | 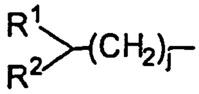 | k | m | n | chirality | $R^3$ | 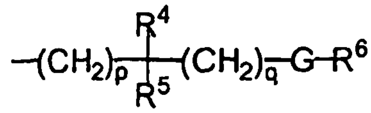 |
|---|---|---|---|---|---|---|---|
| 1562 | 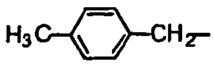 | 1 | 2 | 0 | R | H | 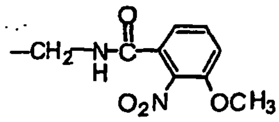 |

**Table 1.143**

| Compd. No. | 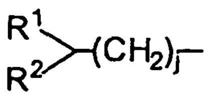 | k | m | n | chirality | $R^3$ | 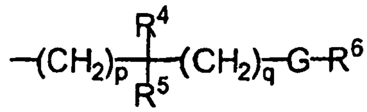 |
|---|---|---|---|---|---|---|---|
| 1563 | 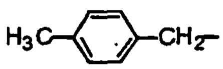 | 1 | 2 | 0 | R | H | 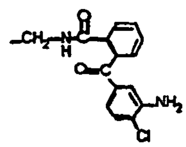 |
| 1564 | 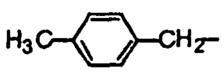 | 1 | 2 | 0 | R | H | 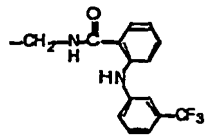 |
| 1565 | 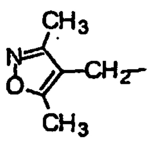 | 1 | 2 | 0 | R | H | 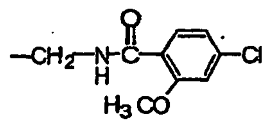 |
| 1566 | 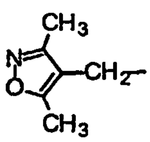 | 1 | 2 | 0 | R | H | 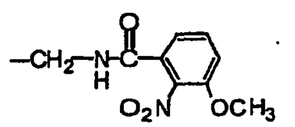 |
| 1567 | 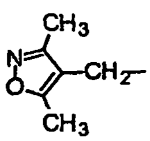 | 1 | 2 | 0 | R | H | 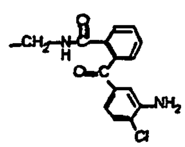 |
| 1568 | 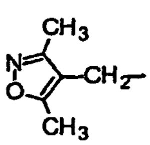 | 1 | 2 | 0 | R | H | 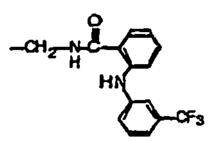 |
| 1569 | 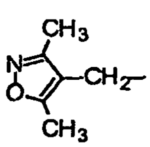 | 1 | 2 | 0 | R | H | 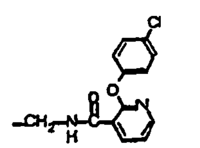 |

(continued)

| Compd. No. | 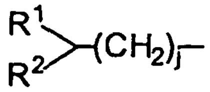 | k | m | n | chirality | $R^3$ | 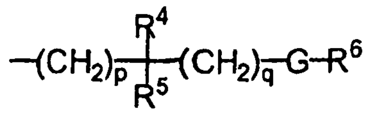 |
|---|---|---|---|---|---|---|---|
| 1570 | 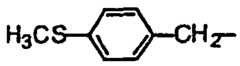 | 2 | 2 | 1 | - | H | 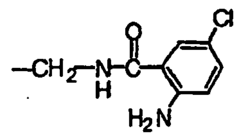 |
| 1571 | 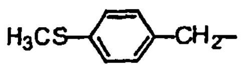 | 2 | 2 | 1 | - | H | 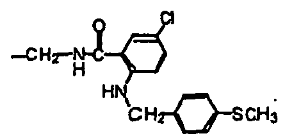 |
| 1572 | 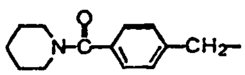 | 2 | 2 | 1 | - | H | 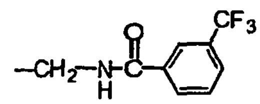 |
| 1573 | 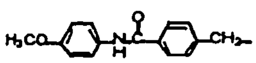 | 2 | 2 | 1 | - | H | 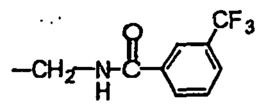 |

**Table 1.144**

| Compd. No. | 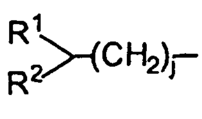 | k | m | n | chirality | $R^3$ | 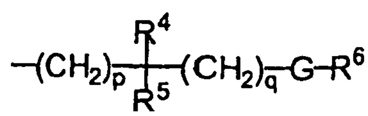 |
|---|---|---|---|---|---|---|---|
| 1574 | 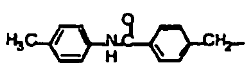 | 2 | 2 | 1 | - | H | 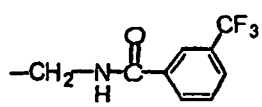 |
| 1575 | 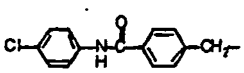 | 2 | 2 | 1 | - | H | 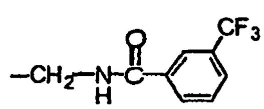 |
| 1576 | 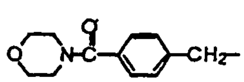 | 2 | 2 | 1 | - | H | 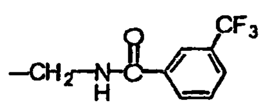 |
| 1577 | 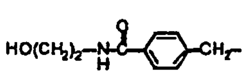 | 2 | 2 | 1 | - | H | 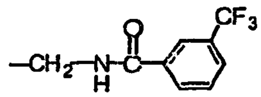 |
| 1578 | 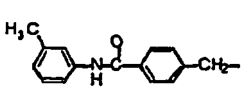 | 2 | 2 | 1 | - | H | 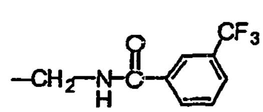 |

(continued)

| Compd. No. | 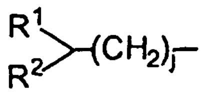 | k | m | n | chirality | $R^3$ | 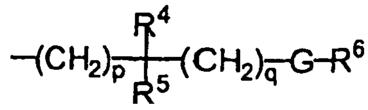 |
|---|---|---|---|---|---|---|---|
| 1579 | 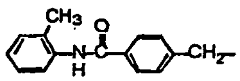 | 2 | 2 | 1 | - | H | 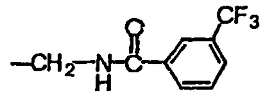 |
| 1580 | 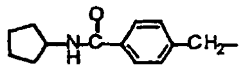 | 2 | 2 | 1 | - | H | 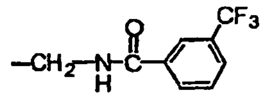 |
| 1581 | 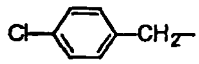 | 2 | 2 | 1 | - | H | 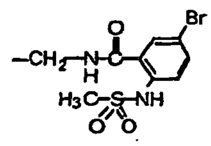 |
| 1582 | 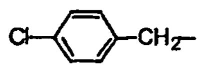 | 2 | 2 | 1 | - | H | 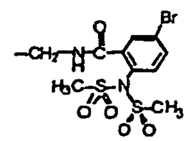 |
| 1583 | 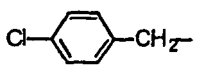 | 1 | 2 | 0 | R | H | 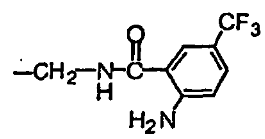 |
| 1584 | 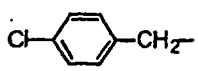 | 1 | 2 | 0 | R | H | 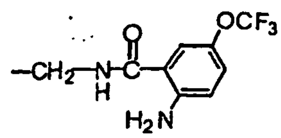 |

**Table 1.145**

| Compd. No. | 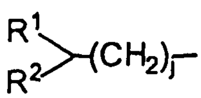 | k | m | n | chirality | $R^3$ | 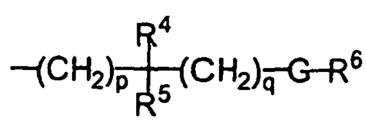 |
|---|---|---|---|---|---|---|---|
| 1585 | 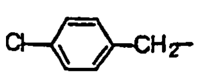 | 1 | 2 | 0 | R | H | 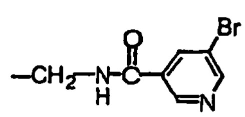 |
| 1586 | 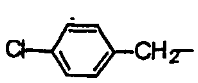 | 1 | 2 | 0 | R | H | 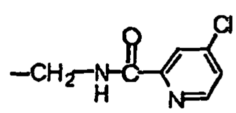 |

(continued)

| Compd. No. | 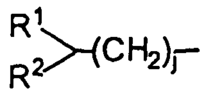 | k | m | n | chirality | $R^3$ | 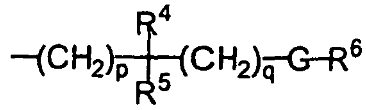 |
|---|---|---|---|---|---|---|---|
| 1587 | 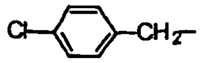 | 1 | 2 | 0 | R | H | 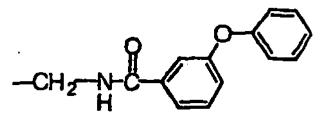 |
| 1588 | 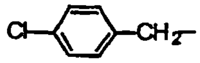 | 1 | 2 | 0 | R | H | 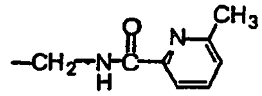 |
| 1589 | 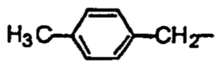 | 1 | 2 | 0 | R | H | 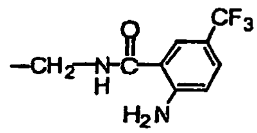 |
| 1590 | 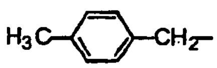 | 1 | 2 | 0 | R | H | 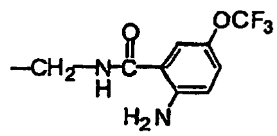 |
| 1591 | 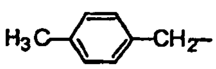 | 1 | 2 | 0 | R | H | 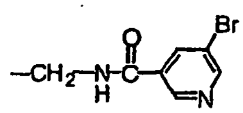 |
| 1592 | 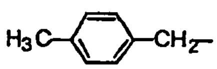 | 1 | 2 | 0 | R | H | 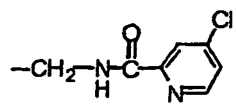 |
| 1593 | 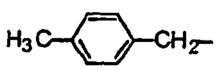 | 1 | 2 | 0 | R | H | 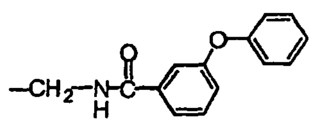 |
| 1594 | 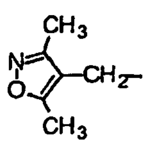 | 1 | 2 | 0 | R | H | 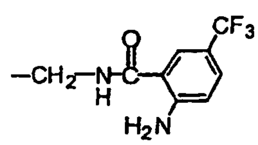 |
| 1595 | 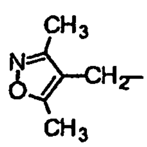 | 1 | 2 | 0 | R | H | 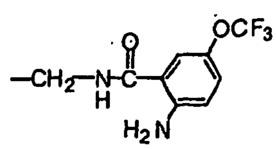 |

**Table 1.146**

| Compd. No. | 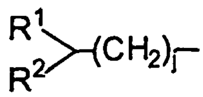 | k | m | n | chirality | $R^3$ | 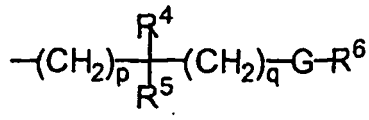 |
|---|---|---|---|---|---|---|---|
| 1596 | 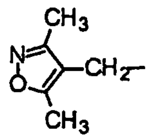 | 1 | 2 | 0 | R | H | 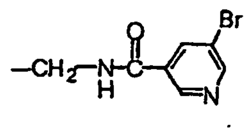 |
| 1597 | 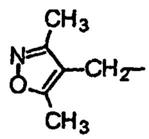 | 1 | 2 | 0 | R | H | 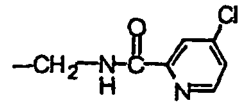 |
| 1598 | 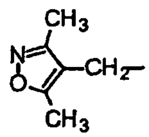 | 1 | 2 | 0 | R | H | 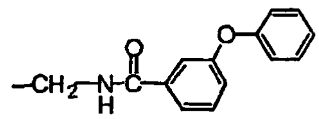 |
| 1599 | 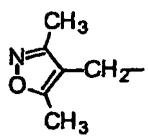 | 1 | 2 | 0 | R | H | 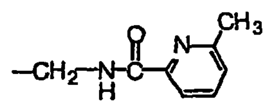 |
| 1600 | 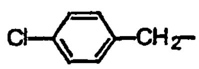 | 2 | 2 | 1 | - | H | 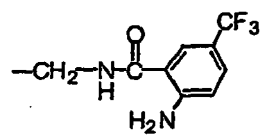 |
| 1601 | 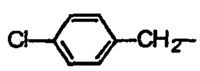 | 2 | 2 | 1 | - | H | 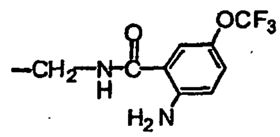 |
| 1602 | 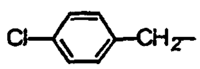 | 2 | 2 | 1 | - | H | 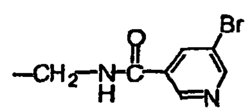 |
| 1603 | 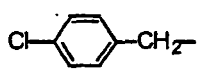 | 2 | 2 | 1 | - | H | 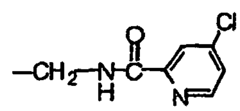 |
| 1604 | 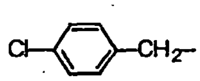 | 2 | 2 | 1 | - | H | 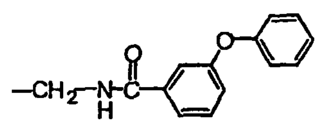 |

(continued)

| Compd. No. | 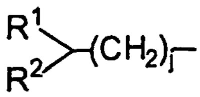 | k | m | n | chirality | $R^3$ | 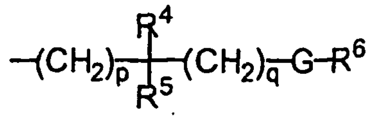 |
|---|---|---|---|---|---|---|---|
| 1605 | 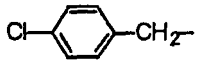 | 2 | 2 | 1 | - | H | 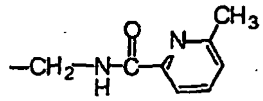 |
| 1606 | 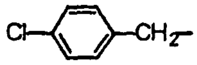 | 1 | 2 | 0 | R | H | 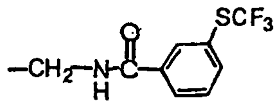 |

**Table 1.147**

| Compd. No. | 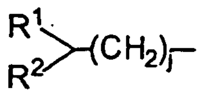 | k | m | n | chirality | $R^3$ | 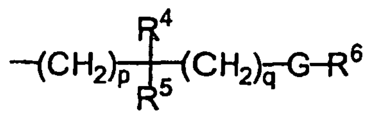 |
|---|---|---|---|---|---|---|---|
| 1607 | 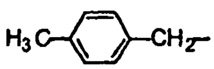 | 1 | 2 | 0 | R | H | 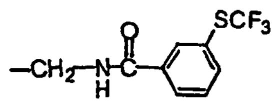 |
| 1608 | 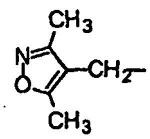 | 1 | 2 | 0 | R | H | 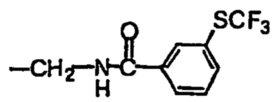 |
| 1609 | 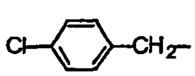 | 2 | 2 | 1 | - | H | 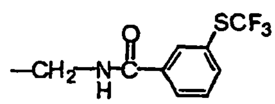 |
| 1610 | 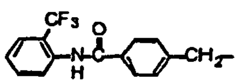 | 2 | 2 | 1 | - | H | 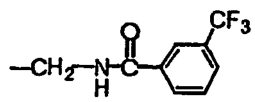 |
| 1611 | 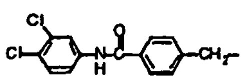 | 2 | 2 | 1 | - | H | 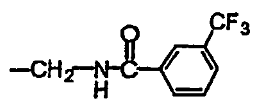 |
| 1612 | 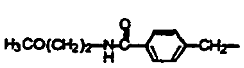 | 2 | 2 | 1 | - | H | 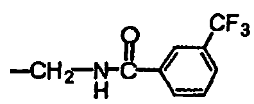 |

(continued)

| Compd. No. | 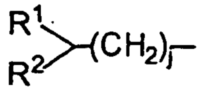 | k | m | n | chirality | $R^3$ | 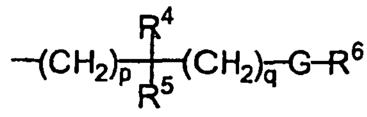 |
|---|---|---|---|---|---|---|---|
| 1613 | 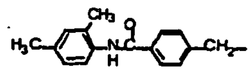 | 2 | 2 | 1 | - | H | 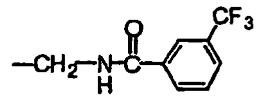 |
| 1614 | 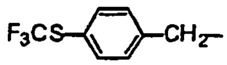 | 1 | 2 | 0 | R | H | 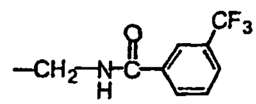 |
| 1615 | 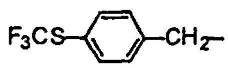 | 2 | 2 | 1 | - | H | 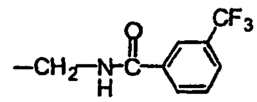 |
| 1616 | 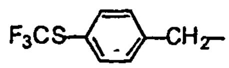 | 2 | 2 | 1 | - | H | 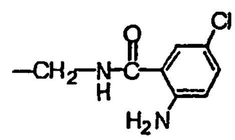 |
| 1617 | 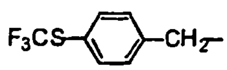 | 2 | 2 | 1 | - | H | 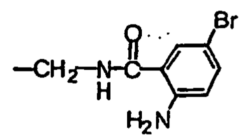 |

**Table 1.148**

| Compd. No. | 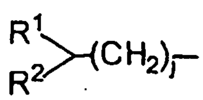 | k | m | n | chirality | $R^3$ | 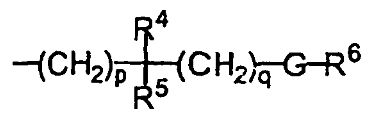 |
|---|---|---|---|---|---|---|---|
| 1618 | 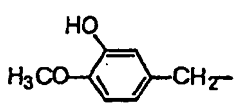 | 1 | 2 | 0 | R | H | 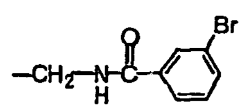 |
| 1619 | 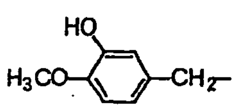 | 1 | 2 | 0 | R | H | 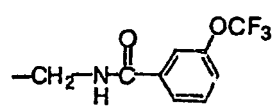 |
| 1620 | 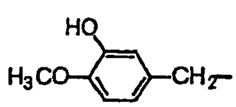 | 1 | 2 | 0 | R | H | 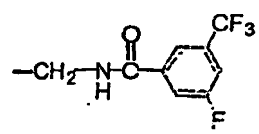 |

(continued)

| Compd. No. | 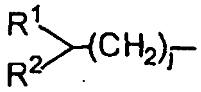 | k | m | n | chirality | $R^3$ | 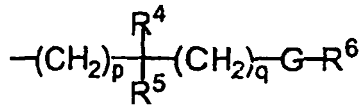 |
|---|---|---|---|---|---|---|---|
| 1621 | 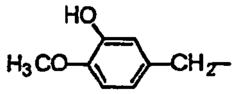 | 1 | 2 | 0 | R | H | 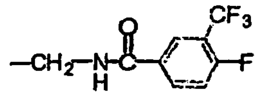 |
| 1622 | 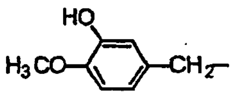 | 1 | 2 | 0 | R | H | 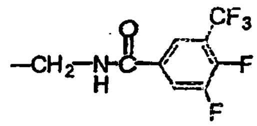 |
| 1623 | 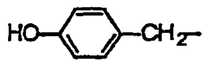 | 1 | 2 | 0 | R | H | 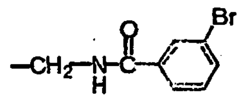 |
| 1624 | 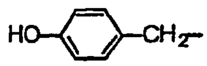 | 1 | 2 | 0 | R | H | 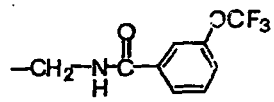 |
| 1625 | 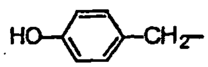 | 1 | 2 | 0 | R | H | 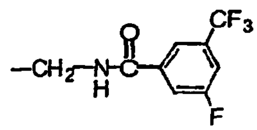 |
| 1626 | 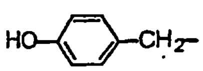 | 1 | 2 | 0 | R | H | 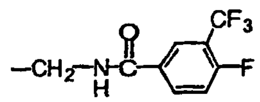 |
| 1627 | 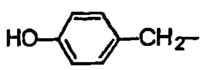 | 1 | 2 | 0 | R | H | 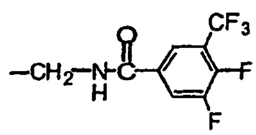 |
| 1628 | 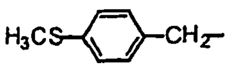 | 1 | 2 | 0 | R | H | 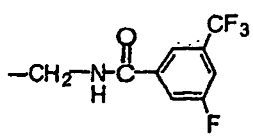 |

**Table 1.149**

| Compd. No. | 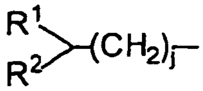 | k | m | n | chirality | $R^3$ | 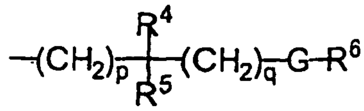 |
|---|---|---|---|---|---|---|---|
| 1629 | 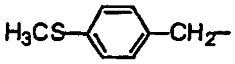 | 1 | 2 | 0 | R | H | 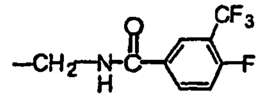 |
| 1630 | 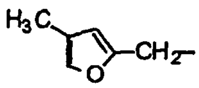 | 1 | 2 | 0 | R | H | 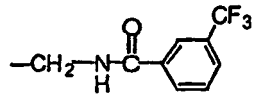 |
| 1631 | 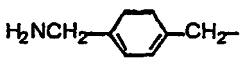 | 1 | 2 | 0 | R | H | 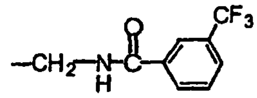 |
| 1632 | 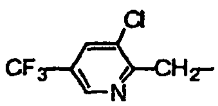 | 1 | 2 | 0 | R | H | 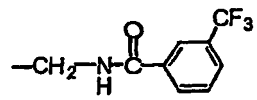 |
| 1633 | 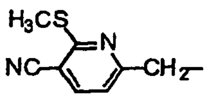 | 1 | 2 | 0 | R | H | 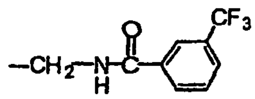 |
| 1634 | 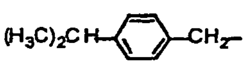 | 1 | 2 | 0 | R | H | 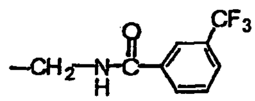 |
| 1635 | 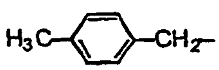 | 1 | 2 | 0 | R | H | 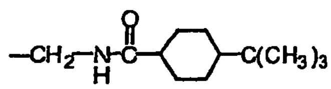 |
| 1636 | 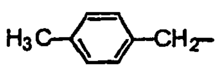 | 1 | 2 | 0 | R | H | 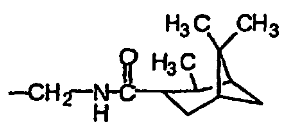 |
| 1637 | 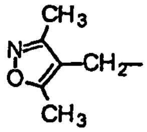 | 1 | 2 | 0 | R | H | 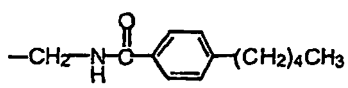 |
| 1638 | 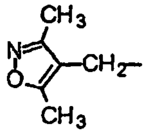 | 1 | 2 | 0 | R | H | 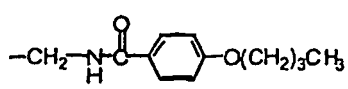 |

(continued)

| Compd. No. | 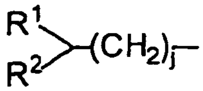 | k | m | n | chirality | $R^3$ | 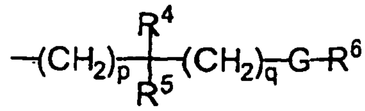 |
|---|---|---|---|---|---|---|---|
| 1639 | 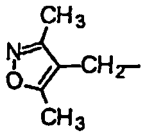 | 1 | 2 | 0 | R | H | 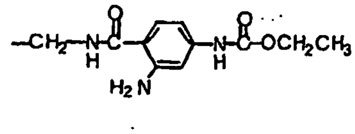 |

**Table 1.150**

| Compd. No. | 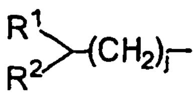 | k | m | n | chirality | $R^3$ | 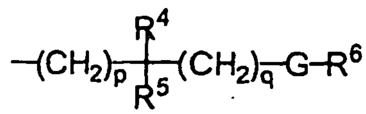 |
|---|---|---|---|---|---|---|---|
| 1640 | 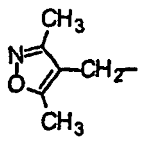 | 1 | 2 | 0 | R | H | 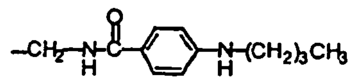 |
| 1641 | 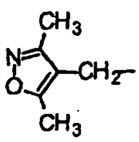 | 1 | 2 | 0 | R | H | 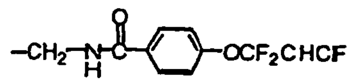 |
| 1642 | 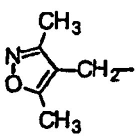 | 1 | 2 | 0 | R | H | 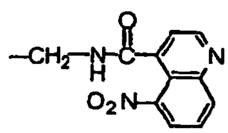 |
| 1643 | 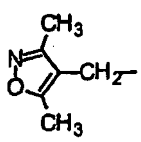 | 1 | 2 | 0 | R | H | 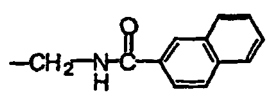 |
| 1644 | 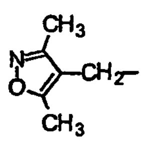 | 1 | 2 | 0 | R | H | 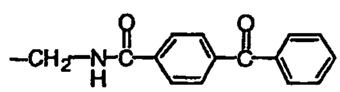 |
| 1645 | 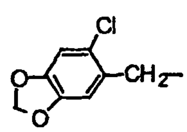 | 1 | 2 | 0 | R | H | 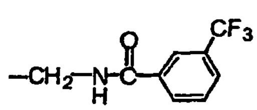 |
| 1646 | 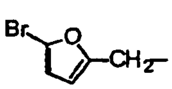 | 1 | 2 | 0 | R | H | 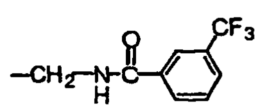 |

(continued)

| Compd. No. | 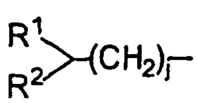 | k | m | n | chirality | $R^3$ | 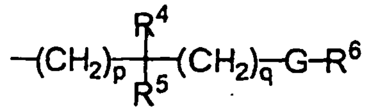 |
|---|---|---|---|---|---|---|---|
| 1647 | 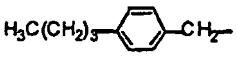 | 2 | 2 | 1 | - | H | 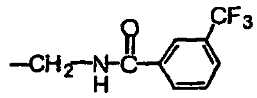 |
| 1648 | 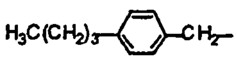 | 1 | 2 | 0 | R | H | 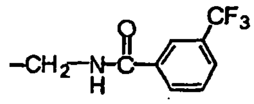 |
| 1649 | 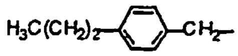 | 2 | 2 | 1 | - | H | 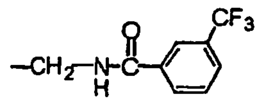 |
| 1650 | 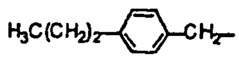 | 1 | 2 | 0 | R | H | 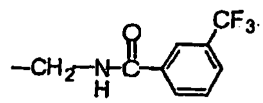 |

**Table 1.151**

| Compd. No. | 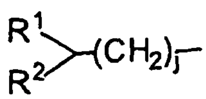 | k | m | n | chirality | $R^3$ | 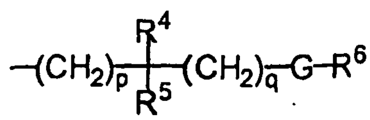 |
|---|---|---|---|---|---|---|---|
| 1651 | 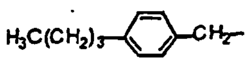 | 2 | 2 | 1 | - | H | 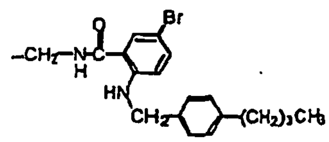 |
| 1652 | 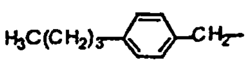 | 2 | 2 | 1 | - | H | 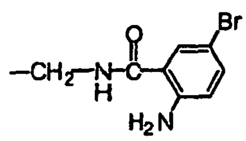 |
| 1653 | 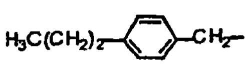 | 2 | 2 | 1 | - | H | 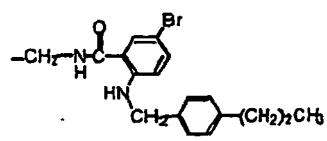 |
| 1654 | 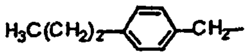 | 2 | 2 | 1 | - | H | 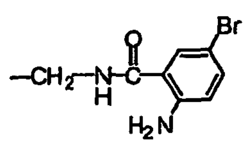 |

(continued)

| Compd. No. | 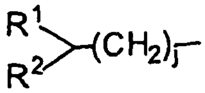 | k | m | n | chirality | $R^3$ | 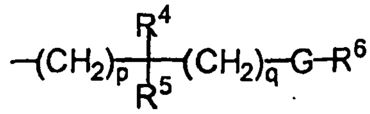 |
|---|---|---|---|---|---|---|---|
| 1655 | 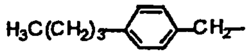 | 2 | 2 | 1 | - | H | 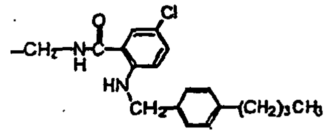 |
| 1656 | 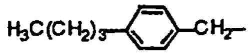 | 2 | 2 | 1 | - | H | 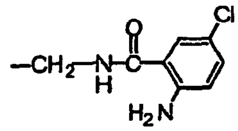 |
| 1657 | 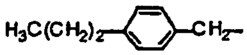 | 2 | 2 | 1 | - | H | 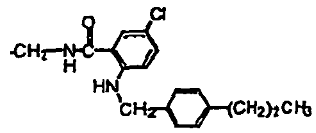 |
| 1658 | 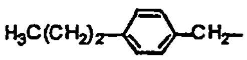 | 2 | 2 | 1 | - | H | 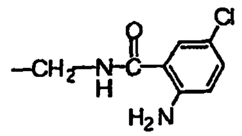 |
| 1659 | 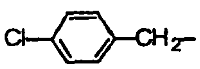 | 2 | 2 | 1 | - | H | 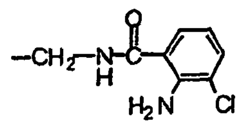 |
| 1660 | 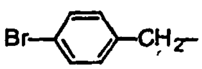 | 1 | 2 | 0 | R | H | 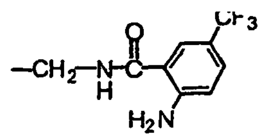 |
| 1661 | 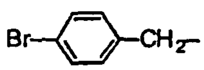 | 1 | 2 | 0 | R | H | 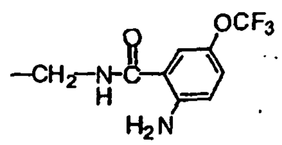 |

**Table 1.152**

| Compd. No. | 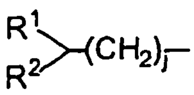 | k | m | n | chirality | $R^3$ | 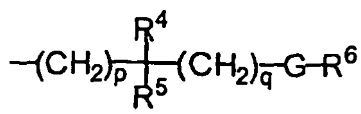 |
|---|---|---|---|---|---|---|---|
| 1662 | 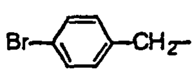 | 1 | 2 | 0 | R | H | 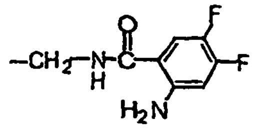 |

(continued)

| Compd. No. | 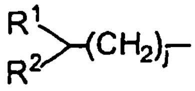 | k | m | n | chirality | $R^3$ | 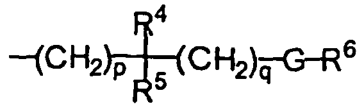 |
|---|---|---|---|---|---|---|---|
| 1663 | 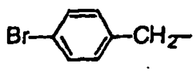 | 1 | 2 | 0 | R | H | 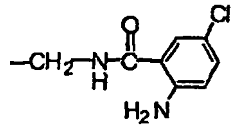 |
| 1664 | 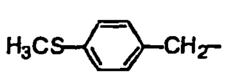 | 2 | 2 | 1 | - | H | 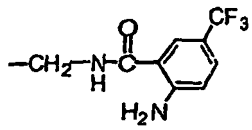 |
| 1665 | 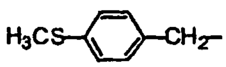 | 2 | 2 | 1 | - | H | 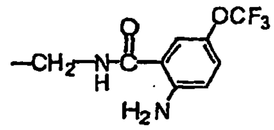 |
| 1666 | 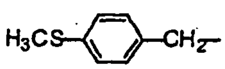 | 2 | 2 | 1 | - | H | 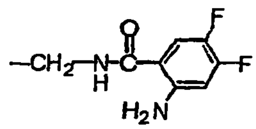 |
| 1667 | 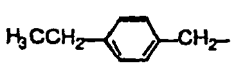 | 2 | 2 | 1 | - | H | 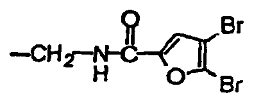 |
| 1668 | 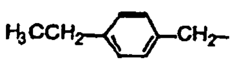 | 2 | 2 | 1 | - | H | 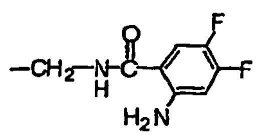 |
| 1669 | 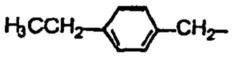 | 2 | 2 | 1 | - | H | 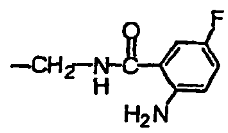 |
| 1670 | 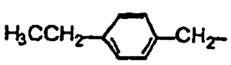 | 2 | 2 | 1 | - | H | 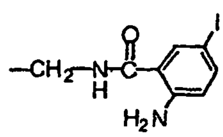 |
| 1671 | 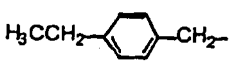 | 2 | 2 | 1 | - | H | 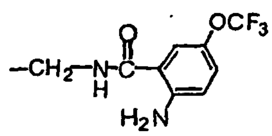 |
| 1672 | 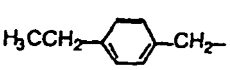 | 2 | 2 | 1 | - | H | 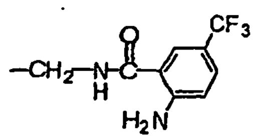 |

**Table 1.153**

| Compd. No. | 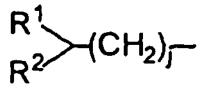 | k | m | n | chirality | $R^3$ | 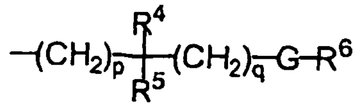 |
|---|---|---|---|---|---|---|---|
| 1673 | 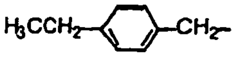 | 2 | 2 | 1 | - | H | 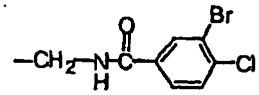 |
| 1674 | 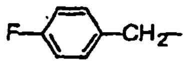 | 2 | 2 | 1 | - | H | 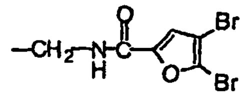 |
| 1675 | 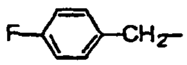 | 2 | 2 | 1 | - | H | 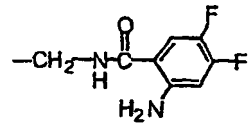 |
| 1676 | 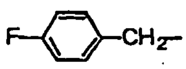 | 2 | 2 | 1 | - | H | 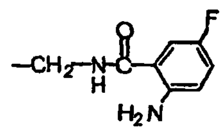 |
| 1677 | 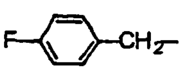 | 2 | 2 | 1 | - | H | 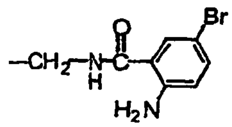 |
| 1678 | 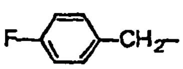 | 2 | 2 | 1 | - | H | 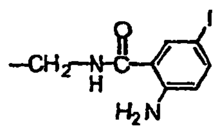 |
| 1679 | 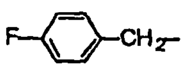 | 2 | 2 | 1 | - | H | 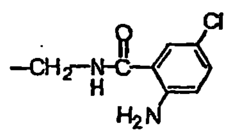 |
| 1680 | 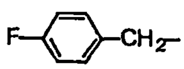 | 2 | 2 | 1 | - | H | 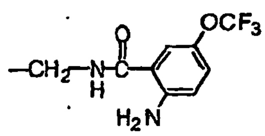 |
| 1681 | 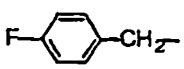 | 2 | 2 | 1 | - | H | 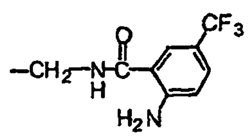 |
| 1682 | 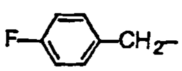 | 2 | 2 | 1 | - | H | 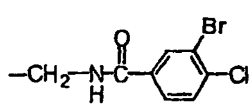 |

(continued)

| Compd. No. | 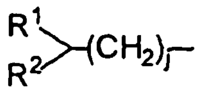 | k | m | n | chirality | $R^3$ | 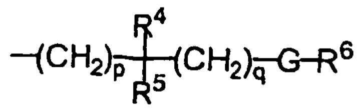 |
|---|---|---|---|---|---|---|---|
| 1683 | 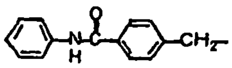 | 2 | 2 | 1 | - | H | 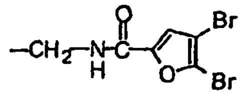 |

**Table 1.154**

| Compd. No. | 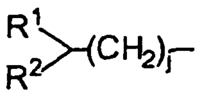 | k | m | n | chirality | $R^3$ | 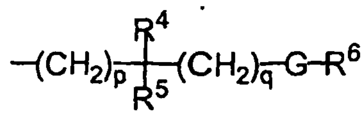 |
|---|---|---|---|---|---|---|---|
| 1684 | 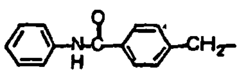 | 2 | 2 | 1 | - | H | 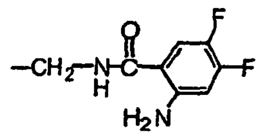 |
| 1685 | 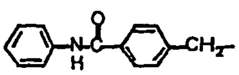 | 2 | 2 | 1 | - | H | 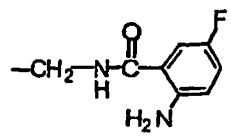 |
| 1686 | 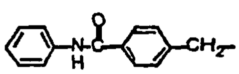 | 2 | 2 | 1 | - | H | 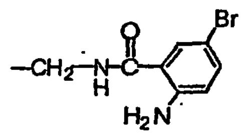 |
| 1687 | 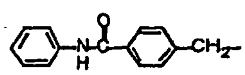 | 2 | 2 | 1 | - | H | 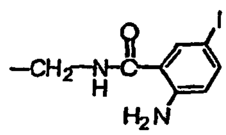 |
| 1688 | 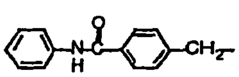 | 2 | 2 | 1 | - | H | 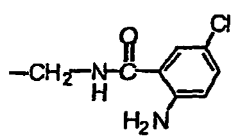 |
| 1689 | 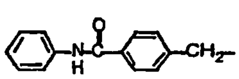 | 2 | 2 | 1 | - | H | 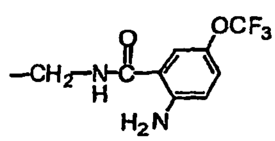 |
| 1690 | 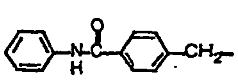 | 2 | 2 | 1 | - | H | 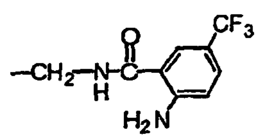 |

(continued)

| Compd. No. | 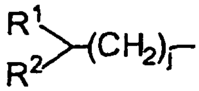 | k | m | n | chirality | $R^3$ | 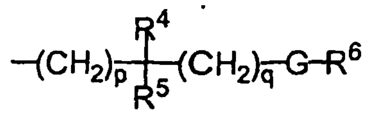 |
|---|---|---|---|---|---|---|---|
| 1691 | 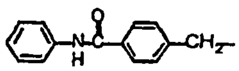 | 2 | 2 | 1 | - | H | 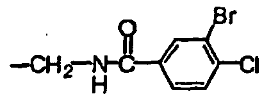 |
| 1692 | 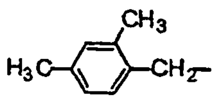 | 1 | 2 | 0 | R | H | 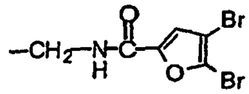 |
| 1693 | 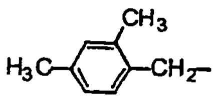 | 1 | 2 | 0 | R | H | 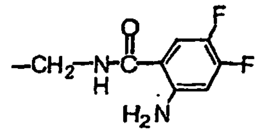 |
| 1694 | 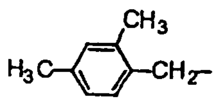 | 1 | 2 | 0 | R | H | 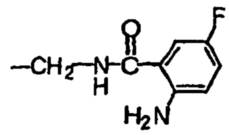 |

**Table 1.155**

| Compd. No. | 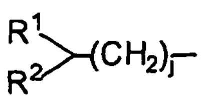 | k | m | n | chirality | $R^3$ | 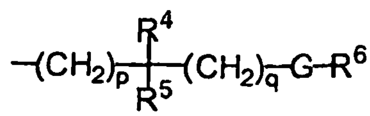 |
|---|---|---|---|---|---|---|---|
| 1695 | 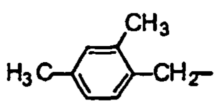 | 1 | 2 | 0 | R | H | 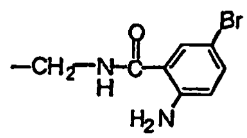 |
| 1696 | 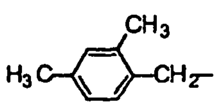 | 1 | 2 | 0 | R | H | 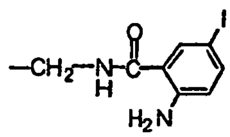 |
| 1697 | 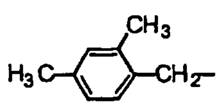 | 1 | 2 | 0 | R | H | 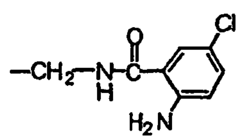 |
| 1698 | 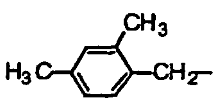 | 1 | 2 | 0 | R | H | 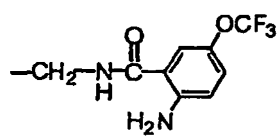 |

(continued)

| Compd. No. | 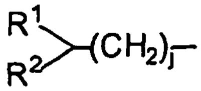 | k | m | n | chirality | $R^3$ | 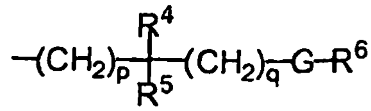 |
|---|---|---|---|---|---|---|---|
| 1699 | 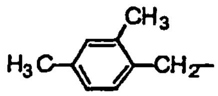 | 1 | 2 | 0 | R | H | 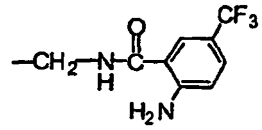 |
| 1700 | 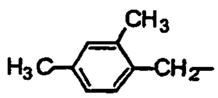 | 1 | 2 | 0 | R | H | 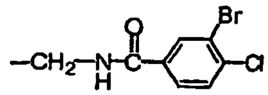 |
| 1701 | 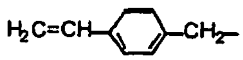 | 1 | 2 | 0 | R | H | 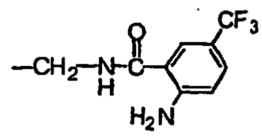 |
| 1702 | 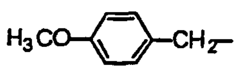 | 1 | 2 | 0 | R | H | 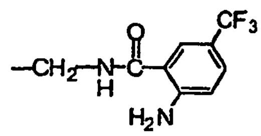 |
| 1703 | 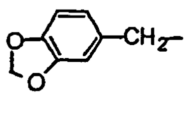 | 1 | 2 | 0 | R | H | 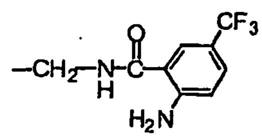 |
| 1704 | 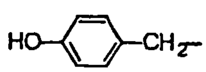 | 1 | 2 | 0 | R | H | 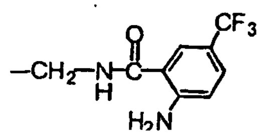 |
| 1705 | 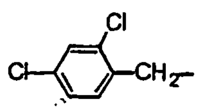 | 1 | 2 | 0 | R | H | 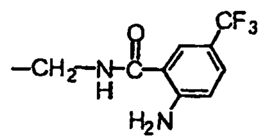 |

**Table 1.156**

| Compd. No. | 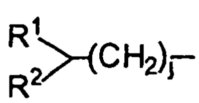 | k | m | n | chirality | $R^3$ | 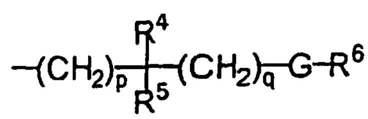 |
|---|---|---|---|---|---|---|---|
| 1706 | 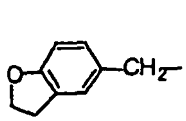 | 1 | 2 | 0 | R | H | 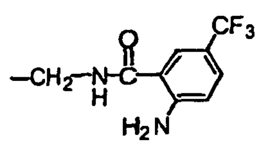 |

(continued)

| Compd. No. | 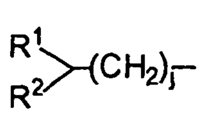 | k | m | n | chirality | $R^3$ | 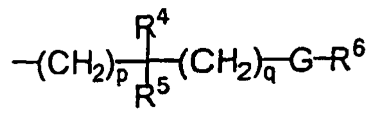 |
|---|---|---|---|---|---|---|---|
| 1707 | 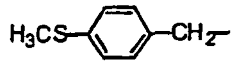 | 1 | 2 | 0 | R | H | 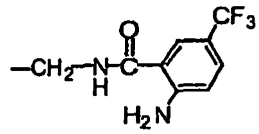 |
| 1708 | 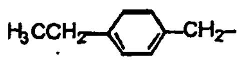 | 1 | 2 | 0 | R | H | 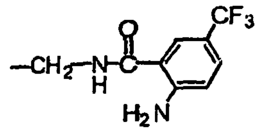 |
| 1709 | 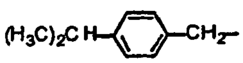 | 1 | 2 | 0 | R | H | 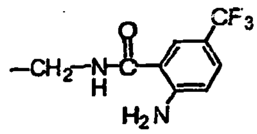 |
| 1710 | 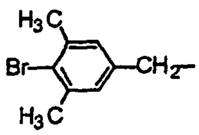 | 1 | 2 | 0 | R | H | 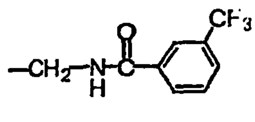 |
| 1711 | 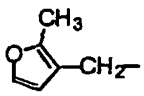 | 1 | 2 | 0 | R | H | 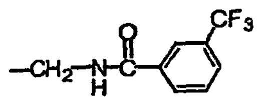 |
| 1712 | 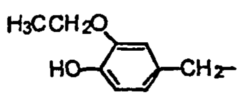 | 1 | 2 | 0 | R | H | 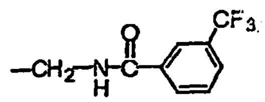 |
| 1713 | 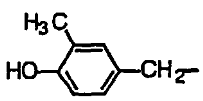 | 1 | 2 | 0 | R | H | 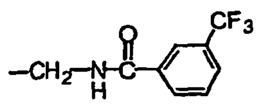 |
| 1714 | 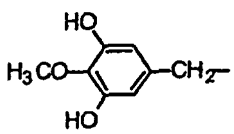 | 1 | 2 | 0 | R | H | 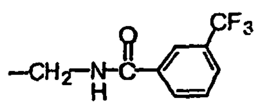 |
| 1715 | 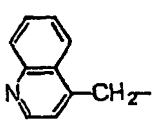 | 1 | 2 | 0 | R | H | 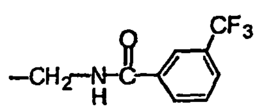 |
| 1716 | 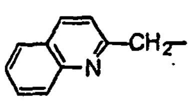 | 1 | 2 | 0 | R | H | 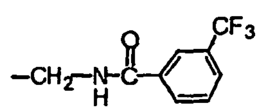 |

**Table 1.157**

| Compd. No. | 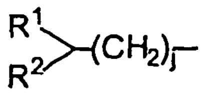 | k | m | n | chirality | $R^3$ | 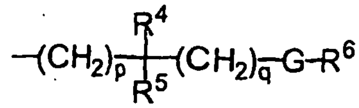 |
|---|---|---|---|---|---|---|---|
| 1717 | 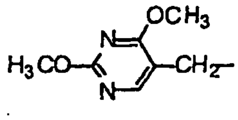 | 1 | 2 | 0 | R | H | 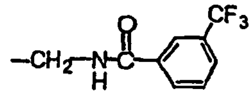 |
| 1718 | 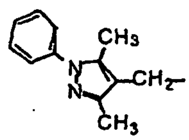 | 1 | 2 | 0 | R | H | 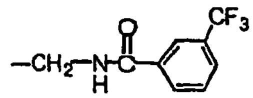 |
| 1719 | 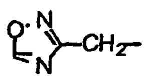 | 1 | 2 | 0 | R | H | 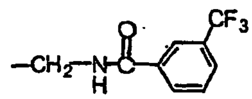 |
| 1720 | 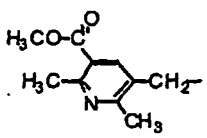 | 1 | 2 | 0 | R | H | 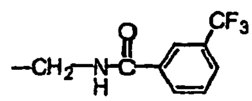 |
| 1721 | 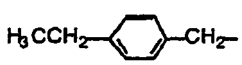 | 1 | 2 | 0 | R | H | 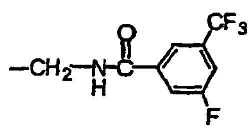 |
| 1722 | 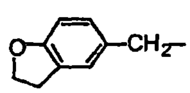 | 1 | 2 | 0 | R | H | 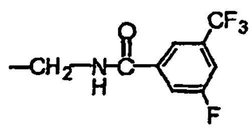 |
| 1723 | 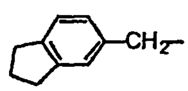 | 1 | 2 | 0 | R | H | 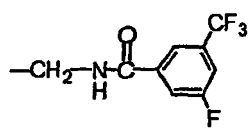 |
| 1724 | 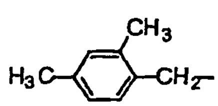 | 1 | 2 | 0 | R | H | 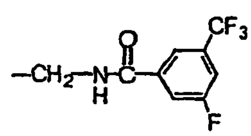 |
| 1725 | 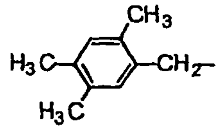 | 1 | 2 | 0 | R | H | 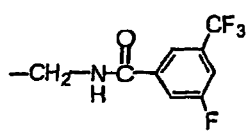 |
| 1726 | 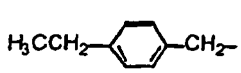 | 1 | 2 | 0 | R | H | 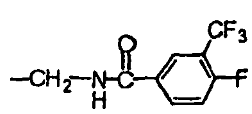 |

(continued)

| Compd. No. | 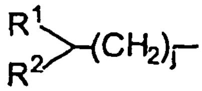 | k | m | n | chirality | $R^3$ | 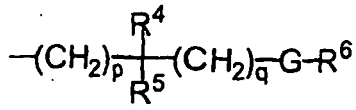 |
|---|---|---|---|---|---|---|---|
| 1727 | 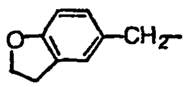 | 1 | 2 | 0 | R | H | 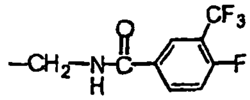 |

**Table 1.158**

| Compd. No. | 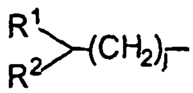 | k | m | n | chirality | $R^3$ | 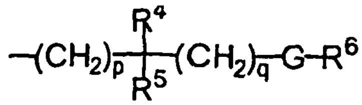 |
|---|---|---|---|---|---|---|---|
| 1728 | 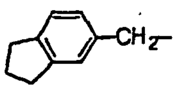 | 1 | 2 | 0 | R | H | 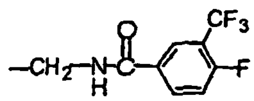 |
| 1729 | 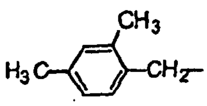 | 1 | 2 | 0 | R | H | 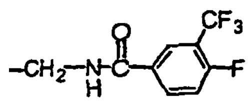 |
| 1730 | 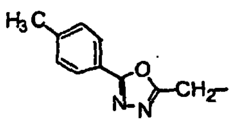 | 1 | 2 | 0 | R | H | 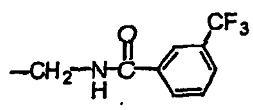 |
| 1731 | 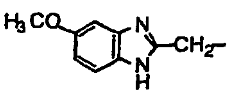 | 1 | 2 | 0 | R | H | 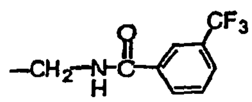 |
| 1732 | 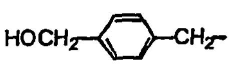 | 1 | 2 | 0 | R | H | 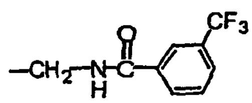 |
| 1733 | 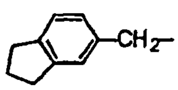 | 1 | 2 | 0 | R | H | 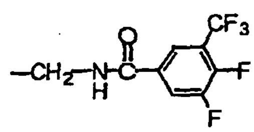 |
| 1734 | 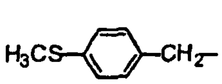 | 1 | 2 | 0 | R | H | 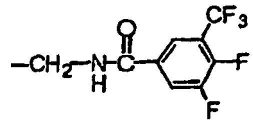 |

(continued)

| Compd. No. | 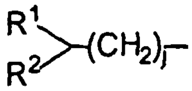 | k | m | n | chirality | $R^3$ | 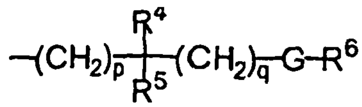 |
|---|---|---|---|---|---|---|---|
| 1735 | 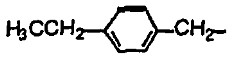 | 1 | 2 | 0 | R | H | 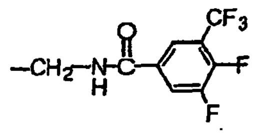 |
| 1736 | 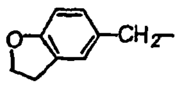 | 1 | 2 | 0 | R | H | 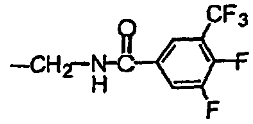 |
| 1737 | 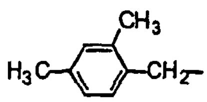 | 1 | 2 | 0 | R | H | 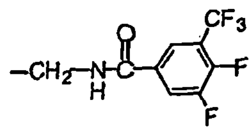 |
| 1738 | 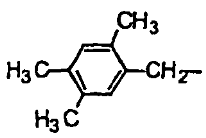 | 1 | 2 | 0 | R | H | 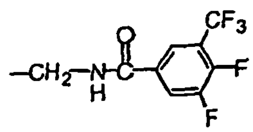 |

**Table 1.159**

| Compd. No. | 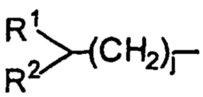 | k | m | n | chirality | $R^3$ | 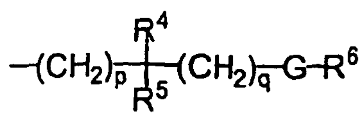 |
|---|---|---|---|---|---|---|---|
| 1739 | 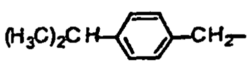 | 1 | 2 | 0 | R | H | 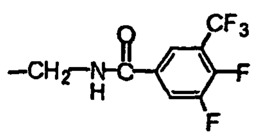 |
| 1740 | 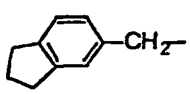 | 1 | 2 | 0 | R | H | 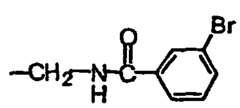 |
| 1741 | 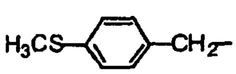 | 1 | 2 | 0 | R | H | 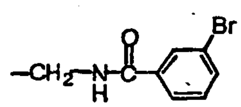 |
| 1742 | 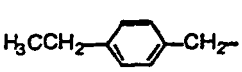 | 1 | 2 | 0 | R | H | 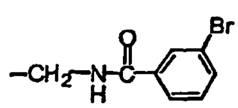 |

(continued)

| Compd. No. | 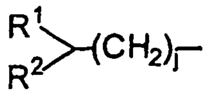 | k | m | n | chirality | $R^3$ | 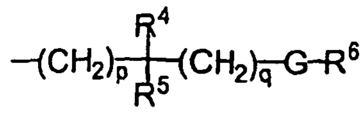 |
|---|---|---|---|---|---|---|---|
| 1743 | 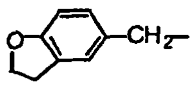 | 1 | 2 | 0 | R | H | 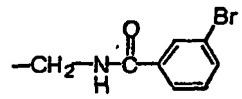 |
| 1744 | 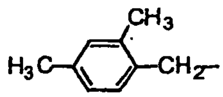 | 1 | 2 | 0 | R | H | 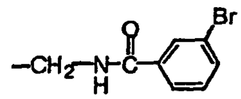 |
| 1745 | 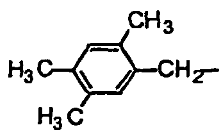 | 1 | 2 | 0 | R | H | 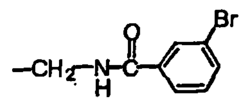 |
| 1746 | 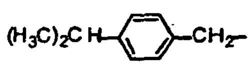 | 1 | 2 | 0 | R | H | 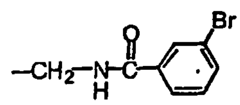 |
| 1747 | 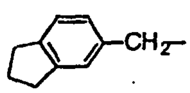 | 1 | 2 | 0 | R | H | 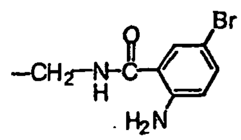 |
| 1748 | 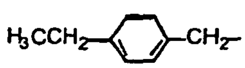 | 1 | 2 | 0 | R | H | 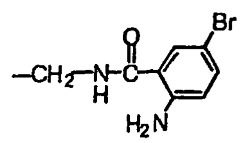 |
| 1749 | 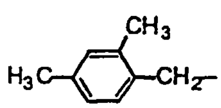 | 1 | 2 | 0 | R | H | 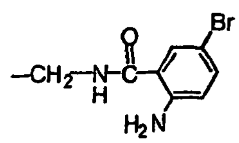 |

**Table 1.160**

| Compd. No. | 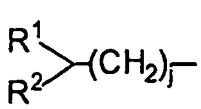 | k | m | n | chirality | $R^3$ | 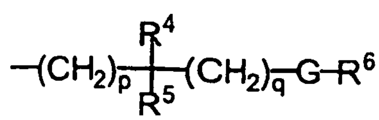 |
|---|---|---|---|---|---|---|---|
| 1750 | 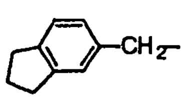 | 1 | 2 | 0 | R | H | 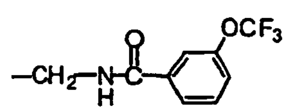 |

(continued)

| Compd. No. | 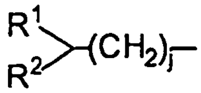 | k | m | n | chirality | $R^3$ | 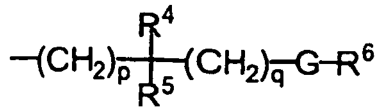 |
|---|---|---|---|---|---|---|---|
| 1751 | 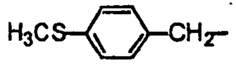 | 1 | 2 | 0 | R | H | 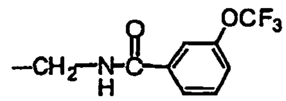 |
| 1752 | 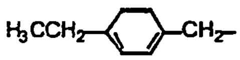 | 1 | 2 | 0 | R | H | 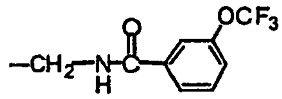 |
| 1753 | 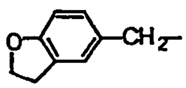 | 1 | 2 | 0 | R | H | 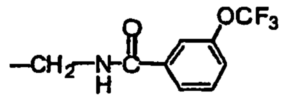 |
| 1754 | 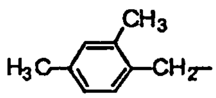 | 1 | 2 | 0 | R | H | 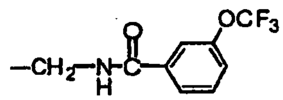 |
| 1755 | 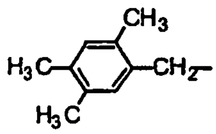 | 1 | 2 | 0 | R | H | 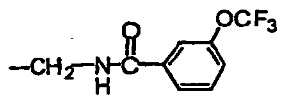 |
| 1756 | 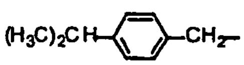 | 1 | 2 | 0 | R | H | 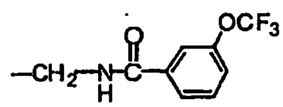 |
| 1757 | 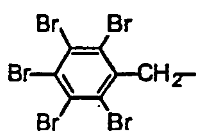 | 1 | 2 | 0 | R | H | 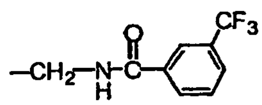 |
| 1758 | 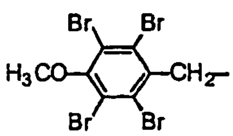 | 1 | 2 | 0 | R | H | 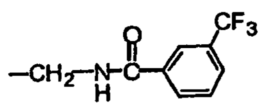 |
| 1759 | 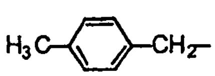 | 1 | 2 | 0 | R | H | 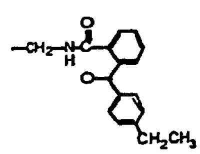 |
| 1760 | 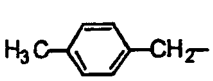 | 1 | 2 | 0 | R | H | 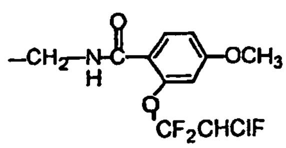 |

**Table 1.161**

| Compd. No. | 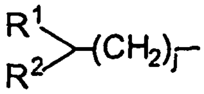 | k | m | n | chirality | $R^3$ | 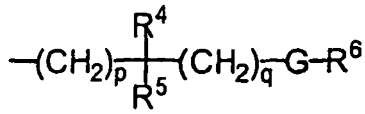 |
|---|---|---|---|---|---|---|---|
| 1761 | 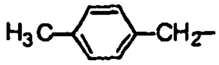 | 1 | 2 | 0 | R | H | 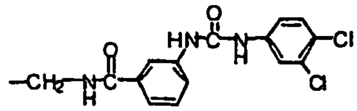 |
| 1762 | 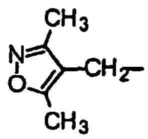 | 1 | 2 | 0 | R | H | 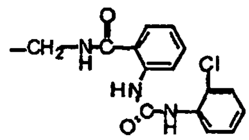 |
| 1763 | 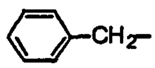 | 2 | 2 | 0 | - | H | 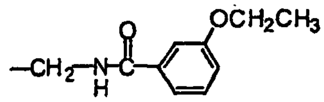 |
| 1764 | 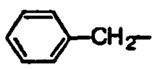 | 2 | 2 | 0 | - | H | 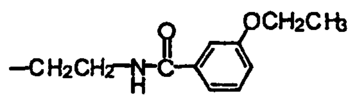 |
| 1765 | 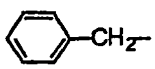 | 2 | 2 | 0 | - | H | 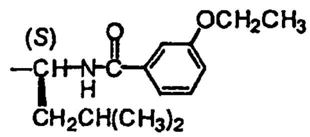 |
| 1766 | 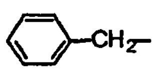 | 2 | 2 | 0 | - | H | 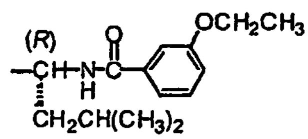 |
| 1767 | 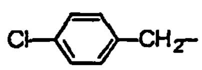 | 1 | 3 | 1 | - | H | 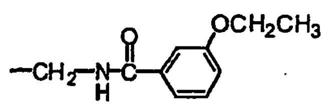 |
| 1768 | 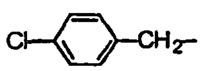 | 1 | 3 | 1 | - | H | 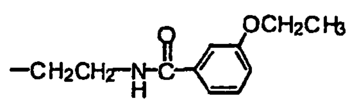 |
| 1769 | 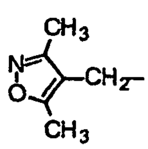 | 1 | 2 | 0 | R | H | 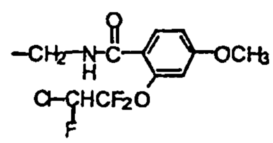 |
| 1770 | 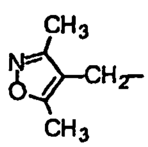 | 1 | 2 | 0 | R | H | 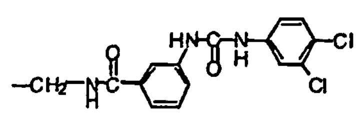 |

(continued)

| Compd. No. | 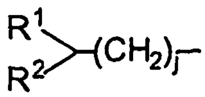 | k | m | n | chirality | $R^3$ | 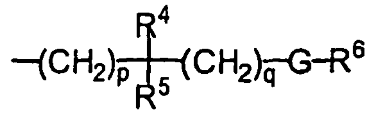 |
|---|---|---|---|---|---|---|---|
| 1771 | 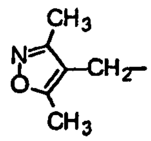 | 1 | 2 | 0 | R | H | 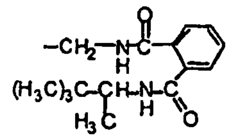 |

**Table 1.162**

| Compd. No. | 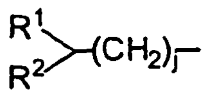 | k | m | n | chirality | $R^3$ | 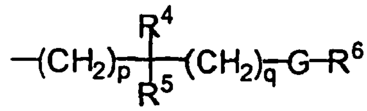 |
|---|---|---|---|---|---|---|---|
| 1772 | 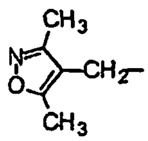 | 1 | 2 | 0 | R | H | 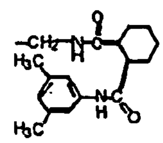 |
| 1773 | 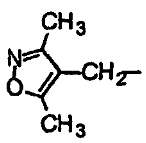 | 1 | 2 | 0 | R | H | 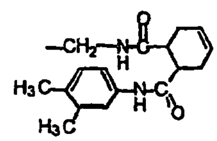 |
| 1774 | 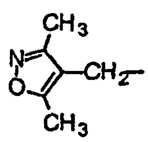 | 1 | 2 | 0 | R | H | 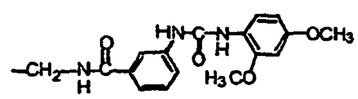 |
| 1775 | 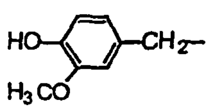 | 1 | 2 | 0 | R | H | 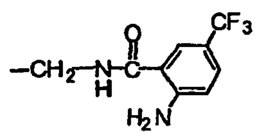 |
| 1776 | 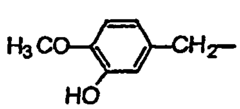 | 1 | 2 | 0 | R | H | 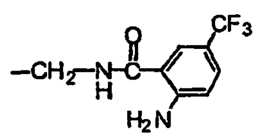 |
| 1777 | 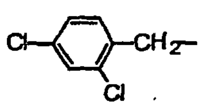 | 2 | 2 | 1 | - | H | 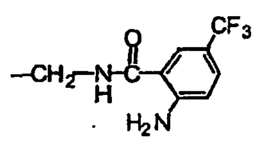 |
| 1778 | 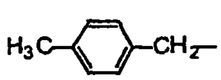 | 2 | 2 | 1 | - | H | 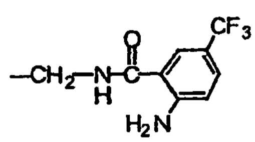 |

(continued)

| Compd. No. | 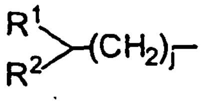 | k | m | n | chirality | $R^3$ | 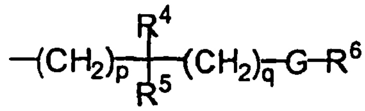 |
|---|---|---|---|---|---|---|---|
| 1779 | 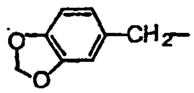 | 2 | 2 | 1 | - | H | 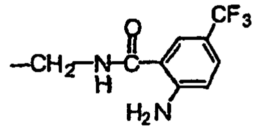 |
| 1780 | 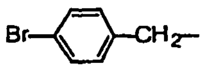 | 2 | 2 | 1 | - | H | 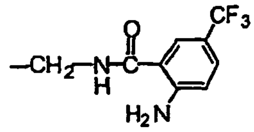 |
| 1781 | 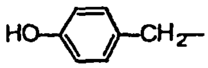 | 2 | 2 | 1 | - | H | 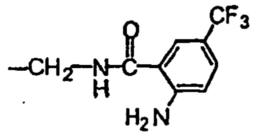 |
| 1782 | 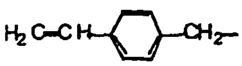 | 2 | 2 | 1 | - | H | 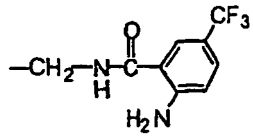 |

**Table 1.163**

| Compd. No. | 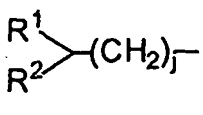 | k | m | n | chirality | $R^3$ | 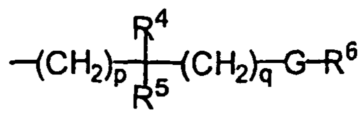 |
|---|---|---|---|---|---|---|---|
| 1783 | 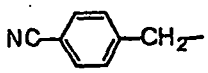 | 2 | 2 | 1 | - | H | 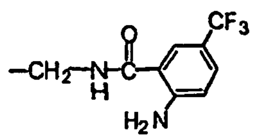 |
| 1784 | 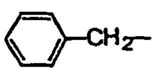 | 2 | 2 | 1 | - | H | 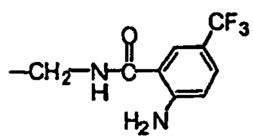 |
| 1785 | 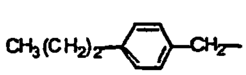 | 2 | 2 | 1 | - | H | 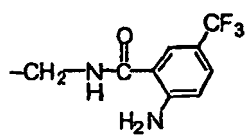 |
| 1786 | 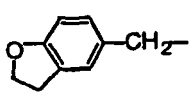 | 2 | 2 | 1 | - | H | 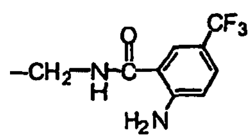 |

(continued)

| Compd. No. | 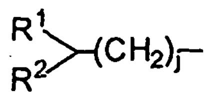 | k | m | n | chirality | $R^3$ | 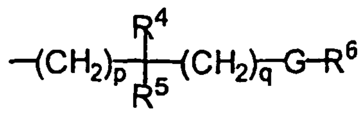 |
|---|---|---|---|---|---|---|---|
| 1787 | 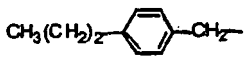 | 1 | 2 | 0 | R | H | 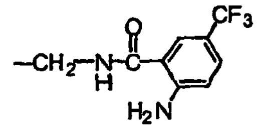 |
| 1788 | 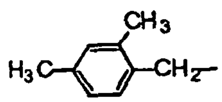 | 2 | 2 | 1 | - | H | 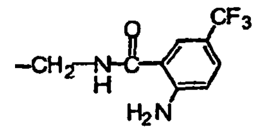 |
| 1789 | 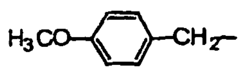 | 2 | 2 | 1 | - | H | 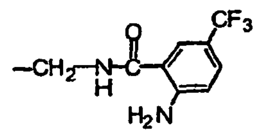 |
| 1790 | 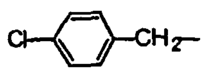 | 1 | 2 | 0 | S | H | 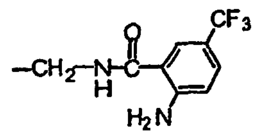 |
| 1791 | 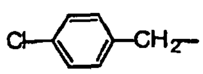 | 1 | 2 | 0 | S | H | 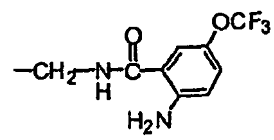 |
| 1792 | 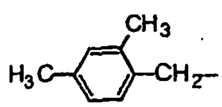 | 2 | 2 | 1 | - | H | 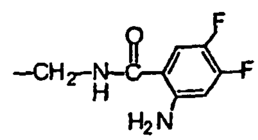 |
| 1793 | 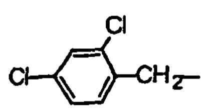 | 2 | 2 | 1 | - | H | 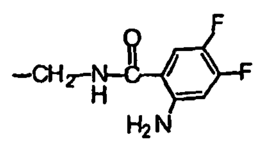 |

**Table 1.164**

| Compd. No. | 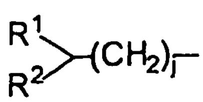 | k | m | n | chirality | $R^3$ | 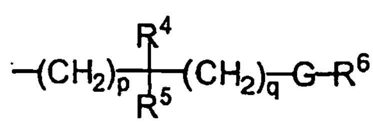 |
|---|---|---|---|---|---|---|---|
| 1794 | 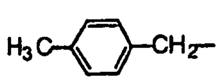 | 2 | 2 | 1 | - | H | 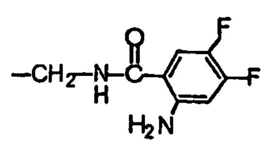 |

(continued)

| Compd. No. | 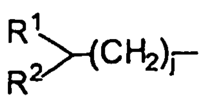 | k | m | n | chirality | $R^3$ | 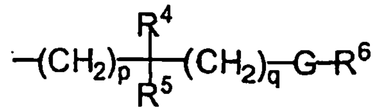 |
|---|---|---|---|---|---|---|---|
| 1795 | 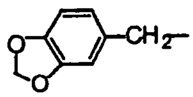 | 2 | 2 | 1 | - | H | 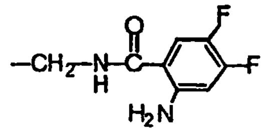 |
| 1796 | 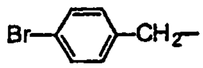 | 2 | 2 | 1 | - | H | 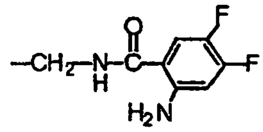 |
| 1797 | 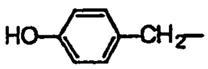 | 2 | 2 | 1 | - | H | 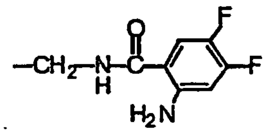 |
| 1798 | 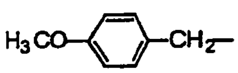 | 2 | 2 | 1 | - | H | 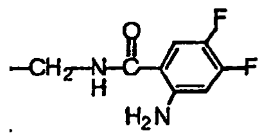 |
| 1799 | 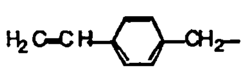 | 2 | 2 | 1 | - | H | 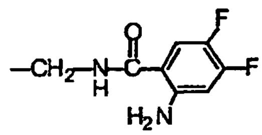 |
| 1800 | 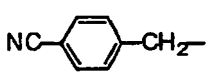 | 2 | 2 | 1 | - | H | 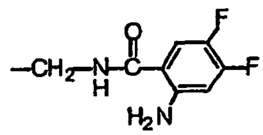 |
| 1801 | 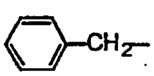 | 2 | 2 | 1 | - | H | 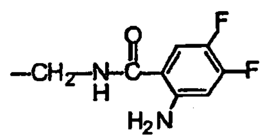 |
| 1802 | 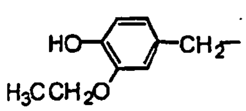 | 1 | 2 | 0 | R | H | 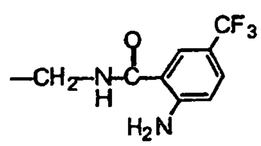 |
| 1803 | 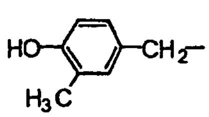 | 1 | 2 | 0 | R | H | 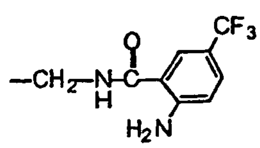 |
| 1804 | 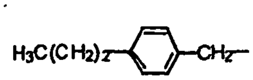 | 2 | 2 | 1 | - | H | 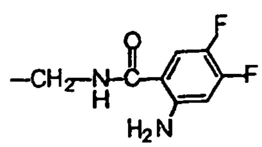 |

**Table 1.165**

| Compd. No. | 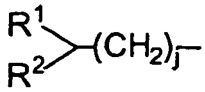 | k | m | n | chirality | $R^3$ | 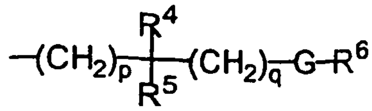 |
|---|---|---|---|---|---|---|---|
| 1805 | 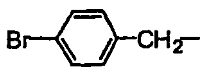 | 1 | 2 | 0 | R | H | 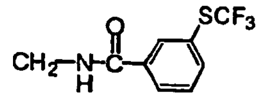 |
| 1806 | 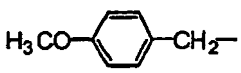 | 1 | 2 | 0 | R | H | 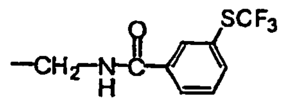 |
| 1807 | 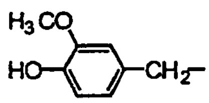 | 1 | 2 | 0 | R | H | 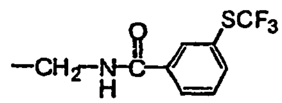 |
| 1808 | 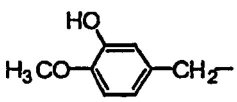 | 1 | 2 | 0 | R | H | 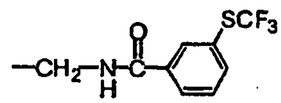 |
| 1809 | 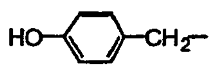 | 1 | 2 | 0 | R | H | 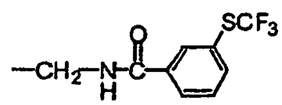 |
| 1810 | 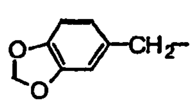 | 1 | 2 | 0 | R | H | 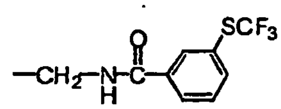 |
| 1811 | 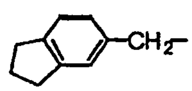 | 1 | 2 | 0 | R | H | 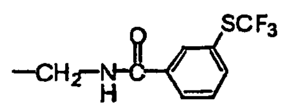 |
| 1812 | 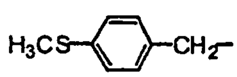 | 1 | 2 | 0 | R | H | 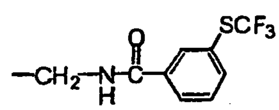 |
| 1813 | 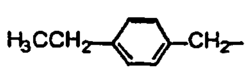 | 1 | 2 | 0 | R | H | 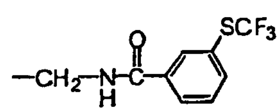 |
| 1814 | 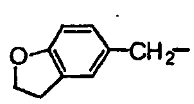 | 1 | 2 | 0 | R | H | 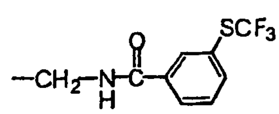 |

(continued)

| Compd. No. | 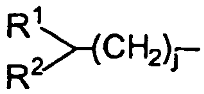 | k | m | n | chirality | $R^3$ | 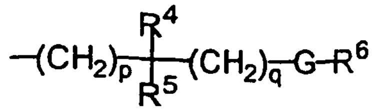 |
|---|---|---|---|---|---|---|---|
| 1815 | 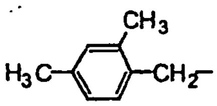 | 1 | 2 | 0 | R | H | 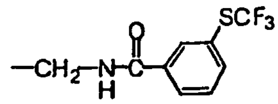 |

**Table 1.166**

| Compd. No. | 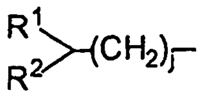 | k | m | n | chirality | $R^3$ | 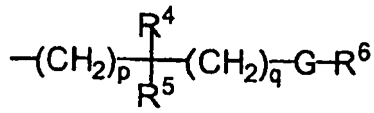 |
|---|---|---|---|---|---|---|---|
| 1816 | 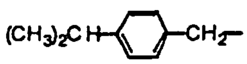 | 1 | 2 | 0 | R | H | 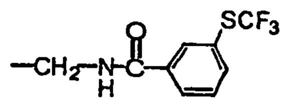 |
| 1817 | 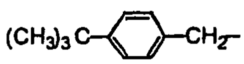 | 1 | 2 | 0 | R | H | 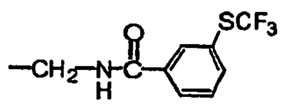 |
| 1818 | 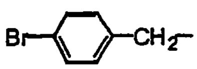 | 1 | 2 | 0 | R | H | 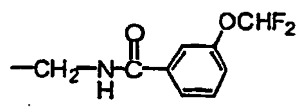 |
| 1819 | 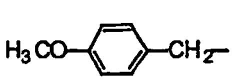 | 1 | 2 | 0 | R | H | 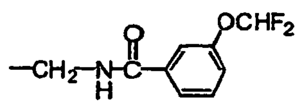 |
| 1820 | 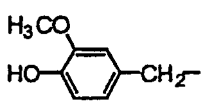 | 1 | 2 | 0 | R | H | 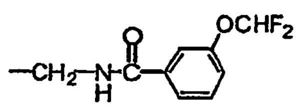 |
| 1821 | 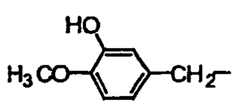 | 1 | 2 | 0 | R | H | 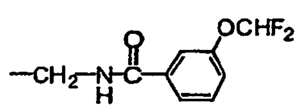 |
| 1822 | 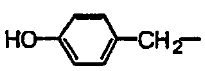 | 1 | 2 | 0 | R | H | 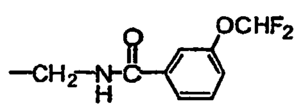 |
| 1823 | 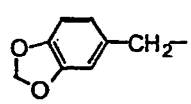 | 1 | 2 | 0 | R | H | 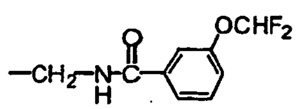 |

(continued)

| Compd. No. | 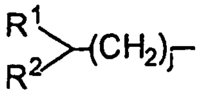 | k | m | n | chirality | $R^3$ | 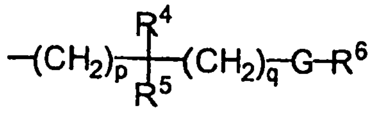 |
|---|---|---|---|---|---|---|---|
| 1824 | 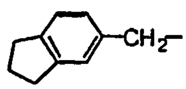 | 1 | 2 | 0 | R | H | 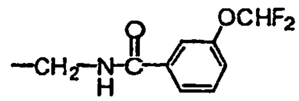 |
| 1825 | 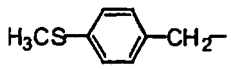 | 1 | 2 | 0 | R | H | 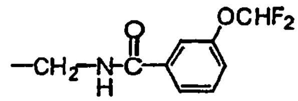 |
| 1826 | 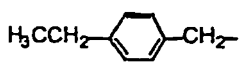 | 1 | 2 | 0 | R | H | 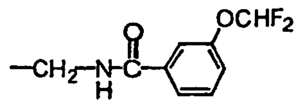 |

**Table 1.167**

| Compd. No. | 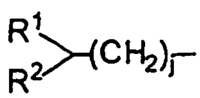 | k | m | n | chirality | $R^3$ | 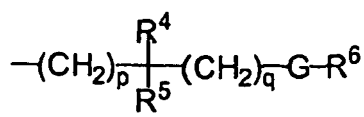 |
|---|---|---|---|---|---|---|---|
| 1827 | 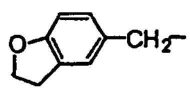 | 1 | 2 | 0 | R | H | 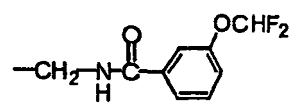 |
| 1828 | 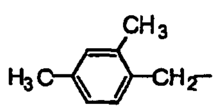 | 1 | 2 | 0 | R | H | 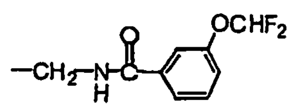 |
| 1829 | 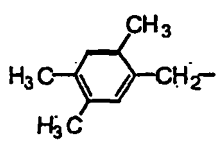 | 1 | 2 | 0 | R | H | 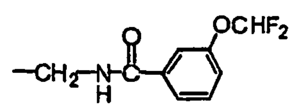 |
| 1830 | 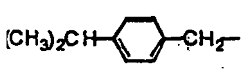 | 1 | 2 | 0 | R | H | 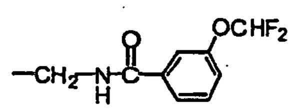 |
| 1831 | 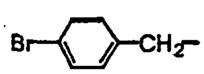 | 1 | 2 | 0 | R | H | 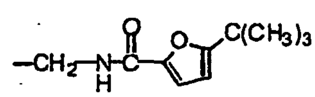 |
| 1832 | 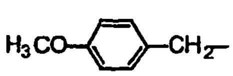 | 1 | 2 | 0 | R | H | 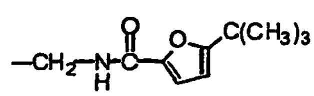 |

(continued)

| Compd. No. | 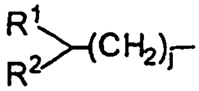 | k | m | n | chirality | $R^3$ | 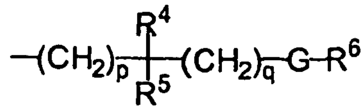 |
|---|---|---|---|---|---|---|---|
| 1833 | 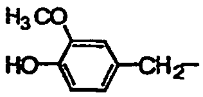 | 1 | 2 | 0 | R | H | 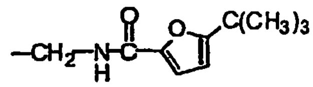 |
| 1834 | 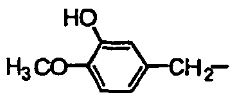 | 1 | 2 | 0 | R | H | 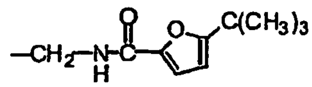 |
| 1835 | 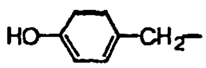 | 1 | 2 | 0 | R | H | 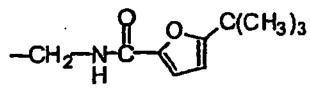 |
| 1836 | 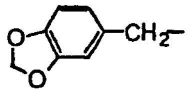 | 1 | 2 | 0 | R | H | 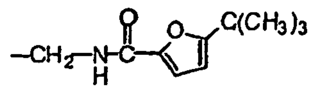 |
| 1837 | 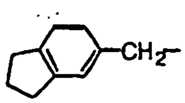 | 1 | 2 | 0 | R | H | 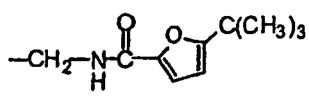 |

**Table 1.168**

| Compd. No. | 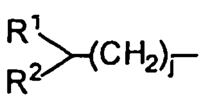 | k | m | n | chirality | $R^3$ | 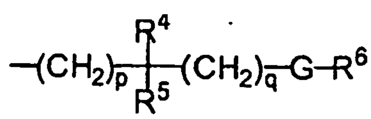 |
|---|---|---|---|---|---|---|---|
| 1838 | 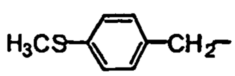 | 1 | 2 | 0 | R | H | 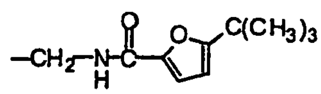 |
| 1839 | 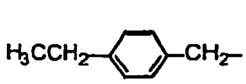 | 1 | 2 | 0 | R | H | 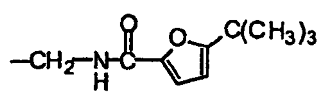 |
| 1840 | 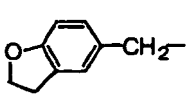 | 1 | 2 | 0 | R | H | 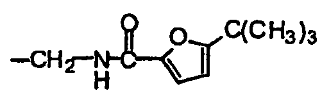 |
| 1841 | 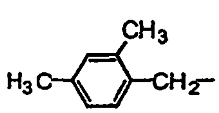 | 1 | 2 | 0 | R | H | 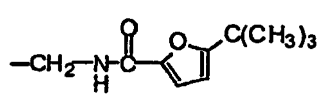 |

(continued)

| Compd. No. | 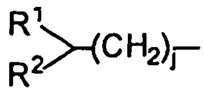 | k | m | n | chirality | $R^3$ | 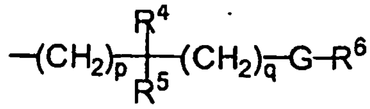 |
|---|---|---|---|---|---|---|---|
| 1842 | 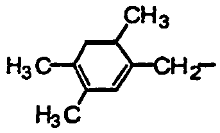 | 1 | 2 | 0 | R | H | 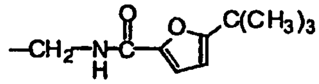 |
| 1843 | 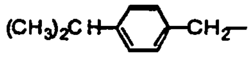 | 1 | 2 | 0 | R | H | 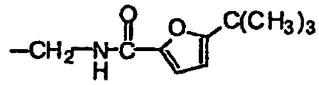 |
| 1844 | 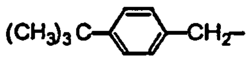 | 1 | 2 | 0 | R | H | 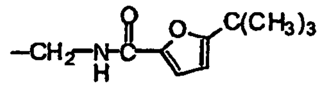 |
| 1845 | 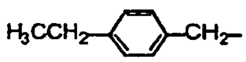 | 1 | 2 | 0 | R | H | 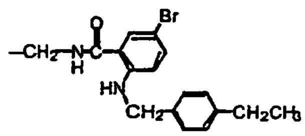 |
| 1846 | 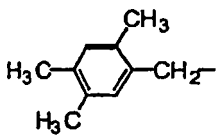 | 1 | 2 | 0 | R | H | 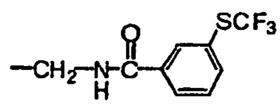 |
| 1847 | 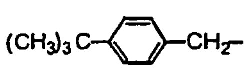 | 1 | 2 | 0 | R | H | 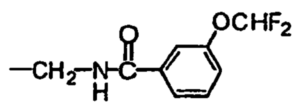 |
| 1848 | 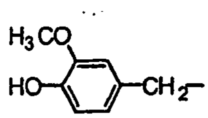 | 1 | 2 | 0 | R | H | 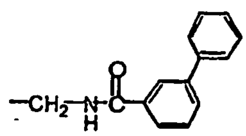 |

**Table 1.169**

| Compd. No. | 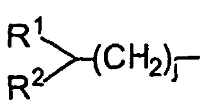 | k | m | n | chirality | $R^3$ | 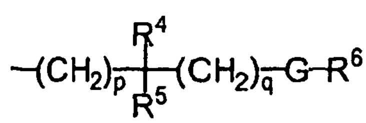 |
|---|---|---|---|---|---|---|---|
| 1849 | 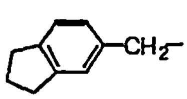 | 1 | 2 | 0 | R | H | 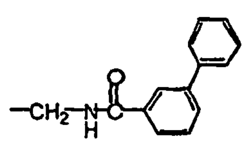 |

(continued)

| Compd. No. | 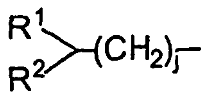 | k | m | n | chirality | $R^3$ | 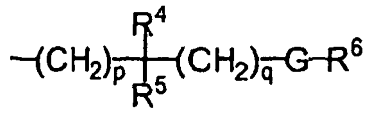 |
|---|---|---|---|---|---|---|---|
| 1850 | 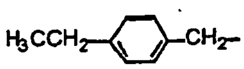 | 1 | 2 | 0 | R | H | 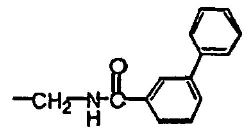 |
| 1851 | 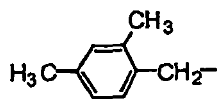 | 1 | 2 | 0 | R | H | 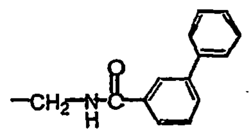 |
| 1852 | 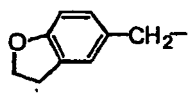 | 1 | 2 | 0 | R | H | 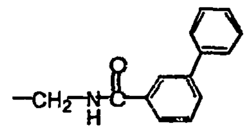 |
| 1853 | 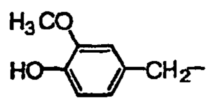 | 1 | 2 | 0 | R | H | 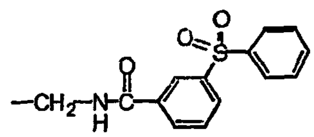 |
| 1854 | 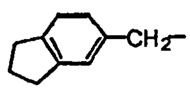 | 1 | 2 | 0 | R | H | 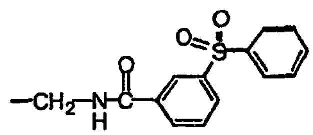 |
| 1855 | 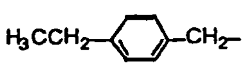 | 1 | 2 | 0 | R | H | 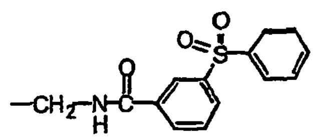 |
| 1856 | 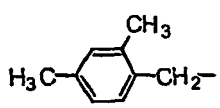 | 1 | 2 | 0 | R | H | 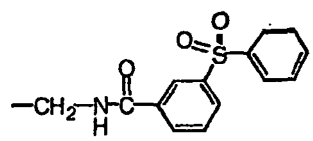 |
| 1857 | 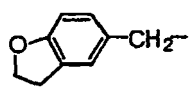 | 1 | 2 | 0 | R | H | 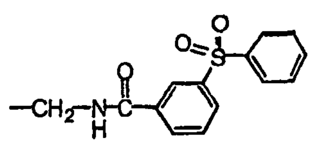 |
| 1858 | 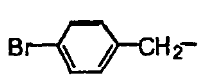 | 1 | 2 | 0 | R | H | 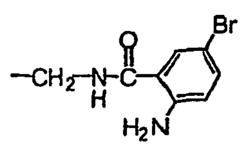 |
| 1859 | 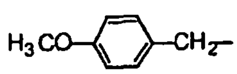 | 1 | 2 | 0 | R | H | 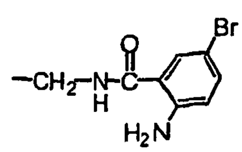 |

**Table 1.170**

| Compd. No. | 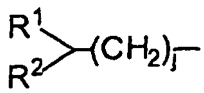 | k | m | n | chirality | $R^3$ | 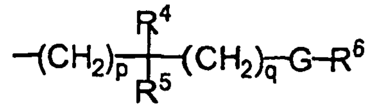 |
|---|---|---|---|---|---|---|---|
| 1860 | 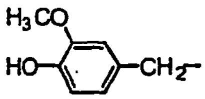 | 1 | 2 | 0 | R | H | 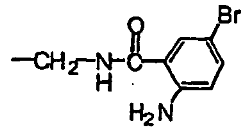 |
| 1861 | 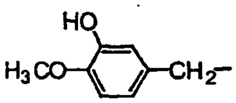 | 1 | 2 | 0 | R | H | 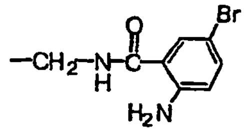 |
| 1862 | 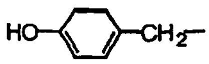 | 1 | 2 | 0 | R | H | 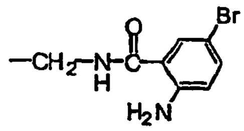 |
| 1863 | 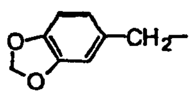 | 1 | 2 | 0 | R | H | 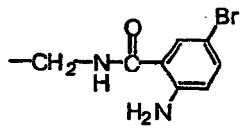 |
| 1864 | 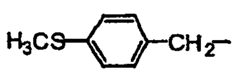 | 1 | 2 | 0 | R | H | 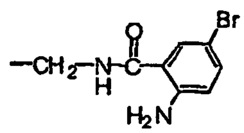 |
| 1865 | 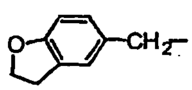 | 1 | 2 | 0 | R | H | 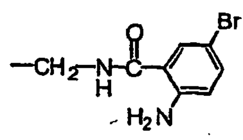 |
| 1866 | 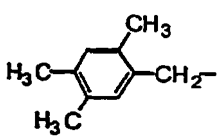 | 1 | 2 | 0 | R | H | 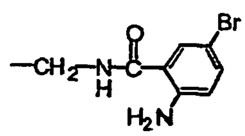 |
| 1867 | 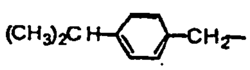 | 1 | 2 | 0 | R | H | 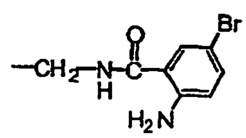 |
| 1868 | 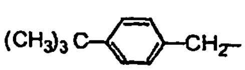 | 1 | 2 | 0 | R | H | 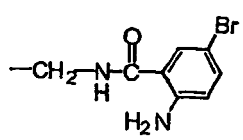 |
| 1869 | 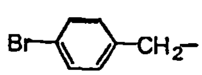 | 1 | 2 | 0 | R | H | 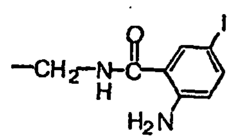 |

(continued)

| Compd. No. | 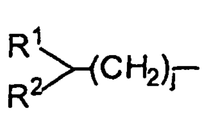 | k | m | n | chirality | $R^3$ | 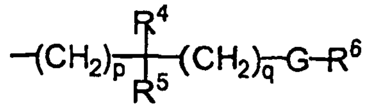 |
|---|---|---|---|---|---|---|---|
| 1870 | 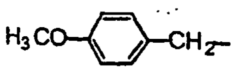 | 1 | 2 | 0 | R | H | 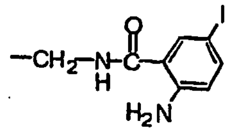 |

**Table 1.171**

| Compd. No. | 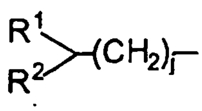 | k | m | n | chirality | $R^3$ | 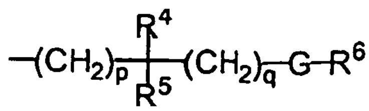 |
|---|---|---|---|---|---|---|---|
| 1871 | 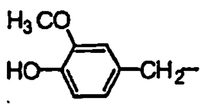 | 1 | 2 | 0 | R | H | 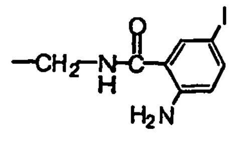 |
| 1872 | 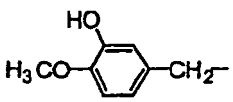 | 1 | 2 | 0 | R | H | 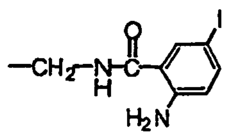 |
| 1873 | 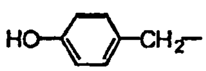 | 1 | 2 | 0 | R | H | 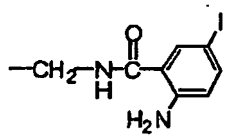 |
| 1874 | 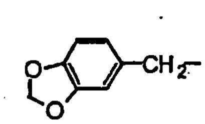 | 1 | 2 | 0 | R | H | 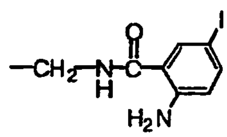 |
| 1875 | 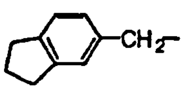 | 1 | 2 | 0 | R | H | 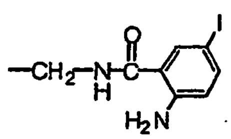 |
| 1876 | 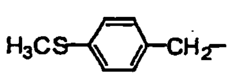 | 1 | 2 | 0 | R | H | 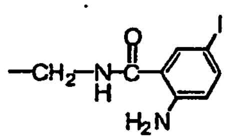 |
| 1877 | 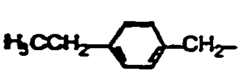 | 1 | 2 | 0 | R | H | 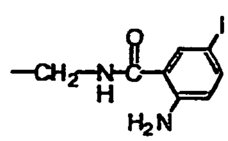 |

(continued)

| Compd. No. | 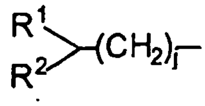 | k | m | n | chirality | $R^3$ | 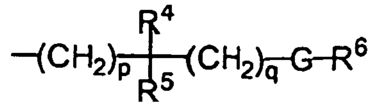 |
|---|---|---|---|---|---|---|---|
| 1878 | 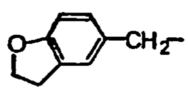 | 1 | 2 | 0 | R | H | 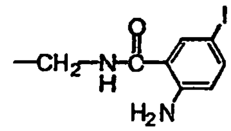 |
| 1879 | 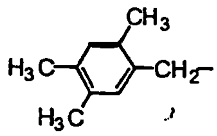 | 1 | 2 | 0 | R | H | 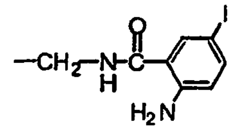 |
| 1880 | 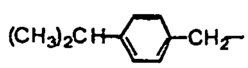 | 1 | 2 | 0 | R | H | 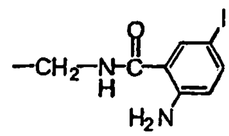 |
| 1881 | 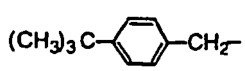 | 1 | 2 | 0 | R | H | 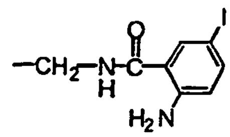 |

**Table 1.172**

| Compd. No. | 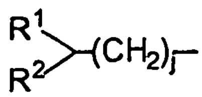 | k | m | n | chirality | $R^3$ | 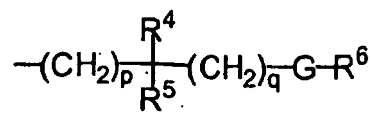 |
|---|---|---|---|---|---|---|---|
| 1882 | 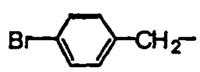 | 1 | 2 | 0 | R | H | 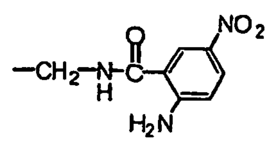 |
| 1883 | 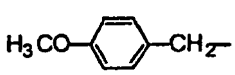 | 1 | 2 | 0 | R | H | 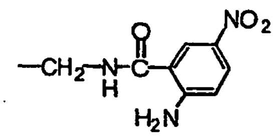 |
| 1884 | 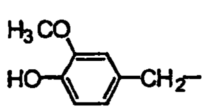 | 1 | 2 | 0 | R | H | 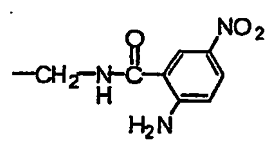 |
| 1885 | 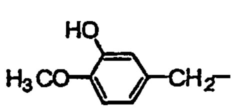 | 1 | 2 | 0 | R | H | 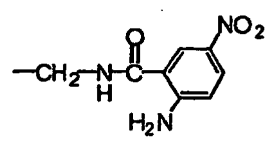 |

(continued)

| Compd. No. | 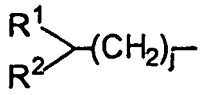 | k | m | n | chirality | $R^3$ | 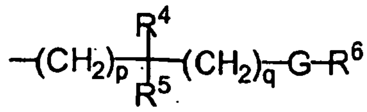 |
|---|---|---|---|---|---|---|---|
| 1886 | 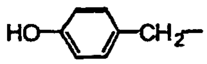 | 1 | 2 | 0 | R | H | 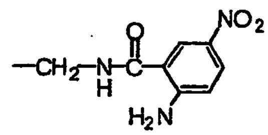 |
| 1887 | 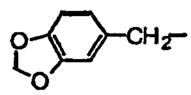 | 1 | 2 | 0 | R | H | 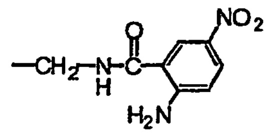 |
| 1888 | 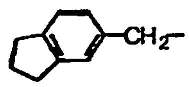 | 1 | 2 | 0 | R | H | 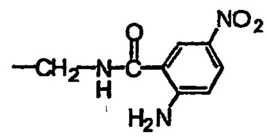 |
| 1889 | 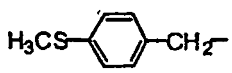 | 1 | 2 | 0 | R | H | 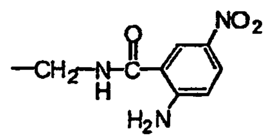 |
| 1890 | 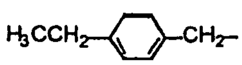 | 1 | 2 | 0 | R | H | 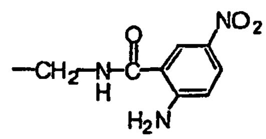 |
| 1891 | 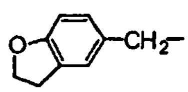 | 1 | 2 | 0 | R | H | 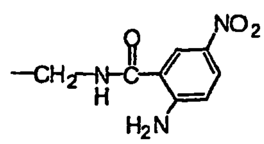 |
| 1892 | 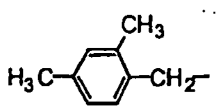 | 1 | 2 | 0 | R | H | 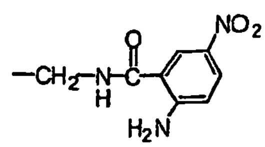 |

**Table 1.173**

| Compd. No. | 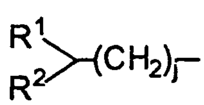 | k | m | n | chirality | $R^3$ | 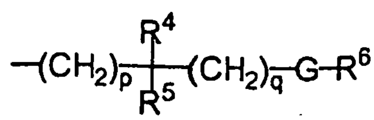 |
|---|---|---|---|---|---|---|---|
| 1893 | 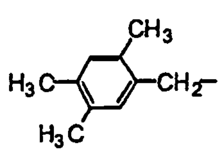 | 1 | 2 | 0 | R | H | 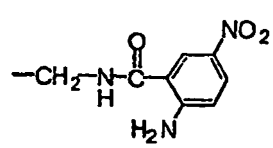 |

(continued)

| Compd. No. | 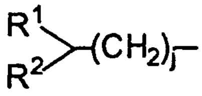 | k | m | n | chirality | $R^3$ | 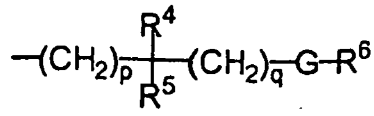 |
|---|---|---|---|---|---|---|---|
| 1894 | 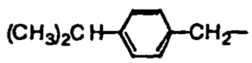 | 1 | 2 | 0 | R | H | 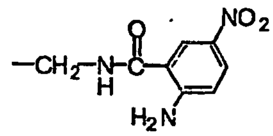 |
| 1895 | 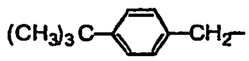 | 1 | 2 | 0 | R | H | 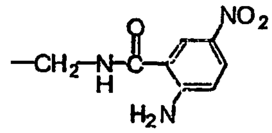 |
| 1896 | 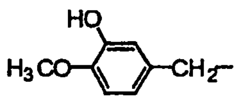 | 1 | 2 | 0 | R | H | 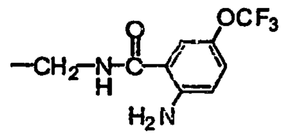 |
| 1897 | 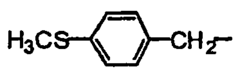 | 1 | 2 | 0 | R | H | 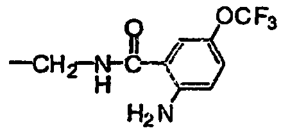 |
| 1898 | 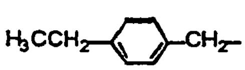 | 1 | 2 | 0 | R | H | 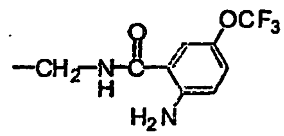 |
| 1899 | 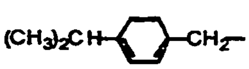 | 1 | 2 | 0 | R | H | 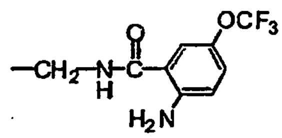 |
| 1900 | 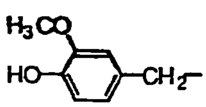 | 1 | 2 | 0 | R | H | 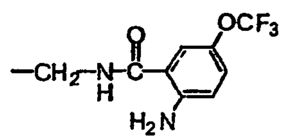 |
| 1901 | 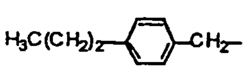 | 1 | 2 | 0 | R | H | 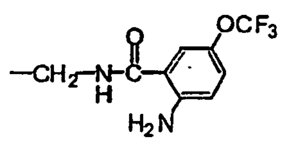 |
| 1902 | 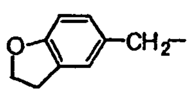 | 1 | 2 | 0 | R | H | 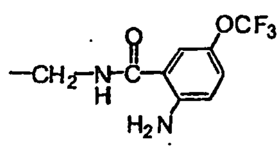 |
| 1903 | 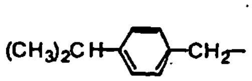 | 2 | 2 | 1 | - | H | 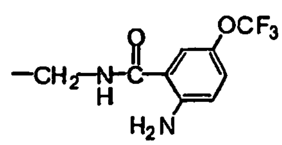 |

**Table 1.174**

| Compd. No. | 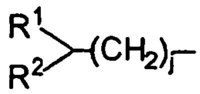 | k | m | n | chirality | $R^3$ | 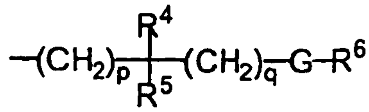 |
|---|---|---|---|---|---|---|---|
| 1904 | 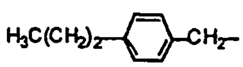 | 2 | 2 | 1 | - | H | 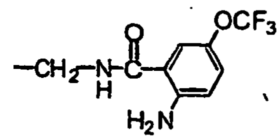 |
| 1905 | 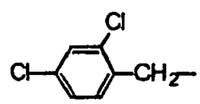 | 1 | 2 | 0 | R | H | 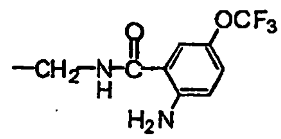 |
| 1906 | 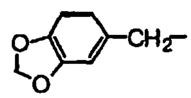 | 1 | 2 | 0 | R | H | 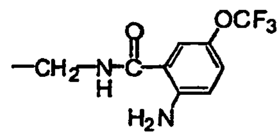 |
| 1907 | 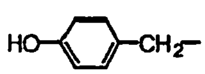 | 1 | 2 | 0 | R | H | 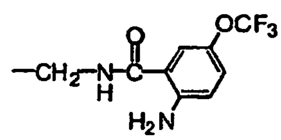 |
| 1908 | 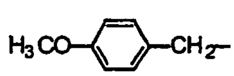 | 1 | 2 | 0 | R | H | 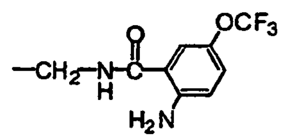 |
| 1909 | 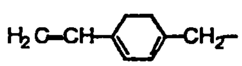 | 1 | 2 | 0 | R | H | 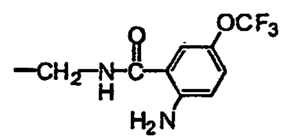 |
| 1910 | 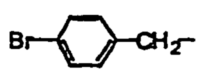 | 2 | 2 | 1 | - | H | 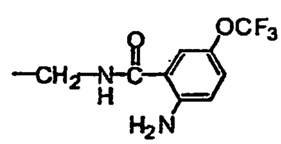 |
| 1911 | 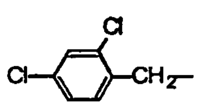 | 2 | 2 | 1 | - | H | 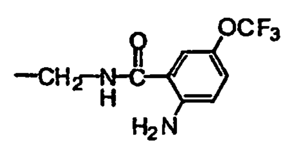 |
| 1912 | 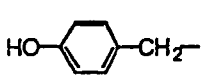 | 2 | 2 | 1 | - | H | 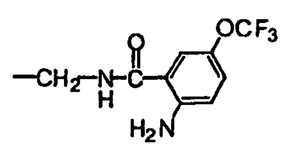 |

(continued)

| Compd. No. | 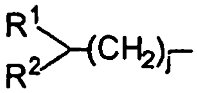 | k | m | n | chirality | $R^3$ | 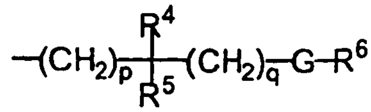 |
|---|---|---|---|---|---|---|---|
| 1913 | 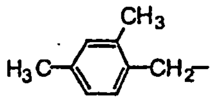 | 2 | 2 | 1 | - | H | 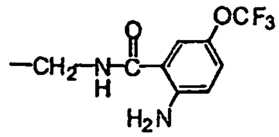 |
| 1914 | 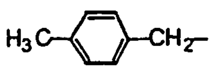 | 2 | 2 | 1 | - | H | 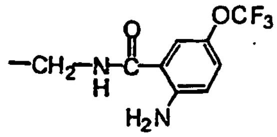 |

**Table 1.175**

| Compd. No. | 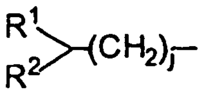 | k | m | n | chirality | $R^3$ | 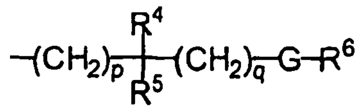 |
|---|---|---|---|---|---|---|---|
| 1915 | 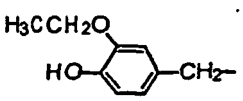 | 1 | 2 | 0 | R | H | 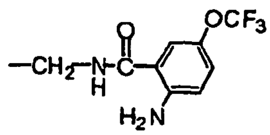 |
| 1916 | 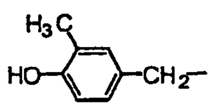 | 1 | 2 | 0 | R | H | 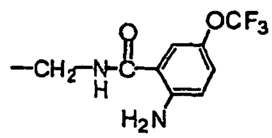 |
| 1917 | 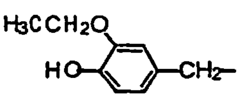 | 2 | 2 | 1 | - | H | 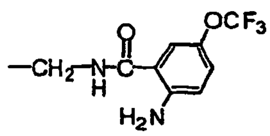 |
| 1918 | 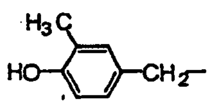 | 2 | 2 | 1 | - | H | 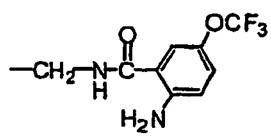 |
| 1919 | 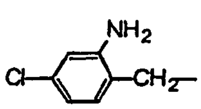 | 2 | 2 | 1 | - | H | 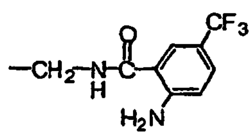 |
| 1920 | 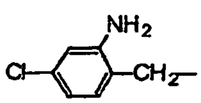 | 2 | 2 | 1 | - | H | 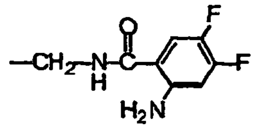 |

(continued)

| Compd. No. | 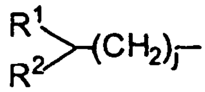 | k | m | n | chirality | $R^3$ | 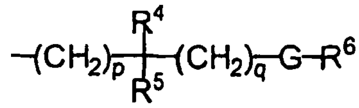 |
|---|---|---|---|---|---|---|---|
| 1921 | 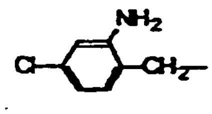 | 1 | 2 | 0 | R | H | 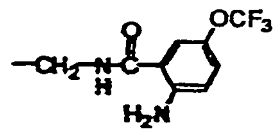 |
| 1922 | 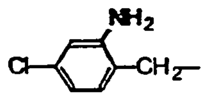 | 2 | 2 | 1 | - | H | 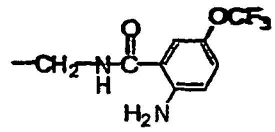 |
| 1923 | 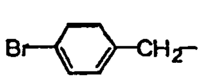 | 2 | 2 | 1 | - | H | 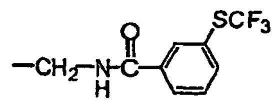 |
| 1924 | 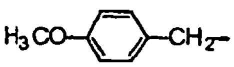 | 2 | 2 | 1 | - | H | 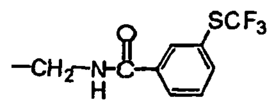 |
| 1925 | 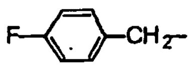 | 2 | 2 | 1 | - | H | 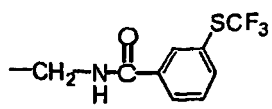 |

**Table 1.176**

| Compd. No. | 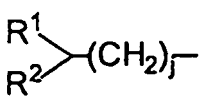 | k | m | n | chirality | $R^3$ | 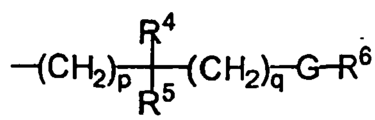 |
|---|---|---|---|---|---|---|---|
| 1926 | 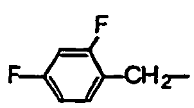 | 2 | 2 | 1 | - | H | 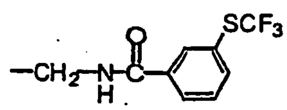 |
| 1927 | 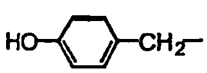 | 2 | 2 | 1 | - | H | 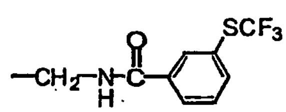 |
| 1928 | 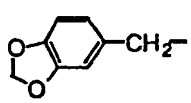 | 2 | 2 | 1 | - | H | 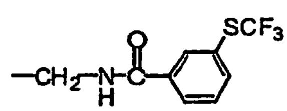 |
| 1929 | 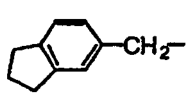 | 2 | 2 | 1 | - | H | 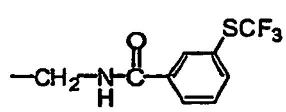 |

(continued)

| Compd. No. | 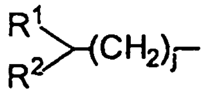 | k | m | n | chirality | $R^3$ | 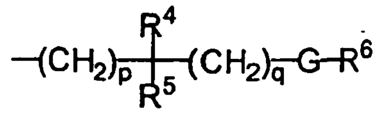 |
|---|---|---|---|---|---|---|---|
| 1930 | 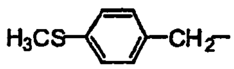 | 2 | 2 | 1 | - | H | 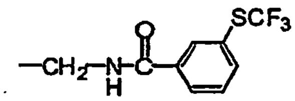 |
| 1931 | 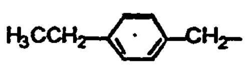 | 2 | 2 | 1 | - | H | 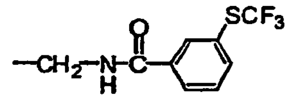 |
| 1932 | 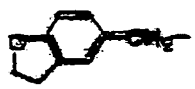 | 2 | 2 | 1 | - | H | 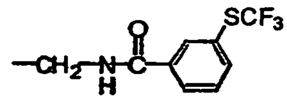 |
| 1933 | 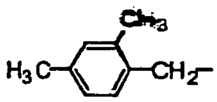 | 2 | 2 | 1 | - | H | 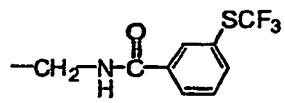 |
| 1934 | 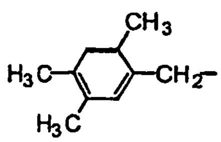 | 2 | 2 | 1 | - | H | 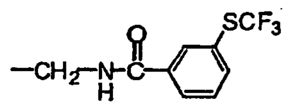 |
| 1935 | 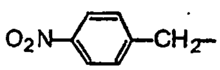 | 2 | 2 | 1 | - | H | 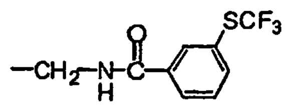 |
| 1936 | 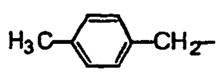 | 2 | 2 | 1 | - | H | 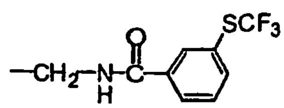 |

**Table 1.177**

| Compd. No. | 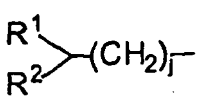 | k | m | n | chirality | $R^3$ | 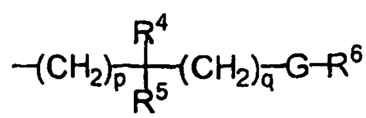 |
|---|---|---|---|---|---|---|---|
| 1937 | 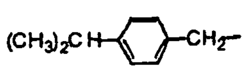 | 2 | 2 | 1 | - | H | 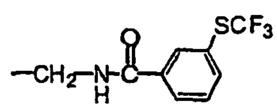 |
| 1938 | 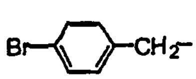 | 2 | 2 | 1 | - | H | 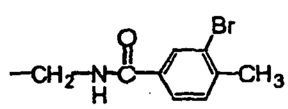 |

(continued)

| Compd. No. | 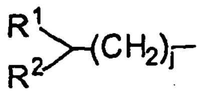 | k | m | n | chirality | $R^3$ | 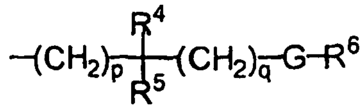 |
|---|---|---|---|---|---|---|---|
| 1939 | 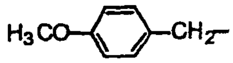 | 2 | 2 | 1 | - | H | 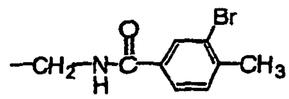 |
| 1940 | 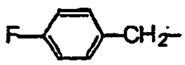 | 2 | 2 | 1 | - | H | 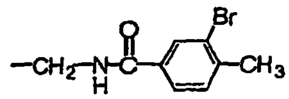 |
| 1941 | 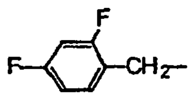 | 2 | 2 | 1 | - | H | 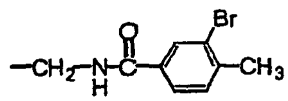 |
| 1942 | 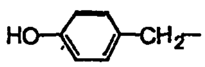 | 2 | 2 | 1 | - | H | 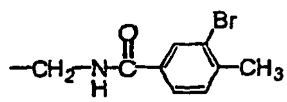 |
| 1943 | 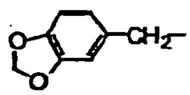 | 2 | 2 | 1 | - | H | 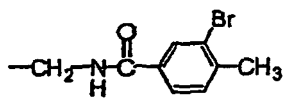 |
| 1944 | 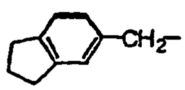 | 2 | 2 | 1 | - | H | 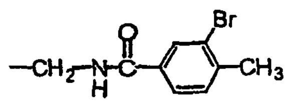 |
| 1945 | 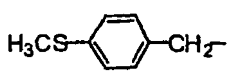 | 2 | 2 | 1 | - | H | 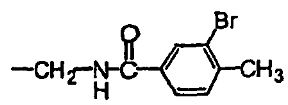 |
| 1946 | 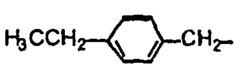 | 2 | 2 | 1 | - | H | 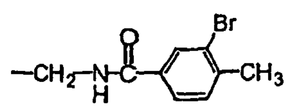 |
| 1947 | 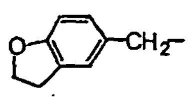 | 2 | 2 | 1 | - | H | 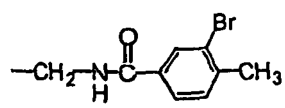 |

Table 1.178

| Compd. No. | 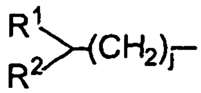 | k | m | n | chirality | $R^3$ | 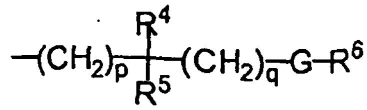 |
|---|---|---|---|---|---|---|---|
| 1948 | 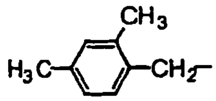 | 2 | 2 | 1 | - | H | 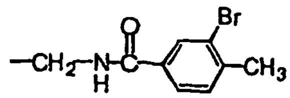 |
| 1949 | 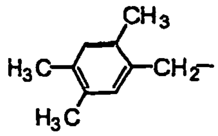 | 2 | 2 | 1 | - | H | 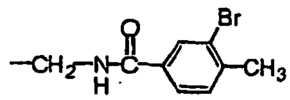 |
| 1950 | 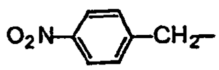 | 2 | 2 | 1 | - | H | 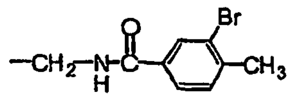 |
| 1951 | 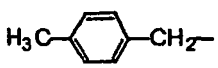 | 2 | 2 | 1 | - | H | 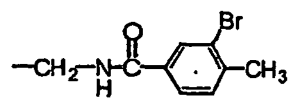 |
| 1952 | 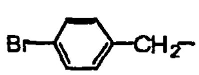 | 2 | 2 | 1 | - | H | 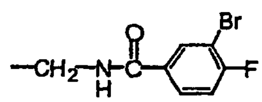 |
| 1953 | 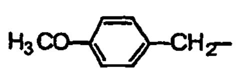 | 2 | 2 | 1 | - | H | 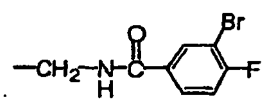 |
| 1954 | 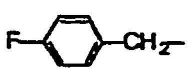 | 2 | 2 | 1 | - | H | 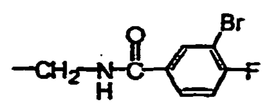 |
| 1955 | 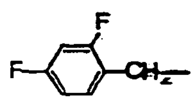 | 2 | 2 | 1 | - | H | 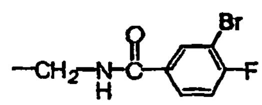 |
| 1956 | 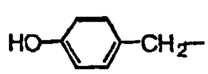 | 2 | 2 | 1 | - | H | 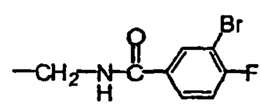 |
| 1957 | 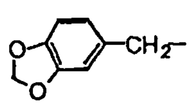 | 2 | 2 | 1 | - | H | 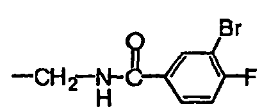 |
| 1958 | 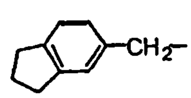 | 2 | 2 | 1 | - | H | 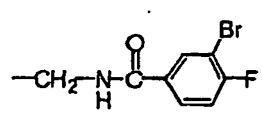 |

**Table 1.179**

| Compd. No. | 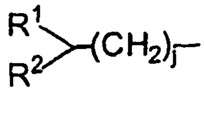 | k | m | n | chirality | $R^3$ | 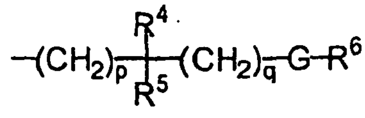 |
|---|---|---|---|---|---|---|---|
| 1959 | 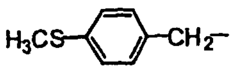 | 2 | 2 | 1 | - | H | 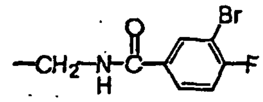 |
| 1960 | 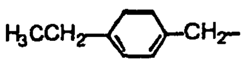 | 2 | 2 | 1 | - | H | 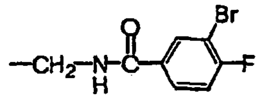 |
| 1961 | 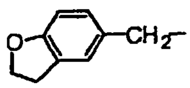 | 2 | 2 | 1 | - | H | 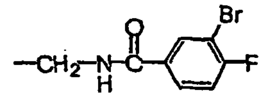 |
| 1962 | 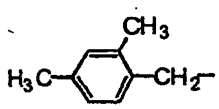 | 2 | 2 | 1 | - | H | 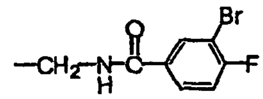 |
| 1963 | 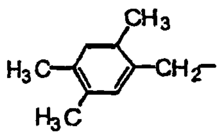 | 2 | 2 | 1 | - | H | 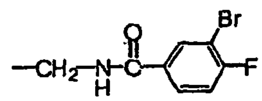 |
| 1964 | 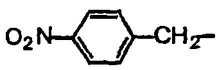 | 2 | 2 | 1 | - | H | 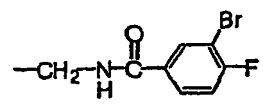 |
| 1965 | 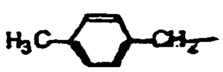 | 2 | 2 | 1 | - | H | 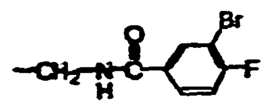 |
| 1966 | 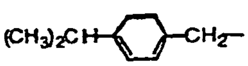 | 2 | 2 | 1 | - | H | 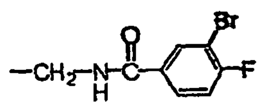 |
| 1967 | 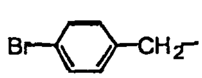 | 2 | 2 | 1 | - | H | 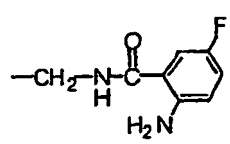 |
| 1968 | 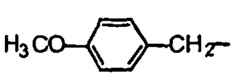 | 2 | 2 | 1 | - | H | 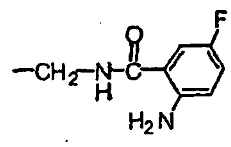 |

(continued)

| Compd. No. | 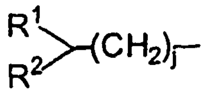 | k | m | n | chirality | $R^3$ | 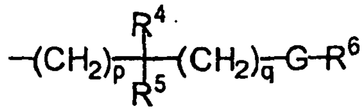 |
|---|---|---|---|---|---|---|---|
| 1969 | 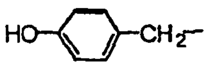 | 2 | 2 | 1 | - | H | 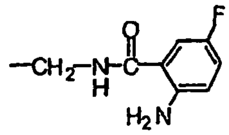 |

**Table 1.180**

| Compd. No. | 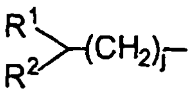 | k | m | n | chirality | $R^3$ | 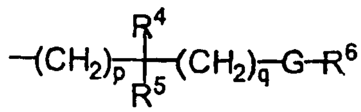 |
|---|---|---|---|---|---|---|---|
| 1970 | 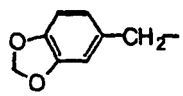 | 2 | 2 | 1 | - | H | 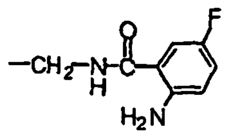 |
| 1971 | 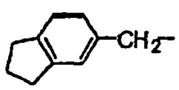 | 2 | 2 | 1 | - | H | 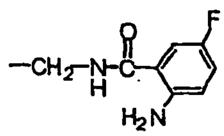 |
| 1972 | 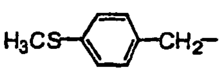 | 2 | 2 | 1 | - | H | 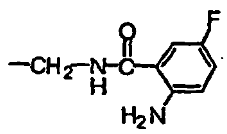 |
| 1973 | 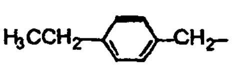 | 2 | 2 | 1 | - | H | 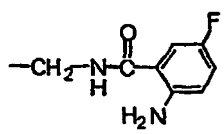 |
| 1974 | 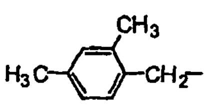 | 2 | 2 | 1 | - | H | 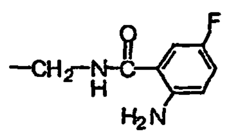 |
| 1975 | 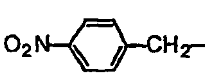 | 2 | 2 | 1 | - | H | 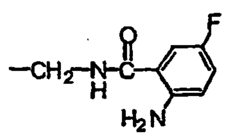 |
| 1976 | 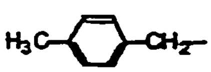 | 2 | 2 | 1 | - | H | 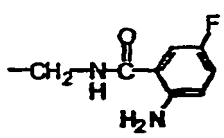 |

(continued)

| Compc No. | 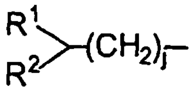 | k | m | n | chirality | $R^3$ | 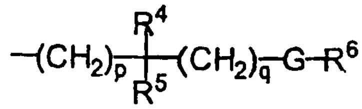 |
|---|---|---|---|---|---|---|---|
| 1977 | 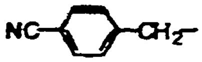 | 2 | 2 | 1 | - | H | 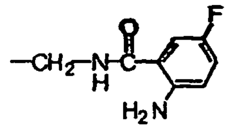 |
| 1978 | 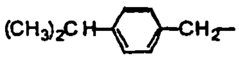 | 2 | 2 | 1 | - | H | 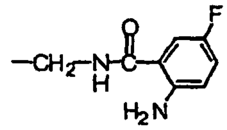 |
| 1979 | 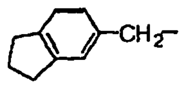 | 2 | 2 | 1 | - | H | 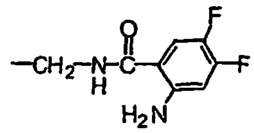 |
| 1980 | 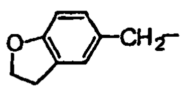 | 2 | 2 | 1 | - | H | 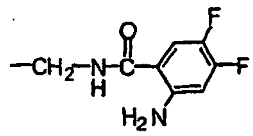 |

**Table 1.181**

| Compd. No. | 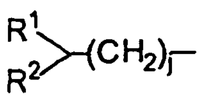 | k | m | n | chirality | $R^3$ | 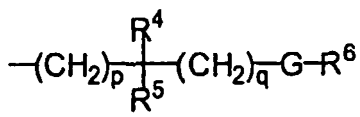 |
|---|---|---|---|---|---|---|---|
| 1981 | 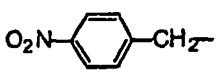 | 2 | 2 | 1 | - | H | 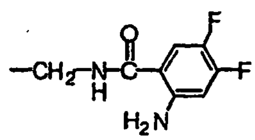 |
| 1982 | 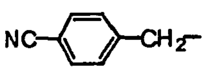 | 2 | 2 | 1 | - | H | 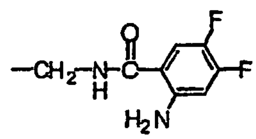 |
| 1983 | 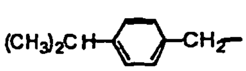 | 2 | 2 | 1 | - | H | 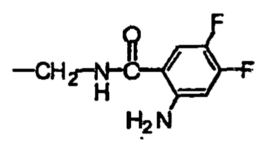 |
| 1984 | 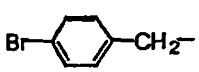 | 2 | 2 | 1 | - | H | 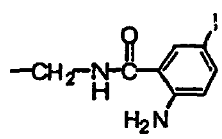 |

(continued)

| Compd. No. | 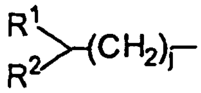 | k | m | n | chirality | $R^3$ | 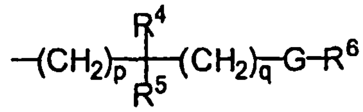 |
|---|---|---|---|---|---|---|---|
| 1985 | 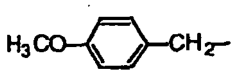 | 2 | 2 | 1 | - | H | 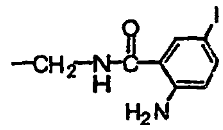 |
| 1986 | 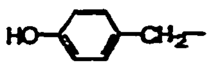 | 2 | 2 | 1 | - | H | 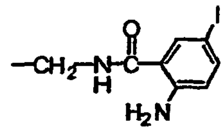 |
| 1987 | 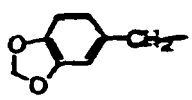 | 2 | 2 | 1 | - | H | 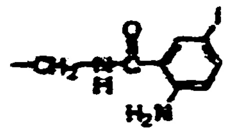 |
| 1988 | 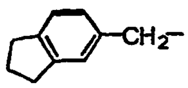 | 2 | 2 | 1 | - | H | 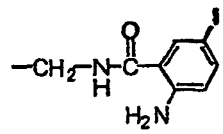 |
| 1989 | 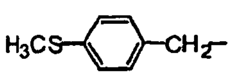 | 2 | 2 | 1 | - | H | 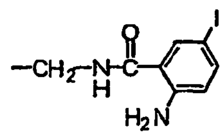 |
| 1990 | 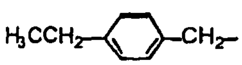 | 2 | 2 | 1 | - | H | 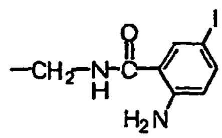 |
| 1991 | 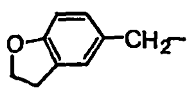 | 2 | 2 | 1 | - | H | 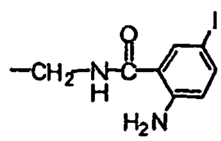 |

**Table 1.182**

| Compd No. | 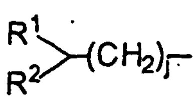 | k | m | n | chirality | $R^3$ | 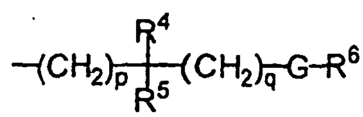 |
|---|---|---|---|---|---|---|---|
| 1992 | 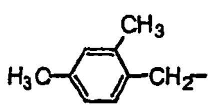 | 2 | 2 | 1 | - | H | 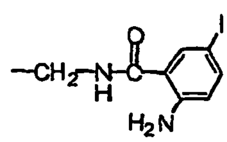 |

(continued)

| Compd No. | 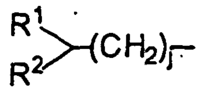 | k | m | n | chirality | $R^3$ | 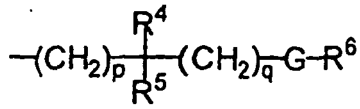 |
|---|---|---|---|---|---|---|---|
| 1993 | 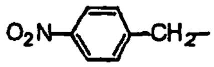 | 2 | 2 | 1 | - | H | 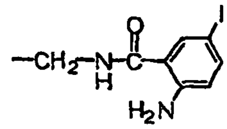 |
| 1994 | 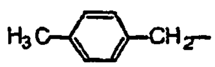 | 2 | 2 | 1 | - | H | 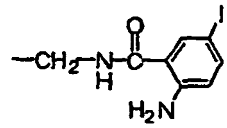 |
| 1995 | 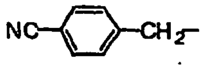 | 2 | 2 | 1 | - | H | 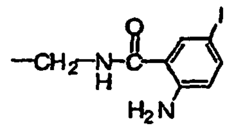 |
| 1996 | 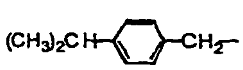 | 2 | 2 | 1 | - | H | 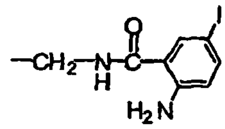 |
| 1997 | 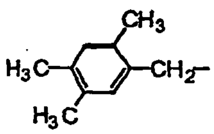 | 2 | 2 | 1 | - | H | 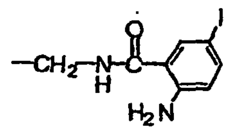 |
| 1998 | 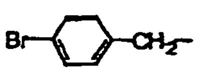 | 2 | 2 | 1 | - | H | 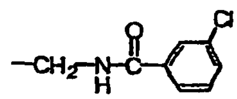 |
| 1999 | 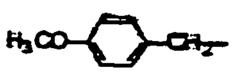 | 2 | 2 | 1 | - | H | 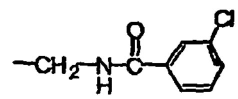 |
| 2000 | 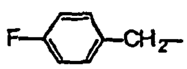 | 2 | 2 | 1 | - | H | 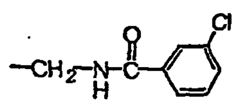 |
| 2001 | 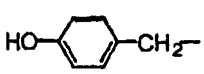 | 2 | 2 | 1 | - | H | 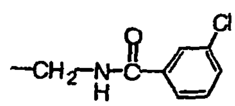 |
| 2002 | 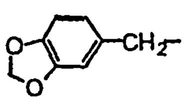 | 2 | 2 | 1 | - | H | 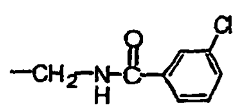 |

**Table 1.183**

| Compd. No. | 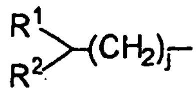 | k | m | n | chirality | R$^3$ | 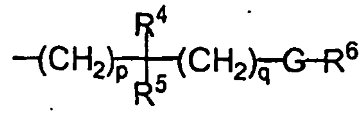 |
|---|---|---|---|---|---|---|---|
| 2003 | 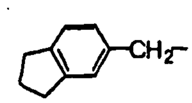 | 2 | 2 | 1 | - | H | 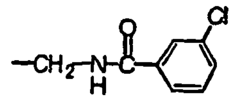 |
| 2004 | 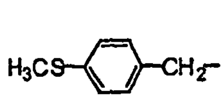 | 2 | 2 | 1 | - | H | 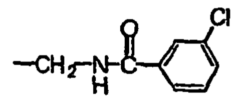 |
| 2005 | 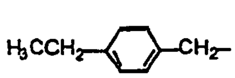 | 2 | 2 | 1 | - | H | 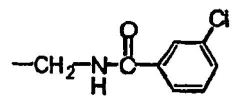 |
| 2006 | 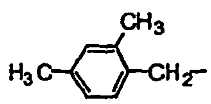 | 2 | 2 | 1 | - | H | 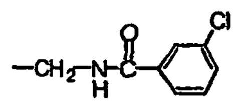 |
| 2007 | 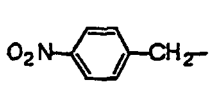 | 2 | 2 | 1 | - | H | 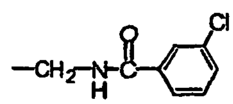 |
| 2008 | 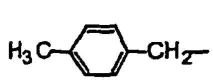 | 2 | 2 | 1 | - | H | 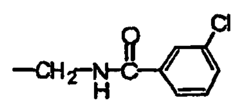 |
| 2009 | 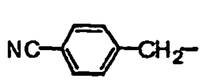 | 2 | 2 | 1 | - | H | 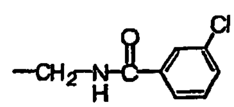 |
| 2010 | 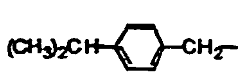 | 2 | 2 | 1 | - | H | 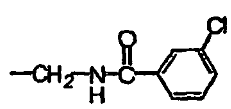 |
| 2011 | 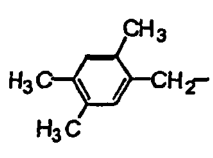 | 2 | 2 | 1 | - | H | 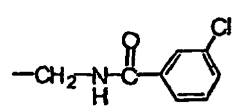 |
| 2012 | 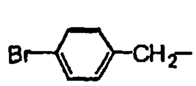 | 2 | 2 | 1 | - | H | 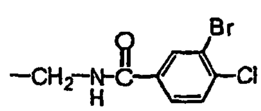 |

(continued)

| Compd. No. | 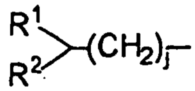 | k | m | n | chirality | $R^3$ | 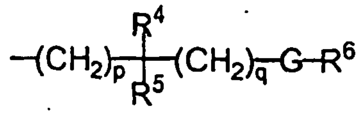 |
|---|---|---|---|---|---|---|---|
| 2013 | 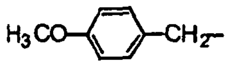 | 2 | 2 | 1 | - | H | 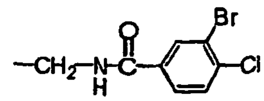 |

**Table 1.184**

| Compd. No. | 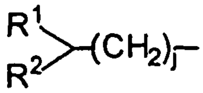 | k | m | n | chirality | $R^3$ | 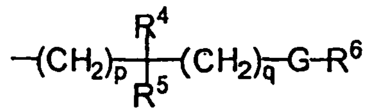 |
|---|---|---|---|---|---|---|---|
| 2014 | 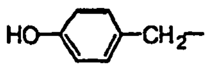 | 2 | 2 | 1 | - | H | 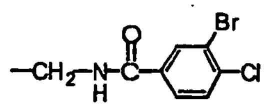 |
| 2015 | 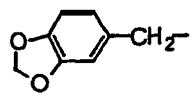 | 2 | 2 | 1 | - | H | 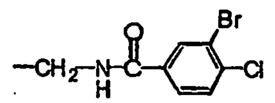 |
| 2016 | 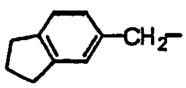 | 2 | 2 | 1 | - | H | 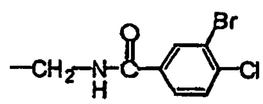 |
| 2017 | 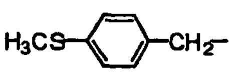 | 2 | 2 | 1 | - | H | 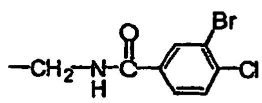 |
| 2018 | 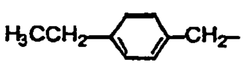 | 2 | 2 | 1 | - | H | 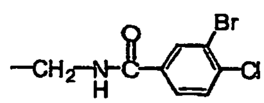 |
| 2019 | 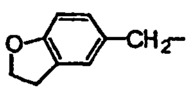 | 2 | 2 | 1 | - | H | 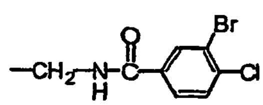 |
| 2020 | 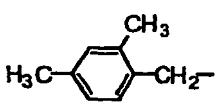 | 2 | 2 | 1 | - | H | 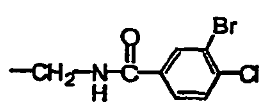 |
| 2021 | 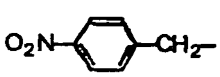 | 2 | 2 | 1 | - | H | 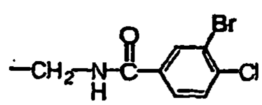 |

(continued)

| Compd. No. | 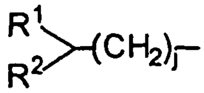 | k | m | n | chirality | $R^3$ | 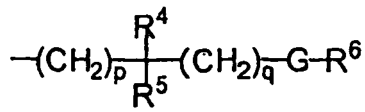 |
|---|---|---|---|---|---|---|---|
| 2022 | 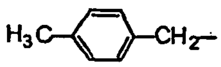 | 2 | 2 | 1 | - | H | 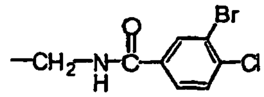 |
| 2023 | 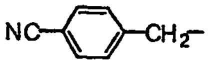 | 2 | 2 | 1 | - | H | 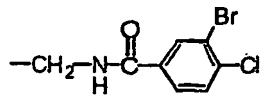 |
| 2024 | 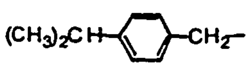 | 2 | 2 | 1 | - | H | 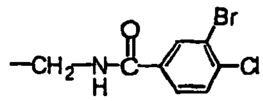 |

**Table 1.185**

| Compd. No. | 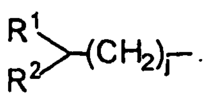 | k | m | n | chirality | $R^3$ | 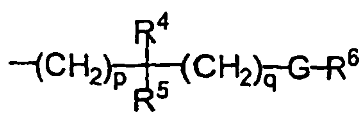 |
|---|---|---|---|---|---|---|---|
| 2025 | 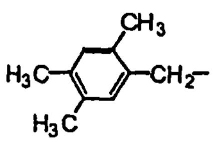 | 2 | 2 | 1 | - | H | 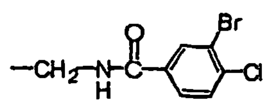 |
| 2026 | 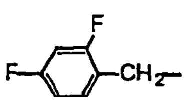 | 2 | 2 | 1 | - | H | 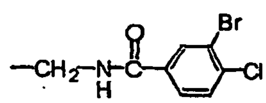 |
| 2027 | 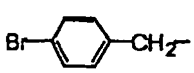 | 2 | 2 | 1 | - | H | 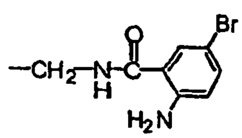 |
| 2028 | 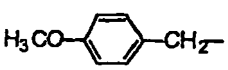 | 2 | 2 | 1 | - | H | 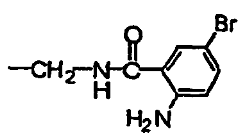 |
| 2029 | 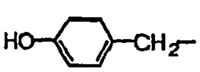 | 2 | ,2 | 1 | - | H | 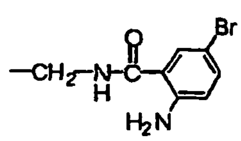 |
| 2030 | 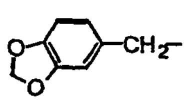 | 2 | 2 | 1 | - | H | 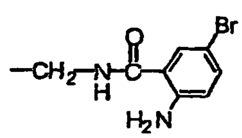 |

(continued)

| Compd. No. | 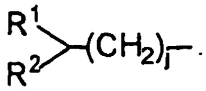 | k | m | n | chirality | $R^3$ | 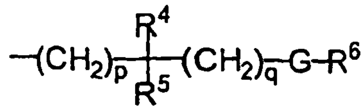 |
|---|---|---|---|---|---|---|---|
| 2031 | 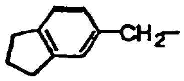 | 2 | 2 | 1 | - | H | 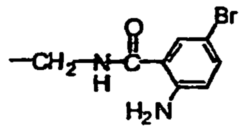 |
| 2032 | 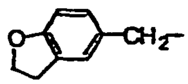 | 2 | 2 | 1 | - | H | 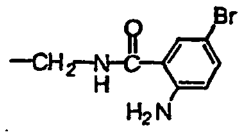 |
| 2033 | 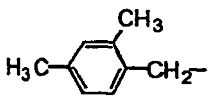 | 2 | 2 | 1 | - | H | 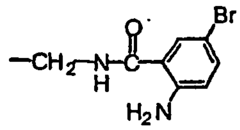 |
| 2034 | 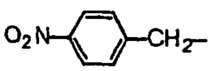 | 2 | 2 | 1 | - | H | 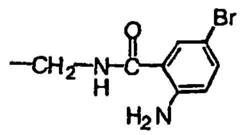 |
| 2035 | 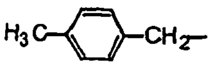 | 2 | 2 | 1 | - | H | 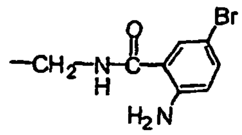 |

Table 1.186

| Compd. No. | 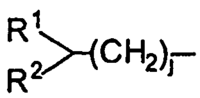 | k | m | n | chirality | $R^3$ | 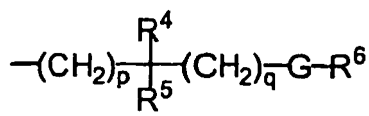 |
|---|---|---|---|---|---|---|---|
| 2036 | 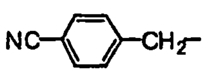 | 2 | 2 | 1 | - | H | 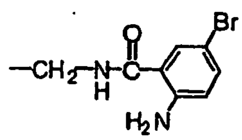 |
| 2037 | 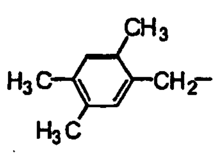 | 2 | 2 | 1 | - | H | 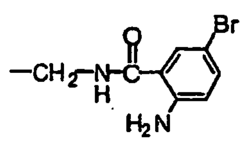 |
| 2038 | 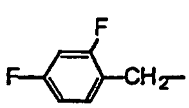 | 2 | 2 | 1 | - | H | 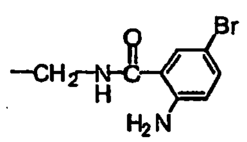 |

(continued)

| Compd. No. | 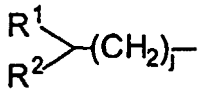 | k | m | n | chirality | $R^3$ | 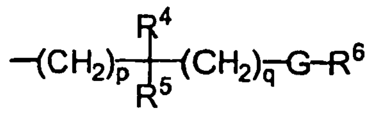 |
|---|---|---|---|---|---|---|---|
| 2039 | 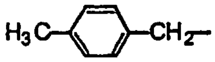 | 2 | 2 | 1 | - | H | 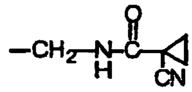 |
| 2040 | 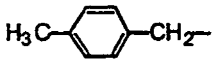 | 1 | 2 | 0 | R | H | 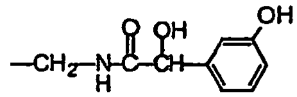 |
| 2041 | 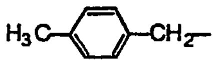 | 1 | 2 | 0 | R | H | 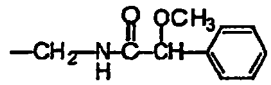 |
| 2042 | 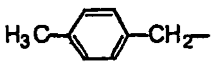 | 1 | 2 | 0 | R | H | 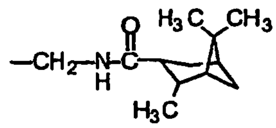 |
| 2043 | 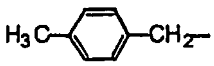 | 1 | 2 | 0 | R | H | 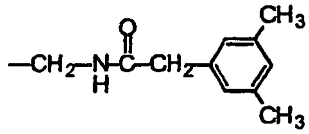 |
| 2044 | 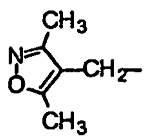 | 1 | 2 | 0 | R | H. | 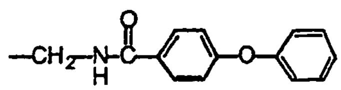 |
| 2045 | 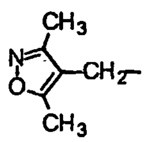 | 1 | 2 | 0 | R | H | 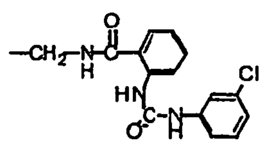 |
| 2046 | 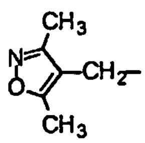 | 1 | 2 | 0 | R | H | 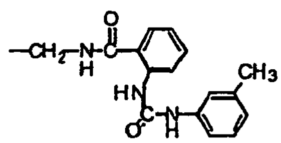 |

**Table 1.187**

| Compd. No. | 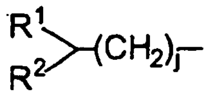 | k | m | n | c chirality | $R^3$ | 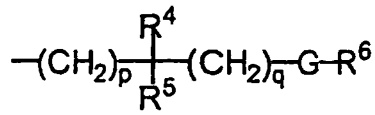 |
|---|---|---|---|---|---|---|---|
| 2047 | 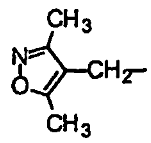 | 1 | 2 | 0 | R | H | 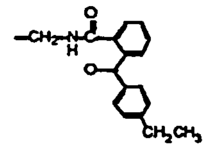 |
| 2048 | 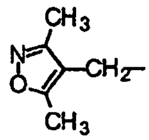 | 1 | 2 | 0 | R | H | 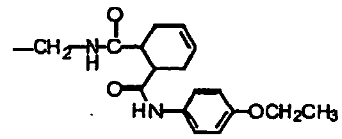 |
| 2049 | 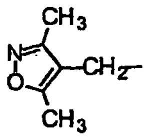 | 1 | 2 | 0 | R | H | 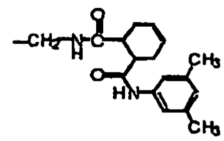 |
| 2050 | 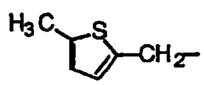 | 1 | 2 | 0 | R | H | 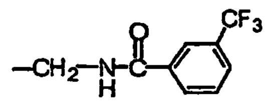 |
| 2051 | 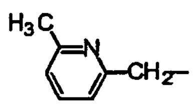 | 1 | 2 | 0 | R | H | 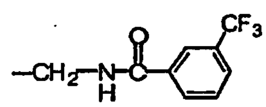 |
| 2052 | 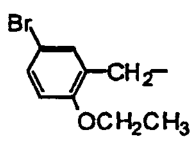 | 2 | 2 | 1 | - | H | 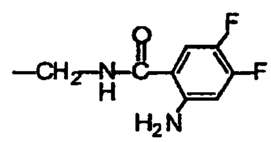 |
| 2053 | 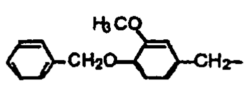 | 2 | 2 | 1 | - | H | 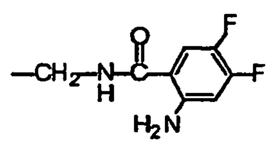 |
| 2054 | 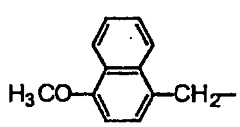 | 2 | 2 | 1 | - | H | 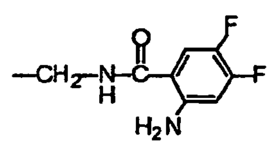 |
| 2055 | 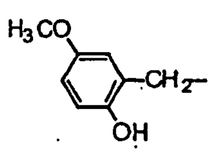 | 2 | 2 | 1 | - | H | 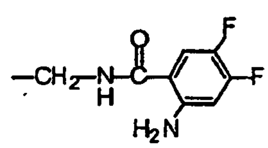 |
| 2056 | 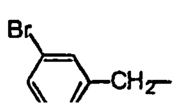 | 2 | 2 | 1 | - | H | 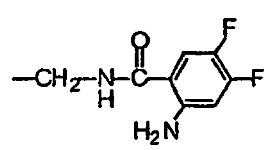 |

(continued)

| Compd. No. | 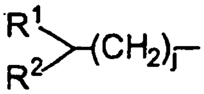 | k | m | n | c chirality | $R^3$ | 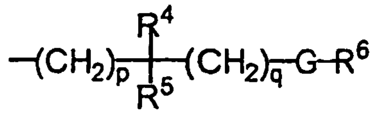 |
|---|---|---|---|---|---|---|---|
| 2057 | 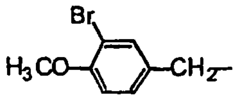 | 2 | 2 | 1 | - | H | 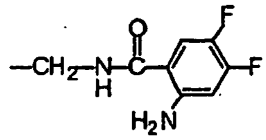 |

**Table 1.188**

| Compd. No. | 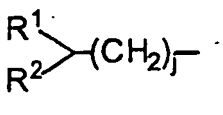 | k | m | n | chirality | $R^3$ | 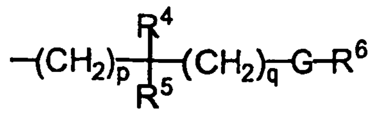 |
|---|---|---|---|---|---|---|---|
| 2058 | 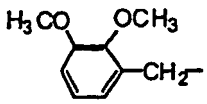 | 2 | 2 | 1 | - | H | 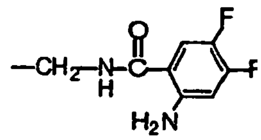 |
| 2059 | 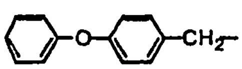 | 2 | 2 | 1 | - | H | 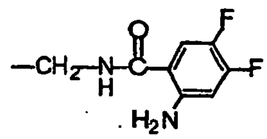 |
| 2060 | 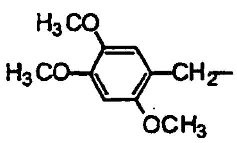 | 2 | 2 | 1 | - | H | 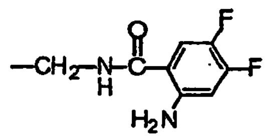 |
| 2061 | 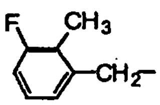 | 2 | 2 | 1 | - | H | 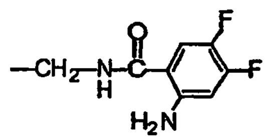 |
| 2062 | 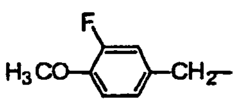 | 2 | 2 | 1 | - | H | 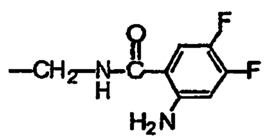 |
| 2063 | 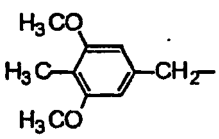 | 2 | 2 | 1 | - | H | 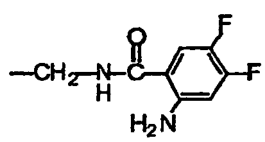 |
| 2064 | 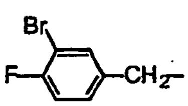 | 2 | 2 | 1 | - | H | 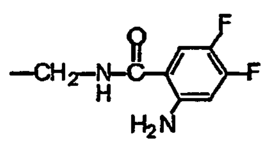 |

(continued)

| Compd. No. | 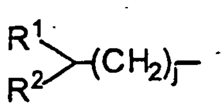 | k | m | n | chirality | $R^3$ | 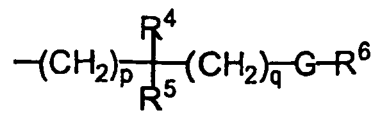 |
|---|---|---|---|---|---|---|---|
| 2065 | 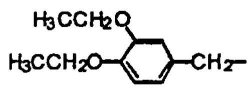 | 2 | 2 | 1 | - | H | 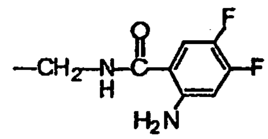 |
| 2066 | 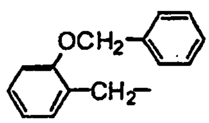 | 2 | 2 | 1 | - | H | 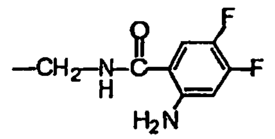 |
| 2067 | 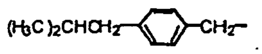 | 2 | 2 | 1 | - | H | 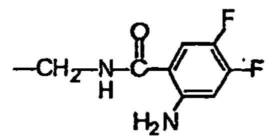 |
| 2068 | 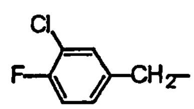 | 2 | 2 | 1 | - | H | 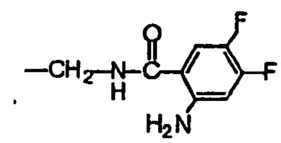 |

**Table 1.189**

| Compd. No. | 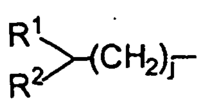 | k | m | n | chirality | $R^3$ | 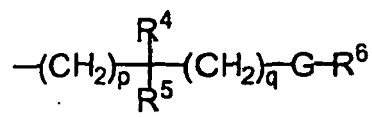 |
|---|---|---|---|---|---|---|---|
| 2069 | 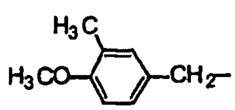 | 2 | 2 | 1 | - | H | 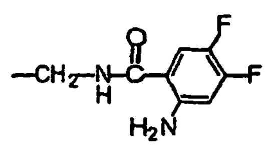 |
| 2070 | 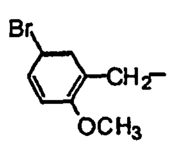 | 2 | 2 | 1 | - | H | 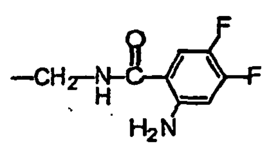 |
| 2071 | 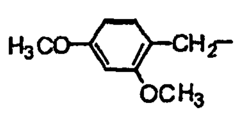 | 2 | 2 | 1 | - | H | 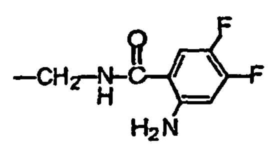 |
| 2072 | 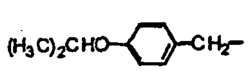 | 2 | 2 | 1 | - | H | 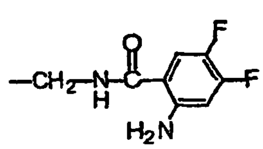 |

(continued)

| Compd. No. | 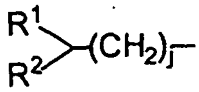 | k | m | n | chirality | $R^3$ | 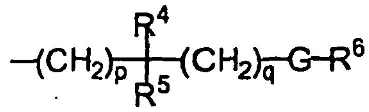 |
|---|---|---|---|---|---|---|---|
| 2073 | 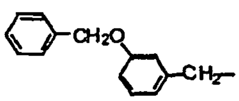 | 2 | 2 | 1 | - | H | 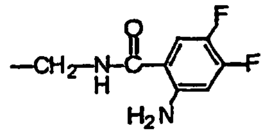 |
| 2074 | 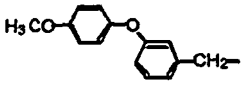 | 2 | 2 | 1 | - | H | 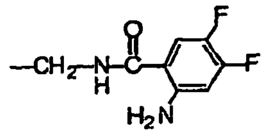 |
| 2075 | 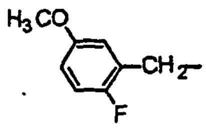 | 2 | 2 | 1 | - | H | 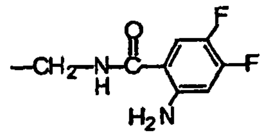 |
| 2076 | 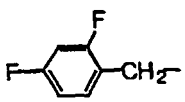 | 2 | 2 | 1 | - | H | 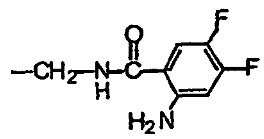 |
| 2077 | 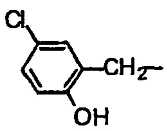 | 2 | 2 | 1 | - | H | 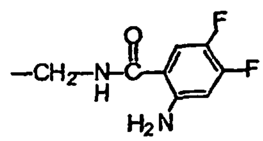 |
| 2078 | 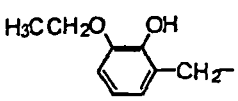 | 2 | 2 | 1 | - | H | 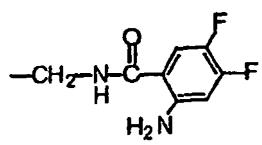 |
| 2079 | 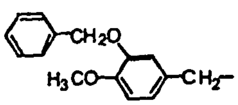 | 2 | 2 | 1 | - | H | 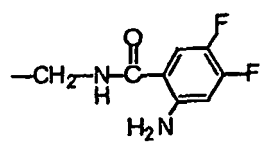 |

**Table 1.190**

| Compd. No. | 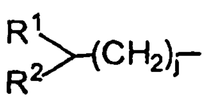 | k | m | n | chirality | $R^3$ | 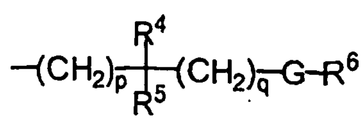 |
|---|---|---|---|---|---|---|---|
| 2080 | 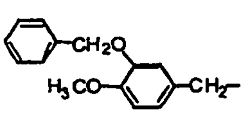 | 2 | 2 | 1 | - | H | 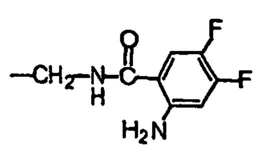 |

(continued)

| Compd. No. | 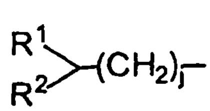 | k | m | n | chirality | $R^3$ | 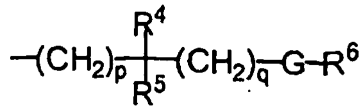 |
|---|---|---|---|---|---|---|---|
| 2081 | 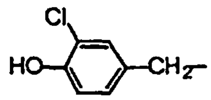 | 2 | 2 | 1 | - | H | 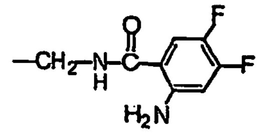 |
| 2082 | 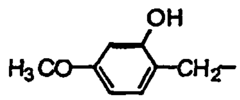 | 2 | 2 | 1 | - | H | 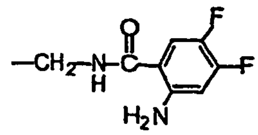 |
| 2083 | 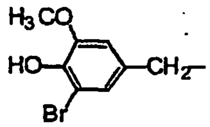 | 1 | 2 | 0 | R | H | 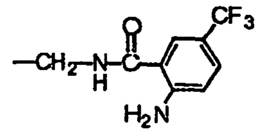 |
| 2084 | 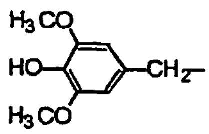 | 1 | 2 | 0 | R | H | 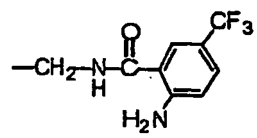 |
| 2085 | 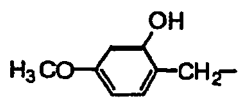 | 1 | 2 | 0 | R | H | 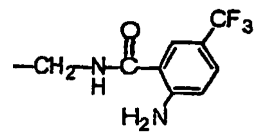 |
| 2086 | 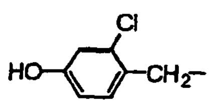 | 1 | 2 | 0 | R | H | 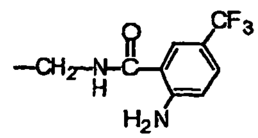 |
| 2087 | 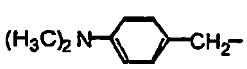 | 1 | 2 | 0 | R | H | 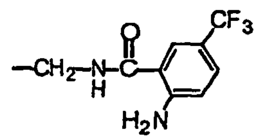 |
| 2088 | 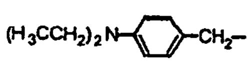 | 1 | 2 | 0 | R | H | 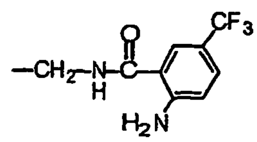 |
| 2089 | 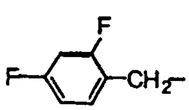 | 1 | 2 | 0 | R | H | 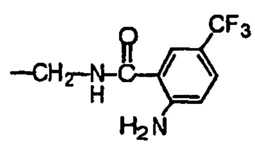 |

(continued)

| Compd. No. | 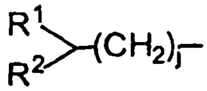 | k | m | n | chirality | $R^3$ | 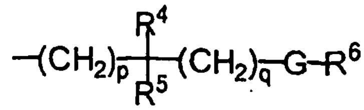 |
|---|---|---|---|---|---|---|---|
| 2090 | 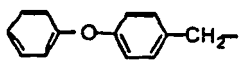 | 1 | 2 | 0 | R | H | 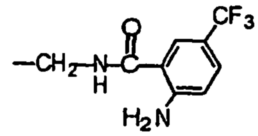 |

**Table 1.191**

| Compd. No. | 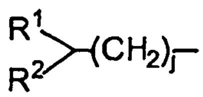 | k | m | n | chirality | $R^3$ | 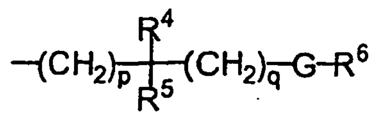 |
|---|---|---|---|---|---|---|---|
| 2091 | 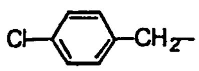 | 2 | 2 | 1 | - | H | 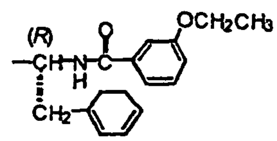 |
| 2092 | 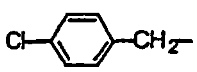 | 2 | 2 | 1 | - | H | 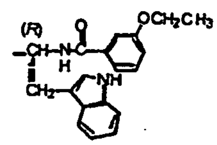 |
| 2093 | 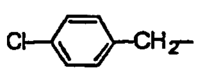 | 2 | 2 | 1 | - | H | 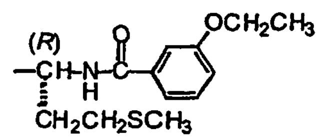 |
| 2094 | 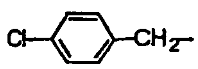 | 2 | 2 | 1 | - | H | 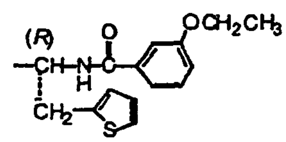 |
| 2095 | 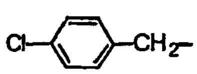 | 2 | 2 | 1 | - | H | 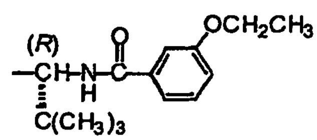 |
| 2096 | 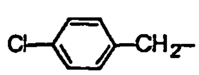 | 2 | 2 | 1 | - | H | 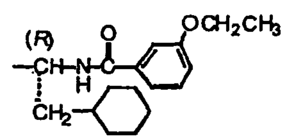 |
| 2097 | 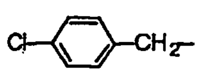 | 2 | 2 | 1 | - | H | 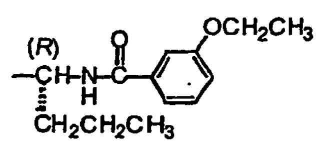 |

(continued)

| Compd. No. | 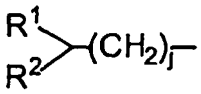 | k | m | n | chirality | $R^3$ | 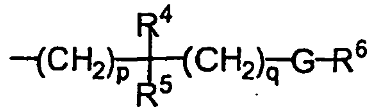 |
|---|---|---|---|---|---|---|---|
| 2098 | 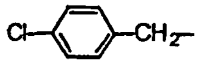 | 2 | 2 | 1 | - | H | 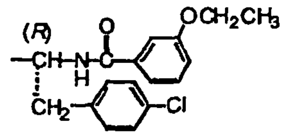 |
| 2099 | 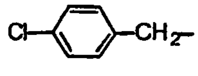 | 2 | 2 | 1 | - | H | 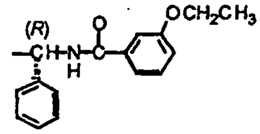 |
| 2100 | 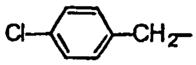 | 2 | 2 | 1 | - | H | 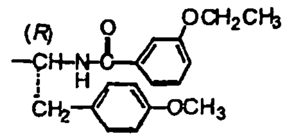 |
| 2101 | 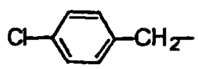 | 2 | 2 | 1 | - | H | 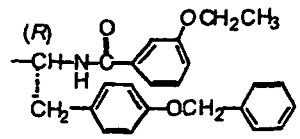 |

**Table 1.192**

| Compd. No. | 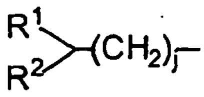 | k | m | n | chirality | $R^3$ | 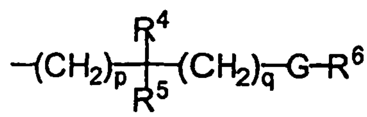 |
|---|---|---|---|---|---|---|---|
| 2102 | 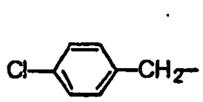 | 2 | 2 | 1 | - | H | 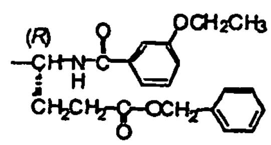 |
| 2103 | 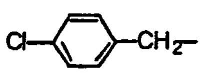 | 2 | 2 | 1 | - | H | 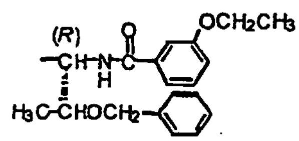 |
| 2104 | 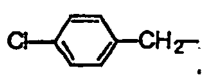 | 2 | 2 | 1 | - | H | 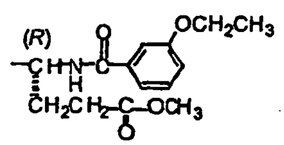 |
| 2105 | 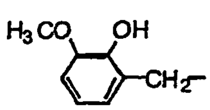 | 2 | 2 | 1 | - | H | 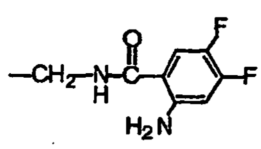 |

(continued)

| Compd. No. | 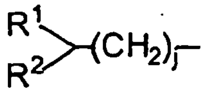 | k | m | n | chirality | $R^3$ | 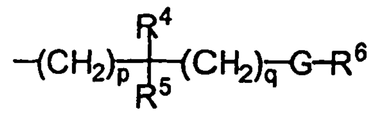 |
|---|---|---|---|---|---|---|---|
| 2106 | 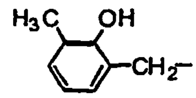 | 2 | 2 | 1 | - | H | 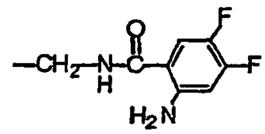 |
| 2107 | 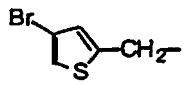 | 2 | 2 | 1 | - | H | 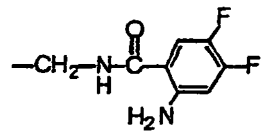 |
| 2108 | 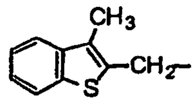 | 2 | 2 | 1 | - | H | 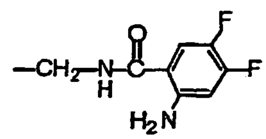 |
| 2109 | 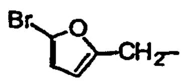 | 2 | 2 | 1 | - | H | 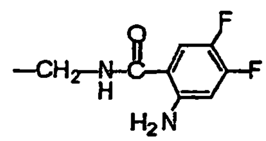 |
| 2110 | 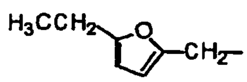 | 2 | 2 | 1 | - | H | 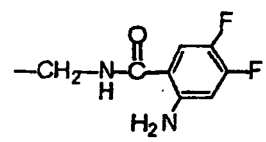 |
| 2111 | 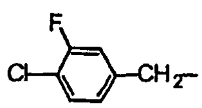 | 2 | 2 | 1 | - | H | 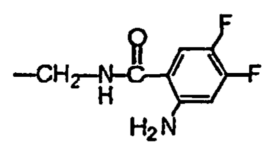 |
| 2112 | 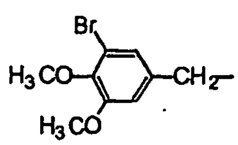 | 2 | 2 | 1 | - | H | 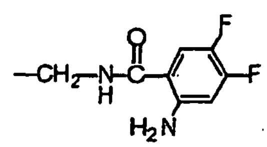 |

**Table 1.193**

| Compd. No. | 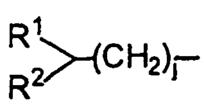 | k | m | n | chirality | $R^3$ | 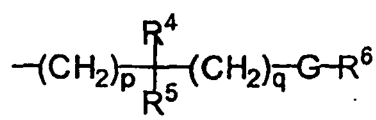 |
|---|---|---|---|---|---|---|---|
| 2113 | 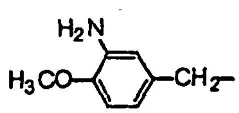 | 2 | 2 | 1 | - | H | 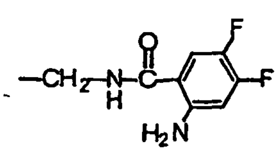 |

(continued)

| Compd. No. | 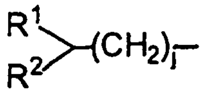 | k | m | n | chirality | $R^3$ | 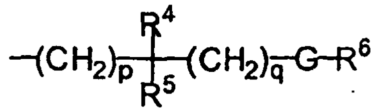 |
|---|---|---|---|---|---|---|---|
| 2114 | 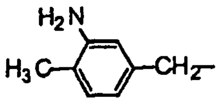 | 2 | 2 | 1 | - | H | 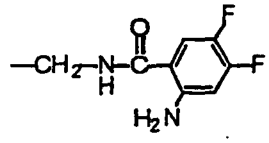 |
| 2115 | 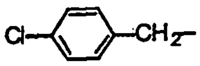 | 2 | 2 | 1 | - | H | 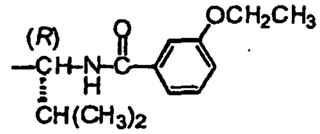 |
| 2116 | 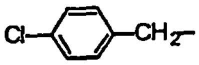 | 2 | 2 | 1 | - | H | 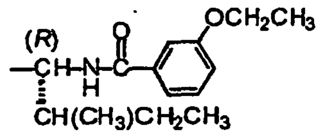 |
| 2117 | 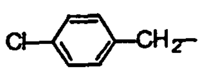 | 2 | 2 | 1 | - | H | 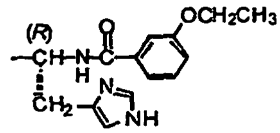 |
| 2118 | 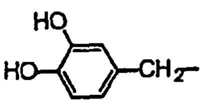 | 1 | 2 | 0 | R | H | 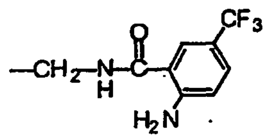 |
| 2119 | 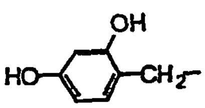 | 1 | 2 | 0 | R | H | 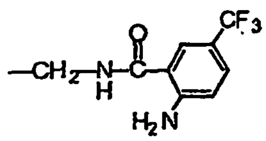 |
| 2120 | 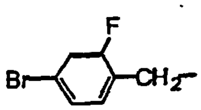 | 1 | 2 | 0 | R | H | 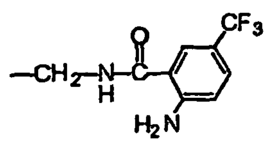 |
| 2121 | 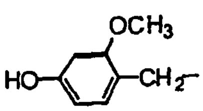 | 1 | 2 | 0 | R | H | 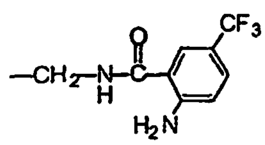 |
| 2122 | 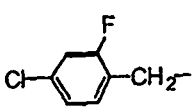 | 1 | 2 | 0 | R | H | 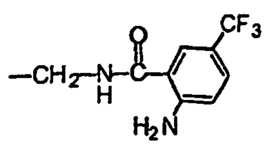 |

(continued)

| Compd. No. | 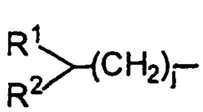 | k | m | n | chirality | $R^3$ | 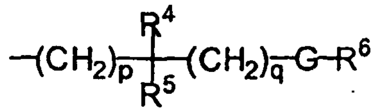 |
|---|---|---|---|---|---|---|---|
| 2123 | 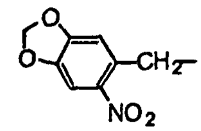 | 1 | 2 | 0 | R | H | 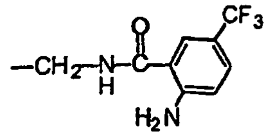 |

Table 1.194

| Compd. No. | 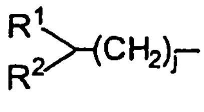 | k | m | n | chirality | $R^3$ | 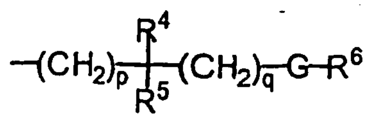 |
|---|---|---|---|---|---|---|---|
| 2124 | 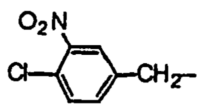 | 1 | 2 | 0 | R | H | 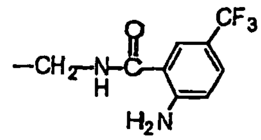 |
| 2125 | 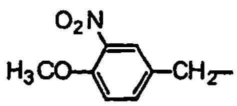 | 1 | 2 | 0 | R | H | 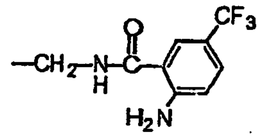 |
| 2126 | 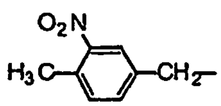 | 1 | 2 | 0 | R | H | 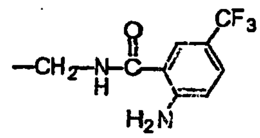 |
| 2127 | 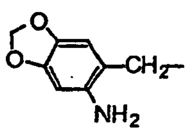 | 1 | 2 | 0 | R | H | 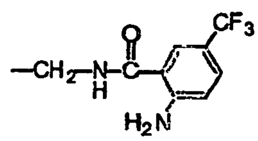 |
| 2128 | 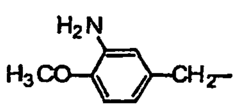 | 1 | 2 | 0 | R | H | 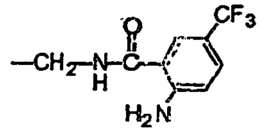 |
| 2129 | 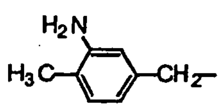 | 1 | 2 | 0 | R | H | 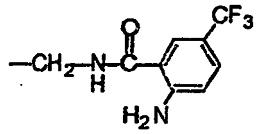 |
| 2130 | 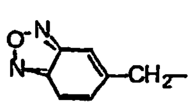 | 2 | 2 | 1 | - | H | 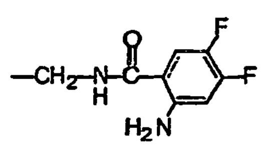 |

(continued)

| Compd. No. | 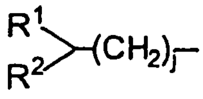 | k | m | n | chirality | $R^3$ | 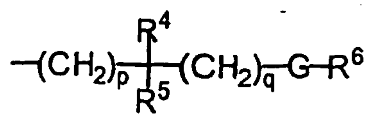 |
|---|---|---|---|---|---|---|---|
| 2131 | 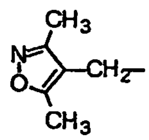 | 2 | 2 | 1 | - | H | 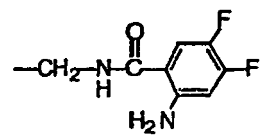 |
| 2132 | 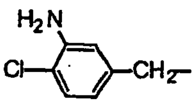 | 1 | 2 | 0 | R | H | 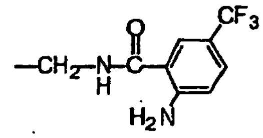 |
| 2133 | 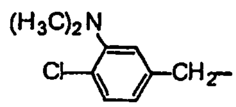 | 1 | 2 | 0 | R | H | 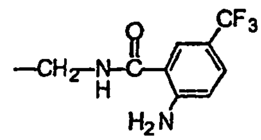 |
| 2134 | 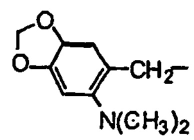 | 1 | 2 | 0 | R | H | 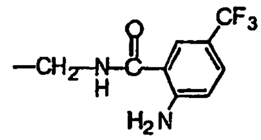 |

**Table 1.195**

| Compd. No. | 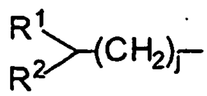 | k | m | n | chirality | $R^3$ | 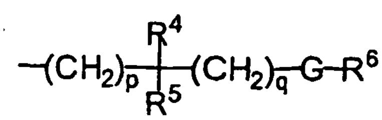 |
|---|---|---|---|---|---|---|---|
| 2135 | 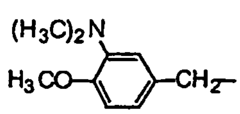 | 1 | 2 | 0 | R | H | 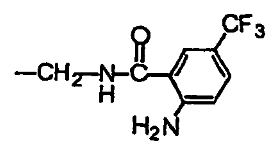 |
| 2136 | 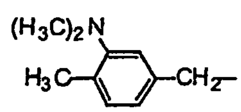 | 1 | 2 | 0 | R | H | 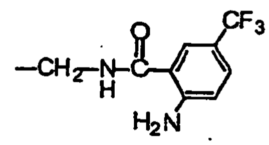 |
| 2137 | 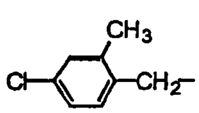 | 1 | 2 | 0 | R | H | 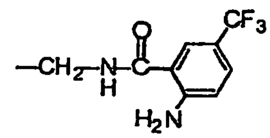 |
| 2138 | 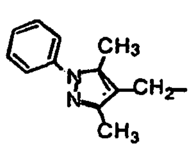 | 1 | 2 | 0 | R | H | 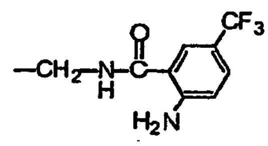 |

(continued)

| Compd. No. | 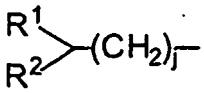 | k | m | n | chirality | $R^3$ | 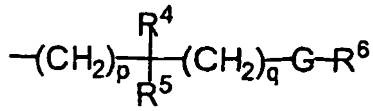 |
|---|---|---|---|---|---|---|---|
| 2139 | 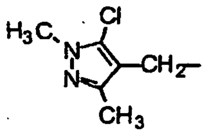 | 1 | 2 | 0 | R | H | 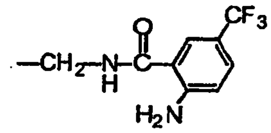 |
| 2140 | 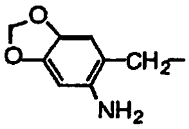 | 2 | 2 | 1 | - | H | 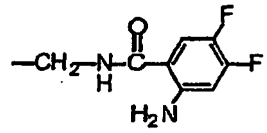 |
| 2141 | 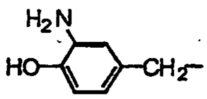 | 2 | 2 | 1 | - | H | 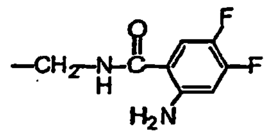 |
| 2142 | 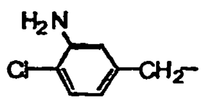 | 2 | 2 | 1 | - | H | 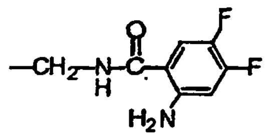 |
| 2143 | 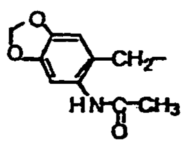 | 2 | 2 | 1 | - | H | 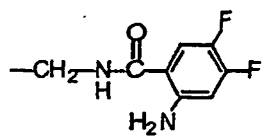 |
| 2144 | 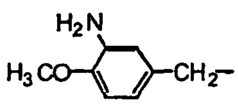 | 2 | 2 | 1 | - | H | 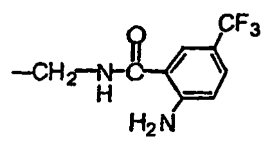 |
| 2145 | 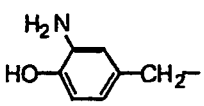 | 2 | 2 | 1 | - | H | 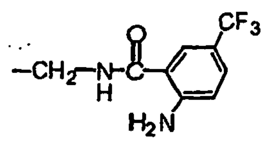 |

**Table 1.196**

| Compd. No. | 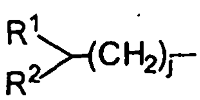 | k | m | n | chirality | $R^3$ | 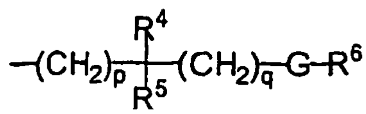 |
|---|---|---|---|---|---|---|---|
| 2146 | 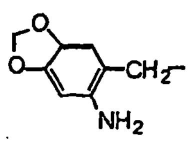 | 2 | 2 | 1 | - | H | 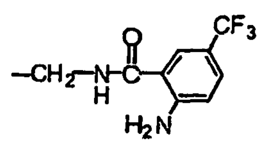 |

(continued)

| Compd. No. | 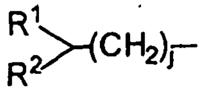 | k | m | n | chirality | $R^3$ | 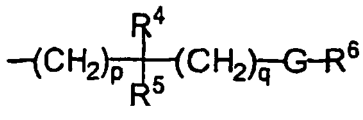 |
|---|---|---|---|---|---|---|---|
| 2147 | 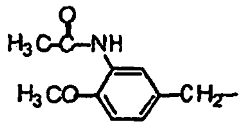 | 2 | 2 | 1 | - | H | 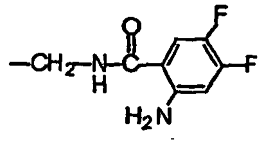 |
| 2148 | 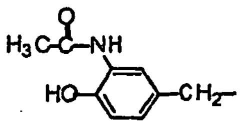 | 2 | 2 | 1 | - | H | 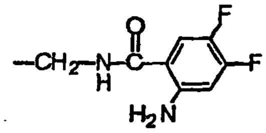 |
| 2149 | 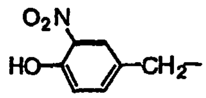 | 1 | 2 | 0 | R | H | 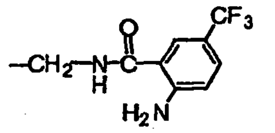 |
| 2150 | 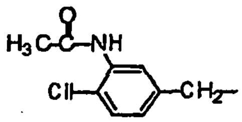 | 1 | 2 | 0 | R | H | 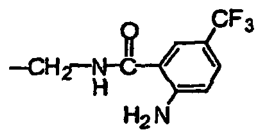 |
| 2151 | 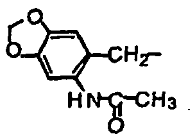 | 1 | 2 | 0 | R | H | 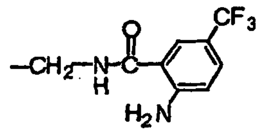 |
| 2152 | 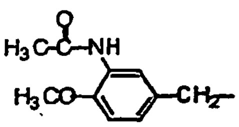 | 1 | 2 | 0 | R | H | 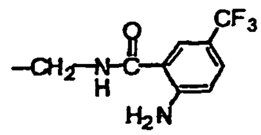 |
| 2153 | 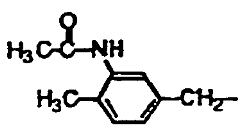 | 1 | 2 | 0 | R | H | 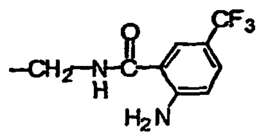 |
| 2154 | 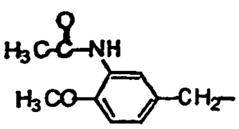 | 2 | 2 | 1 | - | H | 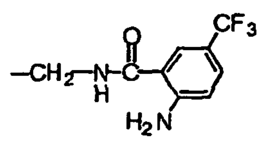 |
| 2155 | 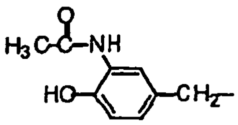 | 2 | 2 | 1 | - | H | 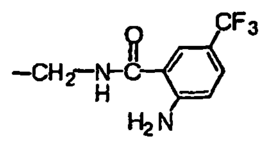 |
| 2156 | 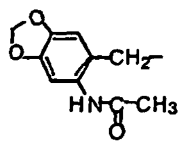 | 2 | 2 | 1 | - | H | 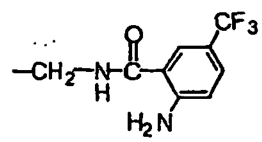 |

**Table 1.197**

| Compd. No. | 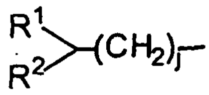 | k | m | n | chirality | $R^3$ | 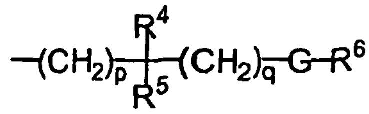 |
|---|---|---|---|---|---|---|---|
| 2157 | 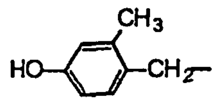 | 1 | 2 | 0 | R | H | 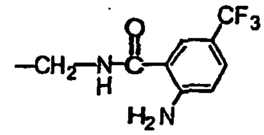 |
| 2158 | 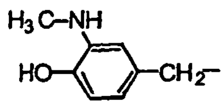 | 1 | 2 | 0 | R | H | 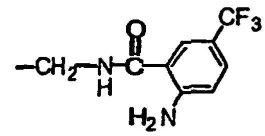 |
| 2159 | 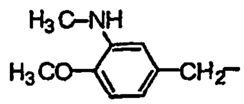 | 2 | 2 | 1 | - | H | 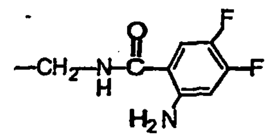 |
| 2160 | 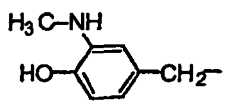 | 2 | 2 | 1 | - | H | 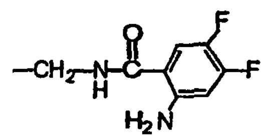 |
| 2161 | 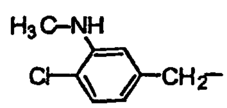 | 2 | 2 | 1 | - | H | 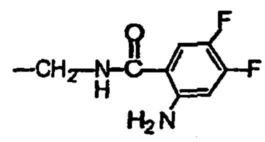 |
| 2162 | 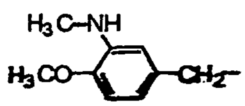 | 2 | 2 | 1 | - | H | 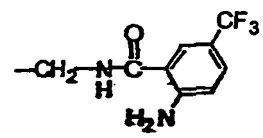 |
| 2163 | 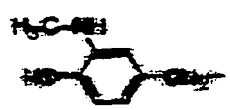 | 2 | 2 | 1 | - | H | 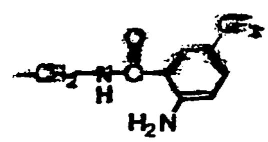 |
| 2164 | 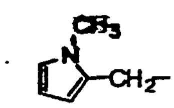 | 1 | 2 | 0 | R | H | 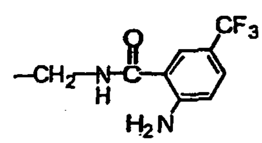 |
| 2165 | 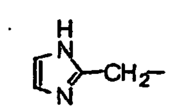 | 1 | 2 | 0 | R | H | 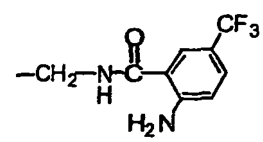 |

(continued)

| Compd. No. | 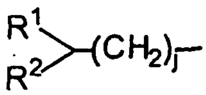 | k | m | n | chirality | $R^3$ | 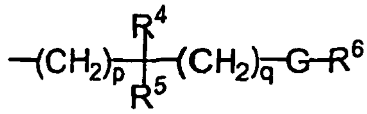 |
|---|---|---|---|---|---|---|---|
| 2166 | 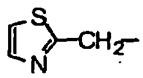 | 1 | 2 | 0 | R | H | 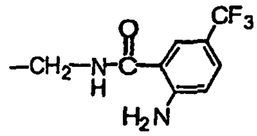 |
| 2167 | 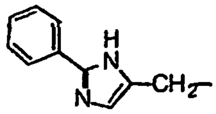 | 1 | 2 | 0 | R | H | 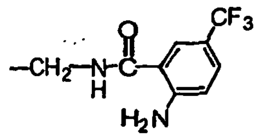 |

**Table 1.198**

| Compd. No. | 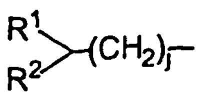 | k | m | n | chirality | $R^3$ | 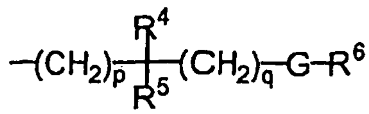 |
|---|---|---|---|---|---|---|---|
| 2168 | 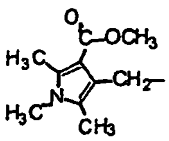 | 1 | 2 | 0 | R | H | 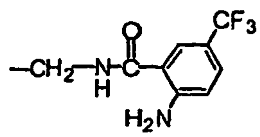 |
| 2169 | 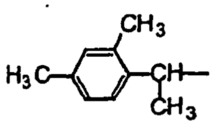 | 1 | 2 | 0 | R | H | 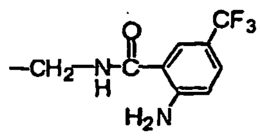 |
| 2170 | 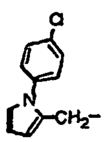 | 1 | 2 | 0 | R | H | 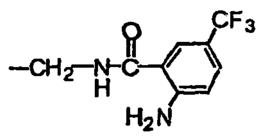 |
| 2171 | 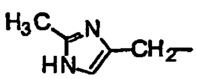 | 1 | 2 | 0 | R | H | 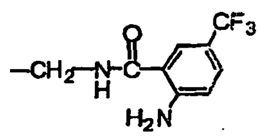 |
| 2172 | 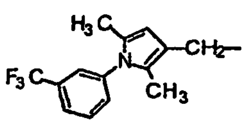 | 1 | 2 | 0 | R | H | 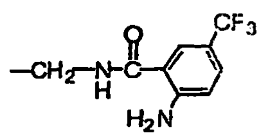 |
| 2173 | 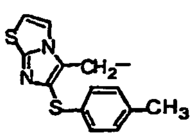 | 1 | 2 | 0 | R | H | 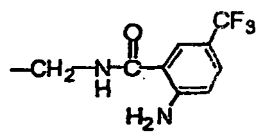 |

(continued)

| Compd. No. | 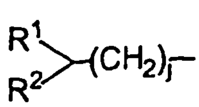 | k | m | n | chirality | $R^3$ | 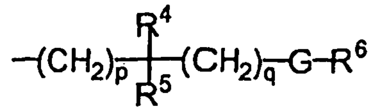 |
|---|---|---|---|---|---|---|---|
| 2174 | 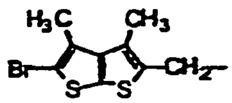 | 1 | 2 | 0 | R | H | 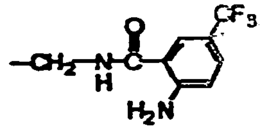 |
| 2175 | 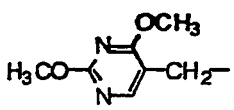 | 1 | 2 | 0 | R | H | 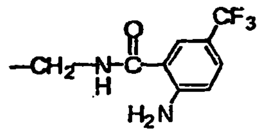 |
| 2176 | 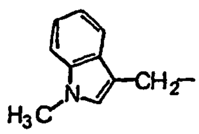 | 1 | 2 | 0 | R | H | 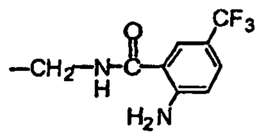 |
| 2177 | 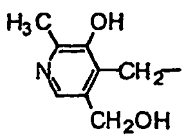 | 1 | 2 | 0 | R | H | 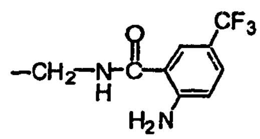 |
| 2178 | 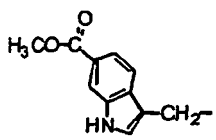 | 1 | 2 | 0 | R | H | 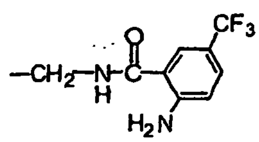 |

**Table 1.199**

| Compd. No. | 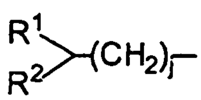 | k | m | n | chirality | $R^3$ | 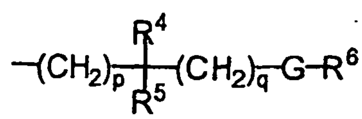 |
|---|---|---|---|---|---|---|---|
| 2179 | 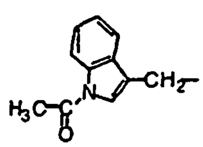 | 1 | 2 | 0 | R | H | 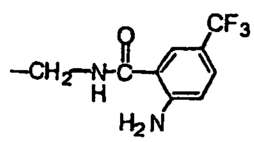 |
| 2180 | 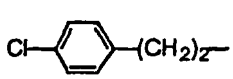 | 1 | 2 | 0 | R | H | 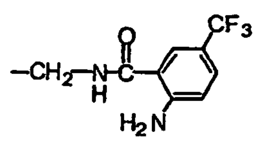 |
| 2181 | 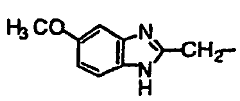 | 1 | 2 | 0 | R | H | 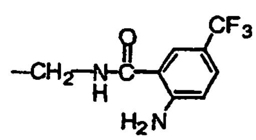 |

(continued)

| Compd. No. | 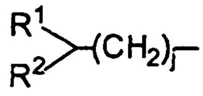 | k | m | n | chirality | $R^3$ | 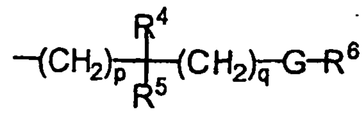 |
|---|---|---|---|---|---|---|---|
| 2182 | 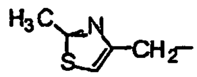 | 1 | 2 | 0 | R | H | 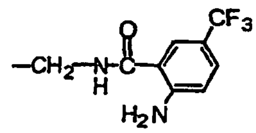 |
| 2183 | 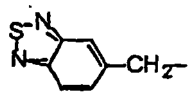 | 1 | 2 | 0 | R | H | 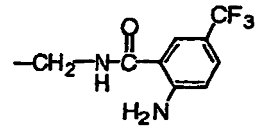 |
| 2184 | 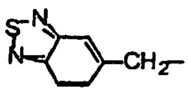 | 2 | 2 | 1 | - | H | 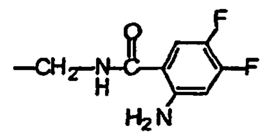 |
| 2185 | 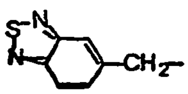 | 2 | 2 | 1 | - | H | 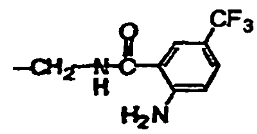 |
| 2186 | 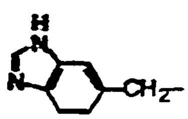 | 2 | 2 | 1 | - | H | 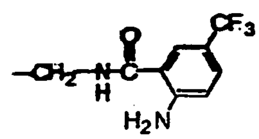 |
| 2187 | 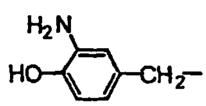 | 1 | 2 | 0 | R | H | 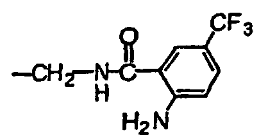 |
| 2188 | 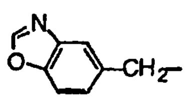 | 2 | 2 | 1 | - | H | 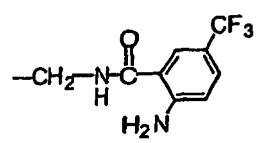 |
| 2189 | 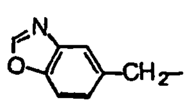 | 1 | 2 | 0 | R | H | 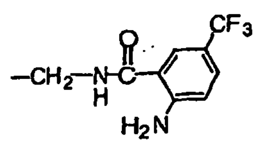 |

**Table 1.200**

| Compd. No. | 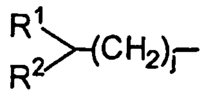 | k | m | n | chirality | $R^3$ | 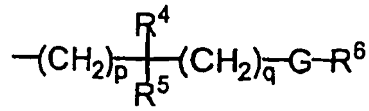 |
|---|---|---|---|---|---|---|---|
| 2190 | 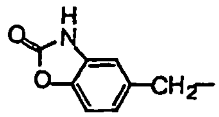 | 2 | 2 | 1 | - | H | 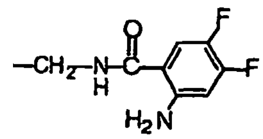 |
| 2191 | 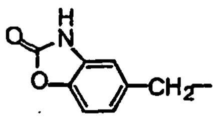 | 2 | 2 | 1 | - | H | 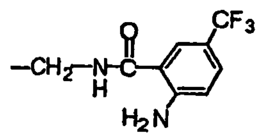 |
| 2192 | 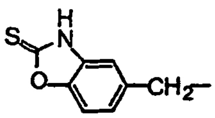 | 2 | 2 | 1 | - | H | 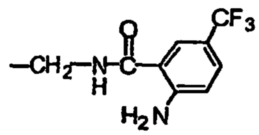 |
| 2193 | 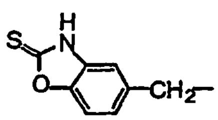 | 2 | 2 | 1 | - | H | 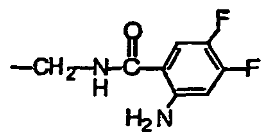 |
| 2194 | 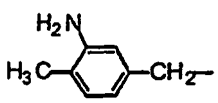 | 2 | 2 | 1 | - | H | 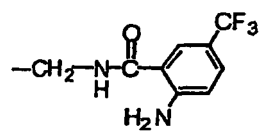 |
| 2195 | 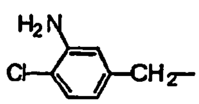 | 2 | 2 | 1 | - | H | 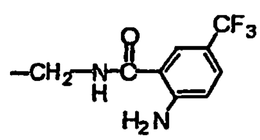 |
| 2196 | 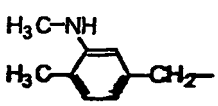 | 1 | 2 | 0 | R | H | 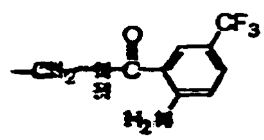 |
| 2197 | 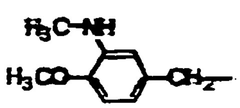 | 1 | 2 | 0 | R | H | 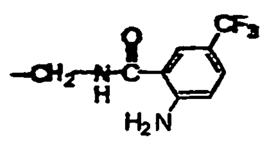 |
| 2198 | 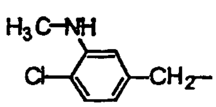 | 1 | 2 | 0 | R | H | 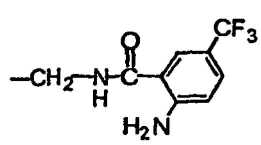 |
| 2199 | 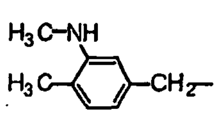 | 2 | 2 | 1 | - | H | 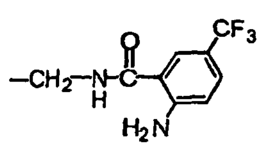 |

(continued)

| Compd. No. | 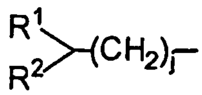 | k | m | n | chirality | R3 | 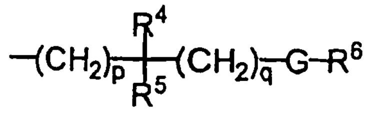 |
|---|---|---|---|---|---|---|---|
| 2200 | 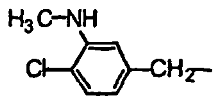 | 2 | 2 | 1 | - | H | 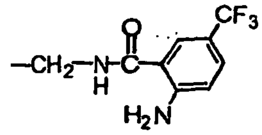 |

**Table 1.201**

| Compd. No. | 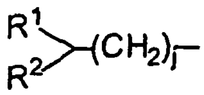 | k | m | n | chirality | $R^3$ | 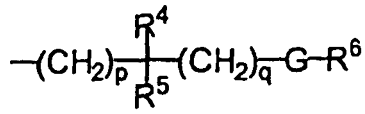 |
|---|---|---|---|---|---|---|---|
| 2201 | 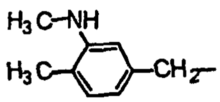 | 2 | 2 | 1 | - | H | 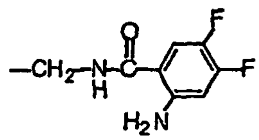 |
| 2202 | 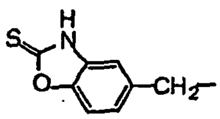 | 1 | 2 | 0 | R | H | 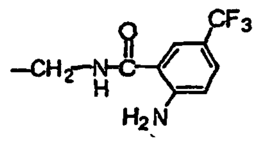 |
| 2203 | 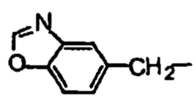 | 2 | 2 | 1 | - | H | 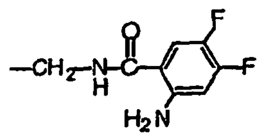 |
| 2204 | 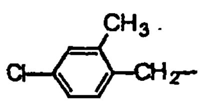 | 2 | 2 | 1 | - | H | 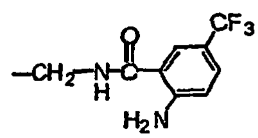 |
| 2205 | 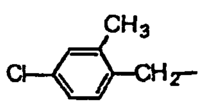 | 2 | 2 | 1 | - | H | 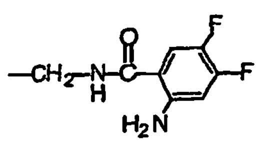 |
| 2206 | 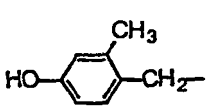 | 2 | 2 | 1 | - | H | 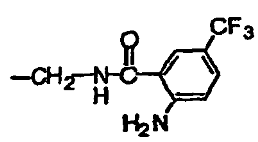 |
| 2207 | 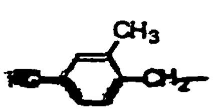 | 2 | 2 | 1 | - | H | 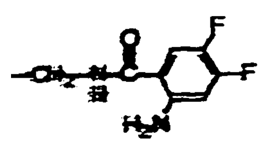 |

(continued)

| Compd. No. | 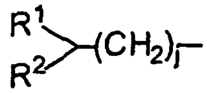 | k | m | n | chirality | $R^3$ | 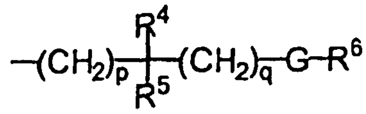 |
|---|---|---|---|---|---|---|---|
| 2208 | 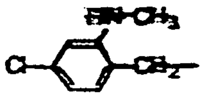 | 2 | 2 | 1 | - | H | 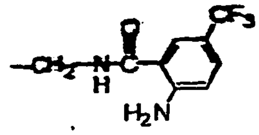 |
| 2209 | 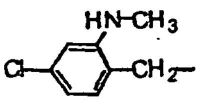 | 2 | 2 | 1 | - | H | 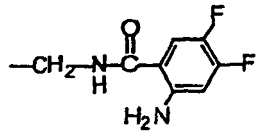 |
| 2210 | 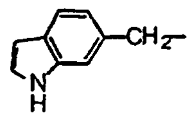 | 1 | 2 | 0 | R | H | 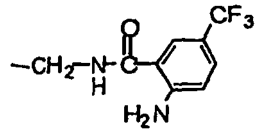 |
| 2211 | 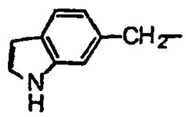 | 2 | 2 | 1 | - | H | 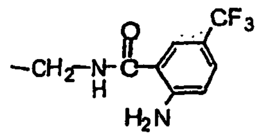 |

**Table 1.202**

| Compd. No. | 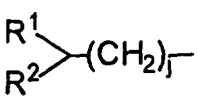 | k | m | n | chirality | $R^3$ | 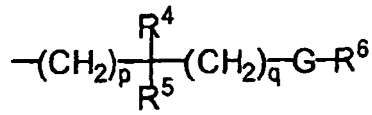 |
|---|---|---|---|---|---|---|---|
| 2212 | 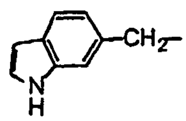 | 2 | 2 | 1 | - | H | 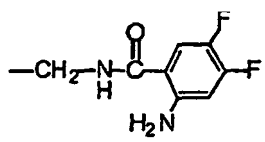 |
| 2213 | 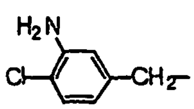 | 2 | 2 | 1 | - | H | 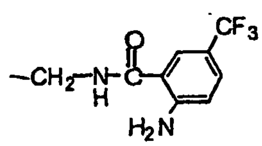 |
| 2214 | 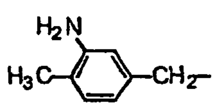 | 2 | 2 | 1 | - | H | 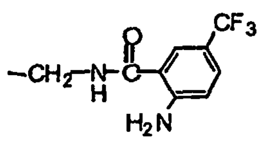 |
| 2215 | 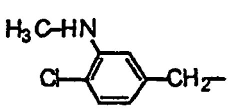 | 1 | 2 | 0 | R | H | 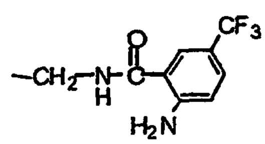 |

(continued)

| Compd. No. | 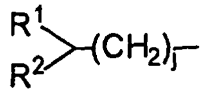 | k | m | n | chirality | $R^3$ | 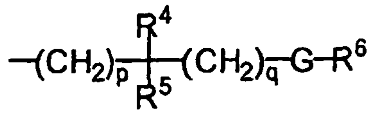 |
|---|---|---|---|---|---|---|---|
| 2216 | 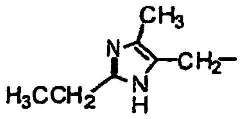 | 1 | 2 | 0 | R | H | 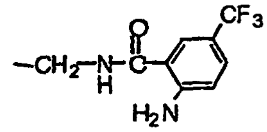 |
| 2217 | 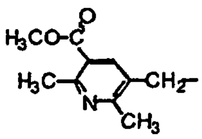 | 1 | 2 | 0 | R | H | 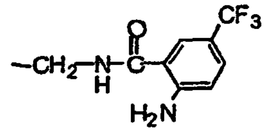 |
| 2218 | 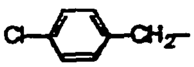 | 1 | 2 | 0 | R | H | 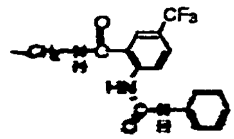 |
| 2219 | 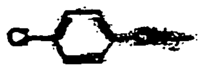 | 1 | 2 | 0 | R | H | 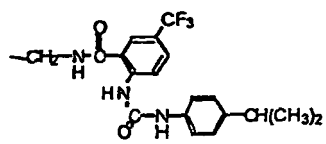 |
| 2220 | 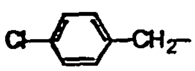 | 1 | 2 | 0 | R | H | 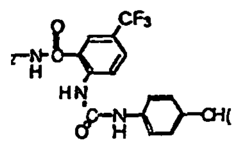 |
| 2221 | 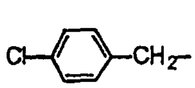 | 1 | 2 | 0 | R | H | 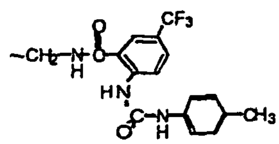 |
| 2222 | 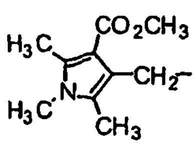 | 1 | 2 | 0 | R | H | 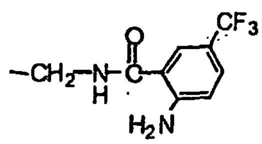 |

**Table 1.203**

| Compd. No. | 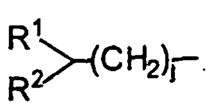 | k | m | n | chirality | $R^3$ | 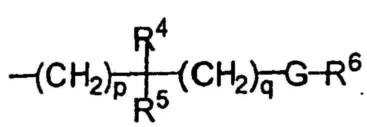 |
|---|---|---|---|---|---|---|---|
| 2223 | 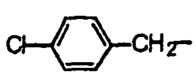 | 1 | 2 | 0 | R | H | 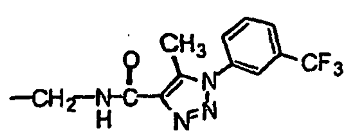 |

(continued)

| Compd. No. | 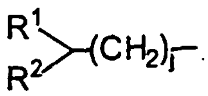 | k | m | n | chirality | $R^3$ | 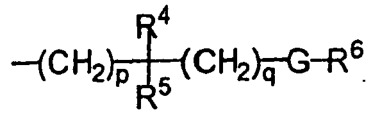 |
|---|---|---|---|---|---|---|---|
| 2224 | 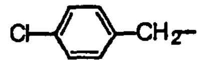 | 1 | 2 | 0 | R | H | 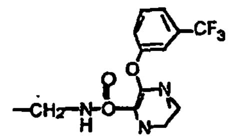 |
| 2225 | 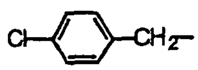 | 1 | 2 | 0 | R | H | 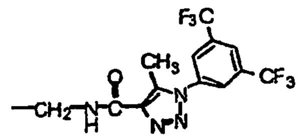 |
| 2226 | 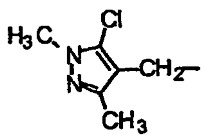 | 1 | 2 | 0 | R | H | 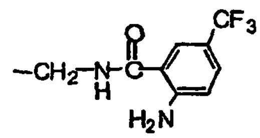 |
| 2227 | 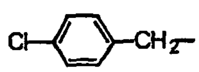 | 1 | 2 | 0 | R | H | 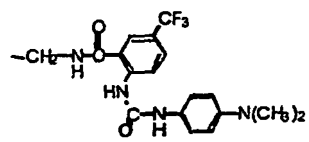 |
| 2228 | 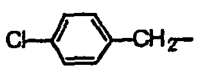 | 1 | 2 | 0 | R | H | 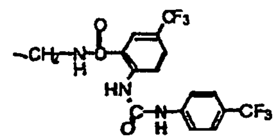 |
| 2229 | 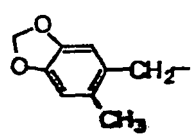 | 1 | 2 | 0 | R | H | 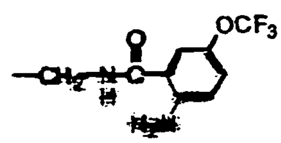 |
| 2230 | 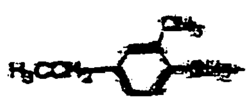 | 1 | 2 | 0 | R | H | 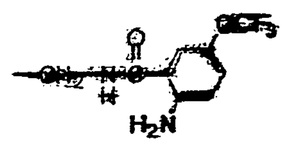 |
| 2231 | 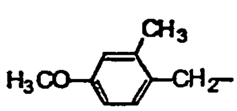 | 1 | 2 | 0 | R | H | 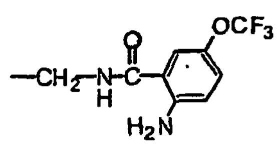 |
| 2232 | 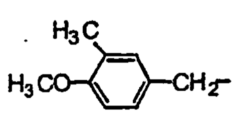 | 1 | 2 | 0 | R | H | 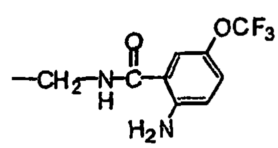 |

(continued)

| Compd. No. | 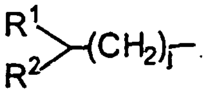 | k | m | n | chirality | $R^3$ | 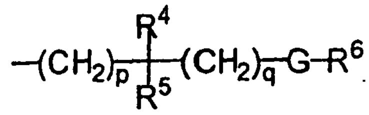 |
|---|---|---|---|---|---|---|---|
| 2233 | 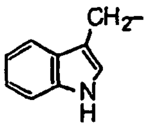 | 1 | 2 | 0 | R | H | 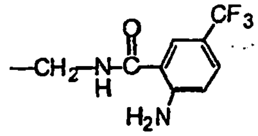 |

Table 1.204

| Compd. No. | 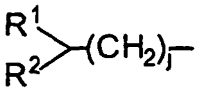 | k | m | n | chirality | $R^3$ | 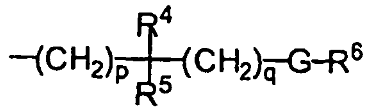 |
|---|---|---|---|---|---|---|---|
| 2234 | 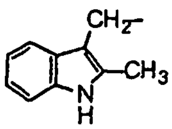 | 1 | 2 | 0 | R | H | 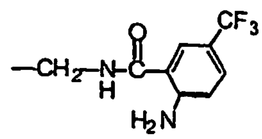 |
| 2235 | 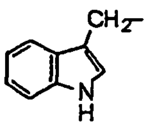 | 1 | 2 | 0 | R | H | 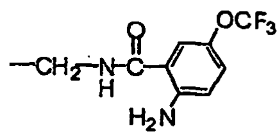 |
| 2236 | 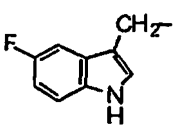 | 1 | 2 | 0 | R | H | 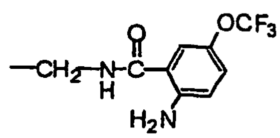 |
| 2237 | 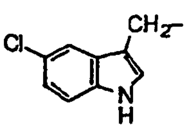 | 1 | 2 | 0 | R | H | 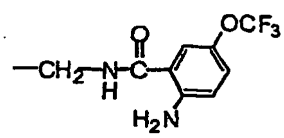 |
| 2238 | 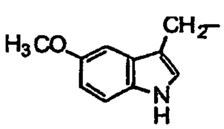 | 1 | 2 | 0 | R | H | 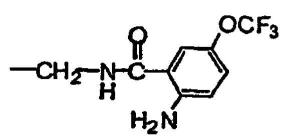 |
| 2239 | 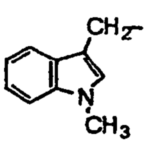 | 1 | 2 | 0 | R | H | 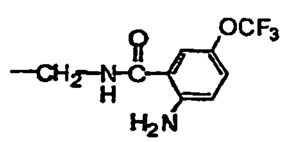 |
| 2240 | 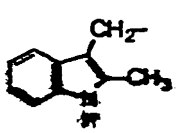 | 1 | 2 | 0 | R | H | 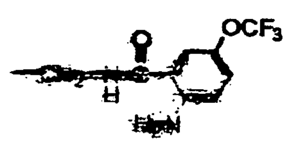 |

(continued)

| Compd. No. | 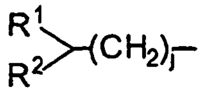 | k | m | n | chirality | $R^3$ | 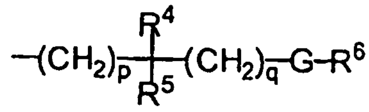 |
|---|---|---|---|---|---|---|---|
| 2241 | 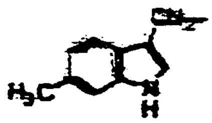 | 1 | 2 | 0 | R | H | 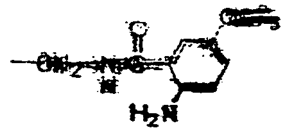 |
| 2242 | 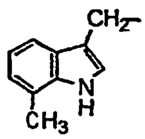 | 1 | 2 | 0 | R | H | 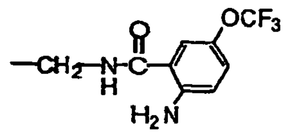 |
| 2243 | 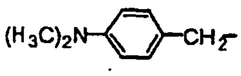 | 1 | 2 | 0 | R | H | 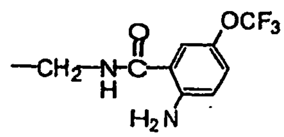 |
| 2244 | 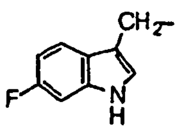 | 1 | 2 | 0 | R | H | 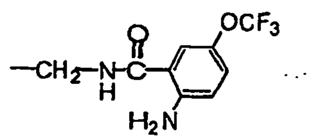 |

**Table 1.205**

| Compd. No. | 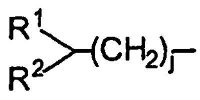 | k | m | n | chirality | $R^3$ | 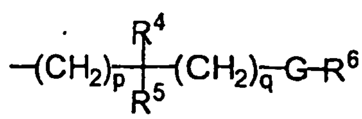 |
|---|---|---|---|---|---|---|---|
| 2245 | 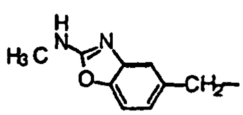 | 1 | 2 | 0 | R | H | 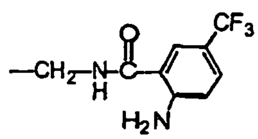 |
| 2246 | 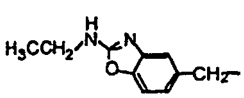 | 1 | 2 | 0 | R | H | 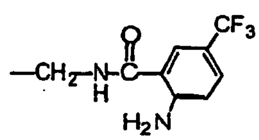 |
| 2247 | 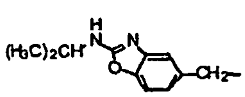 | 1 | 2 | 0 | R | H | 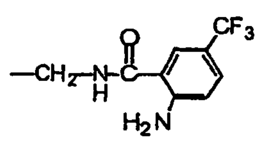 |
| 2248 | 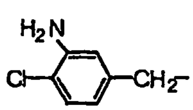 | 1 | 2 | 0 | R | H | 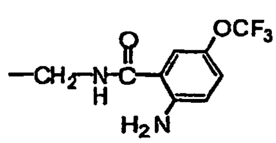 |

(continued)

| Compd. No. | 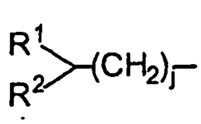 | k | m | n | chirality | $R^3$ | 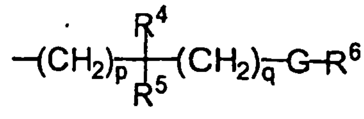 |
|---|---|---|---|---|---|---|---|
| 2249 | 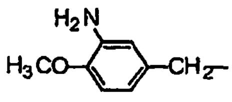 | 1 | 2 | 0 | R | H | 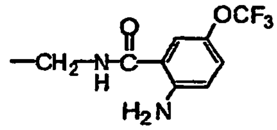 |
| 2250 | 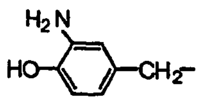 | 1 | 2 | 0 | R | H | 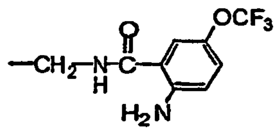 |
| 2251 | 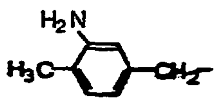 | 1 | 2 | 0 | R | H | 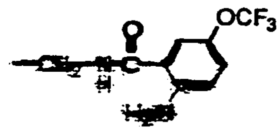 |
| 2252 | 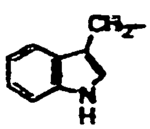 | 2 | 2 | 1 | - | H | 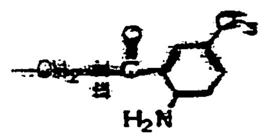 |
| 2253 | 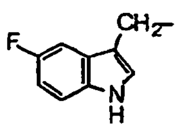 | 2 | 2 | 1 | - | H | 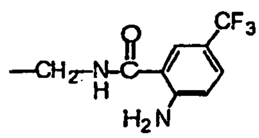 |
| 2254 | 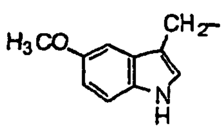 | 2 | 2 | 1 | - | H | 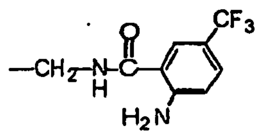 |
| 2255 | 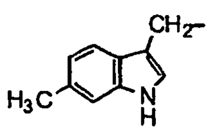 | 2 | 2 | 1 | - | H | 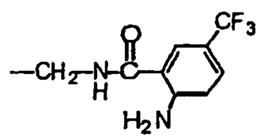 |

**Table 1.206**

| Compd. No. | 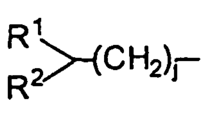 | k | m | n | chirality | $R^3$ | 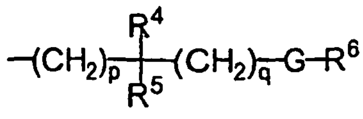 |
|---|---|---|---|---|---|---|---|
| 2256 | 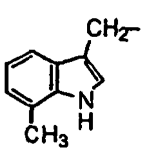 | 2 | 2 | 1 | - | H | 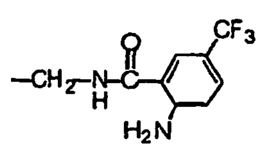 |

(continued)

| Compd. No. | 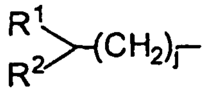 | k | m | n | chirality | $R^3$ | 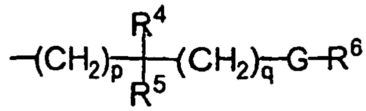 |
|---|---|---|---|---|---|---|---|
| 2257 | 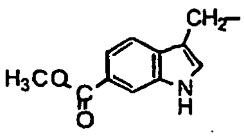 | 2 | 2 | 1 | - | H | 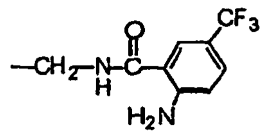 |
| 2258 | 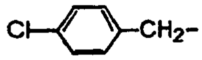 | 1 | 2 | 0 | R | H | 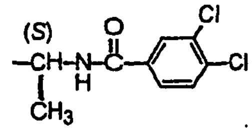 |
| 2259 | 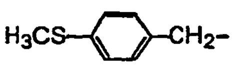 | 1 | 2 | 0 | R | H | 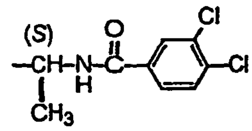 |
| 2260 | 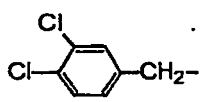 | 1 | 2 | 0 | R | H | 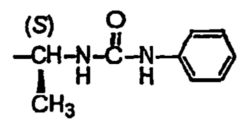 |
| 2261 | 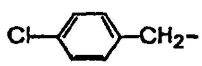 | 1 | 2 | 0 | R | H | 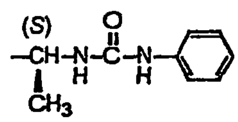 |
| 2262 | 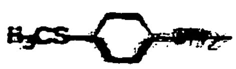 | 1 | 2 | 0 | R | H | 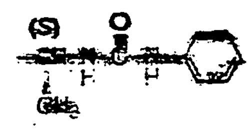 |
| 2263 | 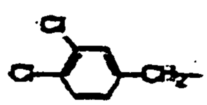 | 1 | 2 | 0 | S | R | 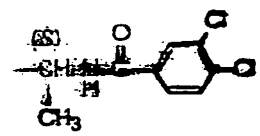 |
| 2264 | 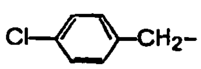 | 1 | 2 | 0 | S | H | 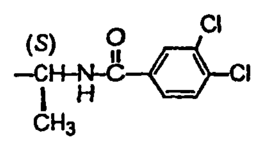 |
| 2265 | 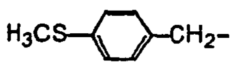 | 1 | 2 | 0 | S | H | 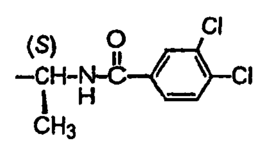 |
| 2266 | 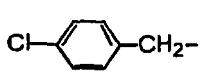 | 1 | 2 | 0 | S | H | 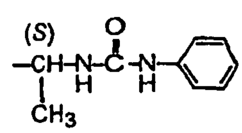 |

**Table 1.207**

| Compd. No. | 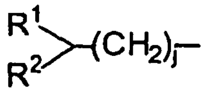 | k | m | n | chirality | $R^3$ | 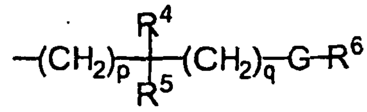 |
|---|---|---|---|---|---|---|---|
| 2267 | 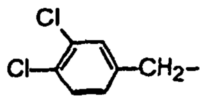 | 2 | 2 | 1 | - | H | 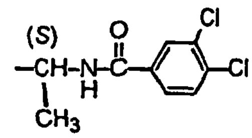 |
| 2268 | 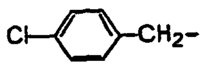 | 2 | 2 | 1 | - | H | 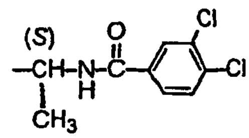 |
| 2269 | 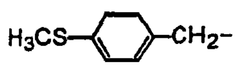 | 2 | 2 | 1 | - | H | 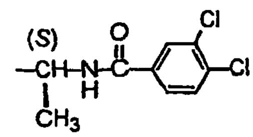 |
| 2270 | 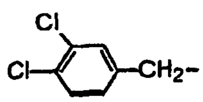 | 2 | 2 | 1 | - | H | 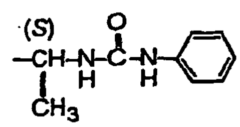 |
| 2271 | 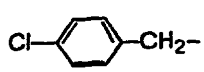 | 2 | 2 | 1 | - | H | 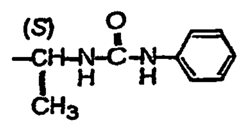 |
| 2272 | 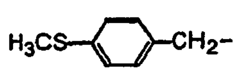 | 2 | 2 | 1 | - | H | 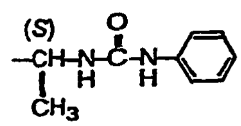 |
| 2273 | 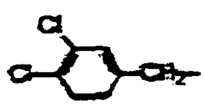 | 2 | 2 | 1 | - | H | 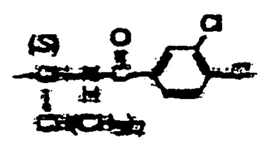 |
| 2274 | 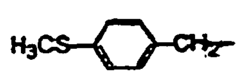 | 2 | 2 | 1 | - | H | 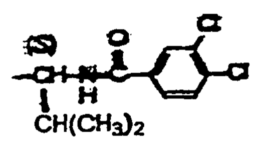 |
| 2275 | 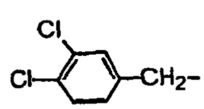 | 2 | 2 | 1 | - | H | 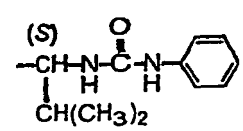 |
| 2276 | 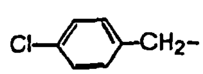 | 2 | 2 | 1 | - | H | 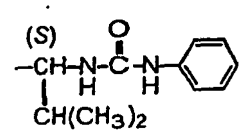 |

(continued)

| Compd. No. | 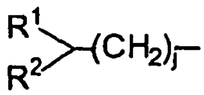 | k | m | n | chirality | $R^3$ | 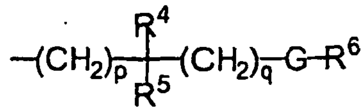 |
|---|---|---|---|---|---|---|---|
| 2277 | 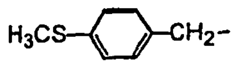 | 2 | 2 | 1 | - | H | 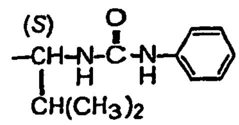 |

**Table 1.208**

| Compd. No. | 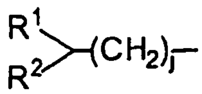 | k | m | n | chirality | $R^3$ | 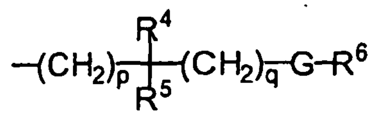 |
|---|---|---|---|---|---|---|---|
| 2278 | 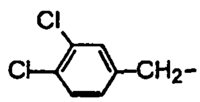 | 1 | 2 | 0 | R | H | 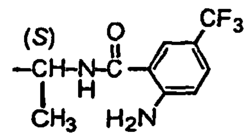 |
| 2279 | 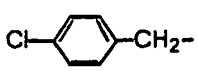 | 1 | 2 | 0 | R | H | 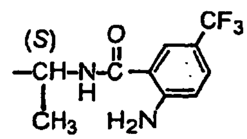 |
| 2280 | 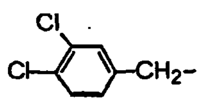 | 1 | 2 | 0 | S | H | 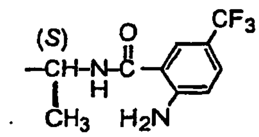 |
| 2281 | 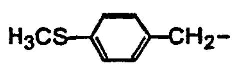 | 1 | 2 | 0 | S | H | 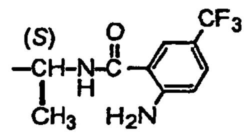 |
| 2282 | 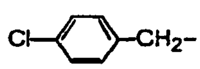 | 2 | 2 | 1 | - | H | 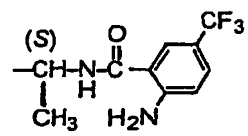 |
| 2283 | 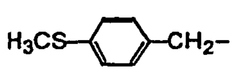 | 2 | 2 | 1 | - | H | 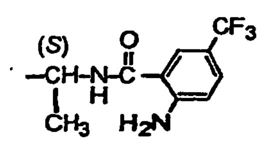 |
| 2284 | 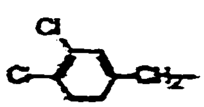 | 2 | 2 | 1 | - | H | 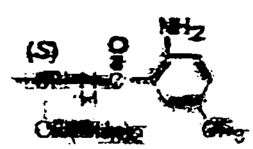 |

(continued)

| Compd. No. | 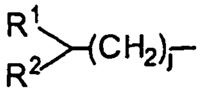 | k | m | n | chirality | $R^3$ | 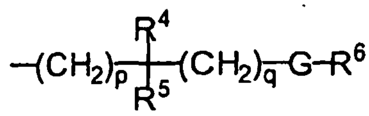 |
|---|---|---|---|---|---|---|---|
| 2285 | 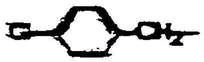 | 2 | 2 | 1 | - | H | 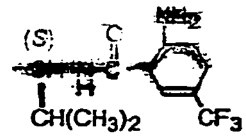 |
| 2286 | 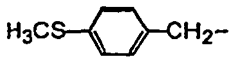 | 2 | 2 | 1 | - | H | 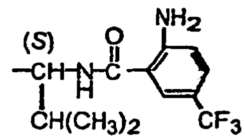 |
| 2287 | 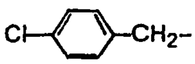 | 2 | 2 | 1 | - | H | 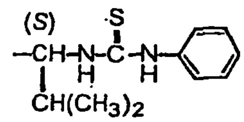 |
| 2288 | 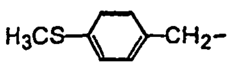 | 2 | 2 | 1 | - | H | 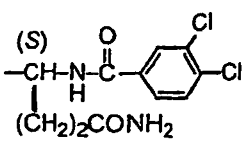 |

**Table 1.209**

| Compd. No. | 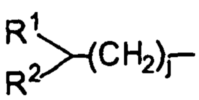 | k | m | n | chirality | $R^3$ | 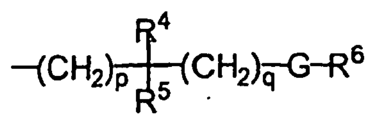 |
|---|---|---|---|---|---|---|---|
| 2289 | 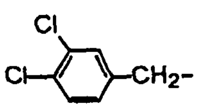 | 2 | 2 | 1 | - | H | 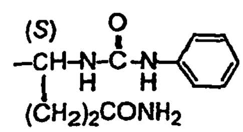 |
| 2290 | 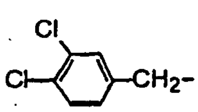 | 2 | 2 | 1 | - | H | 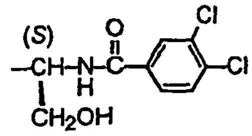 |
| 2291 | 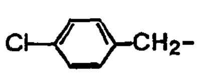 | 2 | 2 | 1 | - | H | 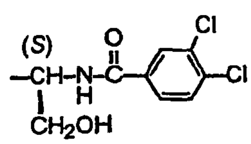 |
| 2292 | 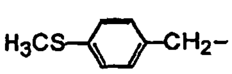 | 2 | 2 | 1 | - | H | 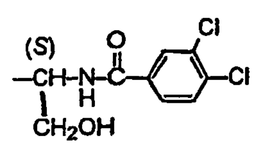 |

(continued)

| Compd. No. | 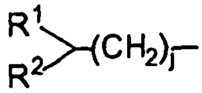 | k | m | n | chirality | $R^3$ | 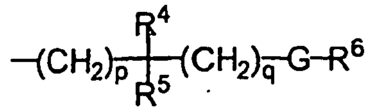 |
|---|---|---|---|---|---|---|---|
| 2293 | 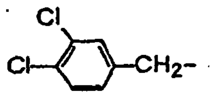 | 2 | 2 | 1 | - | H | 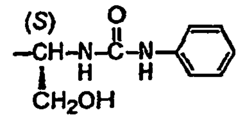 |
| 2294 | 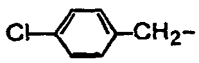 | 2 | 2 | 1 | - | H | 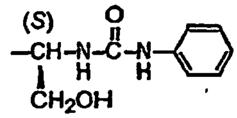 |
| 2295 | 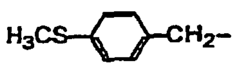 | 2 | 2 | 1 | - | H | 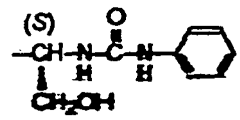 |
| 2296 | 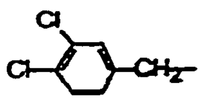 | 1 | 2 | 0 | R | H | 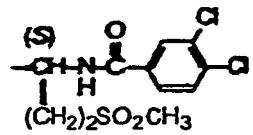 |
| 2297 | 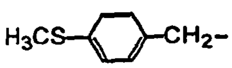 | 1 | 2 | 0 | R | H | 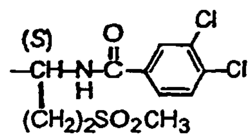 |
| 2298 | 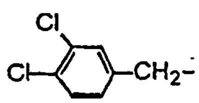 | 1 | 2 | 0 | R | H | 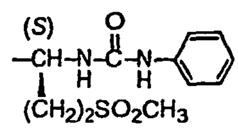 |
| 2299 | 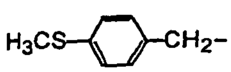 | 1 | 2 | 0 | R | H | 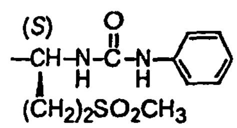 |

**Table 1.210**

| Compd. No. | 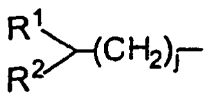 | k | m | n | chirality | $R^3$ | 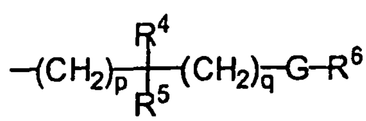 |
|---|---|---|---|---|---|---|---|
| 2300 | 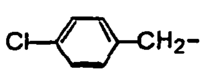 | 1 | 2 | 0 | S | H | 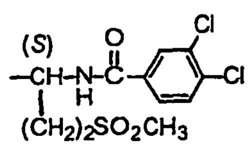 |

(continued)

| Compd. No. | 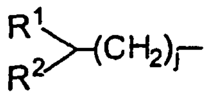 | k | m | n | chirality | $R^3$ | 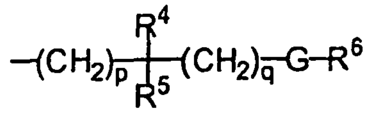 |
|---|---|---|---|---|---|---|---|
| 2301 | 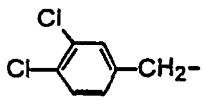 | 1 | 2 | 0 | S | H | 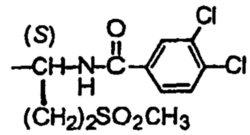 |
| 2302 | 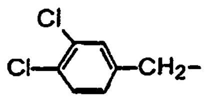 | 1 | 2 | 0 | R | H | 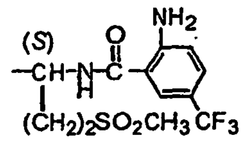 |
| 2303 | 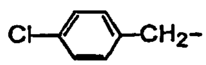 | 1 | 2 | 0 | R | H | 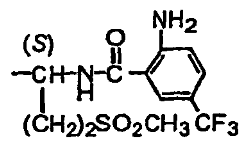 |
| 2304 | 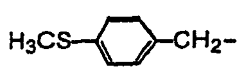 | 1 | 2 | 0 | R | H | 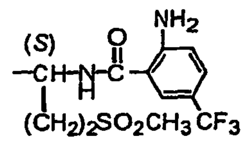 |
| 2305 | 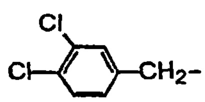 | 1 | 2 | 0 | S | H | 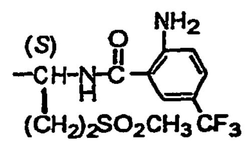 |
| 2306 | 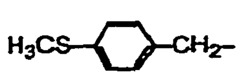 | 1 | 2 | 0 | S | H | 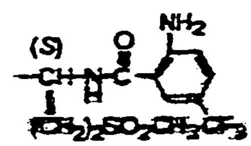 |
| 2307 | 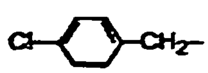 | 1 | 2 | 0 | R | H | 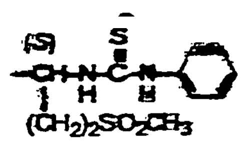 |
| 2308 | 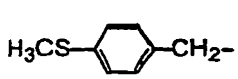 | 1 | 2 | 0 | R | H | 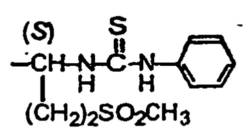 |
| 2309 | 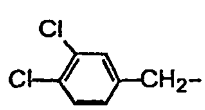 | 1 | 2 | 0 | S | H | 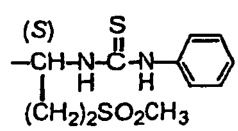 |
| 2310 | 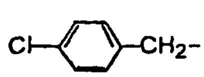 | 1 | 2 | 0 | S | H | 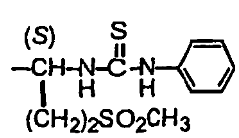 |

**Table 1.211**

| Compd. No. | 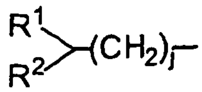 | k | m | n | chirality | $R^3$ | 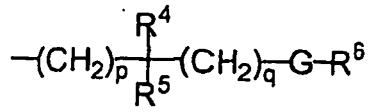 |
|---|---|---|---|---|---|---|---|
| 2311 | 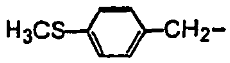 | 1 | 2 | 0 | S | H | 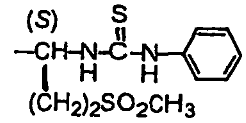 |
| 2312 | 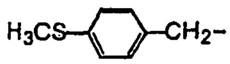 | 1 | 2 | 0 | R | H | 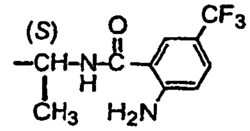 |
| 2313 | 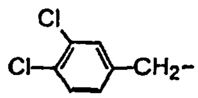 | 1 | 2 | 0 | R | H | 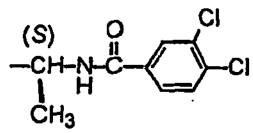 |
| 2314 | 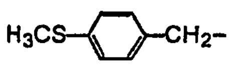 | 1 | 2 | 0 | S | H | 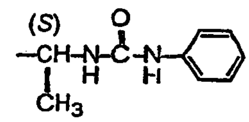 |
| 2315 | 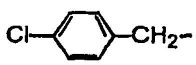 | 2 | 2 | 1 | - | H | 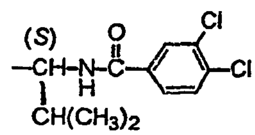 |
| 2316 | 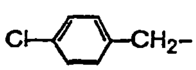 | 1 | 2 | 0 | S | H | 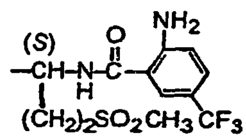 |
| 2317 | 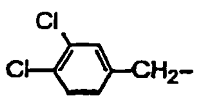 | 2 | 2 | 1 | - | H | 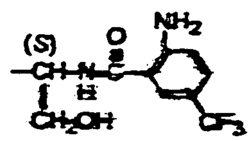 |
| 2318 | 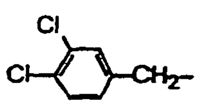 | 1 | 2 | 0 | R | H | 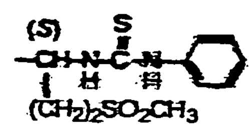 |
| 2319 | 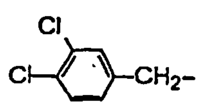 | 2 | 2 | 1 | - | H | 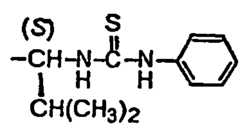 |

(continued)

| Compd. No. | 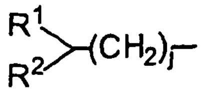 | k | m | n | chirality | $R^3$ | 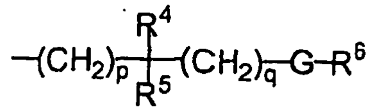 |
|---|---|---|---|---|---|---|---|
| 2320 | 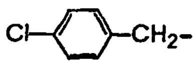 | 2 | 2 | 1 | - | H | 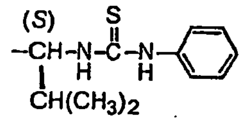 |
| 2321 | 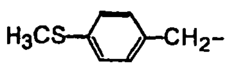 | 2 | 2 | 1 | - | H | 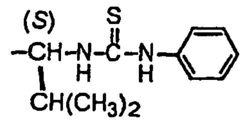 |

**Table 1.212**

| Compd. No. | 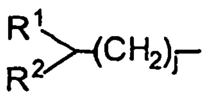 | k | m | n | chirality | $R^3$ | 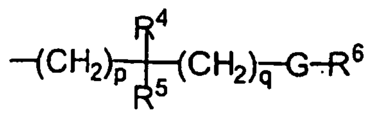 |
|---|---|---|---|---|---|---|---|
| 2322 | 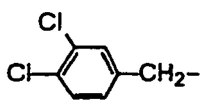 | 2 | 2 | 1 | - | H | 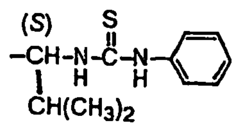 |
| 2323 | 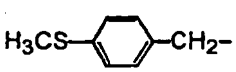 | 2 | 2 | 1 | - | H | 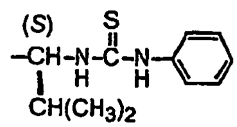 |
| 2324 | 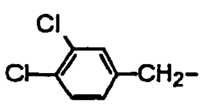 | 2 | 2 | 1 | - | H | 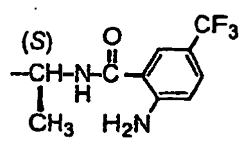 |
| 2325 | 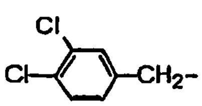 | 1 | 2 | 0 | R | H | 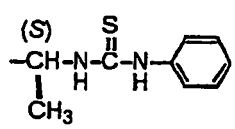 |
| 2326 | 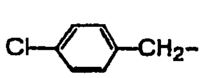 | 1 | 2 | 0 | R | H | 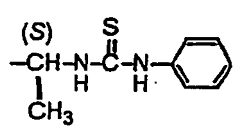 |
| 2327 | 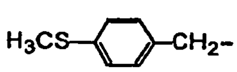 | 1 | 2 | 0 | R | H | 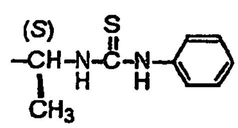 |

(continued)

| Compd. No. | 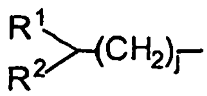 | k | m | n | chirality | $R^3$ | 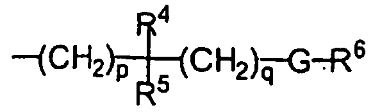 |
|---|---|---|---|---|---|---|---|
| 2328 | 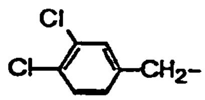 | 1 | 2 | 0 | S | H | 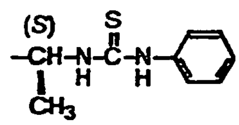 |
| 2329 | 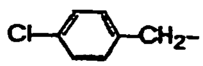 | 1 | 2 | 0 | S | H | 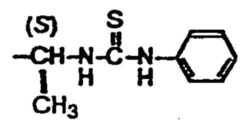 |
| 2330 | 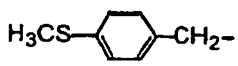 | 1 | 2 | 0 | S | H | 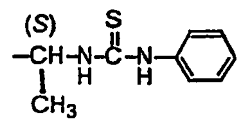 |
| 2331 | 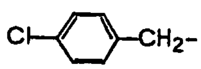 | 1 | 2 | 0 | S | H | 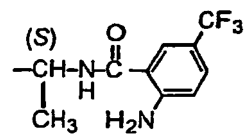 |
| 2332 | 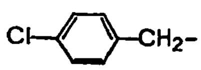 | 1 | 2 | 0 | R | H | 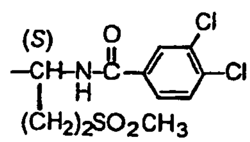 |

**Table 1.213**

| Compd. No. | 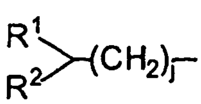 | k | m | n | chirality | $R^3$ | 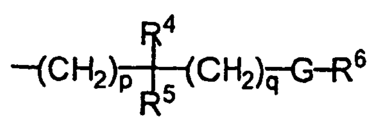 |
|---|---|---|---|---|---|---|---|
| 2333 | 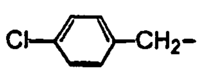 | 1 | 2 | 0 | R | H | 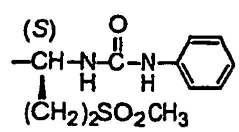 |
| 2334 | 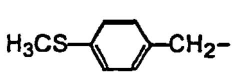 | 1 | 2 | 0 | S | H | 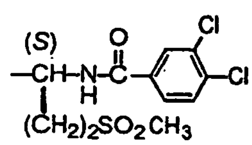 |
| 2335 | 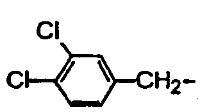 | 1 | 2 | 0 | S | H | 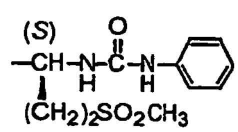 |

(continued)

| Compd. No. | 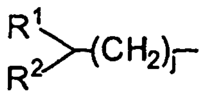 | k | m | n | chirality | $R^3$ | 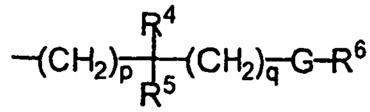 |
|---|---|---|---|---|---|---|---|
| 2336 | 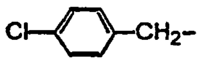 | 1 | 2 | 0 | S | H | 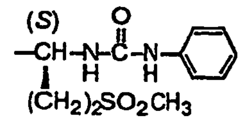 |
| 2337 | 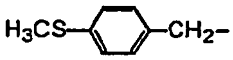 | 1 | 2 | 0 | S | H | 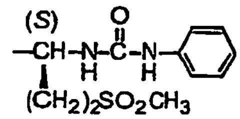 |
| 2338 | 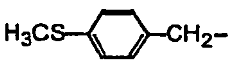 | 2 | 2 | 1 | - | H | 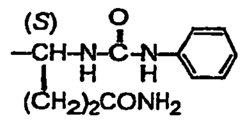 |
| 2339 | 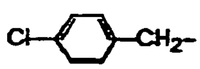 | 2 | 2 | 1 | - | H | 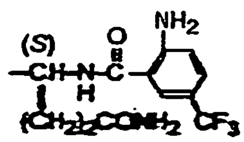 |
| 2340 | 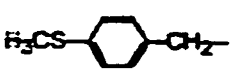 | 2 | 2 | 1 | - | H | 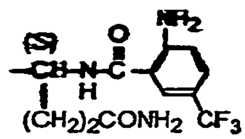 |
| 2341 | 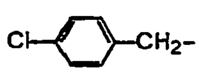 | 2 | 2 | 1 | - | H | 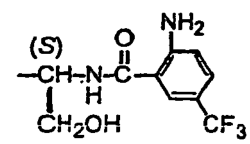 |
| 2342 | 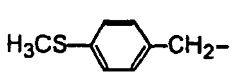 | 2 | 2 | 1 | - | H | 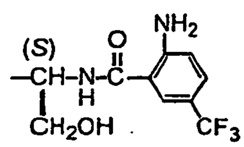 |
| 2343 | 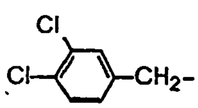 | 2 | 2 | 1 | - | H | 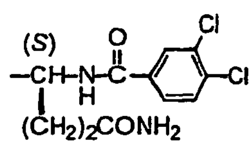 |

**Table 1.214**

| Compd. No. | 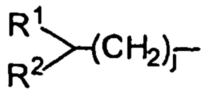 | k | m | n | chirality | $R^3$ | 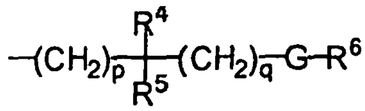 |
|---|---|---|---|---|---|---|---|
| 2344 | 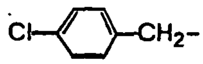 | 2 | 2 | 1 | - | H | 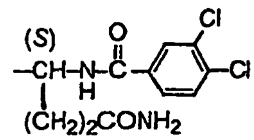 |
| 2345 | 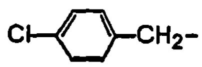 | 2 | 2 | 1 | - | H | 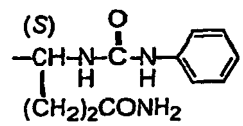 |
| 2346 | 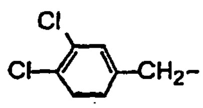 | 2 | 2 | 1 | - | H | 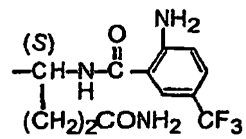 |
| 2347 | 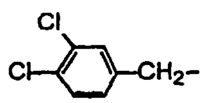 | 1 | 2 | 0 | S | H | 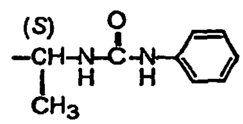 |
| 2348 | 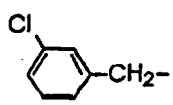 | 1 | 2 | 0 | R | H | 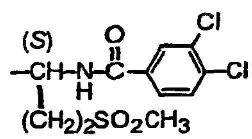 |
| 2349 | 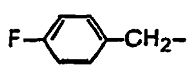 | 1 | 2 | 0 | R | H | 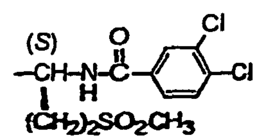 |
| 2350 | 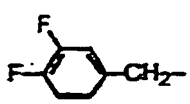 | 1 | 2 | 0 | R | H | 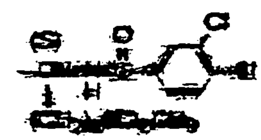 |
| 2351 | 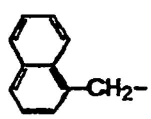 | 1 | 2 | 0 | R | H | 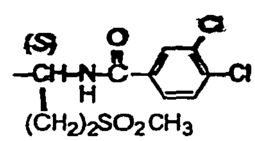 |
| 2352 | 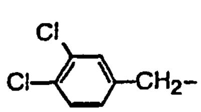 | 2 | 2 | 1 | - | H | 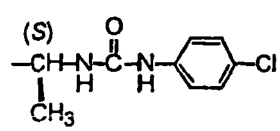 |
| 2353 | 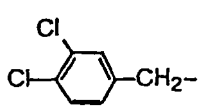 | 2 | 2 | 1 | - | H | 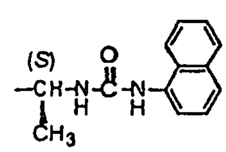 |

(continued)

| Compd. No. | 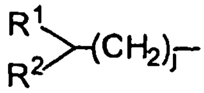 | k | m | n | chirality | $R^3$ | 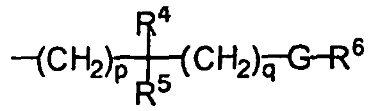 |
|---|---|---|---|---|---|---|---|
| 2354 | 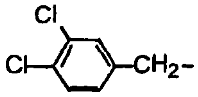 | 1 | 2 | 0 | R | H | 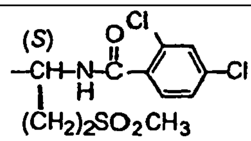 |

**Table 1.215**

| Compd. No. | 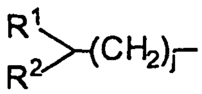 | k | m | n | chirality | $R^3$ | 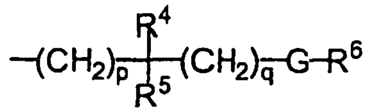 |
|---|---|---|---|---|---|---|---|
| 2355 | 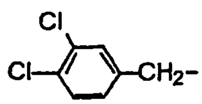 | 1 | 2 | 0 | R | H | 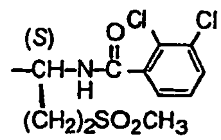 |
| 2356 | 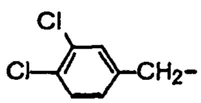 | 1 | 2 | 0 | R | H | 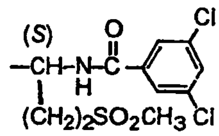 |
| 2357 | 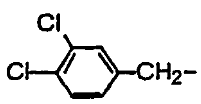 | 1 | 2 | 0 | R | H | 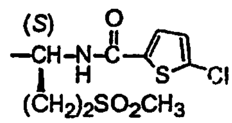 |
| 2358 | 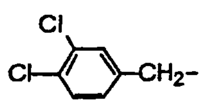 | 1 | 2 | 0 | R | H | 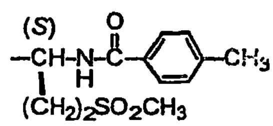 |
| 2359 | 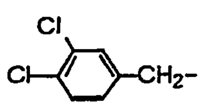 | 1 | 2 | 0 | R | H | 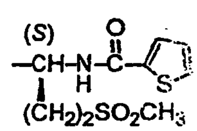 |
| 2360 | 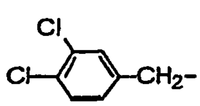 | 1 | 2 | 0 | R | H | 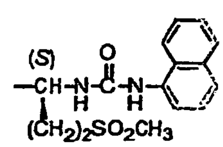 |
| 2361 | 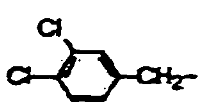 | 1 | 2 | 0 | R | H | 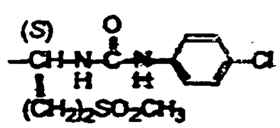 |

(continued)

| Compd. No. | 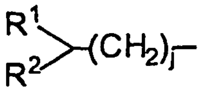 | k | m | n | chirality | $R^3$ | 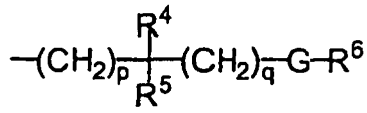 |
|---|---|---|---|---|---|---|---|
| 2362 | 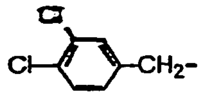 | 1 | 2 | 0 | R | H | 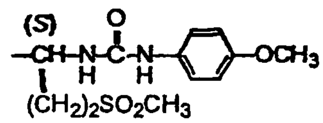 |
| 2363 | 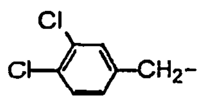 | 2 | 2 | 1 | - | H | 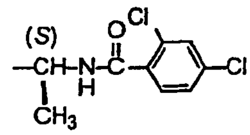 |
| 2364 | 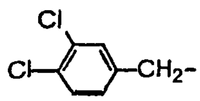 | 2 | 2 | 1 | - | H | 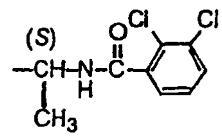 |
| 2365 | 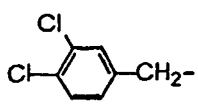 | 2 | 2 | 1 | - | H | 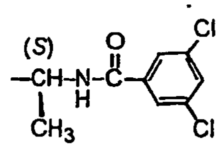 |

**Table 1.216**

| Compd. No. | 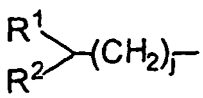 | k | m | n | chirality | $R^3$ | 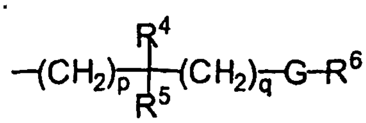 |
|---|---|---|---|---|---|---|---|
| 2366 | 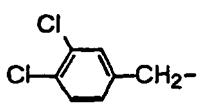 | 2 | 2 | 1 | - | H | 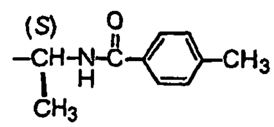 |
| 2367 | 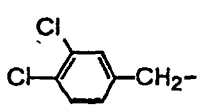 | 2 | 2 | 1 | - | H | 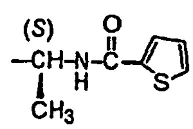 |
| 2368 | 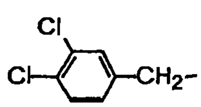 | 2 | 2 | 1 | - | H | 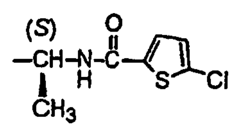 |
| 2369 | 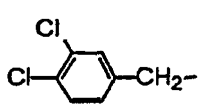 | 2 | 2 | 1 | - | H | 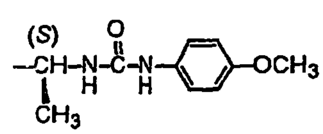 |

(continued)

| Compd. No. | 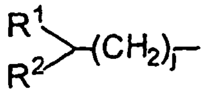 | k | m | n | chirality | $R^3$ | 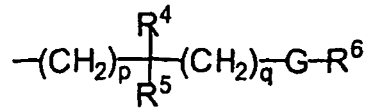 |
|---|---|---|---|---|---|---|---|
| 2370 | 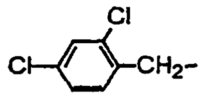 | 2 | 2 | 1 | - | H | 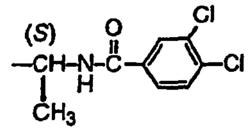 |
| 2371 | 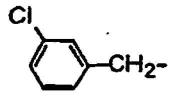 | 2 | 2 | 1 | - | H | 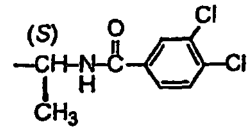 |
| 2372 | 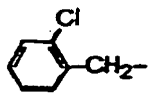 | 2 | 2 | 1 | - | H | 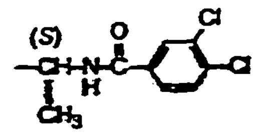 |
| 2373 | 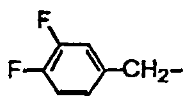 | 2 | 2 | 1 | - | H | 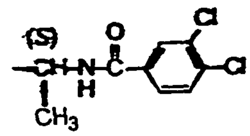 |
| 2374 | 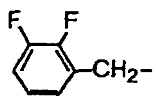 | 2 | 2 | 1 | - | H | 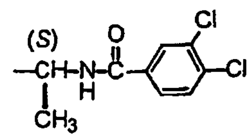 |
| 2375 | 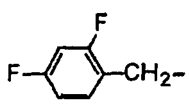 | 2 | 2 | 1 | - | H | 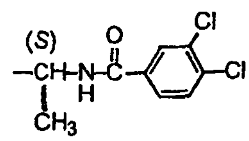 |
| 2376 | 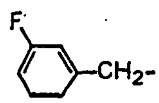 | 2 | 2 | 1 | - | H | 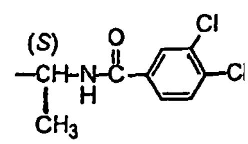 |

**Table 1.217**

| Compd. No. | 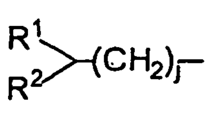 | k | m | n | chirality | $R^3$ | 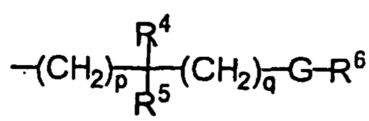 |
|---|---|---|---|---|---|---|---|
| 2377 | 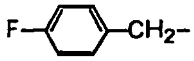 | 2 | 2 | 1 | - | H | 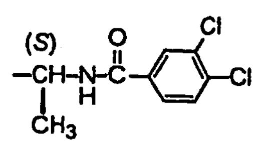 |

(continued)

| Compd. No. | 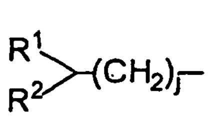 | k | m | n | chirality | $R^3$ | 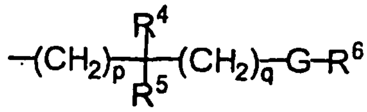 |
|---|---|---|---|---|---|---|---|
| 2378 | 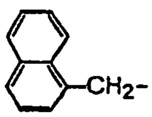 | 2 | 2 | 1 | - | H | 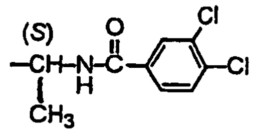 |
| 2379 | 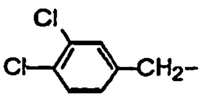 | 2 | 2 | 1 | - | H | 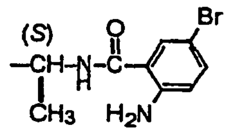 |
| 2380 | 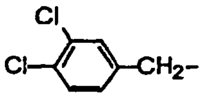 | 2 | 2 | 1 | - | H | 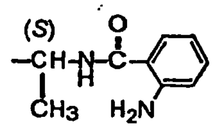 |
| 2381 | 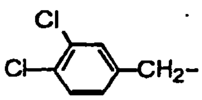 | 2 | 2 | 1 | - | H | 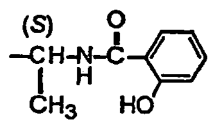 |
| 2382 | 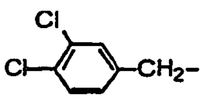 | 2 | 2 | 1 | - | H | 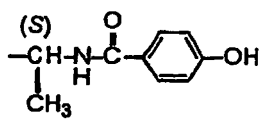 |
| 2383 | 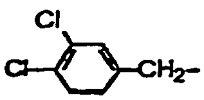 | 2 | 2 | 1 | - | H | 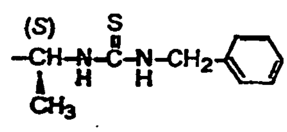 |
| 2384 | 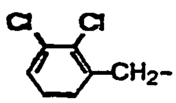 | 1 | 2 | 0 | R | H | 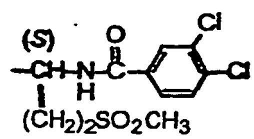 |
| 2385 | 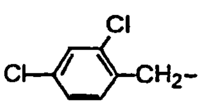 | 1 | 2 | 0 | R | H | 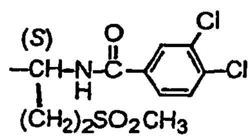 |
| 2386 | 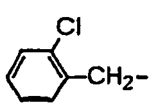 | 1 | 2 | 0 | R | H | 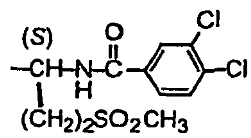 |
| 2387 | 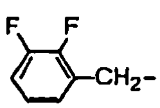 | 1 | 2 | 0 | R | H | 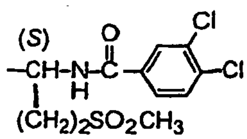 |

**Table 1.218**

| Compd. No. | 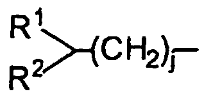 | k | m | n | chirality | $R^3$ | 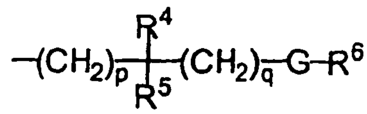 |
|---|---|---|---|---|---|---|---|
| 2388 | 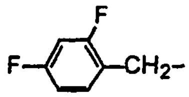 | 1 | 2 | 0 | R | H | 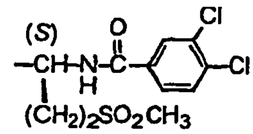 |
| 2389 | 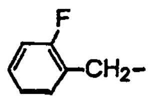 | 1 | 2 | 0 | R | H | 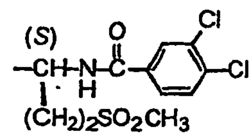 |
| 2390 | 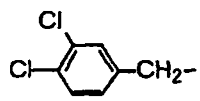 | 1 | 2 | 0 | R | H | 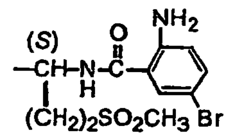 |
| 2391 | 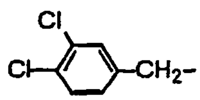 | 1 | 2 | 0 | R | H | 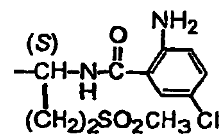 |
| 2392 | 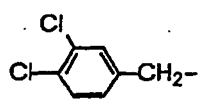 | 1 | 2 | 0 | R | H | 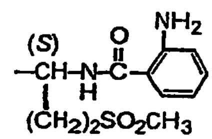 |
| 2393 | 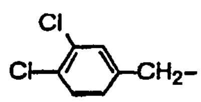 | 1 | 2 | 0 | R | H | 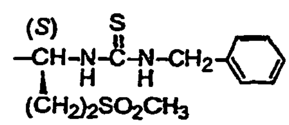 |
| 2394 | 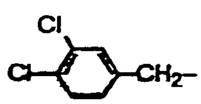 | 2 | 2 | 1 | - | H | 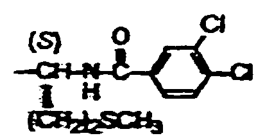 |
| 2395 | 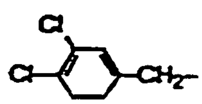 | 2 | 2 | 1 | - | H | 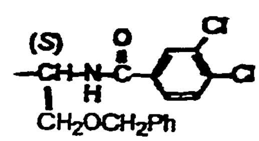 |
| 2396 | 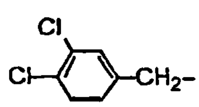 | 2 | 2 | 1 | - | H | 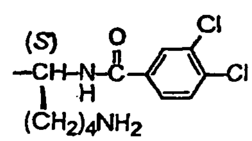 |
| 2397 | 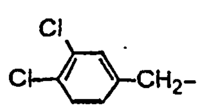 | 2 | 2 | 1 | - | H | 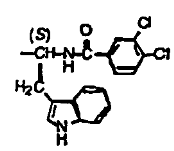 |

(continued)

| Compd. No. | 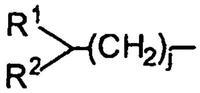 | k | m | n | chirality | $R^3$ | 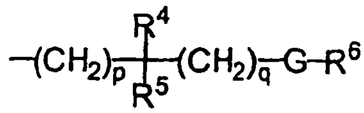 |
|---|---|---|---|---|---|---|---|
| 2398 | 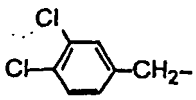 | 2 | 2 | 1 | - | H | 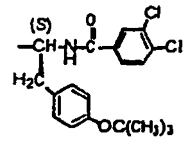 |

**Table 1.219**

| Compd. No. | 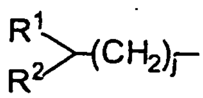 | k | m | n | chirality | $R^3$ | 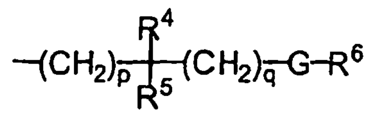 |
|---|---|---|---|---|---|---|---|
| 2399 | 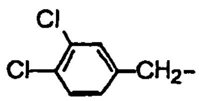 | 2 | 2 | 1 | - | H | 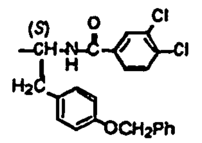 |
| 2400 | 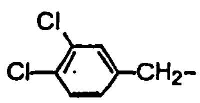 | 2 | 2 | 1 | - | H | 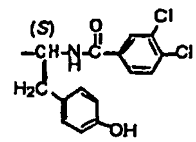 |
| 2401 | 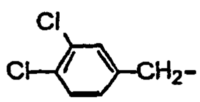 | 2 | 2 | 1 | - | H | 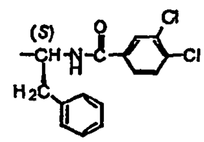 |
| 2402 | 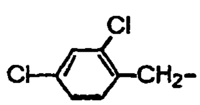 | 2 | 2 | 1 | - | H | 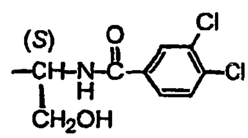 |
| 2403 | 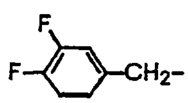 | 2 | 2 | 1 | - | H | 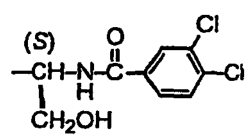 |
| 2404 | 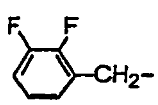 | 2 | 2 | 1 | - | H | 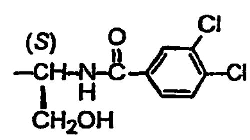 |
| 2405 | 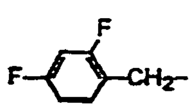 | 2 | 2 | 1 | - | H | 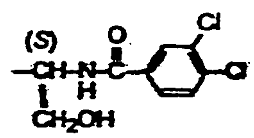 |

(continued)

| Compd. No. | 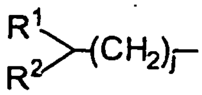 | k | m | n | chirality | $R^3$ | 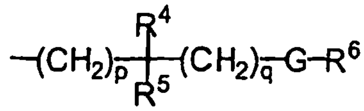 |
|---|---|---|---|---|---|---|---|
| 2406 | 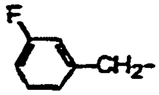 | 2 | 2 | 1 | - | H | 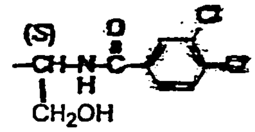 |
| 2407 | 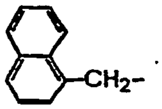 | 2 | 2 | 1 | - | H | 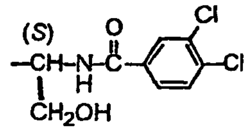 |
| 2408 | 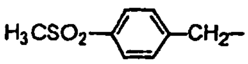 | 2 | 2 | 1 | - | H | 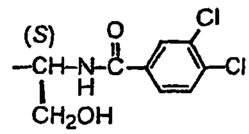 |
| 2409 | 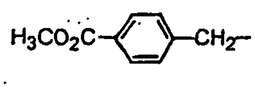 | 2 | 2 | 1 | - | H | 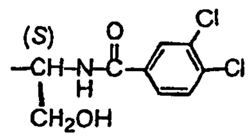 |

**Table 1.220**

| Compd. No. | 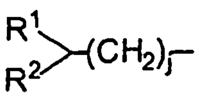 | k | m | n | chirality | $R^3$ | 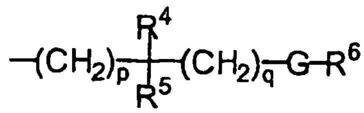 |
|---|---|---|---|---|---|---|---|
| 2410 | 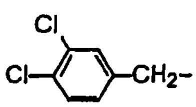 | 2 | 2 | 1 | - | H | 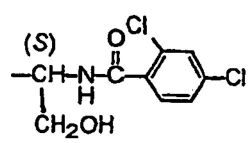 |
| 2411 | 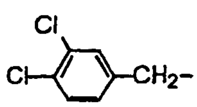 | 2 | 2 | 1 | - | H | 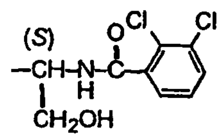 |
| 2412 | 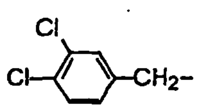 | 2 | 2 | 1 | - | H | 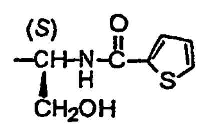 |
| 2413 | 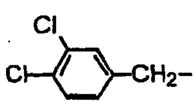 | 2 | 2 | 1 | - | H | 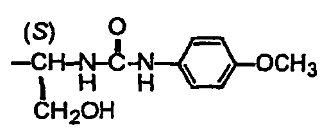 |

(continued)

| Compd. No. | 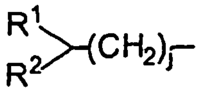 | k | m | n | chirality | $R^3$ | 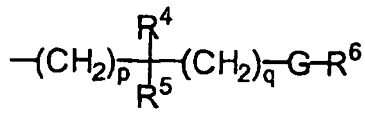 |
|---|---|---|---|---|---|---|---|
| 2414 | 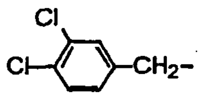 | 2 | 2 | 1 | - | H | 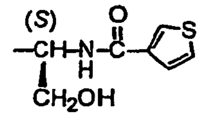 |
| 2415 | 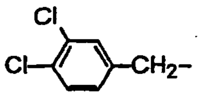 | 2 | 2 | 1 | - | H | 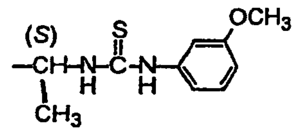 |
| 2416 | 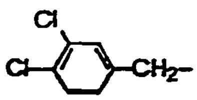 | 2 | 2 | 1 | - | H | 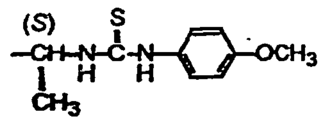 |
| 2417 | 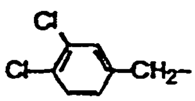 | 2 | 2 | I | - | H | 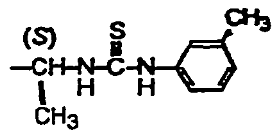 |
| 2418 | 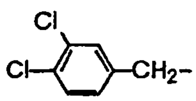 | 2 | 2 | 1 | - | H | 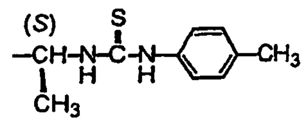 |
| 2419 | 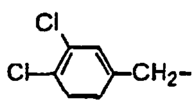 | 2 | 2 | 1 | - | H | 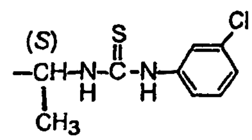 |
| 2420 | 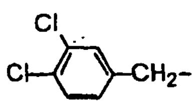 | 2 | 2 | 1 | - | H | 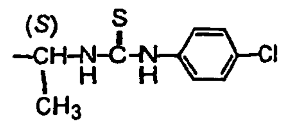 |

**Table 1.221**

| Compd. No. | 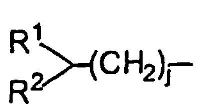 | k | m | n | chirality | $R^3$ | 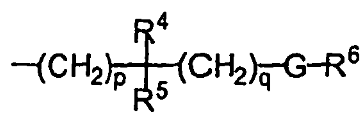 |
|---|---|---|---|---|---|---|---|
| 2421 | 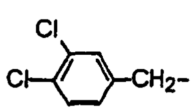 | 2 | 2 | 1 | - | H | 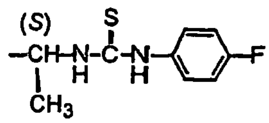 |

(continued)

| Compd. No. | 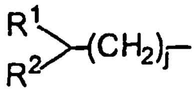 | k | m | n | chirality | $R^3$ | 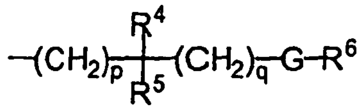 |
|---|---|---|---|---|---|---|---|
| 2422 | 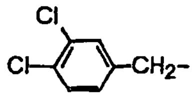 | 1 | 2 | 0 | R | H | 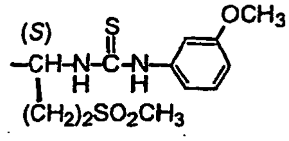 |
| 2423 | 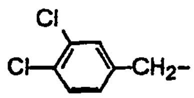 | 1 | 2 | 0 | R | H | 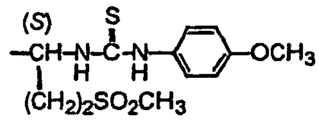 |
| 2424 | 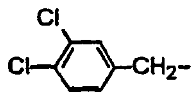 | 1 | 2 | 0 | R | H | 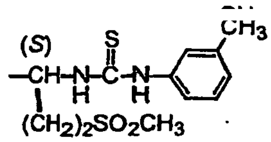 |
| 2425 | 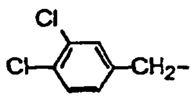 | 1 | 2 | 0 | R | H | 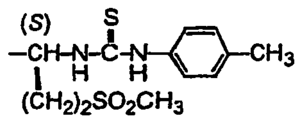 |
| 2426 | 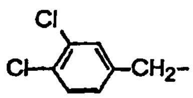 | 1 | 2 | 0 | R | H | 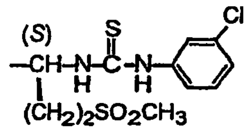 |
| 2427 | 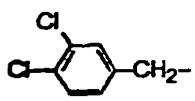 | 1 | 2 | 0 | R | H | 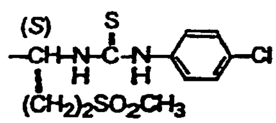 |
| 2428 | 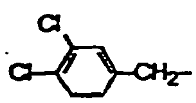 | 1 | 2 | 0 | R | H | 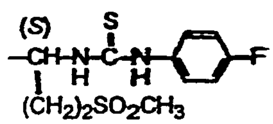 |

The acid addition salt of the cyclic amine compound is also used in the present invention. Examples of the acid include a mineral acid such as hydrochloric acid, hydrobromic acid, sulfuric acid, phosphoric acid or carbonic acid and an organic acid such as maleic acid, citric acid, malic acid, tartaric acid, fumaric acid, methanesulfonic acid, trifluoroacetic acid or formic acid.

Furthermore, $C_1$-$C_6$ alkyl addition salt of the cyclic amine compound, for example, 1-(4-chlorobenzyl)-1-methyl-4-[{N-(3-trifluoromethylbenzoyl)glycyl} aminomethyl]piperidinium iodide is also used in the present invention. The alkyl group preferably includes methyl, ethyl, n-propyl, n-butyl, n-pentyl, n-hexyl, n-heptyl, n-octyl, isopropyl, isobutyl, sec-butyl, tert-butyl, isopentyl, neopentyl, tert-pentyl, 2-methylpentyl and 1-ethylbutyl and the like herein; however, methyl group, ethyl group or the like is especially preferable.

A halide anion such as fluoride, chloride, bromide or iodide is preferable for a counter anion of an ammonium cation.

In the present invention, a racemate and all the possible optically active forms of the compound represented by the above formula (I) can also be used.

The compounds represented by the above formula (I) can be synthesized by using any of the following general preparation methods as described in WO9925686:

(Preparation method 1)

A preparation method comprises reacting one equivalent of a compound represented by the following formula (II):

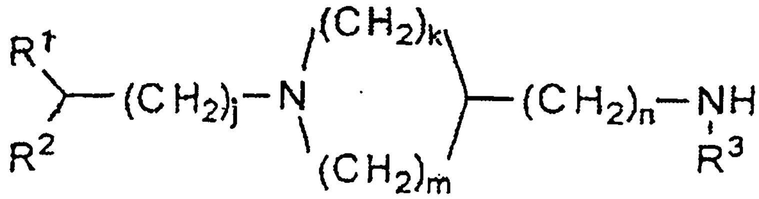

(II)

wherein, $R^1$, $R^2$, $R^3$, j, k, m and n are each the same as defined in the above formula (I), with 0.1 to 10 equivalents of a carboxylic acid represented by the following formula (III):

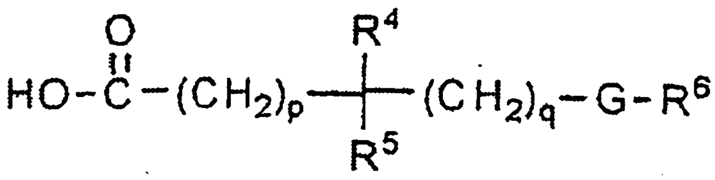

(III)

wherein, $R^4$, $R^5$, $R^6$, G, p and q are each the same as defined in the above formula (I), or a reactive derivative thereof in the absence or presence of a solvent.

The "reactive derivative" of the carboxylic acid represented by the above formula (III) means a carboxylic acid derivative, for example, an acid halide, an acid anhydride or a mixed acid anhydride usually used in the synthetic organic chemistry field and having high reactivity.

The reaction can more smoothly be made to proceed by suitably using an adequate amount of a dehydrating agent such as molecular sieve; a coupling reagent such as dicyclohexylcarbodiimide (DCC), N-ethyl-N'-(3-dimethylaminopropyl)carbodiimide (EDCI or WSC), carbonyldiimidazole (CDI), N-hydroxysuccinimide (HOSu), N-hydroxybenzotriazole (HOBt), benzotriazol-1-yloxytris(pyrrolidino)phosphonium hexafluorophosphate (PyBOP), 2-(1H-benzotriazol-1-yl)-1,1,3,3-tetramethyluronium hexafluorophosphate (HBTU), 2-(1H-benzotriazol-1-yl)-1,1,3,3-tetramethyluronium tetrafluoroborate (TBTU), 2-(5-norbornene-2,3-dicarboxyimide)-1,1,3,3-tetramethyluronium tetrafluoroborate (TNTU), O-(N-succinimidyl)-1,1,3,3-tetramethyluronium tetrafluoroborate (TSTU) or bromotris(pyrrolidino)phosphonium hexafluorophosphate (PyBroP); a base such as an inorganic base such as potassium carbonate, calcium carbonate or sodium hydrogencarbonate; amines such as triethylamine, diisoproylethylamine or pyridine or a polymer supported base such as (piperidinomethyl)polystyrene, (morphohnomethyl)polystyrene, (dimethylaminomethyl)polystyrene or poly(4-vinylpyridine).

(Preparation method 2)

A preparation method comprises reacting one equivalent of an alkylating reagent represented by the following formula (IV):

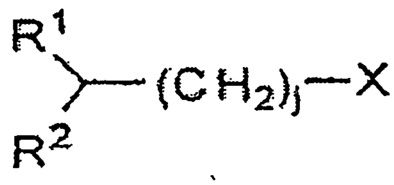

(IV)

wherein, $R^1$, $R^2$ and j are each the same as defined in the above formula (I); X is a halogen atom, an alkylsulfonyloxy group or an arylsulfonyloxy group, with 0.1 to 10 equivalents of a compound represented by the following formula (V):

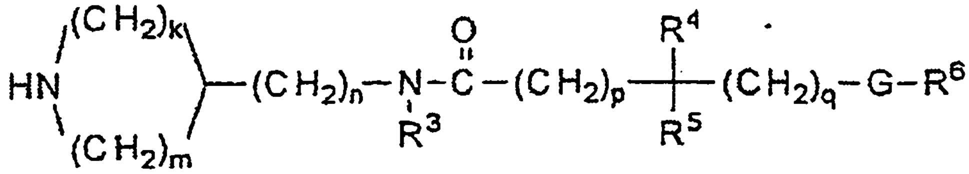

(V)

wherein, $R^3$, $R^4$, $R^5$, $R^6$, G, k, m, n, p and q are each the same as defined in the above formula (I), in the absence or presence of a solvent.

The reaction can more smoothly be made to proceed by suitably using a base similar to that in the preparation method 1. Furthermore, the reaction sometimes can be promoted by the presence of an iodide such as potassium iodide or sodium iodide.

In the above formula (IV), X is a halogen atom, an alkylsulfonyloxy group or an arylsulfonyloxy group. Examples of the halogen atom preferably include a chlorine atom, a bromine atom and an iodine atom. Specific examples of the alkylsulfonyloxy group preferably include a methylsulfonyloxy group, a trifluoromethylsulfonyloxy group and the like, and the specific example of the arylsulfonyloxy group preferably includes tosyloxy group.

(Preparation method 3)

A preparation method comprises reacting one equivalent of an aldehyde represented by the following formula (VI):

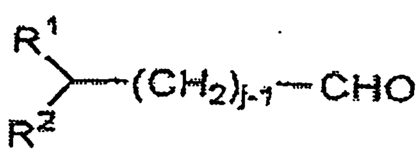

(VI)

wherein, $R^1$ and $R^2$ are each the same as defined in the above formula (I); j is 1 or 2, or an aldehyde represented by the following formula (VII):

$R^1$- CHO (VII)

wherein, $R^1$ is the same as defined for $R^1$ in the above formula (I); the compound corresponds to the case where j is 0, with 0.1 to 10 equivalents of a compound represented by the above formula (V) in the absence or presence of a solvent.

The reaction is usually called a reductive amination reaction and a catalytic hydrogenation reaction using a catalyst containing a metal such as palladium, platinum, nickel or rhodium, a hydrogenation reaction using a complex hydride such as lithium aluminum hydride, sodium borohydride, sodium cyanoborohydride or sodium triacetoxyborohydride and borane, an electrolytic reduction or the like can be used as reductive conditions.

(Preparation method 4)

A preparation method comprises reacting one equivalent of a compound represented by the following formula (VIII):

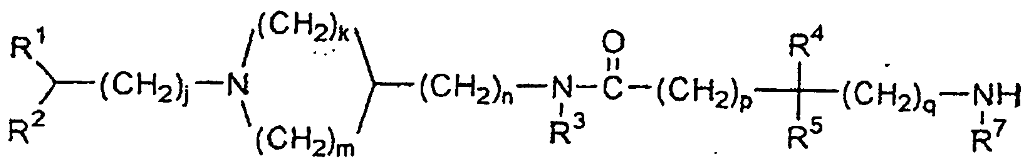

(VIII)

wherein, $R^1$, $R^2$, $R^3$, $R^4$, $R^5$, $R^7$, j, k, m, n, p and q are each the same as defined in the above formula (I), with 0.1 to 10 equivalents of a carboxylic acid or a sulfonic acid represented by the following formula (IX):

$HO\text{-}A\text{-}R^6$ (IX)

wherein, $R^6$ is the same as $R^6$ defined in the above formula (I); A is a carbonyl group or a sulfonyl group, or a reactive derivative thereof in the absence or presence of a solvent.

The reactive derivative of the carboxylic acid or sulfonic acid represented by the above formula (IX) means a carboxylic acid derivative or sulfonic acid derivative, for example, an acid halide, an acid anhydride or a mixed acid anhydride usually used in the synthetic organic chemistry field and having high reactivity.

The reaction can more smoothly be made to proceed by suitably using a dehydrating agent, a coupling reagent or a base similar to that in the above preparation method 1.

(Preparation method 5)

A preparation method comprises reacting one equivalent of a compound represented by the above formula (VIII) with 0.1 to 10 equivalents of an isocyanate or an isothiocyanate represented by the following formula (X):

$Z{=}C{=}N\text{-}R^6$ (X)

wherein, $R^6$ is the same as defined in the above formula (I); Z is an oxygen atom or a sulfur atom, in the absence or presence of a solvent.

(Preparation method 6)

A preparation method comprises reacting one equivalent of a compound represented by the following formula (XI):

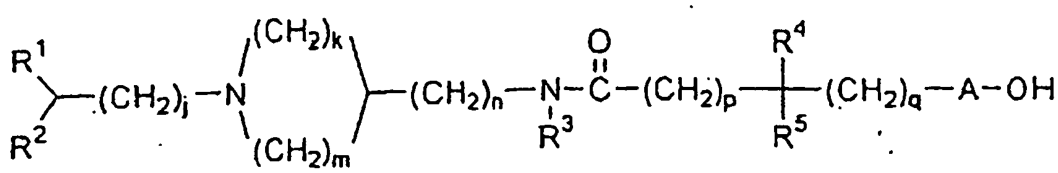

(XI)

wherein, $R^1$, $R^2$, $R^3$, $R^4$, $R^5$, j, k, m, n, p and q are each the same as defined in the above formula (I); A is a carbonyl group or a sulfonyl group, with 0.1 to 10 equivalents of an amine represented by the following formula (XII):

$R^6\text{-}NH_2$ (XII)

wherein, $R^6$ is the same as defined for $R^6$ in the above formula (I), in the absence or presence of a solvent.

The reaction can more smoothly be made to proceed by suitably using a dehydrating agent, a coupling reagent or a base similar to that in the above preparation method 1.

In the above preparation methods 1 to 6, when a substrate used for each reaction has substituents regarded as usually reacting under respective reaction conditions in the organic synthetic chemistry or having adverse effects an the reaction, the functional groups can be protected with a known suitable protecting group, and the substrate can be used for the reaction and there deprotected by a conventional known method to afford the objective compound

In addition, the compounds used in the present invention can be obtained by further converting single or plural substituents of the compound produced by the above preparation methods 1 to 6 using a known reaction usually used in the organic synthetic chemistry, for example, an alkylation reaction, an acylation reaction or a reduction reaction.

In the above respective preparation methods, a halogenated hydrocarbon such as dichloromethane or chloroform, an aromatic hydrocarbon such as benzene or toluene, ethers such as diethyl ether or tetrahydrofuran, esters such as ethyl acetate, an aprotic polar solvent such as dimethylformamide, dimethyl sulfoxide or acetonitrile and alcohols such as methanol, ethanol or isopropyl alcohol are suitably used as a reaction solvent according to the reaction.

In each of the preparation methods, the reaction temperature is within the range of-78 to +150 ˚C, preferably within the range of 0 to 100˚C. After completing the reaction, the objective cyclic amine compound represented by the above formula (I) can be isolated by carrying out usual isolating and purifying operations, i.e., concentration, filtration, extraction, solid-phase extraction, recrystallization or chromatography. The compound can be converted into their pharmaceutically acceptable acid addition salt thereof or their $C_1$-$C_6$ alkyl addition salt thereof according to a usual method.

The specific diseases which the medicament used in the present invention is used to treat are diseases associated with CCR5 selected from the group consisting of rejection after organ transplantation, graft-versus-host diseases (GVHD) and diabetes.

Examples

The present invention will be detailed specifically based on Examples; however, the present invention is not restricted to the Examples. The Compound number (Compd. No.) assigned to each compound in the following Examples corresponds to the Compd. No. assigned to each compound cited as a preferred specific example in Tables 1.1 to 1.221 for use is the invention.

[Reference Example 1] <u>Synthesis of (R)-1-(4-chlorobenzyl)-3[{N-(3,4-difluorobenzoyl)glycyl}mino]pyrrolidine (Compd. No. 69)</u>

The compounds used in the present invention were synthesized according to the preparation method described in WO9925686. For example (R)-1-(4-chlorobenzyl)-3-[{N-(3-(trifluoromethylthio)benzoyl)glycyl}amino]pyrrolidine, which was Compd. No. 1606, was synthesized as follows:

1) <u>3-Amino-1-(4-chlorobenzyl)pyrrolidine dihydrochloride</u>

4-Chlorobenzyl chloride (4.15 g, 25.8 mmol) and $^iPr_2NEt$ (6.67 g, 51.6 mmol) were added to a DMF (50 mL) solution of 3-{(tert-butoxycarbonyl)amino}pyrrolidine (4.81 g, 25.8 mmol). The reaction mixture was stirred at 70 ˚C for 15 hours, and the solvent was removed under reduced pressure. The objective 3-{(tert-butoxycarbonyl)amino}-1-(4-chlorobenzyl)pyrrolidine (6.43 g, 80%) was obtained as an off-white solid by recrystallization ($CH_3CN$, 50 mL).
$^1H$ NMR ($CDCl_3$, 300MHz) δ 1.37 (s, 9 H), 1.5-1.7 (br, 1H), 2.1-2.4 (m, 2 H), 2.5-2.7 (m, 2 H), 2.83 (br, 1H), 3.57 (s, 2 H), 4.1-4.3 (br, 1H), 4.9-5.1 (br, 1H), 7.15-7.35 (br, 4 H);
The purity was determined by RPLC/MS (98%). ESI/MS m/e 311.0 ($M^+$+H, $C_{16}H_{24}ClN_2O_2$).

To a $CH_3OH$ (80 mL) solution of the 3-{(tert-butoxycarbonyl)amino}-1-(4-chlorobenzyl)pyrrolidine (6.38 g, 20.5 mmol), was added 1- M HCl-$Et_2O$ (100 mL). The resulting mixture was stirred at 25˚C for 15 hours. The solvent was removed under reduced pressure to provide a solid, which was purified by recrystallization ($CH_3OH$:$CH_3CN$ = 1:2, 130 mL) to afford 3-amino-1-(4-chlorobenzyl)pyrrolidine dihydrochloride (4.939 g, 85%) as a white powder.
$^1H$ NMR ($d_6$-DMSO, 300MHz) δ 3.15 (br, 1H), 3.3-3.75 (br-m, 4 H), 3.9 (br, 1 H), 4.05 (br, 1H), 4.44 (br, 1H), 4.54 (br, 1H), 7.5-7.7 (m, 4 H), 8.45 (br, 1H), 8.60 (br, 1H);
The purity was determined by RPLC/MS (>99%); ESI/MS m/e 211.0 ($M^+$+H, $C_{11}H_{16}ClN_2$).

Optically active (R)-3-amino-1-(4-chlorobenzyl)pyrrolidine dihydrochloride and (S)-3-amino-1-(4-chlorobenzyl) pyrrolidine dihydrochloride were synthesized by using the respective corresponding starting materials according to the above method. The products exhibited the same $^1H$ NMR as that of the above racemate.

2) (R)-3-{(N-tert-butoxycarbonyl)glycyl}amino-1-(4-chlorobenzyl)pyrrolidine

A mixture of the (R)-3-amino-1-(4-chlorobenzyl)pyrrolidine dihydrochloride (4.54 g, 16.0 mmol) with a 2 M NaOH solution (80 mL) and ethyl acetate (80 mL) was stirred, and the organic layer was separated. The aqueous layer was extracted with ethyl acetate (80 mL × 2). The organic layers were combined, dried over anhydrous sodium sulfate, filtered and concentrated to thereby provide free (R)-3-amino-1-(4-chlorobenzyl)pyrrolidine (3.35 g, 99%).

$Et_3N$ (2.5 mL, 17.6 mmol), N-tert-butoxycarbonylglycine (2.79 g, 16.0 mmol), EDCI (3.07 g, 16.0 mmol) and HOBt (12.16 g, 16 mmol) were added to a $CH_2Cl_2$ (80 mL) solution of the (R)-3-amino-1-(4-chlorobenzyl)pyrrolidine (3.35 g, 16 mmol). The reaction mixture was stirred at 25 °C for 16 hours, and a 2 M NaOH solution (80 mL) was then added to the mixture. The organic layer was separated, and the aqueous layer was extracted with dichloromethane (100 mL × 3). The organic layers were combined and washed with water (100 mL × 2) and brine (100 mL), dried over anhydrous sodium sulfate, filtered, concentrated and purified by column chromatography ($SiO_2$, ethyl acetate) to afford the objective (R)-3-{N-(tert-butoxycarbonyl)glycyl}amino-1-(4-chlorobenzyl)pyrrolidine (5.40 g, 92%).

3) Synthesis of (R)-1-(4-chlorobenzyl)-3-(glycylamino)pyrrolidine

A 4 M HCl dioxane (38 mL) solution was added to a methanol (60 mL) solution of the (R)-3-{N-(tert-butoxycarbonyl)glycyl}amino-1-(4-chlorobenzyl)pyrrolidine (5.39 g, 14.7 mmol). The resulting solution was stirred at room temperature for 2 hours. The reaction mixture was concentrated, and a 2 M NaOH solution (80 mL) was added to the concentrate. The resulting mixture was extracted with dichloromethane (80 mL × 3), and extracts were combined, dried over anhydrous sodium sulfate, concentrated and purified by column chromatography ($SiO_2$, AcOEt:EtOH:$Et_3N$ = 90: 5:5) to provide (R)-3-(glycylamino)-1-(4-chlorobenzyl)pyrrolidine (3.374 g, 86%).

$^1$H-NMR($CDCl_3$, 270MHz) δ 1.77 (dd, J = 1.3 and 6.9 Hz, 1H), 2.20-3.39 (m, 2 H), 2.53 (dd, J = 3.3 and 9.6 Hz, 1H), 2.62 (dd, J = 6.6 and 9.6 Hz, 1H), 2.78-2.87 (m, 1H), 3.31 (s, 2 H), 3.57(s, 2 H), 4.38-4.53 (br, 1H), 7.18-7.32 (m, 4 H), 7.39 (br, s, 1H).

4) (R)-1-(4-chlorobenzyl)-3-[{N-(3-(trifluoromethylthio)benzoyl)glycyl}amino]pyrrolidine (Compd. No. 1606)

A mixture of 3-(trifluoromethylthio)benzoic acid (0.060 mmol) with (R)-1-(4-chlorobenzyl)-3-(glycylamino)pyrrohdine (0.050 mmol), diisopropylcarbodiimide (0.060 mmol), HOBt (0.060 mmol), tert-butanol (0.15 mL) and chloroform (1.35 mL) was stirred at room temperature for 15 hours. The reaction mixture was added to a Varian™ SCX column, successively washed with methanol:chloroform = 1:1 (12 mL) and methanol (12 mL), then eluted with a methanol solution of 4 M ammonia (5 mL) and concentrated to afford (R)-1-(4-chlorobenzyl)-3-[{N-(3-(trifluoromethylthio)benzoyl)glycyl}amino]pyrrolidine (Compd. No. 1606) (17.0 mg, 72%). The purity was determined by RPLC/MS (97˚/). ESI/MS m/e 472.0 ($M^+$+H, $C_{21}H_{21}ClF_3N_3O_2S$).

[Example 1] Measurement of inhibitory activity of a compound against binding of [$^{125}$I]-labeled MIP-1β to membrane fraction of the cells expressing CCR5

To a 96-well plate made of polystyrene, were respectively added 20 μ L of a solution prepared by diluting each test compound with an assay buffer (50 mM HEPES, pH 7.4, 5 mM $MgCl_2$, 1 mM $CaCl_2$, 0.2% BSA), 25 μL of a solution obtained by diluting [$^{125}$I]-labeled MIP-1β (NEN Life Science Products, Inc.) with the assay buffer so as to provide 0.1 to 0.5 nM and 155 μ L (including 4 μ g of the membrane fraction) of a suspension prepared by suspending a membrane fraction of CHO cells expressing human CCR5 (the final volume of the reaction solution: 200 μL). The solutions and suspension was stirred for 2 minutes and then incubated at 27 °C for 60 minutes.

After completing the reaction, the reaction suspension was filtered through Filtermate (Packard Instrument Co.), and the filter was washed with 250 μL of a precoded washing buffer (10 m M HEPES, pH 7.4, 0.5 M NaCl) nine times. Into each well, was added 50 μL of liquid scintillator. The radioactivity was counted using TopCount NXT (Packard Instrument Co.).

The count when 0.2 μM of human MIP-1 α instead of the test compound was added was subtracted as nonspecific binding, and the count when the test compound was not added was taken as 100%. Thereby, the inhibitory activity of the test compound against binding of the human MIP-1β to the membrane fraction of the cells expressing CCR5 was calculated.

$$\text{Inhibition (\%)} = [1 - (A - B)/(C - B)] \times 100$$

(wherein A is the count when the test compound is added; B is the count when the unlabeled human MIP-1α is added ; C is the count when only the [$^{125}$I]-labeled human MIP-1β is added).

When the inhibitory activity of the cyclic amine derivatives of the present invention was measured, for example, the following compounds respectively showed an inhibitory activity of 20% to 50%, 50% to 80% and >80% at a concentration of 10 μM.

Compounds which showed an inhibitory activity of 20% to 50% at a concentration of 10 μM:

Compd.Nos.: 132, 198, 490, 516, 521, 528, 529, 601, 616, 622, 627, 642, 684, 847, 849, 850, 857, 867, 874, 899, 902, 1002, 1003, 1057, 1083, 1189, 1245, 1247, 1472, 1606, 1859,1998, 2093, 2095, 2097 and 2134

Compounds which showed an inhibitory activity of 50% to 80% at a concentration of 10 μM:

Compd.Nos.: 461, 505, 668, 679, 782, 1042, 1073, 1114, 1559, 1583, 1609, 1703, 1718, 1783, 1833, 1836,1855,1917, 2157, 2189 and 2251

Compounds which showed an inhibitory activity of >80% at a concentration of 10 μM:

Compd. Nos. 1709, 1837, 1910, 1919, 2179, 2235 and 2241

[Example 2] Measurement of inhibitory activity of a compound against infection of cells with HIV-1

The inhibitory activity of a compound against infection of cells with HIV-1 was measured by using cells simultaneously expressing CD4 and CCR5 or human peripheral blood monocytes according to methods described in literatures (see, for example Mack, M. et al., J. Exp. Med., 1998, 187, 1215; and Baba, M. et al., Proc. Natl. Acad. Sci. USA, 1999, 96, 5698).

[Example 3] Preparation of a tablet

A tablet of the compound used in the present invention was prepared by, for example the following prescription:

| | |
|---|---|
| Compound used in the present invention | 30 mg |
| Lactose | 87 mg |
| Starch | 30 mg |
| Magnesium stearate | 3 mg |

[Example 4] Preparation of parenteral injections

Solution for injection of the compound used in the present invention was prepared by, for example the following prescription:

| | |
|---|---|
| Hydrochloride of compound used in the present invention | 30 mg |
| Sodium chloride | 900 mg |
| Distilled water for injection | 100 mL |

Industrial Applicability

The cyclic amine compound used in the present invention, pharmaceutically acceptable acid addition salt thereof or pharmaceutically acceptable $C_1$-$C_6$ alkyl addition salt thereof are CCR5 antagonist and have inhibitory actions on actions of in vivo ligands of CCR5 on target cells, and mrdicine comprising the compounds as an active ingredient, therefore, are useful as remedie or prophylactic for diseases in association with CCR5, wherein said diseases are selected from the group consisting of rejection after organ transplantation, graft-versus-host diseases (GVHD) and diabetes.

## Claims

1. Use of a compound having the CCRS antagonistic activity represented by the general formula (I), a pharmaceutically acceptable acid addition salt thereof or a pharmaceutically

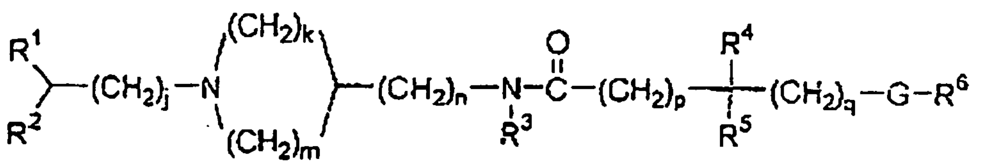

(I)

acceptable $C_1$-$C_6$ alkyl addition salt thereof as an active ingredient:
wherein, $R^1$ is a phenyl group, a $C_3$-$C_8$ cycloalkyl group or an aromatic heterocyclic group having one to three oxygen atoms, sulfur atoms and/or nitrogen atoms as heteroatoms; the phenyl group or the aromatic heterocyclic group in the above $R^1$ may be condensed with a benzene ring, or an aromatic heterocyclic group having one to three oxygen atoms, sulfur atoms and/or nitrogen atoms as heteroatoms to form a condensed ring; the phenyl group, the $C_3$-$C_8$ cycloalkyl group, the aromatic heterocyclic group or the condensed ring in the above $R^1$ may be substituted with an optional number of halogen atoms, hydroxy groups, cyano groups, nitro groups, carboxy groups, carbamoyl groups, $C_1$-$C_6$ alkyl groups, $C_3$-$C_8$ cycloalkyl groups, $C_2$-$C_6$ alkenyl groups, $C_1$-$C_6$ alkoxy groups, $C_1$-$C_6$ alkylthio groups, $C_3$-$C_5$ alkylene groups, $C_2$-$C_4$ alkylenoxy groups, $C_1$-$C_3$ alkylenedioxy groups, phenyl groups, phenoxy groups, phenylthio groups, benzyl groups, benzyloxy groups, benzoylamino groups, $C_2$-$C_7$ alkanoyl groups, $C_2$-$C_7$ alkoxycarbonyl groups, $C_2$-$C_7$ alkanoyloxy groups, $C_2$-$C_7$ alkanoylamino groups, $C_2$-$C_7$ N-alkylcarbamoyl groups, $C_4$-$C_9$ N-cycioalkylcarbamoyl groups, $C_1$-$C_6$ alkylsulfonyl groups, $C_3$-$C_8$ (alkoxycarbonyl)methyl groups, N-phenylcarbamoyl groups, piperidinocarbonyl groups, morpholinocarbonyl groups, 1-pyrrolidinylcarbonyl groups, bivalent groups represented by the formula: -NH(C=O)O-, bivalent groups represented by the formula: -NH(C=S)O-, amino groups, mono($C_1$-$C_6$ alkyl)amino groups or di($C_1$-$C_6$ alkyl)amino groups; the substituents of the phenyl group, the $C_3$-$C_8$ cycloalkyl group, the aromatic heterocyclic group or the condensed ring may further be substituted with an optional number of halogen atoms, hydroxy groups, amino groups, trifluoromethyl groups, $C_1$-$C_6$ alkyl groups or $C_1$-$C_6$ alkoxy groups;
$R^2$ is a hydrogen atom, a $C_1$-$C_6$ alkyl group, a $C_2$-$C_7$ alkoxycarbonyl group, a hydroxy group or a phenyl group; the $C_1$-$C_6$ alkyl group or the phenyl group in the $R^2$ may be substituted with an optional number of halogen atoms, hydroxy groups, $C_1$-$C_6$ alkyl groups or $C_1$-$C_6$ alkoxy groups, with the proviso that $R^2$ is not a hydroxy group when j is 0;
j is an integer of 0 to 2;
k is an integer of 0 to 2;
m is an integer of 2 to 4;
n is 0 or 1;
$R^3$ is a hydrogen atom or a $C_1$-$C_6$ alkyl group which may be substituted (with one or two phenyl groups which may respectively be substituted with the same or different optional number of halogen atoms, hydroxy groups, $C_1$-$C_6$ alkyl groups or $C_1$-$C_6$ alkoxy groups);
$R^4$ and $R^5$ are the same or different and are each a hydrogen atom, a hydroxy group, a phenyl group or a $C_1$-$C_6$ alkyl group; the $C_1$-$C_6$ alkyl group in the $R^4$ and $R^5$ may be substituted with an optional number of halogen atoms, hydroxy groups, cyano groups, nitro groups, carboxy groups, carbamoyl groups, mercapto groups, guanidino groups, $C_3$-$C_8$ cycloalkyl groups, $C_1$-$C_6$ alkoxy groups, $C_1$-$C_6$ alkylthio groups, phenyl groups (which may be substituted with an optional number of halogen atoms, hydroxy groups, $C_1$-$C_6$ alkyl groups, $C_1$-$C_6$ alkoxy groups or benzyloxy groups), phenoxy groups, benzyloxy groups, benzyloxycarbonyl groups, $C_2$-$C_7$ alkanoyl groups, $C_2$-$C_7$ alkoxycarbonyl groups, $C_2$-$C_7$ alkanoyloxy groups, $C_2$-$C_7$ alkanoylamino groups, $C_2$-$C_7$ N-alkylcarbamoyl groups, $C_1$-$C_6$ alkylsulfonyl groups, amino groups, mono($C_1$-$C_6$ alkyl)amino groups, di($C_1$-$C_6$ alkyl)amino groups, or (aromatic heterocyclic groups having one to three oxygen atoms, sulfur atoms and/or nitrogen atoms as heteroatoms or condensed rings formed by condensation of the aromatic heterocyclic groups having the one to three oxygen atoms, sulfur atoms and/or nitrogen atoms as the heteroatoms with the benzene rings), or both $R^4$ and $R^5$ together may form a three- to a six- membered cyclic hydrocarbon;
p is 0 or 1;
q is 0 or 1;
G is a group represented by -CO-, $-SO_2-$, -CO-O-, $-NR^7$-CO-, $-CO-NR^7-$, -NH-CO-NH-, -NH-CS-NH-, $-NR^7-SO_2-$, $-SO_2-NR^7-$, -NH-CO-O- or -O-CO-NH-, wherein, $R^7$ is a hydrogen atom or a $C_1$-$C_6$ alkyl group or $R^7$, together with $R^5$, may form a $C_2$-$C_5$ alkylene group;

$R^6$ is a phenyl group, a $C_3$-$C_8$ cycloalkyl group, a $C_3$-$C_6$ cycloalkenyl group, a benzyl group or an aromatic heterocyclic group having one to three oxygen atoms, sulfur atoms and/or nitrogen atoms as heteroatoms; the phenyl group, the benzyl group or the aromatic heterocyclic group in the $R^6$ may be condensed with a benzene ring or an aromatic heterocyclic group having one to three oxygen atoms, sulfur atoms and/or nitrogen atoms as heteroatoms to form a condensed ring; the phenyl group, the $C_3$-$C_8$ cycloalkyl group, the $C_3$-$C_6$ cycloalkenyl group, the benzyl group, the aromatic heterocyclic group or the condensed ring in the above $R^6$ may further be substituted with an optional number of halogen atoms, hydroxy groups, mercapto groups, cyano groups, nitro groups, thiocyanato groups, carboxy groups, carbamoyl groups, trifluoromethyl groups, $C_1$-$C_6$ alkyl groups, $C_3$-$C_8$ cycloalkyl groups, $C_2$-$C_6$ alkenyl groups, $C_1$-$C_6$ alkoxy groups, $C_3$-$C_8$ cycloalkyloxy groups, $C_1$-$C_6$ alkylthio groups, $C_1$-$C_3$ alkylenedioxy groups, phenyl groups, phenoxy groups, phenylamino groups, benzyl groups, benzoyl groups, phenylsulfinyl groups, phenylsulfonyl groups, 3-phenyhzido groups, $C_2$-$C_7$, alkanoyl groups, $C_2$-$C_7$ alkoxycarbonyl groups, $C_2$-$C_7$ alkanoyloxy groups, $C_2$-$C_7$ alkanoylamino groups, $C_2$-$C_7$ N-alkylcarbamoyl groups, $C_1$-$C_6$ alkylsulfonyl groups, phenylcarbamoyl groups, N,N-di($C_1$-$C_6$ alkyl)sulfamoyl groups, amino groups, mono($C_1$-$C_6$ alkyl)ammo groups, di($C_1$-$C_6$ alkyl)amino groups, benzylamino groups, $C_2$-$C_7$ (alkoxycarbonyl)amino groups, $C_1$-$C_6$ (alkylsulfonyl)amino groups or bis($C_1$-$C_6$ alkylsulfonyl)amino groups; the substituents of the phenyl group, the $C_3$-$C_8$ cycloalkyl group, the $C_3$-$C_8$ cycloalkenyl group, the benzyl group, the aromatic heterocyclic group or the condensed ring may further be substituted with an optional number of halogen atoms, cyano groups, hydroxy groups, amino groups, trifluoromethyl groups, $C_1$-$C_6$ alkyl groups, $C_1$-$C_6$ alkoxy groups, $C_1$-$C_6$ alkylthio groups, mono($C_1$-$C_6$ alkyl)amino groups or di ($C_1$-$C_6$ alkyl)amino groups,
in the manufacture of a medicament for the treatment of diseases associated with CCR5 selected from the group consisting of rejection after organ transplantation, graft-versus-host diseases and diabetes.

2. Use according to claim 1, wherein k is 1 and m is 2 in the above formula (I).

3. Use according to claim 1, wherein k is 0 and m is 3 in the above formula (I).

4. Use according to claim 1, wherein k is 1 and m is 3 in the above formula (I).

5. Use according to claim 1, wherein k is 2 and m is 2 in the above formula (I).

6. Use according to claim 1, wherein k is 1 and m is 4 in the above formula (I).

**Patentansprüche**

1. Verwendung einer durch die allgemeine Formel (I) dargestellten Verbindung mit CCR5-antagonistischer Aktivität, eines pharmazeutisch verträglichen Säureadditionssalzes davon oder eines pharmazeutisch verträglichen $C_1$-$C_6$ Alkyladditionssalzes davon, als ein aktiver Bestandteil,

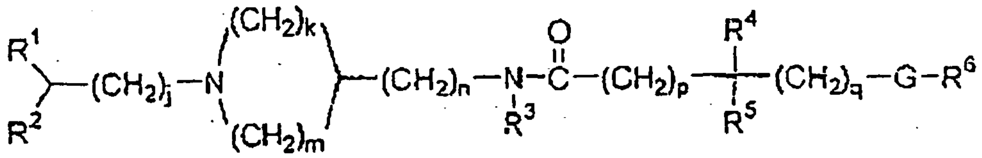

(I)

wobei $R^1$ eine Phenylgruppe, eine $C_3$-$C_8$ Cycloalkylgruppe oder eine aromatische heterocyclische Gruppe mit einem bis drei Sauerstoffatomen, Schwefelatomen und/oder Stickstoffatomen als Heteroatome ist, die Phenylgruppe oder die aromatische heterocyclische Gruppe in dem vorstehenden $R^1$ mit einem Benzolring oder einer aromatischen heterocyclischen Gruppe mit einem bis drei Sauerstoffatomen, Schwefelatomen und/oder Stickstoffatomen als Heteroatome kondensiert sein kann, um einen kondensierten Ring zu bilden, die Phenylgruppe, die $C_3$-$C_8$ Cycloalkylgruppe, die aromatische heterocyclische Gruppe oder der kondensierte Ring in dem vorstehenden $R^1$ mit einer optionalen Zahl von Halogenatomen, Hydroxylgruppen, Cyanogruppen, Nitrogruppen, Carboxylgruppen, Carbamoylgruppen, $C_1$-$C_6$ Alkylgruppen, $C_3$-$C_8$ Cycloalkylgruppen, $C_2$-$C_6$ Alkenylgruppen, $C_1$-$C_6$ Alkoxygruppen, $C_1$-$C_6$ Alkylthiogruppen, $C_3$-$C_5$ Alkylengruppen, $C_2$-$C_4$ Alkylenoxygruppen, $C_1$-$C_3$ Alkylendioxygruppen, Phenylgruppen, Phenoxygruppen, Phenylthiogruppen, Benzylgruppen, Benzyloxygruppen, Benzoylaminogruppen, $C_2$-$C_7$ Alkanoylgruppen, $C_2$-$C_7$ Alkoxycarbonylgruppen, $C_2$-$C_7$ Alkanoyloxygruppen, $C_2$-$C_7$ Alkanoylaminogruppen, $C_2$-$C_7$ N-Al-

kylcarbamoylgruppen, $C_4$-$C_9$ N-Cycloalkylcarbamoylgruppen, $C_1$-$C_6$ Alkylsulfonylgruppen, $C_3$-$C_8$ (Alkoxycarbonyl) methylgruppen, N-Phenylcarbamoylgruppen, Piperidinocarbonylgruppen, Morpholinocarbonylgruppen, 1-Pyrrolidinylcarbonylgruppen, durch die Formel -NH(C=O)O- dargestellte zweiwertige Gruppen, durch die Formel -NH(C=S) O- dargestellte zweiwertige Gruppen, Aminogruppen, Mono($C_1$-$C_6$ alkyl)aminogruppen oder Di($C_1$-$C_6$ alkyl)aminogruppen substituiert sein kann, die Substituenten der Phenylgruppe, der $C_3$-$C_8$ Cycloalkylgruppe, der aromatischen heterocyclischen Gruppe oder des kondensierten Rings weiter mit einer optionalen Zahl von Halogenatomen, Hydroxylgruppen, Aminogruppen, Trifluormethylgruppen, $C_1$-$C_6$ Alkylgruppen oder $C_1$-$C_6$ Alkoxygruppen substituiert sein können,

$R^2$ ein Wasserstoffatom, eine $C_1$-$C_6$ Alkylgruppe, eine $C_2$-$C_7$ Alkoxycarbonylgruppe, eine Hydroxylgruppe oder eine Phenylgruppe ist, die $C_1$-$C_6$ Alkylgruppe oder die Phenylgruppe in $R^2$ mit einer optionalen Zahl von Halogenatomen, Hydroxylgruppen, $C_1$-$C_6$ Alkylgruppen oder $C_1$-$C_6$ Alkoxygruppen substituiert sein kann, mit der Maßgabe, dass $R^2$ keine Hydroxylgruppe ist, wenn j 0 ist,

j eine ganze Zahl von 0 bis 2 ist,

k eine ganze Zahl von 0 bis 2 ist,

m eine ganze Zahl von 2 bis 4 ist,

n 0 oder 1 ist,

$R^3$ ein Wasserstoffatom oder eine $C_1$-$C_6$ Alkylgruppe ist, die (mit einer oder zwei Phenylgruppen, die entsprechend mit der gleichen oder unterschiedlichen optionalen Zahl von Halogenatomen, Hydroxylgruppen, $C_1$-$C_6$ Alkylgruppen oder $C_1$-$C_6$ Alkoxygruppen substituiert sein können) substituiert sein kann,

$R^4$ und $R^5$ gleich oder verschieden sind und jeweils ein Wasserstoffatom, eine Hydroxylgruppe, eine Phenylgruppe oder eine $C_1$-$C_6$ Alkylgruppe sind, wobei die $C_1$-$C_6$ Alkylgruppe in $R^4$ und $R^5$ mit einer optionalen Zahl von Halogenatomen, Hydroxylgruppen, Cyanogruppen, Nitrogruppen, Carboxylgruppen, Carbamoylgruppen, Mercaptogruppen, Guanidinogruppen, $C_3$-$C_8$ Cycloalkylgruppen, $C_1$-$C_6$ Alkoxygruppen, $C_1$-$C_6$ Alkylthiogruppen, Phenylgruppen, (die mit einer optionalen Zahl von Halogenatomen, Hydroxylgruppen, $C_1$-$C_6$ Alkylgruppen, $C_1$-$C_6$ Alkoxygruppen oder Benzyloxygruppen substituiert sein können), Phenoxygruppen, Benzyloxygruppen, Benzyloxycarbonylgruppen, $C_2$-$C_7$ Alkanoylgruppen, $C_2$-$C_7$ Alkoxycarbonylgruppen, $C_2$-$C_7$ Alkanoyloxygruppen, $C_2$-$C_7$ Alkanoylaminogruppen, $C_2$-$C_7$ N-Alkylcarbamoylgruppen, $C_1$-$C_6$ Alkylsulfonylgruppen, Aminogruppen, Mono($C_1$-$C_6$ alkyl)aminogruppen, Di($C_1$-$C_6$ alkyl)aminogruppen oder (aromatischen heterocyclischen Gruppen mit einem bis drei Sauerstoffatomen, Schwefelatomen und/oder Stickstoffatomen als Heteroatome oder kondensierten Ringen, gebildet durch Kondensation der aromatischen heterocyclischen Gruppen mit dem einem bis drei Sauerstoffatomen, Schwefelatomen und/oder Stickstoffatomen als die Heteroatome mit den Benzolringen) substituiert sein kann, oder sowohl $R^4$ als auch $R^5$ zusammen einen drei- bis sechsgliedrigen cyclischen Kohlenwasserstoff bilden können,

p 0 oder 1 ist,

q 0 oder 1 ist,

G eine durch -CO-, $-SO_2-$, -CO-O-, $-NR^7$-CO-, -CO-$NR^7$-, -NH-CO-NH-, -NH-CS-NH-, $-NR^7-SO_2-$, $-SO_2-NR^7-$, -NH-CO-O- oder -O-CO-NH-, wobei $R^7$ ein Wasserstoffatom oder eine $C_1$-$C_6$ Alkylgruppe ist oder $R^7$ zusammen mit $R^5$ eine $C_2$-$C_5$ Alkylengruppe sein kann, dargestellte Gruppe ist,

$R^6$ eine Phenylgruppe, eine $C_3$-$C_8$ Cycloalkylgruppe, eine $C_3$-$C_6$ Cycloalkenylgruppe, eine Benzylgruppe oder eine aromatische heterocyclische Gruppe mit einem bis drei Sauerstoffatomen, Schwefelatomen und/oder Stickstoffatomen als Heteroatome ist, wobei die Phenylgruppe, die Benzylgruppe oder die aromatische heterocyclische Gruppe in $R^6$ mit einem Benzolring oder einer aromatischen heterocyclischen Gruppe mit einem bis drei Sauerstoffatomen, Schwefelatomen und/oder Stickstoffatomen als Heteroatome kondensiert sein kann, um einen kondensierten Ring zu bilden, wobei die Phenylgruppe, die $C_3$-$C_8$ Cycloalkylgruppe, die $C_3$-$C_6$ Cycloalkenylgruppe, die Benzylgruppe, die aromatische heterocyclische Gruppe oder der kondensierte Ring in dem vorstehenden $R^6$ weiter mit einer optionalen Zahl von Halogenatomen, Hydroxylgruppen, Mercaptogruppen, Cyanogruppen, Nitrogruppen, Thiocyanatgruppen, Carboxylgruppen, Carbamoylgruppen, Trifluormethylgruppen, $C_1$-$C_6$ Alkylgruppen, $C_3$-$C_8$ Cycloalkylgruppen, $C_2$-$C_6$ Alkenylgruppen, $C_1$-$C_6$ Alkoxygruppen, $C_3$-$C_8$ Cycloalkyloxygruppen, $C_1$-$C_6$ Alkylthiogruppen, $C_1$-$C_3$ Alkylendioxygruppen, Phenylgruppen, Phenoxygruppen, Phenylaminogruppen, Benzylgruppen, Benzoylgruppen, Phenylsufinylgruppen, Phenylsulfonylgruppen, 3-Phenylureidogruppen, $C_2$-$C_7$ Alkanoylgruppen, $C_2$-$C_7$ Alkoxycarbonylgruppen, $C_2$-$C_7$ Alkanoyloxygruppen, $C_2$-$C_7$ Alkanoylaminogruppen, $C_2$-$C_7$ N-Alkylcarbamoylgruppen, $C_1$-$C_6$ Alkylsulfonylgruppen, Phenylcarbamoylgruppen, N,N-Di($C_1$-$C_6$ alkyl)sulfamoylgruppen, Aminogruppen, Mono($C_1$-$C_6$ alkyl)aminogruppen, Di($C_1$-$C_6$ alkyl)aminogruppen, Benzylaminogruppen, $C_2$-$C_7$ (Alkoxycarbonyl)aminogruppen, $C_1$-$C_6$ (Alkylsulfonyl)aminogruppen oder Bis($C_1$-$C_6$ alkylsulfonyl)-aminogruppen, substituiert sein kann, wobei die Substituenten der Phenylgruppe, der $C_3$-$C_8$ Cycloalkylgruppe, der $C_3$-$C_8$ Cycloalkenylgruppe, der Benzylgruppe, der aromatischen heterocyclischen Gruppe oder des kondensierten Rings weiter mit einer optionalen Zahl von Halogenatomen, Cyanogruppen, Hydroxylgruppen, Aminogruppen, Trifluoromethylgruppen, $C_1$-$C_6$ Alkylgruppen, $C_1$-$C_6$ Alkoxygruppen, $C_1$-$C_6$ Alkylthiogruppen, Mono($C_1$-$C_6$ alkyl)aminogruppen oder Di($C_1$-$C_6$ alkyl) aminogruppen substituiert sein können,

in der Herstellung eines Medikaments für die Behandlung von mit CCR5 verbundenen Krankheiten, ausgewählt aus der Gruppe, bestehend aus Abstoßung nach Organtransplantation, Abstoßungskrankheiten und Diabetes.

**2.** Verwendung nach Anspruch 1, wobei in der vorstehenden Formel (I) k 1 ist und m 2 ist.

**3.** Verwendung nach Anspruch 1, wobei in der vorstehenden Formel (I) k 0 ist und m 3 ist.

**4.** Verwendung nach Anspruch 1, wobei in der vorstehenden Formel (I) k 1 ist und m 3 ist.

**5.** Verwendung nach Anspruch 1, wobei in der vorstehenden Formel (I) k 2 ist und m 2 ist.

**6.** Verwendung nach Anspruch 1, wobei in der vorstehenden Formel (I) k 1 ist und m 4 ist.

## Revendications

**1.** Utilisation d'un composé ayant une activité antagoniste vis-à-vis de CCR5 représenté par la formule générale (I), d'un sel d'addition d'acide pharmaceutiquement acceptable de celui-ci ou d'un sel d'addition d'alkyle en $C_1$-$C_6$ pharmaceutiquement acceptable de celui-ci en tant que principe actif :

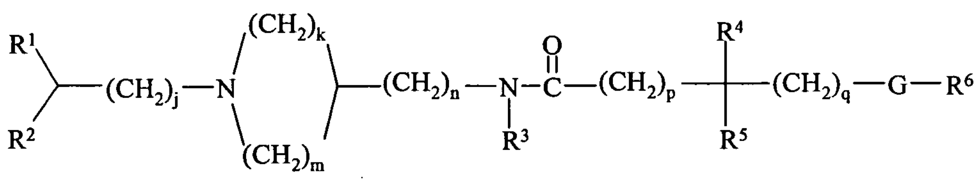

(I)

dans laquelle $R^1$ est un groupe phényle, un groupe cycloalkyle en $C_3$-$C_8$ ou un groupe hétérocyclique aromatique ayant un à trois atomes d'oxygène, atomes de soufre et/ou atomes d'azote en tant qu'hétéroatomes ; le groupe phényle ou le groupe hétérocyclique aromatique en $R^1$ ci-dessus peut être condensé avec un cycle benzène, ou un groupe hétérocyclique aromatique ayant un à trois atomes d'oxygène, atomes de soufre et/ou atomes d'azote en tant qu'hétéroatomes pour former un cycle condensé ; le groupe phényle, le groupe cycloalkyle en $C_3$-$C_8$, le groupe hétérocyclique aromatique ou le cycle condensé en $R^1$ ci-dessus peut être substitué par un nombre facultatif d'atomes d'halogène, groupes hydroxy, groupes cyano, groupes nitro, groupes carboxy, groupes carbamoyle, groupes alkyle en $C_1$-$C_6$, groupes cycloalkyle en $C_3$-$C_8$, groupes alcényle en $C_2$-$C_6$, groupes alcoxy en $C_1$-$C_6$, groupes alkylthio en $C_1$-$C_6$, groupes alkylène en $C_3$-$C_5$, groupes alkylénoxy en $C_2$-$C_4$, groupes alkylènedioxy en $C_1$-$C_3$, groupes phényle, groupes phénoxy, groupes phénylthio, groupes benzyle, groupes benzyloxy, groupes benzoylamino, groupes alcanoyle en $C_2$-$C_7$, groupes alcoxycarbonyle en $C_2$-$C_7$, groupes alcanoyloxy en $C_2$-$C_7$, groupes alcanoylamino en $C_2$-$C_7$, groupes N-alkylcarbamoyle en $C_2$-$C_7$, groupes N-cycloalkylcarbamoyle en $C_4$-$C_9$, groupes alkylsulfonyle en $C_1$-$C_6$, groupes (alcoxycarbonyl)méthyle en $C_3$-$C_8$, groupes N-phénylcarbamoyle, groupes pipéridinocarbonyle, groupes morpholinocarbonyle, groupes 1-pyrrolidinylcarbonyle, groupes divalents représentés par la formule : -NH(C=O)O-, groupes divalents représentés par la formule : -NH(C=S)O-, groupes amino, groupes mono(alkyle en $C_1$-$C_6$)amino ou di(alkyle en $C_1$-$C_6$)amino ; les substituants du groupe phényle, du groupe cycloalkyle en $C_3$-$C_8$, du groupe hétérocyclique aromatique ou du cycle condensé peuvent en outre être substitués par un nombre facultatif d'atomes d'halogène, groupes hydroxy, groupes amino, groupes trifluorométhyle, groupes alkyle en $C_1$-$C_6$ ou groupes alcoxy en $C_1$-$C_6$ ;
$R^2$ est un atome d'hydrogène, un groupe alkyle en $C_1$-$C_6$, un groupe alcoxycarbonyle en $C_2$-$C_7$, un groupe hydroxy ou un groupe phényle ; le groupe alkyle en $C_1$-$C_6$ ou le groupe phényle en $R^2$ peut être substitué par un nombre facultatif d'atomes d'halogène, groupes hydroxy, groupes alkyle en $C_1$-$C_6$ ou groupes alcoxy en $C_1$-$C_6$, à la condition que $R^2$ ne soit pas un groupe hydroxy lorsque j vaut 0 ;
j est un nombre entier de 0 à 2;
k est un nombre entier de 0 à 2;
m est un nombre entier de 2 à 4;
n vaut 0 ou 1 ;
$R^3$ est un atome d'hydrogène ou un groupe alkyle en $C_1$-$C_6$ pouvant être substitué (avec un ou deux groupes phényle pouvant respectivement être substitués par un nombre facultatif identique ou différent d'atomes d'halogène, groupes hydroxy, groupes alkyle en $C_1$-$C_6$ ou groupes alcoxy en $C_1$-$C_6$);

$R^4$ et $R^5$ sont identiques ou différents et sont chacun un atome d'hydrogène, un groupe hydroxy, un groupe phényle ou un groupe alkyle en $C_1$-$C_6$ ; le groupe alkyle en $C_1$-$C_6$ en $R^4$ ou $R^5$ peut être substitué par un nombre facultatif d'atomes d'halogène, groupes hydroxy, groupes cyano, groupe nitro, groupes carboxy, groupes carbamoyle, groupes mercapto, groupes guanidino, groupes cycloalkyle en $C_3$-$C_8$, groupes alcoxy en $C_1$-$C_6$, groupes alkylthio en $C_1$-$C_6$, groupes phényle (pouvant être substitués par un nombre facultatif d'atomes d'halogène, groupes hydroxy, groupes alkyle en $C_1$-$C_6$, groupes alcoxy en $C_1$-$C_6$ ou groupes benzyloxy), groupes phénoxy, groupes benzyloxy, groupes benzyloxycarbonyle, groupes alcanoyle en $C_2$-$C_7$, groupes alcoxycarbonyle en $C_2$-$C_7$, groupes alcanoyloxy en $C_2$-$C_7$, groupes alcanoylamino en $C_2$-$C_7$, groupes N-alkylcarbamoyle en $C_2$-$C_7$, groupes alkylsulfonyle en $C_1$-$C_6$, groupes amino, groupes mono(alkyle en $C_1$-$C_6$)amino, groupes di(alkyle en $C_1$-$C_6$)amino, ou (des groupes hétérocycliques aromatiques ayant un à trois atomes d'oxygène, atomes de soufre et/bu atomes d'azote en tant qu'hétéroatomes ou cycles condensés formés par la condensation des groupes hétérocycliques aromatiques ayant les un à trois atomes d'oxygène, atomes de soufre et/ou atomes d'azote en tant qu'hétéroatomes avec les cycles benzène), ou $R^4$ et $R^5$ peuvent tous deux former ensemble un hydrocarbure cyclique à trois à six éléments ;

p vaut 0 ou 1 ;

q vaut 0 ou 1 ;

G est un groupe représenté par -CO-, $-SO_2-$, -CO-O-, $-NR^7-CO-$, $-CO-NR^7-$ -NH-CO-NH-, -NH-CS-NH-, $-NR^7-SO_2-$, $-SO_2-NR^7-$, -NH-CO-O- ou -O-CO-NH-, où $R^7$ est un atome d'hydrogène ou un groupe alkyle en $C_1$-$C_6$ ou $R^7$, conjointement avec $R^5$, peut former un groupe alkylène en $C_2$-$C_5$ ;

$R^6$ est un groupe phényle, un groupe cycloalkyle en $C_3$-$C_8$, un groupe cycloalcényle en $C_3$-$C_6$, un groupe benzyle ou un groupe hétérocyclique aromatique ayant un à trois atomes d'oxygène, atomes de soufre et/ou atomes d'azote en tant qu'hétéroatomes ; le groupe phényle, le groupe benzyle ou le groupe hétérocyclique aromatique en $R^6$ peut être condensé avec un cycle benzène ou un groupe hétérocyclique aromatique ayant un à trois atomes d'oxygène, atomes de soufre et/ou atomes d'azote en tant qu'hétéroatomes pour former un cycle condensé ; le groupe phényle, le groupe cycloalkyle en $C_3$-$C_8$, le groupe cycloalcényle en $C_3$-$C_6$, le groupe benzyle, le groupe hétérocyclique aromatique ou le cycle condensé en $R^6$ ci-dessus peut en outre être substitué par un nombre facultatif d'atomes d'halogène, groupes hydroxy, groupes mercapto, groupes cyano, groupes nitro, groupes thiocyanato, groupes carboxy, groupes carbamoyle, groupes trifluorométhyle, groupes alkyle en $C_1$-$C_6$, groupes cycloalkyle en $C_3$-$C_8$, groupes alcényle en $C_2$-$C_6$, groupes alcoxy en $C_1$-$C_6$, groupes cycloalkyloxy en $C_3$-$C_8$, groupes alkylthio en $C_1$-$C_6$, groupes alkylènedioxy en $C_1$-$C_3$, groupes phényle, groupes phénoxy, groupes phénylamino, groupes benzyle, groupes benzoyle, groupes phénylsulfinyle, groupes phénylsulfonyle, groupes 3-phényluréido, groupes alcanoyle en $C_2$-$C_7$, groupes alcoxycarbonyle en $C_2$-$C_7$, groupes alcanoyloxy en $C_2$-$C_7$, groupes alcanoylamino en $C_2$-$C_7$, groupes N-alkylcarbamoyle en $C_2$-$C_7$, groupes alkylsulfonyle en $C_1$-$C_6$, groupes phénylcarbamoyle, groupes N,N-di(alkyle en $C_1$-$C_6$)sulfamoyle, groupes amino, groupes mono(alkyle en $C_1$-$C_6$)amino, groupes di(alkyle en $C_1$-$C_6$) amino, groupes benzylamino, groupes (alcoxycarbonyl)amino en $C_2$-$C_7$, groupes (alkylsulfonyl)amino en $C_1$-$C_6$ ou groupes bis(alkylsulfonyle en $C_1$-$C_6$)amino ; les substituants du groupe phényle, du groupe cycloalkyle en $C_3$-$C_8$, du groupe cycloalcényle en $C_3$-$C_8$, du groupe benzyle, du groupe hétérocyclique aromatique ou du cycle condensé peuvent en outre être substitués par un nombre facultatif d'atomes d'halogène, groupes cyano, groupes hydroxy, groupes amino, groupes trifluorométhyle, groupes alkyle en $C_1$-$C_6$, groupes alcoxy en $C_1$-$C_6$, groupes alkylthio en $C_1$-$C_6$, groupes mono(alkyle en $C_1$-$C_6$)amino ou groupes di(alkyle en $C_1$-$C_6$)amino,

dans la fabrication d'un médicament destiné au traitement de maladies associées au CCR5 choisies dans le groupe constitué par un rejet après une greffe d'organe, les réactions du greffon contre hôte et le diabète.

**2.** Utilisation selon la revendication 1, dans laquelle k vaut 1 et m vaut 2 dans la formule (I) ci-dessus.

**3.** Utilisation selon la revendication 1, dans laquelle k vaut 0 et m vaut 3 dans la formule (I) ci-dessus.

**4.** Utilisation selon la revendication 1, dans laquelle k vaut 1 et m vaut 3 dans la formule (I) ci-dessus.

**5.** Utilisation selon la revendication 1, dans laquelle k vaut 2 et m vaut 2 dans la formule (I) ci-dessus.

**6.** Utilisation selon la revendication 1, dans laquelle k vaut 1 et m vaut 4 dans la formule (I) ci-dessus.